# EUROPÄISCHE PATENTSCHRIFT

(11) **EP 2 513 057 B1**
(45) Veröffentlichungstag und Bekanntmachung des Hinweises auf die Patenterteilung: **04.09.2013**
(21) Anmeldenummer: 10782202.5
(22) Anmeldetag: 22.11.2010
(51) Int. Cl.: C07D 213/63, C07D 239/42, C07D 251/40, C07D 401/14, C07D 403/04, C07D 295/145, A61K 31/4427, A61K 31/506, A61K 31/53, A61P 35/00, A61P 29/00

(54) **INHIBITOREN DER SPHINGOSINKINASE**
SPHINGOSINE KINASE INHIBITORS
INHIBITEURS DE LA SPHINGOSINE KINASE

(30) Priorität: 14.12.2009 EP 09015422; 18.12.2009 EP 09015754
(43) Veröffentlichungstag der Anmeldung: 24.10.2012
(73) Patentinhaber: Merck Patent GmbH, 64293 Darmstadt (DE)
(72) Erfinder: STIEBER, Frank, 69121 Heidelberg (DE); WIENKE, Dirk, 64287 Darmstadt (DE)
(86) Internationale Anmeldenummer: PCT/EP2010/007057
(87) Internationale Veröffentlichungsnummer: WO 2011/072791

(56) Entgegenhaltungen:
- WO-A1-2009/146112
- WO-A2-2006/138660
- WO-A2-2007/100610
- US-A1- 2007 032 531
- ZOOROB, H. H. ET AL: "Reactivity of 2-cinnamoyl-5,6,7,8-tetrahydronaphthalene towards ethyl cyanoacetate, cyanoacetamide and some reactions with the adducts formed", INDIAN JOURNAL OF CHEMISTRY, SECTION B: ORGANIC CHEMISTRY INCLUDING MEDICINAL CHEMISTRY , 16B(1), 53-6 CODEN: IJSBDB; ISSN: 0376-4699, 1978, XP009143770,

## Beschreibung

### Technisches Gebiet

Die vorliegende Erfindung betrifft Inhibitoren der Sphingosinkinase sowie ihre physiologisch unbedenklichen Salze, Solvate, Tautomere und Stereoisomere, einschließlich deren Mischungen in allen Verhältnissen, zur Verwendung in der Behandlung von Krankheiten, die durch Inhibierung von Sph-Kinase 1 beeinflußt werden.

### Hintergrund der Erfindung

Der Erfindung lag die Aufgabe zugrunde, neue Verbindungen mit wertvollen Eigenschaften aufzufinden, insbesondere solche, die zur Herstellung von Arzneimitteln verwendet werden können.

Die vorliegende Erfindung betrifft Verbindungen und die Verwendung von Verbindungen, zur Behandlung von Krankheiten, die mit einer Erhöhung des Sphingosinphosphatspiegels einhergehen, ferner pharmazeutische Zusammensetzungen, die diese Verbindungen enthalten.

Im einzelnen betrifft die vorliegende Erfindung Verbindungen der Formel (I), die bevorzugt das Enzym Sphingosinkinase 1 hemmen, das den Sphingosinphosphatspiegel durch Phosphorylierung von Sphingosin reguliert, Zusammensetzungen, die diese Verbindungen enthalten, sowie Verfahren zu ihrer Verwendung zur Behandlung von Krankheiten und Leiden wie Krebs, Tumorentstehung, -wachstum und -verbreitung, Arteriosklerose, Augenerkrankungen, choroidale Neovaskularisierung und diabetische Retinopathie, Entzündungserkrankungen, Arthritis, Neurodegeneration, Restenose, Herzerkrankungen, Wundheilung oder Transplantatabstossung. Insbesondere eignen sich die erfindungsgemäßen Verbindungen zur Therapie von Krebserkrankungen.

Sphingosinphosphat gehört zu der Molekülfamilie der Sphingolipide, die, neben ihrer Rolle als strukturelle Bausteine von Zellmembranen, auch wichtige Funktionen als extra- und intrazelluläre Signalmoleküle ausüben. Sphingosinphosphat (S1P) entsteht in der Zelle aus Sphingomyelin, das zunächst zu Ceramid und Sphingosin abgebaut und Letzteres durch Sphingosinkinasen phosphoryliert wird. Von den zwei bislang identifizierten Sphingosinkinasen wird Sphingosinkinase 1 (SphK1) die größere Bedeutung bei der Bildung von S1P im Serum zugeschrieben (Zemann et al., 2006 Blood Vol 107 Seite 1454). Während Ceramid und Sphingosin Zelltod und Zellwachstumshemmung induzieren (Kolesnick 2002, J Clin Invest Vol 110, Seite 3; Ogretmen et al. 2004 Nat Rev Cancer Vol 4, Seite 604), hat Sphingosinphosphat einen gegenteiligen Effekt auf die Zelle und steigert die Widerstandsfähigkeit gegen Apoptose, das Zellwachstum und den Ausstoß von Botenstoffen, die die Durchblutung des Gewebes und damit auch von Tumoren fördern (Cuvilier et al. 1996, Nature Vol 381, Seite 800; Perez et al. 1997, Nat Med Vol 3, Seite 1228). Das Verhältnis von Ceramid und Sphingosin auf der einen Seite und S1P auf der anderen entscheidet folglich über das Zellwachstum und durch Inhibierung der SphK 1 kann somit nicht nur die Bildung des wachstumsfördernden Sphingosinphosphats unterbunden, sondern auch die zelluläre Konzentration der wachstumshemmenden Moleküle Ceramid und Sphingosin erhöht werden.

Eine Vielzahl von zellulären Effekten, die durch S1 P ausgelöst werden, wird durch Sekretion des S1 P und dessen Bindung an bislang 5 verschiedene G-Proteingekoppelte Rezeptoren (bezeichnet als S1P₁₋₅) vermittelt. Die Signalweiterleitung erfolgt wiederum über verschiedene G-Proteine (Gᵢ, G_{q}, G_{12/13}), so dass eine Reihe unterschiedlicher zellulärer Signalwege, wie z.B. ERK oder PI3K aktiviert werden, die insbesondere bei Krebsentstehung und -wachstum wichtig sind. Eine wachsende Zahl an Publikationen zeigt außerdem, dass S 1 P ein wichtiger Faktor bei der tumoralen Angiogense ist. Angiogenese ist ein wichtiger Vorgang beim Tumorwachstum, durch den ausgehend von bereits bestehenden Blutgefäßen neue gebildet werden und somit die Versorgung des Tumors mit Nährstoffen gesichert wird. Aus diesem Grund ist die Hemmung der Angiogenese ein wichtiger Ansatzpunkt der Krebs- und Tumortherapie. (Folkman, 2007, Nature Reviews Drug Discovery Vol. 6, Seite 273-286). S1 P stimuliert chemotaktische Bewegung von Endothelzellen und induziert die Differenzierung zu multizellulären Strukturen, beides frühe Schritte bei der Bildung von neuen Blutgefäßen (Lee et al. 1999 Biochem Biophys Res Commun Vol 264 Seite 325; Argraves et al. 2004 J Biol Chem Vol 279 Seite 50580). Außerdem fördert S1P die Wanderung von knochenmarkstämmigen Endothelvorläuferzellen zu neovaskulären Initiationsstellen (Annabi et al. 2003 Exp Hematology Vol 31 Seite 640) und transaktiviert den Rezeptor von VEGF, einer der wichtigsten proangiogenen Faktoren insbesondere in der Tumorbiologie (Tanimoto et al. 2002 J Biol Chem Vol 277 Seite 42997; Endo et al. 2002 J Biol Chem Vol 277 Seite 23747). Ein direkter Beweis der Aktivität von S1 P in der Tumorangiogenese konnte durch Experimente mit einem Antikörper, der an S1P spezifisch bindet, erbracht werden. Der S1P-Antikörper inhibierte die Wanderung und die Gefäßbildung von Endothelzellen in vitro, blockierte die S1P-abhängige Sekretion von proangiogenen Faktoren wie VEGF, IL-8 und IL-6 in vitro und in vivo und reduzierte signifikant das Wachstum von Tumormodellen der Brust, Lunge und Ovarien in Maus-Xenograftexperimenten (Visentin 2006 Cancer Cell Vol 9 Seite 225).

Daneben hat S1P auch intrazelluläre Funktionen, wie zum Beispiel die Aktivierung des Transkriptionsfaktors NF-κB, der bei der Apoptoseresistenz von Krebszellen eine große Rolle spielt (Xia et al. 2002 J Biol Chem Vol 277 Seite 7996). Allerdings sind die intrazellulären Interaktionspartner von S1 P noch nicht identifiziert.

Daraus folgert, dass im Gegensatz zu einer ebenfalls denkbaren Intervention mit der krebsfördernden Wirkung von S1P durch pharmakologische Blockade der extrazellulären Rezeptoren eine Inhibierung des für die S1P-Bildung verantwortlichen Enzyms SphK1 den Vorteil aufweist, damit auch die intrazellulären Aktivitäten von S1P zu unterbinden. Unterstützt wird dieser Ansatz durch Untersuchungen von Xia et al. (2000 Curr Biol Vol 10 Seite 1527), die zeigen, dass nichttumorgene Fibroblasten durch ektopische Expression von SphK1 transformiert werden und in Mäusen Tumore bilden können. SphK1 kann somit als Onkogen klassifiziert werden. In verschiedene Expressionstudien konnten erhöhte SphK1-mRNA-Konzentrationen in Tumorgeweben des Gehirns, der Brust, der Lunge, der Eierstöcke, des Magens, der Gebärmutter, der Nieren sowie des Dünn- und des Dickdarms gegenüber gesundem Gewebe festgestellt werden (French et al. 2003 Cancer Research Vol. 63 Seite 5962; Johnson et al. 2005 J Histochem Cytochem Vol 53 Seite 1159; Van Brocklyn et al. 2005 J Neuropathol Exp Neurol Vol 64 Seite 695). Darüber hinaus korreliert eine erhöhte Expression von SphK1 mit schlechterer Prognose in Patienten mit Glioblastoma Multiform (Van Brocklyn et al. 2005 J Neuropathol Exp Neurol Vol 64 Seite 695).

Eine wichtige Rolle kommt SphK1 bei der Modulierung der durch Chemotherapeutika induzierten Apoptose von Krebszellen zu. So erhöht sich durch Überexpremierung von SphK1 die Resistenz von Brustkrebs-, Prostatakrebs und Leukämiezellen gegenüber Chemotherapeutika wie Anthrazyklinen, Docetaxel, Camptothecin oder Doxorubicin (Nava et al. 2002 Exp Cell Res Vol 281 Seite 115; Pchejetski 2005 Cancer Res Vol 65 Seite 11667; Bonhoure 2006 Leukemia Vol 20 Seite 95). Es konnte gezeigt werden, dass die vermehrte Anwesenheit von SphK1 zu einer Verschiebung des Ceramid/S1P-Gleichgewichts in Richtung des apoptoseresistenzvermittelnden S1P führt. Ein möglicher Mechanismus ist dabei die Inhibierung des mitochondrialen Cytochrome C-Ausstoßes durch SphK1, was normalerweise ein frühes Ereignis beim programmierten Zelltod darstellt (Cuvilier et al. 2001 Blood Vol 98 Seite 2828; Bonhoure 2006 Leukemia Vol 20 Seite 95).

In umgekehrter Weise kann durch gezielte Blockade der SphK1-Expremierung mittels siRNA in Tumorzellmodellen verschiedener Indikationen wie Leukämie, Brustkrebs, Glioblastoma oder Prostatakrebs Apoptose ausgelöst oder die Wirkung von Chemotherapeutika gesteigert werden (Bonhoure 2006 Leukemia Vol 20 Seite 95; Taha et al. 2004 J Biol Chem Vol 279 Seite 20546; Taha et al. 2006 FASEB J Vol 20 Seite 482; Van Brocklyn et al. 2005 J Neuropathol Exp Neurol Vol 64 Seite 695; Pchejetski 2005 Cancer Res Vol 65 Seite 11667).

In einem Mausmodell konnte gezeigt werden, dass durch Überexpression von SphK1 degenerative Veränderungen von Kardiomyozyten und myocardiale Fibrose ausgelöst wird, die sich mit zunehmendem Alter der Versuchstiere verstärkte. Untermauert wird eine Funktion des S1P-Signalweges in Herzerkrankungen auch dadurch, dass in Mäuse, in denen die Expression des S1P3-Rezeptors gezielt unterbunden wurde, die Bildung von cardiovaskulären Fibrosen stark inhibiert ist (Takuwa 2008 Biochimica and Biophysica Acta in press). Auch in anderen Organen, wie zum Beispiel der Lunge kommt S1P eine Rolle bei der Differenzierung von Fibroblasten hin zu Myofibroblasten und somit bei der Entstehung und Progression von fibrosen Erkrankungen zu (Kono et al. 2007 Am J Respir Cell Mol Biol Seite 395).

Es wurde gefunden, dass die erfindungsgemäßen Verbindungen eine spezifische Inhibierung der Sphingosinkinase 1, aber nicht der Sphingosinkinase 2 bewirken. Die erfindungsgemäßen Verbindungen zeigen bevorzugt eine vorteilhafte biologische Aktivität, die in den, zum Beispiel hierin beschriebenen, Tests nachweisbar ist. In derartigen Tests zeigen und bewirken die erfindungsgemäßen Verbindungen einen inhibierenden Effekt, der gewöhnlich durch IC₅₀-Werte in einem geeigneten Bereich, bevorzugt im mikromolaren Bereich und bevorzugter im nanomolaren Bereich dokumentiert wird.

Generell können alle soliden und nicht soliden Tumore mit den Verbindungen der Formel (I) behandelt werden, wie z.B. die Monozytenleukämie, Hirn-, Urogenital-, Lymphsystem-, Magen-, Kehlkopf- Ovarial- und Lungenkarzinom, darunter Lungenadenokarzinom und kleinzelliges Lungenkarzinom. Zu weiteren Beispielen zählen Prostata-, Bauchspeicheldrüsen- und Brustkarzinom.

Wie hierin besprochen, sind Wirkungen der erfindungsgemäßen Verbindung für verschiedene Erkrankungen relevant. Dementsprechend sind die erfindungsgemäßen Verbindungen nützlich bei der Prophylaxe und/oder Behandlung von Erkrankungen, die durch Inhibierung von SphK1 beeinflusst werden.

Gegenstand der vorliegenden Erfindung sind deshalb erfindungsgemäße Verbindungen als Arzneimittel und/oder Arzneimittelwirkstoffe bei der Behandlung und/oder Prophylaxe der genannten Erkrankungen und die Verwendung von erfindungsgemäßen Verbindungen zur Herstellung eines Pharmazeutikums für die Behandlung und/oder Prophylaxe der genannten Erkrankungen wie auch ein Verfahren zur Behandlung der genannten Erkrankungen umfassend die Verabreichung eines oder mehrerer erfindungsgemäßer Verbindungen an einen Patienten mit Bedarf an einer derartigen Verabreichung..

Der Wirt oder Patient kann jeglicher Säugerspezies angehören, z.B. einer Primatenspezies, besonders Menschen; Nagetieren, einschließlich Mäusen, Ratten und Hamstern, Kaninchen, Pferden, Rindern, Hunden, Katzen usw. Tiermodelle sind für experimentelle Untersuchungen von Interesse, wobei sie ein Modell zur Behandlung einer Krankheit des Menschen darstellen.

Die Sensitivität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer kombiniert, die ausreicht, um den aktiven Mitteln zu ermöglichen, die intrazelluläre S1P-Konzentration zu senken und darüber hinaus die Sekretion von angiogenesefördernden Substanzen zu blockieren oder Zelltod zu induzieren. Zum Testen in vitro können kultivierte Zellen aus einer Biopsieprobe oder etablierte Krebszelllinien, in denen SphK1 expremiert wird, verwendet werden.

Die Dosis variiert abhängig von der verwendeten spezifischen Verbindung, der spezifischen Erkrankung, dem Patientenstatus usw. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z.B. mindestens ca. 50 % Verminderung der Zelllast und kann fortgesetzt werden, bis im Wesentlichen keine unerwünschten Zellen mehr im Körper nachgewiesen werden können.

### Verwendung

Wie in der Einleitung beschrieben sind SphK1, S1P und dessen Zelloberflächenrezeptoren S1P₁₋₅ in einer Vielzahl physiologischer und pathophysiologischer Prozesse involviert. Aus diesem Grund ist zu erwarten, dass die Hemmung der SphK1 durch die hier beschriebenen Substanzen bei verschiedenen Erkrankungen zu therapeutischen Zwecken genutzt werden kann.

Die Bildung von S1P durch SphK1 und die damit verbundene Verschiebung des Ceramid/S1P-Gleichgewichts führt, wie oben ausgeführt, dazu, dass die Zellen stärker proliferieren und widerstandsfähiger gegen apoptotische Stimuli werden. Daraus lässt sich eine generelle Funktion von SphK1 bei hyperproliferativen Erkrankungen wie Krebs, Psoriasis, Restenosen und Arteriosklerose ableiten. Die dieser Erfindung zugrunde liegenden Verbindungen der Formel I, die SphK1 hemmen und damit den S1P-Spiegel regulieren und/oder modulieren, Zusammensetzungen, die diese Verbindungen enthalten, sowie die beschriebenen Verfahren können somit zur Behandlung dieser Krankheiten eingesetzt werden. Generell können alle soliden und nicht soliden Tumore mit den Verbindungen der Formel X behandelt werden, wie z.B. die Monozytenleukämie, Hirn-, Urogenital-, Lymphsystem-, Magen-, Kehlkopf- Ovarial- und Lungenkarzinom, darunter Lungenadenokarzinom und kleinzelliges Lungenkarzinom. Zu weiteren Beispielen zählen Darm-, Prostata-, Bauchspeicheldrüsen- und Brustkarzinom.

Neben der Funktion beim Zellwachstum spielt S1P auch eine Rolle bei der Neubildung von Blutgefäßen (Angiogenese). Bei vielen Krankheitsprozessen steht die Angiogenese entweder ursächlich im Mittelpunkt der Erkrankung oder wirkt sich verschlimmernd auf die Progression der Erkrankung aus. Beispielsweise im Krebsgeschehen führt die Angiogenese dazu, dass der Tumor sich vergrößern und in andere Organe übertreten kann. Weitere Erkrankungen, bei denen Angiogenese eine wichtige Rolle spielt sind Psoriasis, Arthrose, Arteriosklerose sowie Augenerkrankungen wie diabetische Retinopathie, altersbedingte makulare Degeneration, Rubeosis iridis oder neovasculares Glaukom. Die dieser Erfindung zugrunde liegenden Verbindungen der Formel I, die SphK1 hemmen und damit den S1 P-Spiegel regulieren und/oder modulieren, Zusammensetzungen, die diese Verbindungen enthalten, sowie die beschriebenen Verfahren können somit zur Behandlung dieser Krankheiten eingesetzt werden.

Des Weiteren beeinflussen SphK1 und S1P die Proliferation, Differenzierung, Migration und Sekretion von Immunzellen (Rosen und Goetzl 2005 Nat Rev Immunol Vol 5 Seite 560) und sind somit an verschiedenen Funktionen des Immunsystems und an Entzündungsprozessen beteiligt. Stimulierung des Immunsystems steigert die Bildung und den Ausstoß von S1P in Mastzellen, Blutplättchenzellen und einigen mononukleären Phagozyten (Stunff et al. 2004 J Cell Biochem Vol 92 Seite 882; Olivera und Rivera 2005 j Immunol Vol 174 Seite 1153). Die Aktivität von SphK1 wird insbesondere durch Faktoren wie Tumour-Necrosis-Factor (TNF) und Vernetzung von IgG-Rezeptoren stark erhöht (Stunff et al. 2004 J Cell Biochem Vol 92 Seite 882; Delon et al. 2004 J Biol Chem Vol 279 Seite 44763). Es konnte außerdem gezeigt werden, dass SphK1 und S1 P für die TNF-abhängige Bildung proinflammatorischer Enzyme wie Cyclooxygenase-2 (COX-2) und Nitric Oxide Synthase (NOS) wichtig sind (Pettus et al. 2003 FASEB J Vol 17 Seite 1411; Kwon et al.2001 J Biol Chem Vol 276 Seite 10627-33). Die dieser Erfindung zugrunde liegenden Verbindungen der Formel I, die SphK1 hemmen und damit den S1P-Spiegel regulieren und/oder modulieren, Zusammensetzungen, die diese Verbindungen enthalten, sowie die beschriebenen Verfahren können somit zur Behandlung von entzündungsbedingten Krankheiten wie Arthrose, Arteriosklerose, Psoriasis, Multiple Sklerose, chronisch-entzündlichen Darmerkrankungen (Morbus Crohn, Colitis ulcerosa) Asthma und andere allergische Erkrankungen eingesetzt werden.

Weiterhin können die Verbindungen der Formel (I) zur Isolierung und zur Untersuchung der Aktivität oder Expression von Sph-Kinase verwendet werden. Außerdem eigenen sie sich insbesondere zur Verwendung in diagnostischen Verfahren zu Erkrankungen im Zusammenhang mit unregulierter oder gestörter Sph-Kinase-Aktivität.

Es kann gezeigt werden, dass die erfindungsgemäßen Verbindungen in einem Xenotransplantat-Tumor-Modell eine in vivo antiproliferative Wirkung aufweisen. Die erfindungsgemäßen Verbindungen werden an einen Patienten mit einer hyperproliferativen Erkrankung verabreicht, z.B. zur Inhibition des Tumorwachstums, zur Verminderung der mit einer lymphoproliferativen Erkrankung einhergehenden Entzündung, zur Inhibition der Transplantatabstoßung oder neurologischer Schädigung aufgrund von Gewebereparatur usw. Die vorliegenden Verbindungen sind nützlich für prophylaktische oder therapeutische Zwecke. Wie hierin verwendet, wird der Begriff "Behandeln" als Bezugnahme sowohl auf die Verhinderung von Krankheiten als auch die Behandlung vorbestehender Leiden verwendet. Die Verhinderung von Proliferation wird durch Verabreichung der erfindungsgemäßen Verbindungen vor Entwicklung der evidenten Krankheit, z.B. zur Verhinderung des Tumorwachstums, Verhinderung metastatischen Wachstums, der Herabsetzung von mit kardiovaskulärer Chirurgie einhergehenden Restenosen usw. erreicht. Als Alternative werden die Verbindungen zur Behandlung andauernder Krankheiten durch Stabilisation oder Verbesserung der klinischen Symptome des Patienten verwendet.

Der Wirt oder Patient kann jeglicher Säugerspezies angehören, z.B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Suszeptibilität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer kombiniert, die ausreicht, um den aktiven Mitteln zu ermöglichen, Zelltod zu induzieren oder Migration zu inhibieren, gewöhnlich zwischen ungefähr einer Stunde und einer Woche. Zum Testen in vitro können kultivierte Zellen aus einer Biopsieprobe verwendet werden. Die nach der Behandlung zurückbleibenden lebensfähigen Zellen werden dann gezählt.

Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z.B. mindestens ca. 50 % Verminderung der Zelllast und kann fortgesetzt werden, bis im Wesentlichen keine unerwünschten Zellen mehr im Körper nachgewiesen werden.

Zur Identifizierung eines Signalübertragungswegs und zum Nachweis von Wechselwirkungen zwischen verschiedenen Signalübertragungswegen wurden von verschiedenen Wissenschaftlern geeignete Modelle oder Modellsysteme entwickelt, z.B. Zellkulturmodelle (z.B. Khwaja et al., EMBO, 1997, 16, 2783-93) und Modelle transgener Tiere (z.B. White et al., Oncogene, 2001, 20, 7064-7072). Zur Bestimmung bestimmter Stufen in der Signalübertragungskaskade können wechselwirkende Verbindungen genutzt werden, um das Signal zu modulieren (z.B. Stephens et al., Biochemical J., 2000, 351, 95-105). Die erfindungsgemäßen Verbindungen können auch als Reagenzien zur Testung kinaseabhängiger Signalübertragungswege in Tieren und/oder Zellkulturmodellen oder in den in dieser Anmeldung genannten klinischen Erkrankungen verwendet werden.

Zur Identifizierung eines Signalübertragungswegs und zum Nachweis von Wechselwirkungen zwischen verschiedenen Signalübertragungswegen wurden von verschiedenen Wissenschaftlern geeignete Modelle oder Modellsysteme entwickelt, z.B. Zellkulturmodelle (z.B. Khwaja et al., EMBO, 1997, 16, 2783-93) und Modelle transgener Tiere (z.B. White et al., Oncogene, 2001, 20, 7064-7072). Zur Bestimmung bestimmter Stufen in der Signalübertragungskaskade können wechselwirkende Verbindungen genutzt werden, um das Signal zu modulieren (z.B. Stephens et al., Biochemical J., 2000, 351, 95-105). Die erfindungsgemäßen Verbindungen können auch als Reagenzien zur Testung kinaseabhängiger Signalübertragungswege in Tieren und/oder Zellkulturmodellen oder in den in dieser Anmeldung genannten klinischen Erkrankungen verwendet werden.

Die Messung der Kinaseaktivität ist eine dem Fachmann wohlbekannte Technik. Generische Testsysteme zur Bestimmung der Kinaseaktivität mit Substraten, z.B. Histon (z.B. Alessi et al., FEBS Lett. 1996, 399, 3, Seiten 333-338) oder dem basischen Myelinprotein sind in der Literatur beschrieben (z.B. Campos-González, R. und Glenney, Jr., J.R. 1992, J. Biol. Chem. 267, Seite 14535).

Zur Identifikation von Kinase-Inhibitoren stehen verschiedene Assay-Systeme zur Verfügung. Beim Scintillation-Proximity-Assay (Sorg et al., J. of. Biomolecular Screening, 2002, 7, 11-19) und dem FlashPlate-Assay wird die radioaktive Phosphorylierung eines Proteins, Peptids oder im Fall von SphK1 eines Lipids als Substrat mit gamma-ATP gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer- (HTR-FRET-) und Fluoreszenzpolarisations- (FP-) Technologien als Assay-Verfahren nützlich (Sills et al., J. of Biomolecular Screening, 2002, 191-214).

Ein anderes nicht radioaktives ELISA-Assay-Verfahren verwendet einen spezifische Antikörper gegen S1 P zur Quantifizierung von S1 P (Assaysystem der Firma Echelon).

Es gibt viele mit einer Deregulation der Zellproliferation und des Zelltods (Apoptose) einhergehende Erkrankungen. Die Leiden von Interesse schließen die folgenden Leiden ein, sind aber nicht darauf beschränkt. Die erfindungsgemäßen Verbindungen sind nützlich bei der Behandlung einer Reihe verschiedener Leiden, bei denen Proliferation und/oder Migration glatter Muskelzellen und/oder Entzündungszellen in die Intimaschicht eines Gefäßes vorliegt, resultierend in eingeschränkter Durchblutung dieses Gefäßes, z.B. bei neointimalen okklusiven Läsionen. Zu okklusiven Transplantat-Gefäßerkrankungen von Interesse zählen Atherosklerose, koronare Gefäßerkrankung nach Transplantation, Venentransplantatstenose, peri-anastomotische Prothesenrestenose, Restenose nach Angioplastie oder Stent-Platzierung und dergleichen.

### Stand der Technik

In der WO 2003/077921 sind Azinylaminoazole als Proteinkinase-Inhibitoren beschrieben.

In der WO 2003/078423 sind Zusammensetzungen beschrieben, die als Proteinkinase-Inhibitoren geeignet sind.

In der WO 2003/078426 und der WO 2003/078427 sind Azolylaminoazine als Proteinkinase-Inhibitoren beschrieben.

In der WO 2004/043925 sind 3-substituierte 6-Arylpyridine beschrieben, die als Liganden von C5a Rezeptoren wirken.

In der WO 2004/058149 sind 1-(Amino)indane und (1,2-Dihydro-3-amino)benzofurane, Benzothophene und Indole als ADG Rezeptoragonisten beschrieben.

In der WO 2007/100610 sind Pyridin-, Pyrimidin- und Pyrazin-Derivate als CXCR3 Rezeptormodulatoren beschrieben.

WO 2006/138660, WO 2009/146112 und US 2007/0032531 beschreiben Inhibitoren der Sphingosinkinase.

### Zusammenfassung der Erfindung

Die Erfindung betrifft Verbindungen gemäß der Formel (I) 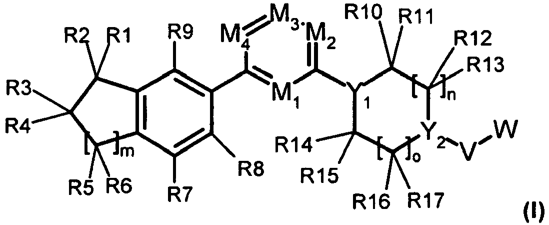 worin jeweils unabhängig voneinander bedeuten:
- R¹, R², R³, R⁴,:
- R⁵, R⁶, R⁷, R⁸, R⁹,:
- R¹⁰, R¹¹, R¹², R¹³, R¹⁴, R¹⁵, R¹⁶, R¹⁷: H, D (Deuterium), A, OR¹⁸, CN, F, Cl und NR¹⁸R^{18'}; wobei R¹ und R², R³ und R⁴, R⁵ und R⁶, R¹⁰ und R¹¹, R¹² und R¹³, R¹⁴ und R¹⁵, R¹⁶ und R¹⁷ zusammen jeweils auch =O (Carbonylsauerstoff) bilden können; wobei R⁹ und R¹, R¹ und R², R² und R³, R³ und R⁴, R⁴ und R⁵, R⁵ und R⁶, R⁶ und R⁷, R⁷ und R⁸, R¹⁰ und R¹¹, R¹¹ und R¹², R¹² und R¹³ , R¹⁴ und R¹⁵, R¹⁵ und R¹⁶, R¹⁶ und R¹⁷ zusammen jeweils auch cyclisches Alkyl mit 3, 4, 5, 6 oder 7 C-Atomen oder Het mit 3, 4, 5, 6 oder 7 Ringatomen bilden können; wobei R¹⁰ und R¹⁹, wenn Y₁ = CR¹⁹, R¹¹ und R¹², R¹³ und R¹⁹, wenn Y₂ = CR¹⁹, R¹⁴ und R¹⁹, wenn Y₁ = CR¹⁹, R¹⁵ und R¹⁶, R¹⁷ und R¹⁹, wenn Y₂ = CR¹⁹ zusammen jeweils auch mit der Einfachbindung und den C-Atomen, an denen sie befestigt sind, eine C=C-Doppelbindung bilden können;
- R¹⁸, R^{18'}: H, D oder A;
- R¹⁹, R^{19'}: H, D, A, OR¹⁸, NR¹⁸R^{18'}, F, Cl, Br, CN oder Het;
- M₁, M₂, M₃, M₄: CR¹⁹ oder N;
- Y₁, Y₂: CR¹⁹ oder N;
- V: C(R¹⁹)(R¹⁹), NR¹⁹ oder fehlend;
- W: [C(R¹⁹)(R^{19'})]ₚZ, CO-[C(R¹⁹)(R^{19'})]ₚZ, [C(R¹⁹)(R^{19'})]ₚN(R¹⁹)-Z, CO-N(R¹⁹)-[C(R¹⁹)(R^{19'})]ₚZ, N(R¹⁹)-CO-[C(R¹⁹)(R¹⁹)]ₚZ, CO-O-[C(R¹⁹)(R^{19'})]ₚZ, C(O)OR¹⁸, OR¹⁸, H oder D;
- wobei: V, W und Y₂ zusammen jeweils auch cyclisches Alkyl mit 3, 4, 5, 6 oder 7 C-Atomen, worin bevorzugt 1, 2, 3, 4, 5, 6 oder 7 H-Atome durch F, Cl, Br, CN und/oder OH, OR¹⁹, OC(O)R¹⁹, NR¹⁹C(O)OZ, C(O)OR¹⁹, C(O)N(R¹⁹)(R^{19'}) oder N(R¹⁹)(R^{19'}) ersetzt sein können; oder Het mit 3, 4, 5, 6 oder 7 Ringatomen bilden können, wobei Het bevorzugt einen gesättigten, ungesättigten oder aromatischen Heterocyclus mit 1 bis 4 N-, O- und/oder S-Atomen darstellt, der unsubstituiert oder ein-, zwei- oder dreifach durch Hal, F, Cl, Br, CN, A, OR¹⁸, W, SR¹⁸, NO₂, N(R¹⁹)(R¹⁹), NR¹⁸COOZ, OCONHZ, NR¹⁸SO₂Z, SO₂N(R¹⁸)Z, S(O)ₘZ, COZ, CHO, COZ, =S, =NH, =NA, Oxy (-O⁻) und/oder =O (Carbonylsauerstoff) substituiert sein kann;
- Z: Het, Ar oder A;
- A: unverzweigtes oder verzweigtes Alkyl mit 1, 2, 3, 4, 5, 6, 7, 8, 9 oder 10 C-Atomen, worin 1, 2, 3, 4, 5, 6 oder 7 H-Atome durch F, Cl, Br, CN und/oder OH, OR¹⁹, OC(O)R¹⁹, NR¹⁹C(O)OZ, C(O)OR¹⁹, C(O)N(R¹⁹)(R^{19'}) oder N(R¹⁹)(R^{19'}) ersetzt sein können; und/oder worin eine oder zwei CH₂-Gruppen durch O, S, SO, SO₂, CO, COO, NR¹⁸, NR¹⁸CO, CONR¹⁸, cyclisches Alkyl mit 3, 4, 5, 6 oder 7 C-Atomen, CH=CH und/oder CH≡CH-Gruppen ersetzt sein können; oder cyclisches Alkyl mit 3, 4, 5, 6 oder 7 C-Atomen, worin bevorzugt 1, 2, 3, 4, 5, 6 oder 7 H-Atome durch F, Cl, Br, CN und/oder OH, OR¹⁹, OC(O)R¹⁹, NR¹⁹C(O)OZ, C(O)OR¹⁹, C(O)N(R¹⁹)(R^{19'}) oder N(R¹⁹)(R^{19'}) ersetzt sein können;
- Ar: ein-, zwei- oder dreifach durch Hal, F, Cl, Br, CN, A, OR¹⁸, W, SR¹⁸, NO₂, N(R¹⁹)(R¹⁹), NR¹⁸COOZ, OCONHZ, NR¹⁸SO₂Z, SO₂N(R¹⁸)Z, S(O)ₘZ, COZ, CHO, COZ substituiertes Phenyl, Naphthyl oder Biphenyl,
- Het: jeweils unabhängig voneinander einen ein-, zwei- oder dreikernigen gesättigten, ungesättigten oder aromatischen Heterocyclus mit 1 bis 4 N-, O- und/oder S-Atomen, der unsubstituiert oder ein-, zwei- oder dreifach durch Hal, F, Cl, Br, CN, A, OR¹⁸, W, SR¹⁸, NO₂, N(R¹⁹)(R^{19'}), NR¹⁸COOZ, OCONHZ, NR¹⁸SO₂Z, SO₂N(R¹⁸)Z, S(O)ₘZ, COZ, CHO, COZ, =S, =NH, =NA, Oxy (-O⁻) und/oder =O (Carbonylsauerstoff) substituiert sein kann,
- m: 1, 2 oder 3,
- n, o: 0, 1 oder 2,
- p: 0, 1, 2, 3 oder 4 mit der Maßgabe, dass Verbindungen der Formel (I) ausgeschlossen sind,
worin
(a) V fehlend ist, und
(b) W = C(O)-CH₂-Het ist;
**mit der weiteren Maßgabe, dass die folgende Verbindung ebenfalls**
**ausgeschlossen ist:** 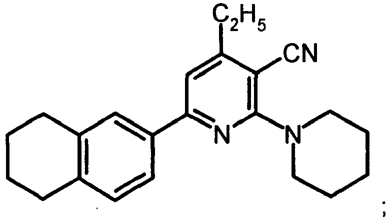 sowie ihre physiologisch unbedenklichen Salze, Solvate, Tautomere und Stereoisomere, einschließlich deren Mischungen in allen Verhältnissen.

Die Erfindung betrifft ferner bevorzugte jeweils unabhängige Ausführungsformen von Verbindungen gemäß der Formel (1), worin jeweils unabhängig voneinander bedeuten:

Bevorzugte Ausführungsform (A): R¹, R², R³, R⁴, R⁵, R⁶, R⁷, R⁸, R⁹, R¹⁰, R¹¹, R¹² , R¹³ R¹⁴, R¹⁵, R¹⁶, R¹⁷ H, D, A, I OR¹⁸ oder NR¹⁸R^{18'};
wobei R¹ und R², R³ und R⁴, R⁵ und R⁶, R¹⁰ und R¹¹, R¹² und R¹³, R¹⁴ und R¹⁵, R¹⁶ und R¹⁷ zusammen jeweils auch =O (Carbonylsauerstoff) bilden können;
wobei R⁹ und R¹, R¹ und R², R² und R³, R³ und R⁴, R⁴ und R⁵, R⁵ und R⁶, R⁶ und R⁷, R⁷ und R⁸, R¹⁰ und R¹¹, R¹¹ und R¹², R¹² und R¹³, R¹⁴ und R¹⁵, R¹⁵ und R¹⁶, R¹⁶ und R¹⁷ zusammen jeweils auch cyclisches Alkyl mit 3, 4, 5, 6 oder 7 C-Atomen oder Het mit 3, 4, 5, 6 oder 7 Ringatomen bilden können;

Bevorzugte Ausführungsform (B): R¹⁹, R^{19'} H, A, OR¹⁸, F;

Bevorzugte Ausführungsform (C): M₁, M₂, M₃, M₄ CR¹⁹;

Bevorzugte Ausführungsform (D): M₁ N, und M₂, M₃, M₄ CR¹⁹;

Bevorzugte Ausführungsform (E): M₂ N, und M₁, M₃, M₄ CR¹⁹;

Bevorzugte Ausführungsform (F): M₄ N, und M₁, M₂, M₃ CR¹⁹;

Bevorzugte Ausführungsform (G): M₁, M₂ N, und M₃, M₄ CR¹⁹;

Bevorzugte Ausführungsform (H): M₁, M₃ N, und M₂, M₄ CR¹⁹;

Bevorzugte Ausführungsform (J): M₁, M₄ N, und M₂, M₃ CR¹⁹;

Bevorzugte Ausführungsform (K): M₁, M₂, M₄ N, und M₃ CR¹⁹;

Bevorzugte Ausführungsform (L): Y₁, Y₂ N;

Bevorzugte Ausführungsform (M): Y₁ N, und Y₂ CR¹⁹;

Bevorzugte Ausführungsform (N): Y₁ CR¹⁹ und Y₂ N;

Bevorzugte Ausführungsform (O): W [C(R¹⁹)(R^{19'})]ₚZ, CO-[C(R¹⁹)(R^{19'})]ₚZ, CO-O-[C(R¹⁹)(R^{19'})]ₚZ, [C(R¹⁹)(R^{19'})]ₚN(R¹⁹)-Z, N(R¹⁹)-CO-[C(R¹⁹)(R^{19'})]ₚZ, C(O)OR¹⁸, OR¹⁸ oder H;

Bevorzugte Ausführungsform (P): Z Het oder A;

Bevorzugte Ausführungsform (Q): m 1 oder 2;

Bevorzugte Ausführungsform (R): n, o 0, 1 oder 2;

Bevorzugte Ausführungsform (S): p 0, 1 oder 2;
sowie jeweils deren physiologisch unbedenklichen Salze, Solvate, Tautomere und Stereoisomere, einschließlich deren Mischungen in allen Verhältnissen.

Die Erfindung betrifft ferner in einer bevorzugten Ausführungsform Verbindungen gemäß Formel (I) sowie hier dargestellten bevorzugten Ausführungsformen, worin jeweils unabhängig voneinander bedeuten:

R¹, R², R³, R⁴, R⁵, R⁶, R⁷, R⁸, R⁹, R¹⁰, R¹¹, R¹², R¹³, R¹⁴, R¹⁵, R¹⁶, R¹⁷ H, D, A, OR¹⁸ oder NR¹⁸R^{18'};
wobei R¹ und R², R³ und R⁴, R⁵ und R⁶, R¹⁰ und R¹¹, R¹² und R¹³, R¹⁴ und R¹⁵, R¹⁶ und R¹⁷ zusammen jeweils auch =O (Carbonylsauerstoff) bilden können;
wobei R⁹ und R¹, R¹ und R², R² und R³, R³ und R⁴, R⁴ und R⁵, R⁵ und R⁶, R⁶ und R⁷, R⁷ und R⁸, R¹⁰ und R¹¹, R¹¹ und R¹², R¹² und R¹³, R¹⁴ und R¹⁵, R¹⁵ und R¹⁶ , R¹⁶ und R¹⁷ zusammen jeweils auch cyclisches Alkyl mit 3, 4, 5, 6 oder 7 C-Atomen oder Het mit 3, 4, 5, 6 oder 7 Ringatomen bilden können;
R¹⁹, R^{19'} H, A, OR¹⁸, F;
M₁, M₂, M₃, M₄ CR¹⁹, oder
M₁ N, und M₂, M₃, M₄ CR¹⁹, oder
M₂ N, und M₁, M₃, M₄ CR¹⁹, oder
M₄ N, und M₁, M₂, M₃ CR¹⁹, oder
M₁, M₂ N, und M₃, M₄ CR¹⁹, oder
M₁, M₃ N, und M₂, M₄ CR¹⁹, oder
M₁, M₄ N, und M₂, M₃ CR¹⁹, oder
M₁, M₂, M₄ N, und M₃ CR¹⁹;
- Y₁, Y₂: N, oder
- Y₁: N, und Y₂ CR¹⁹, oder
- Y₁: CR¹⁹ und Y₂ N;
- W: [C(R¹⁹)(R^{19'})]ₚZ, CO-[C(R¹⁹)(R^{19'})]ₚZ, CO-O-[C(R¹⁹)(R^{19'})]ₚZ, [C(R¹⁹)(R^{19'})]ₚN(R¹⁹)-Z, N(R¹⁹)-CO-[C(R¹⁹)(R^{19'})]ₚZ, C(O)OR¹⁸, OR¹⁸ oder H;
- Z: Het oder A;
- m: 1 oder 2;
- n, o: 0, 1 oder 2;
- p: 0, 1 oder 2;
sowie ihre physiologisch unbedenklichen Salze, Solvate, Tautomere und Stereoisomere, einschließlich deren Mischungen in allen Verhältnissen.

Die Erfindung betrifft weiterhin Verbindungen ausgewählt aus der Gruppe bestehend aus: 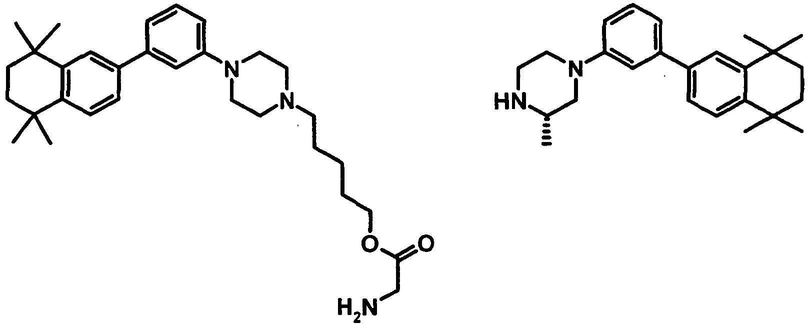 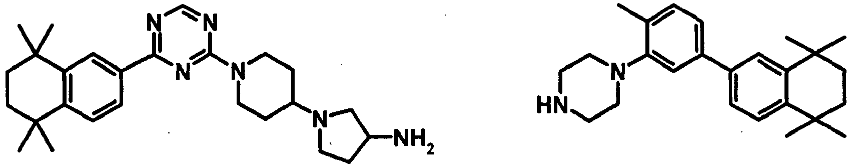 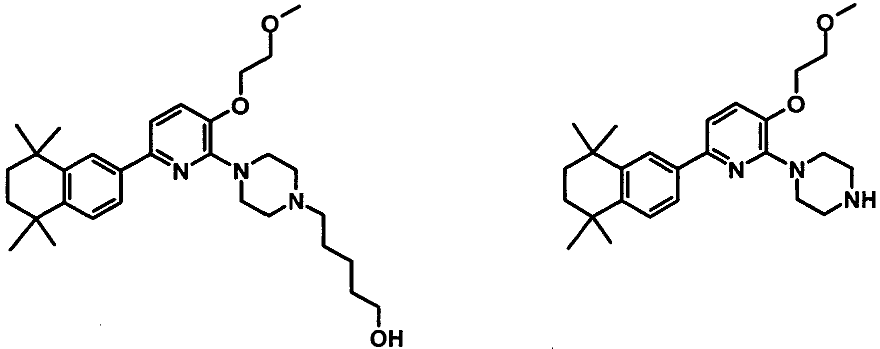 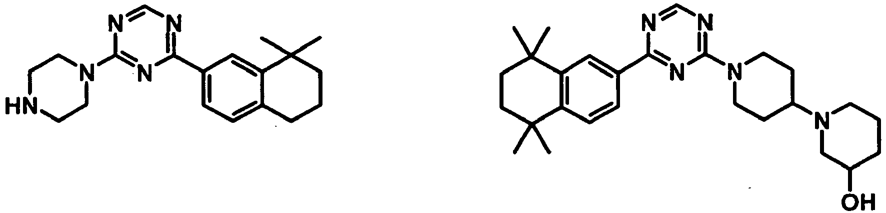 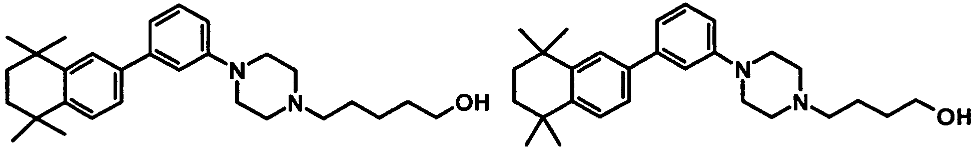 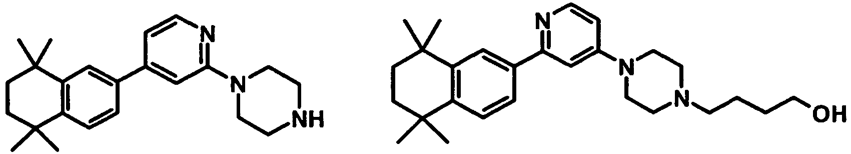 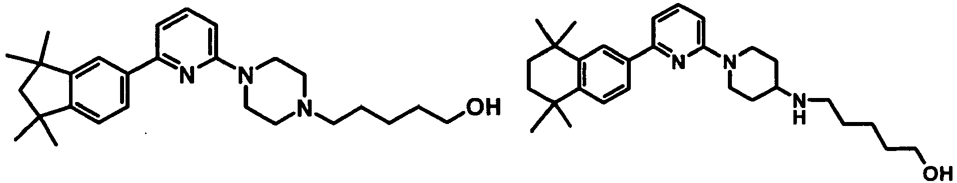 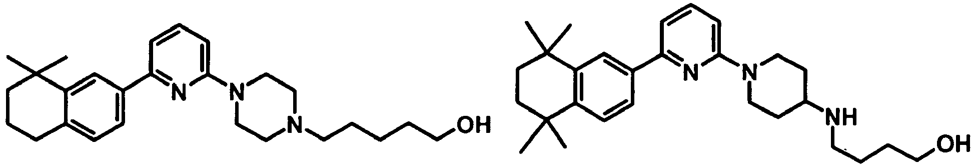 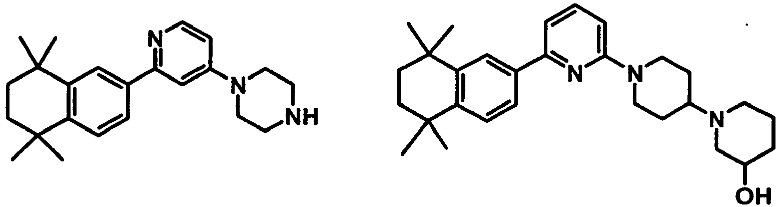 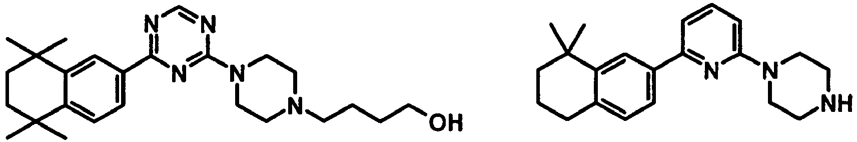 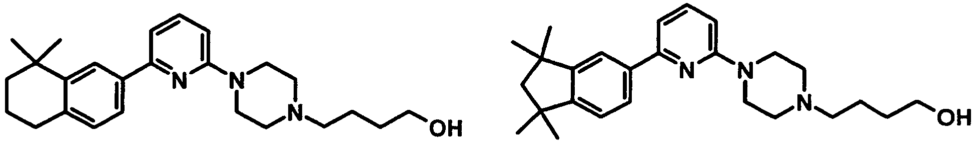 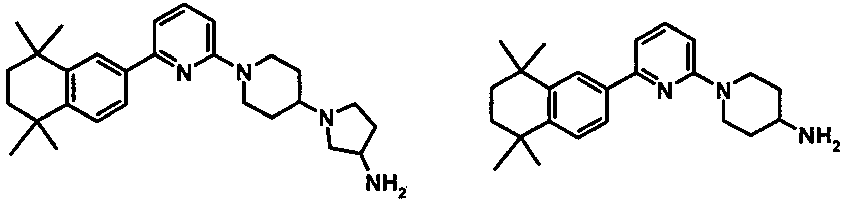 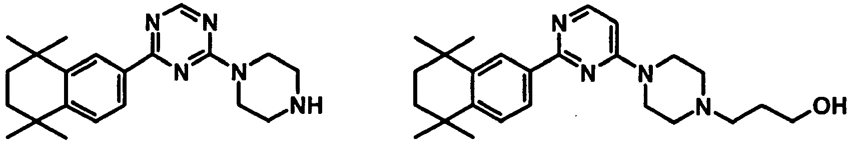 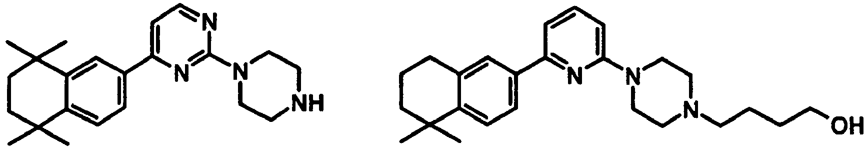 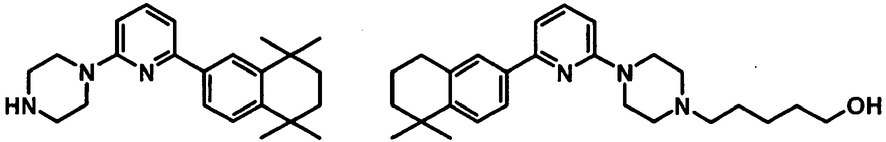 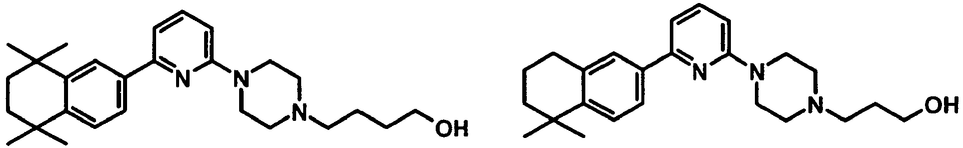 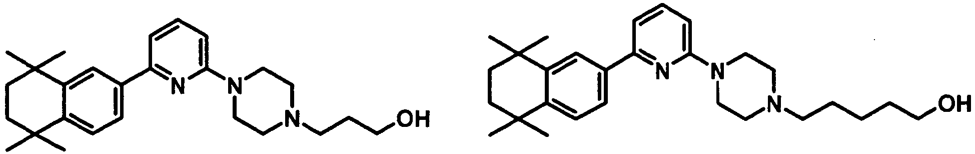 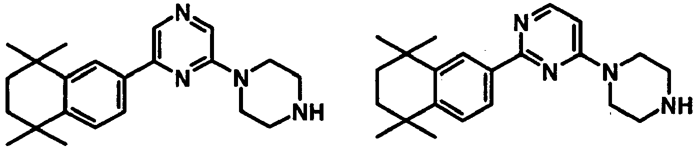 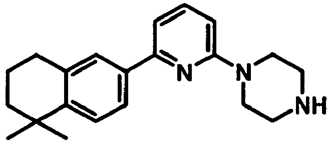 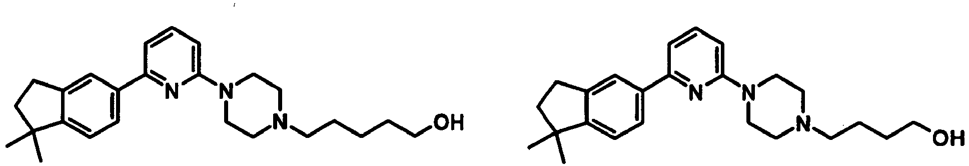 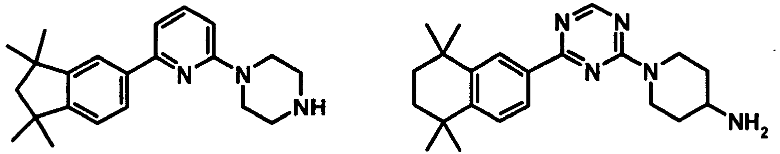 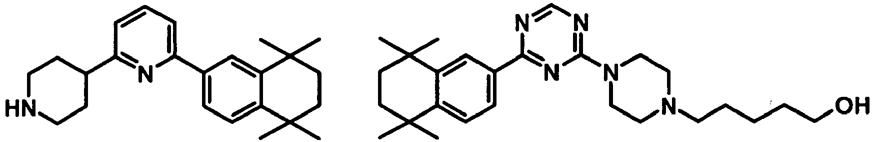 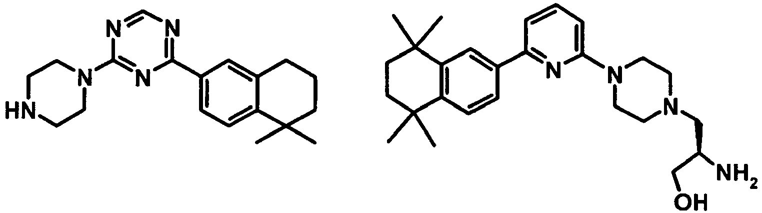 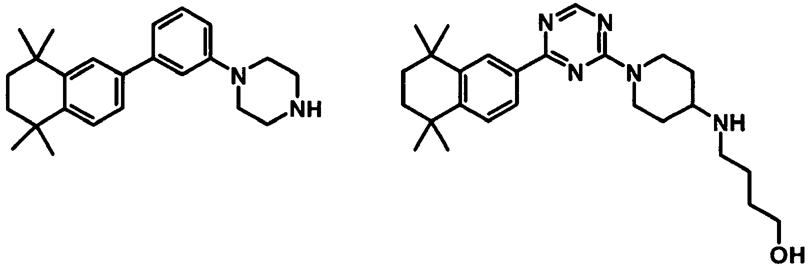 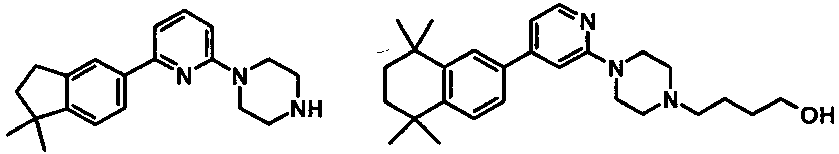 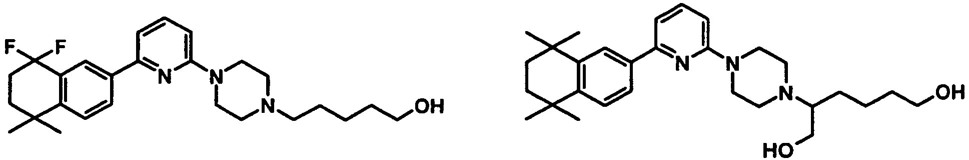 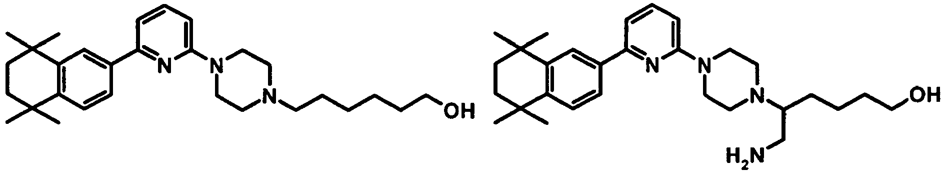 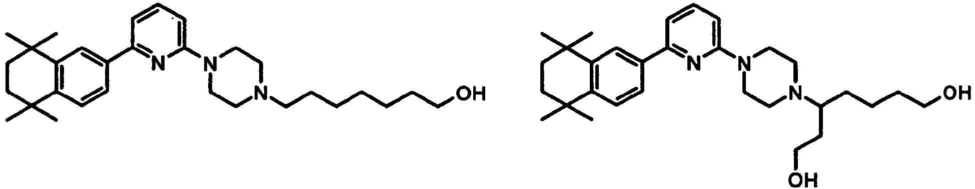 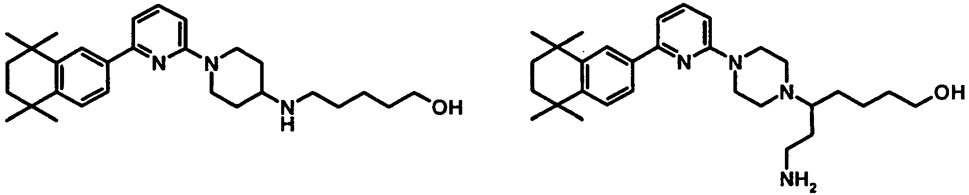 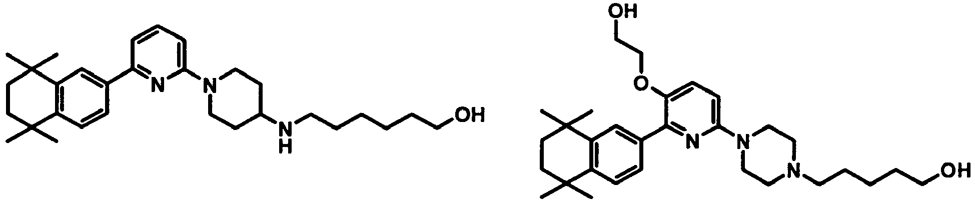 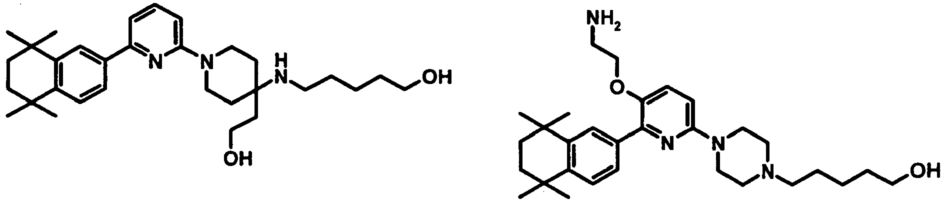 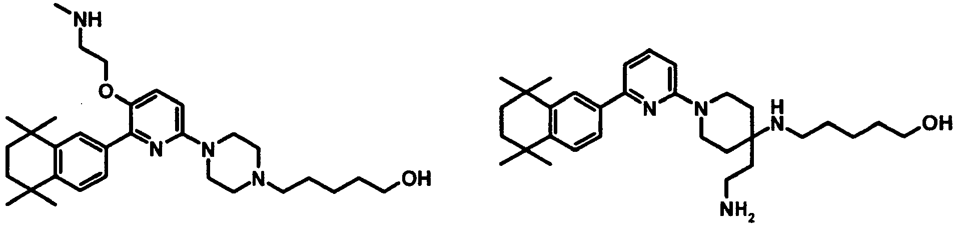 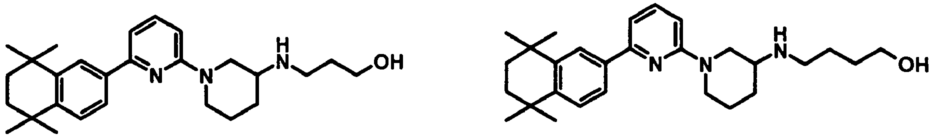 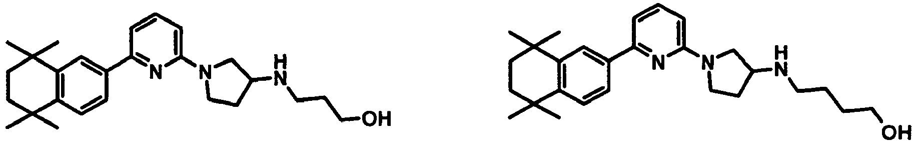 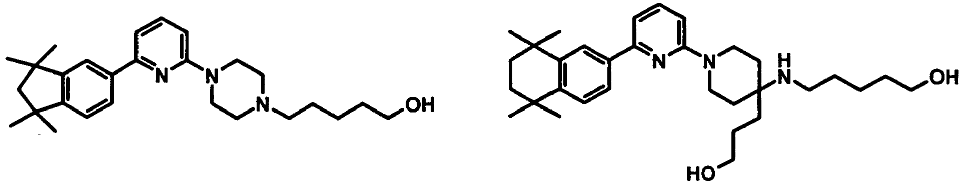 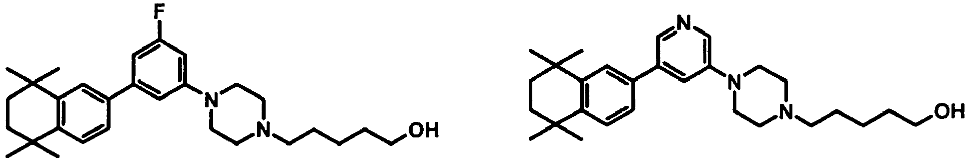 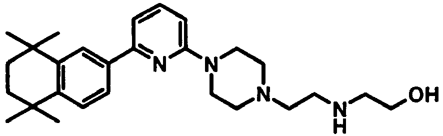 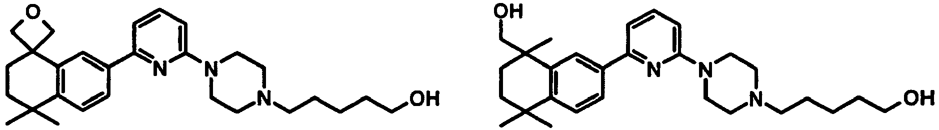 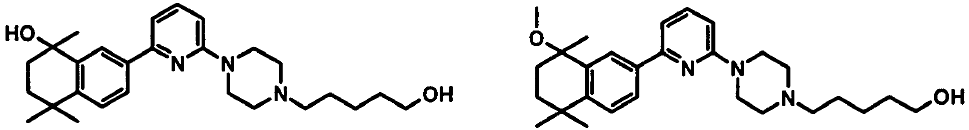 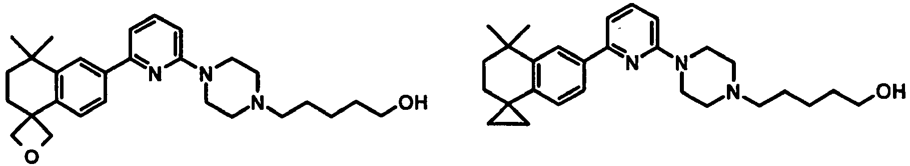 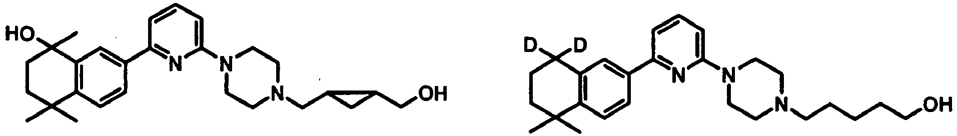 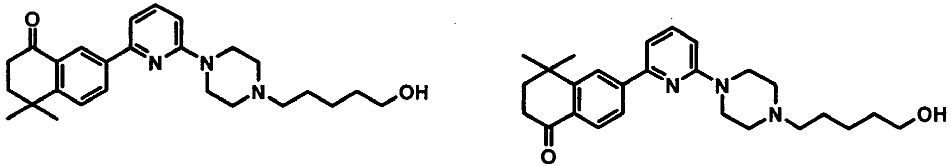 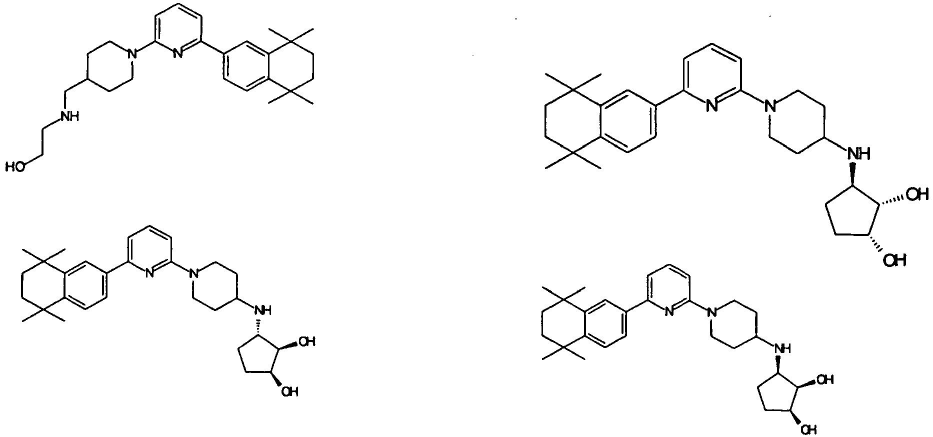 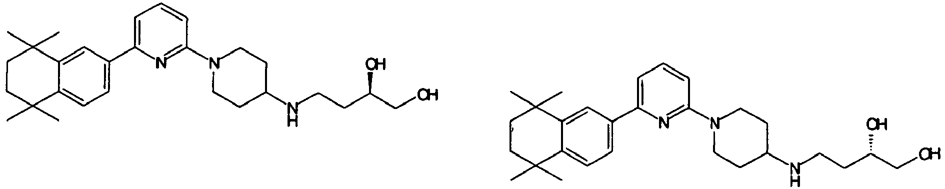 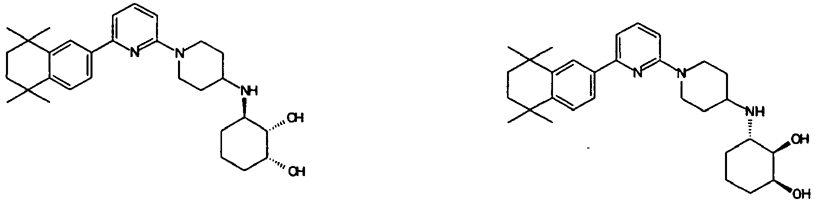 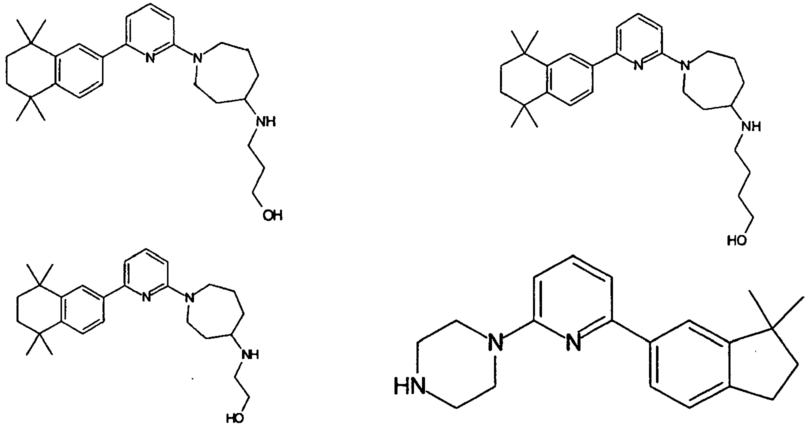 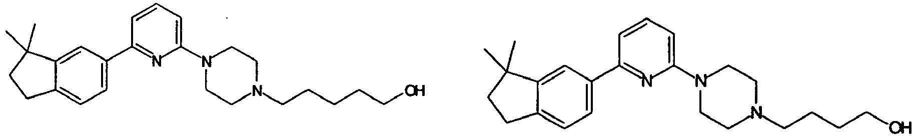 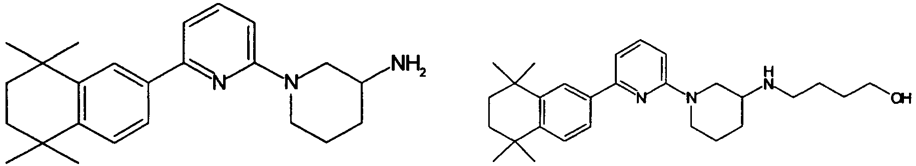 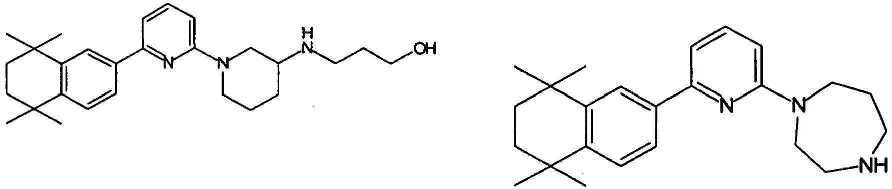 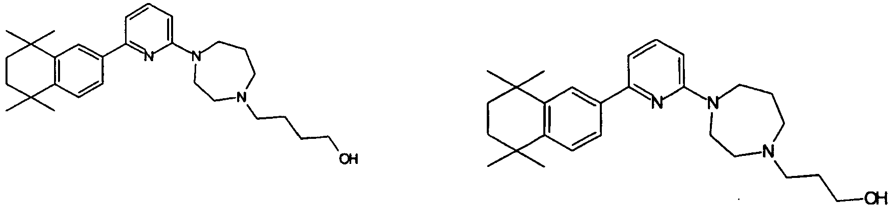 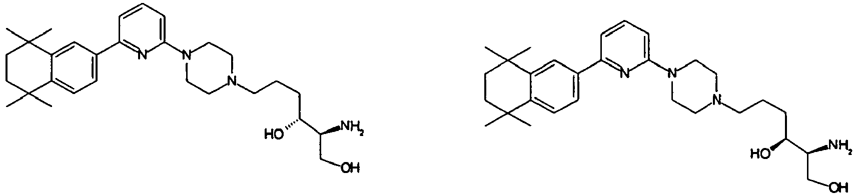 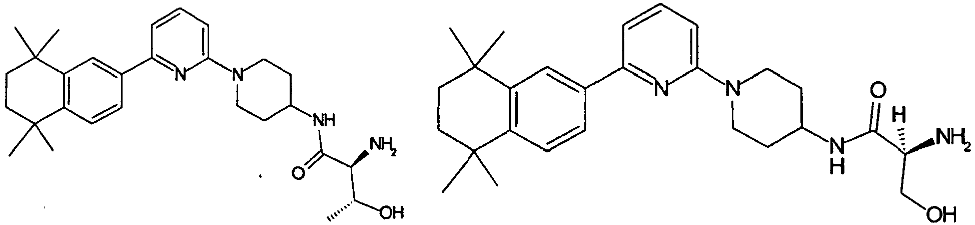 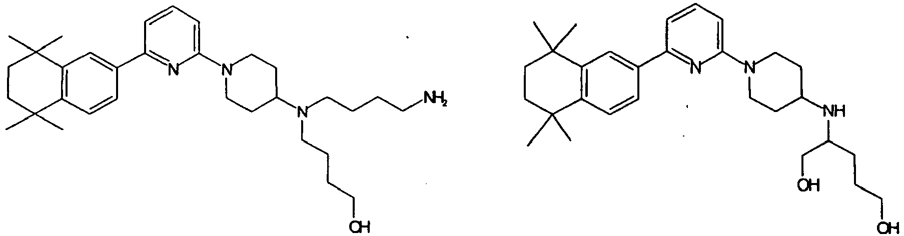 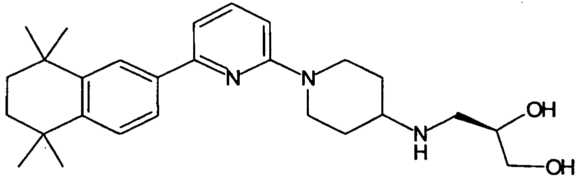 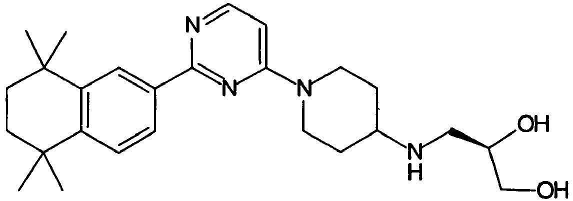 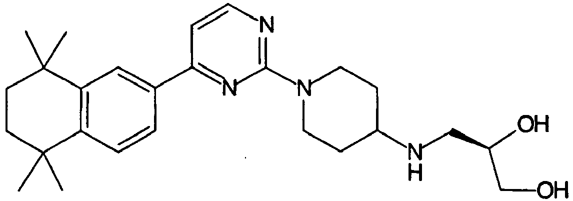 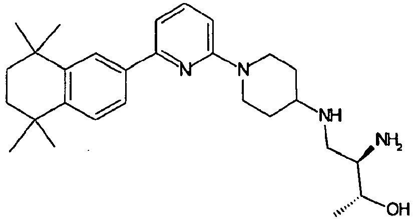 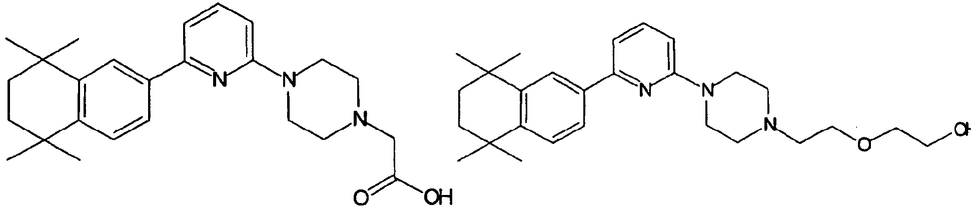 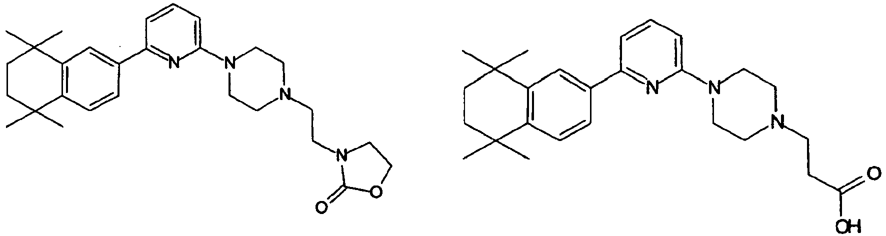 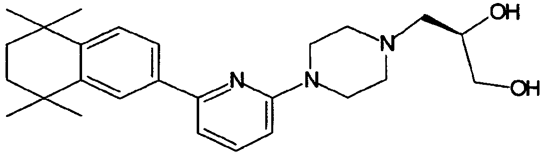 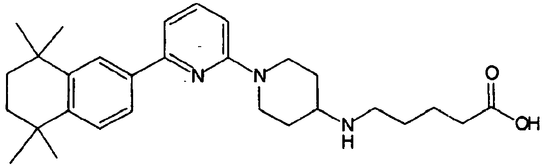 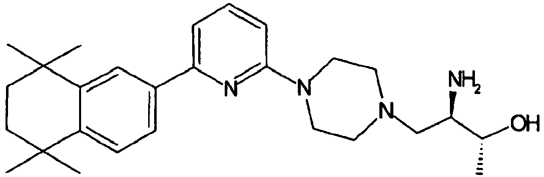 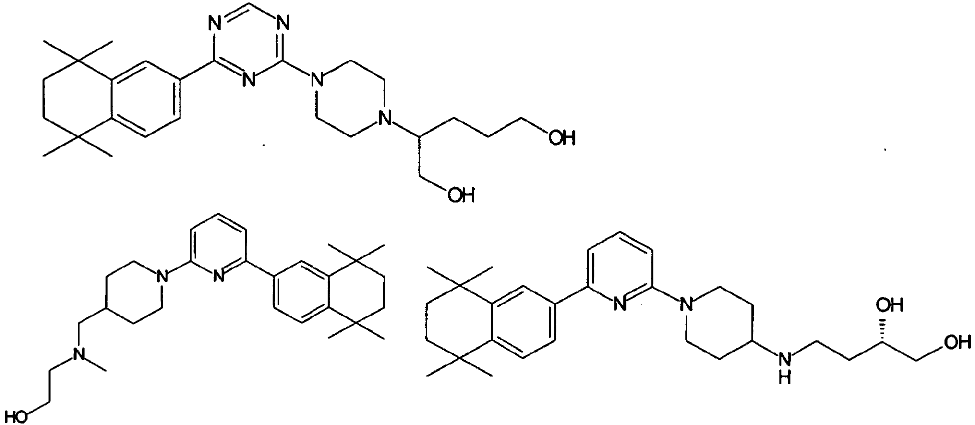 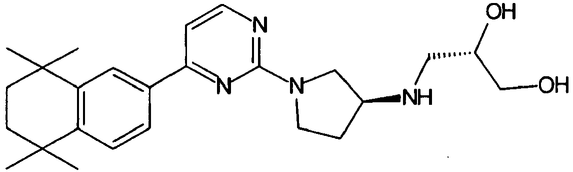 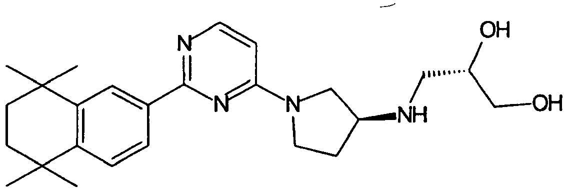 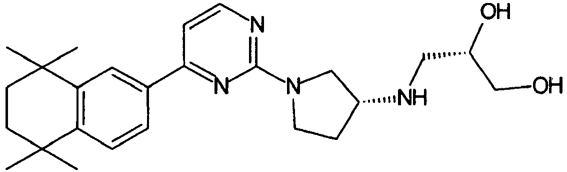 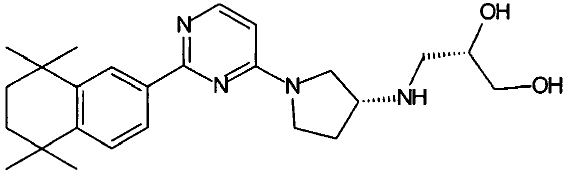 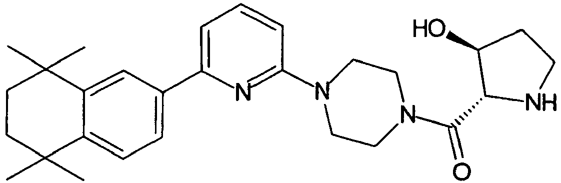 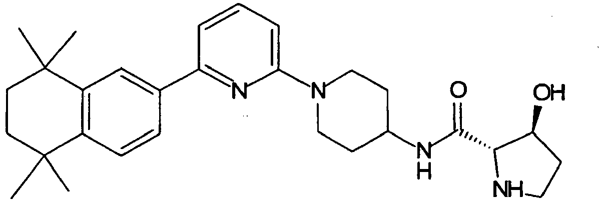 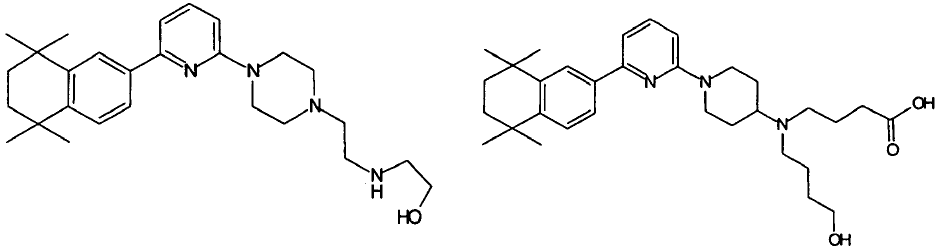 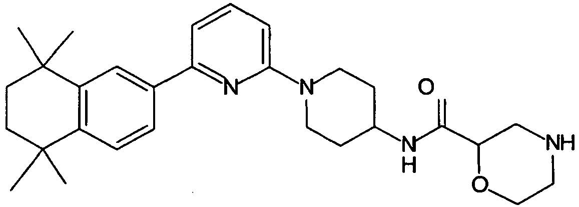 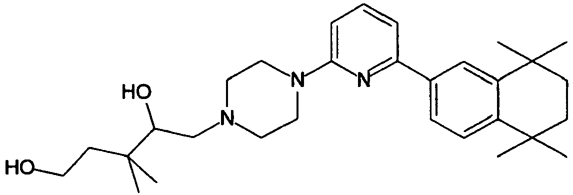 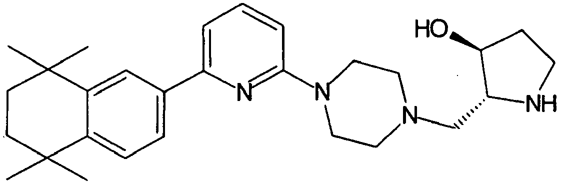 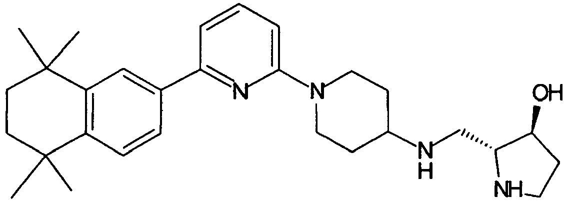 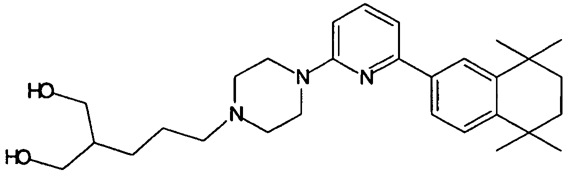 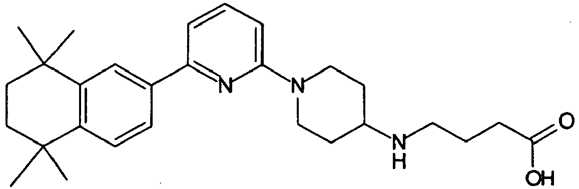 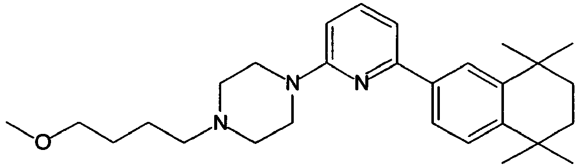 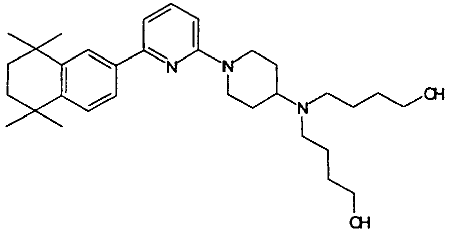 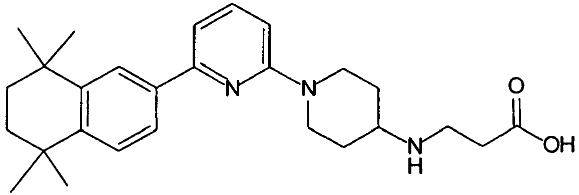 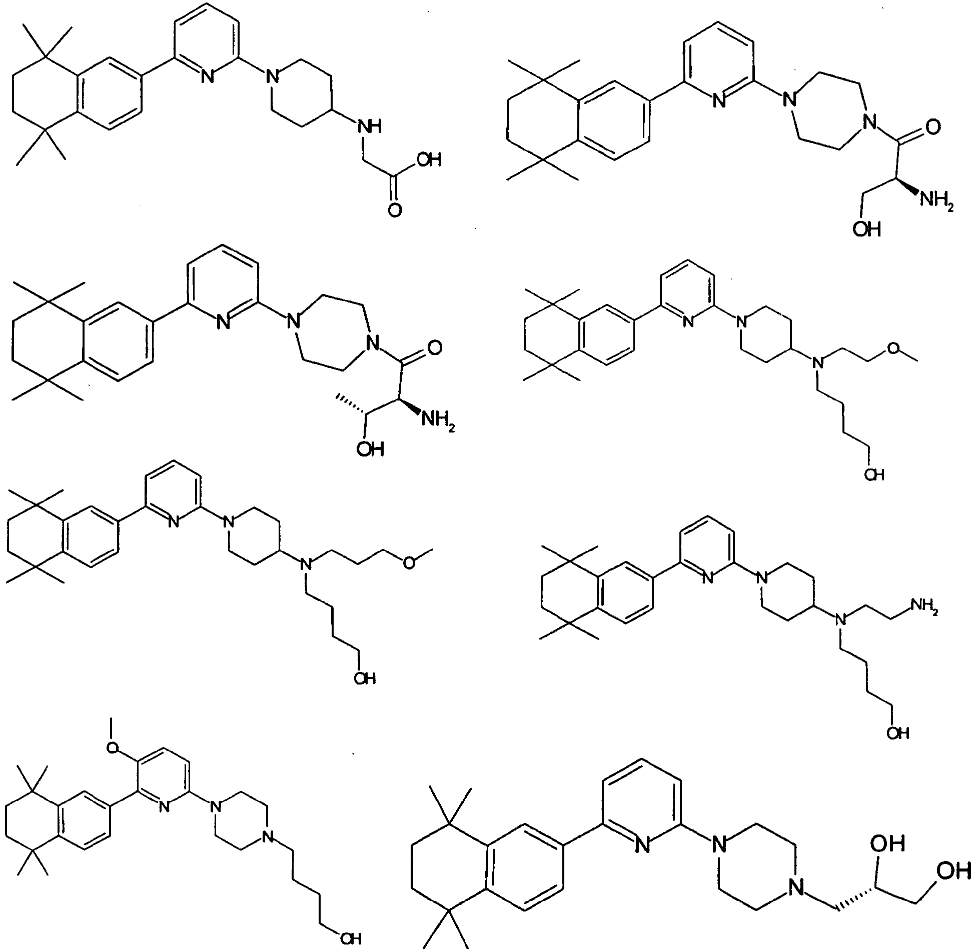 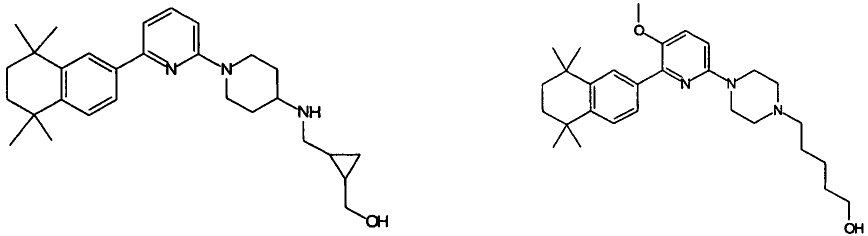 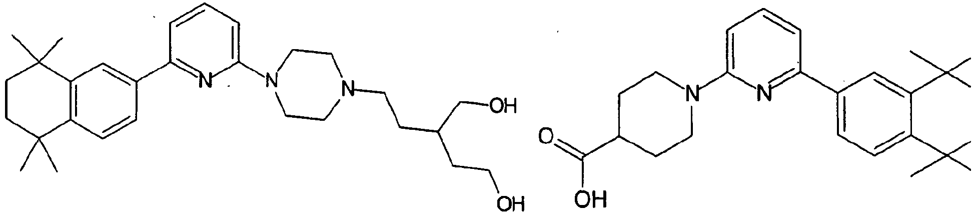 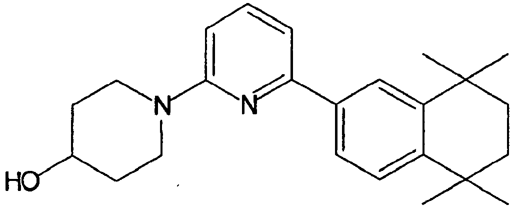 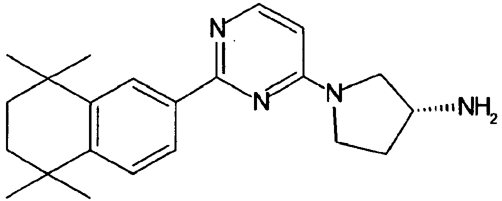 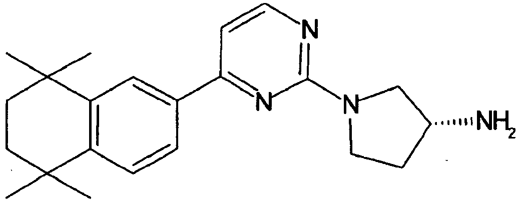 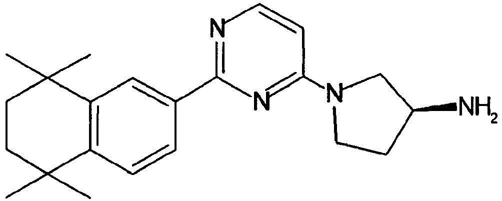 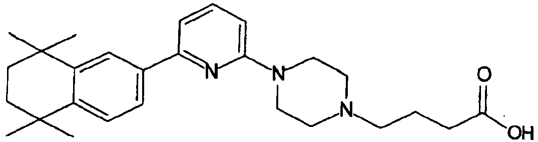 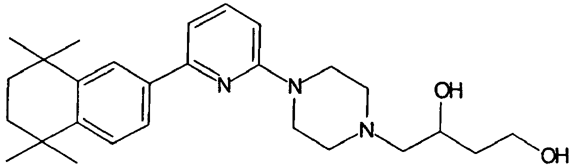 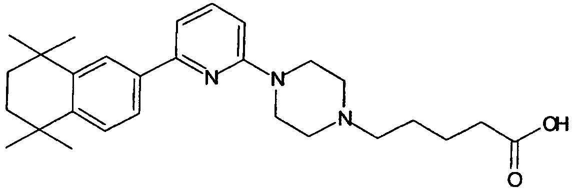 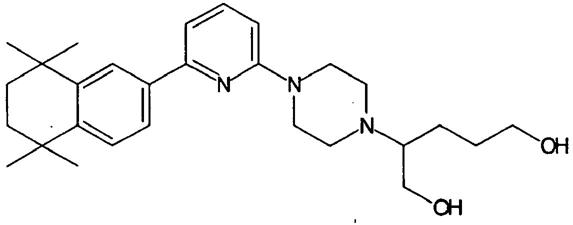 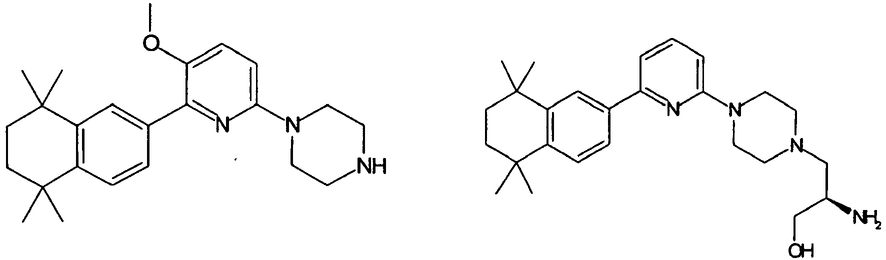 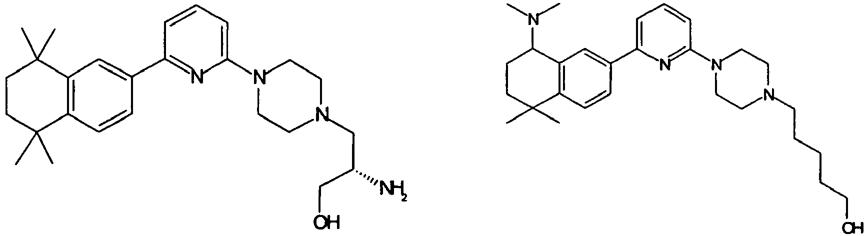 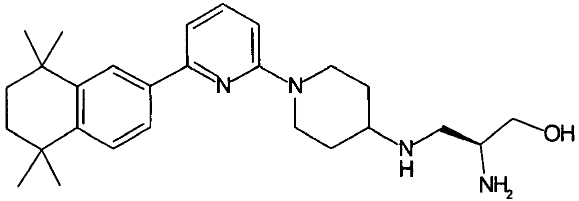 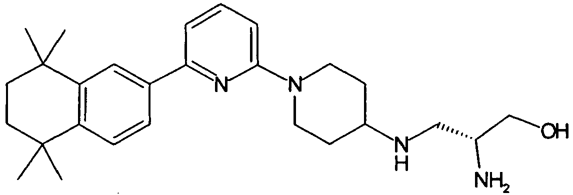 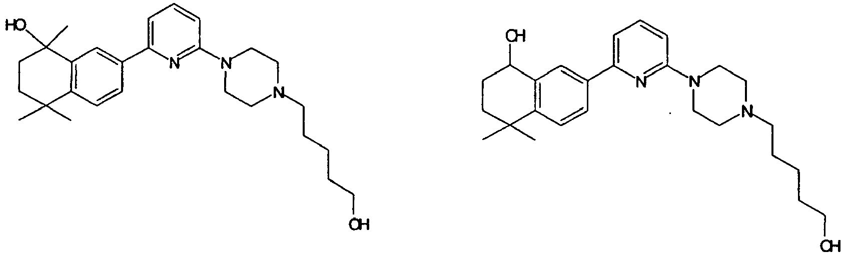 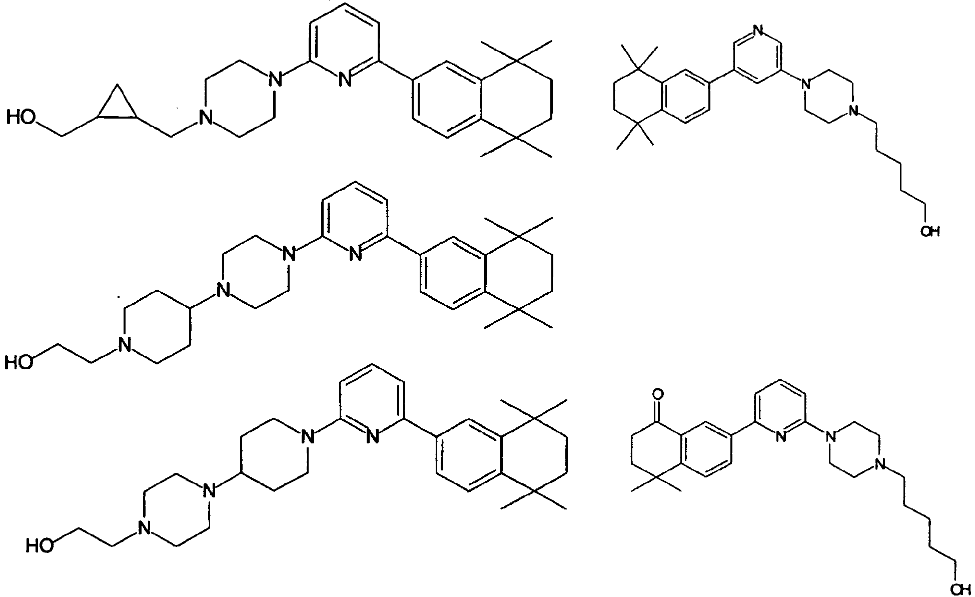 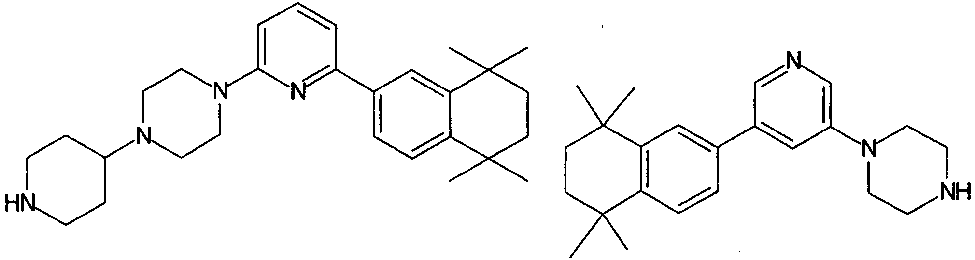 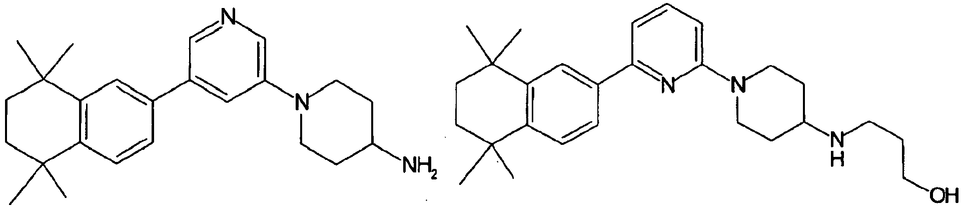 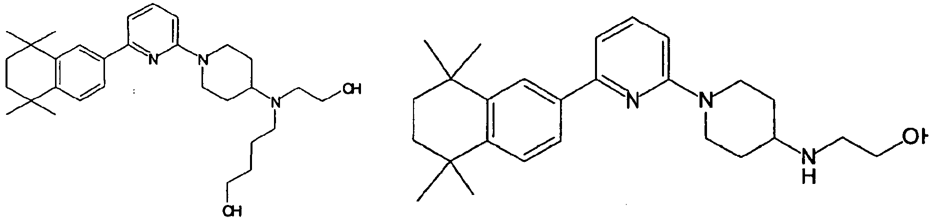 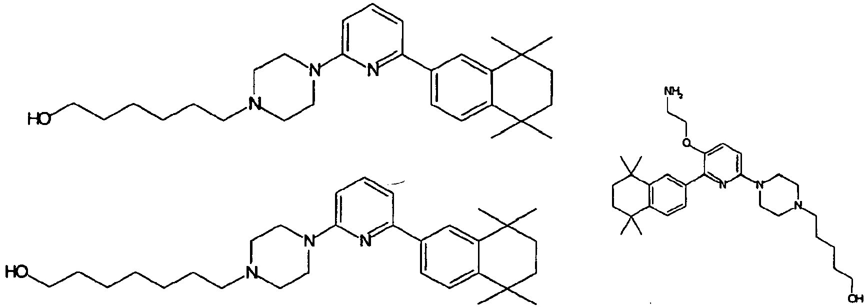 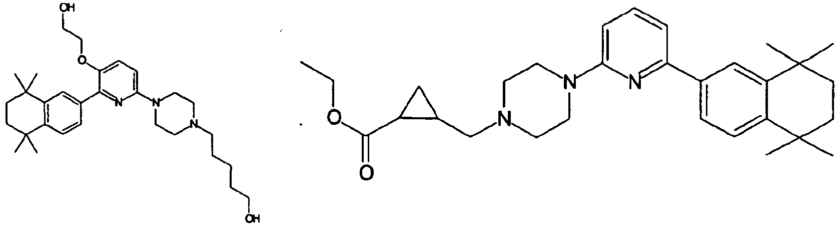 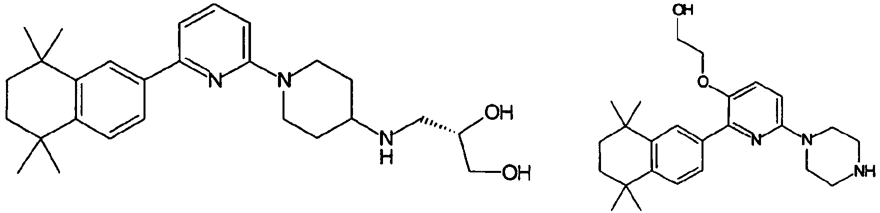 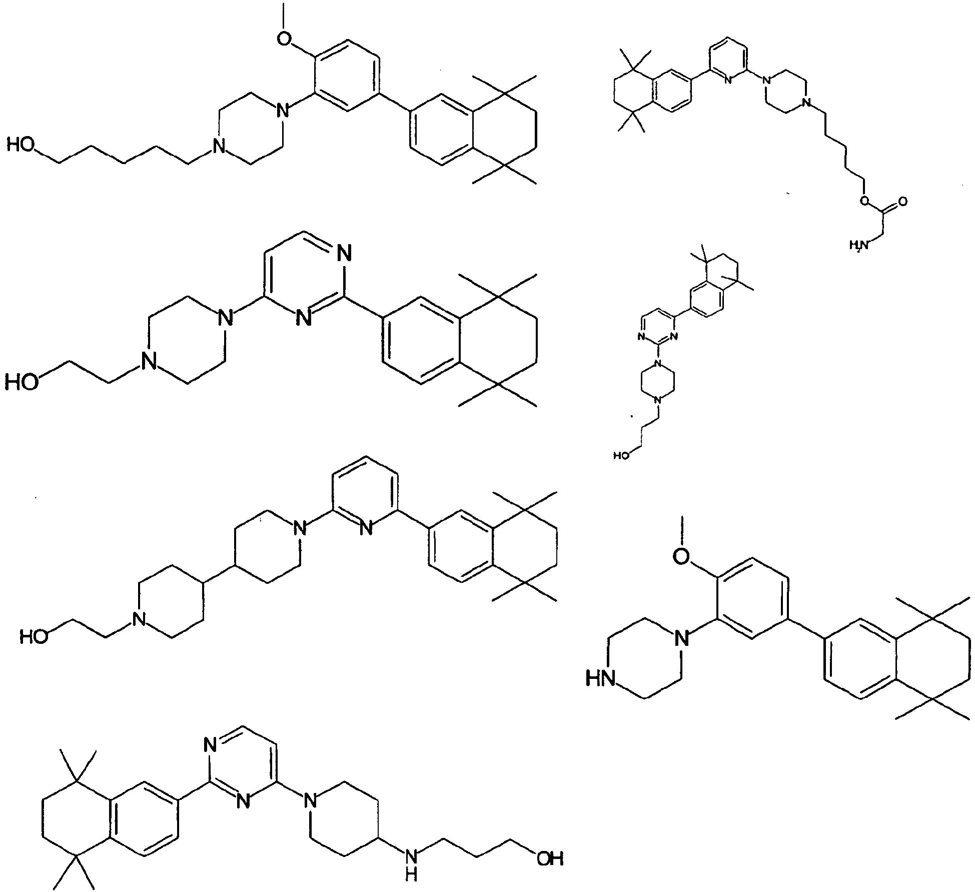 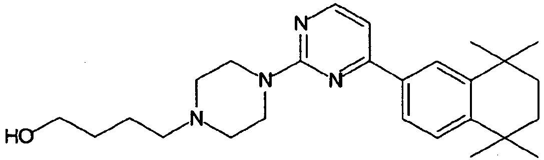 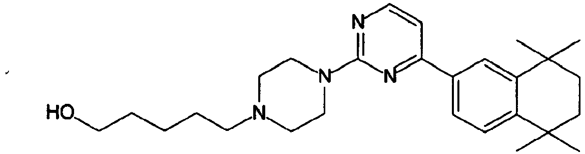 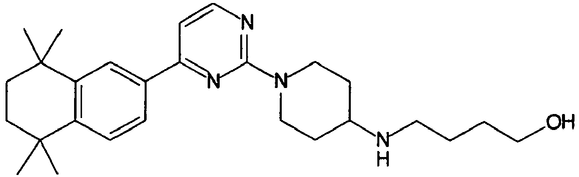 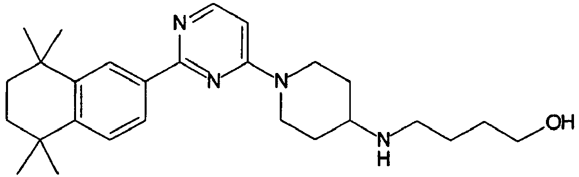 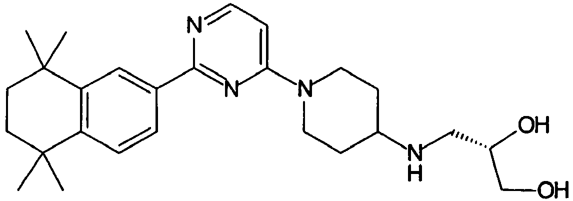 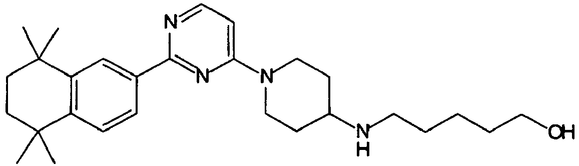 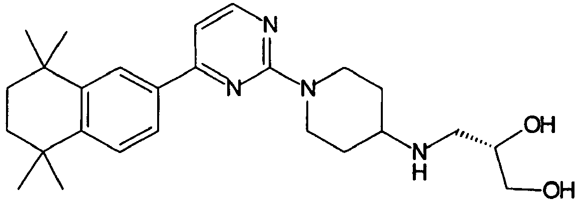 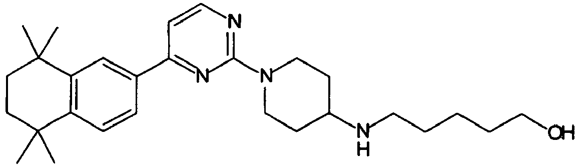 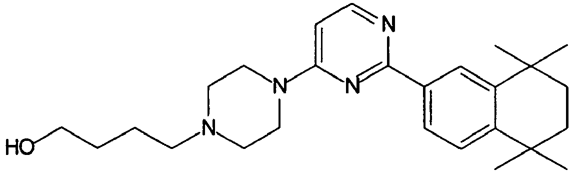 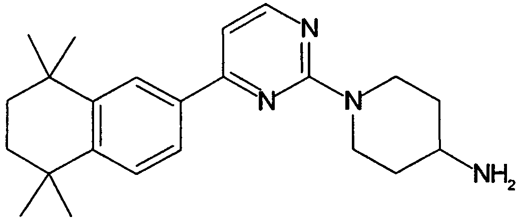 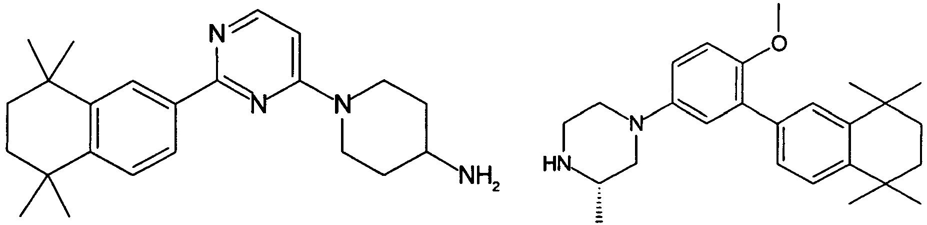 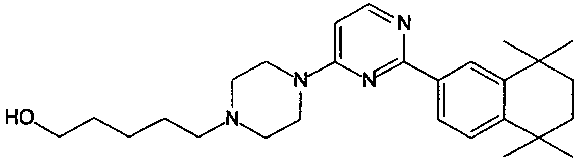 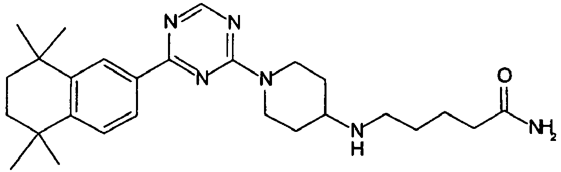 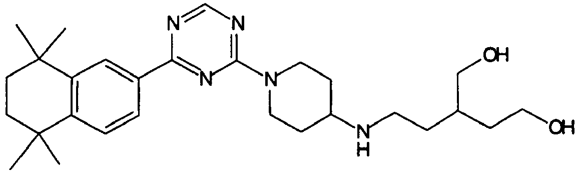 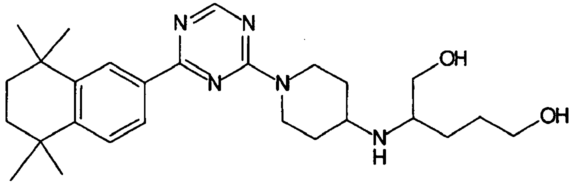 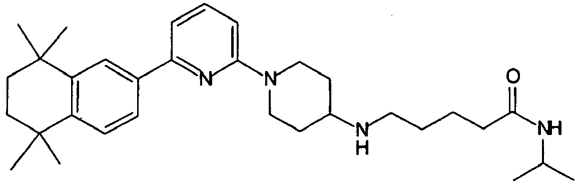 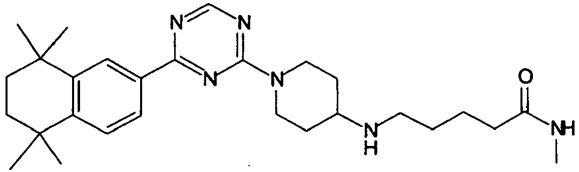 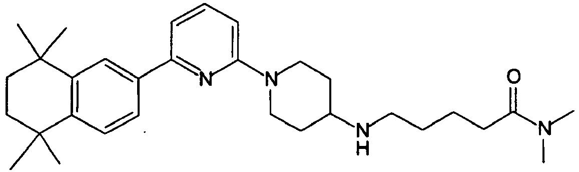 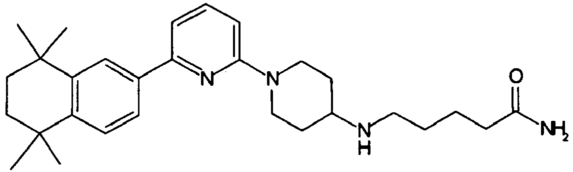 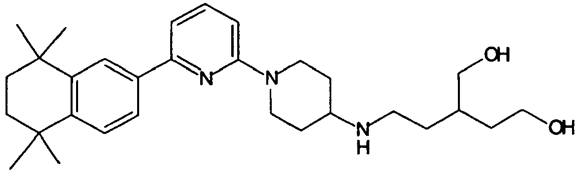 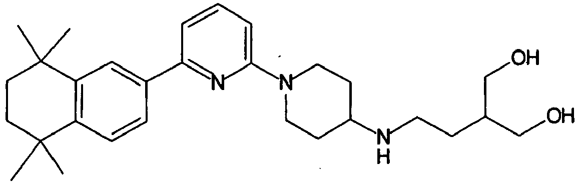 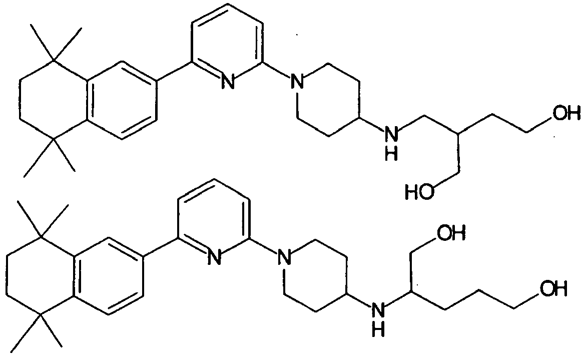 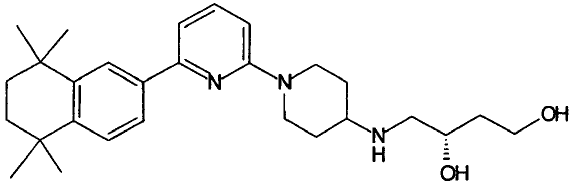 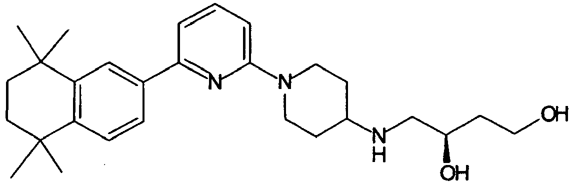 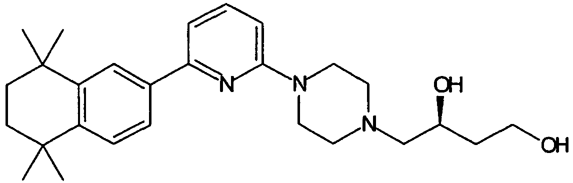 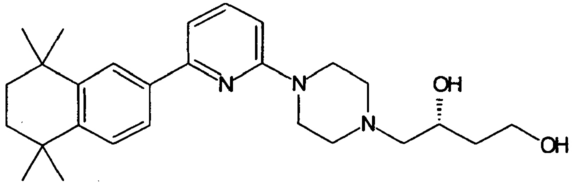 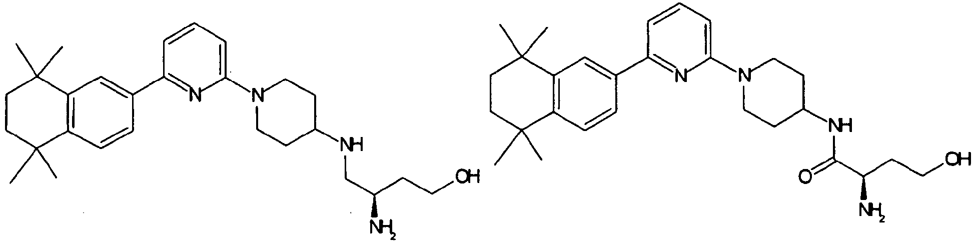 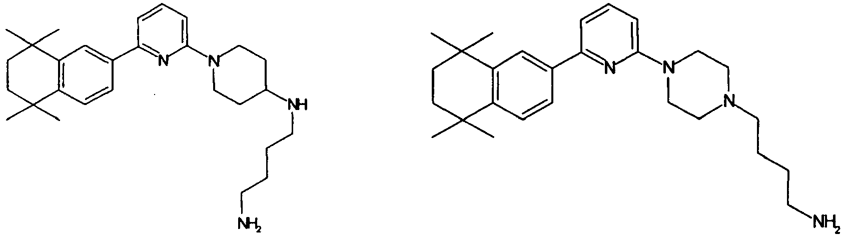 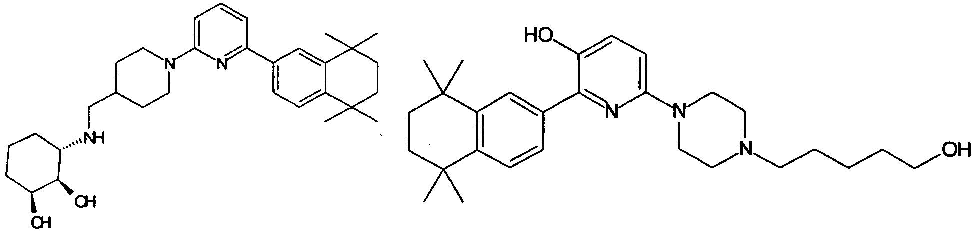 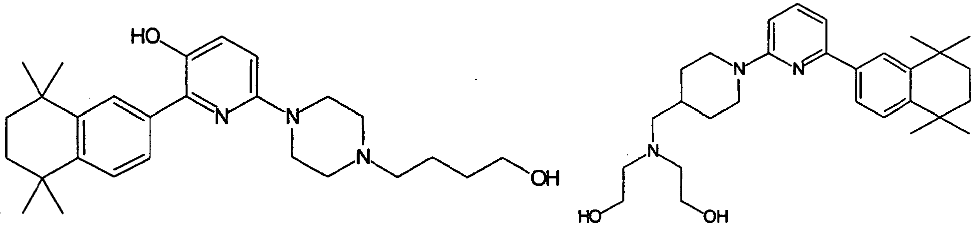 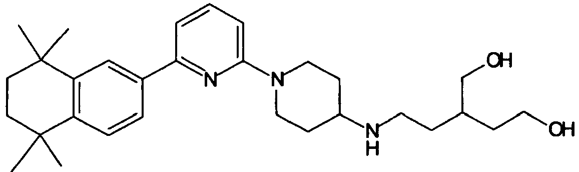 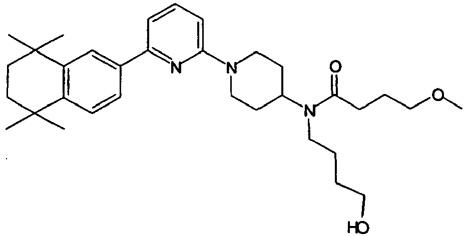 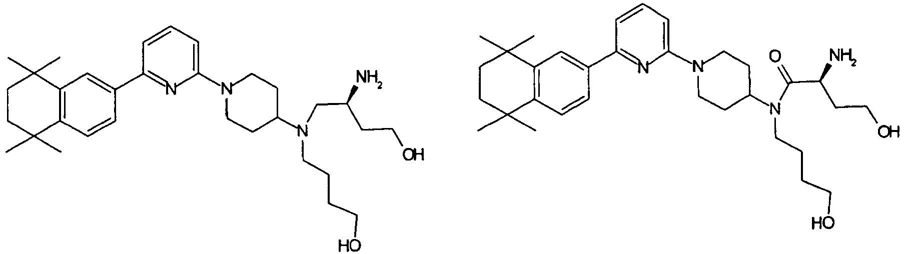 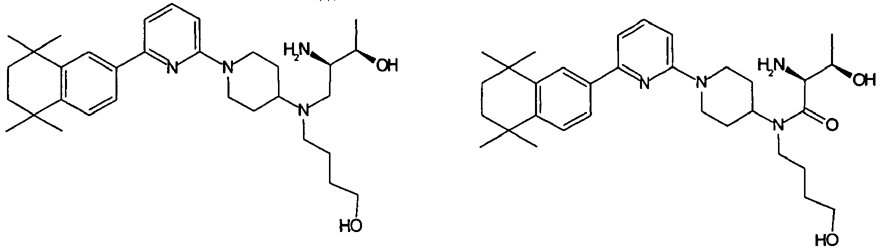 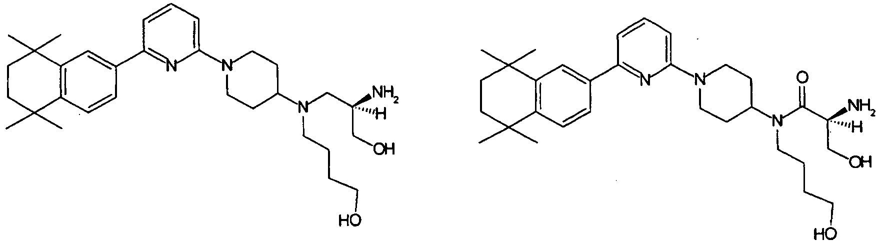 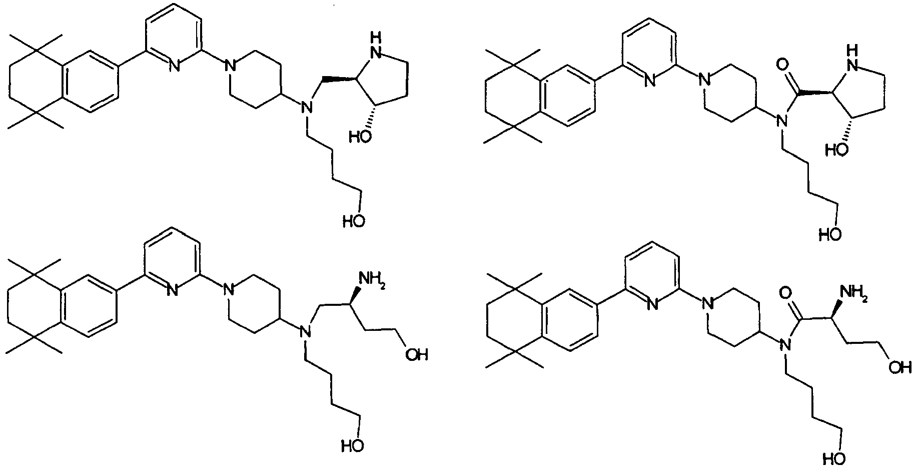 sowie ihre physiologisch unbedenklichen Salze, Solvate, Tautomere und Stereoisomere, einschließlich deren Mischungen in allen Verhältnissen.

Gegenstand der Erfindung sind auch die Vorstufen der Verbindungen der Formel (I), Arzneimittel, die diese Verbindungen enthalten, sowie deren Verwendung zu Behandlung von Krankheiten, wie für die Verbindungen der Formel (I) beschrieben.

Unter Verbindungen der Formel (I) versteht man auch die Hydrate und Solvate dieser Verbindungen.

Gegenstand der Erfindung sind auch die optisch aktiven Formen (Stereoisomeren), die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.

Darüber hinaus bedeutet der Ausdruck "therapeutisch wirksame Menge" eine Menge, die, verglichen zu einem entsprechenden Subjekt, das diese Menge nicht erhalten hat, folgendes zur Folge hat:
verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.

Die Bezeichnung "therapeutisch wirksame Menge" umfaßt auch die Mengen, die wirkungsvoll sind, die normale physiologische Funktion zu erhöhen.

Gegenstand der Erfindung ist auch die Verwendung von Mischungen der Verbindungen der Formel I, z.B. Gemische zweier Diastereomerer z.B. im Verhältnis 1:1, 1:2, 1:3, 1:4, 1:5, 1:10, 1:100 oder 1:1000. Besonders bevorzugt handelt es sich dabei um Mischungen stereoisomerer Verbindungen.

Die Erfindung betrifft ferner ein Verfahren zur Herstellung von Verbindungen gemäß der Formel (I) und hier dargestellten bevorzugten Ausführungsformen und offenbarten Verbindungen sowie ihrer physiologisch unbedenklichen Salze, Solvate, Tautomere und Stereoisomere, dadurch gekennzeichnet, dass man
(a) eine Verbindung der Formel (II) 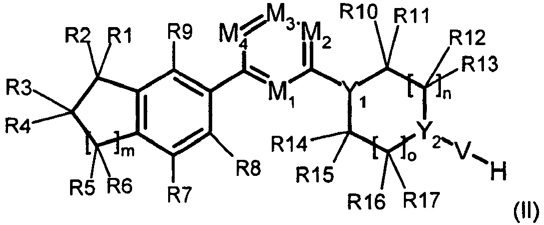 worin R¹, R², R³, R⁴, R⁵, R⁶, R⁷, R⁸, R⁹, R¹⁰, R¹¹, R¹², R¹³, R¹⁴, R¹⁵, R¹⁶, R¹⁷, M₁, M₂, M₃, M₄, Y₁, Y₂, V, n, m und o die hierin angegebenen Bedeutungen haben,
   mit einer Verbindung der Formel (III)

   L-W (III)

   worin W die hierin angebene Bedeutung hat und L Cl, Br, I oder eine freie oder reaktionsfähig funktionell abgewandelte OH-Gruppe bedeutet,
   umsetzt,
   oder
(b) eine Verbindung der Formel (IV) 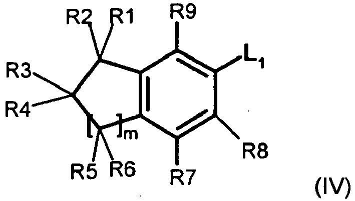 worin R¹, R², R³, R⁴, R⁵, R⁶, R⁷, R⁸, R⁹ und m die hierin angegebenen Bedeutungen haben und L₁ B(OH)₂ oder B(OR)₂ bedeutet, wobei B(OR)₂ ein cyclischer oder linearer Boronsäurealkylester ist,
   mit einer Verbindung der Formel (V) 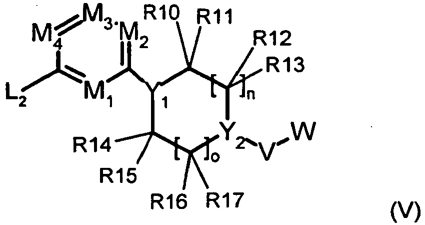 worin R¹⁰, R¹¹, R¹², R¹³, R¹⁴, R¹⁵, R¹⁶, R¹⁷, M₁, M₂, M₃, M₄, Y₁, Y₂, V, W, n, und o die hierin angegebenen Bedeutungen haben und L₂ Cl, Br oder I bedeutet,
   umsetzt,
   oder
(c) sie aus einem ihrer funktionellen Derivate (z.B. mit Schutzgruppen) durch Behandeln mit einem sauren, basischen, solvolysierenden oder hydrogenolysierenden Mittel in Freiheit setzt,
und/oder eine Base oder Säure der Formel (I) in eines ihrer Salze umwandelt.

Der Ausdruck "Carbamoyl" bedeutet "Aminocarbonyloxy" und umgekehrt.

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.

A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.

Cyclisches Alkyl (Cycloalkyl) bedeutet vorzugsweise Cyclopropyl, Cyclobutyl, Cylopentyl, Cyclohexyl oder Cycloheptyl.

Ar bedeutet z.B. Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Hydroxyphenyl, o-, m- oder p-Nitrophenyl, o-, m- oder p-Aminophenyl, o-, m- oder p-(N-Methylamino)-phenyl, o-, m- oder p-(N-Methylaminocarbonyl)-phenyl, o-, m- oder p-Acetamidophenyl, o-, m- oder p-Methoxyphenyl, o-, m- oder p-Ethoxyphenyl, o-, m- oder p-Ethoxycarbonylphenyl, o-, m- oder p-(N,N-Dimethylamino)-phenyl, o-, m- oder p-(N,N-Dimethylaminocarbonyl)-phenyl, o-, m- oder p-(N-Ethylamino)-phenyl, o-, m- oder p-(N,N-Diethylamino)-phenyl, o-, m- oder p-Fluorphenyl, o-, m- oder p-Bromphenyl, o-, m- oder p- Chlorphenyl, o-, m- oder p-(Methylsulfonamido)-phenyl, o-, m- oder p-(Methylsulfonyl)-phenyl, o-, m- oder p-Methylsulfanylphenyl, o-, m- oder p-Cyanphenyl, o-, m- oder p-Carboxyphenyl, o-, m- oder p-Methoxycarbonylphenyl, o-, m- oder p-Formylphenyl, o-, m- oder p-Acetylphenyl, o-, m- oder p-Aminosulfonylphenyl, o-, m- oder p-(Morpholin-4-ylcarbonyl)-phenyl, o-, m- oder p-(Morpholin-4-ylcarbonyl)-phenyl, o-, m- oder p-(3-Oxo-morpholin-4-yl)-phenyl, o-, m- oder p-(Piperidinyl-carbonyl)-phenyl, o-, m- oder p-[2-(Morpholin-4-yl)ethoxy]-phenyl, o-, m- oder p-[3-(N,N-Diethylamino)propoxy]-phenyl, o-, m- oder p-[3-(3-Diethylaminopropyl)-ureido]-phenyl, o-, m- oder p-(3-Diethylaminopropoxy-carbonylamino)-phenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4- oder 2,5-Dinitrophenyl, 2,5- oder 3,4-Dimethoxyphenyl, 3-Nitro-4-chlorphenyl, 3-Amino-4-chlor-, 2-Amino-3-chlor-, 2-Amino-4-chlor-, 2-Amino-5-chlor- oder 2-Amino-6-chlorphenyl, 2-Nitro-4-N,N-dimethylamino- oder 3-Nitro-4-N,N-dimethylaminophenyl, 2,3-Diaminophenyl, 2,3,4-, 2,3,5-, 2,3,6-, 2,4,6- oder 3,4,5-Trichlorphenyl, 2,4,6-Trimethoxyphenyl, 2-Hydroxy-3,5-dichlorphenyl, p-lodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Chlor-4-acetamidophenyl, 3-Fluor-4-methoxyphenyl, 3-Amino-6-methylphenyl, 3-Chlor-4-acetamidophenyl oder 2,5-Dimethyl-4-chlorphenyl.

Het bedeutet, ungeachtet weiterer Substitutionen, z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-. 4-, 5-, 6- oder 7-Indolyl, 4- oder 5-Isoindolyl, Indazolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder -5-yl, 2,1,3-Benzoxadiazol-5-yl oder Dibenzofuranyl.

Die heterocyclischen Reste können auch teilweise oder vollständig hydriert sein.

Ungeachtet weiterer Substitutionen kann Het also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder-4-pyrazolyl, 1,4-Dihydro-1-, -2-, -3- oder-4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder -8-isochinolyl, 2-, 3-, 5-, 6-, 7- oder 8-3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydrobenzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl, 2,3-Dihydro-2-oxo-furanyl, 3,4-Dihydro-2-oxo-1H-chinazolinyl, 2,3-Dihydro-benzoxazolyl, 2-Oxo-2,3-dihydro-benzoxazolyl, 2,3-Dihydro-benzimidazolyl, 1,3-Dihydroindol, 2-Oxo-1,3-dihydro-indol oder 2-Oxo-2,3-dihydro-benzimidazolyl.

Hal bedeutet vorzugsweise F, Cl oder Br, aber auch I, besonders bevorzugt F oder Cl.

Für die gesamte Erfindung gilt, daß sämtliche Reste, die mehrfach auftreten, gleich oder verschieden sein können, d.h. unabhängig voneinander sind.

Die Verbindungen der Formel (I) können ein oder mehrere chirale Zentren besitzen und daher in verschiedenen stereoisomeren Formen vorkommen. Die Formel (I) umschließt alle diese Formen.

Dementsprechend sind Gegenstand der Erfindung insbesondere diejenigen Verbindungen der Formel (I) und deren Verwendung, in denen mindestens einer der genannten Reste eine der vorstehend angegebenen bevorzugten Bedeutungen hat.

Die Verbindungen der Formel (I) und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsverbindungen der Formeln (II), (III), (IV) und (V) sind in der Regel bekannt. Sind sie neu, so können sie aber nach an sich bekannten Methoden hergestellt werden.

Die Umsetzung zu den Verbindungen der Formel (I) erfolgt in der Regel in Gegenwart eines säurebindenden Mittels vorzugsweise einer organischen Base wie DIPEA, Triethylamin, Dimethylanilin, Pyridin oder Chinolin.

Auch der Zusatz eines Alkali- oder Erdalkalimetall-hydroxids, -carbonats oder - bicarbonats oder eines anderen Salzes einer schwachen Säure der Alkali- oder Erdalkalimetalle, vorzugsweise des Kaliums, Natriums, Calciums oder Cäsiums kann günstig sein.

Die Reaktionszeit liegt je nach den angewendeten Bedingungen zwischen einigen Minuten und 14 Tagen, die Reaktionstemperatur zwischen etwa -30° und 140°, normalerweise zwischen -10° und 90°, insbesondere zwischen etwa 0° und etwa 70°.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Besonders bevorzugt ist Acetonitril, Dichlormethan und/oder DMF.

Ferner kann man freie Aminogruppen in üblicher Weise mit einem Säurechlorid oder -anhydrid acylieren oder mit einem unsubstituierten oder substituierten Alkylhalogenid alkylieren, zweckmäßig in einem inerten Lösungsmittel wie Dichlormethan oder THF und /oder in Gegenwart einer Base wie Triethylamin oder Pyridin bei Temperaturen zwischen -60 und +30°.

Die Verbindungen der Formel (I) können ferner erhalten werden, indem man sie aus ihren funktionellen Derivaten (z.B. mit Schutzgruppen) durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, z. B. solche, die der Formel (I) entsprechen, aber anstelle einer NH₂-Gruppe eine NHR'-Gruppe (worin R' eine Aminoschutzgruppe bedeutet, z. B. BOC oder CBZ) enthalten.

Ferner sind Ausgangsstoffe bevorzugt, die anstelle des H-Atoms einer Hydroxygruppe eine Hydroxyschutzgruppe tragen, z. B. solche, die der Formel (I) entsprechen, aber anstelle einer Hydroxyalkylgruppe eine R"O-alkylgruppe enthalten (worin R" eine Hydroxyschutzgruppe bedeutet).

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC, 2-lodethoxycarbonyl; Aralkyloxycarbonyl wie CBZ ("Carbobenzoxy"), 4-Methoxybenzyloxycarbonyl, FMOC; Arylsulfonyl wie Mtr, Pbf oder Pmc. Bevorzugte Aminoschutzgruppen sind BOC und Mtr, ferner CBZ, Fmoc, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. tert.-Butoxycarbonyl, Benzyl, p-Nitrobenzoyl, p-Toluolsulfonyl, tert.-Butyl und Acetyl, wobei Benzyl und tert.-Butyl besonders bevorzugt sind. Die COOH-Gruppen in Asparaginsäure und Glutaminsäure werden bevorzugt in Form ihrer tert.-Butylester geschützt (z. B. Asp(OBut)).

Das In-Freiheit-Setzen der Verbindungen der Formel (I) aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die Gruppen BOC, OBut, Pbf, Pmc und Mtr können z. B. bevorzugt mit TFA in Dichlormethan oder mit etwa 3 bis 5N HCl in Dioxan bei 15-30° abgespalten werden, die FMOC-Gruppe mit einer etwa 5- bis 50 %igen Lösung von Dimethylamin, Diethylamin oder Piperidin in DMF bei 15-30°.

Hydrogenolytisch entfernbare Schutzgruppen (z. B. CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Ether wie THF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10 %igem Pd/C in Methanol oder THF oder mit Ammomiumformiat (anstelle von Wasserstoff) an Pd/C in Methanol/DMF bei 20-30°.

Pharmazeutische Salze und andere Formen

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel (I) werden größtenteils konventionell hergestellt. Sofern die Verbindung der Formel (I) eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel (I) zählen ebenfalls dazu. Bei bestimmten Verbindungen der Formel (I) lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel (I) die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxy-ethansulfonat, Iodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium- , Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel I, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Isopropylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkylhalogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl wasser- als auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Phosphat, Stearat, Sulfat, Sulfosalicylat, Tartrat, Thiomalat, Tosylat und Tromethamin, was jedoch keine Einschränkung darstellen soll.

Besonders bevorzugt sind Hydrochlorid, Dihydrochlorid, Hydrobromid, Maleat, Mesylat, Phosphat, Sulfat und Succinat.

Die Säureadditionssalze basischer Verbindungen der Formel (I) werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel (I) mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel (I) in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Gegenstand der Erfindung sind ferner Arzneimittel umfassend eine oder mehrere Verbindungen gemäß der Formel (I) und hier dargestellten bevorzugten Ausführungsformen und offenbarten Verbindungen, wobei Verbindungen der Formel (I), worin (a) V fehlend ist, und (b) W = C(O)-CH₂-Het ist, nicht ausgeschlossen sind, sowie ihrer physiologisch unbedenklichen Salze, Solvate, Tautomere und Stereoisomere, einschließlich deren Mischungen in allen Verhältnissen.

Gegenstand der Erfindung sind ferner Arzneimittel umfassend eine oder mehrere Verbindungen gemäß der Formel (I) und hier dargestellten bevorzugten Ausführungsformen und offenbarten Verbindungen, wobei Verbindungen der Formel (I), worin (a) V fehlend ist, und (b) W = C(O)-CH₂-Het ist, nicht ausgeschlossen sind, sowie ihrer physiologisch unbedenklichen Salze, Solvate, Tautomere und Stereoisomere, einschließlich deren Mischungen in allen Verhältnissen, zur Verwendung in der Behandlung von Krankheiten, die durch Inhibierung von Sph-Kinase 1 durch die Verbindungen gemäß Formel (I) und und der hier dargestellten Ausführungsformen und offenbarten Verbindungen beeinflußt werden. Eine entsprechende Verwendung zur Herstellung eines Arzneimittels für die Behandlung und/oder Prophylaxe der vorgenannten Leiden soll inbegriffen sein.

In einer bevorzugten Ausführungsform werden ferner Arzneimittel beansprucht umfassend eine oder mehrere Verbindungen gemäß der Formel (I) und hier dargestellten bevorzugten Ausführungsformen und offenbarten Verbindungen, wobei Verbindungen der Formel (I), worin (a) V fehlend ist, und (b) W = C(O)-CH₂-Het ist, ausgeschlossen sind, sowie ihrer physiologisch unbedenklichen Salze, Solvate, Tautomere und Stereoisomere, einschließlich deren Mischungen in allen Verhältnissen.

In einer bevorzugten Ausführungsform werden ferner Arzneimittel beansprucht umfassend eine oder mehrere Verbindungen gemäß der Formel (I) und hier dargestellten bevorzugten Ausführungsformen und offenbarten Verbindungen, wobei Verbindungen der Formel (I), worin (a) V fehlend ist, und (b) W = C(O)-CH₂-Het ist, ausgeschlossen sind, sowie ihrer physiologisch unbedenklichen Salze, Solvate, Tautomere und Stereoisomere, einschließlich deren Mischungen in allen Verhältnissen, zur Verwendung in der Behandlung von Krankheiten, die durch Inhibierung von Sph-Kinase 1 durch die Verbindungen gemäß Formel (I) und und der hier dargestellten Ausführungsformen und offenbarten Verbindungen beeinflußt werden. Eine entsprechende Verwendung zur Herstellung eines Arzneimittels für die Behandlung und/oder Prophylaxe der vorgenannten Leiden soll inbegriffen sein.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon, eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-Öl-Flüssigemulsionen dargereicht werden.

So läßt sich beispielsweise bei der oralen Verabreichung in Form einer Tablette oder Kapsel die Wirkstoffkomponente mit einem oralen, nichttoxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die Verbindungen der Formel (I) sowie Salze, Solvate und physiologisch funktionelle Derivate davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die Verbindungen der Formel (I) sowie die Salze, Solvate und physiologisch funktionellen Derivate davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, **Polyhydroxyethylaspart**amidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflasters mittels lontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendem mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der Formel (I) hängt von einer Reihe von Faktoren ab, einschließlich z.B. dem Alter und Gewicht, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung von neoplastischem Wachstum, z.B. Dickdarm- oder Brustkarzinom, im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der erfindungsgemäßen Verbindung *per se* bestimmt werden. Es läßt sich annehmen, daß ähnliche Dosierungen für die Behandlung der anderen, obenerwähnten Krankheitszustände geeignet sind.

Die Erfindung betrifft ferner pharmazeutische Zusammensetzung enthaltend eine therapeutisch wirksame Menge mindestens einer Verbindung gemäß der Formel (I) und der hier dargestellten bevorzugten Ausführungsformen und offenbarten Verbindungen.

In einer bevorzugten Ausführungsform wird eine pharmazeutische Zusammensetzung wie hier dargestellt beansprucht enthaltend mindestens eine zusätzliche Verbindung ausgewählt aus der Gruppe bestehend aus physiologisch unbedenklichen Streckmitteln, Hilfsstoffen, Zusatzstoffen, Verdünnern, Trägerstoffen und/oder zusätzlicher pharmazeutisch aktiver Substanz außer den Verbindungen gemäß der Formel (I) und der hier dargestellten bevorzugten Ausführungsformen und offenbarten Verbindungen.

Gegenstand der Erfindung ist auch ein Kit enthaltend eine therapeutisch wirksame Menge mindestens einer Verbindung gemäß der Formel (I) und der hier dargestellten bevorzugten Ausführungsformen und offenbarten Verbindungen und/oder mindestens eine pharmazeutische Zusammensetzung wie hier dargestellt und eine therapeutisch wirksame Menge mindestens einer weiteren pharmakologisch aktiven Substanz außer den Verbindungen gemäß der Formel (I) und der hier dargestellten bevorzugten Ausführungsformen und offenbarten Verbindungen.

Der Kit enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel (I) und/oder ihrer pharmazeutisch verwendbaren Derivate, Solvate, Tautomere und Stereoisomere, einschließlich deren Mischungen in allen Verhältnissen, und einer wirksamen Menge eines weiteren Arzneimittelwirkstoffs gelöst oder in lyophilisierter Form vorliegt.

### Verwendung

Die vorliegenden Verbindungen eignen sich als pharmazeutische Wirkstoffe für Säugetiere, insbesondere für den Menschen, bei der Behandlung von sphingosinkinasebedingten Krankheiten. Zu diesen Krankheiten zählen die Proliferation von Tumorzellen, die pathologische Gefäßneubildung (oder Angiogenese), die das Wachstum fester Tumoren fördert, die Gefäßneubildung im Auge (diabetische Retinopathie, altersbedingte Makula-Degeneration und dergleichen) sowie Entzündung (Schuppenflechte, rheumatoide Arthritis und dergleichen).

Die vorliegende Erfindung umfasst die Verwendung der Verbindungen der Formel (I) und/oder ihre physiologisch unbedenklichen Salze und Solvate zur Herstellung eines Arzneimittels zur Behandlung oder Vorbeugung von Krebs. Bevorzugte Karzinome für die Behandlung stammen aus der Gruppe Hirnkarzinom, Urogenitaltraktkarzinom, Karzinom des lymphatischen Systems, Magenkarzinom, Kehlkopfkarzinom und Lungenkarzinom. Eine weitere Gruppe bevorzugter Krebsformen sind Monozytenleukämie, Lungenadenokarzinom, kleinzellige Lungenkarzinome, Prostatakrebs, Darmkrebs, Bauchspeicheldrüsenkrebs, Ovarialkarzinom, Nierenkrebs, Leberkarzinom, Glioblastome und Brustkarzinom.

Ebenfalls umfasst ist die Verwendung der erfindungsgemäßen Verbindungen und/oder ihre physiologisch unbedenklichen Salze und Solvate zur Herstellung eines Arzneimittels zur Behandlung oder Vorbeugung einer Krankheit, an der Angiogenese beteiligt ist.

Eine derartige Krankheit, an der Angiogenese beteiligt ist, ist eine Augenkrankheit, wie Retina-Vaskularisierung, diabetische Retinopathie, altersbedingte Makula-Degeneration und dergleichen.

Die Verwendung von Verbindungen der Formel (I) und/oder ihre physiologisch unbedenklichen Salze und Solvate zur Herstellung eines Arzneimittels zur Behandlung oder Vorbeugung von Entzündungskrankheiten, fällt ebenfalls unter den Umfang der vorliegenden Erfindung. Zu solchen Entzündungskrankheiten zählen zum Beispiel rheumatoide Arthritis, Schuppenflechte, Kontaktdermatitis, Spät-Typ der Überempfindlichkeitsreaktion und dergleichen.

Ebenfalls umfasst ist die Verwendung der Verbindungen der Formel (I) und/oder ihre physiologisch unbedenklichen Salze und Solvate zur Herstellung eines Arzneimittels zur Behandlung oder Vorbeugung einer Krankheit bzw. eines Leidens bei einem Säugetier, wobei man diesem Verfahren einem kranken Säugetier, das einer derartigen Behandlung bedarf, eine therapeutisch wirksame Menge einer erfindungsgemäßen Verbindung verabreicht. Die therapeutische Menge hängt von der jeweiligen Krankheit ab und kann vom Fachmann ohne allzu großen Aufwand bestimmt werden.

Die vorliegende Erfindung umfasst auch die Verwendung von Verbindungen der Formel (I) und/oder ihre physiologisch unbedenklichen Salze und Solvate zur Herstellung eines Arzneimittels zur Behandlung oder Vorbeugung von Retina-Vaskularisierung.

Neben den Verbindungen der Formel (I) können auch deren Vorstufen zur Behandlung der genannten Erkrankungeen verwendet werden.

Die Verbindungen der Formel (I) können an Patienten zur Behandlung von Krebs, insbesondere schnell wachsenden Tumoren, verabreicht werden.

Gegenstand der Erfindung ist somit die Verwendung von Verbindungen der Formel I, sowie ihrer pharmazeutisch verwendbaren Derivate, Solvate, Tautomere und Stereoisomere, einschließlich deren Mischungen in allen Verhältnissen, zur Herstellung eines Arzneimittels zur Behandlung von Krankheiten, bei denen die Hemmung, Regulierung und/oder Modulation der Signaltransduktion von Kinasen eine Rolle spielt.

Bevorzugt ist hierbei die Sph-Kinase.

Bevorzugt ist die Verwendung von Verbindungen der Formel (I), sowie ihrer pharmazeutisch verwendbaren Derivate, Solvate, Tautomere und Stereoisomere, einschließlich deren Mischungen in allen Verhältnissen, zur Herstellung eines Arzneimittels zur Behandlung von Krankheiten, die durch Inhibierung der SphK 1 durch die Verbindungen gemäß der Formel (I) und der hier dargestellten bevorzugten Ausführungsformen und offenbarten Verbindungen beeinflußt werden.

Die zu behandelnden Krankheiten sind vorzugsweise ausgewählt aus der Gruppe hyperproliferative Erkrankung, inflammatorische Erkrankung, angiogene Erkrankung.

Die hyperproliferative Erkrankung ist vorzugsweise ausgewählt aus der Gruppe Krebs (Tumorerkrankung), Atherosclerose, Restenose, proliferative Erkrankung der Mesangiumzellen, Psoriasis.

Die Tumorerkrankung ist vorzugsweise ausgewählt aus der Gruppe Tumor des Plattenepithel, der Blase, des Magens, der Nieren, von Kopf und Hals, des Ösophagus, des Gebärmutterhalses, der Schilddrüse, des Darms, der Leber, des Gehirns, der Prostata, des Urogenitaltrakts, des lymphatischen Systems, des Magens, des Kehlkopfs, der Lunge, der Haut, Monozytenleukämie, Lungenadenokarzinom, kleinzelliges Lungenkarzinom, Bauchspeicheldrüsenkrebs, Glioblastom, Brustkarzinom, akute myelotische Leukämie, chronische myelotische Leukämie, akute lymphatische Leukämie, chronische lymphatische Leukämie, Hodgkin-Lymphom, non-Hodgkin-Lymphom.

Die proliferative Erkrankung der Mesangiumzellen ist vorzugsweise ausgewählt aus der Gruppe
Glomerulonephritis, diabetische Nephropathie, maligne Nephrosclerose, thrombotisches Mikroangiopathie-Syndrom, Transplantatabstossung, Glomerulopathie.

Die inflammatorische Erkrankung ist vorzugsweise ausgewählt aus der Gruppe
entzündliche Darmerkrankung, Arthritis, Atherosclersose, Asthma, Allergien, entzündliche Nierenerkrankungen, multiple Sklerose, chronisch obstruktive Lungenerkrankung, entzündliche Hauterkrankungen, Pardontalerkrankungen, Psoriasis, durch T-Zellen - vermittelte Immunerkrankung.

Die entzündliche Darmerkrankung ist vorzugsweise ausgewählt aus der Gruppe ulcerative Colitis, Morbus Crohn, unbestimmte Colitis.

Die durch T-Zellen - vermittelte Immunerkrankung ist vorzugsweise ausgewählt aus der Gruppe
allergische Encephalomyelitis, allergische Neuritis, Transplantatabstossung, Graft-versus-Host-Reaktion, Myocarditis, Thyroiditis, Nephritis, systemischer Lupus erythematodes, insulinabhängiger Diabetes mellitus.

Die Arthritis-Erkrankung ist vorzugsweise ausgewählt aus der Gruppe
rheumatoide Arthritis, Osteoarthritis, Caplan Syndrom, Felty Syndrom, Sjogren Syndrom, Spondylitis ankylosans, Morbus Still, Chondrocalcinosis, metabolische Arthritis, rheumatisches Fieber, Morbus Reiter, Wissler Syndrom.

Die entzündliche Nierenerkrankung ist vorzugsweise ausgewählt aus der Gruppe
Glomerulonephritis, glomeruläre Verletzung, nephrotisches Syndrom, interstitielle Nephritis, Lupus nephritis, Goodpasture-Syndrom, Wegener-Granulomatose, Nierenvaskulitis, IgA-Nephropathie, idiopatische glomeruläre Erkrankung.

Die entzündliche Hauterkrankung ist vorzugsweise ausgewählt aus der Gruppe Psoriasis, atopische Dermatitis, Kontaktempfindlichkeit, Akne.

Die angiogene Erkrankung ist vorzugsweise ausgewählt aus der Gruppe

diabetische Retinopathie, Arthritis, Krebs, Psoriasis, Kaposi Sarkom, Hemangioma, myocardiale Angiogenesis, atherosklerotische Plaque-Neovaskularisation, angiogene Augenerkrankungen, choroidale Neovaskularisation, retrolentale Fibroplasie, makulare Degeneration, corneale Transplantatabstossung, Rubeosis iridis, neurosculares Glaukom, Oster Webber Syndrom.

Gegenstand der Erfindung sind ferner Arzneimittel umfassend eine oder mehrere Verbindungen gemäß der Formel (I) und hier dargestellten bevorzugten Ausführungsformen und offenbarten Verbindungen, wobei Verbindungen der Formel (I), worin (a) V fehlend ist, und (b) W = C(O)-CH₂-Het ist, nicht ausgeschlossen sind, sowie ihrer physiologisch unbedenklichen Salze, Solvate, Tautomere und Stereoisomere, einschließlich deren Mischungen in allen Verhältnissen, zur Verwendung in der Behandlung von Krankheiten, die durch Inhibierung von Sph-Kinase 1 durch die Verbindungen gemäß Formel (I) und und der hier dargestellten Ausführungsformen und offenbarten Verbindungen beeinflußt werden, wobei die zu behandelnden Krankheiten ausgewählt sind aus der Gruppe bestehend aus: "hyperproliferative Erkrankung, inflammatorische Erkrankung, angiogene Erkrankung, fibrotische Erkrankung der Lunge, Niere, Leber und des Herzens, Krebs (Tumorerkrankung), Atherosclerose, Restenose, proliferative Erkrankung der Mesangiumzellen, Psoriasis, Tumor des Plattenepithel, der Blase, des Magens, der Nieren, von Kopf und Hals, des Ösophagus, des Gebärmutterhalses, der Schilddrüse, des Darms, der Leber, des Gehirns, der Prostata, des Urogenitaltrakts, des lymphatischen Systems, des Magens, des Kehlkopfs, der Lunge, der Haut, Monozytenleukämie, Lungenadenokarzinom, kleinzelliges Lungenkarzinom, Bauchspeicheldrüsenkrebs, Glioblastom, Brustkarzinom, akute myelotische Leukämie, chronische myelotische Leukämie, akute lymphatische Leukämie, chronische lymphatische Leukämie, Hodgkin-Lymphom, non-Hodgkin-Lymphom, Glomerulonephritis, diabetische Nephropathie, maligne Nephrosclerose, thrombotisches Mikroangiopathie-Syndrom, Transplantatabstossung, Glomerulopathie, entzündliche Darmerkrankung, Arthritis, Asthma, Allergien, entzündliche Nierenerkrankungen, multiple Sklerose, chronisch obstruktive Lungenerkrankung, entzündliche Hauterkrankungen, Pardontalerkrankungen, durch T-Zellen - vermittelte Immunerkrankung, ulcerative Colitis, Morbus Crohn, unbestimmte Colitis, allergische Encephalomyelitis, allergische Neuritis, Transplantatabstossung, Graft-versus-Host-Reaktion, Myocarditis, Thyroiditis, Nephritis, systemischer Lupus erythematodes, insulinabhängiger Diabetes mellitus, rheumatoide Arthritis, Osteoarthritis, Caplan Syndrom, Felty Syndrom, Sjogren Syndrom, Spondylitis ankylosans, Morbus Still, Chondrocalcinosis, metabolische Arthritis, rheumatisches Fieber, Morbus Reiter, Wissler Syndrom, Glomerulonephritis, glomeruläre Verletzung, nephrotisches Syndrom, interstitielle Nephritis, Lupus nephritis, Goodpasture-Syndrom, Wegener-Granulomatose, Nierenvaskulitis, IgA-Nephropathie, idiopatische glomeruläre Erkrankung, atopische Dermatitis, Kontaktempfindlichkeit, Akne, diabetische Retinopathie, Kaposi Sarkom, Hemangioma, myocardiale Angiogenesis, atherosklerotische Plaque-Neovaskularisation, angiogene Augenerkrankungen, choroidale Neovaskularisation, retrolentale Fibroplasie, makulare Degeneration, corneale Transplantatabstossung, Rubeosis iridis, neurosculares Glaukom, Oster Webber Syndrom". Eine entsprechende Verwendung zur Herstellung eines Arzneimittels für die Behandlung und/oder Prophylaxe der vorgenannten Leiden wie auch ein Verfahren zur Behandlung der genannten Erkrankungen umfassend die Verabreichung einer oder mehrerer Verbindungen gemäß der Formel (I) und der hier dargestellten bevorzugten Ausführungsformen und offenbarten Verbindungen an einen Patienten mit Bedarf an einer derartigen Verabreichung soll hierbei mit umfasst sein.

In einer bevorzugten Ausführungsform werden ferner Arzneimittel beansprucht umfassend eine oder mehrere Verbindungen gemäß der Formel (I) und hier dargestellten bevorzugten Ausführungsformen und offenbarten Verbindungen, wobei Verbindungen der Formel (I), worin (a) V fehlend ist, und (b) W = C(O)-CH₂-Het ist, ausgeschlossen sind, sowie ihrer physiologisch unbedenklichen Salze, Solvate, Tautomere und Stereoisomere, einschließlich deren Mischungen in allen Verhältnissen, zur Verwendung in der Behandlung von Krankheiten, die durch Inhibierung von Sph-Kinase 1 durch die Verbindungen gemäß Formel (I) und und der hier dargestellten Ausführungsformen und offenbarten Verbindungen beeinflußt werden, wobei die zu behandelnden Krankheiten ausgewählt sind aus der Gruppe bestehend aus: "hyperproliferative Erkrankung, inflammatorische Erkrankung, angiogene Erkrankung, fibrotische Erkrankung der Lunge, Niere, Leber und des Herzens, Krebs (Tumorerkrankung), Atherosclerose, Restenose, proliferative Erkrankung der Mesangiumzellen, Psoriasis, Tumor des Plattenepithel, der Blase, des Magens, der Nieren, von Kopf und Hals, des Ösophagus, des Gebärmutterhalses, der Schilddrüse, des Darms, der Leber, des Gehirns, der Prostata, des Urogenitaltrakts, des lymphatischen Systems, des Magens, des Kehlkopfs, der Lunge, der Haut, Monozytenleukämie, Lungenadenokarzinom, kleinzelliges Lungenkarzinom, Bauchspeicheldrüsenkrebs, Glioblastom, Brustkarzinom, akute myelotische Leukämie, chronische myelotische Leukämie, akute lymphatische Leukämie, chronische lymphatische Leukämie, Hodgkin-Lymphom, non-Hodgkin-Lymphom, Glomerulonephritis, diabetische Nephropathie, maligne Nephrosclerose, thrombotisches Mikroangiopathie-Syndrom, Transplantatabstossung, Glomerulopathie, entzündliche Darmerkrankung, Arthritis, Asthma, Allergien, entzündliche Nierenerkrankungen, multiple Sklerose, chronisch obstruktive Lungenerkrankung, entzündliche Hauterkrankungen, Pardontalerkrankungen, durch T-Zellen - vermittelte Immunerkrankung, ulcerative Colitis, Morbus Crohn, unbestimmte Colitis, allergische Encephalomyelitis, allergische Neuritis, Transplantatabstossung, Graft-versus-Host-Reaktion, Myocarditis, Thyroiditis, Nephritis, systemischer Lupus erythematodes, insulinabhängiger Diabetes mellitus, rheumatoide Arthritis, Osteoarthritis, Caplan Syndrom, Felty Syndrom, Sjogren Syndrom, Spondylitis ankylosans, Morbus Still, Chondrocalcinosis, metabolische Arthritis, rheumatisches Fieber, Morbus Reiter, Wissler Syndrom, Glomerulonephritis, glomeruläre Verletzung, nephrotisches Syndrom, interstitielle Nephritis, Lupus nephritis, Goodpasture-Syndrom, Wegener-Granulomatose, Nierenvaskulitis, IgA-Nephropathie, idiopatische glomeruläre Erkrankung, atopische Dermatitis, Kontaktempfindlichkeit, Akne, diabetische Retinopathie, Kaposi Sarkom, Hemangioma, myocardiale Angiogenesis, atherosklerotische Plaque-Neovaskularisation, angiogene Augenerkrankungen, choroidale Neovaskularisation, retrolentale Fibroplasie, makulare Degeneration, corneale Transplantatabstossung, Rubeosis iridis, neurosculares Glaukom, Oster Webber Syndrom". Eine entsprechende Verwendung zur Herstellung eines Arzneimittels für die Behandlung und/oder Prophylaxe der vorgenannten Leiden wie auch ein Verfahren zur Behandlung der genannten Erkrankungen umfassend die Verabreichung einer oder mehrerer Verbindungen gemäß der Formel (I) und der hier dargestellten bevorzugten Ausführungsformen und offenbarten Verbindungen an einen Patienten mit Bedarf an einer derartigen Verabreichung soll inbegriffen sein.

In einer bevorzugten Ausführungsform enthält ein derartiges Arzneimittel mindestens eine zusätzliche pharmakologisch aktive Substanz (Therapeutikum, Medikament, Inhaltsstoff).

In einer weiterhin bevorzugten Ausführungsform wird das Arzneimittel vor und/oder während und/oder nach Behandlung mit mindestens einer zusätzlichen pharmakologisch aktiven Substanz angewendet.

Die offenbarten Verbindungen der Formel (I) können in Verbindung mit anderen Therapeutika (pharmakologisch aktiven Substanzen), einschließlich Antikrebsmitteln, verabreicht werden. Wie hier verwendet, betrifft der Begriff "Antikrebsmittel" jedes Mittel, das einem Patienten mit Krebs zum Zweck der Behandlung des Krebses verabreicht wird.

Die hier definierte Antikrebsbehandlung kann als alleinige Therapie angewendet werden oder zusätzlich zu der erfindungsgemäßen Verbindung herkömmliche Operation oder Strahlungstherapie oder Chemotherapie umfassen. Eine derartige Chemotherapie kann eine oder mehrere der folgenden Kategorien von Antitumormitteln umfassen:
(i) antiproliferative/antineoplastische/DNA schädigende Mittel und Kombinationen davon, wie in der medizinischen Onkologie verwendet, wie Alkylierungsmittel (zum Beispiel Cisplatin, Carboplatin, Cyclophosphamid, Nitrogen Mustard, Melphalan, Chlorambucil, Busulphan und Nitrosoharnstoffe); Antimetaboliten (z.B. Antifolate, wie Fluorpyrimidine, wie 5-Fluoruracil und Tegafur, Raltitrexed, Methotrexat, Cytosinarabinosid, Hydroxyharnstoff und Gemcitabin); Antitumor-Antibiotika (z.B. Anthracycline, wie Adriamycin, Bleomycin, Doxorubicin, Daunomycin, Epirubicin, Idarubicin, Mitomycin-C, Dactinomycin und Mithramycin); antimitotische Mittel (zum Beispiel Vinca-Alkaloide, wie Vincristin, Vinblastin, Vindesin und Vinorelbin, und Taxoide, wie Taxol und Taxoter); Topoisomerase-Inhibitoren (zum Beispiel Epipodophyllotoxine, wie Etoposid und Teniposid, Amsacrin, Topotecan, Irinotecan und Camptothecin) und zelldifferenzierende Mittel (zum Beispiel all-trans-Retinsäure, 13-cis-Retinsäure und Fenretinid);
(ii) zytostatische Mittel, wie Anti-Östrogene (z.B. Tamoxifen, Toremifen, Raloxifen, Droloxifen und lodoxyfen), den Östrogenrezeptor nach unten regulierende Mittel (zum Beispiel Fulvestrant), Anti-Androgene (z.B. Bicalutamid, Flutamid, Nilutamid und Cyproteronacetat), LHRH-Antagonisten oder LHRH-Agonisten (zum Beispiel Goserelin, Leuprorelin und Buserelin), Progesterone (zum Beispiel Megestrolacetat), Aromatase-Inhibitoren (zum Beispiel Anastrozol, Letrozol, Vorazol und Exemestan) und Inhibitoren der 5α-Reduktase, wie Finasterid;
(iii) Mittel, die die Invasion von Krebszellen hemmen (zum Beispiel Metallo-proteinase-Inhibitoren, wie Marimastat und Inhibitoren der Urokinase-Plasminogenaktivator-Rezeptor-Funktion);
(iv) Inhibitoren der Wachstumsfaktor-Funktion, zum Beispiel umfassen solche Inhibitoren Wachstumsfaktor-Antikörper, Wachstumsfaktor-Rezeptor-Antikörper (zum Beispiel den Anti-erbb2-Antikörper Trastuzumab [Herceptin™] und den Anti-erbb1-Antikörper Cetuximab [C225]), Farnesyltransferase-Inhibitoren, Tyrosinkinase-Inhibitoren und Serin / Threonin-Kinase-Inhibitoren, zum Beispiel Inhibitoren der epidermalen Wachstumsfaktor-Familie (zum Beispiel Inhibitoren der Tyrosinkinasen der EGFR-Familie, wie N-(3-Chlor-4-fluorphenyl)-7-methoxy-6-(3-morpholinopropoxy)-chinazolin-4-amin (Gefitinib, AZD1839), N-(3-Ethinylphenyl)-6,7-bis(2-methoxyethoxy)chinazolin-4-amin (Erlotinib, OSI-774) und 6-Acrylamido-N-(3-chlor-4-fluorphenyl)-7-(3-morpholinopropoxy)chinazolin-4-amin (CI 1033)), zum Beispiel Inhibitoren der von Plättchen abstammenden Wachstumsfaktor-Familie und zum Beispiel Inhibitoren der Hepatozytenwachstumsfaktor-Familie;
(v) antiangiogene Mittel, wie solche, die die Wirkungen des vaskulären endothelialen Wachstumsfaktors hemmen (zum Beispiel der Antikörper gegen den vaskulären Endothelzell-Wachstumsfaktor Bevacizumab [Avastin™], Verbindungen, wie die in den veröffentlichten internationalen Patentanmeldungen WO 97/22596, WO 97/30035, WO 97/32856 und WO 98/13354 offenbarten) und Verbindungen, die durch andere Mechanismen wirken (zum Beispiel Linomid, Inhibitoren der Integrin-αvß3-Funktion und Angiostatin);
(vi) gefäßschädigende Mittel, wie Combretastatin A4 und in den internationalen Patentanmeldungen WO 99/02166, WO 00/40529, WO 00/41669, WO 01/92224, WO 02/04434 und WO 02/08213 offenbarte Verbindungen;
(vii) Antisense-Therapien, zum Beispiel diejenigen, die gegen die vorstehend aufgelisteten Ziele gerichtet sind, wie ISIS 2503, ein anti-Ras-Antisense;
(viii) Genetherapieansätze, einschließlich beispielsweise Ansätze zum Ersetzen von veränderten Genen, wie verändertem p53 oder verändertem BRCA1 oder BRCA2, GDEPT- (gene-directed enzyme pro-drug-Therapie-) Ansätze, die diejenigen, die Cytosindesaminase, Thymidinkinase oder ein bakterielles Nitroreduktase-Enzym verwenden, sowie Ansätze zur Erhöhung der Patiententoleranz gegenüber Chemotherapie oder Strahlungstherapie, wie Multi-Drug-Resistence-Gen-Therapie; und
(ix) Immuntherapieansätze, einschließlich beispielsweise Ex-vivo- und In-vivo-Ansätzen zur Erhöhung der Immunogenität von Patiententumorzellen, wie Transfektion mit Cytokinen, wie Interleukin 2, Interleukin 4 oder Granulozyten-Makrophagen-Koloniestimutierendem Faktor, Ansätze zur Verringerung der T-Zell-Anergie, Ansätze unter Verwendung transfizierter Immunzellen, wie mit Cytokin transfizierter dendritischer Zellen, Ansätze unter Verwendung mit Cytokin transfizierter Tumorzelllinien und Ansätze unter Verwendung anti-idiotypischer Antikörper.

Bevorzugt aber nicht ausschliesslich werden die Arzneimittel der nachstehenden Tabelle 1 mit den Verbindungen der Formel (I) kombiniert.

| Tabelle 1. | | |
|---|---|---|
| Alkylierungsmittel | Cyclophosphamid | Lomustin |
| | Busulfan | Procarbazin |
| | Ifosfamid | Altretamin |
| | Melphalan | Estramustinphosphat |
| | Hexamethylmelamin | Mechlorethamin |
| | Thiotepa | Streptozocin |
| | Chlorambucil | Temozolomid |
| | Dacarbazin | Semustin |
| | Carmustin | |
| | | |
| Platinmittel | Cisplatin | Carboplatin |
| | Oxaliplatin | ZD-0473 (AnorMED) |
| | Spiroplatin | Lobaplatin (Aetema) |
| | Carboxyphthalatoplatinum | Satraplatin (Johnson Matthey) |
| | Tetraplatin | BBR-3464 (Hoffrnann-La Roch |
| | Ormiplatin | SM-11355 (Sumitomo) |
| | Iproplatin | AP-5280 (Access) |
| | | |
| Antimetabolite | Azacytidin | Tomudex |
| | Gemcitabin | Trimetrexate |
| | Capecitabin | Deoxycoformycin |
| | 5-Fluoruracil | Fludarabin |
| | Floxuridin | Pentostatin |
| | 2-Chlordesoxyadenosin | Raltitrexed |
| | 6-Mercaptopurin | Hydroxyharnstoff |
| | 6-Thioguanin | Decitabin (SuperGen) |
| | Cytarabin | Clofarabin (Bioenvision) |
| | 2-Fluordesoxycytidin | Irofulven (MGI Pharrna) |
| | Methotrexat | DMDC (Hoffmann-La Roche) |
| | Idatrexate | Ethinylcytidin (Taiho) |
| | | |
| Topoisomerase-Inhibitoren | Amsacrin | Rubitecan (SuperGen) |
| | Epirubicin | Exatecanmesylat (Daiichi) |
| | Etoposid | Quinamed (ChemGenex) |
| | Teniposid oder Mitoxantron | Gimatecan (Sigma- Tau) |
| | Irinotecan (CPT-11) | Diflomotecan (Beaufour-Ipsen) |
| | 7-Ethyl-10-hydroxycamptothec | TAS-103 (Taiho) |
| | Topotecan | Elsamitrucin (Spectrum) |
| | Dexrazoxanet (TopoTarget) | J-107088 (Merck & Co) |
| | Pixantron (Novuspharrna) | BNP-1350 (BioNumerik) |
| | Rebeccamycin-Analogon | CKD-602 (Chong Kun Dang) |
| | (Exelixis) | KW-2170 (Kyowa Hakko) |
| | BBR-3576 (Novuspharrna) | |
| | | |
| Antitumor-Antibiotika | Dactinomycin (Actinomycin D) | Amonafid |
| | Doxorubicin (Adriamycin) | Azonafid |
| | Deoxyrubicin | Anthrapyrazol |
| | Valrubicin | Oxantrazol |
| | Daunorubicin (Daunomycin) | Losoxantron |
| | Epirubicin | Bleomycinsulfat (Blenoxan) |
| | Therarubicin | Bleomycinsäure |
| | Idarubicin | Bleomycin A |
| | Rubidazon | Bleomycin B |
| | Plicamycinp | Mitomycin C |
| | Porfiromycin | MEN-10755 (Menarini) |
| | Cyanomorpholinodoxorubicin | GPX-100 (Gem Pharmaceutica |
| | Mitoxantron (Novantron) | |
| | | |
| Antimitotische Mittel | Paclitaxel | SB 408075 (GlaxoSmithKline) |
| | Docetaxel | E7010 (Abbott) |
| | Colchicin | PG-TXL (Cell Therapeutics) |
| | Vinblastin | IDN 5109 (Bayer) |
| | Vincristin | A 105972 (Abbott) |
| | Vinorelbin | A 204197 (Abbott) |
| | Vindesin | LU 223651 (BASF) |
| | Dolastatin 10 (NCl) | D 24851 (ASTA Medica) |
| | Rhizoxin (Fujisawa) | ER-86526 (Eisai) |
| | Mivobulin (Warner-Lambert) | Combretastatin A4 (BMS) |
| | Cemadotin (BASF) | Isohomohalichondrin-B |
| | RPR 109881A (Aventis) | (PharmaMar) |
| | TXD 258 (Aventis) | ZD 6126 (AstraZeneca) |
| | Epothilon B (Novartis) | PEG-Paclitaxel (Enzon) |
| | T 900607 (Tularik) | AZ10992 (Asahi) |
| | T 138067 (Tularik) | !DN-5109 (Indena) |
| | Cryptophycin 52 (Eli Lilly) | AVLB (Prescient NeuroPharm |
| | Vinflunin (Fabre) | Azaepothilon B (BMS) |
| | Auristatin PE (Teikoku Hormor | BNP- 7787 (BioNumerik) |
| | BMS 247550 (BMS) | CA-4-Prodrug (OXiGENE) |
| | BMS 184476 (BMS) | Dolastatin-10 (NrH) |
| | BMS 188797 (BMS) | CA-4 (OXiGENE) |
| | Taxoprexin (Protarga) | |
| | | |
| Aromatase-Inhibitore | Aminoglutethimid | Exemestan |
| | Letrozol | Atamestan (BioMedicines) |
| | Anastrazol | YM-511 (Yamanouchi) |
| | Formestan | |
| Thymidylatsynthase-Inhibitoren | Pemetrexed (Eli Lilly) | Nolatrexed (Eximias) |
| | ZD-9331 (BTG) | CoFactor™ (BioKeys) |
| | | |
| DNA-Antagonisten | Trabectedin (PharmaMar) | Mafosfamid (Baxter Internation |
| | Glufosfamid (Baxter International) | Apaziquon (Spectrum Pharmaceuticals) |
| | Albumin + 32P (Isotope Solutions) | O6-Benzylguanin (Paligent) |
| | Thymectacin (NewBiotics) | |
| | Edotreotid (Novartis) | |
| | | |
| Farnesyltransferase-Inhibitoren | Arglabin (NuOncology Labs) | Tipifarnib (Johnson & Johnson |
| | Ionafarnib (Schering-Plough) | Perillylalkohol (DOR BioPharm |
| | BAY-43-9006 (Bayer) | |
| | | |
| Pumpen-Inhibitoren | CBT-1 (CBA Pharma) | Zosuquidar-Trihydrochlorid (EI Lilly) |
| | Tariquidar (Xenova) | |
| | MS-209 (Scherinq AG) | Biricodar-Dicitrat (Vertex) |
| | | |
| Histonacetyltransferase-Inhibitoren | Tacedinalin (Pfizer) | Pivaloyloxymethylbutyrat (Titar |
| | SAHA (Aton Pharma) | Depsipeptid (Fujisawa) |
| | MS-275 (Schering AG) | |
| | | |
| Metalloproteinase-Inhibitoren | Neovastat (Aeterna Laboratorii | CMT -3 (CollaGenex) |
| | Marimastat (British Biotech) | BMS-275291 (Celltech) |
| Ribonucleosidredukta - Inhibitoren | Galliummaltolat (Titan) | Tezacitabin (Aventis) |
| | Triapin (Vion) | Didox (Molecules for Health) |
| | | |
| TNF-alpha-Agonisten / Antagonisten | Virulizin (Lorus Therapeutics) | Revimid (Celgene) |
| | CDC-394 (Celgene) | |
| | | |
| Endothelin-A-Rezept | Atrasentan (Abbot) | YM-598 (Yamanouchi) |
| Antagonisten | ZD-4054 (AstraZeneca) | |
| | | |
| Retinsäurerezeptor- Aqonisten | Fenretinid (Johnson & Johnsor | Alitretinoin (Ligand) |
| | LGD-1550 (Ligand) | |
| | | |
| Immunmodulatoren | Interferon | Dexosom-Therapie (Anosys) |
| | Oncophage (Antigenics) | Pentrix (Australian Cancer |
| | GMK (Progenics) | Technology) |
| | Adenokarzinom-Impfstoff (Biomira) | JSF-154 (Tragen) |
| | | Krebsimpfstoff (Intercell) |
| | CTP-37 (AVI BioPharma) | Norelin (Biostar) |
| | JRX-2 (Immuno-Rx) | BLP-25 (Biomira) |
| | PEP-005 (Peplin Biotech) | MGV (Progenics) |
| | Synchrovax-Impfstoffe (CTL | !3-Alethin (Dovetail) |
| | Immuno) | CLL-Thera (Vasogen) |
| | Melanom-Impfstoff (CTL | |
| | Immuno) | |
| | p21-RAS-Impfstoff (GemVax) | |
| | | |
| Hormonelle und antihormonelle Mittel | Östrogene | Prednison |
| | konjugierte Östrogene | Methylprednisolon |
| | Ethinylöstradiol | Prednisolon |
| | Chlortrianisen | Aminoglutethimid |
| | Idenestrol | Leuprolid |
| | Hydroxyprogesteroncaproat | Goserelin |
| | Medroxyprogesteron | Leuporelin |
| | Testosteron | Bicalutamid |
| | Testosteronpropionat | Flutamid |
| | Fluoxymesteron | Octreotid |
| | Methyltestosteron | Nilutamid |
| | Diethylstilbestrol | Mitotan |
| | Megestrol | P-04 (Novogen) |
| | Tamoxifen | 2-Methoxyöstradiol (EntreMed |
| | Toremofin | Arzoxifen (Eli Lilly) |
| | Dexamethason | |
| | | |
| Photodynamische Mi | Talaporfin (Light Sciences) | Pd-Bacteriopheophorbid (Yeda |
| | Theralux (Theratechnologies) | Lutetium-Texaphyrin (Pharmacyclics) |
| | Motexafin-Gadolinium (Pharmacyclics) | |
| | | Hypericin |
| | | |
| Tyrosinkinase- Inhibitoren | Imatinib (Novartis) | Kahalid F (PharmaMar) |
| | Leflunomid (Sugen/Pharmacia | CEP- 701 (Cephalon) |
| | ZDI839 (AstraZeneca) | CEP-751 (Cephalon) |
| | Erlotinib (Oncogene Science) | MLN518 (Millenium) |
| | Canertjnib (Pfizer) | PKC412 (Novartis) |
| | Squalamin (Genaera) | Phenoxodiol O |
| | SU5416 (Pharmacia) | Trastuzumab (Genentech) |
| | SU6668 (Pharmacia) | C225 (ImClone) |
| | ZD4190 (AstraZeneca) | rhu-Mab (Genentech) |
| | ZD6474 (AstraZeneca) | MDX-H210 (Medarex) |
| | Vatalanib (Novartis) | 2C4 (Genentech) |
| | PKI166 (Novartis) | MDX-447 (Medarex) |
| | GW2016 (GlaxoSmithKline) | ABX-EGF (Abgenix) |
| | EKB-509 (Wyeth) | IMC-1C11 (ImClone) |
| | EKB-569 (Wyeth) | |
| Verschiedene Mittel | SR-27897 (CCK-A-Inhibitor, Sanofi-Synthelabo) | BCX-1777 (PNP-Inhibitor, BioCryst) |
| | Tocladesin (cyclisches-AMP- Agonist, Ribapharm) | Ranpirnase (Ribonuclease- Stimulans, Alfacell) |
| | Alvocidib (CDK-Inhibitor, Aven | Galarubicin (RNA-Synthese- Inhibitor, Dong-A) |
| | CV-247 (COX-2-Inhibitor, Ivy Medical) | |
| | | Tirapazamin (Reduktionsmittel SRI International) |
| | P54 (COX-2-Inhibitor, Phytopharm) | |
| | | N-Acetylcystein (Reduktionsmittel, Zambon) |
| | CapCell™ (CYP450-Stimulans Bavarian Nordic) | |
| | | R-Flurbiprofen (NF-kappaB- |
| | GCS-IOO (gal3-Antagonist, | Inhibitor, Encore) |
| | GlycoGenesys) | 3CPA (NF-kappaB-Inhibitor,Active Biotech) |
| | G17DT-Immunogen (Gastrin-Inhibitor, Aphton)- | Seocalcitol (Vitamin-D-RezeptAgonist, Leo) |
| | Efaproxiral (Oxygenator, AllosTherapeutics) | 131-I-TM-601 (DNA-AntagoniTransMolecular) |
| | PI-88 (Heparanase-Inhibitor,Progen) | Eflornithin (ODC-Inhibitor, ILEOncology) |
| | Tesmilifen (Histamin-AntagonisYM BioSciences) | Minodronsäure (Osteoclasten-Inhibitor, Yamanouchi)- |
| | Histamin (Histamin-H2-RezeptAgonist, Maxim) | Indisulam (p53-Stimulans, Eis |
| | Tiazofurin (IMPDH-Inhibitor,Ribapharm) | Aplidin (PPT-Inhibitor,PharmaMar) |
| | Cilengitid (Integrin-Antagonist,Merck KGaA) | Rituximab (CD20-Antikörper,Genentech) |
| | SR-31747 (IL-1-Antagonist,Sanofi-Synthelabo) | Gemtuzumab (CD33-AntikörpeWyeth Ayerst) |
| | CCI-779 (mTOR-Kinase-InhibilWyeth) | PG2 (Hämatopoese-VerstärkePharmagenesis) |
| | Exisulind (PDE-V-Inhibitor, CelPathways) | Immunol™ (Triclosan-Oralspülung, Endo)- |
| | CP-461 (PDE-V-Inhibitor, CellPathways) | Triacetyluridin (Uridin-Prodrug.Wellstat) |
| | AG-2037 (GART-Inhibitor, Pfiz | SN-4071 (Sarkom-Mittel,Signature BioScience) |
| | WX-UK1 (PlasminogenaktivatcInhibitor, Wilex) | TransMID-107™ (ImmunotoxinKS Biomedix) |
| | PBI-1402 (PMN-Stimulans,ProMetic LifeSciences) | PCK-3145 (Apoptose-FördererProcyon) |
| | Bortezomib (Proteasom-InhibitMillennium) | Doranidazol (Apoptose-FörderPola) |
| | SRL-172 (T-Zell-Stimulans, SFPharma) | CHS-828 (cytotoxisches Mittel Leo) |
| | TLK-286 (Glutathion-S-Transferase-Inhibitor, Telik)- | trans-Retinsäure (DifferentiatoNIH) |
| | PT-100 (Wachstumsfaktor-Agonist, Point Therapeutics)- | MX6 (Apoptose-Förderer,MAXIA) |
| | Midostaurin (PKC-Inhibitor,Novartis) | Apomin (Apoptose-Förderer,ILEX Oncology) |
| | Bryostatin-1 (PKC-Stimulans,GPC Biotech) | Urocidin (Apoptose-Förderer,Bioniche) |
| | CDA-II (Apoptose-Förderer,Everlife) | Ro-31-7453 (Apoptose-FörderLa Roche) |
| | SDX-101 (Apoptose-Förderer,Salmedix) | Brostallicin (Apoptose-FörderePharmacia) |
| | Ceflatonin (Apoptose-FördererChemGenex) | |
| Alkylierungsmittel | Cyclophosphamid | Lomustin |
| | Busulfan | Procarbazin |
| | Ifosfamid | Altretamin |
| | Melphalan | Estramustinphosphat |
| | Hexamethylmelamin | Mechlorethamin |
| | Thiotepa | Streptozocin |
| | Chlorambucil | Temozolomid |
| | Dacarbazin | Semustin |
| | Carmustin | |
| | | |
| Platinmittel | Cisplatin | Carboplatin |
| | Oxaliplatin | ZD-0473 (AnorMED) |
| | Spiroplatin | Lobaplatin (Aetema) |
| | Carboxyphthalatoplatinum | Satraplatin (Johnson Matthey) |
| | Tetraplatin | BBR-3464 (Hoffrnann-La Roch |
| | Ormiplatin | SM-11355 (Sumitomo) |
| | Iproplatin | AP-5280 (Access) |
| | | |
| Antimetabolite | Azacytidin | Tomudex |
| | Gemcitabin | Trimetrexate |
| | Capecitabin | Deoxycoformycin |
| | 5-Fluoruracil | Fludarabin |
| | Floxuridin | Pentostatin |
| | 2-Chlordesoxyadenosin | Raltitrexed |
| | 6-Mercaptopurin | Hydroxyharnstoff |
| | 6-Thioguanin | Decitabin (SuperGen) |
| | Cytarabin | Clofarabin (Bioenvision) |
| | 2-Fluordesoxycytidin | Irofulven (MGI Pharrna) |
| | Methotrexat | DMDC (Hoffmann-La Roche) |
| | Idatrexate | Ethinylcytidin (Taiho) |
| | | |
| Topoisomerase-Inhibitoren | Amsacrin | Rubitecan (SuperGen) |
| | Epirubicin | Exatecanmesylat (Daiichi) |
| | Etoposid | Quinamed (ChemGenex) |
| | Teniposid oder Mitoxantron | Gimatecan (Sigma- Tau) |
| | Irinotecan (CPT-11) | Diflomotecan (Beaufour-Ipsen) |
| | 7-Ethyl-10-hydroxycamptothec | TAS-103 (Taiho) |
| | Topotecan | Elsamitrucin (Spectrum) |
| | Dexrazoxanet (TopoTarget) | J-107088 (Merck & Co) |
| | Pixantron (Novuspharrna) | BNP-1350 (BioNumerik) |
| | Rebeccamycin-Analogon(Exelixis) | CKD-602 (Chong Kun Dang) |
| | | KW-2170 (Kyowa Hakko) |
| | BBR-3576 (Novuspharrna) | |
| Antitumor-Antibiotika | Dactinomycin (Actinomycin D) | Amonafid |
| | Doxorubicin (Adriamycin) | Azonafid |
| | Deoxyrubicin | Anthrapyrazol |
| | Valrubicin | Oxantrazol |
| | Daunorubicin (Daunomycin) | Losoxantron |
| | Epirubicin | Bleomycinsulfat (Blenoxan) |
| | Therarubicin | Bleomycinsäure |
| | Idarubicin | Bleomycin A |
| | Rubidazon | Bleomycin B |
| | Plicamycinp | Mitomycin C |
| | Porfiromycin | MEN-10755 (Menarini) |
| | Cyanomorpholinodoxorubicin | GPX-100 (Gem Pharmaceutica |
| | Mitoxantron (Novantron) | |
| | | |
| Antimitotische Mittel | Paclitaxel | SB 408075 (GlaxoSmithKline) |
| | Docetaxel | E7010 (Abbott) |
| | Colchicin | PG-TXL (Cell Therapeutics) |
| | Vinblastin | IDN 5109 (Bayer) |
| | Vincristin | A 105972 (Abbott) |
| | Vinorelbin | A 204197 (Abbott) |
| | Vindesin | LU 223651 (BASF) |
| | Dolastatin 10 (NCI) | D 24851 (ASTA Medica) |
| | Rhizoxin (Fujisawa) | ER-86526 (Eisai) |
| | Mivobulin (Warner-Lambert) | Combretastatin A4 (BMS) |
| | Cemadotin (BASF) | Isohomohalichondrin-B(PharmaMar) |
| | RPR 109881A (Aventis) | |
| | TXD 258 (Aventis) | ZD 6126 (AstraZeneca) |
| | Epothilon B (Novartis) | PEG-Paclitaxel (Enzon) |
| | T 900607 (Tularik) | AZ10992 (Asahi) |
| | T 138067 (Tularik) | !DN-5109 (Indena) |
| | Cryptophycin 52 (Eli Lilly) | AVLB (Prescient NeuroPharm |
| | Vinflunin (Fabre) | Azaepothilon B (BMS) |
| | Auristatin PE (Teikoku Hormor | BNP- 7787 (BioNumerik) |
| | BMS 247550 (BMS) | CA-4-Prodrug (OXiGENE) |
| | BMS 184476 (BMS) | Dolastatin-10 (NrH) |
| | BMS 188797 (BMS) | CA-4 (OXiGENE) |
| | Taxoprexin (Protarga) | |
| | | |
| Aromatase-Inhibitore | Aminoglutethimid | Exemestan |
| | Letrozol | Atamestan (BioMedicines) |
| | Anastrazol | YM-511 (Yamanouchi) |
| | Formestan | |
| | | |
| Thymidylatsynthase-Inhibitoren | Pemetrexed (Eli Lilly) | Nolatrexed (Eximias) |
| | ZD-9331 (BTG) | CoFactor™ (BioKeys) |
| | | |
| DNA-Antagonisten | Trabectedin (PharmaMar) | Mafosfamid (Baxter Internatio |
| | Glufosfamid (BaxterInternational) | Apaziquon (SpectrumPharmaceuticals) |
| | Albumin + 32P (IsotopeSolutions) | O6-Benzylguanin (Paligent) |
| | Thymectacin (NewBiotics) | |
| | Edotreotid (Novartis) | |
| | | |
| Farnesyltransferase-Inhibitoren | Arglabin (NuOncology Labs) | Tipifarnib (Johnson & Johnson |
| | lonafarnib (Schering-Plough) | Perillylalkohol (DOR BioPharm |
| | BAY-43-9006 (Bayer) | |
| | | |
| Pumpen-Inhibitoren | CBT-1 (CBA Pharma) | Zosuquidar-Trihydrochlorid (EI |
| | Tariquidar (Xenova) | Lilly) |
| | MS-209 (Schering AG) | Biricodar-Dicitrat (Vertex) |
| | | |
| Histonacetyltransfera - Inhibitoren | Tacedinalin (Pfizer) | Pivaloyloxymethylbutyrat (Titar |
| | SAHA (Aton Pharma) | Depsipeptid (Fujisawa) |
| | MS-275 (Schering AG) | |
| | | |
| Metalloproteinase-Inhibitoren Ribonucleosidredukt | Neovastat (Aeterna Laboratorii | CMT -3 (CollaGenex) |
| | Marimastat (British Biotech) | BMS-275291 (Celltech) |
| | Galliummaltolat (Titan) | Tezacitabin (Aventis) |
| - Inhibitoren | Triapin (Vion) | Didox (Molecules for Health) |
| | | |
| TNF-alpha-Agonisten/Antagonis | Virulizin (Lorus Therapeutics) | Revimid (Celgene) |
| | CDC-394 (Celgene) | |
| | | |
| Endothelin-A-Rezept Antaqonisten | Atrasentan (Abbot) | YM-598 (Yamanouchi) |
| | ZD-4054 (AstraZeneca) | |
| | | |
| Retinsäurerezeptor-Agonisten | Fenretinid (Johnson & Johnsor | Alitretinoin (Ligand) |
| | LGD-1550 (Ligand) | |
| | | |
| Immunmodulatoren | Interferon | Dexosom-Therapie (Anosys) |
| | Oncophage (Antigenics) | Pentrix (Australian Cancer Technology) |
| | GMK (Progenics) | |
| | Adenokarzinom-Impfstoff (Biomira) | JSF-154 (Tragen) |
| | | Krebsimpfstoff (Intercell) |
| | CTP-37 (AVI BioPharma) | Norelin (Biostar) |
| | JRX-2 (Immuno-Rx) | BLP-25 (Biomira) |
| | PEP-005 (Peplin Biotech) | MGV (Progenics) |
| | Synchrovax-Impfstoffe (CTLImmuno) | !3-Alethin (Dovetail) |
| | | CLL-Thera (Vasogen) |
| | Melanom-Impfstoff (CTL Immuno) | |
| | p21-RAS-Impfstoff (GemVax) | |
| | | |
| Hormonelle und antihormonelle Mittel | Östrogene | Prednison |
| | konjugierte Östrogene | Methylprednisolon |
| | Ethinylöstradiol | Prednisolon |
| | Chlortrianisen | Aminoglutethimid |
| | Idenestrol | Leuprolid |
| | Hydroxyprogesteroncaproat | Goserelin |
| | Medroxyprogesteron | Leuporelin |
| | Testosteron | Bicalutamid |
| | Testosteronpropionat | Flutamid |
| | Fluoxymesteron | Octreotid |
| | Methyltestosteron | Nilutamid |
| | Diethylstilbestrol | Mitotan |
| | Megestrol | P-04 (Novogen) |
| | Tamoxifen | 2-Methoxyöstradiol (EntreMed |
| | Toremofin | Arzoxifen (Eli Lilly) |
| | Dexamethason | |
| | | |
| Photodynamische Mi | Talaporfin (Light Sciences) | Pd-Bacteriopheophorbid (Yed |
| | Theralux (Theratechnologies) | Lutetium-Texaphyrin (Pharmacyclics) |
| | Motexafin-Gadolinium (Pharmacyclics) | |
| | | Hypericin |
| | | |
| Tyrosinkinase-Inhibitoren | Imatinib (Novartis) | Kahalid F (PharmaMar) |
| | Leflunomid (Sugen/Pharmacia | CEP- 701 (Cephalon) |
| | ZDI839 (AstraZeneca) | CEP-751 (Cephalon) |
| | Erlotinib (Oncogene Science) | MLN518 (Millenium) |
| | Canertjnib (Pfizer) | PKC412 (Novartis) |
| | Squalamin (Genaera) | Phenoxodiol O |
| | SU5416 (Pharmacia) | Trastuzumab (Genentech) |
| | SU6668 (Pharmacia) | C225 (ImClone) |
| | ZD4190 (AstraZeneca) | rhu-Mab (Genentech) |
| | ZD6474 (AstraZeneca) | MDX-H210 (Medarex) |
| | Vatalanib (Novartis) | 2C4 (Genentech) |
| | PKI166 (Novartis) | MDX-447 (Medarex) |
| | GW2016 (GlaxoSmithKline) | ABX-EGF (Abgenix) |
| | EKB-509 (Wyeth) | IMC-1C11 (ImClone) |
| | EKB-569 (Wyeth) | |
| | | |
| Verschiedene Mittel | SR-27897 (CCK-A-Inhibitor, Sanofi-Synthelabo) | BCX-1777 (PNP-Inhibitor, BioCryst) |
| | Tocladesin (cyclisches-AMP- Agonist, Ribapharm) | Ranpirnase (Ribonuclease- Stimulans, Alfacell) |
| | Alvocidib (CDK-Inhibitor, Aventis) | Galarubicin (RNA-Synthese- Inhibitor, Dong-A) |
| | CV-247 (COX-2-Inhibitor, Ivy Medical) | Tirapazamin (Reduktionsmittel, SRI International) |
| | P54 (COX-2-Inhibitor, Phytopharm) | N-Acetylcystein (Reduktionsmit Zambon) |
| | CapCell™ (CYP450-Stimulan Bavarian Nordic) | R-Flurbiprofen (NF-kappaB- Inhibitor, Encore) |
| | GCS-IOO (gal3-Antagonist, GlycoGenesys) | 3CPA (NF-kappaB-Inhibitor, Active Biotech) |
| | G17DT-Immunogen (Gastrin- Inhibitor, Aphton) | Seocalcitol (Vitamin-D-Rezeptol Agonist, Leo) |
| | Efaproxiral (Oxygenator, Allos Therapeutics) | 131-I-TM-601 (DNA-Antagonist TransMolecular) |
| | PI-88 (Heparanase-Inhibitor, Progen) | Eflornithin (ODC-Inhibitor, ILEX Oncology) |
| | Tesmilifen (Histamin-Antagoni YM BioSciences) | Minodronsäure (Osteoclasten- Inhibitor, Yamanouchi) |
| | Histamin (Histamin-H2-Rezeptor- Agonist, Maxim)- | Indisulam (p53-Stimulans, Eisa |
| | | Aplidin (PPT-Inhibitor,PharmaMar) |
| | Tiazofurin (IMPDH-Inhibitor, Ribapharm) | Rituximab (CD20-Antikörper,Genentech) |
| | Cilengitid (Integrin-AntagonistMerck KGaA) | |
| | | Gemtuzumab (CD33-AntikörpeWyeth Ayerst) |
| | SR-31747 (IL-1-Antagonist,Sanofi-Synthelabo) | |
| | | PG2 (Hämatopoese-VerstärkerPharmagenesis) |
| | CCI-779 (mTOR-Kinase-Inhibitor, Wyeth)- | |
| | | Immunol™ (Triclosan-Oralspülung, Endo)- |
| | Exisulind (PDE-V-Inhibitor, CεPathways) | |
| | | Triacetyluridin (Uridin-Prodrug,Wellstat) |
| | CP-461 (PDE-V-Inhibitor, CellPathways) | |
| | | SN-4071 (Sarkom-Mittel,Signature BioScience) |
| | AG-2037 (GART-Inhibitor,Pfizer) | |
| | | TransMID-107™ (ImmunotoxinKS Biomedix) |
| | WX-UK1 (PlasminogenaktivatInhibitor, Wilex) | |
| | | PCK-3145 (Apoptose-FördererProcyon) |
| | PBI-1402 (PMN-Stimulans,ProMetic LifeSciences) | |
| | | Doranidazol (Apoptose-FörderePola) |
| | Bortezomib (Proteasom-Inhibitor, Millennium)- | |
| | | CHS-828 (cytotoxisches Mittel,Leo) |
| | SRL-172 (T-Zell-Stimulans, SIPharma) | |
| | | trans-Retinsäure (DifferentiatorNIH) |
| | TLK-286 (Glutathion-S-Transferase-Inhibitor, Telik)- | |
| | | MX6 (Apoptose-Förderer, MAX |
| | PT-100 (Wachstumsfaktor- Agonist, Point Therapeutics) | Apomin (Apoptose-Förderer, IL Oncology) |
| | Midostaurin (PKC-Inhibitor, Novartis) | Urocidin (Apoptose-Förderer, Bioniche) |
| | Bryostatin-1 (PKC-Stimulans, GPC Biotech) | Ro-31-7453 (Apoptose-Fördere La Roche) |
| | CDA-II (Apoptose-Förderer, Everlife) | Brostallicin (Apoptose-Förderer Pharmacia) |
| | SDX-101 (Apoptose-Förderer, Salmedix) | |
| | Ceflatonin (Apoptose-Fördere ChemGenex) | |

Eine derartige gemeinsame Behandlung kann mithilfe gleichzeitiger, aufeinander folgender oder getrennter Dosierung der einzelnen Komponenten der Behandlung erzielt werden. Solche Kombinationsprodukte setzen die erfindungsgemäßen Verbindungen ein.

Ferner betrifft die Erfindung Verbindungen ausgewählt aus der Gruppe bestehend aus:

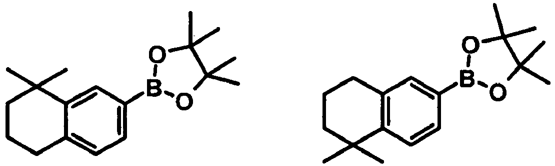

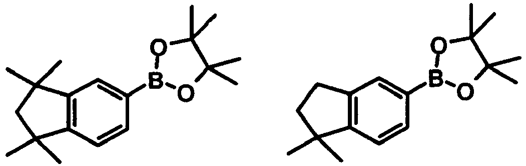

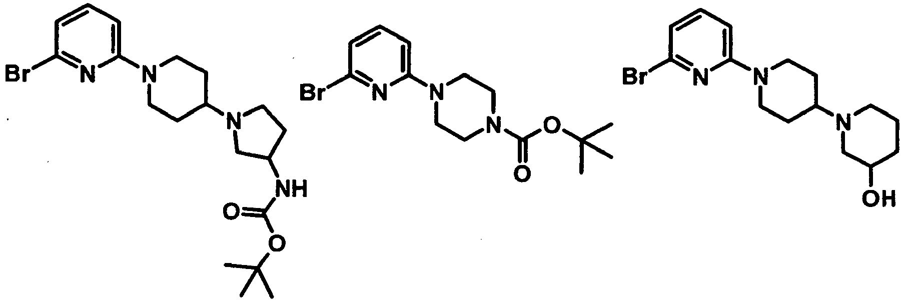

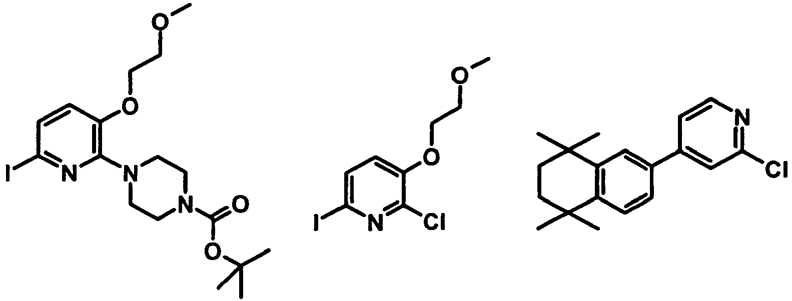

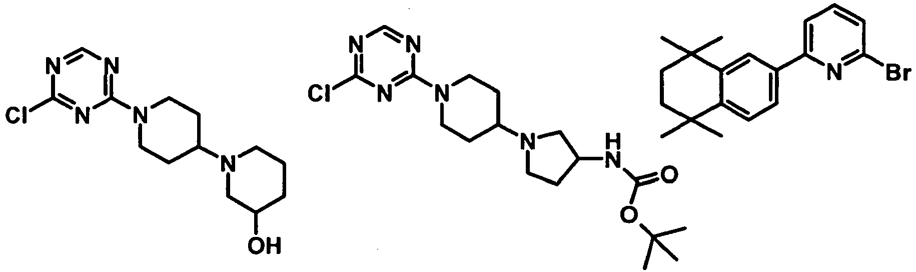

Auch ohne weitere Ausführungen wird davon ausgegangen, dass ein Fachmann die obige Beschreibung im weitesten Umfang nutzen kann. Die bevorzugten Ausführungsformen sind deswegen lediglich als beschreibende Offenbarung aufzufassen, die keineswegs in irgendeiner Weise limitierend ist.

Vor- und nachstehend sind alle Temperaturen in °C angegeben. In den nachstehenden Beispielen bedeutet "übliche Aufarbeitung": Man entfernt, falls erforderlich, das Lösungsmittel, gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, wäscht die organische Phase mit gesättigter NaHCO₃-Lösung, gegebenenfalls mit Wasser und gesättigter NaCl-Lösung, trocknet die organische Phase über Natriumsulfat, filtriert, engt ein und reinigt durch Chromatographie an Kieselgel, durch präparative HPLC und/oder durch Kristallisation. Die gereinigten Verbindungen werden gegebenenfalls gefriergetrocknet.

| | |
|---|---|
| Massenspektrometrie (MS): | EI (Elektronenstoß-Ionisation) M⁺ |
| | FAB (Fast Atom Bombardment) (M+H)⁺ |
| | ESI (Electrospray lonization) (M+H)⁺ |
| | APCI-MS (atmospheric pressure chemical ionization mass spectrometry) (M+H)⁺ |

### HPLC-Methoden:

### Methode A:

Gradient: 4.2 min
Fluss: 2 ml/min 99:01 - 0:100 Wasser + 0.1%(Vol.) TFA : Acetonitril + 0.1%(Vol.) TFA
0.0 bis 0.2 min: 99:01
0.2 bis 3.8 min: 99:01---> 0:100
3.8 bis 4.2 min: 0:100
Säule: Chromolith Performance RP18e; 100 mm lang, Innendurchmesser 3 mm
Wellenlänge: 220nm

### Methode B:

Fluss: 2.75 ml/min 90:10 - 0:100 Wasser + 0.01%(Vol.) TFA : Acetonitril +
0.01 %(Vol.) TFA
0.0 bis 3.5 min: 90:10 ---> 0:100
3.5 bis 4.3 min: 0:100
Säule: Chromolith SpeedRod RP18e; 50 mm lang, Innendurchmesser 4.6 mm
Wellenlänge: 220nm

### Methode C:

Fluss: 2.4 ml/min 85:15 - 0:100 Wasser + 0.05%(Vol.) AcOH : Acetonitril + 0.05%(Vol.) AcOH
0.0 bis 2.8 min: 85:15 ---> 0:100
2.8 bis 3.3 min: 0:100
Säule: Chromolith SpeedRod RP18e; 50 mm lang, Innendurchmesser 4.6 mm
Wellenlänge: 220nm

Liste der Abkürzungen und Akronyme:

AcOH Essigsäure, wf. wasserfrei, atm Atmosphäre(n), BOC tert-Butoxycarbonyl CDI 1,1'-Carbonyldiimidazol, konz. konzentriert, d Tag(e), Zers. Zersetzung, DMAC N,N-Dimethylacetamid, DMPU 1,3-Dimethyl-3,4,5,6-tetrahydro-2(1H)-pyrimidinon, DMF N,N-Dimethylformamid, DMSO Dimethylsulfoxid, DPPA Diphenylphosphorylazid, EDCI 1-(3-Dimethylaminopropyl)-3-ethylcarbodiimid, EtOAc Ethylacetat, EtOH Ethanol (100%), Et₂O Diethylether, Et₃N Triethylamin, h Stunde(n), MeOH Methanol, Pet.-Ether Petrolether (Siedebereich 30-60°C), Temp. Temperatur, THF Tetrahydrofuran, TFA TrifluorAcOH, Tf Trifluormethansulfonyl, RT Raumtemperatur.

Die Erfindung wird durch die folgenden Beispiele näher erläutert.

### Beispiele

### I. Synthese ausgewählter Verbindungen der Erfindung

Die folgenden Verbindungen wurden synthetisiert und charakterisiert. Es gehört jedoch zu den Kenntnissen des Fachmanns, diese Verbindungen auf andere Weise herzustellen und zu charakterisieren.

### FS101:

### 4-[6-(5,5,8,8-Tetramethyl-5,6,7,8-tetrahydro-naphthalen-2-yl)-pyridin-2-yl]-piperazine-1-carboxylic acid tert-butyl ester:

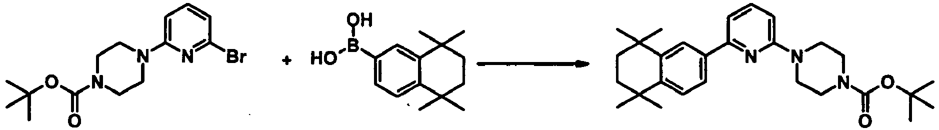

150 mg (0.44 mmol) 4-(6-Bromo-pyridin-2-yl)-piperazine -1-carboxylic acid tert-butyl ester, 112 mg (0.48 mmol) 5,5,8,8-Tetramethyl-5,6,7,8-tetrahydronaphthalen-2-yl-boronsäure und 202 mg (0.88 mmol) Trikaliumphosphat Monohydrat werden in 6 ml Ethylenglycolmonomethylether suspendiert, mehrfach entgast und unter Stickstoffatmosphäre mit 25 mg (0.04 mmol) Bis(triphenylphosphin)-palladium(II)-dichlorid versetzt. Das Reaktionsgemisch wird 16 h bei 80°C gerührt, auf RT abgekühlt und mit 20 ml Wasser versetzt. Dieses Gemisch wird dreimal mit Essigester extrahiert, über Na₂SO₄ getrocknet und eingeengt. Das Rohprodukt wird mittels Flashchromatographie an Kieselgel aufgereinigt.

220 mg Öl, Rt. = 3.75 min (Methode A), LCMS: 450 (M+H).

### FS102:

### 4-[6-(5,5-Dimethyl-5,6,7,8-tetrahydro-naphthalen-2-yl)-pyridin-2-yl]-piperazine-1-carboxylic acid tert-butyl ester:

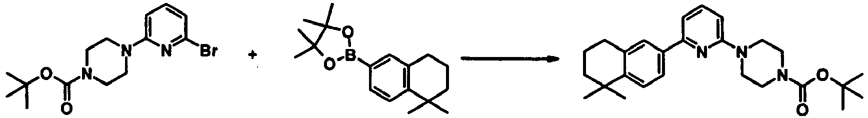

500 mg (1.46 mmol) 4-(6-Bromo-pyridin-2-yl)-piperazine -1-carboxylic acid tert-butyl ester, 460 mg (1.61 mmol) 2-(5,5-Dimethyl-5,6,7,8-tetrahydro-naphthalen-2-yl)-4,4,5,5-tetramethyl-[1,3,2]dioxaborolane und 620 mg (2.92 mmol) Trikaliumphosphat Trihydrat werden in 10 ml Ethylenglycolmonomethylether suspendiert, mehrfach entgast und unter Stickstoffatmosphäre mit 82 mg (0.04 mmol) Bis(triphenylphosphin)-palladium(II)-dichlorid versetzt. Das Reaktionsgemisch wird 10 min im Ultraschallbad behandelt, anschließend 90 min bei 100°C in der Mikrowelle bestrahlt, mit 50 ml Wasser und 50 ml Ethylacetat versetzt und filtriert. Der Rückstand wird verworfen. Die organische Phase des Filtrats wird abgetrennt und die wässrige Phase wird dreimal mit Essigester extrahiert. Die vereinigten organischen Phasen werden über Na₂SO₄ getrocknet und eingeengt. Das Rohprodukt wird mittels Flashchromatographie an Kieselgel aufgereinigt:

482 mg Öl, Rt. = 3.93 min (Methode B), LCMS: 422 (M+H).

### FS 103:

### 4-[2-(5,5,8,8-Tetramethyl-5,6,7,8-tetrahydro-naphthalen-2-yl)-pyrimidin-4-yl]-piperazine-1-carboxylic acid tert-butyl ester:

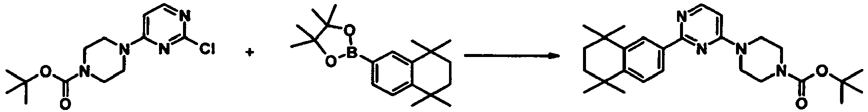

100 mg (0.33 mmol) 4-(2-Chloro-pyrimidin-4-yl)-piperazine-1-carboxylic acid tert-butyl ester (Herstellung analog US 2005/176722) und 147 mg (0.47 mmol) 4,4,5,5-Tetramethyl-2-(5,5,8,8-tetramethyl-5,6,7,8-tetrahydro-naphthalen-2-yl)-[1,3,2]dioxaborolane werden in mit 2.6 ml Toluol/Ethanol (4:1) und 335 µl 2N Kaliumcarbonatlösung versetzt. Das Reaktionsgemisch wird mehrfach entgast, unter Stickstoffatmosphäre mit 16 mg (0.01 mmol) Tetrakis(triphenylphosphin)-palladium(0) versetzt, 10 min im Ultraschallbad behandelt und anschließend 10 min bei 140°C in der Mikrowelle bestrahlt. Das Reaktionsgemisch wird mit Wasser und Dichlormethan versetzt. Die organische Phase wird abgetrennt und die wässrige Phase wird dreimal mit Essigester extrahiert. die vereinigten organischen Phasen werden über Na₂SO₄ getrocknet und eingeengt. Das Rohprodukt wird mittels Flashchromatographie an Kieselgel aufgereinigt. 81 mg, weißer Feststoff, Rt. = 3.31 min (Methode A), LCMS: 451 (M+H).

### FS104:

### 4-[4-(5,5,8,8-Tetramethyl-5,6,7,8-tetrahydro-naphthalen-2-yl)-pyrimidin-2-yl]-piperazine-1-carboxylic acid tert-butyl ester:

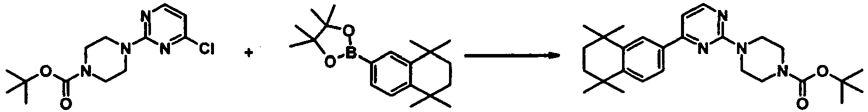

Die Herstellung erfolgt analog FS 103 ausgehend von 4-(4-Chloro-pyrimidin-2-yl)-piperazine-1-carboxylic acid tert-butyl ester (Herstellung analog US 2005/176722) und 4,4,5,5-Tetramethyl-2-(5,5,8,8-tetramethyl-5,6,7,8-tetrahydro-naphthalen-2-yl)-[1,3,2]dioxaborolane.

120 mg, weißer Feststoff, Rt. = 3.71 min (Methode A), LCMS: 451 (M+H).

### FS105:

### 6'-(5,5,8,8-Tetramethyl-5,6,7,8-tetrahydro-naphthalen-2-yl)-2,3,5,6-tetrahydro-(1,2']bipyrazinyl-4-carboxylic acid tert-butyl ester:

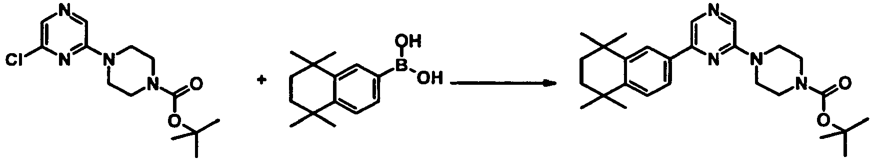

Die Herstellung erfolgt analog FS 102 ausgehend von 6'-Chloro-2,3,5,6-tetrahydro-[1,2']bipyrazinyl-4-carboxylic acid tert-butyl ester (Herstellung analog US 2005/176722) und 5,5,8,8-Tetramethyl-5,6,7,8-tetrahydro-naphthalen-2-yl-boronsäure.

700 mg, gelbes Öl, Rt. = 4.06 min (Methode A), LCMS: 451 (M+H).

### FS106:

### 4-[4-(5,5,8,8-Tetramethyl-5,6,7,8-tetrahydro-naphthalen-2-yl)-[1,3,5]triazin-2-yl]-piperazine-1-carboxylic acid tert-butyl ester:

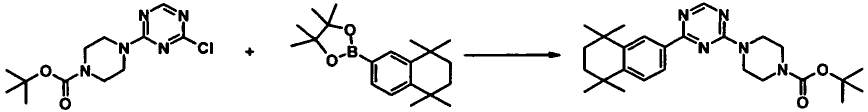

Die Herstellung erfolgt analog FS 102 ausgehend von 4-(4-Chloro-[1,3,5]triazin-2-yl)-piperazine-1-carboxylic acid tert-butyl ester (Herstellung analog US 2005/59668) und 4,4,5,5-Tetramethyl-2-(5,5,8,8-tetramethyl-5,6,7,8-tetrahydro-naphthalen-2-yl)-[1, 3,2]dioxaborolane.

213 mg, farbloses Öl, Rt. = 3.82 min (Methode A), LCMS: 452 (M+H).

### FS107:

### 4-[6-(8,8-Dimethyl-5,6,7,8-tetrahydro-naphthalen-2-yl)-pyridin-2-yl]-piperazine-1-carboxylic acid tert-butyl ester:

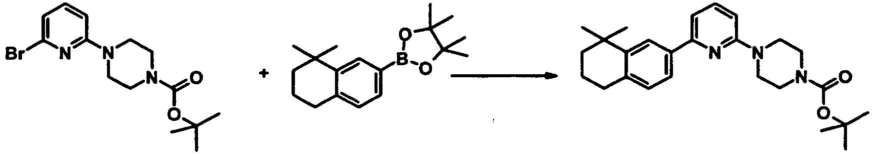

Die Herstellung erfolgt analog FS 102 ausgehend von 4-(6-Bromo-pyridin-2-yl)-piperazine-1-carboxylic acid tert-butyl ester (US 2005/176722) und 2-(8,8-Dimethyl-5,6,7,8-tetrahydro-naphthalen-2-yl)-4,4,5,5-tetramethyl-[1,3,2]dioxaborolane.

747 mg, gelbes Öl, Rt. = 3.88 min (Methode B), LCMS: 422 (M+H).

¹H NMR (400 MHz, DMSO/deuteriertes TFA) δ 8.00 (dd, *J* = 8.8, 7.6, 1H), 7.79 (d, *J* = 1.5.1H), 7.55 (dd, *J* = 7.9, 1.7, 1 H), 7.27 - 7.17 (m, 3H), 3.82 - 3.70 (m, 4H), 3.63 - 3.50 (m, 4H), 2.82 (t, *J* = 6.1, 2H), 1.86 -1.77 (m, 2H), 1.72 -1.66 (m, 2H), 1.45 (s, 9H), 1.34 (s, 6H).

### FS108:

### 4-[6-(1,1,3,3-Tetramethyl-indan-5-yl)-pyridin-2-yl]-piperazine-1-carboxylic acid tert-butyl ester:

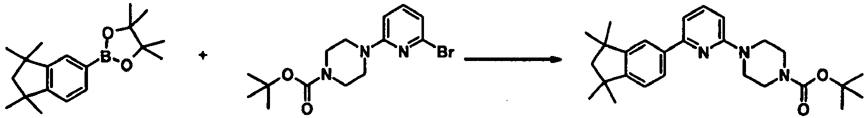

Die Herstellung erfolgt analog FS 102 ausgehend von 4-(6-Bromo-pyridin-2-yl)-piperazine-1-carboxylic acid tert-butyl ester (US 2005/176722) und 4,4,5,5-Tetramethyl-2-(1,1,3,3-tetramethyl-indan-5-yl)-[1,3,2]dioxaborolane.

219 mg, farbloses Öl, Rt. = 3.27 min (Methode B), LCMS: 436 (M+H).

¹H NMR (500 MHz, DMSO/deuteriertes TFA) δ 8.06 (dd, *J* = 9.1, 7.4, 1H), 7.65 (dd, *J* = 7.9, 1.7, 1H),7.58(d, *J* = 1.6, 1 H), 7.38 (d, *J* = 7.9, 1H), 7.31 (d, *J* = 9.1, 1H), 7.19 (d, *J* = 7.3, 1 H), 3.82 - 3.76 (m, 4H), 3.63- 3.57 (m, 4H), 1.99 (d, *J* = 1.7, 2H), 1.46 (s, 9H), 1.34 (t, *J* = 14.1, 12H).

### FS109:

### 4-[2-(5,5,8,8-Tetramethyl-5,6,7,8-tetrahydro-naphthalen-2-yl)-pyridin-4-yl]-piperazine-1-carboxylic acid tert-butyl ester:

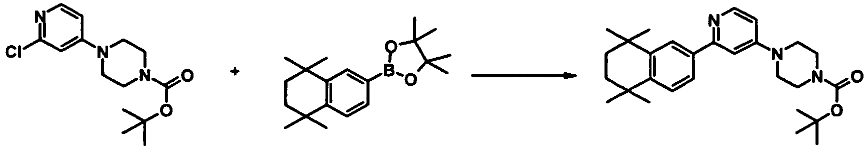

202 mg (0.68 mmol) 4-(2-Chloro-pyridin-4-yl)-piperazine-1-carboxylic acid tert-butyl ester, 256 mg (0.81 mmol) 4,4,5,5-Tetramethyl-2-(5,5,8,8-tetramethyl-5,6,7,8-tetrahydro-naphthalen-2-yl)-[1,3,2]dioxaborolane und 187 mg (1.36 mmol) Kaluimcarbonat werden in 6 ml Acetonitril und 650 µl Wasser gelöst. Das Reaktionsgemisch wird mehrfach entgast, unter Stickstoffatmosphäre mit 78 mg (0.07 mmol) Tetrakis(triphenylphosphin)-palladium versetzt, 90 min bei 120°C in der Mikrowelle bestrahlt, anschließend mit Wasser verdünnt und dreimal mit Essigester extrahiert. Die vereinigten organischen Phasen werden über Na₂SO₄ getrocknet und eingeengt. Das Rohprodukt wird säulenchromatographisch an RP-Kieselgel aufgereinigt.

196 mg Öl, Rt. = 2.93 min (Methode B), LCMS: 450 (M+H).

### FS110:

### 4-[6-(1,1-Dimethyl-indan-5-yl)-pyridin-2-yl]-piperazine-1-carboxylic acid tert-butyl ester:

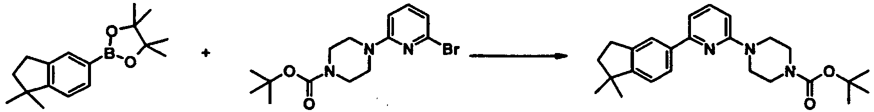

Die Herstellung erfolgt analog FS 102 ausgehend von 4-(6-Bromo-pyridin-2-yl)-piperazine-1-carboxylic acid tert-butyl ester (US 2005/176722) und 2-(1,1-Dimethyl-indan-5-yl)-4,4,5,5-tetramethyl-[1,3,2]dioxaborolane.

267 mg, gelbes Öl, Rt. = 3.77 min (Methode B), LCMS: 408 (M+H).

### FS111:

### 4-[3-(5,5,8,8-Tetramethyl-5,6,7,8-tetrahydro-naphthalen-2-yl)-phenyl]-piperazine-1-carboxylic acid tert-butyl ester:

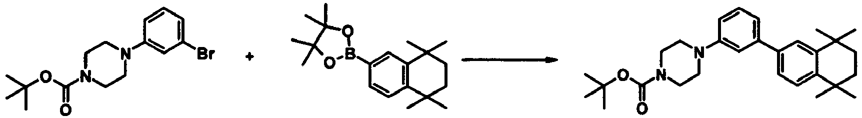

Die Herstellung erfolgt analog FS 102 ausgehend von 4-(3-Bromo-phenyl)-piperazine-1-carboxylic acid tert-butyl ester (WO2007/97937) und 4,4,5,5-Tetramethyl-2-(5,5,8,8-tetramethyl-5,6,7,8-tetrahydro-naphthalen-2-yl)-[1,3,2]dioxaborolane.

554 mg, gelbes Öl, Rt. = 4.24 min (Methode B).

¹H NMR (400 MHz, DMSO/deuteriertes TFA) δ 7.96 (s, 1 H), 7.76 (d, *J* = 7.5, 1 H), 7.70 - 7.60 (m, 3H), 7.51 - 7.44 (m, 2H), 3.79 (d, *J* = 36.9, 8H), 1.72 (s, 4H), 1.48 (s, 9H), 1.33 (d, *J* = 20.2, 12H).

### FS112:

### 4-[4-(5,5-Dimethyl-5,6,7,8-tetrahydro-naphthalen-2-yl)-[1,3,5]triazin-2-yl]-piperazine-1-carboxylic acid tert-butyl ester:

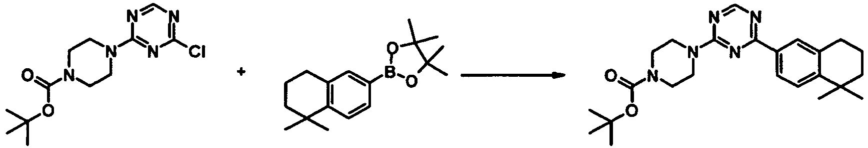

Die Herstellung erfolgt analog FS 102 ausgehend von 4-(4-Chloro-[1,3,5]triazin-2-yl)-piperazine-1-carboxylic acid tert-butyl ester (US 2005/59668) und 2-(5,5-Dimethyl-5,6,7,8-tetrahydro-naphthalen-2-yl)-4,4,5,5-tetramethyl-[1,3,2]dioxaborolane.

62 mg, farbloses Öl, Rt. = 3.78 min (Methode A), LCMS: 424 (M+H).

### FS113:

### 4-[4-(8,8-Dimethyl-5,6,7,8-tetrahydro-naphthalen-2-yl)-[1,3,5]triazin-2-yl]-piperazine-1-carboxylic acid tert-butyl ester:

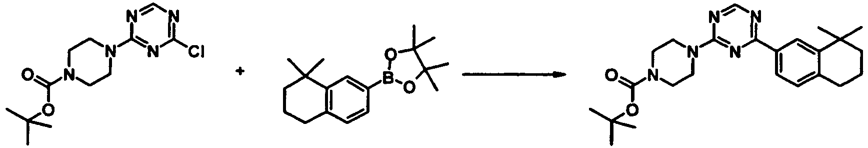

Die Herstellung erfolgt analog FS 102 ausgehend von 4-(4-Chloro-[1,3,5]triazin-2-yl)-piperazine-1-carboxylic acid tert-butyl ester (US 2005/59668) und 2-(8,8-Dimethyl-5,6,7,8-tetrahydro-naphthalen-2-yl)-4,4,5,5-tetramethyl-[1,3,2]dioxaborolane.

55 mg, gelbes Öl, Rt. =4.13 min (Methode A), LCMS: 424 (M+H).

### FS114:

### 1-[2-Methyl-5-(5,5,8,8-tetramethyl-5,6,7,8-tetrahydro-naphthalen-2-yl)-phenyl]-piperazine:

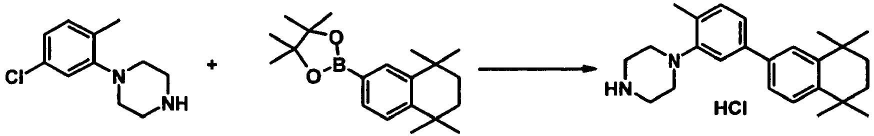

100 mg (0.48 mmol) 1-(5-Chloro-2-methyl-phenyl)-piperazine und 300 mg (0.95 mmol) und 4,4,5,5-Tetramethyl-2-(5,5,8,8-tetramethyl-5,6,7,8-tetrahydronaphthalen-2-yl)-[1,3,2]dioxaborolane werden in 2 ml THF gelöst, mit 302 mg (1.43 mmol) Kaliumphosphat, 3 mg Palladiumacetat und 11 mg 2-Dicylcohexylphosphino-2',4',6'-tri-i-propyl-l,l'-biphenyl versetzt. Das Reaktionsgemisch wird mehrfach entgast und unter Stickstoffatmosphäre 4 h bei 120°C gerührt, anschließend mit Wasser verdünnt und dreimal mit Essigester extrahiert. Die vereinigten organischen Phasen werden über Na₂SO₄ getrocknet und eingeengt. Das Rohprodukt wird mittels präparativer HPLC aufgereinigt und mit methanolischer HCl ins Hydrochlorid überführt. 6 mg beigefarbener Feststoff, Rt. = 3.33 min (Methode A), LCMS: 363 (M+H).

¹H NMR (400 MHz, DMSO/deuteriertes TFA) ö 7.50 (d, *J* = 1.7, 1H), 7.42 - 7.33 (m, 2H), 7.27 (s, 2H), 7.21 (s, 1 H), 3.35 - 3.26 (m, 4H), 3.20 - 3.12 (m, 4H), 2.32 (s, 3H), 1.69 (s, 4H), 1.30 (d, *J* = 14.3, 12H).

### FS115:

### (S)-3-Methyl-1-[3-(5,5,8,8-tetramethyl-5,6,7,8-tetrahydro-naphthalen-2-yl)-phenyl]-piperazine:

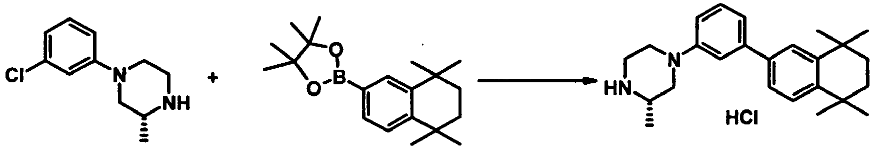

Die Herstellung erfolgt analog FS 114.

49 mg, beigefarbener Feststoff, Rt. = 3.21 min (Methode A), LCMS: 363 (M+H).

¹H NMR (400 MHz, DMSO/deuteriertes TFA) δ 7.52 (s, 1 H), 7.42 - 7.31 (m, 3H), 7.21 (s, 1H), 7.13 (d, *J* = 7.8, 1H), 7.02 (dd, *J* = 8.2, 1.9, 1H), 3.87 (t, *J* = 14.4, 2H), 3.45 (d, *J* = 12.4, 2H), 3.29 - 3.17 (m, 1 H), 3.08 (t, *J* = 11.1, 1H), 2.86 (dd, *J* = 12.9, 10.7, 1 H), 1.70 (s, 4H), 1.39 -1.27 (m, 15H).

### FS116:

### (S)-1-[4-Methoxy-3-(5,5,8,8-tetramethyl-5,6,7,8-tetrahydro-naphthalen-2-yl)-phenyl]-3-methyl-piperazine:

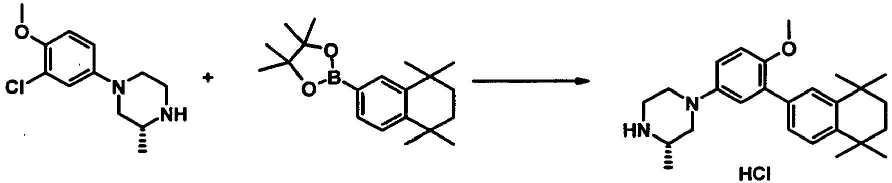

Die Herstellung erfolgt analog FS 114. Produkt liegt als Hyrochlorid vor. 8 mg, beigefarbener Feststoff, Rt. = 3.14 min (Methode A), LCMS: 393 (M+H). ¹H NMR (500 MHz, DMSO/deuteriertes TFA) δ 7.49 (d, *J* = 1.7, 1H), 7.37 (d, *J* = 8.2, 1 H), 7.27 (dd, J = 8.2, 1.8, 1H), 7.23 - 7.15 (m, 2H), 7.10 (d, J = 8.8, 1 H), 3.82 - 3.72 (m, 5H), 3.57 (ddd, J = 25.4, 16.2, 7.7, 2H), 3.39 - 3.25 (m, 2H), 3.15 - 3.06 (m, 1 H), 1.72 (s, 4H), 1.37 - 1.28 (m, 15H).

### FS117:

### (1-[2-(5,5,8,8-Tetramethyl-5,6,7,8-tetrahydro-naphthalen-2-yl)-pyrimidin-4-yl]-piperidin-4-yl)-carbaminsäure-tert-butylester:

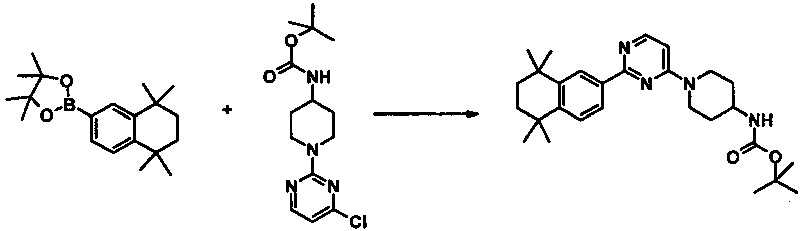

Die Herstellung erfolgt analog FS103.

Ausbeute: 454 mg, farbloses Öl. Rt. = 3.21 min (Methode A), LCMS: 465 (M+H). 1 H NMR (500 MHz, DMSO/deuteriertes TFA) δ 8.14 (d, J = 7.5 Hz, 1 H), 8.03 (d, J = 2.0 Hz, 1H), 7.84 (dd, J = 8.4, 2.0 Hz, 1 H), 7.44 (d, J = 8.4 Hz, 1 H), 6.98 (d, J = 7.6 Hz, 1 H), 4.80 (d, J = 13.6 Hz, 1 H), 4.08 (d, J = 14.0 Hz, 1 H), 3.91 (q, J = 7.1 Hz, 1 H), 3.63 (s, 1 H), 3.40 - 3.27 (m, 2H), 1.88 (d, J = 10.1 Hz, 2H), 1.60 (s, 4H), 1.49 - 1.36 (m, 2H), 1.30 (s, 9H), 1.20 (d, J = 21.8 Hz, 12H).

### FS118:

### {1-(4-(5,5,8,8-Tetramethyl-5,6,7,8-tetrahydro-naphthalen-2-yl)-pyrimidin-2-yl]-piperidin-4-yl}-carbaminsäre-tert-butyl ester:

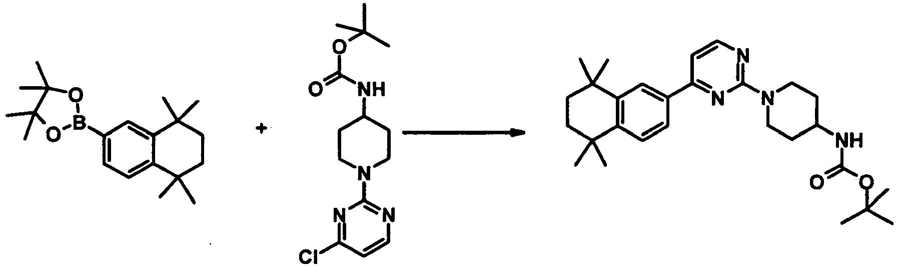

Die Herstellung erfolgt analog FS103.

Ausbeute: 516 mg, farbloses Öl. Rt. = 3.47 min (Methode A), LCMS: 465 (M+H). 1 H NMR (500 MHz, DMSO/deuteriertes TFA) δ 8.38 (d, J = 6.7 Hz, 1 H), 8.19 (d, J = 1.8 Hz, 1 H), 8.00 (dd, J = 8.3, 1.6 Hz, 1H), 7.58 (dd, J = 18.5, 11.1 Hz, 2H), 4.51 (s, 2H), 3.75 (s, 1 H), 3.48 (dd, J = 17.9, 6.9 Hz, 2H), 2.10 - 1.97 (m, 2H), 1.74 (s, 4H), 1.60 (ddd, J = 17.4, 13.7, 6.5 Hz, 2H), 1.44 (s, 9H), 1.34 (d, J = 17.8 Hz, 12H).

### FS119:

### 2-[6-(5,5,8,8-Tetramethyl-5,6,7,8-tetrahydro-naphthalen-2-yl)-3',4',5',6',3",4",5",6"-octahydro-2'H,2"H-[2,1';4',4"]terpyridin-1"-yl]-ethanol:

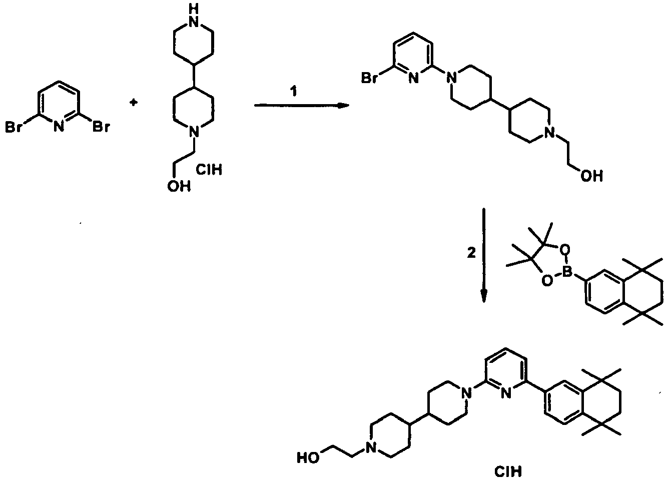

### Stufe 1:

223 mg (0.94 mmol) 2,6-Dibrompyridin, 234 mg (0.94 mmol) 2-[4,4']Bipiperidinyl-1-yl-ethanol Hydrochlorid und 364 mg Kaliumcarbonat werden in 5 ml NMP supendiert und 18 h bei 100°C gerührt. Anschließend wird filtriert, das Filtrat mit ges. Natriumcarbonatlösung versetzt und der gebildete Niederschlag wird abgesaugt. Das Rohprodukt wird getrocknet und direkt weiter umgesetzt. 176 mg, gelblicher Feststoff, Rt. = 2.41 min (Methode A), LCMS: 369 (M+H)

### Stufe 2:

100 mg (0.27 mmol) 2-(6-Bromo-3',4',5',6',3",4",5",6"-octahydro-2'H,2"H-[2,1';4',4"]terpyridin-1"-yl)-ethanol, 85 mg (0.27 mmol) 4,4,5,5-Tetramethyl-2-(5,5,8,8-tetramethyl-5,6,7,8-tetrahydro-naphthalen-2-yl)-[1,3,2]dioxaborolane und 27 mg (0.33 mmol) Natriumhydrogencarbonat werden in 10 ml DMF und 3 ml Wasser gelöst, entgast und unter Stickstoffatmosphäre mit 7 mg Bis(triphenylphosphin)-palladium(II)-chlorid versetzt. Das Reaktionsgemisch wird 18 h bei 80°C gerührt, anschließend mit 20 ml Wasser verdünnt, mit 2 * 20 ml Ethylacetat extrahiert und über Natriumsulfat getrocknet. Das Lösungsmittel wird am Rotationsverdampfer entfernt und das Rohprodukt mittels präp HPLC aufgereinigt. Das Produkt wird mit methanolischer HCl ins Hydrochlorid überführt. Ausbeute: 20 mg, gelbes, zähes Öl. Rt. = 2.69 min (Methode A), LCMS: 476 (M+H).

¹H NMR (500 MHz, DMSO/deuteriertes TFA) δ 7.93 (dd, J = 9.1, 7.4 Hz, 1 H), 7.58 (d, J = 1.8 Hz, 1 H), 7.49 (d, J = 8.2 Hz, 1 H), 7.42 (dd, J = 8.2, 1.9 Hz, 1 H), 7.25 (d, J = 9.2 Hz, 1 H), 7.01 (d, J = 7.3 Hz, 1H), 4.28 (d, J = 13.4 Hz, 2H), 3.77 - 3.69 (m, 2H), 3.53 (d, J = 13.2 Hz, 2H), 3.27 - 3.06 (m, 4H), 2.86 (t, J = 11.8 Hz, 2H), 1.84 (t, J = 10.3 Hz, 4H), 1.68 (s, 4H), 1.63 -1.29 (m, 6H), 1.27 (d, J = 10_{.}4 Hz, 12H).

### FS120:

### 1-[2-Methoxy-5-(5,5,8,8-tetramethyl-5,6,7,8-tetrahydro-naphthalen-2-yl)-phenyl]-piperazine:

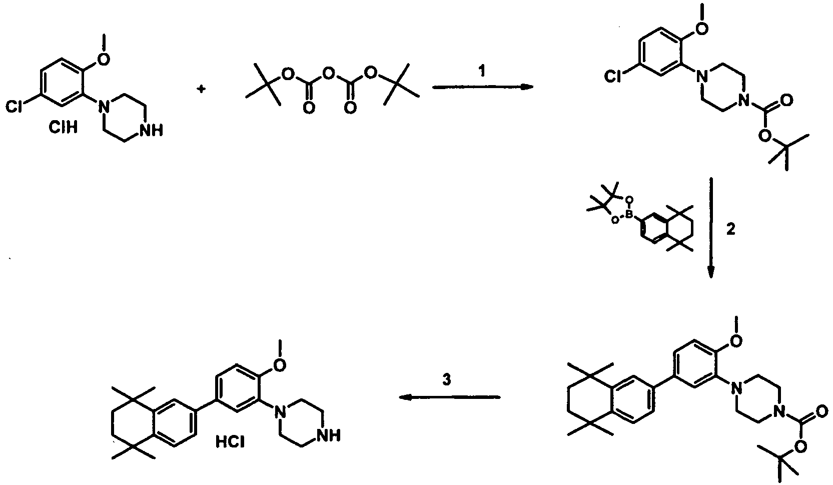

### Stufe 1:

500 mg (1.9 mmol) 1-(5-Chloro-2-methoxy-phenyl)-piperazine Hydrochlorid wird in 15 ml DMF gelöst, mit 316 µl (2.28 mmol) Triethylamin versetzt und 5 min gerührt. Anschließend wird eine Lösung aus 447 µl (2.09 mmol) Di-tert-butyldicarbonat zugetropft und 18 h bei Raumtemperatur gerührt. Das Reaktionsgemisch wird eingedampft, mit Dichlormethan versetzt, extrahiert, getrocknet und eingedampft. Das Rohprodukt wurde ohne weitere Aufreinigung weiter umgesetzt.

Rt. = 3.26 min (Methode A), LCMS: 327 (M+H).

### Stufe 2:

Die Umsetzung mit 4,4,5,5-Tetramethyl-2-(5,5,8,8-tetramethyl-5,6,7,8-tetrahydro-naphthalen-2-yl)-(1,3,2)dioxaborolan erfolgt analog FS114.

Rt. = 3.47 min (Methode A), LCMS: 479 (M+H).

### Stufe 3:

Die Abspaltung der Schutzgruppe erfolgt analog FS201. Das Produkt liegt als Hydrochlorid vor.

Ausbeute: 25 mg, gelbes, zähes Öl. Rt. = 3.12 min (Methode A), LCMS: 379 (M+H).

¹H NMR (500 MHz, DMSO/deuteriertes TFA) 7.46 (d, J = 1.7 Hz, 1H), 7.37 - 7.28 (m, 3H), 7.23 (d, J = 2.1 Hz, 1 H), 7.05 (d, J = 8.5 Hz, 1 H), 3.85 (s, 3H), 3.41 (d, J = 5.6 Hz, 4H), 3.34 (d, J = 5.4 Hz, 4H), 1.66 (s, 4H), 1.26 (d, J = 19.7 Hz, 12H).

### FS121:

### 2-[6-Piperazin-1-yl-2-(5,5,8,8-tetramethyl-5,6,7,8-tetrahydro-naphthalen-2-yl)-pyridin-3-yloxy]-ethanol:

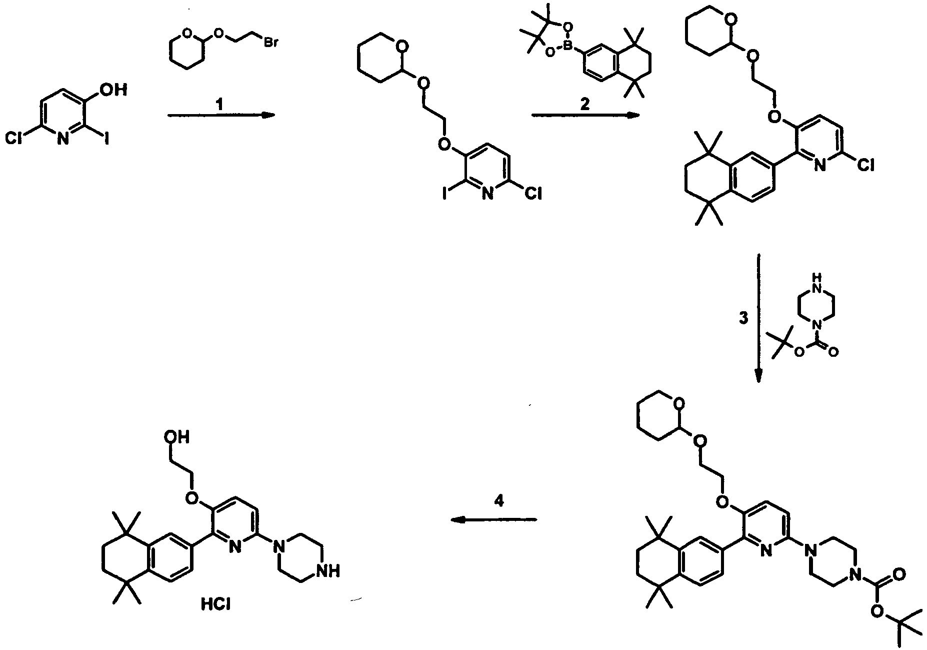

### Stufe 1:

500 mg (1.96 mmol) 6-Chloro-2-iodo-pyridin-3-ol und 700 mg (2.15 mmol) Cäsiumcarbonat werden in DMF suspendiert und mit 325 µl (2.15 mmol) 2-(2-Bromoethoxy)tetrahydro-2H-pyran versetzt. Das Reaktionsgemisch wird 18h bei RT gerührt, anschließend mit Wasser versetzt und mehrfach mit Ethylacetat extrahiert, getrocknet und eingedampft. Das Rohprodukt wird direkt weiter umgesetzt. Ausbeute: 846 mg. Rt. = 3.11 min (Methode A), LCMS: 384 (M+H).

### Stufe 2:

Die Suzuki Reaktion mit 4,4,5,5-Tetramethyl-2-(5,5,8,8-tetramethyl-5,6,7,8-tetrahydro-naphthalen-2-yl)-[1,3,2]dioxaborolane erfolgt analog FS102.

Ausbeute: 46 mg. Rt. = 4.15 min (Methode A), LCMS: 444/446 (M+H).

### Stufe 3:

46 mg (0.104 mmol) 6-Chloro-3-[2-(tetrahydro-pyran-2-yloxy)-ethoxy]-2-(5,5,8,8-tetramethyl-5,6,7,8-tetrahydro-naphthalen-2-yl)-pyridine wurde mit 39 mg (0.207 mmol) *tert*-Butyl-1-piperazinecarboxylate eingewogen und in 1 ml Toluol gelöst. Das Reaktionsgemisch wurde entgast und mit 10 mg Tris(dibenzylideneacetone)dipalladium(0), 7 mg *rac*-2,2'-Bis(diphenylphosphino)-1,1'-binaphtyl und 23 mg Kalium-tert-butoxid zugegeben. Das Reaktionsgemisch wurde nochmals entgast und unter Stickstoffatmosphäre wurde das Reaktionsgemisch 18h bei 90°C gerührt. Nach dem Abkühlen wurde Ether zugegeben und mit Wasser gewaschen, getrocknet und zum Rückstand abgezogen. Dieser wurde ohne weitere Aufreinigung direkt weiter umgesetzt.

### Stufe 4:

Die Abspaltung der Schutzgruppen wird analog FS201 durchgeführt. Das Produkt liegt als Hydrochlorid vor.

Ausbeute: 19 mg, weißer Feststoff. Rt. = 2.68 min (Methode A), LCMS: 410 (M+H).

¹H NMR (500 MHz, DMSO/deuteriertes TFA) δ 7.94 (d, J = 1.8 Hz, 1 H), 7.79 (d, J = 9.3 Hz, 1 H), 7.65 (dd, J = 8.2, 1.8 Hz, 1 H), 7.41 (d, J = 8.3 Hz, 1 H), 7.08 (d, J = 9.3 Hz, 1 H), 4.08 (t, J = 5.0 Hz, 2H), 3.80 - 3.68 (m, 6H), 3.32 - 3.22 (m, 4H), 1.70 (s, 4H), 1.29 (d, J = 6.5 Hz, 12H).

### FS121:

### 2-(2-[6-Piperazin-1-yl-2-(5,5,8,8-tetramethyl-5,6,7,8-tetrahydro-naphthalen-2-yl)-pyridin-3-yloxy]-ethyl)-isoindole-1,3-dione

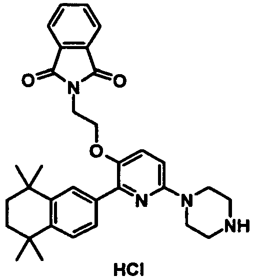

Die Herstellung erfolgt analog FS120. Das Produkt liegt als Hydrochlorid vor. Ausbeute: 74 mg. Rt. = 3.09 min (Methode A), LCMS: 539 (M+H).

### FS122:

### 5'-(5,5,8,8-Tetramethyl-5,6,7,8-tetrahydro-naphthalen-2-yl)-3,4,5,6-tetrahydro-2H-[1,3']bipyridinyl-4-ylamine

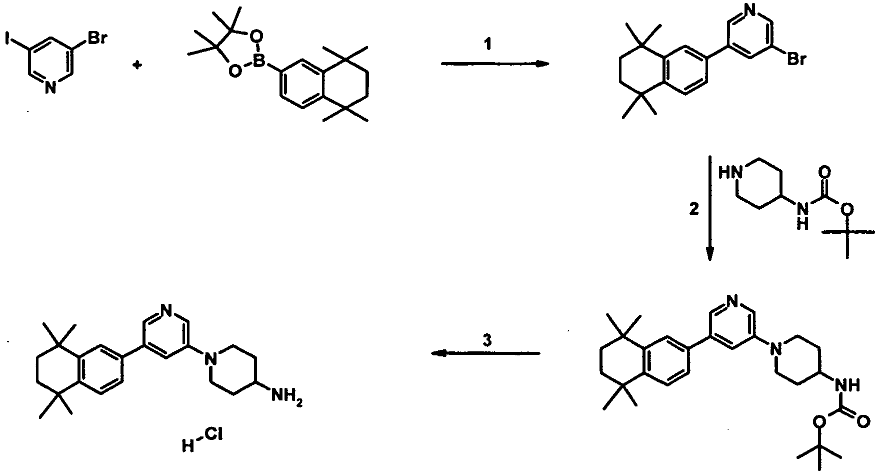

### Stufe 1:

Die Suzuki Reatkion wurde analog STI102 durchgeführt.

Ausbeute: 1.6 g. Rt. = 3.88 min (Methode A), LCMS: 344/346 (M+H).

### Stufe 2:

Die Umsetzung erfolgt analog Stufe 3 von FS120. Das Rohprodukt wird direkt weiter umgesetzt.

### Stufe 3:

Die Abspaltung der Schutzgruppe wird analog FS201 durchgeführt. Das Produkt liegt als Hydrochlorid vor.

Ausbeute: 6 mg. Rt. = 2.51 min (Methode A), LCMS: 364 (M+H).

¹H NMR (400 MHz, DMSO/deuteriertes TFA) δ 8.53 (d, J = 1.0 Hz, 1H), 8.47 (d, J = 2.5 Hz, 1H), 8.21 (dd, J = 2.5, 1.5 Hz, 1 H), 7.73 (d, J = 1.9 Hz, 1 H), 7.60 - 7.56 (m, 1 H), 7.52 (d, J = 8.3 Hz, 1 H), 4.17 (d, J = 13.2 Hz, 2H), 3.43 - 3.34 (m, 1 H), 3.16 - 3.06 (m, 2H), 2.06 (d, J = 10.2 Hz, 2H), 1.76 -1.60 (m, 6H), 1.33 (d, J = 19.8 Hz, 12H).

### FS123:

### 1-[5-(5,5,8,8-Tetramethyl-5,6,7,8-tetrahydro-naphthalen-2-yl)-pyridin-3-yl]-piperazine:

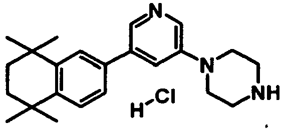

Die Herstellung erfolgt analog FS122. Das Produkt liegt als Hydrochlorid vor. Ausbeute: 35 mg. Rt. = 2.48 min (Methode A), LCMS: 350 (M+H).

¹H NMR (400 MHz, DMSO/deuteriertes TFA) δ 8.63 (d, J = 1.2 Hz, 1 H), 8.52 (d, J = 2.5 Hz, 1 H), 8.27 (d, J = 1.6 Hz, 1 H), 7.75 (d, J = 2.0 Hz, 1H), 7.60 (dd, J = 8.3, 2.0 Hz, 1 H), 7.52 (d, J = 8.3 Hz, 1 H), 3.85 - 3.73 (m, 4H), 3.38 - 3.26 (m, 4H), 1.71 (s, 4H), 1.33 (d, J = 20.5 Hz, 12H).

### FS124:

### 1-Piperidin-4-yl-4-[6-(5,5,8,8-tetramethyl-5,6,7,8-tetrahydro-naphthalen-2-yl)-pyridin-2-yl]-piperazin

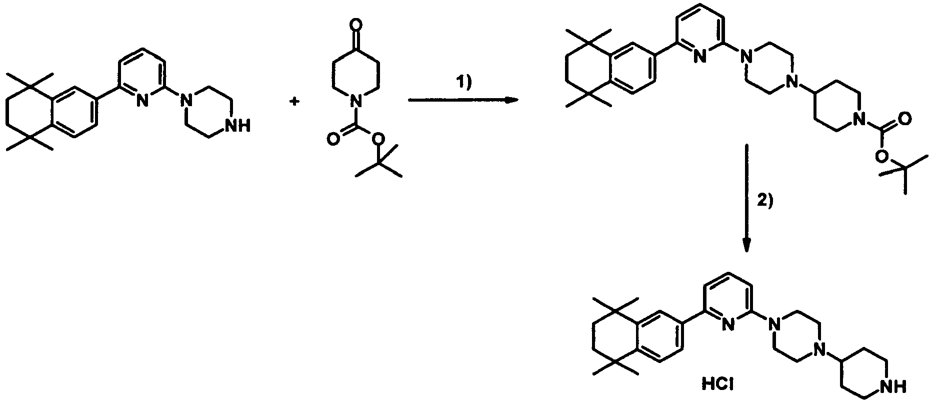

### Stufe 1:

Die reduktive Aminierung erfolgt analog FS501.

Ausbeute: 420 mg. Rt. = 3.32 min (Methode A), LCMS: 533 (M+H).

### Stufe 2:

Die Abspaltung der Schutzgruppe erfolgt analog FS201. Das Produkt liegt als Hydrochlorid vor.

Ausbeute: 370 mg. Rt. = 2.78 min (Methode A), LCMS: 433 (M+H).

¹H NMR (500 MHz, DMSO/deuteriertes TFA) δ 7.90 (dd, J = 8.7, 7.6 Hz, 1H), 7.73 (d, J = 1.9 Hz, 1 H), 7.55 (dd, J = 8.2, 1.9 Hz, 1 H), 7.45 (d, J = 8.3 Hz, 1 H), 7.18 (dd, J = 16.5, 8.1 Hz, 2H), 3.80 - 3.73 (m, 1 H), 3.35 - 3.62 (m, 6H), 3.23 - 3.15 (m, 1 H), 3.01 - 2.87 (m, 3H), 2.29 (d, J = 12.5 Hz, 2H), 1.99 -1.81 (m, 3H), 1.67 (s, 4H), 1.65 - 1.56 (m, 1 H), 1.26 (d, J = 14.3 Hz, 12H).

### FS125: 1-[5-Methoxy-6-(5,5,8,8-tetramethyl-5,6,7,8-tetrahydro-naphthalen-2-yl)-pyridin-2-yl]-piperazine:

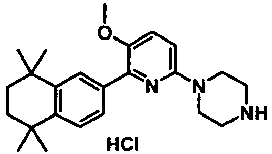

Die Herstellung erfolgt analog FS120. Das Produkt liegt als Hydrochlorid vor. Ausbeute: 12 mg. Rt. = 2.99 min (Methode A), LCMS: 380 (M+H).

¹H NMR (400 MHz, DMSO/deuteriertes TFA) δ 7.84 (d, J = 1.8 Hz, 1 H), 7.61 (dd, J = 8.3, 1.8 Hz, 1 H), 7.52 (d, J = 9.1 Hz, 1 H), 7.34 (d, J = 8.3 Hz, 1H), 6.90 (d, J = 9.0 Hz, 1 H), 3.77 (s, 3H), 3.72 - 3.61 (m, 4H), 3.18 (d, J = 10.3 Hz, 4H), 1.67 (s, 4H), 1.27 (s, 12H).

### FS126:

### 6'-(5,5,8,8-Tetramethyl-5,6,7,8-tetrahydro-naphthalen-2-yl)-3,4,5,6-tetrahydro-2H-[1,2']bipyridinyl-4-ol:

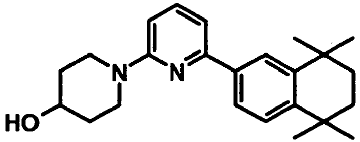

Die Herstellung erfolgt analog FS119.

Ausbeute: 6 mg. Rt. = 2.87 min (Methode A), LCMS: 365 (M+H).

¹H NMR (500 MHz, DMSO/deuteriertes TFA) δ 7.95 (dd, J = 9.2, 7.3 Hz, 1H), 7.61 (d, J = 1.9 Hz, 1 H), 7.50 (d, J = 8.2 Hz, 1 H), 7.45 (dd, J = 8.2, 1.9 Hz, 1 H), 7.29 (d, J = 9.1 Hz, 1 H), 7.03 (d, J = 7.3 Hz, 1 H), 3.99 - 3.83 (m, 3H), 3.58 - 3.49 (m, 2H), 1.94 -1.84 (m, 2H), 1.68 (s, 4H), 1.63 - 1.54 (m, 2H), 1.28 (d, J = 11.5 Hz, 12H).

### FS127:

### 6'-(5,5,8,8-Tetramethyl-5,6,7,8-tetrahydro-naphthalen-2-yl)-3,4,5,6-tetrahydro-2H-[1,2']bipyridinyl-4-carboxylic acid:

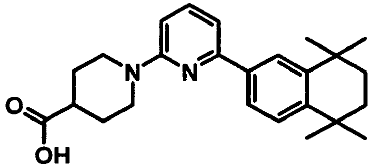

Die Herstellung erfolgt analog FS119. Anschließend wurde der Ethylester mit Lithiumhydroxidlösung in THF zur Zielverbindung gespalten.

Ausbeute: 11 mg. Rt. = 2.94 min (Methode A), LCMS: 393 (M+H).

¹H NMR (500 MHz, DMSO/deuteriertes TFA): δ 7.97 (dd, J = 9.1, 7.4 Hz, 1H), 7.62 (d, J = 1.9 Hz, 1 H), 7.50 (d, J = 8.2 Hz, 1 H), 7.46 (dd, J = 8.2, 1.9 Hz, 1 H), 7.30 (d, J = 9.2 Hz, 1 H), 7.05 (d, J = 7.2 Hz, 1 H), 4.15 (d, J = 13.6 Hz, 2H), 3.36 (dd, J = 18.0, 7.0 Hz, 2H), 2.64 (ddd, J = 14.7, 10.4, 4.1 Hz, 1H), 2.01 (dd, J = 13.7, 3.3 Hz, 2H), 1.81 - 1.71 (m, 2H), 1.68 (s, 4H), 1.28 (d, J = 11.7 Hz, 12H).

### FS201:

### 1-[6-(5,5,8,8-Tetramethyl-5,6,7,8-tetrahydro-naphthalen-2-yl)-pyridin-2-yl]-piperazine:

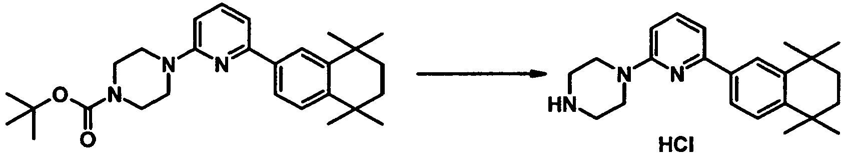

115 mg (0.26 mmol) 4-[6-(5,5,8,8-Tetramethyl-5,6,7,8-tetrahydro-naphthalen-2-yl)-pyridin-2-yl]-piperazine-1-carboxylic acid tert-butyl ester werden in 5 ml Dioxan gelöst, mit 2.5 ml 4N HCl in Dioxan versetzt und 15 h bei RT gerührt. Das Reaktionsgemisch wird eingedampft, der Rückstand mit Wasser versetzt, mit 1 N NaOH basisch gestellt und mit Ethylacetat extrahiert. Die organische Phase wird über Na₂SO₄ getrocknet und eingeengt. Das Rohprodukt wird mittels präparativer HPLC aufgereinigt, der Rückstand mit 1.25 M HCl in Methanol versetzt und eingedampft. 26 mg, weißer Feststoff. Produkt ist das Hydrochlorid.

Rt. = 3.08 min (Methode A), LCMS: 350 (M+H).

¹H NMR (400 MHz, DMSO/deuteriertes TFA) δ 7.97 (t, *J* = 8.1, 1 H), 7.82 (d, *J* = 1.5, 1H), 7.65 (dd, *J*= 8.2,1.4, 1 H), 7.51 (d, *J* = 8.2, 1 H), 7.25 (dd, *J* = 25.3, 8.1, 2H), 3.97 (b, 4H), 3.35 (b, 4H), 1.73 (s, 4H), 1.39 - 1.14 (m, 12H).

### FS202: 1-[6-(5,5-Dimethyl-5,6,7,8-tetrahydro-naphthalen-2-yl)-pyridin-2-yl]-piperazine:

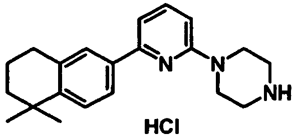

Die obige Verbindung wird analog FS201 ausgehend von 4-[6-(5,5-Dimethyl-5,6,7,8-tetrahydro-naphthalen-2-yl)-pyridin-2-yl]-piperazine-1-carboxylic acid tert-butyl ester hergestellt. Produkt ist das Hydrochlorid.

Ausbeute: 318 mg, beigefarbener Feststoff. Rt. = 2.52 min (Methode B), LCMS: 322 (M+H).

¹H NMR (500 MHz, DMSO/deuteriertes TFA) ö 7.92 - 7.87 (m, 1H), 7.70 (dd, *J* = 8.2, 1.5, 1H), 7.61 (d, *J* = 0.8, 1 H), 7.51 (d, *J* = 8.2, 1 H), 7.27 (d, *J* = 7.4, 1H), 7.12 (d, *J* = 8.7, 1 H), 3.95 - 3.90 (m, 4H), 3.34 - 3.29 (m, 4H), 2.84 (t, *J* = 6.2, 2H), 1.85 -1.78 (m, 2H), 1.72 -1.67 (m, 2H), 1.30 (s, 6H).

### FS203: 4-Piperazin-1-yl-2-(5,5,8,8-tetramethyl-5,6,7,8-tetrahydro-naphthalen-2-yl)-pyrimidine:

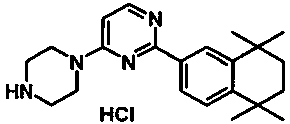

Die obige Verbindung wird analog FS201 ausgehend von 4-[2-(5,5,8,8-Tetramethyl-5,6,7,8-tetrahydro-naphthalen-2-yl)-pyrimidin-4-yl]-piperazine-1-carboxylic acid tert-butyl ester hergestellt. Produkt ist das Hydrochlorid. Ausbeute: 36 mg, weißer Feststoff. Rt. = 2.48 min (Methode A), LCMS: 351 (M+H).

¹H NMR (500 MHz, DMSO/deuteriertes TFA) δ 8.51 (d, *J* = 7.5, 1H), 8.20 (d, *J* = 1.9, 1 H), 8.02 (dd, *J* = 8.4, 1.8, 1 H), 7.60 (d, *J* = 8.4, 1 H), 7.26 (d, *J* = 7.5, 1 H), 4.23 (d, *J* = 139.4, 4H), 3.41 - 3.35 (m, 4H), 1.73 (s, 4H), 1.33 (d, *J* = 21.6, 12H).

### FS204: 2-Piperazin-1-yl-4-(5,5,8,8-tetramethyl-5,6,7,8-tetrahydro-naphthalen-2-yl)-pyrimidine:

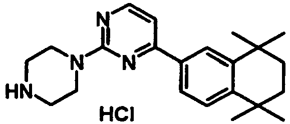

Die obige Verbindung wird analog FS201 ausgehend von 4-[4-(5,5,8,8-Tetramethyl-5,6,7,8-tetrahydro-naphthalen-2-yl)-pyrimidin-2-yl]-piperazine-1-carboxylic acid tert-butyl ester hergestellt. Produkt ist das Hydrochlorid. Ausbeute: 50 mg, weißer Feststoff. Rt. = 2.97 min (Methode A), LCMS: 351 (M+H).

¹H NMR (400 MHz, DMSO/deuteriertes TFA) δ 8.52 (d, J = 5.8, 1 H), 8.13 (d, *J* = 1.9, 1 H), 7.94 (dd, *J* = 8.3, 1.9, 1 H), 7.49 (dd, *J* = 9.5, 7.1, 2H), 4.18 - 4.11 (m, 4H), 3.37 - 3.28 (m, 4H), 1.72 (s, 4H), 1.33 (d, *J* = 16.2, 12H).

### FS205: 6'-(5,5,8,8-Tetramethyl-5,6,7,8-tetrahydro-naphthalen-2-yl)-3,4,5,6-tetrahydro-2H-[1,2']bipyrazinyl:

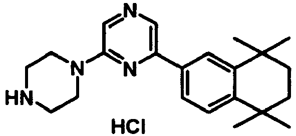

Die obige Verbindung wird analog FS201 ausgehend von 6'-(5,5,8,8-Tetramethyl-5,6,7,8-tetrahydro-naphthalen-2-yl)-2,3,5,6-tetrahydro-[1,2']bipyrazinyl-4-carboxylic acid tert-butyl ester hergestellt. Produkt ist das Hydrochlorid.

Ausbeute: 602 mg, gelber Feststoff. Rt. = 2.85 min (Methode A), LCMS: 351 (M+H).

¹H NMR (500 MHz, DMSO/deuteriertes TFA) δ 8.60 (s, 1 H), 8.40 (s, 1 H), 8.02 (d, *J* = 1.8, 1 H), 7.83 (dd, *J* = 8.3, 1.9, 1 H), 7.47 (d, *J* = 8.3, 1 H), 4.02 - 3.90 (m, 4H), 3.36 - 3.21 (m, 4H), 1.70 (s, 4H), 1.31 (d, *J* = 21.7, 12H).

### FS206:

### 2-Piperazin-1-yl-4-(5,5,8,8-tetramethyl-5,6,7,8-tetrahydro-naphthalen-2-yl)-[1,3,5]triazine:

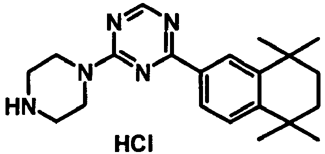

Die obige Verbindung wird analog FS201 ausgehend von 4-[4-(5,5,8,8-Tetramethyl-5,6,7,8-tetrahydro-naphthalen-2-yl)-[1,3,5]triazin-2-yl]-piperazine-1-carboxylic acid tert-butyl ester hergestellt. Produkt ist das Hydrochlorid. Ausbeute: 115 mg, weißer Feststoff. Rt. = 2.78 min (Methode A), LCMS: 352 (M+H).

¹H NMR (400 MHz, DMSO/deuteriertes TFA) δ 9.00 (s, 1H), 8.34 (d, *J* = 2.0, 1 H), 8.14 (dd, *J* = 8.4, 2.0, 1 H), 7.58 (d, *J* = 8.4, 1 H), 4.28 (d, *J* = 37.1, 4H), 3.36 (s, 4H), 1.73 (s, 4H), 1.33 (d, *J* = 14.1, 12H).

### FS207:

### 1-[6-(8,8-Dimethyl-5,6,7,8-tetrahydro-naphthalen-2-yl)-pyridin-2-yl]-piperazine:

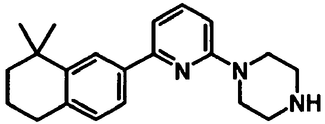

Die obige Verbindung wird analog FS201 ausgehend von 4-[6-(8,8-Dimethyl-5,6,7,8-tetrahydro-naphthalen-2-yl)-pyridin-2-yl]-piperazine-1-carboxylic acid tert-butyl ester hergestellt.

Ausbeute: 470 mg, Öl. Rt. = 2.53 min (Methode B), LCMS: 322 (M+H).

¹H NMR (400 MHz, DMSO/deuteriertes TFA) δ 7.92 (d, *J* = 1.5, 1 H), 7.88 - 7.82 (m, 1 H), 7.67 (dd, *J* = 7.9, 1.7, 1 H), 7.30 (d, *J* = 7.5, 1 H), 7.18 (d, *J* = 8.0, 1 H), 7.08 (d, *J* = 8.6, 1H), 3.96 - 3.85 (m, 4H), 3.36 - 3.27 (m, 4H), 2.80 (t, *J* = 6.2, 2H), 1.84 - 1.76 (m, 2H), 1.73 - 1.66 (m, 2H), 1.33 (s, 6H).

### FS208:

### 1-[6-(1,1,3,3-Tetramethyl-indan-5-yl)-pyridin-2-yl]-piperazine:

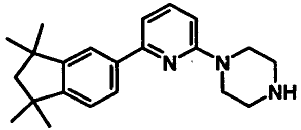

Die obige Verbindung wird analog FS201 ausgehend von 4-[6-(1,1,3,3-Tetramethyl-indan-5-yl)-pyridin-2-yl]-piperazine-1-carboxylic acid tert-butyl ester hergestellt. Ausbeute: 169 mg, Öl. Rt. = 2.73 min (Methode B), LCMS: 336 (M+H).

¹H NMR (400 MHz, DMSO/deuteriertes TFA) δ 7.98 (dd, *J* = 8.8, 7.5, 1H), 7.73 (dd, *J* = 7.9, 1.7, 1H), 7.64 (d, *J* = 1.5, 1 H), 7.34 (d, *J* = 7.9, 1 H), 7.28 (d, *J* = 7.3, 1H), 7.23 (d, *J* = 8.8, 1 H), 4.00 - 3.94 (m, 4H), 3.38 - 3.32 (m, 4H), 1.98 (s, 2H), 1.36 (d, *J* = 9.2, 12H).

### FS209:

### 1-[2-(5,5,8,8-Tetramethyl-5,6,7,8-tetrahydro-naphthalen-2-yl)-pyridin-4-yl]-piperazine:

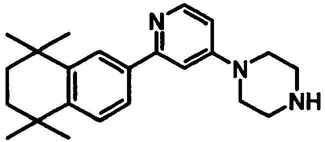

Die obige Verbindung wird analog FS201 ausgehend von 4-[2-(5,5,8,8-Tetramethyl-5,6,7,8-tetrahydro-naphthalen-2-yl)-pyridin-4-yl]-piperazine-1-carboxylic acid tert-butyl ester hergestellt.

Ausbeute: 86 mg, zähes Öl. Rt. = 1.97 min (Methode B), LCMS: 350 (M+H).

¹H NMR (400 MHz, DMSO/deuteriertes TFA) δ 8.34 (d, *J* = 7.1, 1H), 7.76 (s, 1 H), 7.64 (d, *J* = 8.4, 1H), 7.56 (d, *J* = 8.1, 1H), 7.41 (d, *J* = 2.6, 1H), 7.23 (dd, *J* = 7.4, 2.2, 1H), 4.04 - 3.94 (m, 4H), 3.37 - 3.23 (m, 4H), 1.68 (s, 4H), 1.30 (d, *J* = 15.9, 12H).

### FS210:

### 1-[6-(1,1-Dimethyl-indan-5-yl)-pyridin-2-yl]-piperazine:

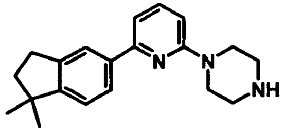

Die obige Verbindung wird analog FS201 ausgehend von 4-[6-(1,1-Dimethyl-indan-5-yl)-pyridin-2-yl]-piperazine-1-carboxylic acid tert-butyl ester hergestellt. Ausbeute: 192 mg, gelbes Öl. Rt. = 1.72 min (Methode B), LCMS: 308 (M+H).

¹H NMR (400 MHz, DMSO/deuteriertes TFA) ¹H NMR (400 MHz, DMSO) δ 7.90 (dd, *J* = 8.8, 7.6, 1H), 7.67 - 7.60 (m, 2H), 7.27 (d, *J* = 7.8, 1H), 7.19 (d, *J* = 7.4, 1 H), 7.14 (d, *J* = 8.8, 1H), 3.93 - 3.83 (m, 4H), 3.31 - 3.22 (m, 4H), 2.90 (t, *J* = 7.2, 2H), 1.90 (t, *J* = 7.2, 2H), 1.21 (s, 7H).

### FS211:

### 1-[3-(5,5,8,8-Tetramethyl-5,6,7,8-tetrahydro-naphthalen-2-yl)-phenyl]-piperazine:

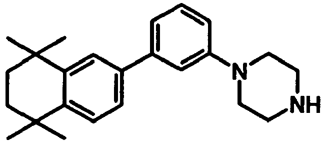

Die obige Verbindung wird analog FS201 ausgehend von 4-[3-(5,5,8,8-Tetramethyl-5,6,7,8-tetrahydro-naphthalen-2-yl)-phenyl]-piperazine-1-carboxylic acid tert-butyl ester hergestellt.

Ausbeute: 423 mg, gelbes Öl. Rt. = 2.81 min (Methode B), LCMS: 349 (M+H). ¹H NMR (400 MHz, DMSO/deuteriertes TFA) δ 7.54 (s, 1H), 7.41 - 7.33 (m, 3H), 7.22 (s, 1H), 7.16 (d, *J* = 7.7, 1H), 7.02 (dd, *J* = 8.2, 1.9, 1H), 3.53 - 3.46 (m, 4H), 3.36 - 3.30 (m, 4H), 1.71 (s, 4H), 1.31 (d, *J* = 15.2, 12H).

### FS212:

### 2-(5,5-Dimethyl-5,6,7,8-tetrahydro-naphthalen-2-yl)-4-pi perazin-1-yl-[1,3,5]triazine

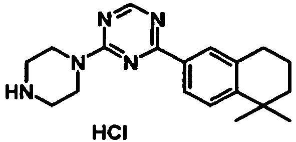

Die obige Verbindung wird analog FS201 ausgehend von 4-[4-(5,5-Dimethyl-5,6,7,8-tetrahydro-naphthalen-2-yl)-[1,3,5]triazin-2-yl]-piperazine-1-carboxylic acid tert-butyl ester hergestellt. Das Produkt liegt als Hydrochlorid vor. Ausbeute: 250 mg, farbloses Öl. Rt. = 2.78 min (Methode A), LCMS: 352 (M+H). ¹H NMR (500 MHz, DMSO/deuteriertes TFA) ö 8.92 (s, 1H), 8.13 (d, *J* = 8.3, 1H), 8.08 (s, 1H), 7.58 (d, *J* = 8.4, 1H), 4.26 (d, *J* = 49.0, 4H), 3.35 (s, 4H), 2.86 (t, *J* = 6.2, 2H), 1.87 - 1.79 (m, 2H), 1.71 (dd, *J* = 7.5, 3.9, 2H), 1.31 (s, 6H).

### FS213:

### 2-(8,8-Dimethyl-5,6,7,8-tetrahydro-naphthalen-2-yl)-4-pi perazi n-1-yl-[1,3,5]triazine

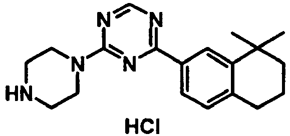

Die obige Verbindung wird analog FS201 ausgehend von 4-[4-(8,8-Dimethyl-5,6,7,8-tetrahydro-naphthalen-2-yl)-[1,3,5]triazin-2-yl]-piperazine-1-carboxylic acid tert-butyl ester ester hergestellt. Das Produkt liegt als Hydrochlorid vor. Ausbeute: 7 mg, beigefarbener Feststoff. Rt. = 2.61 min (Methode A), LCMS: 324 (M+H).

¹H NMR (400 MHz, DMSO/deuteriertes TFA) ö 8.90 (s, 1H), 8.14 (dd, *J*= 8.3, 1.9, 1H), 8.08 (s, 1H), 7.58 (d, *J* = 8.3, 1H), 4.23 (d, *J* = 39.9, 4H), 3.33 (s, 4H), 2.85 (t, *J* = 6.1, 2H), 1.87 - 1.77 (m, 2H), 1.75 - 1.65 (m, 2H), 1.31 (s, 6H).

### FS214:

### 1-{1-[4-(5,5,8,8-Tetramethyl-5,6,7,8-tetrahydro-naphthalen-2-yl)-[1,3,5]triazin-2-yl]-piperidin-4-yl}-pyrrolidin-3-ylamine:

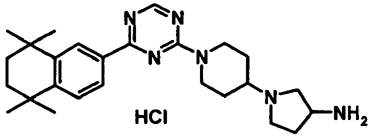

Die obige Verbindung wird analog FS201 ausgehend von (1-{1-[4-(5,5,8,8-Tetramethyl-5,6,7,8-tetrahydro-naphthalen-2-yl)-[1,3,5]triazin-2-yl]-piperidin-4-yl}-pyrrolidin-3-yl)-carbamic acid tert-butyl ester hergestellt. Das Produkt liegt als Hydrochlorid vor.

Ausbeute: 7 mg, beigefarbener Feststoff. Rt. = 2.26 min (Methode B), LCMS: 435 (M+H).

¹H NMR (400 MHz, DMSO/deuteriertes TFA) 8.86 (s, 1 H), 8.30 (d, J = 1.6, 1 H), 8.10 (dd, *J* = 8.4, 1.7, 1H), 7.55 (d, *J* = 8.4, 1H), 5.06 (d, *J* = 12.4, 1H), 4.90 (d, *J* = 13.3, 1H), 3.98 (s, 2H), 3.77 - 3.08 (m, 7H), 2.27 (b, 2H), 2.18 - 1.94 (m, 1H), 1.77 -1.60 (m, 6H), 1.31 (d, *J* = 9.6, 12H).

### FS215:

### 1-[2-(5,5,8,8-Tetramethyl-5,6,7,8-tetrahydro-naphthalen-2-yl)-pyrimidin-4-yl]-piperidin-4-ylamin:

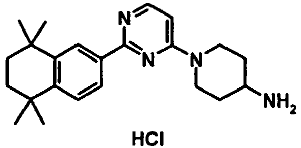

Die obige Verbindung wird analog FS201 ausgehend {1-[2-(5,5,8,8-Tetramethyl-5,6,7,8-tetrahydro-naphthalen-2-yl)-pyrimidin-4-yl]-piperidin-4-yl}-carbaminsäure-tert-butylester hergestellt. Das Produkt liegt als Hydrochlorid vor.

Ausbeute: 391 mg, weißer Feststoff. Rt. = 2.43 min (Methode A), LCMS: 365 (M+H).

¹H NMR (500 MHz, DMSO/deuteriertes TFA) δ 8.38 (d, J = 7.5 Hz, 1H), 8.19 (d, J = 2.0 Hz, 1 H), 8.01 (dd, J = 8.4, 2.0 Hz, 1 H), 7.59 (d, J = 8.4 Hz, 1 H), 7.20 (d, J = 7.6 Hz, 1 H), 5.20 (d, J = 12.6 Hz, 1 H), 4.39 (d, J = 13.1 Hz, 1 H), 3.58 - 3.50 (m, 1 H), 3.46 (t, J = 11.3 Hz, 1 H), 3.31 (t, J = 12.3 Hz, 1 H), 2.20 (b, 2H), 1.74 (s, 4H), 1.60 - 1.75 (m, 2H), 1.34 (d, J = 22.3 Hz, 12H).

### FS216:

### 1-[4-(5,5,8,8-Tetramethyl-5,6,7,8-tetrahydro-naphthalen-2-yl)-pyrimidin-2-yl]-piperidin-4-ylamine:

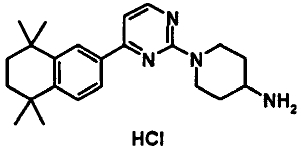

Die obige Verbindung wird analog FS201 {1-[4-(5,5,8,8-Tetramethyl-5,6,7,8-tetrahydro-naphthalen-2-yl)-pyrimidin-2-yl]-piperidin-4-yl}-carbaminsäre-tert-butyl ester hergestellt. Das Produkt liegt als Hydrochlorid vor.

Ausbeute: 437 mg, weißer Feststoff. Rt. = 2.72 min (Methode A), LCMS: 365 (M+H).

¹H NMR (500 MHz, DMSO/deuteriertes TFA) δ 8.24 (d, J = 6.7 Hz, 1 H), 8.05 (d, J = 1.8 Hz, 1 H), 7.86 (dd, J = 8.4, 1.8 Hz, 1 H), 7.40 - 7.45 (m, 2H), 4.60 (b, 2H), 3.42 - 3.32 (m, 1 H), 3.25 (t, J = 11.9 Hz, 2H), 2.09 (d, J = 10.3 Hz, 2H), 1.71 - 1.58 (m, 6H), 1.21 (d, J = 16.6 Hz, 12H).

### FS217: (S)-1-[2-(5,5,8,8-Tetramethyl-5,6,7,8-tetrahydro-naphthalen-2-yl)-pyrimidin-4-yl]-pyrrolidin-3-ylamine:

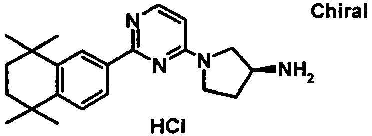

Die obige Verbindung wird analog zu FS203 hergestellt. Produkt ist das Hydrochlorid.

Ausbeute: 333 mg, weißer Feststoff. Rt. = 2.38 min (Methode A), LCMS: 351 (M+H).

¹H NMR (500 MHz, DMSO/deuteriertes TFA) ö 8.42 (t, J = 7.3 Hz, 1 H), 8.24 (dd, J = 3.9, 2.1 Hz, 1 H), 8.06 - 7.98 (m, 1 H), 7.62 (dd, J = 8.4, 4.5 Hz, 1 H), 6.95 (dd, J = 14.0, 7.4 Hz, 1 H), 4.19 - 3.75 (m, 5H), 2.54 - 2.36 (m, 1 H), 2.35 - 2.18 (m, 1 H), 1.73 (s, 4H), 1.42 -1.30 (m, 12H).

### FS218: (R)-1-[4-(5,5,8,8-Tetramethyl-5,6,7,8-tetrahydro-naphthalen-2-yl)-pyrirmidin-2-yl]-pyrrolidin-3-ylamine:

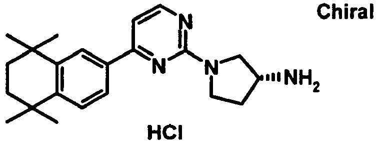

Die obige Verbindung wird analog zu FS203 hergestellt. Produkt ist das Hydrochlorid.

Ausbeute: 207 mg, weißer Feststoff. Rt. = 2.59 min (Methode A), LCMS: 351 (M+H).

¹H NMR (500 MHz, DMSO/deuteriertes TFA) δ 8.51 (d, J = 6.6 Hz, 1 H), 8.22 (t, J = 5.0 Hz, 1 H), 7.99 (d, J = 8.1 Hz, 1 H), 7.67 (d, J = 6.6 Hz, 1 H), 7.53 (d, J = 8.3 Hz, 1 H), 4.17 - 3.58 (m, 5H), 2.49 - 2.09 (m, 2H), 1.68 (s, 4H), 1.29 (d, J = 20.1 Hz, 12H).

### FS219: (R)-1-[2-(5,5,8,8-Tetramethyl-5,6,7,8-tetrahydro-naphthalen-2-yl)-pyrimidin-4-yl]-pyrrolidin-3-ylamine:

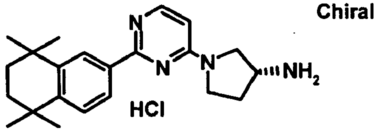

Die obige Verbindung wird analog zu FS203 hergestellt. Produkt liegt als Hydrochlorid vor.

Ausbeute: 347 mg, Feststoff. Rt. = 2.38 min (Methode A), LCMS: 351 (M+H). ¹H NMR (500 MHz, DMSO/deuteriertes TFA) ö 8.37 (t, J = 7.5 Hz, 1H), 8.20 (dd, J = 4.0, 2.1 Hz, 1H), 7.98 (ddd, J = 10.6, 8.4, 2.0 Hz, 1H), 7.57 (dd, J = 8.4, 4.3 Hz, 1 H), 6.90 (dd, J = 15.7, 7.4 Hz, 1H), 4.15 - 3.72 (m, 5H), 2.51 - 2.15 (m, 2H), 1.70 (s, 4H), 1.35 - 1.27 (m, 12H).

### FS220:

### Piperidin-4-yl-[6-(5,5,8,8-tetramethyl-5,6,7,8-tetrahydro-naphthalen-2-yl)-pyridin-2-yl]-amine:

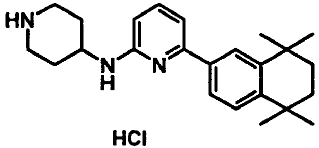

Die obige Verbindung wird analog zu FS203 hergestellt. Produkt liegt als Hydrochlorid vor.

Ausbeute: 360 mg, Feststoff. Rt. = 2.54 min (Methode A), LCMS: 364 (M+H). ¹H NMR (400 MHz, DMSO/deuteriertes TFA): δ 8.02 (t, J = 8.2 Hz, 1H), 7.74 (s, 1 H), 7.58 - 7.49 (m, 2H), 7.21 - 7.10 (m, 2H), 4.12 - 3.99 (m, 1H), 3.40 (d, J = 13.1 Hz, 2H), 3.13 - 2.97 (m, 2H), 2.15 (d, J = 11.0 Hz, 2H), 1.87 - 1.73 (m, 2H), 1.69 (s, 4H), 1.30 (d, J = 15.5 Hz, 12H).

### FS301:

### 3-{4-[6-(5,5,8,8-Tetramethyl-5,6,7,8-tetrahydro-naphthaten-2-yl)-pyridin-2-yl]-piperazin-1-yl}-propan-1-ol

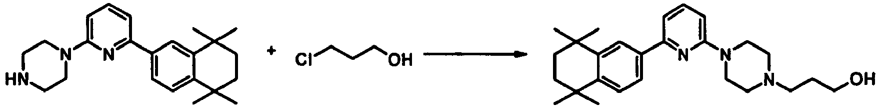

88 mg (0.25 mmol) 1-[6-(5,5,8,8-Tetramethyl-5,6,7,8-tetrahydro-naphthalen-2-yl)-pyridin-2-yl]-piperazine werden mit 42µl (0.50 mmol) 3-Chloro-propan-1-ol in 2 ml Ethanol und 70 µl (0.50 mmol) Triethylamin 2 h bei 140°C in der Mikrowelle bestrahlt. Das Reaktionsgemisch wird eingedampft und mittels päparativer HPLC aufgereinigt.

Ausbeute: 53 mg, beigefarbener Feststoff. Rt. = 2.61 min (Methode B), LCMS: 408 (M+H).

¹H NMR (500 MHz, DMSO/deuteriertes TFA) δ 7.90 (t, *J* = 8.1, 1H), 7.86 (s, 1H), 7.68 (d, *J* = 8.2, 1H), 7.48 (d, *J* = 8.3, 1H), 7.29 (d, *J* = 7.5, 1H), 7.15 (d, *J* = 8.7, 1H), 4.54 (d, *J* = 12.9, 2H), 3.71 (d, *J* = 11.0, 2H), 3.58 (t, *J* = 5.8, 2H), 3.45 (t, *J* = 12.5, 2H), 3.32 - 3.27 (m, 2H), 3.27 - 3.17 (m, 2H), 1.92 (td, *J* = 11.5, 5.8, 2H), 1.72 (s, 4H), 1.32 (d, *J* = 16.6, 12H).

### FS302:

### 3-{4-[6-(5,5-Dimethyl-5,6,7,8-tetrahydro-naphthalen-2-yl)-pyridin-2-yl]-piperazin-1-yl}-propan-1-ol:

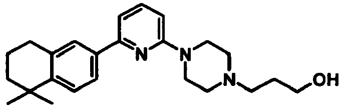

### Die Herstellung erfolgt analog FS301.

Ausbeute: 37 mg, weißer Feststoff. Rt. = 2.63 min (Methode B), LCMS: 380 (M+H).

¹H NMR (500 MHz, DMSO/deuteriertes TFA) δ 7.93 - 7.87 (m, 1H), 7.74 - 7.68 (m, 1H), 7.61 (d, *J* = 9.3, 1H), 7.51 (d, *J* = 8.3, 1H), 7.29 (d, *J* = 7.4, 1H), 7.15 (d, *J* = 8.5, 1H), 4.55 (d, *J* = 14.3, 2H), 3.71 (d, *J* = 10.7, 2H), 3.57 (t, *J* = 5.8, 2H), 3.47 - 3.37 (m, 2H), 3.36 - 3.17 (m, 4H), 2.84 (t, *J* = 6.1, 2H), 1.97 - 1.87 (m, 2H), 1.87 - 1.77 (m, 2H), 1.75 - 1.65 (m, 2H), 1.31 (s, 6H).

### FS303:

### 3-{4-[2-(5,5,8,8-Tetramethyl-5,6,7,8-tetrahydro-naphthalen-2-yl)-pyrimidin-4-yl]-piperazin-1-yl}-propan-1-ol:

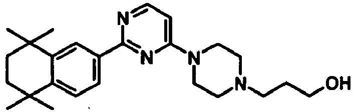

Die Herstellung erfolgt analog FS301.

Ausbeute: 13 mg, gelbes Öl. Rt. = 2.47 min (Methode A), LCMS: 409 (M+H).

### FS304:

### 3-{4-[4-(5,5,8,8-Tetramethyl-5,6,7,8-tetrahydro-naphthalen-2-yl)-pyrimidin-2-yl]-piperazin-1-yl}-propan-1-ol:

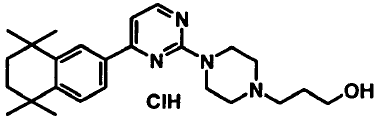

Die obige Verbindung wird analog FS301 hergestellt. Das Produkt liegt als Hydrochlorid vor.

Ausbeute: 47 mg, beigefarbener Feststoff. Rt. = 2.93 min (Methode A), LCMS: 409 (M+H).

### FS305:

### 2-{4-[2-(5,5,8,8-Tetramethyl-5,6,7,8-tetrahydro-naphthalen-2-yl)-pyrimidin-4-yl]-piperazin-1-yl}-ethanol:

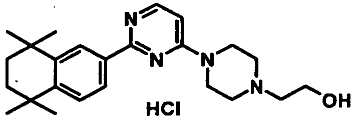

Die obige Verbindung wird analog FS301 hergestellt. Das Produkt liegt als Hydrochlorid vor.

Ausbeute: 36 mg, beigefarbener Feststoff. Rt. = 2.41 min (Methode A), LCMS: 395 (M+H).

### FS306:

### 4-{4-[4-(5,5,8,8-Tetramethyl-5,6,7,8-tetrahydro-naphthalen-2-yl)-pyrimidin-2-yl]-piperazin-1-yl}-butan-1-ol:

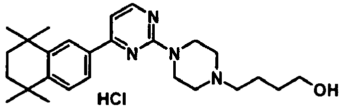

Die Herstellung erfolgt analog FS301. Das Produkt liegt als Hydrochlorid vor. Ausbeute: 42 mg, beigefarbener Feststoff. Rt. = 2.96 min (Methode A), LCMS: 423 (M+H).

¹H NMR (500 MHz, DMSO/deuteriertes TFA) δ 8.49 (d, J = 5.7 Hz, 1H), 8.09 (d, J = 1.8 Hz, 1H), 7.90 (dd, J = 8.3, 1.8 Hz, 1 H), 7.47 (d, J = 8.3 Hz, 1H), 7.41 (d, J = 5.7 Hz, 1 H), 4.85 (d, J = 14.0 Hz, 2H), 3.66 (d, J = 11.7 Hz, 2H), 3.55 - 3.37 (m, 4H), 3.19 (dd, J = 18.2, 10.3 Hz, 4H), 1.78 (dd, J = 15.9, 8.1 Hz, 2H), 1.69 (s, 4H), 1.57 - 1.45 (m, 2H), 1.30 (d, J = 20.5 Hz, 12H).

### FS307:

### 7-{4-[6-(5,5,8,8-Tetramethyl-5,6,7,8-tetrahydro-naphthalen-2-yl)-pyridin-2-yl]-piperazin-1-yl}-heptan-1-ol:

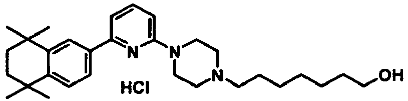

Die Herstellung erfolgt analog FS301. Das Produkt liegt als Hydrochlorid vor. Ausbeute: 25 mg, weißer Feststoff. Rt. = 3.17 min (Methode A), LCMS: 464 (M+H).

¹H NMR (500 MHz, DMSO/deuteriertes TFA) δ 7.96 - 7.86 (m, 1H), 7.64 - 7.56 (m, 1H), 7.47 - 7.35 (m, 2H), 7.20 - 7.07 (m, 2H), 4.38 (d, J = 12.7 Hz, 2H), 3.66 - 3.46 (m, 4H), 3.39 (t, J = 6.6 Hz, 2H), 3.20 - 2.99 (m, 4H), 1.72 -1.58 (m, 7H), 1.44 - 1.36 (m, 2H), 1.34 -1.15 (m, 18H).

### FS308:

### 6-{4-[6-(5,5,8,8-Tetramethyl-5,6,7,8-tetrahydro-naphthalen-2-yl)-pyridin-2-yl]-piperazin-1-yl}-hexan-1-ol:

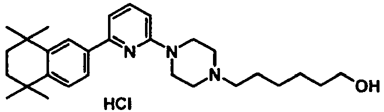

Die Herstellung erfolgt analog FS301 unter Verwendung von (6-Bromo-hexyloxy)-tert-butyl-dimethyl-silan und anschließender TBDMS-Abspaltung analog FS520. Das Produkt liegt als Hydrochlorid vor.

Ausbeute: 59 mg, weißer Feststoff. Rt. = 3.09 min (Methode A), LCMS: 450 (M+H).

¹H NMR (400 MHz, DMSO/deuteriertes TFA) δ 7.92 (d, J = 1.8 Hz, 1H), 7.82 - 7.69 (m, 2H), 7.44 (d, J = 8.3 Hz, 1H), 7.30 (d, J = 7.5 Hz, 1H), 7.00 (d, J = 8.6 Hz, 1H), 4.52 (d, J = 14.1 Hz, 2H), 3.65 (d, J = 11.9 Hz, 2H), 3.43 (t, J = 6.4 Hz, 2H), 3.40 - 3.27 (m, 2H), 3.18 - 3.08 (m, 4H), 1.80 -1.72 (m, 2H), 1.70 (s, 4H), 1.53 -1.42 (m, 2H), 1.42 -1.34 (m, 4H), 1.31 (d, J = 14.6 Hz, 12H).

### FS309:

### 5-{4-[6-(5,5,8,8-Tetramethyl-5,6,7,8-tetrahydro-naphthalen-2-yl)-pyridin-2-yl]-piperazin-1-yl}-pentanoic acid:

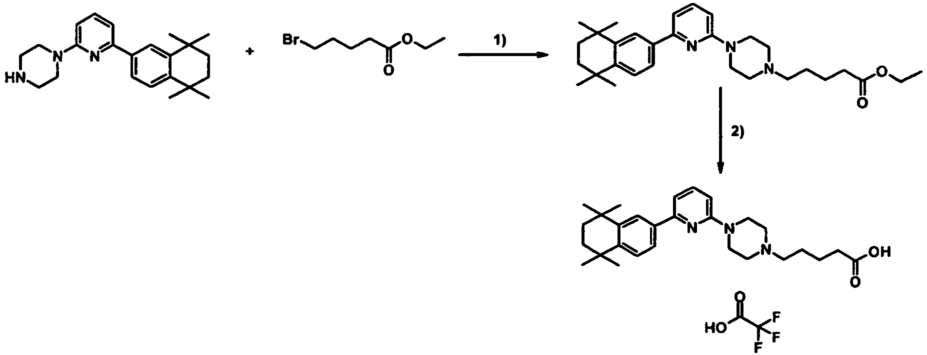

### Stufe 1:

100 mg (0.29 mmol) 1-[6-(5,5,8,8-Tetramethyl-5,6,7,8-tetrahydro-naphthalen-2-yl)-pyridin-2-yl]-piperazin, 104 mg (0.572 mmol) 5-Bromvaleriansäureethylester, 466 mg (1.43 mmol) Cäsiumcarbonat und 43 mg (0.29 mmol) Natriumiodid werden in 2 ml NMP suspendiert und 18 h bei 110°C gerührt. Das Reaktionsgemisch wird mit Wasser versetzt und mehrfach mit Ethylacetat extrahiert, getrocknet und eingedampft. Das Produkt wird mittels Säulenchromatographie an Kieselgel aufgereinigt.

Farbloses Öl. Rt. = 3.25 min (Methode A), LCMS: 478 (M+H).

### Stufe 2:

87 mg (0.175 mmol) des oben hergestellten Esters werden in 5 ml THF und 0.5 ml Wasser suspendiert und mit 21 mg (0.875 mmol) Lithiumhydroxid versetzt und 3 Tage bei Raumtemperatur gerührt. Die Lösung wird neutralisiert, eingedampft und mittels präp. HPLC aufgereinigt. Das Produkt liegt als Trifluoracetat vor. Ausbeute: 35 mg, weißer Feststoff. Rt. = 3.05 min (Methode A), LCMS: 450 (M+H).

¹H NMR (500 MHz, DMSO/deuteriertes TFA) δ 7.89 - 7.81 (m, 2H), 7.66 (d, *J* = 8.2 Hz, 1H), 7.45 (d, *J* = 8.3 Hz, 1H), 7.27 (d, *J* = 7.5 Hz, 1H), 7.10 (d, *J* = 8.6 Hz, 1H), 4.51 (d, *J* = 13.9 Hz, 2H), 3.66 (d, *J* = 11.9 Hz, 2H), 3.38 (t, *J* = 12.7 Hz, 2H), 3.25 - 3.09 (m, 4H), 2.31 (t, *J* = 7.2 Hz, 2H), 1.80 -1.70 (m, 2H), 1.69 (s, 4H), 1.63 - 1.55 (m, 2H), 1.29 (d, *J* = 16.8 Hz, 12H).

### FS310:

### 4-{4-[6-(5,5,8,8-Tetramethyl-5,6,7,8-tetrahydro-naphthalen-2-yl)-pyridin-2-yl]-piperazin-1-yl}-butyric acid:

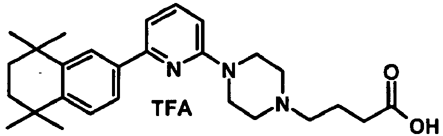

Die Herstellung erfolgt analog FS309. Das Produkt liegt als Trifluoracetat vor. Ausbeute: 28 mg, beigefarbener Feststoff. Rt. = 3.02 min (Methode A), LCMS: 436 (M+H).

¹H NMR (400 MHz, DMSO/deuteriertes TFA) δ 7.89 (d, J = 1.3 Hz, 1H), 7.81 (t, J = 8.0 Hz, 1H), 7.71 (dd, J = 8.2, 1.4 Hz, 1H), 7.44 (d, J = 8.3 Hz, 1H), 7.30 (d, J = 7.5 Hz, 1 H), 7.05 (d, J = 8.5 Hz, 1H), 4.53 (d, J = 14.0 Hz, 2H), 3.69 (d, J = 11.5 Hz, 2H), 3.33 (t, J = 12.5 Hz, 2H), 3.27 - 3.08 (m, 4H), 2.39 (t, J = 7.1 Hz, 2H), 2.05-1.91 (m, 2H), 1.70 (s, 4H), 1.31 (d, J = 14.4 Hz, 12H).

### FS311:

### 2-{4-[6-(5,5,8,8-Tetramethyl-5,6,7,8-tetrahydro-naphthalen-2-yl)-pyridin-2-yl]-piperazin-1-yl}-pentane-1,5-diol:

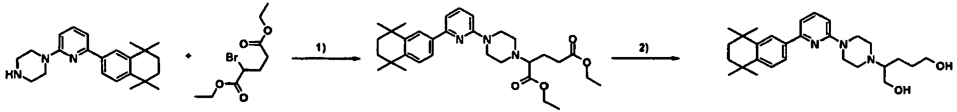

### Stufe 1:

Die Herstellung erfolgt analog FS 301. Anstelle von Ethanol wurde DMF als Lösungsmittel verwendet und das Reaktionsgemisch wurde 18 h bei Raumtemperatur gerührt.

Ausbeute: 576 mg, Feststoff. Rt. = 3.40 min (Methode A), LCMS: 536 (M+H).

### Stufe 2:

225 mg (0.32 mmol) der in Stufe 1 hergestellten Verbindung werden in 10 ml THF gelöst und unter Stickstoffatmosphäre mit 37 mg (0.97 mmol) Lithiumaluminiumhydrid versetzt. Das Reaktionsgemisch wurde 18 h bei Raumtemperatur gerührt, bei 0°C mit 3 ml Wasser versetzt, über Celite abgesaugt und mit Ethylacetat nachgewaschen. Das Filtrat wurde getrocknet und zum Rückstand eingedampft. Das Rohprodukt wurde säulenchromatographisch an Kieselgel aufgereinigt.

Ausbeute: 61 mg, Feststoff. Rt. = 2.95 min (Methode A), LCMS: 452 (M+H).

¹H NMR (400 MHz, DMSO/deuteriertes TFA: δ 7.93 (dd, J = 8.9, 7.5 Hz, 1H), 7.70 (d, J = 1.9 Hz, 1H), 7.53 (dd, J = 8.2, 1.9 Hz, 1H), 7.46 (d, J = 8.3 Hz, 1H), 7.18 (dd, J = 8.2, 2.6 Hz, 2H), 4.51 (b, 2H), 3.87 (dd, J = 13.1, 2.9 Hz, 1H), 3.30 - 3.70 (m, 10H), 1.91 - 1.69 (m, 2H), 1.68 (s, 4H), 1.64 - 1.38 (m, 2H), 1.27 (d, J = 11.2 Hz, 12H).

### FS312:

### 4-{4-[6-(5,5,8,8-Tetramethyl-5,6,7,8-tetrahydro-naphthalen-2-yl)-pyridin-2-yl]-piperazin-1-yl}-butane-1,3-diol:

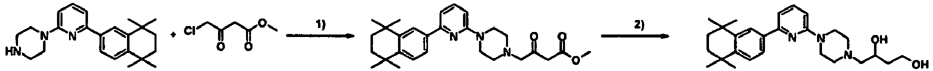

### Stufe 1:

Die Herstellung erfolgt analog FS 301. Anstelle von Ethanol wurde Dichlormethan als Lösungsmittel verwendet und das Reaktionsgemisch wurde 18 h bei Raumtemperatur gerührt.

Ausbeute: 82 mg, Feststoff. Rt. = 3.14 min (Methode A), LCMS: 464 (M+H).

### Stufe 2:

Die Herstellung erfolgt analog FS 311 Stufe 2 unter Verwendung von 1.5 eq. Lithiumaluminiumhydrid.

Ausbeute: 13 mg, Feststoff. Rt. = 2.94 min (Methode A), LCMS: 438 (M+H). ¹H NMR (400 MHz, DMSO/deuteriertes TFA: δ 7.87 (d, J = 1.8 Hz, 1H), 7.86 - 7.79 (m, 1H), 7.70 (dd, J = 8.2, 1.9 Hz, 1H), 7.44 (d, J = 8.3 Hz, 1H), 7.28 (d, J = 7.4 Hz, 1 H), 7.06 (d, J = 8.6 Hz, 1H), 4.47 (dd, J = 28.4, 14.3 Hz, 2H), 4.14 (d, J = 7.5 Hz, 1H), 3.75 - 3.36 (m, 6H), 3.36 - 3.02 (m, 4H), 1.70 (s, 4H), 1.60 (dd, J = 12.4, 6.2 Hz, 2H), 1.30 (d, J = 14.1 Hz, 12H).

### FS313:

### 2-(2-{4-[6-(5,5,8,8-Tetramethyl-5,6,7,8-tetrahydro-naphthalen-2-yl)-pyridin-2-yl]-piperazin-1-yl}-ethyl)-butane-1,4-diol:

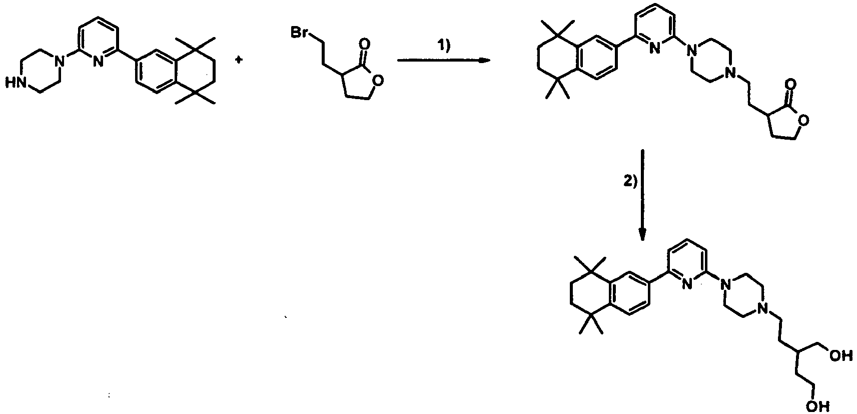

### Stufe 1:

Die Herstellung erfolgt analog FS 301. Anstelle von Ethanol wurde NMP als Lösungsmittel verwendet und das Reaktionsgemisch wurde 12 h bei 70°C gerührt.

Ausbeute: 306 mg, Feststoff. Rt. = 3.07 min (Methode A), LCMS: 462 (M+H).

### Stufe 2:

Die Herstellung erfolgt analog FS 312 Stufe 2.

Ausbeute: 144 mg, farbloses Öl. Rt. = 2.94 min (Methode A), LCMS: 466 (M+H). ¹H NMR (500 MHz, DMSO/deuteriertes TFA: δ 7.86 (t, J = 8.1 Hz, 1 H), 7.74 (s, 1 H), 7.57 (dd, J = 8.2, 1.8 Hz, 1H), 7.42 (d, J = 8.3 Hz, 1H), 7.20 (d, J = 7.4 Hz, 1H), 7.11 (d, J = 8.8 Hz, 1H), 4.45 (d, J = 13.7 Hz, 2H), 3.63 (d, J = 11.2 Hz, 2H), 3.51 - 3.08 (m, 10H), 1.82 - 1.55 (m, 7H), 1.53 - 1.34 (m, 3H), 1.24 (d, J = 15.4 Hz, 12H).

### FS314:

### 4-{(3-Methoxy-propyl)-[6'-(5,5,8,8-tetramethyl-5,6,7,8-tetrahydro-naphthalen-2-yl)-3,4,5,6-tetrahydro-2H-[1,2']bipyridinyl-4-yl]-amino}-butan-1-ol

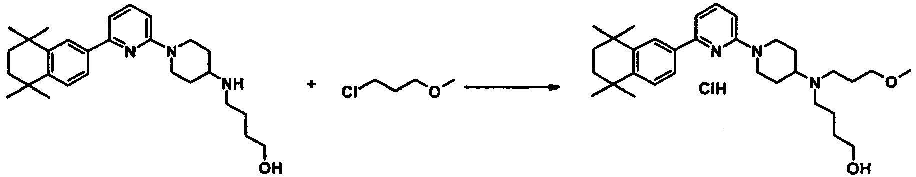

100 mg (0.23 mmol) 4-[6'-(5,5,8,8-Tetramethyl-5,6,7,8-tetrahydro-naphthalen-2-yl)-3,4,5,6-tetrahydro-2H-[1,2']bipyridinyl-4-ylamino]-butan-1-ol wurde mit 25 mg (0.23 mmol) 3-Chlorpropylmethylether in 3 ml n-Butanol gelöst und mit 38 mg (0.23 mmol) kaliumiodid sowie 73 mg (0.69 mmol) Natriumcarbonat versetzt und 48 h bei 120°C gerührt. Das RG wurde mit Wasser versetzt und mit Ethylacetat extrahiert. Die org. Phase wurde getrocknet und zum Rückstand abgezogen. Dieser wurde säulenchromatographisch an Kieselgel aufgereinigt. Das Produkt wurde mit methanolischer HCl ins Hydrochlorid überführt.

Ausbeute: 27 mg. Rt. = 2.85 min (Methode A), LCMS: 508 (M+H).

¹H NMR (500 MHz, DMSO/deuteriertes TFA: δ 7.96 (dd, J = 9.1, 7.4 Hz, 1H), 7.61 (d, J = 1.8 Hz, 1H), 7.46 (dt, J = 8.3, 5.1 Hz, 2H), 7.28 (d, J = 9.1 Hz, 1H), 7.07 (d, J = 7.2 Hz, 1H), 4.41 (d, J = 13.6 Hz, 2H), 3.78 - 3.67 (m, 1H), 3.48 (t, J = 6.0 Hz, 2H), 3.39 (t, J = 5.8 Hz, 2H), 3.34 - 3.16 (m, 7H), 3.09 (dt, J = 15.2, 7.7 Hz, 2H), 2.16 (d, J = 10.7 Hz, 2H), 2.02 - 1.72 (m, 6H), 1.67 (s, 4H), 1.56 - 1.45 (m, 2H), 1.26 (d, J = 9.1 Hz, 12H).

### FS315:

### 4-{(3-Methoxy-propyl)-[6'-(5,5,8,8-tetramethyl-5,6,7,8-tetrahydro-naphthalen-2-yl)-3,4,5,6-tetrahydro-2H-[1,2']bipyridinyl-4-yl]-amino}-butan-1-ol

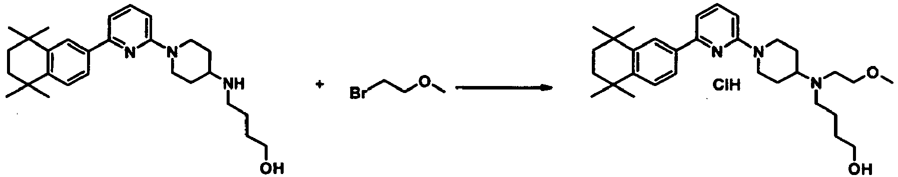

Die Herstellung erfolgt analog FS314. Das Produkt liegt als Hydrochlorid vor. Ausbeute: 33 mg. Rt. = 2.82 min (Methode A), LCMS: 494 (M+H).

¹H NMR (500 MHz, DMSO/deuteriertes TFA: δ 7.96 (dd, J = 9.1, 7.4 Hz, 1H), 7.62 (d, J = 1.7 Hz, 1H), 7.51 - 7.42 (m, 2H), 7.29 (d, J = 9.0 Hz, 1H), 7.07 (d, J = 7.2 Hz, 1H), 4.41 (d, J = 13.5 Hz, 2H), 3.73 (t, J = 12.0 Hz, 1H), 3.64 (d, J = 4.2 Hz, 2H), 3.47 (dd, J = 13.3, 7.3 Hz, 3H), 3.36 - 3.04 (m, 8H), 2.15 (d, J = 13.6 Hz, 2H), 1.96 - 1.72 (m, 4H), 1.68 (s, 4H), 1.55 - 1.40 (m, 2H), 1.27 (d, J = 9.1 Hz, 12H).

### FS316:

### [6'-(5,5,8,8-Tetramethyl-5,6,7,8-tetrahydro-naphthalen-2-yl)-3,4,5,6-tetrahydro-2H-[1,2']bipyridinyl-4-ylamino]-acetic acid:

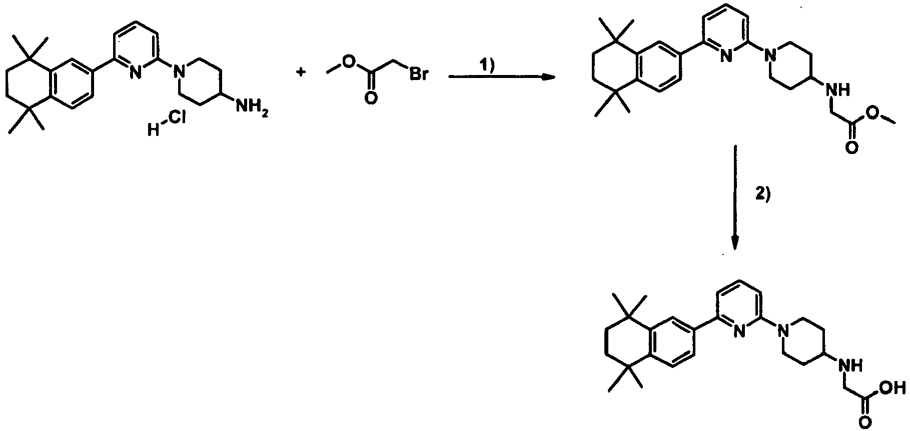

### Stufe 1:

150 mg (0.41 mmol) 6'-(5,5,8,8-Tetramethyl-5,6,7,8-tetrahydro-naphthalen-2-yl)-3,4,5,6-tetrahydro-2H-[1,2']bipyridinyl-4-ylamine hydrochloride wurde mit 75 µl (0.81 mmol) Bromessigsäuremethylester in 3 ml NMP gelöst und mit 661 mg (2.03 mmol) Cäsiumcarbonat sowie 61 mg (0.41 mmol) Natriumiodid versetzt. Das RG rührte 18 h bei 110°C. Es wurde erneut 38 µl (0.41 mmol) Bromessigsäuremethylester zugegeben und weitere 24 h bei 110°C gerührt. Das Reaktionsgemisch wurde mit Wasser versetzt und mit Ethylacetat extrahiert. Die org. Phase wurde getrocknet und zum Rückstand abgezogen. Dieser wurde säulenchromatographisch an Kieselgel aufgereinigt.

### Stufe 2:

Das Produkt aus Stufe 1 wurde in 3 ml THF/Wasser (10:1) gelöst und mit 53 mg LiOH versetzt. Das Reaktionsgemisch wurde 18 h bei Raumtemperatur gerührt. Das THF wurde abdestilliert, mit 1 N HCl leicht angesäuert und mittels präp HPLC aufgereinigt.

Ausbeute: 3 mg. Rt. = 2.81 min (Methode A), LCMS: 470 (M+H).

¹H NMR (400 MHz, DMSO/deuteriertes TFA: δ 7.98 (dd, J = 9.1, 7.4 Hz, 1H), 7.63 (d, J = 1.6 Hz, 1H), 7.51 - 7.43 (m, 2H), 7.27 (d, J = 9.2 Hz, 1H), 7.10 (d, J = 7.4 Hz, 1H), 4.37 - 4.26 (m, 2H), 3.94 (s, 2H), 3.23 (t, J = 12.4 Hz, 2H), 2.26 - 2.13 (m, 4H), 1.73 (dd, J = 12.9, 9.4 Hz, 2H), 1.68 (s, 4H), 1.34 (s, 12H).

### FS317:

### 3-[6'-(5,5,8,8-Tetramethyl-5,6,7,8-tetrahydro-naphthalen-2-yl)-3,4,5,6-tetrahydro-2H-[1,2']bipyridinyl-4-ylamino]-propionic acid:

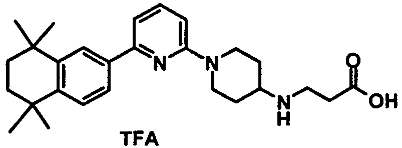

Die Herstellung erfolgt analog FS316. Das Produkt liegt als Trifluoracetat vor. Ausbeute: 28 mg. Rt. = 2.61 min (Methode A), LCMS: 436 (M+H).

¹H NMR (500 MHz, DMSO/deuteriertes TFA): δ 7.98 (dd, J = 9.1, 7.4 Hz, 1H), 7.62 (d, J = 1.7 Hz,1H), 7.53 - 7.42 (m, 2H), 7.27 (d, J = 9.1 Hz, 1H), 7.10 (d, J = 7.3 Hz, 1H), 4.32 (d, J = 13.5 Hz, 2H), 3.62 - 3.52 (m, 2H), 3.44 (t, J = 11.3 Hz, 1H), 3.30 - 3.17 (m, 2H), 2.68 (t, J = 6.7 Hz, 2H), 2.24 - 2.13 (m, 2H), 1.77 - 1.70 (m, 2H), 1.68 (s, 4H), 1.27 (d, J = 9.5 Hz, 12H).

### FS318:

### 4-[6'-(5,5,8,8-Tetramethyl-5,6,7,8-tetrahydro-naphthalen-2-yl)-3,4,5,6-tetrahydro-2H-[1,2']bipyridinyl-4-ylamino]-butyric acid:

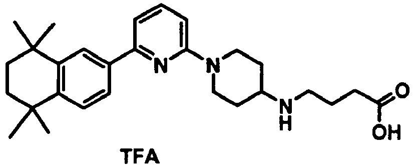

Die Herstellung erfolgt analog FS316. Das Produkt liegt als Trifluoracetat vor. Ausbeute: 37 mg. Rt. = 2.64 min (Methode A), LCMS: 450 (M+H).

¹H NMR (500 MHz, DMSO/deuteriertes TFA): δ 7.99 (dd, J = 9.0, 7.4 Hz, 1H), 7.67 (s, 1H), 7.54 - 7.48 (m, 2H), 7.30 (d, J = 9.1 Hz, 1H), 7.14 (d, J = 7.3 Hz, 1H), 4.35 (d, J = 13.6 Hz, 2H), 3.61 - 3.55 (m, 2H), 3.44 (td, J = 11.3, 5.7 Hz, 1H), 3.26 (t, J = 12.2 Hz, 2H), 3.06 - 2.98 (m, 2H), 2.38 (t, J = 7.1 Hz, 2H), 2.18 (d, J = 12.9 Hz, 2H), 1.93 - 1.83 (m, 2H), 1.77 - 1.63 (m, 4H), 1.29 (d, J = 12.6 Hz, 12H).

### FS319:

### 4-{(4-Hydroxy-butyl)-[6'-(5,5,8,8-tetramethyl-5,6,7,8-tetrahydro-naphthalen-2-yl)-3,4,5,6-tetrahydro-2H-[1,2']bipyridinyl-4-yl]-amino}-butyric acid:

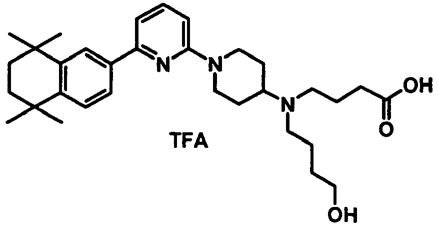

Die Herstellung erfolgt analog FS316. Das Produkt liegt als Trifluoracetat vor. Ausbeute: 106 mg. Rt. = 2.66 min (Methode A), LCMS: 522 (M+H).

¹H NMR (400 MHz, DMSO/deuteriertes TFA): δ 7.99 (dd, J = 9.0, 7.4 Hz, 1H), 7.70 (d, J = 1.6 Hz, 1H), 7.57 - 7.48 (m, 2H), 7.32 (d, J = 9.1 Hz, 1H), 7.14 (d, J = 7.3 Hz, 1H), 4.47 (d, J = 13.2 Hz, 2H), 3.76 (t, J = 11.6 Hz, 1H), 3.60 (ddd, J = 6.6, 4.2, 2.6 Hz, 2H), 3.53 - 3.44 (m, 2H), 3.35 - 3.18 (m, 4H), 3.17 - 3.01 (m, 2H), 2.38 (t, J = 7.0 Hz, 2H), 2.18 (s, 2H), 2.03 - 1.63 (m, 8H), 1.57 - 1.47 (m, 2H), 1.30 (d, J = 10.1 Hz, 12H).

### FS320:

### 5-[6'-(5,5,8,8-Tetramethyl-5,6,7,8-tetrahydro-naphthalen-2-yl)-3,4,5,6-tetrahydro-2H-[1,2']bipyridinyl-4-ylamino]-pentanoic acid:

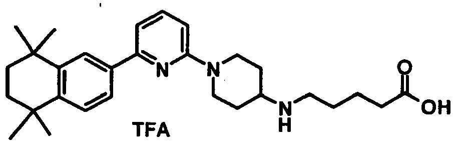

Die Herstellung erfolgt analog FS316. Das Produkt liegt als Trifluoracetat vor. Ausbeute: 26 mg. Rt. = 2.64 min (Methode A), LCMS: 464 (M+H).

¹H NMR (400 MHz, DMSO/deuteriertes TFA): δ 8.00 (dd, J = 9.0, 7.4 Hz, 1H), 7.70 (d, J = 1.7 Hz, 1H), 7.56 - 7.48 (m, 2H), 7.30 (d, J = 9.0 Hz, 1H), 7.15 (d, J = 7.3 Hz, 1H), 4.36 (d, J = 13.7 Hz, 2H), 3.63 - 3.55 (m, 2H), 3.42 (dd, J = 13.6, 9.5 Hz, 1H), 3.25 (t, J = 12.1 Hz, 2H), 3.05 - 2.94 (m, 2H), 2.29 (t, J = 6.9 Hz, 2H), 2.18 (d, J = 8.6 Hz, 2H), 1.78 -1.56 (m, 8H), 1.30 (d, J = 10.3 Hz, 12H).

### FS321:

### 3-{4-[6-(5,5,8,8-Tetramethyl-5,6,7,8-tetrahydro-naphthalen-2-yl)-pyridin-2-yl]-piperazin-1-yl}-propionic acid:

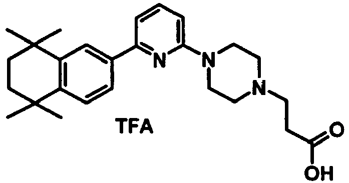

Die Herstellung erfolgt analog FS316. Das Produkt liegt als Trifluoracetat vor. Ausbeute: 78 mg. Rt. = 2.91 min (Methode A), LCMS: 422 (M+H).

¹H NMR (400 MHz, DMSO/deuteriertes TFA): ö 7.87 (dd, J = 8.5, 7.7 Hz, 1H), 7.80 (d, J = 1.9 Hz, 1H), 7.63 (dd, J = 8.2, 1.9 Hz, 1H), 7.45 (t, J = 7.1 Hz, 1H), 7.24 (d, J = 7.4 Hz, 1H), 7.17 - 7.09 (m, 1 H), 4.65 - 4.30 (m, 2H), 3.85 - 3.13 (m, 8H), 2.83 (t, J = 7.2 Hz, 2H), 1.69 (s, 4H), 1.28 (d, J = 16.3 Hz, 12H).

### FS322:

### {4-[6-(5,5,8,8-Tetramethyl-5,6,7,8-tetrahydro-naphthalen-2-yl)-pyridin-2-yl]-piperazin-1-yl}-acetic acid:

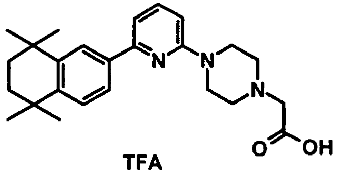

Die Herstellung erfolgt analog FS316. Das Produkt liegt als Trifluoracetat vor. Ausbeute: 37 mg. Rt. = 2.94 min (Methode A), LCMS: 408 (M+H).

¹H NMR (400 MHz, DMSO/deuteriertes TFA): δ 7.83 (dd, J = 11.4, 5.0 Hz, 2H), 7.67 (dd, J = 8.2, 1.9 Hz, 1 H), 7.43 (d, J = 8.3 Hz, 1 H), 7.27 (d, J = 7.4 Hz, 1 H), 7.05 (d, J = 8.7 Hz, 1 H), 4.24 (s, 2H), 3.49 (s, 8H), 1.69 (s, 4H), 1.29 (d, J = 13.8 Hz, 12H).

### FS323:

### 1-(4-Methoxy-butyl)-4-[6-(5,5,8,8-tetramethyl-5,6,7,8-tetrahydro-naphthalen-2-yl)-pyridin-2-yl]-piperazine:

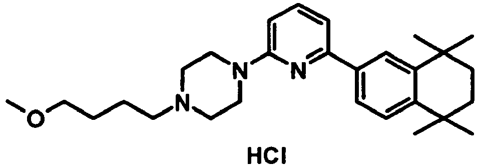

Die Herstellung erfolgt analog FS301. Das Produkt liegt als Hydrochlorid vor. Ausbeute: 50 mg. Rt. = 3.06 min (Methode A), LCMS: 436 (M+H).

¹H NMR (500 MHz, DMSO/deuteriertes TFA): δ 7.91 (dd, J = 8.7, 7.5 Hz, 1H), 7.74 (d, J = 1.8 Hz, 1H), 7.57 (dd, J = 8.2, 1.9 Hz, 1H), 7.45 (d, J = 8.3 Hz, 1H), 7.21 (d, J = 7.4 Hz, 1H), 7.17 (d, J = 8.8 Hz, 1H), 4.47 (d, J = 13.4 Hz, 2H), 3.64 (d, J = 11.6 Hz, 2H), 3.48 (s, 2H), 3.37 - 3.30 (m, 2H), 3.25 - 3.11 (m, 7H), 1.82 -1.72 (m, 2H), 1.68 (s, 4H), 1.60 - 1.52 (m, 2H), 1.27 (d, J = 14.4 Hz, 12H).

### FS324:

### 2-(3-{4-[6-(5,5,8,8-Tetramethyl-5,6,7,8-tetrahydro-naphthalen-2-yl)-pyridin-2-yl]-piperazin-1-yl}-propyl)-propane-1,3-diol

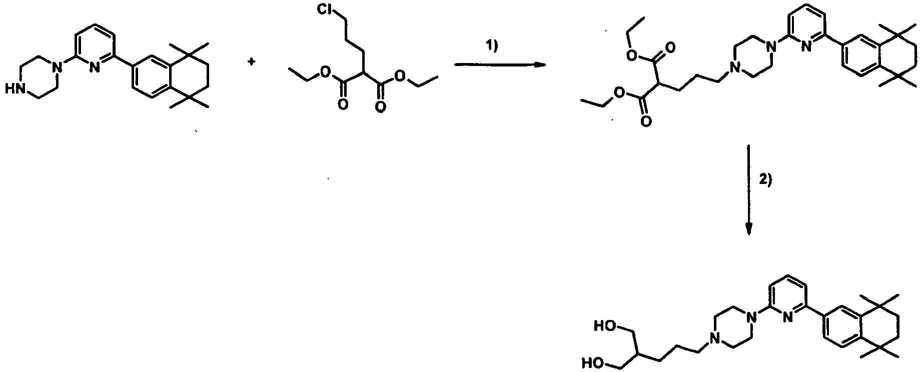

Die Herstellung erfolgt analog FS311.

Ausbeute: 20 mg. Rt. = 2.84 min (Methode A), LCMS: 466 (M+H).

¹H NMR (500 MHz, DMSO/deuteriertes TFA): δ 7.92 - 7.76 (m, 2H), 7.65 (dd, J = 8.2, 1.9 Hz, 1H), 7.45 (d, J = 8.3 Hz, 1H), 7.26 (d, J = 7.4 Hz, 1H), 7.10 (d, J = 8.7 Hz, 1H), 4.46 (dd, J = 48.1, 9.9 Hz, 2H), 3.66 (d, J = 11.7 Hz, 2H), 3.52 - 3.34 (m, 6H), 3.24 - 3.07 (m, 4H), 1.86 -1.73 (m, 2H), 1.69 (s, 4H), 1.60 - 1.50 (m, 1 H), 1.38 - 1.22 (m, 14H).

### FS325:

### 3,3-Dimethyl-5-{4-[6-(5,5,8,8-tetramethyl-5,6,7,8-tetrahydro-naphthalen-2-yl)-pyridin-2-yl]-piperazin-1-yl}-pentane-1,4-diol

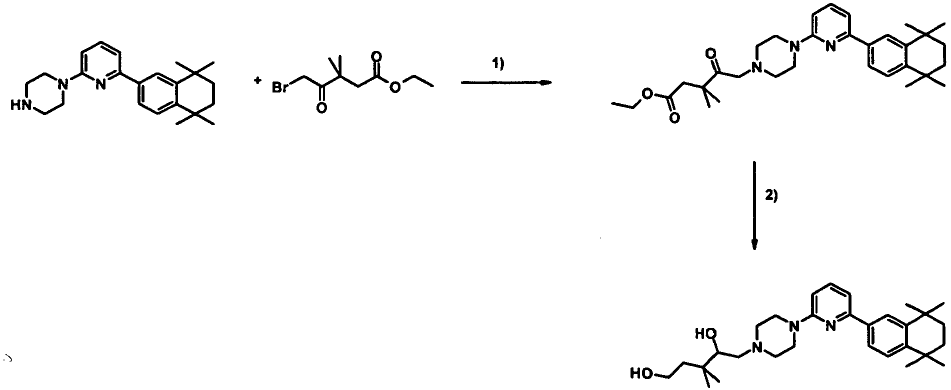

Die Herstellung erfolgt analog FS324.

Ausbeute: 31 mg. Rt. = 2.99 min (Methode A), LCMS: 480 (M+H).

¹H NMR (500 MHz, DMSO/deuteriertes TFA): δ 7.93 - 7.86 (m, 1H), 7.78 (d, J = 1.4 Hz, 1H), 7.61 (dd, J = 8.2, 1.7 Hz, 1H), 7.45 (d, J = 8.3 Hz, 1H), 7.23 (d, J = 7.4 Hz, 1 H), 7.15 (d, J = 8.8 Hz, 1H), 4.44 (dd, J = 35.2, 13.7 Hz, 2H), 3.78 - 3.43 (m, 6H), 3.34 - 3.10 (m, 5H), 1.69 (s, 4H), 1.54 (dt, J = 13.4, 6.5 Hz, 1 H), 1.47 - 1.36 (m, 1H), 1.28 (d, J = 15.6 Hz, 12H), 0.89 (d, J = 6.9 Hz, 6H).

### FS326:

### 2-{4-[4-(5,5,8,8-Tetramethyl-5,6,7,8-tetrahydro-naphthalen-2-yl)-[1,3,5]triazin-2-yl]-piperazin-1-yl}-pentane-1,5-diol

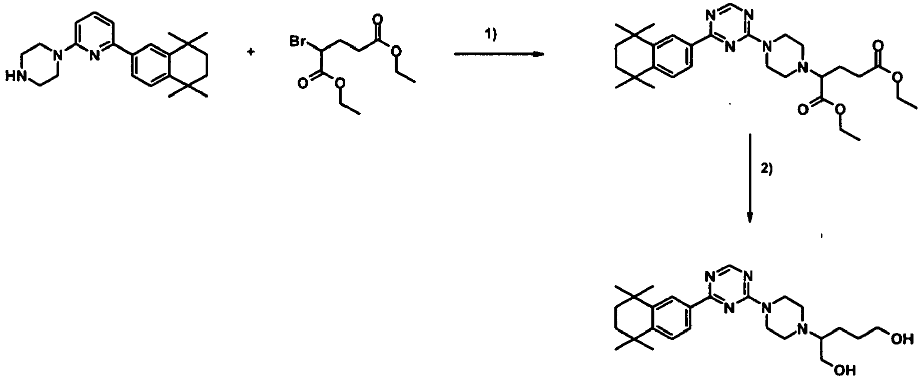

Die Herstellung erfolgt analog FS324.

Ausbeute: 8 mg. Rt. = 2.65 min (Methode A), LCMS: 454 (M+H).

¹H NMR (500 MHz, DMSO/deuteriertes TFA): δ 8.92 (d, J = 1.0 Hz, 1H), 8.32 (d, J = 1.5 Hz, 1H), 8.11 (dd, J = 8.4, 1.7 Hz, 1H), 7.59 - 7.49 (m, 1H), 4.95 (t, J = 64.3 Hz, 2H), 3.88 (dd, J = 13.0, 2.8 Hz, 1H), 3.79 - 3.23 (m, 10H), 1.89 - 1.78 (m, 1H), 1.80 - 1.65 (m, 5H), 1.65 - 1.41 (m, 2H), 1.30 (d, J = 16.3 Hz, 12H).

### FS327:

### 2-[6'-(5,5,8,8-Tetramethyl-5,6,7,8-tetrahydro-naphthalen-2-yl)-3,4,5,6-tetrahydro-2H-[1,2']bipyridinyl-4-ylamino]-pentane-1,5-diol

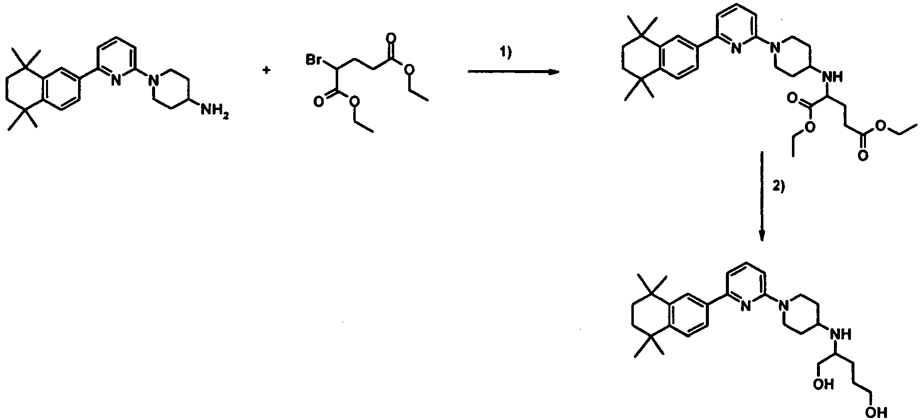

Die Herstellung erfolgt analog FS324.

Ausbeute: 11 mg. Rt. = 2.57 min (Methode A), LCMS: 466 (M+H).

¹H NMR (500 MHz, DMSO/deuteriertes TFA): δ 8.00 (dd, J = 9.0, 7.4 Hz, 1 H), 7.71 (d, J = 1.8 Hz, 1H), 7.54 (dt, J = 18.3, 5.1 Hz, 2H), 7.32 (d, J = 9.1 Hz, 1H), 7.16 (d, J = 7.3 Hz, 1H), 4.38 (d, J = 13.8 Hz, 2H), 3.77 (dd, J = 12.2, 3.1 Hz, 1H), 3.66 - 3.55 (m, 2H), 3.52 - 3.42 (m, 2H), 3.33 - 3.21 (m, 3H), 2.25 - 2.14 (m, 2H), 1.81 - 1.66 (m, 8H), 1.65 - 1.45 (m, 2H), 1.30 (d, J = 13.3 Hz, 12H).

### FS328

### 3-(2-{4-[6-(5,5,8,8-Tetramethyl-5,6,7,8-tetrahydro-naphthalen-2-yl)-pyridin-2-yl]-piperazin-1-yl}-ethyl)-oxazolidin-2-one

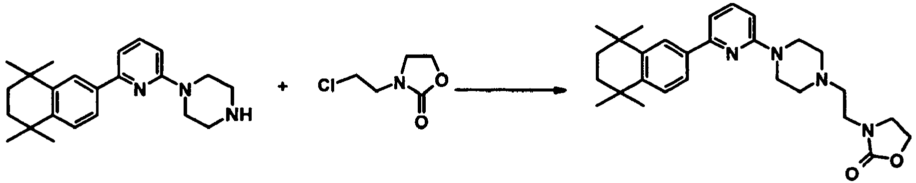

Die Herstellung erfolgt analog FS301.

Ausbeute: 75 mg. Rt. = 2.93 min (Methode A), LCMS: 463 (M+H).

¹H NMR (500 MHz, DMSO/deuteriertes TFA): δ 7.91 (dd, J = 8.7, 7.6 Hz, 1H), 7.74 (d, J = 1.9 Hz, 1H), 7.58 (dd, J = 8.2, 1.9 Hz, 1H), 7.45 (d, J = 8.3 Hz, 1H), 7.22 (d, J = 7.4 Hz, 1H), 7.17 (d, J = 8.8 Hz, 1H), 4.34 - 4.26 (m, 2H), 3.61 (dd, J = 10.8, 4.9 Hz, 4H), 3.43 (t, J = 5.8 Hz, 2H), 4.60 - 3.10 (b, 8H), 1.68 (s, 4H), 1.27 (d, J = 15.6 Hz, 12H).

### FS329

### 2-(2-{4-[6-(5,5,8,8-Tetramethyl-5,6,7,8-tetrahydro-naphthalen-2-yl)-pyridin-2-yl]-piperazin-1-yl}-ethoxy)-ethanol

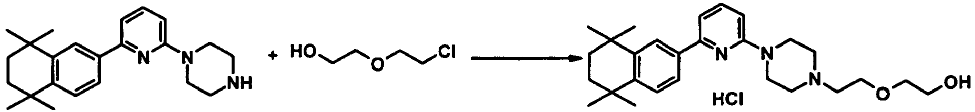

Die Herstellung erfolgt analog FS301.

Ausbeute: 51 mg. Rt. = 2.88 min (Methode A), LCMS: 438 (M+H).

¹H NMR (500 MHz, DMSO/deuteriertes TFA): ö 7.90 (dd, J = 8.6, 7.6 Hz, 1H), 7.78 (d, J = 1.9 Hz, 1H), 7.61 (dd, J = 8.2, 1.9 Hz, 1H), 7.48 - 7.44 (m, 1H), 7.23 (d, J = 7.4 Hz, 1H), 7.15 (d, J = 8.8 Hz, 1H), 4.46 (b, 2H), 3.83 - 3.79 (m, 2H), 3.76 - 3.45 (m, 8H), 3.45 - 3.38 (m, 2H), 3.27 (b, 2H), 1.68 (s, 4H), 1.28 (d, J = 15.9 Hz, 12H).

### FS401:

### Acetic acid 4-{4-[6-(5,5,8,8-tetramethyl-5,6,7,8-tetrahydro-naphthalen-2-yl)-pyridin-2-yl]-piperazin-1-yl}-butyl ester:

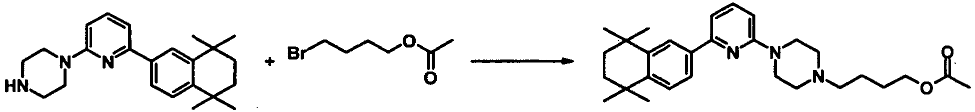

100 mg (0.29 mmol) 1-[6-(5,5,8,8-Tetramethyl-5,6,7,8-tetrahydro-naphthalen-2-yl)-pyridin-2-yl]-piperazine werden in 2 ml DMF gelöst und 47 mg (0.29 mmol) Kaliumcarbonat werden zugegeben. Anschließend werden 42µl (0.29 mmol) 4-Brom-butylacetat zugegeben und das Gemisch wird 24 h bei 50°C gerührt. Anschließend werden weitere 11 µl (0.07 mmol) 4-Brom-butylacetat zugegeben und es wird weitere 48h bei 50°C gerührt. Das Reaktionsgemisch wird eingedampft, der Rückstand mit Wasser versetzt, mit 1 N NaOH basisch gestellt und dreimal mit Ethylacetat extrahiert. Die organische Phase wird über Na₂SO₄ getrocknet und eingeengt. Das Rohprodukt wird mittels Säulenchromatographie an Kieselgel aufgereinigt.

133 mg, Öl. Rt. = 2.88 min (Methode B), LCMS: 464 (M+H).

### FS402:

### 4-{4-[6-(5,5,8,8-Tetramethyl-5,6,7,8-tetrahydro-naphthalen-2-yl)-pyridin-2-yl]-piperazin-1-yl}-butan-1-ol:

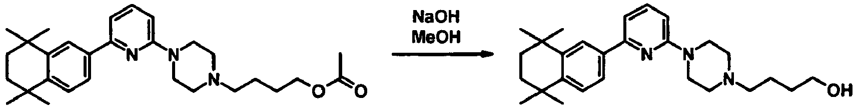

133 mg (0.29 mmol) Acetic acid 3-{4-[6-(5,5,8,8-tetramethyl-5,6,7,8-tetrahydro-naphthalen-2-yl)-pyridin-2-yl]-piperazin-1-yl}-propyl ester werden in 4ml Methanol gelöst und mit 430 µl (0.43 mmol) 1 N NaOH versetzt. Das Reaktionsgemisch wird über Nacht bei RT gerührt. Anschließend wird mit 430 µl 1 N HCl neutralisiert, das Lösungsmittel wird abdestilliert und der Rückstand wird mittels Säulenchromatographie an RP-Kieselgel aufgereinigt. 78 mg, weißer Feststoff. Rt. = 2.69 min (Methode B), LCMS: 422 (M+H).

¹H NMR (500 MHz, DMSO/deuteriertes TFA) δ 7.91 (d, *J* = 1.3, 1H), 7.80 - 7.70 (m, 2H), 7.43 (d, *J* = 8.3, 1H), 7.30 (d, *J* = 7.5, 1H), 6.99 (d, *J* = 8.5, 1H), 4.52 (d, *J* = 14.1, 2H), 3.65 (d, *J* = 11.7, 2H), 3.47 (t, *J* = 6.2, 2H), 3.30 (t, *J* = 12.5, 2H), 3.23 - 3.09 (m, 4H), 1.82 - 1.73 (m, 2H), 1.70 (s, 4H), 1.55 - 1.46 (m, 2H), 1.30 (d, *J* = 18.4, 12H).

### FS403:

### Acetic acid 4-{4-[6-(5,5-dimethyl-5,6,7,8-tetrahydro-naphthalen-2-yl)-pyridin-2-yl]-piperazin-1-yl}-butyl ester:

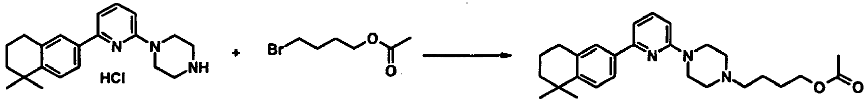

Die Herstellung erfolgt analog FS401 unter Verwendung von 2 equiv. Kaliumcarbonat.

Ausbeute: 89 mg, gelbes Öl. Rt. = 2.83 min (Methode B), LCMS: 436 (M+H).

### FS404:

### 4-{4-[6-(5,5-Dimethyl-5,6,7,8-tetrahydro-naphthalen-2-yl)-pyridin-2-yl]-piperazin-1-yl}-butan-1-ol:

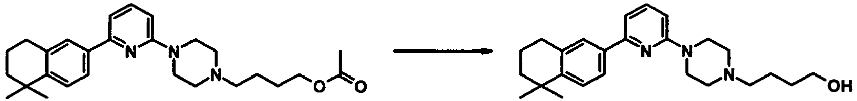

Die Herstellung erfolgt analog FS402.

Ausbeute: 48 mg, weißer Feststoff. Rt. = 2.64 min (Methode B), LCMS: 394 (M+H).

¹H NMR (500 MHz, DMSO/deuteriertes TFA) δ 7.80 (t, *J* = 8.0, 1H), 7.74 (d, *J* = 8.2, 1H), 7.64 (s, 1H), 7.47 (d, *J* = 8.3, 1H), 7.28 (d, *J* = 7.5, 1H), 7.02 (d, *J* = 8.6, 1H), 4.54 (d, *J* = 14.0, 2H), 3.65 (d, *J* = 11.5, 2H), 3.49 (t, *J* = 6.1, 2H), 3.32 (t, *J* = 12.3, 2H), 3.25 - 3.11 (m, 4H), 2.82 (t, *J* = 6.3, 2H), 1.84 - 1.75 (m, 4H), 1.72 - 1.65 (m, 2H), 1.56 - 1.48 (m, 2H), 1.29 (s, 6H).

### FS405: Acetic acid 4-{4-[6-(8,8-dimethyl-5,6,7,8-tetrahydro-naphthalen-2-yl)-pyridin-2-yl]-piperazin-1-yl}-butyl ester:

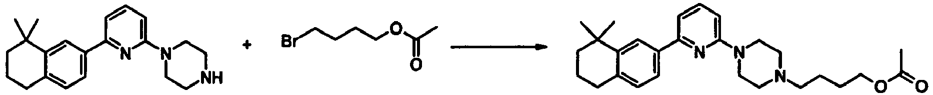

Die Herstellung erfolgt analog FS401.

Ausbeute: 81 mg, farbloses Öl. Rt. = 2.70 min (Methode B), LCMS: 436 (M+H).

### FS406:

### 4-{4-[6-(8,8-Dimethyl-5,6,7,8-tetrahydro-naphthalen-2-yl)-pyridin-2-yl]-piperazin-1-yl}-butan-1-ol:

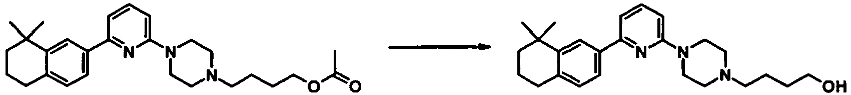

Die Herstellung erfolgt analog FS402.

Ausbeute: 49 mg, zähes Öl. Rt. = 2.63 min (Methode B), LCMS: 394 (M+H).

¹H NMR (500 MHz, DMSO/deuteriertes TFA) δ 7.96 (d, *J* = 1.5, 1H), 7.76 (t, *J* = 8.0, 1H), 7.70 (dd, *J* = 7.9, 1.7, 1H), 7.31 (d, *J* = 7.5, 1H), 7.14 (d, *J* = 8.0, 1H), 6.99 (d, *J* = 8.5, 1H), 4.53 (d, *J* = 13.9, 2H), 3.65 (d, *J* = 11.8, 2H), 3.48 (t, *J* = 6.1, 2H), 3.30 (t, *J* = 12.7, 2H), 3.25 - 3.09 (m, 4H), 2.78 (t, *J* = 6.3, 2H), 1.83 - 1.74 (m, 4H), 1.70 -1.65 (m, 2H), 1.55 - 1.48 (m, 2H), 1.33 (s, 6H).

### FS407:

### Acetic acid 4-{4-[6-(1,1,3,3-tetramethyl-indan-5-yl)-pyridin-2-yl]-piperazin-1-yl}-butyl ester:

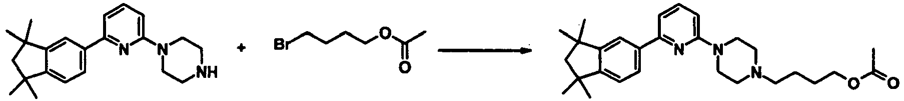

Die Herstellung erfolgt analog FS401.

Ausbeute: 50 mg, farbloses Öl. Rt. = 2.93 min (Methode B), LCMS: 450 (M+H).

### FS408:

### 4-{4-[6-(1,1,3,3-Tetramethyl-indan-5-yl)-pyridin-2-yl]-piperazin-1-yl}-butan-1-ol:

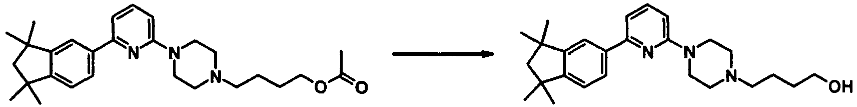

Die Herstellung erfolgt analog FS402.

Ausbeute: 44 mg, zähes Öl. Rt. = 2.71 min (Methode B), LCMS: 408 (M+H).

¹H NMR (400 MHz, DMSO/deuteriertes TFA) δ 7.97 - 7.88 (m, 1H), 7.75 (dd, *J* = 7.9, 1.5, 1H), 7.66 (d, *J* = 1.2, 1H), 7.30 (t, *J* = 7.2, 2H), 7.18 (d, *J* = 8.8, 1H), 4.53 (d, *J* = 13.9, 2H), 3.68 (d, *J* = 11.8, 2H), 3.53 - 3.39 (m, 4H), 3.26 - 3.13 (m, 4H), 1.95 (s, 2H), 1.88 - 1.74 (m, 2H), 1.58 - 1.48 (m, 2H), 1.33 (d, *J* = 10.0, 12H).

### FS409:

### Acetic acid 4-[6'-(5,5,8,8-tetramethyl-5,6,7,8-tetrahydro-naphthalen-2-yl)-3,4,5,6-tetrahydro-2H-[1,2']bipyridinyl-4-ylamino]-butyl ester:

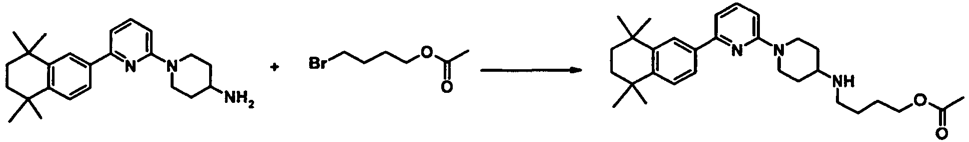

Die Herstellung erfolgt analog FS401.

Ausbeute: 60 mg, gelbes Öl. Rt. = 2.90 min (Methode A), LCMS: 478 (M+H).

### FS410:

### 4-[6'-(5,5,8,8-Tetramethyl-5,6,7,8-tetrahydro-naphthalen-2-yl)-3,4,5,6-tetrahydro-2H-[1,2']bipyridinyl-4-ylamino]-butan-1-ol:

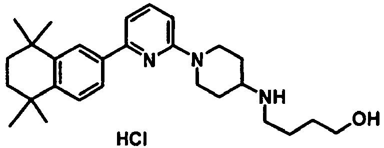

Die Herstellung erfolgt analog FS402. Das Produkt liegt als Hydrochlorid vor. Ausbeute: 25 mg, Beigefarbener Feststoff. Rt. = 2.76 min (Methode B), LCMS: 436 (M+H).

¹H NMR (500 MHz, DMSO/deuteriertes TFA) δ 8.04 (dd, *J* = 8.9, 7.5, 1H), 7.73 (d, *J* = 1.7, 1H), 7.56 (dt, *J* = 14.4, 5.0, 2H), 7.34 (d, *J* = 9.0, 1H), 7.19 (d, *J* = 7.4, 1H), 4.40 (d, *J* = 13.7, 2H), 3.54 - 3.42 (m, 3H), 3.33 - 3.25 (m, 2H), 3.07 - 3.00 (m, 2H), 2.22 (d, *J* = 10.3, 2H), 1.75 (d, *J* = 20.1, 8H), 1.60 -1.53 (m, 2H), 1.33 (d, *J* = 12.9, 12H).

### FS411:

### Acetic acid 4-{4-[4-(5,5,8,8-tetramethyl-5,6,7,8-tetrahydro-naphthalen-2-yl)-[1,3,5]triazin-2-yl]-piperazin-1-yl}-butyl ester:

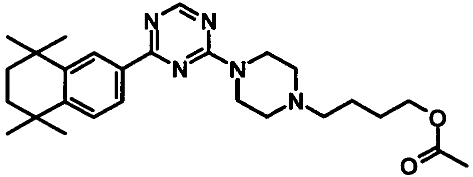

Die Herstellung erfolgt analog FS401.

Ausbeute: 65 mg, Öliger Rückstand. Rt. = 2.97 min (Methode A), LCMS: 466 (M+H).

### FS412:

### 4-{4-(4-(5,5,8,8-Tetramethyl-5,6,7,8-tetrahydro-naphthalen-2-yl)-[1,3,5]triazin-2-yl)-piperazin-1-yl}-butan-1-ol:

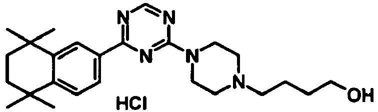

Die Herstellung erfolgt analog FS402. Das Produkt liegt als Hydrochlorid vor. Ausbeute: 30 mg, Beigefarbener Feststoff. Rt. = 2.83 min (Methode A), LCMS: 424 (M+H).

¹H NMR (500 MHz, DMSO/deuteriertes TFA) δ 8.86 (s, 1 H), 8.32 (d, *J* = 1.9, 1H), 8.11 (dd, *J* = 8.3, 1.9, 1H), 7.51 (d, *J* = 8.4, 1H), 4.91 (d, *J* = 67.8, 2H), 3.65 (s, 2H), 3.51 (s, 2H), 3.45 (t, *J* = 6.1, 2H), 3.25 - 3.07 (m, 4H), 1.84 - 1.72 (m, 2H), 1.68 (s, 4H), 1.55-1.44 (m, 2H), 1.28 (d, *J* = 14.4, 12H).

### FS413:

### Acetic acid 4-{4-[2-(5,5,8,8-tetramethyl-5,6,7,8-tetrahydro-naphthalen-2-yl)-pyridin-4-yl]-piperazin-1-yl}-butyl ester:

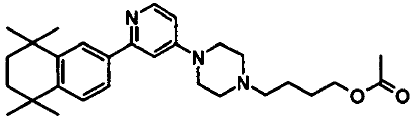

Die Herstellung erfolgt analog FS401.

Ausbeute: 56 mg, Öliger Rückstand. Rt. = 2.31 min (Methode B), LCMS: 464 (M+H).

### FS414:

### 4-{4-[2-(5,5,8,8-Tetramethyl-5,6,7,8-tetrahydro-naphthalen-2-yl)-pyridin-4-yl]-piperazin-1-yl}-butan-1-ol:

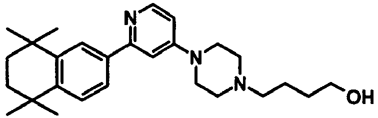

Die Herstellung erfolgt analog FS402.

Ausbeute: 50 mg, farbloses Öl. Rt. = 1.97 min (Methode B), LCMS: 422 (M+H).

¹H NMR (400 MHz, DMSO/deuteriertes TFA) δ 8.32 (d, *J* = 7.3, 1H), 7.74 (d, *J* = 2.0, 1H), 7.62 (dd, *J* = 8.3, 2.0, 1H), 7.54 (d, *J* = 8.3, 1H), 7.44 (d, *J* = 2.6, 1H), 7.25 (dd, *J* = 7.4, 2.7, 1H), 4.64 - 4.47 (m, 2H), 3.72 - 3.48 (m, 4H), 3.45 (t, *J* = 6.1, 2H), 3.23 - 3.16 (m, 4H), 1.81 -1.64 (m, 6H), 1.55-1.42 (m, 2H), 1.26 (t, *J* = 15.2, 12H).

### FS415:

### Acetic acid 4-{4-[4-(5,5,8,8-tetramethyl-5,6,7,8-tetrahydro-naphthalen-2-yl)-pyridin-2-yl]-piperazin-1-yl}-butyl ester:

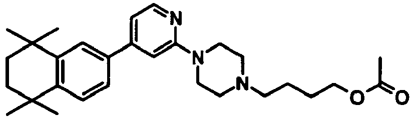

Die Herstellung erfolgt analog FS401.

Ausbeute: 108 mg, Öliger Rückstand. Rt. = 2.53 min (Methode B), LCMS: 464 (M+H).

### FS416:

### 4-{4-[4-(5,5,8,8-Tetramethyl-5,6,7,8-tetrahydro-naphthalen-2-yl)-pyridin-2-yl]-piperazin-1-yl}-butan-1-ol:

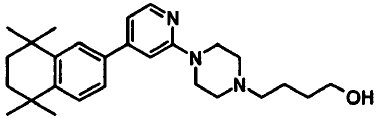

Die Herstellung erfolgt analog FS402.

Ausbeute: 82 mg, weißer Feststoff. Rt. = 2.43 min (Methode B), LCMS: 422 (M+H).

**¹H** NMR (400 MHz, DMSO/deuteriertes TFA) δ 8.20 (d, *J* = 6.6, 1H), 7.78 (d, *J* = 2.0, 1H), 7.68 (dd, *J* = 8.3, 2.0, 1H), 7.60 (s, 1H), 7.54 (d, *J* = 8.3, 1H), 7.45 (dd, J = 6.6, 1.4, 1H), 4.50 (s, 2H), 3.65 (d, *J* = 50.2, 4H), 3.48 (t, *J* = 6.1, 2H), 3.22 (d, *J* = 8.0, 3H), 1.85-1.73 (m, 2H), 1.71 (s, 4H), 1.50 (td, *J* = 13.7, 6.7, 2H), 1.32 (d, *J* = 19.5, 12H).

### FS417:

**Acetic acid 4-{4-[6-(1,1-dimethyl-indan-5-yl)-pyridin-2-yl]-piperazin-1-yl}-butyl ester:**

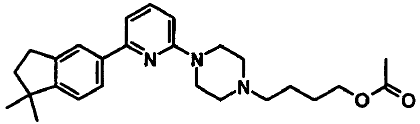

Die Herstellung erfolgt analog FS401.

Ausbeute: 77 mg, gelbes Öl. Rt. = 1.85 min (Methode B), LCMS: 422 (M+H).

### FS418:

### 4-{4-[6-(1,1-Dimethyl-indan-5-yl)-pyridin-2-yl]-piperazin-1-yl}-butan-1-ol:

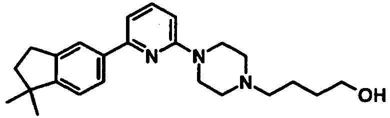

Die Herstellung erfolgt analog FS402.

Ausbeute: 69 mg, farbloses Öl. Rt. = 1.66 min (Methode B), LCMS: 380 (M+H).

¹H NMR (500 MHz, DMSO/deuteriertes TFA) δ 7.85 - 7.74 (m, 3H), 7.28 (dd, *J* = 7.6, 5.0, 2H), 7.00 (d, *J* = 8.5, 1H), 4.54 (d, *J* = 13.9, 2H), 3.65 (d, *J* = 12.5, 2H), 3.48 (t, *J* = 6.1, 2H), 3.30 (t, *J* = 13.0, 2H), 3.24 - 3.09 (m, 4H), 2.94 (t, *J* = 7.2, 2H), 1.94 (dd, *J* = 11.9, 4.6, 2H), 1.84 - 1.72 (m, 2H), 1.56 - 1.47 (m, 2H), 1.27 (s, 7H).

### FS419:

### Acetic acid 4-{4-[3-(5,5,8,8-tetramethyl-5,6,7,8-tetrahydro-naphthalen-2-yl)-phenyl]-piperazin-1-yl}-butyl ester:

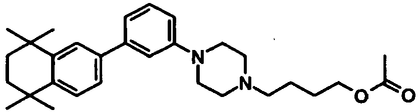

Die Herstellung erfolgt analog FS401.

Ausbeute: 108 mg, farbloses Öl. Rt. = 3.03 min (Methode B), LCMS: 463 (M+H). ¹H NMR (400 MHz, DMSO/deuteriertes TFA) 7.47 (s, 1H), 7.37 - 7.25 (m, 3H), 7.15 (s, 1H), 7.08 (d, *J* = 7.8, 1H), 6.95 (dd, *J* = 8.2, 1.9, 1H), 4.02 (t, *J* = 6.3, 2H), 3.90 (d, *J* = 12.5, 2H), 3.58 (d, *J* = 10.7, 2H), 3.24 - 2.99 (m, 4H), 1.96 (s, 3H), 1.82 - 1.69 (m, 2H), 1.68 - 1.56 (m, 6H), 1.23 (t, *J* = 12.9, 12H).

### FS420:

### 4-{4-[3-(5,5,8,8-Tetramethyl-5,6,7,8-tetrahydro-naphthalen-2-yl)-phenyl]-piperazin-1-yl}-butan-1-ol:

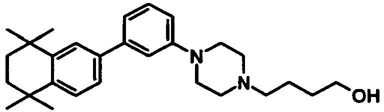

Die Herstellung erfolgt analog FS402.

Ausbeute: 79 mg, farbloses Öl. Rt. = 2.91 min (Methode B), LCMS: 421 (M+H).

¹H NMR (400 MHz, DMSO/deuteriertes TFA) δ 7.48 (s, 1H), 7.33 (s, 2H), 7.29 (t, *J* = 7.9, 1H), 7.15 (b, 1H), 7.08 (d, *J* = 7.7, 1H), 6.95 (dd, *J* = 8.1, 2.0, 1H), 3.91 (d, *J* = 13.1, 2H), 3.58 (d, *J* = 11.7, 2H), 3.45 (t, *J* = 6.1, 2H), 3.20 - 3.12 (m, 4H), 3.07 (t, *J* = 11.9, 2H), 1.74 (dt, *J* = 15.5, 7.7, 2H), 1.64 (s, 4H), 1.52 - 1.43 (m, 2H), 1.25 (d, *J* = 15.2, 12H).

### FS421:

### Acetic acid 4-{4-[4-(5,5,8,8-tetramethyl-5,6,7,8-tetrahydro-naphthalen-2-yl)-[1,3,5]triazin-2-yl]-piperazin-1-yl}-butyl ester:

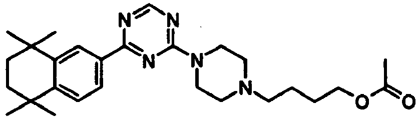

Die Herstellung erfolgt analog FS401.

Ausbeute: 65 mg, Öl. Rt. = 2.97 min (Methode A), LCMS: 466 (M+H).

### FS422:

### 4-{4-[4-(5,5,8,8-Tetramethyl-5,6,7,8-tetrahydro-naphthalen-2-yl)-[1,3,5]triazin-2-yl]-piperazin-1-yl}-butan-1-ol

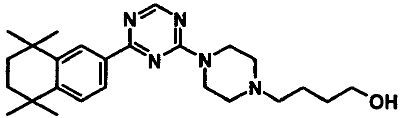

Die Herstellung erfolgt analog FS402. Das Produkt liegt als Hydrochlorid vor. Ausbeute: 30 mg, gelber Feststoff. Rt. = 2.83 min (Methode A), LCMS: 424 (M+H).

¹H NMR (500 MHz, DMSO/deuteriertes TFA) δ 8.86 (s, 1H), 8.32 (d, *J* = 1.9, 1H), 8.11 (dd, *J* = 8.3, 1.9, 1H), 7.51 (d, *J* = 8.4, 1H), 4.91 (d, *J* = 67.8, 2H), 3.58 (d, *J* = 71.3, 4H), 3.45 (t, *J* = 6.1, 2H), 3.22 - 3.09 (m, 4H), 1.82 - 1.71 (m, 2H), 1.68 (s, 4H), 1.52 - 1.45 (m, 2H), 1.28 (d, *J* = 14.4, 12H).

### FS423:

### Acetic acid 4-{1-[4-(5,5,8,8-tetramethyl-5,6,7,8-tetrahydro-naphthalen-2-yl)-[1,3,5]triazin-2-yl]-piperidin-4-ylamino}-butyl ester:

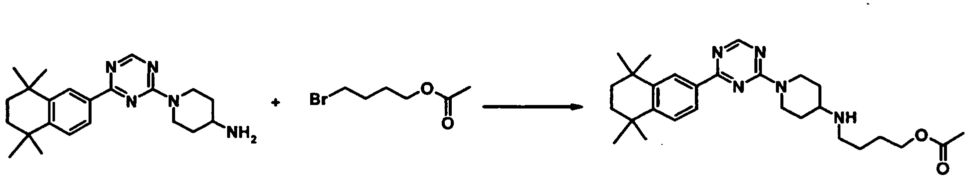

Die Herstellung erfolgt analog FS401.

Ausbeute: 16 mg, farbloses Öl., Rt. = 2.69 min (Methode B), LCMS:480 (M+H).

### FS424:

### 4-{1-[4-(5,5,8,8-Tetramethyl-5,6,7,8-tetrahydro-naphthalen-2-yl)-[1,3,5]triazin-2-yl]-piperidin-4-ylamino}-butan-1-ol:

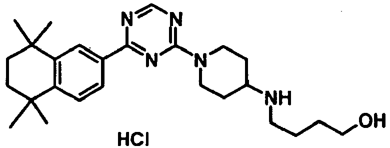

Die Herstellung erfolgt analog FS402. Das Produkt liegt als Hydrochlorid vor. Ausbeute: 22 mg, Beigefarbener Feststoff. Rt. = 2.49 min (Methode B), LCMS: 438 (M+H).

¹H NMR (500 MHz, DMSO/deuteriertes TFA) ö 8.89 (s, 1H), 8.20 (d, *J* = 2.0, 1H), 8.01 (dd, *J* = 8.4, 2.0, 1H), 7.52 (d, *J* = 8.5, 1H), 5.02 (d, *J* = 14.0, 1H), 4.89 (d, *J* = 14.3, 1H), 3.45 (t, *J* = 6.0, 2H), 3.28 (q, *J* = 13.1, 2H), 2.99 - 2.93 (m, 2H), 2.27 - 2.18 (m, 2H), 1.75 - 1.59 (m, 8H), 1.54 - 1.46 (m, 2H), 1.26 (d, *J* = 18.6, 12H).

### FS425:

### Acetic acid 4-{4-[2-(5,5,8,8-tetramethyl-5,6,7,8-tetrahydro-naphthalen-2-yl)-pyrimidin-4-yl]-piperazin-1-yl}-butyl ester:

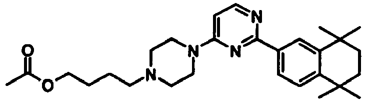

Die Herstellung erfolgt analog FS401. Das Produkt wurde direkt weiter umgesetzt.

Rt. = 2.54 min (Methode A), LCMS: 465 (M+H).

### FS426:

### 4-{4-[2-(5,5,8,8-Tetramethyl-5,6,7,8-tetrahydro-naphthalen-2-yl)-pyrimidin-4-yl]-piperazin-1-yl}-butan-1-ol:

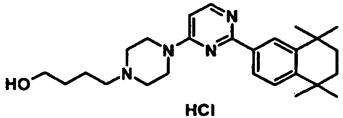

Die Herstellung erfolgt analog FS402. Das Produkt liegt als Hydrochlorid vor. Ausbeute: 34 mg, beigefarbener Feststoff. Rt. = 2.41 min (Methode A), LCMS: 423 (M+H).

¹H NMR (500 MHz, DMSO/deuteriertes TFA) δ 8.50 (d, J = 7.5 Hz, 1H), 8.21 (d, J = 2.0 Hz, 1H), 8.03 (dd, J = 8.4, 2.0 Hz, 1H), 7.60 (d, J = 8.4 Hz, 1H), 7.29 (d, J = 7.5 Hz, 1H), 5.29 (b, 1H), 4.59 (b, 1H), 3.55 - 3.85 (m, 4H), 3.54 (t, J = 6.0 Hz, 2H), 3.35 - 3.18 (m, 4H), 1.93 - 1.80 (m, 2H), 1.75 (s, 4H), 1.61 -1.52 (m, 2H), 1.35 (d, J = 23.2 Hz, 12H).

### FS427:

### Acetic acid 4-{1-[2-(5,5,8,8-tetramethyl-5,6,7,8-tetrahydro-naphthalen-2-yl)-pyrimidin-4-yl]-piperidin-4-ylamino}-butyl ester:

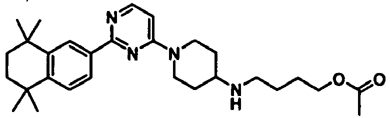

Die Herstellung erfolgt analog FS401.

Ausbeute: 38 mg, Rt. = 2.56 min (Methode A), LCMS: 479 (M+H).

### FS428:

### 4-{1-[2-(5,5,8,8-Tetramethyl-5,6,7,8-tetrahydro-naphthalen-2-yl)-pyrimidin-4-yl]-piperidin-4-ylamino}-butan-1-ol

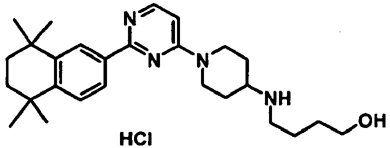

Die Herstellung erfolgt analog FS402. Das Produkt liegt als Hydrochlorid vor. Ausbeute: 12 mg, beigefarbener Feststoff. Rt. = 2.46 min (Methode A), LCMS: 437 (M+H).

¹H NMR (500 MHz, DMSO/deuteriertes TFA) δ 8.27 (d, J = 7.5 Hz, 1H), 8.12 (s, 1 H), 7.93 (d, J = 8.3 Hz, 1H), 7.51 (d, J = 8.4 Hz, 1H), 7.11 (d, J = 7.6 Hz, 1H), 5.21 (d, J = 12.3 Hz, 1H), 4.36 (d, J = 14.3 Hz, 1H), 3.53 - 3.42 (m, 3H), 3.35 (t, J = 13.0 Hz, 1H), 3.18 (t, J = 12.9 Hz, 1H), 3.01 - 2.92 (m, 2H), 2.24 (d, J = 12.6 Hz, 2H), 1.77 - 1.57 (m, 8H), 1.55 - 1.47 (m, 2H), 1.27 (d, J = 21.5 Hz, 12H).

### FS429:

### Acetic acid 4-{1-[2-(5,5,8,8-tetramethyl-5,6,7,8-tetrahydro-naphthalen-2-yl)-pyrimidin-4-yl]-piperidin-4-ylamino}-butyl ester:

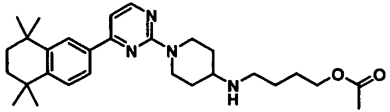

Die Herstellung erfolgt analog FS401.

Ausbeute: 37 mg, Rt. = 2.89 min (Methode A), LCMS: 479 (M+H).

### FS430:

### 4-{1-[2-(5,5,8,8-Tetramethyl-5,6,7,8-tetrahydro-naphthalen-2-yl)-pyrimidin-4-yl]-piperidin-4-ylamino}-butan-1-ol

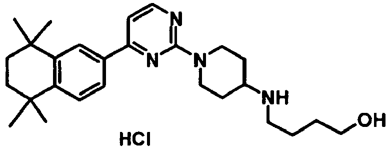

Die Herstellung erfolgt analog FS402. Das Produkt liegt als Hydrochlorid vor. Ausbeute: 8 mg, beigefarbener Feststoff. Rt. = 2.76 min (Methode A), LCMS: 437 (M+H).

¹H NMR (500 MHz, DMSO/deuteriertes TFA) δ 8.41 (d, J = 6.5 Hz, 1H), 8.13 (d, J = 1.9 Hz, 1H), 7.95 (dd, J = 8.4, 1.9 Hz, 1H), 7.55 (d, J = 6.6 Hz, 1H), 7.49 (d, J = 8.4 Hz, 1 H), 4.72 (b, 2H), 3.52 - 3.41 (m, 3H), 3.27 (t, J = 12.1 Hz, 2H), 3.03 - 2.94 (m, 2H), 2.23 (d, J = 10.2 Hz, 2H), 1.76 - 1.64 (m, 8H), 1.56 -1.48 (m, 2H), 1.28 (d, J = 19.3 Hz, 12H).

### FS431:

### Acetic acid 4-{4-[4-(5,5,8,8-tetramethyl-5,6,7,8-tetrahydro-naphthalen-2-yl)-pyrimidin-2-yl]-piperazin-1-yl}-butyl ester:

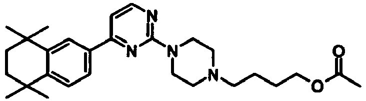

Die Herstellung erfolgt analog FS401. Das Rohprodukt wird direkt in der Actylabspaltung eingesetzt.

Rt. = 3.10 min (Methode A), LCMS: 465 (M+H).

### FS432:

### 4-{4-[4-(5,5,8,8-Tetramethyl-5,6,7,8-tetrahydro-naphthalen-2-yl)-pyrimidin-2-yl]-piperazin-1-yl}-butan-1-ol

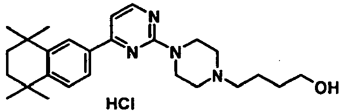

Die Herstellung erfolgt analog FS402. Das Produkt liegt als Hydrochlorid vor. Ausbeute: 42 mg, beigefarbener Feststoff. Rt. = 2.96 min (Methode A), LCMS: 423 (M+H).

**¹H** NMR (500 MHz, DMSO/deuteriertes TFA) δ 8.49 (d, J = 5.7 Hz, 1H), 8.08 (d, J = 1.8 Hz, 1H), 7.90 (dd, J = 8.3, 1.8 Hz, 1H), 7.46 (d, J = 8.3 Hz, 1H), 7.41 (d, J = 5.7 Hz, 1H), 4.85 (d, J = 14.0 Hz, 2H), 3.66 (d, J = 11.7 Hz, 2H), 3.55 - 3.38 (m, 4H), 3.26 - 3.06 (m, 4H), 1.86 - 1.76 (m, 2H), 1.69 (s, 4H), 1.58 - 1.45 (m, 2H), 1.29 (d, J = 20.5 Hz, 12H).

### FS433:

### Acetic acid 4-{4-[4-(5,5,8,8-tetramethyl-5,6,7,8-tetrahydro-naphthalen-2-yl)-pyrimidin-2-yl]-piperazin-1-yl}-butyl ester:

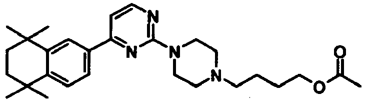

Die Herstellung erfolgt analog FS401. Das Rohprodukt wird direkt in der Actylabspaltung eingesetzt.

Rt. = 3.15 min (Methode A), LCMS: 494 (M+H).

### FS434:

### 4-{4-[5-Methoxy-6-(5,5,8,8-tetramethyl-5,6,7,8-tetrahydro-naphthalen-2-yl)-pyridin-2-yl]-piperazin-1-yl}-butan-1-ol acid

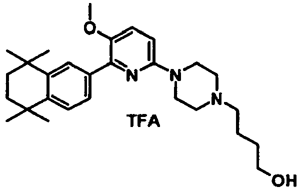

Die Herstellung erfolgt analog FS402. Das Produkt liegt als Trifluoracetat vor. Ausbeute: 14 mg, farbloses Öl. Rt. = 2.98 min (Methode A), LCMS: 452 (M+H). ¹H NMR (500 MHz, DMSO/deuteriertes TFA) δ 7.82 (d, J = 9.4 Hz, 1H), 7.72 (s, 1H), 7.49 (dd, J = 8.2, 1.8 Hz, 1H), 7.40 (d, J = 8.3 Hz, 1H), 7.15 (d, J = 9.4 Hz, 1H), 4.27 (d, J = 13.2 Hz, 2H), 3.80 (s, 3H), 3.67 - 3.56 (m, 2H), 3.48 (t, J = 6.0 Hz, 2H), 3.40 - 3.30 (m, 2H), 3.20 - 3.08 (m, 4H), 1.81 - 1.73 (m, 2H), 1.67 (s, 4H), 1.54 - 1.46 (m, 2H), 1.26 (s, 12H).

### FS501:

### Herstellung von 5-{4-[6-(5,5,8,8-Tetramethyl-5,6,7,8-tetrahydro-naphthalen-2-yl)-pyridin-2-yl]-piperazin-1-yl}-pentan-1-ol:

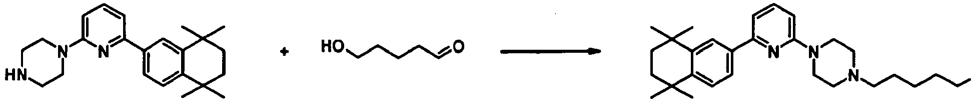

100 mg (0.29 mmol) 1-[6-(5,5,8,8-Tetramethyl-5,6,7,8-tetrahydro-naphthalen-2-yl)-pyridin-2-yl]-piperazine werden mit 5 ml THF und 200 µl Eisessig versetzt und 58 mg (0.57 mmol) 5-Hydroxy-pentanal wurden zugegeben. Das Reaktionsgemisch wird 15 min gerührt. Anschließend werden 128 mg (0.57 mmol) Natriumtrisacetoxyborhydrid zugegeben und das Reaktionsgemisch wird 18 h bei Raumtemperatur gerührt und anschließend filtriert. Die Mutterlauge wird eingedampft und der Rückstand mittels Reversed Phase Chromatographie aufgereinigt.

Ausbeute: 64 mg, weißer Feststoff. Rt. = 2.69 min (Methode B), LCMS: 436 (M+H).

¹H NMR (400 MHz, DMSO/deuteriertes TFA) δ 7.90 - 7.84 (m, 2H), 7.69 (dd, *J* = 8.2, 1.9, 1H), 7.47 (d, *J* = 8.3, 1H), 7.29 (d, *J* = 7.4, 1H), 7.11 (d, *J* = 8.7, 1H), 4.54 (d, *J* = 13.9, 2H), 3.68 (d, *J* = 11.6, 2H), 3.47 (t, *J* = 6.3, 2H), 3.40 (t, *J* = 12.4, 2H), 3.24 - 3.14 (m, 4H), 1.79 - 1.72 (m, 2H), 1.72 (s, 4H), 1.58 - 1.47 (m, 2H), 1.47 - 1.37 (m, 2H), 1.32 (d, *J* = 13.5, 12H).

Die nachfolgenden Verbindungen wurden analog FS501 hergestellt. Wenn das Hydrochlorid als Edukt eingesetzt wurde, wurde das Edukt mit 2 Äuiv. DIPEA in THF suspendiert, 30 min gerührt und anschließend mit Eisessig, Aldehyd und Reduktionsmittel versetzt.

### FS502: 5-{4-[6-(5,5-Dimethyl-5,6,7,8-tetrahydro-naphthalen-2-yl}-pyridin-2-yl]-piperazin-1-yl}-pentan-1-ol:

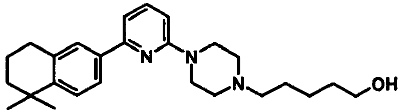

Die Herstellung erfolgt analog F501.

Ausbeute: 92 mg, beigefarbener Feststoff. Rt. = 2.65 min (Methode B), LCMS: 408 (M+H).

¹H NMR (400 MHz, DMSO/deuteriertes TFA) ö 7.97 - 7.90 (m, 1H), 7.68 (d, *J* = 8.3, 1H), 7.59 (d, *J* = 8.3, 1H), 7.53 (d, *J* = 8.3, 1H), 7.29 (d, *J* = 7.4, 1H), 7.19 (d, *J* = 8.8, 1H), 4.55 (d, *J* = 13.7, 2H), 3.69 (d, *J* = 11.3, 2H), 3.49 (t, *J* = 6.2, 3H), 3.38 - 3.31 (m, 1H), 3.29 - 3.15 (m, 4H), 2.85 (t, *J* = 6.3, 2H), 1.87 - 1.67 (m, 6H), 1.59 - 1.49 (m, 2H), 1.43 (dd, *J* = 14.8, 7.9, 2H), 1.31 (s, 6H).

### FS503:

### 5-{4-[6-(8,8-Dimethyl-5,6,7,8-tetrahydro-naphthalen-2-yl)-pyridin-2-yl]-piperazin-1-yl}-pentan-1-ol:

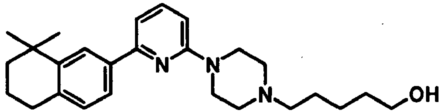

Die Herstellung erfolgt analog F501.

Ausbeute: 99 mg, gelbes Öl., Rt. = 2.59 min (Methode B), LCMS: 408 (M+H).

¹H NMR (500 MHz, DMSO/deuteriertes TFA) δ 7.96 (s, 1H), 7.79 - 7.73 (m, 1H), 7.73 - 7.66 (m, 1H), 7.31 (d, *J* = 7.4, 1H), 7.14 (d, *J* = 8.0, 1H), 6.98 (d, *J* = 8.0, 1H), 4.52 (d, *J* = 14.1, 2H), 3.65 (d, *J* = 12.0, 2H), 3.45 (t, *J* = 6.3, 2H), 3.34 - 3.24 (m, 2H), 3.21 - 3.10 (m, 4H), 2.78 (t, *J* = 6.2, 2H), 1.83 - 1.64 (m, 6H), 1.53 - 1.46 (m, 2H), 1.39 (dd, *J* = 15.1, 7.9, 2H), 1.32 (s, 6H).

### FS504:

### 5-[6'-(5,5,8,8-Tetramethyl-5,6,7,8-tetrahydro-naphthalen-2-yl)-3,4,5,6-tetrahydro-2H-[1,2']bipyridinyl-4-ylamino]-pentan-1-ol:

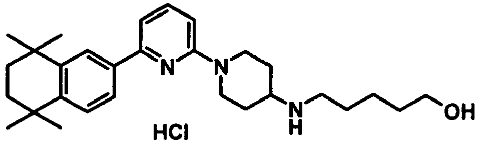

Die Herstellung erfolgt analog F501.

Ausbeute: 24 mg, hellgelber Feststoff. Rt. = 2.77 min (Methode A), LCMS: 450 (M+H).

¹H NMR (500 MHz, DMSO/deuteriertes TFA) δ 8.03 (dd, *J* = 9.0, 7.5, 1H), 7.74 (d, *J* = 1.8, 1H), 7.60 - 7.52 (m, 2H), 7.33 (d, *J* = 9.0, 1H), 7.20 (d, *J* = 7.3, 1H), 4.40 (d, *J* = 13.4, 2H), 3.51 - 3.42 (m, 3H), 3.27 (t, *J* = 12.1, 2H), 3.03 - 2.98 (m, 2H), 2.21 (d, *J* = 10.3, 2H), 1.78 -1.63 (m, 8H), 1.55 - 1.39 (m, 4H), 1.33 (d, *J* = 13.0, 12H).

### FS505:

### 5-{4-[6-(1,1,3,3-Tetramethyl-indan-5-yl)-pyridin-2-yl]-piperazin-1-yl}-pentan-1-ol:

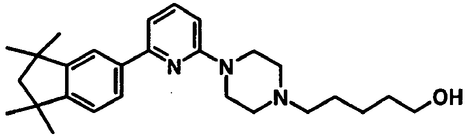

Die Herstellung erfolgt analog F501.

Ausbeute: 98 mg, Öl. Rt. = 2.72 min (Methode B), LCMS: 422 (M+H).

¹H NMR (400 MHz, DMSO/deuteriertes TFA) δ 7.88 - 7.79 (m, 2H), 7.72 (d, *J* = 1.4, 1H), 7.30 (dd, *J* = 10.4, 7.7, 2H), 7.08 (d, *J* = 8.6, 1H), 4.54 (d, *J* = 13.9, 2H), 3.67 (d, *J* = 12.5, 2H), 3.46 (t, *J* = 6.3, 2H), 3.36 (t, *J* = 12.5, 2H), 3.25 - 3.12 (m, 4H), 1.96 (s, 2H), 1.74 (dt, *J* = 15.5, 7.9, 2H), 1.52 (dt, *J* = 13.9, 6.8, 2H), 1.46 -1.28 (m, 14H).

### FS506:

### 5-{4-[6-(1,1-Dimethyl-indan-5-yl)-pyridin-2-yl]-piperazin-1-yl}-pentan-1-ol:

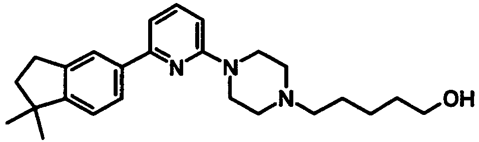

Die Herstellung erfolgt analog F501.

Ausbeute: 105 mg, zähes Öl. Rt. = 1.71 min (Methode C), LCMS: 394 (M+H).

¹H NMR (400 MHz, DMSO/deuteriertes TFA) ö 7.90 (t, *J* = 8.1, 1H), 7.81 - 7.72 (m, 2H), 7.31 (dd, *J* = 10.1, 7.7, 2H), 7.14 (d, *J* = 8.7, 1H), 4.55 (d, *J* = 14.4, 2H), 3.68 (d, *J* = 12.0, 2H), 3.48 (t, *J* = 6.3, 2H), 3.46 - 3.35 (m, 2H), 3.29 - 3.09 (m, 4H), 2.97 (t, *J* = 7.2, 2H), 1.97 (t, *J* = 7.2, 2H), 1.76 (dt, *J* = 15.5, 7.8, 2H), 1.58 - 1.37 (m, 4H), 1.28 (s, 6H).

### FS507:

### (R)-2-Amino-3-{4-[6-(5,5,8,8-tetramethyl-5,6,7,8-tetrahydro-naphthalen-2-yl)-pyridin-2-yl]-piperazin-1-yl}-propan-1-ol:

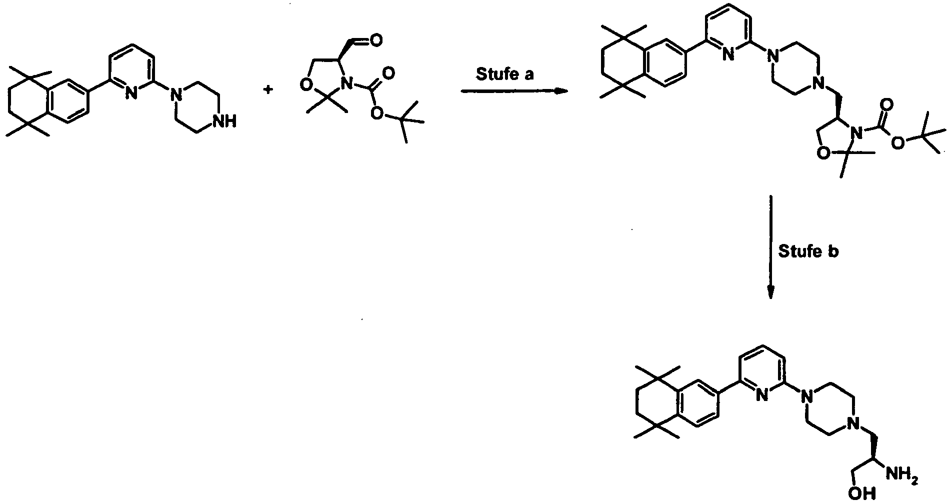

### Stufe a:

Die Umsetzung erfolgt analog F501.

Ausbeute: 220 mg, Öl. Rt. = 3.59 min (Methode A), LCMS: 563 (M+H).

### Stufe b:

Die Abspaltung der Schutzgruppe erfolgt analog FS 201:

Ausbeute: 51 mg, gelber Feststoff. Rt. = 2.78 min (Methode A), LCMS: 423 (M+H).

¹H NMR (500 MHz, DMSO/deuteriertes TFA) δ 7.91 - 7.84 (m, 2H), 7.71 (dd, *J* = 8.3, 1.9, 1H), 7.47 (d, *J* = 8.3, 1H), 7.31 (d, *J* = 7.5, 1H), 7.12 (d, *J* = 8.7, 1H), 4.02 (b, 3H), 3.90 - 3.81 (m, 1H), 3.73 (ddd, *J* = 16.9, 11.6, 4.9, 2H), 3.55 (dd, *J* = 14.1, 4.6, 6H), 3.40 (dd, *J* = 14.2, 6.6, 1H), 1.72 (s, 4H), 1.32 (d, *J* = 17.6, 12H).

### FS508:

### 5-{4-[3-(5,5,8,8-Tetramethyl-5,6,7,8-tetrahydro-naphthalen-2-yl)-phenyl]-piperazin-1-yl}-pentan-1-ol:

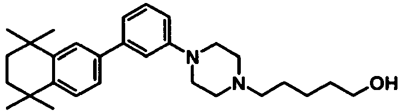

Die Herstellung erfolgt analog F501.

Ausbeute: 165 mg, gelbes Öl. Rt. = 2.88 min (Methode B), LCMS: 435 (M+H).

¹H NMR (400 MHz, DMSO/deuteriertes TFA) δ 7.54 (s, 1H), 7.43 - 7.32 (m, 3H), 7.21 (s, 1H), 7.15 (d, *J* = 7.9, 1H), 7.01 (dd, *J* = 8.1, 1.9, 1H), 3.97 (d, *J* = 12.8, 2H), 3.64 (d, *J* = 11.5, 2H), 3.48 (t, *J* = 6.2, 2H), 3.29 - 3.07 (m, 6H), 1.84 -1.68 (m, 6H), 1.59 -1.38 (m, 4H), 1.31 (d, *J* = 15.1, 12H).

### FS509:

### 5-{4-[3-(2-Methoxy-ethoxy)-6-(5,5,8,8-tetramethyl-5,6,7,8-tetrahydro-naphthalen-2-yl)-pyridin-2-yl]-piperazin-1-yl}-pentan-1-ol:

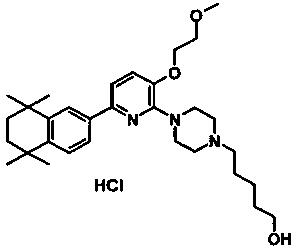

Die Herstellung erfolgt analog F501. Das Produkt liegt als Hydrochlorid vor. Ausbeute: 36 mg, gelbes Öl. Rt. = 3.15 min (Methode A), LCMS: 510 (M+H).

¹H NMR (400 MHz, DMSO/deuteriertes TFA) δ 7.90 (d, *J* = 1.8, 1H), 7.69 (dd, *J* = 8.2, 1.7, 1H), 7.42 (dt, *J* = 14.7, 8.3, 3H), 4.33 (d, *J* = 13.5, 2H), 4.25 - 4.20 (m, 2H), 3.78 - 3.72 (m, 2H), 3.63 (d, *J* = 11.6, 2H), 3.47 (t, *J* = 6.3, 2H), 3.38 (s, 3H), 3.36 - 3.15 (m, 6H), 1.73 (d, *J* = 24.6, 6H), 1.57 - 1.47 (m, 2H), 1.41 (d, *J* = 7.3, 2H), 1.31 (d, *J* = 21.0, 12H).

### FS510:

### 5-{4-[2-(5,5,8,8-Tetramethyl-5,6,7,8-tetrahydro-naphthalen-2-yl)-pyrimidin-4-yl]-piperazin-1-yl}-pentan-1-ol:

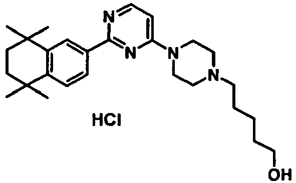

Die Herstellung erfolgt analog FS501. Das Produkt liegt als Hydrochlorid vor. Ausbeute: 77 mg, beigefarbener Feststoff. Rt. = 2.43 min (Methode A), LCMS: 437 (M+H).

¹H NMR (500 MHz, DMSO) δ 11.03 (s, 1H), 8.46 (d, *J* = 7.1 Hz, 1H), 8.29 (d, *J* = 1.8 Hz, 1H), 8.06 (dd, *J* = 8.4, 1.9 Hz, 1H), 7.55 (d, *J* = 8.4 Hz, 1H), 7.17 (d, *J* = 6.1 Hz, 1H), 4.77 (b, 1H), 3.6 - 4.1 (überlagert, 6H), 3.42 (t, *J* = 6.3 Hz, 2H), 3.25 - 3.05 (m, 4H), 1.84 - 1.61 (m, 6H), 1.53 -1.41 (m, 2H), 1.41 - 1.19 (m, 14H).

### FS 511:

### 5-{1-[4-(5,5,8,8-Tetramethyl-5,6,7,8-tetrahydro-naphthalen-2-yl)-pyrimidin-2-yl]-piperidin-4-ylamino}-pentan-1-ol:

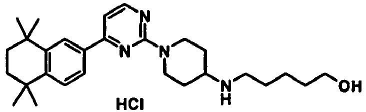

Die Herstellung erfolgt analog FS501 unter Verwendung von 1 Äq. 5-Hydroxypentanal. Das Produkt liegt als Hydrochlorid vor.

Ausbeute: 23 mg, beigefarbener Feststoff. Rt. = 2.80 min (Methode A), LCMS: 451 (M+H).

1H NMR (400 MHz, DMSO/deuteriertes TFA) δ 8.51 (d, *J* = 6.3 Hz, 1H), 8.17 (d, *J* = 1.9 Hz, 1H), 8.00 (dd, *J* = 8.4, 1.9 Hz, 1H), 7.57 (dd, *J* = 20.1, 7.4 Hz, 2H), 4.77 (b, 2H), 3.54 - 3.42 (m, 3H), 3.26 (t, *J* = 12.1 Hz, 2H), 3.04 - 2.95 (m, 2H), 2.24 (d, *J* = 10.3 Hz, 2H), 1.78 - 1.61 (m, 8H), 1.55 - 1.39 (m, 4H), 1.33 (d, *J* = 16.5 Hz, 12H).

### FS512:

### 5-{1-[2-(5,5,8,8-Tetramethyl-5,6,7,8-tetrahydro-naphthalen-2-yl)-pyrimidin-4-yl]-piperidin-4-ylamino}-pentan-1-ol:

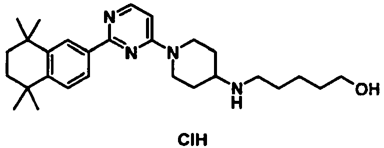

Die Herstellung erfolgt analog FS511. Das Produkt liegt als Hydrochlorid vor. Ausbeute: 30 mg, beigefarbener Feststoff. Rt. = 2.49 min (Methode A), LCMS: 451 (M+H).

1 H NMR (500 MHz, DMSO/deuteriertes TFA) δ 8.39 (d, J = 7.5 Hz, 1H), 8.20 (d, J = 2.0 Hz, 1H), 8.01 (dd, J = 8.4, 2.0 Hz, 1H), 7.59 (d, J = 8.4 Hz, 1H), 7.22 (d, J = 7.6 Hz, 1H), 5.26 (d, J = 13.3 Hz, 1H), 4.45 (d, J = 13.6 Hz, 1H), 3.55 (dd, J = 13.3, 9.2 Hz, 1H), 3.49 (t, J = 6.2 Hz, 2H), 3.42 (t, J = 12.7 Hz, 1H), 3.30 - 3.21 (m, 2H), 3.06 - 2.97 (m, 2H), 2.29 (s, 2H), 1.80 - 1.63 (m, 8H), 1.57 - 1.49 (m, 2H), 1.49 - 1.40 (m, 2H), 1.34 (d, J = 22.5 Hz, 12H).

### FS513:

### 5-{4-[5-(2-Hydroxy-ethoxy)-6-(5,5,8,8-tetramethyl-5,6,7,8-tetrahydro-naphthalen-2-yl)-pyridin-2-yl]-piperazin-1-yl}-pentan-1-ol:

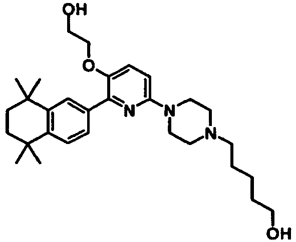

### Die Herstellung erfolgt analog FS501.

Ausbeute: 63 mg, beigefarbener Feststoff. Rt. = 2.75 min (Methode A), LCMS: 496 (M+H).

¹H NMR (500 MHz, DMSO/deuteriertes TFA) δ 8.01 (s, 1H), 7.72 (d, J = 8.3 Hz, 1H), 7.61 (dd, J = 9.1, 2.7 Hz, 1H), 7.37 (d, J = 8.3 Hz, 1H), 6.97 (dd, J = 9.1, 1.7 Hz, 1H), 4.32 (d, J = 13.4 Hz, 2H), 4.04 (t, J = 5.0 Hz, 2H), 3.73 (t, J = 5.0 Hz, 2H), 3.61 (d, J = 11.7 Hz, 2H), 3.43 (t, J = 6.3 Hz, 2H), 3.26 - 3.07 (m, 6H), 1.78 - 1.63 (m, 6H), 1.53 - 1.44 (m, 2H), 1.40 - 1.34 (m, 2H), 1.29 (d, J = 6.5 Hz, 12H).

### FS514:

### 2-{2-[6-[4-(5-Hydroxy-pentyl)-piperazin-1-yl]-2-(5,5,8,8-tetramethyl-5,6,7,8-tetrahydro-naphthalen-2-yl)-pyridin-3-yloxy]-ethyl}-isoindole-1,3-dion:

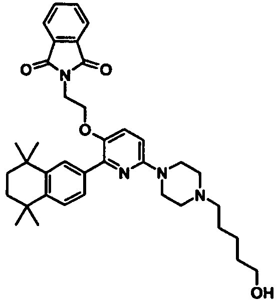

Die Herstellung erfolgt analog FS501.

Ausbeute: 74 mg. Rt. = 3,14 min (Methode A), LCMS: 625 (M+H).

### FS515:

### 5-{4-[5-(5,5,8,8-Tetramethyl-5,6,7,8-tetrahydro-naphthalen-2-yl)-pyridin-3-yl]-piperazin-1-yl}-pentan-1-ol:

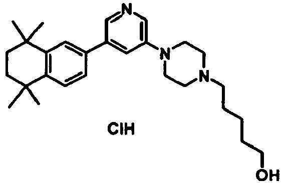

Die Herstellung erfolgt analog FS501. Das Produkt liegt als Hydrochlorid vor. Ausbeute: 29 mg, beigefarbener Feststoff. Rt. = 2.51 min (Methode A), LCMS: 436 (M+H).

¹H NMR (500 MHz, DMSO/deuteriertes TFA) ö 8.58 (d, J = 1.0 Hz, 1H), 8.52 (d, J = 2.4 Hz, 1H), 8.29 (s, 1 H), 7.71 (s, 1H), 7.62 - 7.47 (m, 2H), 4.31 (d, J = 14.0 Hz, 2H), 3.71 (d, J = 12.1 Hz, 2H), 3.55 (t, J = 6.2 Hz, 2H), 3.46 (t, J = 12.3 Hz, 2H), 3.23 (dd, J = 21.1, 12.8 Hz, 4H), 1.81 (dt, J = 16.1, 8.0 Hz, 2H), 1.75 (s, 4H), 1.64 - 1.53 (m, 2H), 1.53 - 1.41 (m, 2H), 1.34 (d, J = 17.0 Hz, 12H).

### FS516:

### 5-{4-[5-Methoxy-6-(5,5,8,8-tetramethyl-5,6,7,8-tetrahydro-naphthalen-2-yl)-pyridin-2-yl]-piperazin-1-yl}-pentan-1-ol:

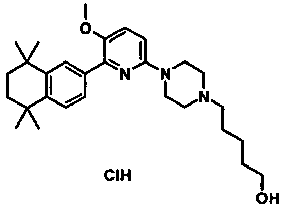

Die Herstellung erfolgt analog FS501. Das Produkt liegt als Hydrochlorid vor. Ausbeute: 70 mg, zähes Öl. Rt. = 3,05 min (Methode A), LCMS: 466 (M+H).

### FS517:

### (S)-3-{1-[4-(5,5,8,8-Tetramethyl-5,6,7,8-tetrahydro-naphthalen-2-yl)-pyrimidin-2-yl]-piperidin-4-ylamino}-propane-1,2-diol:

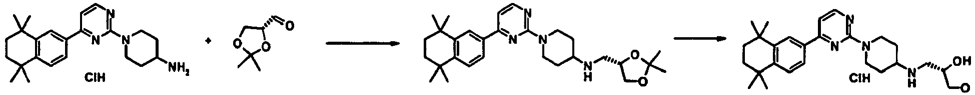

Die Herstellung erfolgt analog FS511. Das Zwischenprodukt wird zur Acetalspaltung in Methanol gelöst und mit methanolischer HCl versetzt und 1 h bei Raumtempereatur gerührt. Das Rohprodukt wurde mittels präparativer HPLC aufgereinigt und mit methanolischer HCl ins Hydrochlorid überführt.

Ausbeute: 16 mg, zähes Öl. Rt. = 2,70 min (Methode A), LCMS: 439 (M+H).

¹H NMR (500 MHz, DMSO/deuteriertes TFA) δ 8.49 (d, 1H), 8.19 (d, J = 1.8 Hz, 1H), 8.01 (dd, J = 8.4, 1.8 Hz, 1H), 7.61 (d, J = 6.5 Hz, 1H), 7.55 (d, J = 8.4 Hz, 1H), 4.78 (b, 2H), 3.92 - 3.83 (m, 1H), 3.59 - 3.49 (m, 2H), 3.46 - 3.39 (m, 1H), 3.28 (t, J = 12.4 Hz, 2H), 3.19 (dd, J = 12.6, 2.8 Hz, 1H), 2.96 (dd, J = 12.6, 9.5 Hz, 1H), 2.35 - 2.23 (m, 2H), 1.85 - 1.69 (m, 6H), 1.34 (d, J = 20.5 Hz, 12H).

### FS518:

### (S)-3-{1-[2-(5,5,8,8-Tetramethyl-5,6,7,8-tetrahydro-naphthalen-2-yl)-pyrimidin-4-yl]-piperidin-4-ylamino}-propane-1,2-diol:

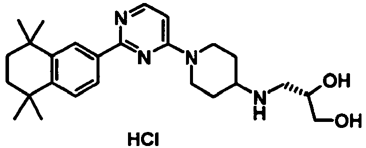

Die Herstellung erfolgt analog FS517. Das Produkt liegt als Hydrochlorid vor. Ausbeute: 22 mg, Feststoff. Rt. = 2,42 min (Methode A), LCMS: 439 (M+H).

¹H NMR (500 MHz, DMSO/deuteriertes TFA) δ 8.36 (d, J = 7.5 Hz, 1H), 8.20 (d, J = 2.0 Hz, 1H), 8.01 (dd, J = 8.4, 2.0 Hz, 1 H), 7.59 (d, J = 8.4 Hz, 1H), 7.19 (d, J = 7.5 Hz, 1H), 5.30 (d, J = 12.8 Hz, 1H), 4.45 (d, J = 12.5 Hz, 1H), 3.96 - 3.85 (m, 1H), 3.64 - 3.50 (m, 2H), 3.50 - 3.33 (m, 2H), 3.27 - 3.16 (m, 2H), 2.98 (dd, J = 12.6, 9.5 Hz, 1H), 2.41 - 2.25 (m, 2H), 1.86 - 1.67 (m, 6H), 1.43-1.28 (m, 12H).

### FS519:

### 5-{4-[4-(5,5,8,8-Tetramethyl-5,6,7,8-tetrahydro-naphthalen-2-yl)-pyrimidin-2-yl]-piperazin-1-yl}-pentan-1-ol:

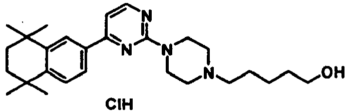

Die Herstellung erfolgt analog FS501. Das Produkt liegt als Hydrochlorid vor. Ausbeute: 58 mg, beigefarbener Feststoff. Rt. = 2.98 min (Methode A), LCMS: 437 (M+H).

¹H NMR (400 MHz, DMSO/deuteriertes TFA) δ 8.47 (d, J = 5.6 Hz, 1H), 8.05 (d, J = 1.9 Hz, 1H), 7.87 (dd, J = 8.3, 1.9 Hz, 1H), 7.44 (d, J = 8.4 Hz, 1H), 7.38 (d, J = 5.6 Hz, 1 H), 4.82 (d, J = 14.6 Hz, 2H), 3.62 (d, J = 11.7 Hz, 2H), 3.48 - 3.34 (m, 4H), 3.14 - 3.03 (m, 4H), 1.68 (d, J = 15.8 Hz, 6H), 1.51 - 1.41 (m, 2H), 1.35 (dd, J = 15.1, 7.9 Hz, 2H), 1.26 (d, J = 16.5 Hz, 12H).

### FS520:

### 3-{1-[2-(5,5,8,8-Tetramethyl-5,6,7,8-tetrahydro-naphthalen-2-yl)-pyrimidin-4-yl]-piperidin-4-ylamino}-propan-1-ol:

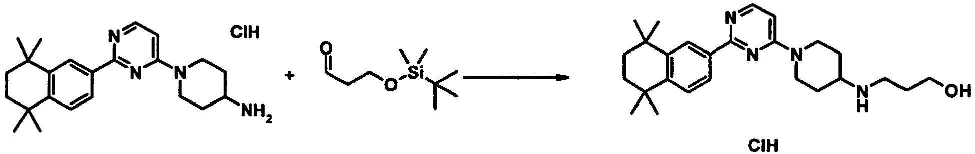

Die Herstellung erfolgt analog FS511. Die Schutzgruppe wurde durch Behandeln mit TMAF in THF bei Raumtemperatur abgespalten. Das Produkt liegt als Hydrochlorid vor.

Ausbeute: 18 mg, beigefarbener Feststoff. Rt. = 2.46 min (Methode A), LCMS: 423 (M+H).

¹H NMR (400 MHz, DMSO/deuteriertes TFA) δ 8.31 (d, J = 7.5 Hz, 1H), 8.14 (d, J = 2.0 Hz, 1H), 7.95 (dd, J = 8.4, 1.9 Hz, 1H), 7.53 (d, J = 8.4 Hz, 1H), 7.14 (d, J = 7.6 Hz, 1H), 5.22 (d, J = 13.3 Hz, 1H), 4.38 (d, J = 13.4 Hz, 1H), 3.58 - 3.46 (m, 3H), 3.36 (t, J = 12.6 Hz, 1H), 3.26 - 3.12 (m, 1 H), 3.07 (t, J = 7.5 Hz, 2H), 2.25 (d, J = 12.0 Hz, 2H), 1.86 - 1.75 (m, 2H), 1.75 - 1.57 (m, 6H), 1.30 (d, J = 16.4, 12H).

### FS521:

### (S)-3-{1-[2-(5,5,8,8-Tetramethyl-5,6,7,8-tetrahydro-naphthalen-2-yl)-pyrimidin-4-yl]-piperidin-4-ylamino}-propane-1,2-diol:

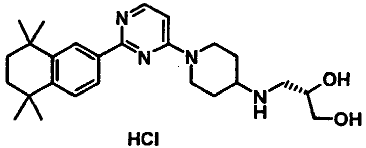

Die Herstellung erfolgt analog FS517. Das Produkt liegt als Hydrochlorid vor. Ausbeute: 17 mg, Feststoff. Rt. = 2,68 min (Methode A), LCMS: 438 (M+H). ¹H NMR (400 MHz, DMSO/deuteriertes TFA) δ 7.98 (dd, J = 9.1, 7.4 Hz, 1H), 7.63 (d, J = 1.3 Hz, 1H), 7.52 - 7.41 (m, 2H), 7.28 (d, J = 9.1 Hz, 1H), 7.10 (d, J = 7.3 Hz, 1H), 4.33 (d, J = 13.7 Hz, 2H), 3.83 (q, J = 9.0 Hz, 1H), 3.53 - 3.33 (m, 3H), 3.24 (t, J = 12.1 Hz, 2H), 3.12 (dd, J = 12.6, 2.9 Hz, 1H), 2.91 (dd, J = 12.6, 9.5 Hz, 1H), 2.21 (t, J = 13.1 Hz, 2H), 1.89 - 1.70 (m, 2H), 1.67 (s, 4H), 1.27 (d, J = 9.9 Hz, 12H).

### FS522:

### 5-{4-[2-Methoxy-5-(5,5,8,8-tetramethyl-5,6,7,8-tetrahydro-naphthalen-2-yl)-phenyl]-piperazin-1-yl}-pentan-1-ol:

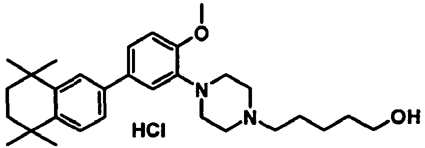

Die Herstellung erfolgt analog FS501. Das Produkt liegt als Hydrochlorid vor. Ausbeute: 13 mg, weißer Feststoff. Rt. = 3.13 min (Methode A), LCMS: 465 (M+H).

¹H NMR (500 MHz, DMSO/deuteriertes TFA) ö 7.47 (d, J = 1.5 Hz, 1H), 7.38 - 7.30 (m, 2H), 7.27 (dd, J = 8.4, 2.0 Hz, 1H), 7.13 (d, J = 1.9 Hz, 1H), 7.05 (d, J = 8.5 Hz, 1H), 3.85 (s, 3H), 3.64 (dd, J = 34.9, 11.7 Hz, 4H), 3.45 (t, J = 6.3 Hz, 2H), 3.31 - 3.21 (m, 2H), 3.21 - 3.14 (m, 2H), 3.14 - 3.03 (m, 2H), 1.77 - 1.69 (m, 2H), 1.68 (s, 4H), 1.54 - 1.46 (m, 2H), 1.44 - 1.34 (m, 2H), 1.28 (d, J = 19.2 Hz, 12H).

### FS523:

### 2-{4-[6-(5,5,8,8-Tetramethyl-5,6,7,8-tetrahydro-naphthalen-2-yl)-pyridin-2-yl]-piperazin-1-ylmethyl}-cyclopropanecarboxylic acid ethyl ester:

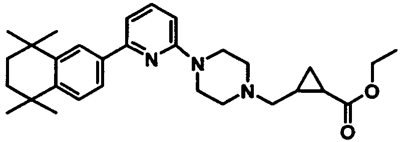

Die Herstellung erfolgt analog FS501.

Ausbeute: 107 mg, weißer Feststoff. Rt. = 3.25 min (Methode A), LCMS: 467 (M+H).

### FS524:

### 2-{2-[6-[4-(5-Hydroxy-pentyl)-piperazin-1-yl]-2-(5,5,8,8-tetramethyl-5,6,7,8-tetrahydro-naphthalen-2-yl)-pyridin-3-yloxy]-ethyl}-isoindole-1,3-dion:

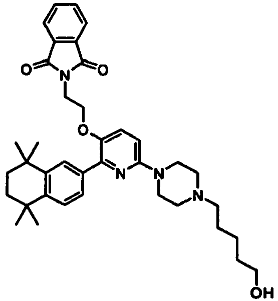

Die Herstellung erfolgt analog FS501.

Ausbeute: 74 mg, beigefarbener Feststoff. Rt. = 3.14 min (Methode A), LCMS: 625 (M+H).

### FS525:

### 2-[6'-(5,5,8,8-Tetramethyl-5,6,7,8-tetrahydro-naphthalen-2-yl)-3,4,5,6-tetrahydro-2H-[1,2']bipyridinyl-4-ylamino]-ethanol

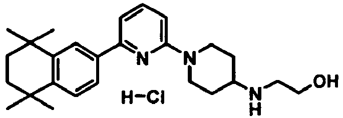

Die Herstellung erfolgt analog FS520. Das Produkt liegt als Hydrochlorid vor. Ausbeute: 13 mg, beigefarbener Feststoff. Rt. = 2.70 min (Methode A), LCMS: 408 (M+H).

¹H NMR (500 MHz, DMSO/deuteriertes TFA) δ 8.00 (dd, J = 8.9, 7.5 Hz, 1H), 7.72 (d, J = 1.8 Hz, 1H), 7.56 (dd, J = 8.2, 1.9 Hz, 1H), 7.51 (d, J = 8.3 Hz, 1H), 7.31 (d, J = 9.0 Hz, 1H), 7.17 (d, J = 7.3 Hz, 1H), 4.38 (d, J = 13.8 Hz, 2H), 3.74 - 3.69 (m, 2H), 3.46 (dd, J = 13.6, 9.2 Hz, 1H), 3.23 (t, J = 12.2 Hz, 2H), 3.12 - 3.06 (m, 2H), 2.21 (d, J = 10.4 Hz, 2H), 1.79 - 1.66 (m, 6H), 1.30 (d, J = 13.4 Hz, 12H).

### FS526:

### 3-[6'-(5,5,8,8-Tetramethyl-5,6,7,8-tetrahydro-naphthalen-2-yl)-3,4,5,6-tetrahydro-2H-[1,2']bipyridinyl-4-ylamino]-propan-1-ol

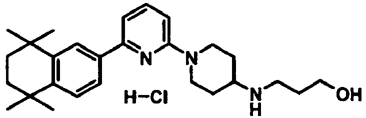

Die Herstellung erfolgt analog FS520. Das Produkt liegt als Hydrochlorid vor. Ausbeute: 6 mg, beigefarbener Feststoff. Rt. = 2.72 min (Methode A), LCMS: 422 (M+H).

¹H NMR (500 MHz, DMSO/deuteriertes TFA) δ 7.84 (s, 2H), 7.67 (d, J = 8.0 Hz, 1H), 7.49 (d, J = 8.3 Hz, 1H), 7.19 (t, J = 14.2 Hz, 2H), 4.45 (d, J = 13.8 Hz, 2H), 3.53 (t, J = 5.9 Hz, 2H), 3.44 (s, 1H), 3.18 - 3.02 (m, 4H), 2.15 (d, J = 11.0 Hz, 2H), 1.85 - 1.76 (m, 2H), 1.72 (s, 4H), 1.65 (d, J = 10.2 Hz, 2H), 1.34 (t, J = 13.7 Hz, 12H).

### FS527:

### (R)-2-Amino-3-[6'-(5,5,8,8-tetramethyl-5,6,7,8-tetrahydro-naphthalen-2-yl)-3,4,5,6-tetrahydro-2H-[1,2']bipyridinyl-4-ylamino]-propan-1-ol:

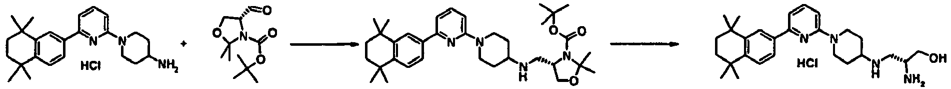

Die Herstellung erfolgt analog FS517. Das Produkt liegt als Hydrochlorid vor. Ausbeute: 9 mg, Feststoff. Rt. = 2,59 min (Methode A), LCMS: 437 (M+H). ¹H NMR (500 MHz, DMSO/deuteriertes TFA) δ 7.99 (dd, J = 9.0, 7.5 Hz, 1H), 7.68 - 7.62 (m, 1H), 7.50 - 7.47 (m, 2H), 7.30 (d, J = 9.1 Hz, 1H), 7.12 (d, J = 7.4 Hz, 1H), 4.35 (d, J = 13.3 Hz, 2H), 3.75 - 3.64 (m, 2H), 3.62 - 3.48 (m, 2H), 3.36 - 3.19 (m, 4H), 2.26 - 2.16 (m, 2H), 1.84 - 1.73 (m, 2H), 1.68 (s, 4H), 1.27 (d, J = 12.5 Hz, 12H).

### FS528:

### (S)-2-Amino-3-[6'-(5,5,8,8-tetramethyl-5,6,7,8-tetrahydro-naphthalen-2-yl)-3,4,5,6-tetrahydro-2H-[1,2']bipyridinyl-4-ylamino]-propan-1-ol:

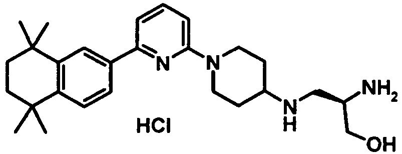

Die Herstellung erfolgt analog FS527. Das Produkt liegt als Hydrochlorid vor. Ausbeute: 8 mg, Feststoff. Rt. = 2,59 min (Methode A), LCMS: 437 (M+H).

¹H NMR (500 MHz, DMSO/deuteriertes TFA) δ 7.99 (dd, J = 9.0, 7.5 Hz, 1H), 7.64 (d, J = 1.1 Hz, 1H), 7.52 - 7.45 (m, 2H), 7.29 (d, J = 9.1 Hz, 1H), 7.11 (d, J = 7.4 Hz, 1H), 4.35 (d, J = 13.5 Hz, 2H), 3.74 - 3.61 (m, 2H), 3.61 - 3.47 (m, 2H), 3.36 - 3.19 (m, 4H), 2.26 - 2.17 (m, 2H), 1.86 - 1.73 (m, 2H), 1.68 (s, 4H), 1.27 (d, J = 12.5 Hz, 12H).

### FS529:

### (S)-2-Amino-3-{4-[6-(5,5,8,8-tetramethyl-5,6,7,8-tetrahydro-naphthalen-2-yl)-pyridin-2-yl]-piperazin-1-yl}-propan-1-ol

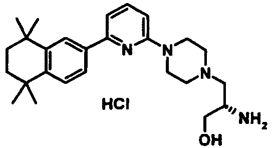

Die Herstellung erfolgt analog FS527. Das Produkt liegt als Hydrochlorid vor. Ausbeute: 86 mg, Feststoff. Rt. = 2,75 min (Methode A), LCMS: 423 (M+H).

¹H NMR (500 MHz, DMSO/deuteriertes TFA) δ 7.88 - 7.80 (m, 2H), 7.68 (dd, J = 8.3, 1.9 Hz, 1H), 7.44 (d, J = 8.3 Hz, 1H), 7.28 (d, J = 7.5 Hz, 1H), 7.09 (d, J = 8.6 Hz, 1H), 4.22 - 3.81 (m, 4H), 3.69 (dd, J = 8.0, 3.0 Hz, 2H), 3.66 - 3.46 (m, 6H), 3.44 - 3.33 (m, 1H), 1.69 (s, 4H), 1.29 (d, J = 14.4 Hz, 12H).

### FS530:

### (R)-2-Amino-3-{4-[6-(5,5,8,8-tetramethyl-5,6,7,8-tetrahydro-naphthalen-2-yl)-pyridin-2-yl]-piperazin-1-yl}-propan-1-ol

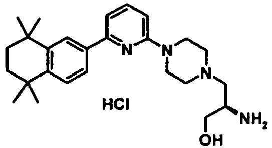

Die Herstellung erfolgt analog FS527. Das Produkt liegt als Hydrochlorid vor. Ausbeute: 52 mg, Feststoff. Rt. = 2,77 min (Methode A), LCMS: 423 (M+H).

¹H NMR (500 MHz, DMSO/deuteriertes TFA) δ 7.89 (s, 1H), 7.83 (t, J = 8.0 Hz, 1 H), 7.71 (d, J = 8.2 Hz, 1 H), 7.44 (d, J = 8.3 Hz, 1 H), 7.30 (d, J = 7.5 Hz, 1 H), 7.09 (d, J = 8.6 Hz, 1H), 3.90 (dd, J = 26.4, 19.6 Hz, 2H), 3.75 - 3.42 (m, 10H), 3.39 (dd, J = 14.2, 7.2 Hz, 1H), 1.69 (s, 4H), 1.30 (d, J = 18.6 Hz, 12H).

### FS531:

### (S)-3-{4-[6-(5,5,8,8-Tetramethyl-5,6,7,8-tetrahydro-naphthalen-2-yl)-pyridin-2-yl]-piperazin-1-yl}-propane-1,2-diol

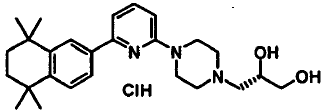

Die Herstellung erfolgt analog FS517. Das Produkt liegt als Hydrochlorid vor. Ausbeute: 37 mg, weißer Feststoff. Rt. = 2,89 min (Methode A), LCMS: 424 (M+H).

¹H NMR (500 MHz, DMSO/deuteriertes TFA) δ 7.88 (dd, J = 8.6, 7.6 Hz, 1H), 7.81 (d, J = 1.9 Hz, 1 H), 7.64 (dd, J = 8.2, 1.9 Hz, 1 H), 7.45 (d, J = 8.3 Hz, 1 H), 7.25 (d, J = 7.4 Hz, 1 H), 7.13 (d, J = 8.7 Hz, 1 H), 4.55 - 4.36 (m, 2H), 4.06 - 3.96 (m, 1 H), 3.78 - 3.10 (m, 10H), 1.69 (s, 4H), 1.29 (d, J = 13.4 Hz, 12H).

### FS532:

### 2-{4-[6'-(5,5,8,8-Tetramethyl-5,6,7,8-tetrahydro-naphthalen-2-yl)-3,4,5,6-tetrahydro-2H-[1,2']bipyridinyl-4-yl]-piperazin-1-yl}-ethanol

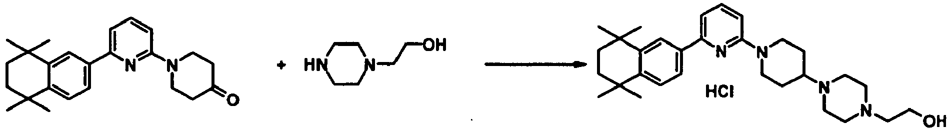

Die Herstellung erfolgt analog FS502. Das Produkt liegt als Hydrochlorid vor. Ausbeute: 15 mg, weißer Feststoff. Rt. = 2,65 min (Methode A), LCMS: 477 (M+H).

¹H NMR (500 MHz, DMSO/deuteriertes TFA) δ 7.90 (dd, J = 9.1, 7.4 Hz, 1H), 7.57 (s, 1 H), 7.41 (s, 2H), 7.22 (d, J = 9.1 Hz, 1 H), 7.04 (d, J = 7.0 Hz, 1 H), 4.37 (d, J = 13.0 Hz, 2H), 3.80 - 3.35 (m, 11 H), 3.30 - 3.22 (m, 2H), 3.15 (t, J = 13.3 Hz, 2H), 2.22 - 2.13 (m, 2H), 1.83 -1.69 (m, 2H), 1.60 (s, 4H), 1.19 (d, J = 9.7 Hz, 12H).

### FS533:

### 5-{4-[5-Methoxy-6-(5,5,8,8-tetramethyl-5,6,7,8-tetrahydro-naphthalen-2-yl)-pyridin-2-yl]-piperazin-1-yl}-pentan-1-ol:

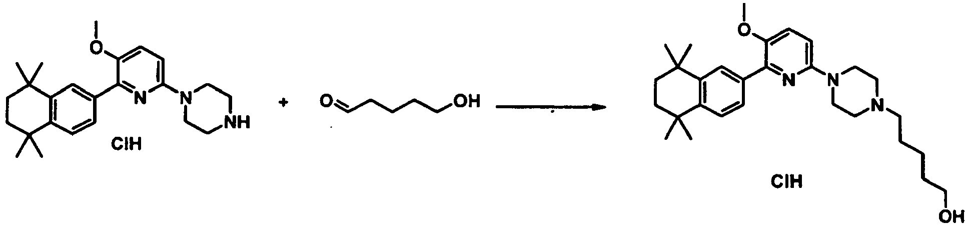

Die Herstellung erfolgt analog FS501. Das Produkt liegt als Hydrochlorid vor. Ausbeute: 70 mg. Rt. = 3.05 min (Methode A), LCMS: 466 (M+H).

¹H NMR (500 MHz, DMSO) δ 7.85 (dd, J = 4.9, 1.8 Hz, 1 H), 7.65 - 7.60 (m, 1 H), 7.52 (dt, J = 10.6, 5.3 Hz, 1 H), 7.33 (t, J = 7.8 Hz, 1 H), 6.96 - 6.89 (m, 1 H), 4.28 (d, J = 13.9 Hz, 1 H), 3.80 - 3.73 (m, 5H), 3.69 - 3.35 (m, 6H), 3.27 - 2.96 (m, 4H), 1.86 - 1.54 (m, 8H), 1.50 - 1.31 (m, 2H), 1.31 -1.22 (m, 12H).

### FS534:

### (2-{[6'-(5,5,8,8-Tetramethyl-5,6,7,8-tetrahydro-naphthalen-2-yl)-3,4,5,6-tetrahydro-2H-[1,2']bipyridinyl-4-ylamino]-methyl}-cyclopropyl)-methanol:

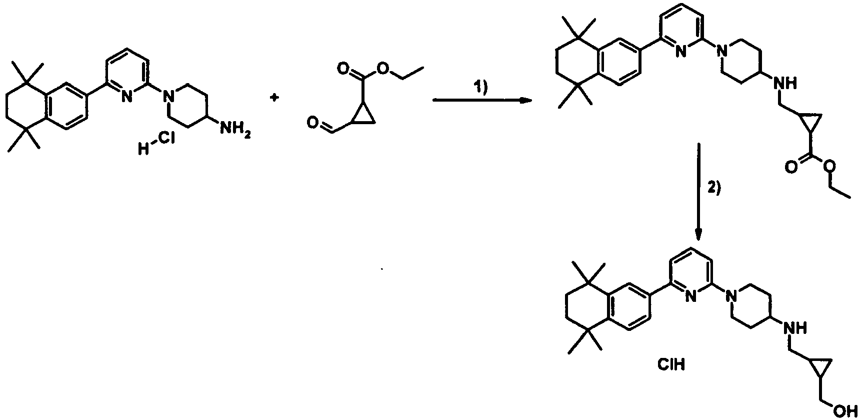

### Stufe 1:

Die Herstellung erfolgt analog FS501.

Ausbeute: 151 mg. Rt. = 2.95 min (Methode A), LCMS: 490 (M+H).

### Stufe 2:

Die Reduktion erfolgt in THF unter Zugabe von 5 eq. DIBAH. Das Gemisch wurde 18 h bei 60°C gerührt. Die Aufarbeitung erfolgt analog FS 311 Stufe 2. Ausbeute: 23 mg. Rt. = 2.71 min (Methode A), LCMS: 448 (M+H).

¹H NMR (500 MHz, DMSO) δ 8.02 - 7.95 (m, 1 H), 7.65 (s, 1 H), 7.49 (s, 2H), 7.30 - 7.26 (m, 1 H), 7.11 (dd, J = 11.6, 7.3 Hz, 1 H), 4.49 - 4.30 (m, 2H), 3.67 - 3.40 (m, 2H), 3.35 - 3.18 (m, 3H), 3.15 - 2.80 (m, 2H), 2.18 (d, J = 11.2 Hz, 2H), 1.98 -1.77 (m, 1 H), 1.76 - 1.63 (m, 5H), 1.26 (t, J = 18.1 Hz, 12H), 1.19 - 0.72 (m, 3H), 0.66 - 0.46 (m, 2H).

### FS535:

### 4-{(2-Amino-ethyl)-[6'-(5,5,8,8-tetramethyl-5,6,7,8-tetrahydro-naphthalen-2-yl)-3,4,5,6-tetrahydro-2H-[1,2']bipyridinyl-4-yl]-amino}-butan-1-ol

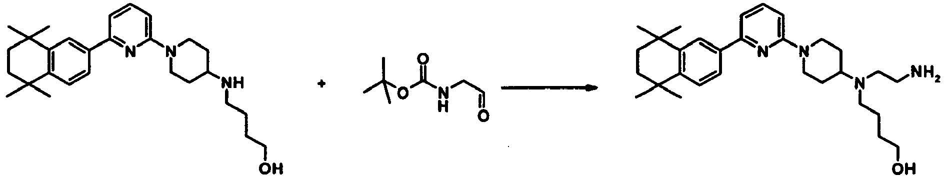

Die Herstellung erfolgt ähnlich zu FS501. Die Boc-Schutzgruppe wurde mit HCl in Dioxan abgespalten. Das Produkt liegt als Hydrochlorid vor.

Ausbeute: 14 mg. Rt. = 2.58 min (Methode A), LCMS: 479 (M+H).

¹H NMR (500 MHz, DMSO/deuteriertes TFA): δ 7.98 (dd, J = 9.0, 7.4 Hz, 1H), 7.67 (s, 1 H), 7.54 - 7.47 (m, 2H), 7.31 (t, J = 8.8 Hz, 1 H), 7.12 (d, J = 7.3 Hz, 1 H), 4.46 (d, J = 13.6 Hz, 2H), 3.82 (dd, J = 23.3, 11.4 Hz, 1 H), 3.54 - 3.04 (m, 10H), 2.23 (d, J = 10.7 Hz, 2H), 1.97 - 1.75 (m, 4H), 1.69 (s, 4H), 1.58 - 1.48 (m, 2H), 1.28 (d, J = 12.7 Hz, 12H).

### FS536:

### (S)-3-{(R)-1-[2-(5,5,8,8-Tetramethyl-5,6,7,8-tetrahydro-naphthalen-2-yl)-pyrimidin-4-yl]-pyrrolidin-3-ylamino}-propane-1,2-diol

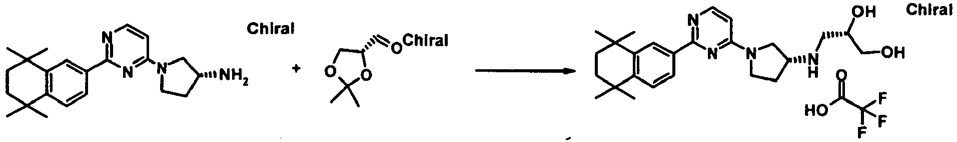

Die Herstellung erfolgt ähnlich zu FS501. Das Acetal wurde mit methanolischer HCl abgespalten. Das Produkt liegt als Trifluoracetat vor.

Ausbeute: 44 mg. Rt. = 2.40 min (Methode A), LCMS: 425 (M+H).

¹H NMR (500 MHz, DMSO/deuteriertes TFA): ö 8.25 (t, J = 8.3 Hz, 1H), 8.19 - 8.12 (m, 1 H), 7.99 - 7.92 (m, 1 H), 7.52 (dd, J = 8.4, 1.9 Hz, 1 H), 6.83 - 6.75 (m, 1H), 4.22 - 3.95 (m, 3H), 3.88 - 3.78 (m, 2H), 3.55 - 3.34 (m, 2H), 3.25 - 3.14 (m, 2H), 3.02 - 2.94 (m, 1 H), 2.50 - 2.24 (m, 2H), 1.67 (s, 4H), 1.35 - 1.21 (m, 12H).

### FS537:

### (S)-3-{(R)-1-[4-(5,5,8,8-Tetramethyl-5,6,7,8-tetrahydro-naphthalen-2-yl)-pyrimidin-2-yl]-pyrrolidin-3-ylamino}-propane-1,2-diol

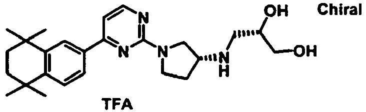

Die Herstellung erfolgt analog zu FS536. Das Produkt liegt als Trifluoracetat vor. Ausbeute: 35 mg. Rt. = 2.57 min (Methode A), LCMS: 425 (M+H).

¹H NMR (500 MHz, DMSO/deuteriertes TFA): δ 8.45 (d, J = 6.7 Hz, 1 H), 8.21 (s, 1 H), 7.99 (d, J = 7.9 Hz, 1 H), 7.63 (d, J = 6.6 Hz, 1 H), 7.51 (d, J = 8.4 Hz, 1 H), 3.93 (dd, J = 163.8, 54.5 Hz, 6H), 3.49 (dd, J = 10.8, 4.7 Hz, 1H), 3.44 - 3.34 (m, 1 H), 3.29 - 3.13 (m, 1 H), 3.06 - 2.88 (m, 1 H), 2.52 - 2.28 (m, 2H), 1.68 (s, 4H), 1.28 (d, J = 20.5 Hz, 12H).

### FS538:

### (S)-3-{(S)-1-[2-(5,5,8,8-Tetramethyl-5,6,7,8-tetrahydro-naphthalen-2-yl)-pyrimidin-4-yl]-pyrrolidin-3-ylamino}-propane-1,2-diol

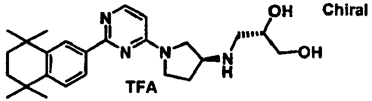

Die Herstellung erfolgt analog zu FS536. Das Produkt liegt als Trifluoracetat vor. Ausbeute: 15 mg. Rt. = 2.38 min (Methode A), LCMS: 425 (M+H).

¹H NMR (400 MHz, DMSO/deuteriertes TFA): δ 8.37 (d, J = 7.4 Hz, 1H), 8.20 (dd, J = 4.4, 2.1 Hz, 1 H), 8.03 - 7.96 (m, 1 H), 7.57 (dd, J = 8.4, 3.1 Hz, 1 H), 6.92 - 6.84 (m, 1 H), 4.26 - 3.98 (m, 3H), 3.95 - 3.62 (m, 3H), 3.57 - 3.34 (m, 2H), 3.30 - 3.15

(m, 1H), 3.07 - 2.91 (m, 1 H), 2.51 - 2.22 (m, 2H), 1.70 (s, 4H), 1.32 (dd, J = 11.7, 9.3 Hz, 12H).

### FS539:

### (S)-3-{(S)-1-[4-(5,5,8,8-Tetramethyl-5,6,7,8-tetrahydro-naphthalen-2-yl)-pyrimidin-2-yl]-pyrrolidin-3-ylamino}-propane-1,2-diol

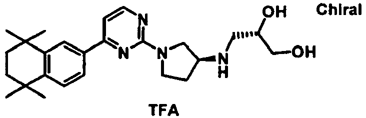

Die Herstellung erfolgt analog zu FS536. Das Produkt liegt als Trifluoracetat vor. Ausbeute: 38 mg. Rt. = 2.57 min (Methode A), LCMS: 425 (M+H).

¹H NMR (500 MHz, DMSO/deuteriertes TFA): δ 8.39 (d, J = 6.7 Hz, 1H), 8.18 (s, 1 H), 7.95 (d, J = 8.0 Hz, 1 H), 7.57 (d, J = 6.6 Hz, 1 H), 7.49 (d, J = 8.4 Hz, 1 H), 4.25 - 3.93 (m, 3H), 3.84 (b, 2H), 3.68 (b, 1 H), 3.56 - 3.33 (m, 2H), 3.23 (dd, J = 12.5, 2.7 Hz, 1 H), 3.05 - 2.88 (m, 1 H), 2.52 - 2.28 (m, 2H), 1.67 (s, 4H), 1.26 (d, J = 19.6 Hz, 12H).

### FS540:

### (R)-3-{4-[6-(5,5,8,8-Tetramethyl-5,6,7,8-tetrahydro-naphthalen-2-yl)-pyridin-2-yl]-piperazin-1-yl}-propane-1,2-diol

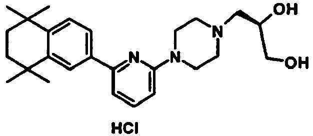

Die Herstellung erfolgt analog zu FS536. Das Produkt liegt als Hydrochlorid vor. Ausbeute: 58 mg. Rt. = 2.82 min (Methode A), LCMS: 424 (M+H).

¹H NMR (500 MHz, DMSO/deuteriertes TFA): δ 7.94 (dd, J = 8.8, 7.5 Hz, 1H), 7.69 (d, J = 1.9 Hz, 1 H), 7.52 (dd, J = 8.2, 1.9 Hz, 1 H), 7.46 (d, J = 8.3 Hz, 1 H), 7.24 - 7.15 (m, 2H), 4.52 - 4.27 (m, 2H), 4.03 - 3.95 (m, 1 H), 3.76 - 3.52 (m, 4H), 3.52 - 3.35 (m, 2H), 3.35 - 3.20 (m, 3H), 3.20 - 3.09 (m, 1 H), 1.67 (s, 4H), 1.26 (d, J = 13.6 Hz, 12H).

### FS541:

### (R)-3-[6'-(5,5,8,8-Tetramethyl-5,6,7,8-tetrahydro-naphthalen-2-yl)-3,4,5,6-tetrahydro-2H-[1,2']bipyridinyl-4-ylamino]-propane-1,2-diol

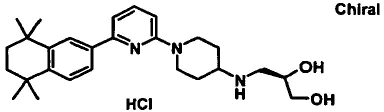

Die Herstellung erfolgt analog zu FS536. Das Produkt liegt als Hydrochlorid vor. Ausbeute: 36 mg. Rt. = 2.55 min (Methode A), LCMS: 438 (M+H).

¹H NMR (500 MHz, DMSO/deuteriertes TFA): δ 7.97 (dd, J = 9.1, 7.4 Hz, 1H), 7.61 (d, J = 1.8 Hz, 1 H), 7.46 (dt, J = 8.2, 5.1 Hz, 2H), 7.26 (d, J = 9.1 Hz, 1 H), 7.08 (d, J = 7.3 Hz, 1 H), 4.32 (d, J = 13.4 Hz, 2H), 3.83 (td, J = 9.0, 5.7 Hz, 1 H), 3.51 - 3.37 (m, 3H), 3.23 (t, J = 12.1 Hz, 2H), 3.11 (dd, J = 12.6, 2.9 Hz, 1 H), 2.91 (dd, J = 12.6, 9.4 Hz, 1H), 2.19 (dd, J = 30.6, 15.2 Hz, 2H), 1.86 - 1.69 (m, 2H), 1.67 (s, 4H), 1.26(d, J = 11.7 Hz, 12H).

### FS542:

### (R)-3-{1-[2-(5,5,8,8-Tetramethyl-5,6,7,8-tetrahydro-naphthalen-2-yl)-pyrimidin-4-yl]-piperidin-4-ylamino}-propane-1,2-diol

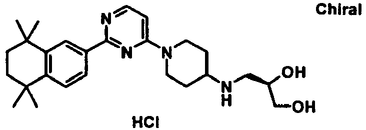

Die Herstellung erfolgt analog zu FS536. Das Produkt liegt als Hydrochlorid vor. Ausbeute: 26 mg. Rt. = 2.32 min (Methode A), LCMS: 439 (M+H).

¹H NMR (400 MHz, DMSO/deuteriertes TFA): δ 8.37 (d, J = 7.5 Hz, 1 H), 8.16 (d, J = 2.0 Hz, 1H), 7.98 (dd, J = 8.4, 2.0 Hz, 1H), 7.56 (d, J = 8.4 Hz, 1H), 7.20 (d, J = 7.5 Hz, 1 H), 5.23 (d, J = 13.3 Hz, 1 H), 4.42 (d, J = 13.2 Hz, 1H), 3.91 - 3.78 (m, 1H), 3.62 - 3.45 (m, 2H), 3.45 - 3.28 (m, 2H), 3.27 - 3.10 (m, 2H), 2.93 (dd, J = 12.6, 9.5 Hz, 1 H), 2.36 - 2.20 (m, 2H), 1.83 - 1.59 (m, 6H), 1.31 (d, J = 17.7 Hz, 12H).

### FS543:

### (R)-3-{1-[4-(5,5,8,8-Tetramethyl-5,6,7,8-tetrahydro-naphthalen-2-yl)-pyrimidin-2-yl]-piperidin-4-ylamino}-propane-1,2-diol

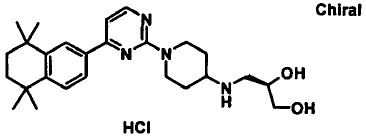

Die Herstellung erfolgt analog zu FS536. Das Produkt liegt als Hydrochlorid vor. Ausbeute: 41 mg. Rt. = 2.59 min (Methode A), LCMS: 439 (M+H).

¹H NMR (400 MHz, DMSO/deuteriertes TFA): δ 8.45 (d, J = 6.4 Hz, 1H), 8.14 (d, J = 1.9 Hz, 1 H), 7.97 (dd, J = 8.4, 1.9 Hz, 1 H), 7.57 (d, J = 6.4 Hz, 1H), 7.51 (d, J = 8.4 Hz, 1H), 4.75 (b, 2H), 3.84 (q, J = 9.2 Hz, 1 H), 3.55 - 3.43 (m, 2H), 3.38 (dd, J = 11.1, 6.2 Hz, 1 H), 3.30 - 3.11 (m, 3H), 2.92 (dd, J = 12.6, 9.5 Hz, 1H), 2.25 (t, J = 13.6 Hz, 2H), 1.82 - 1.62 (m, 6H), 1.30 (d, J = 16.5 Hz, 12H).

### FS601:

### Herstellung von 6-(5,5,8,8-Tetramethyl-5,6,7,8-tetrahydro-naphthalen-2-yl)-1',2',3',4',5',6'-hexahydro-[2,4']bipyridinyl und 6-(5,5,8,8-Tetramethyl-5,6,7,8-tetrahydro-naphthalen-2-yl)-1',2',3',6'-tetrahydro-[2,4']bipyridinyl:

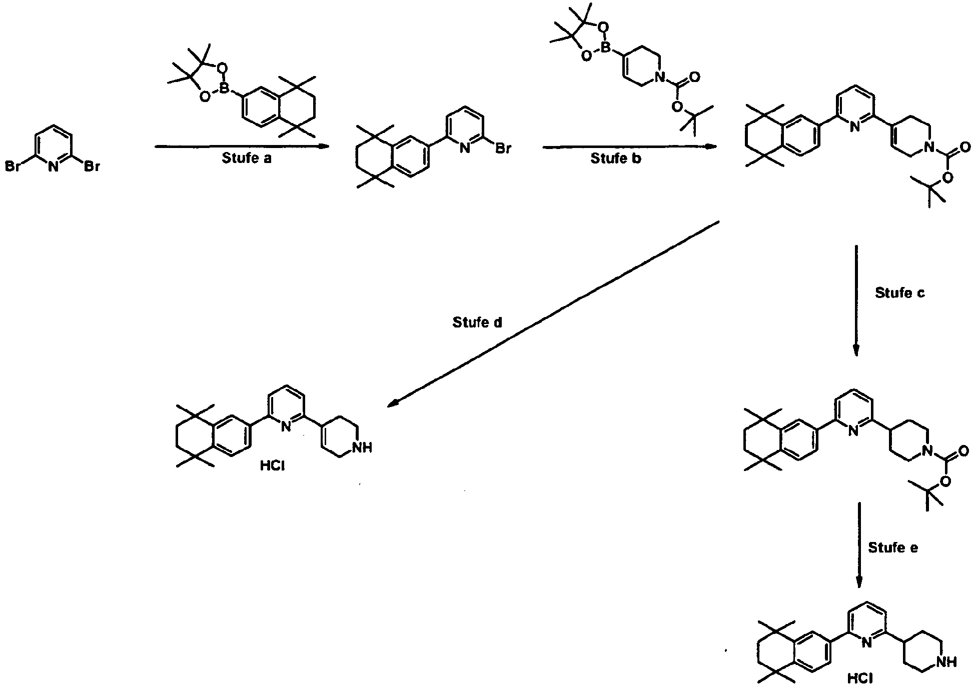

### Stufe a:

### 2-Bromo-6-(5,5,8,8-tetramethyl-5,6,7,8-tetrahydro-naphthalen-2-yl)-pyridine:

200 mg (0.84 mmol) 2,6-Dibrompyridin und 265 mg (0.84 mmol) 4,4,5,5-Tetramethyl-2-(5,5,8,8-tetramethyl-5,6,7,8-tetrahydro-naphthalen-2-yl)-[1 ,3,2]dioxaborolane werden in 6 ml 2M Natriumcarbonatlösung und in 2.5 ml Dimethoxyethan gelöst. Das Reaktionsgemisch wird mehrfach entgast und unter Stickstoffatmosphäre mit 49 mg (0.04 mmol) Tetrakis(triphenylphosphin)-palladium versetzt, 16 h bei 80°C gerührt und anschließend 30 min in der Mikrowelle bestrahlt. Das Reaktionsgemisch wird eingeengt und mit gesättigter Natriumhydrogencarbonatlösung versetzt. Dieses Gemisch wird dreimal mit Essigester und dreimal mit Dichlormethan extrahiert, die vereinigten organischen Phasen über Na₂SO₄ getrocknet und eingeengt. Das Rohprodukt wird ohne weitere Aufreinigung weiter umgesetzt.

210 mg Öl, Rt. = 4.25 min (Methode A), LCMS: 345 (M+H).

### Stufe b:

### 6-(5,5,8,8-Tetramethyl-5,6,7,8-tetrahydro-naphthalen-2-yl)-3',6'-dihydro-2'H-[2,4']bipyridinyl-1'-carboxylic acid tert-butyl ester:

100 mg (0.29 mmol) 2-Bromo-6-(5,5,8,8-tetramethyl-5,6,7,8-tetrahydro-naphthalen-2-yl)-pyridine und 135 mg (0.44 mmol) 4-(4,4,5,5-Tetramethyl-[1,3,2]dioxaborolan-2-yl)-3,6-dihydro-2H-pyridine-1-carboxylic acid tert-butyl ester werden in 2 ml 2M Natriumcarbonatlösung und in 2 ml Dimethoxyethan gelöst. Das Reaktionsgemisch wird mehrfach entgast, unter Stickstoffatmosphäre mit 34 mg (0.03 mmol) Tetrakis(triphenylphosphin)-palladium versetzt und 30 min in der Mikrowelle bestrahlt, anschließend eingeengt und mit gesättigter Natriumhydrogencarbonatlösung versetzt. Dieses Gemisch wird dreimal mit Essigester extrahiert, die vereinigten organischen Phasen über Na₂SO₄ getrocknet und eingeengt. Das Rohprodukt wird säulenchromatographisch an Kieselgel aufgereinigt.

67 mg Öl, Rt. = 4.14 min (Methode A), LCMS: 447 (M+H).

### Stufe c:

### 6-(5,5,8,8-Tetramethyl-5,6,7,8-tetrahydro-naphthalen-2-yl)-3',4',5',6'-tetrahydro-2'H-[2,4']bipyridinyl-1'-carboxylic acid tert-butyl ester:

52 mg (0.11 mmol) 6-(5,5,8,8-Tetramethyl-5,6,7,8-tetrahydro-naphthalen-2-yl)-3',6'-dihydro-2'H-[2,4']bipyridinyl-1'-carboxylic acid tert-butyl ester werden in 20 ml Ethanol gelöst und unter Verwendung einer Pd/C-Kartusche (10%, 30x4 mm) im H-Cube (Thales Nanotechnology) hydriert. Anschließend wird das Lösungsmittel abdestilliert.

42 mg weißer Feststoff, Rt. = 4.17 min (Methode A), LCMS: 449 (M+H).

### Stufe d:

### 6-(5,5,8,8-Tetramethyl-5,6,7,8-tetrahydro-naphthalen-2-yl)-1',2',3',6'-tetrahydro-[2,4']bipyridinyl:

Die Abspaltung der Schutzgruppe erfolgt entsprechend Beispiel FS201. Das Produkt liegt als Hydrochlorid vor.

8 mg, beigefarbener Feststoff. Rt. = 3.01 min (Methode A), LCMS: 347 (M+H). ¹H NMR (500 MHz, DMSO/deuteriertes TFA) δ 8.05 (d, *J* = 1.9, 1H), 7.95 (t, *J* = 7.8, 1 H), 7.89 (d, *J* = 7.6, 1 H), 7.83 (dd, *J* = 8.2, 1.9, 1 H), 7.61 (d, *J* = 7.6, 1 H), 7.46 (d, *J* = 8.3, 1 H), 6.83 (s, 1 H), 3.91 (s, 2H), 3.43 (t, *J* = 6.0, 2H), 2.92 (s, 2H), 1.71 (s, 4H), 1.32 (d, *J* = 21.1, 12H).

### Stufe e:

### 6-(5,5,8,8-Tetramethyl-5,6,7,8-tetrahydro-naphthalen-2-yl)-1',2',3',4',5',6'-hexahydro-[2,4']bipyridinyl:

Die Abspaltung der Schutzgruppe erfolgt entsprechend Beispiel FS201. Das Produkt liegt als Hydrochlorid vor.

14 mg, beigefarbener Feststoff. Rt. = 2.66 min (Methode A), LCMS: 349 (M+H).

¹H NMR (500 MHz, DMSO/deuteriertes TFA) δ 8.44 (t, *J* = 8.0, 1H), 8.12 (d, *J* = 7.9, 1H), 7.88(d, *J* = 1.9, 1 H), 7.75 (d, *J* = 7.9, 1 H), 7.71 (dd, *J* = 8.2, 1.9, 1 H), 7.60 (d, *J* = 8.3, 1 H), 3.52 (d, *J* = 12.9, 2H), 3.44 (dd, *J* = 13.3, 10.6, 1H), 3.10 (dd, *J* = 12.9, 10.3, 2H), 2.22 (d, *J* = 13.5, 2H), 2.04 (qd, *J* = 13.4, 3.8, 2H), 1.74 (s, 4H), 1.34 (d, *J* = 16.2, 12H).

### FS602:

### 1-[4-(5,5,8,8-Tetramethyl-5,6,7,8-tetrahydro-naphthalen-2-yl)-pyridin-2-yl]-piperazine:

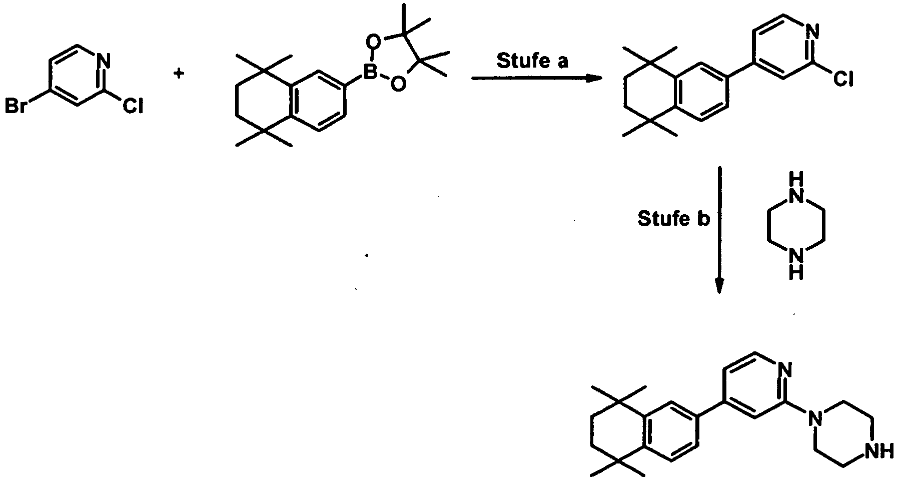

### Stufe a:

173 µl (1.56 mmol) 4-Brom-2-chlorpyridin, 735 mg (2.34 mmol) 4,4,5,5-Tetramethyl-2-(5,5,8,8-tetramethyl-5,6,7,8-tetrahydro-naphthalen-2-yl)-[1,3,2]dioxaborolane, 18 mg (0.03 mmol) Xantphos, 687 mg (3.24 mmol) Trikaliumphosphat Trihydrat und 11 mg (0.05 mmol Palladium(II)-acetat werden in 5 ml Toluol und 500 µl Wasser suspendiert. Das Reaktionsgemisch wird mehrfach entgast, unter Stickstoffatmosphäre 90 min bei 100°C in der Mikrowelle bestrahlt, mit 30 ml Wasser verdünnt und dreimal mit Essigester extrahiert. Die vereinigten organischen Phasen werden über Na₂SO₄ getrocknet und eingeengt. Das Rohprodukt wird säulenchromatographisch an Kieselgel aufgereinigt.

417 mg, zähes Öl, Rt. = 3.95 min (Methode B), LCMS: 300 (M+H).

¹H NMR (400 MHz, DMSO/deuteriertes TFA) δ 8.41 (d, *J* = 5.2, 1 H), 7.80 (d, *J* = 0.6, 1H), 7.72 (d, *J* = 2.0, 1H), 7.70 (dd, *J* = 5.3, 1.1, 1 H), 7.54 (dd, *J* = 8.3, 1.9, 1H), 7.45 (d, *J* = 8.3, 1 H), 1.66 (s, 4H), 1.28 (d, *J* = 19.4, 12H).

### Stufe b:

145 mg (0.48 mmol) 2-Chloro-4-(5,5,8,8-tetramethyl-5,6,7,8-tetrahydro-naphthalen-2-yl)-pyridine und 333 mg (3.87 mmol) Piperazin werden mit 2 ml Toluol versetzt und 8 h bei 180°C in der Mikrowelle bestrahlt. Der Rückstand wird mit Wasser versetzt, mit NaOH leicht basisch gestellt und anschließend dreimal mit Ethylacetat extrahiert. Die vereinigten organischen Phasen werden über Na₂SO₄ getrocknet und eingeengt. Das Rohprodukt wird säulenchromatographisch an RP-Kieselgel aufgereinigt.

125 mg, zähes Öl, Rt. = 2.33 min (Methode B), LCMS: 350 (M+H).

¹H NMR (400 MHz, DMSO/deuteriertes TFA) ¹H NMR (400 MHz, DMSO) δ 8.17 (d, *J* = 6.6, 1 H), 7.78 (d, *J* = 2.0, 1 H), 7.67 (dd, *J* = 8.3, 2.0, 1 H), 7.61 (s, 1 H), 7.54 (d, *J* = 8.3, 1 H), 7.43 (dd, *J* = 6.7, 1.4, 1 H), 4.09 - 3.98 (m, 4H), 3.45 - 3.36 (m, 4H), 1.72 (s, 4H), 1.33 (d, *J* = 19.3, 12H).

### FS603:

### 1'-(4-Chloro-[1,3,5]triazin-2-yl)-[1,4']bipiperidinyl-3-ol:

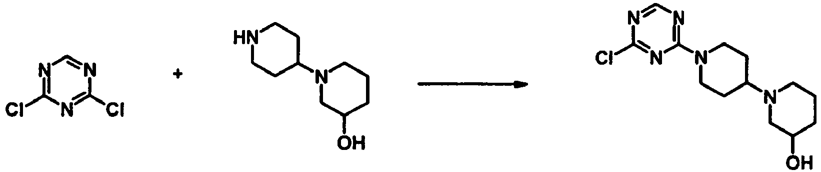

200 mg (1.27 mmol) 2,4-Dichlor-1,3,5-triazin werden in 2 ml Acetonitril gelöst, auf 0°C gekühlt, mit 176 µl Triethylamin und 223 mg (1.27 mmol)

[1,4']Bipiperidinyl-3-ol versetzt. Das Reaktionsgemisch wird 2 h bei 0°C und anschließend 48 h bei Raumtemperatur gerührt. Anschließend wird ges. NaCl-Lösung zugegeben und dreimal mit Ethylacetat extrahiert. Die vereinigten organischen Phasen werden mit 1 N NaOH und Wasser gewaschen, über Na₂SO₄ getrocknet und eingedampft.

245 mg, zähes Öl, Rt. = 2.27 min (Methode B), LCMS: 298 (M+H).

### FS604:

### 1'-[4-(5,5,8,8-Tetramethyl-5,6,7,8-tetrahydro-naphthaten-2-yl)-[1,3,5]triazin-2-yl]-[1,4']bipiperidinyl-3-ol

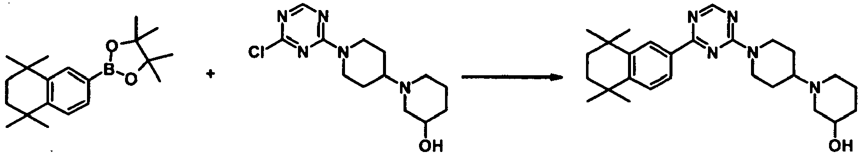

50 mg (0.17 mmol) 1'-(4-Chloro-[1,3,5]triazin-2-yl)-[1,4']bipiperidinyl-3-ol und 139 mg (0.42 mmol) 4,4,5,5-Tetramethyl-2-(5,5,8,8-tetramethyl-5,6,7,8-tetrahydro-naphthalen-2-yl)-[1,3,2]dioxaborolane werden in 3.2 ml Toluol/Ethanol/Wasser (3.2/3.2/1) und 45 mg (0.42 mmol) Natriumcarbonat versetzt. Das Reaktionsgemisch wird mehrfach entgast und unter Stickstoffatmosphäre mit 19 mg (0.017 mmol) Tetrakis(triphenylphosphin)-palladium(0) versetzt, 10 min im Ultraschallbad behandelt und anschließend 5 h bei 90°C erhitzt. Das Reaktionsgemisch wird mit Wasser versetzt und dreimal mit Ethylacetat/Ether extrahiert. Die vereinigten organischen Phasen werden über Na₂SO₄ getrocknet und eingeengt. Das Rohprodukt wird mittels präparativer HPLC aufgereinigt. 14 mg, weißer Feststoff, Rt. = 2.61 min (Methode B), LCMS: 450 (M+H).

### FS605:

### {1-[1-(4-Chloro-[1,3,5]triazin-2-yl)-piperidin-4-yl]-pyrrolidin-3-yl}-carbamic acid tert-butyl ester:

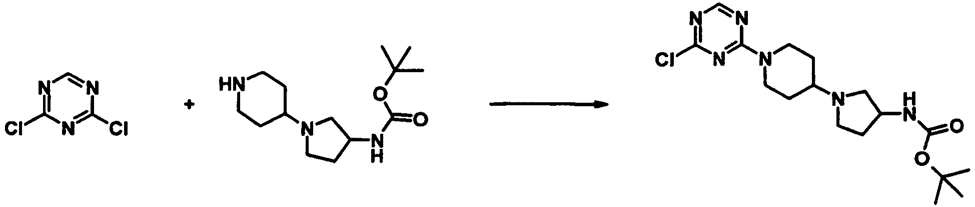

Die Herstellung erfolgt analog FS603.

650 mg, gelbes Öl, Rt. = 2.71 min (Methode B), LCMS: 383 (M+H).

### FS606:

### (1-{1-[4-(5,5,8,8-Tetramethyl-5,6,7,8-tetrahydro-naphthalen-2-yl)-[1,3,5]triazin-2-yl]-piperidin-4-yl}-pyrrolidin-3-yl)-carbamic acid tert-butyl ester:

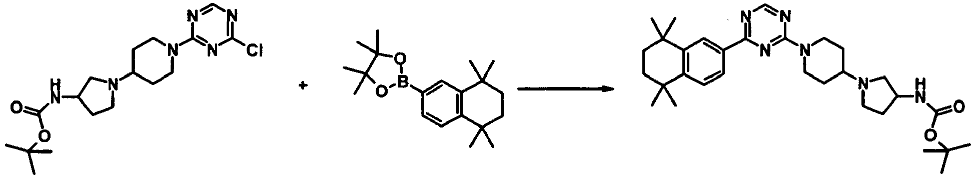

50 mg (0.11 mmol) {1-[1-(4-Chloro-[1,3,5]triazin-2-yl)-piperidin-4-yl]-pyrrolidin-3-yl}-carbamic acid tert-butyl ester und 45 mg (0.12 mmol) 4,4,5,5-Tetramethyl-2-(5,5,8,8-tetramethyl-5,6,7,8-tetrahydro-naphthalen-2-yl)-[1,3,2]dioxaborolane werden in 1.5 ml DMF/DME/Methanol/Wasser (1/1/0.9/0.3) und 23 mg (0.22 mmol) Natriumcarbonat versetzt. Das Reaktionsgemisch wird mehrfach entgast, unter Stickstoffatmosphäre mit 8 mg (0.017 mmol) Bis(triphenylphosphin)-palladium(II)-dichlorid versetzt, 10 min im Ultraschallbad behandelt und anschließend 20 min bei 100°C in der Mikrowelle bestrahlt. Das Reaktionsgemisch wird mit Methanol verdünnt, filtriert und das Rohprodukt wird mittels präparativer HPLC aufgereinigt.

24 mg, weißer Feststoff, Rt. = 2.95 min (Methode B), LCMS: 535 (M+H).

### FS607:

### (2-{4-[6-(5,5,8,8-Tetramethyl-5,6,7,8-tetrahydro-naphthalen-2-yl)-pyridin-2-yl]-piperazin-1-ylmethyl}-cyclopropyl)-methanol

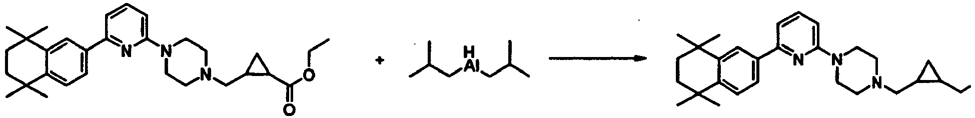

100 mg (0.21 mmol) 2-{4-[6-(5,5,8,8-Tetramethyl-5,6,7,8-tetrahydro-naphthalen-2-yl)-pyridin-2-yl]-piperazin-1-ylmethyl}-cyclopropanecarboxylic acid ethyl ester wird in 4 ml THF gelöst und mit 1.05 ml (1.05 mmol) Diisobutylaluminiumhydrid (1.0 M in THF) versetzt. Das Reaktionsgemisch wird unter Stickstoffatmosphäre 18 h bei Raumtemperatur gerührt. Das Reaktionsgemsich wird mit Wasser versetzt und kurz gerührt, mit Ethylacetat versetzt und über Celite filtriert. Es wird mit Ethylacetat und Methanol gewaschen und die Mutterlauge eingeengt. Der Rückstand wird säulenchromatographisch an Kieselgel aufgereinigt.

Ausbeute: 50 mg, gelbes Öl. Rt. = 3.07 min (Methode A), LCMS: 434 (M+H).

¹H NMR (500 MHz, DMSO/deuteriertes TFA) δ 7.90 (d, J = 1.6 Hz, 1H), 7.81 (t, J = 8.1 Hz, 1 H), 7.72 (d, J = 8.1 Hz, 1H), 7.44 (d, J = 8.3 Hz, 1 H), 7.30 (d, J = 7.4 Hz, 1 H), 7.04 (d, J = 8.5 Hz, 1 H), 4.55 (d, J = 13.2 Hz, 2H), 3.84 - 3.64 (m, 2H), 3.57 (dd, J = 11.2, 5.4 Hz, 1 H), 3.43 - 3.16 (m, 6H), 3.07 - 2.98 (m, 1 H), 1.70 (s, 4H), 1.31 (d, *J* = 14.7 Hz, 12H), 1.16 - 1.07 (m, 1H), 1.07 - 0.94 (m, 1 H), 0.67 - 0.60 (m, 1 H), 0.60 - 0.53 (m, 1 H).

### FS608:

### 4-{(4-Hydroxy-butyl)-[6'-(5,5,8,8-tetramethyl-5,6,7,8-tetrahydro-naphthalen-2-yl)-3,4,5,6-tetrahydro-2H-[1,2']bipyridinyl-4-yl]-amino}-butan-1-ol

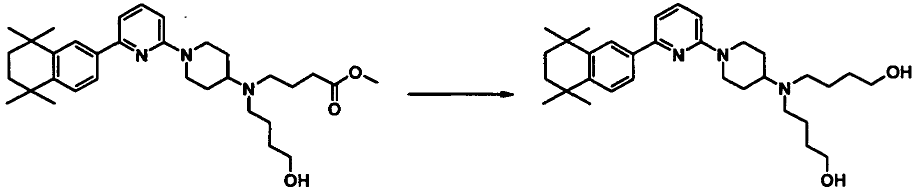

Die Herstellung erfolgt ähnlich zu FS311 Stufe 2.

Ausbeute: 27 mg. Rt. = 2.66 min (Methode A), LCMS: 508 (M+H).

¹H NMR (500 MHz, DMSO/deuteriertes TFA): δ 7.99 (dd, J = 8.9, 7.5 Hz, 1H), 7.71 (d, J = 1.5 Hz, 1 H), 7.58 - 7.49 (m, 2H), 7.31 (d, J = 9.0 Hz, 1 H), 7.15 (d, J = 7.4 Hz, 1 H), 4.47 (d, J = 13.6 Hz, 2H), 3.74 (s, 1 H), 3.55 - 3.43 (m, 4H), 3.34 - 3.16 (m, 4H), 3.15 - 3.00 (m, 2H), 2.16 (d, J = 10.4 Hz, 2H), 1.94 - 1.73 (m, 6H), 1.70 (s, 4H), 1.59 - 1.43 (m, 4H), 1.37 - 1.24 (m, 12H).

### FS609:

### (2R,3S)-2-{[6'-(5,5,8,8-Tetramethyl-5,6,7,8-tetrahydro-naphthalen-2-yl)-3,4,5,6-tetrahydro-2H-[1,2']bipyridinyl-4-ylamino]-methyl}-pyrrolidin-3-ol

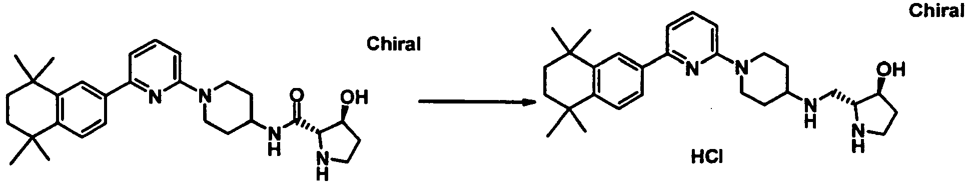

184 mg (0.39 mmol) (2S,3S)-3-Hydroxy-pyrrolidine-2-carboxylic acid [6'-(5,5,8,8-tetramethyl-5,6,7,8-tetrahydro-naphthalen-2-yl)-3,4,5,6-tetrahydro-2H-[1,2']bipyridinyl-4-yl]-amide wurde in 5 ml THF gelöst und unter Stickstoffatmosphäre bei 70°C mit 579 µl 2M Boran Dimethylsufid Lösung in THF versetzt und anschließend 2 Stunden bei 70°C gerührt. Es wurde nochmals 579 µl Boran-Dimethylsulfid-Lösung zugegeben und weitere 4 Stunden bei 70°C im Heizblock erhitzt. Zur Zersetzung des Boran-Überschusses wurde tropfenweise mit 1 ml MeOH versetzt. Das Reaktionsgemisch wurde eingeengt und der ölige RS mit 1 ml Wasser und 1 ml konz HCl versetzt. Es wurde 2 h bei Raumtemperatur gerührt, mit 2 molarer NaOH alkalisch gestellt und mit Ethylacetat extrahiert. Die organische Phase wurde getrocknet und säulenchromatographisch an Kieselgel aufgereinigt. Ausbeute: 51 mg, beigefarbener Feststoff. Rt. = 2.51 min (Methode A), LCMS: 463 (M+H).

¹H NMR (500 MHz, DMSO/deuteriertes TFA) δ 7.94 (dd, J = 9.0, 7.4 Hz, 1H), 7.56 (d, J = 1.9 Hz, 1 H), 7.46 (d, J = 8.3 Hz, 1 H), 7.40 (dd, J = 8.2, 1.9 Hz, 1 H), 7.20 (d, J = 9.1 Hz, 1 H), 7.05 (d, J = 7.3 Hz, 1 H), 4.35 - 4.17 (m, 3H), 3.67 (dt, J = 8.7, 4.5 Hz, 1 H), 3.47 (t, J = 11.1 Hz, 1 H), 3.42 - 3.16 (m, 6H), 2.14 (ddd, J = 21.5, 18.1, 9.9 Hz, 3H), 1.87 (ddd, J = 30.3, 19.0, 8.5 Hz, 3H), 1.66 (s, 4H), 1.24 (d, J = 8.7 Hz, 12H).

### FS610:

### (2R,3S)-2-{4-[6-(5,5,8,8-Tetramethyl-5,6,7,8-tetrahydro-naphthalen-2-yl)-pyridin-2-yl]-piperazin-1-ylmethyl}-pyrrolidin-3-ol

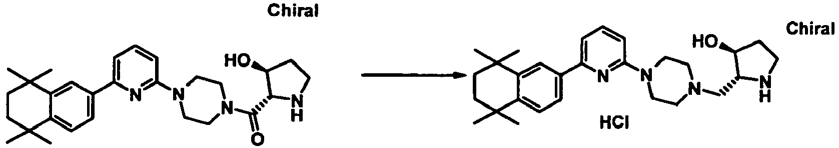

Die Herstellung erfolgt analog FS609.

Ausbeute: 79 mg. Rt. = 2.67 min (Methode A), LCMS: 449 (M+H).

¹H NMR (500 MHz, DMSO/deuteriertes TFA δ 7.85 (dd, J = 12.6, 5.0 Hz, 2H), 7.68 (dd, J = 8.2, 1.9 Hz, 1 H), 7.44 (d, J = 8.3 Hz, 1 H), 7.28 (d, J = 7.5 Hz, 1 H), 7.10 (d, J = 8.6 Hz, 1 H), 3.99 (d, J = 39.3 Hz, 6H), 3.65 - 3.33 (m, 8H), 2.17 (td, J = 13.6, 7.8 Hz, 1 H), 1.90 (dq, J = 8.1, 6.2 Hz, 1H), 1.69 (s, 4H), 1.29 (d, J = 18.4 Hz, 12H).

### FS611:

### (2R,3R)-3-Amino-4-{4-[6-(5,5,8,8-tetramethyl-5,6,7,8-tetrahydro-naphthalen-2-yl)-pyridin-2-yl]-piperazin-1-yl}-butan-2-ol

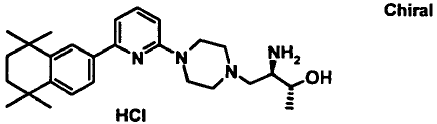

Die Herstellung erfolgt analog FS609.

Ausbeute: 37 mg. Rt. = 2.67 min (Methode A), LCMS: 437 (M+H).

¹H NMR (400 MHz, DMSO/deuteriertes TFA): δ 7.89 - 7.80 (m, 2H), 7.65 (dd, J = 8.2, 1.9 Hz, 1 H), 7.44 (d, J = 8.3 Hz, 1 H), 7.26 (d, J = 7.4 Hz, 1 H), 7.12 (d, J = 8.7 Hz, 1 H), 4.27 - 3.33 (m, 12H), 1.69 (s, 4H), 1.29 (d, J = 14.0 Hz, 12H), 1.23 (d, J = 6.4 Hz, 3H).

### FS612:

### (2R,3R)-3-Amino-4-[6'-(5,5,8,8-tetramethyl-5,6,7,8-tetrahydro-naphthalen-2-yl)-3,4,5,6-tetrahydro-2H-[1,2']bipyridinyl-4-ylamino]-butan-2-ol

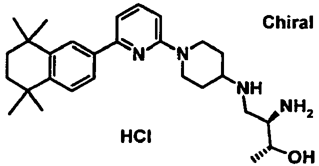

Die Herstellung erfolgt analog FS609.

Ausbeute: 30 mg. Rt. = 2.50 min (Methode A), LCMS: 451 (M+H).

¹H NMR (400 MHz, DMSO/deuteriertes TFA δ 8.00 (dd, J = 9.0, 7.4 Hz, 1 H), 7.73 (d, J = 1.9 Hz, 1 H), 7.54 (dt, J = 21.8, 5.1 Hz, 2H), 7.30 (d, J = 9.1 Hz, 1 H), 7.18 (d, J = 7.3 Hz, 1 H), 4.40 (d, J = 13.2 Hz, 2H), 3.98 - 3.90 (m, 1 H), 3.59 - 3.49 (m, 1 H), 3.45 - 3.15 (m, 5H), 2.22 (s, 2H), 1.78 (d, J = 12.1 Hz, 2H), 1.70 (s, 4H), 1.32 (t, J = 11.5 Hz, 12H), 1.23 (t, J = 5.3 Hz, 3H).

### FS701:

### 6'-(5,5,8,8-Tetramethyl-5,6,7,8-tetrahydro-naphthalen-2-yl)-3,4,5,6-tetrahydro-2H-[1,2']bipyridinyl-4-ylamine:

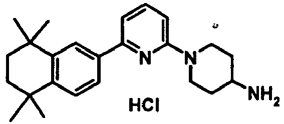

Die Herstellung erfolgt analog FS 102 ausgehend von (6'-Bromo-3,4,5,6-tetrahydro-2H-[1,2']bipyridinyl-4-yl)-carbamic acid tert-butyl ester (Herstellung analog WO2005/26149) und 5,5,8,8-Tetramethyl-5,6,7,8-tetrahydro-naphthalen-2-yl-boronsäure. Die anschließende Abspaltung der Schutzgruppe erfolgt entsprechend FS201. Das Produkt liegt als Hydrochlorid vor. 55 mg, gelbes Öl, Rt. = 2.68 min (Methode A), LCMS: 364 (M+H).

¹H NMR (500 MHz, DMSO/deuteriertes TFA) δ 7.96 (dd, *J* = 8.5, 7.8, 1 H), 7.78 (d, *J* = 1.7, 1 H), 7.61 (dd, *J* = 8.2, 1.8, 1 H), 7.51 (d, *J* = 8.3, 1 H), 7.24 (d, *J* = 8.9, 1 H), 7.19 (d, *J* = 7.4, 1H), 4.36 (d, *J* = 13.6, 2H), 3.46 - 3.38 (m, 1 H), 3.24 (t, *J* = 12.1, 2H), 2.10 - 2.02 (m, 2H), 1.71 (s, 4H), 1.65 (ddd, *J* = 15.7, 12.4, 3.9, 2H), 1.32 (d, *J* = 13.7, 12H).

### FS702:

### (S)-2-Amino-3-hydroxy-1-{4-[6-(5,5,8,8-tetramethyl-5,6,7,8-tetrahydro-naphthalen-2-yl)-pyridin-2-yl]-piperazin-1-yl}-butan-1-on

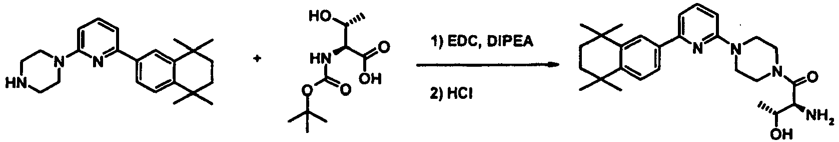

200 mg (0,57 mmol) 1-[6-(5,5,8,8-Tetramethyl-5,6,7,8-tetrahydro-naphthalen-2-yl)-pyridin-2-yl]-piperazine, 138 mg (0.629 mmol) N-(tert-Butoxycarbonyl)-L-threonine, 105 mg (0.69 mmol) HOBt, 132 mg (0.69 mmol) N-(3-Dimethylaminopropyl)-N'-ethylcarbodiimidhydrochlorid und 291 µl (1.72 mmol) DIPEA werden in 5 ml THF gelsöt und 18 h bei Raumtemperatur gerührt. Das Rohprodukt wurde säulenchromatographisch and Kieselgel aufgereinigt, in Dioxan gelöst und mit einem Überschuss an 4N HCl in Dioxan versetzt. Das Reaktionsgemisch wird 12 h bei Raumtemperatur gerührt, eingedampft und mittels präparativer HPLC aufgereinigt. Das Produkt wird mit methanolischer HCl ins Hydrochlorid überführt. Ausbeute: 95 mg, beigefarbener Feststoff. Rt. = 2.75 min (Methode A), LCMS: 451 (M+H).

¹H NMR (500 MHz, DMSO/deuteriertes TFA) δ 7.96 (dd, J = 8.8, 7.6 Hz, 1 H), 7.59 (s, 1 H), 7.48 (d, J = 8.2 Hz, 1 H), 7.43 (d, J = 8.2 Hz, 1H), 7.21 (d, J = 9.1 Hz, 1 H), 7.08 (d, J = 7.4 Hz, 1 H), 4.32 (d, J = 4.9 Hz, 1 H), 4.04 - 3.96 (m, 1 H), 3.89 - 3.64 (m, 8H), 1.67 (s, 4H), 1.32 - 1.21 (m, 12H), 1.18 (d, J = 6.5 Hz, 3H).

### FS703:

### (S)-2-Amino-3-hydroxy-1-{4-[6-(5,5,8,8-tetramethyl-5,6,7,8-tetrahydro-naphthalen-2-yl)-pyridin-2-yl]-piperazin-1-yl}-propan-1-on:

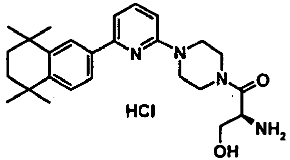

Die Herstellung erfolgt analog FS702. Das Produkt liegt als Hydrochlorid vor. Ausbeute: 105 mg, beigefarbener Feststoff. Rt. = 2,70 min (Methode A), LCMS: 437 (M+H).

¹H NMR (500 MHz, DMSO/deuteriertes TFA): δ 8.00 (dd, J = 9.0, 7.5 Hz, 1H), 7.67 (b, 1 H), 7.50 (b, 2H), 7.27 (d, J = 9.1 Hz, 1 H), 7.14 (d, J = 7.4 Hz, 1 H), 4.49 (t, J = 5.3 Hz, 1 H), 3.91 - 3.65 (m, 10H), 1.69 (s, 4H), 1.28 (d, J = 13.4 Hz, 12H).

### FS704:

### Morpholine-2-carboxylic acid [6-(5,5,8,8-tetramethyl-5,6,7,8-tetrahydro-naphthalen-2-yl)-pyridin-2-yl]-amide:

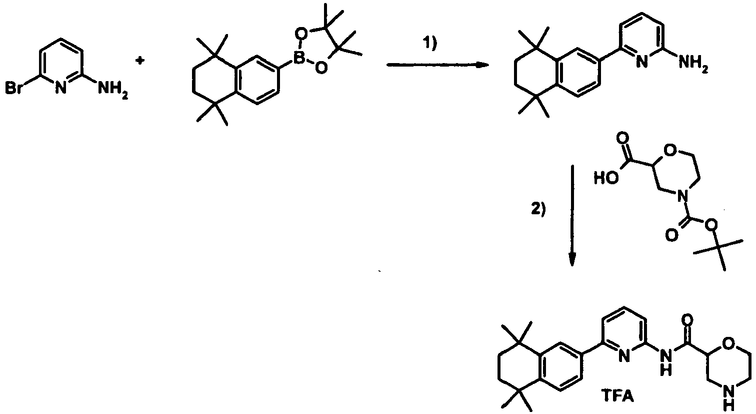

### Stufe 1:

Die Suzuki Reaktion wurde mit Tetrakis(triphenylphosphine)-palladium(0) und 2M Natriumcarbonat-Lösung in Dioxan durchgeführt. Das Reaktionsgemisch wurde 18 h refluxiert. Aufarbeitung wie bei den übrigen Suzuki Reaktionen beschrieben.

Ausbeute: 4.4 g, beigefarbener Feststoff. Rt. = 2,39 min (Methode B), LCMS: 281 (M+H).

### Stufe 2:

Die Herstellung erfolgt analog FS702. Das Produkt liegt als Trifluoracetat vor. Ausbeute: 23 mg, beigefarbener Feststoff. Rt. = 2,63 min (Methode B), LCMS: 394 (M+H).

### FS705:

### Morpholine-2-carboxylic acid [6'-(5,5,8,8-tetramethyl-5,6,7,8-tetrahydro-naphthalen-2-yl)-3,4,5,6-tetrahydro-2H-[1,2']bipyridinyl-4-yl]-amide:

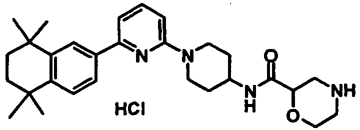

Die Herstellung erfolgt analog FS702. Das Produkt liegt als Hydrochlorid vor. Ausbeute: 244 mg, beigefarbener Feststoff. Rt. = 2,58 min (Methode A), LCMS: 477 (M+H).

¹H NMR (500 MHz, DMSO/deuteriertes TFA) ö 7.98 (dd, J = 9.1, 7.4 Hz, 1H), 7.62 (d, J = 1.6 Hz, 1 H), 7.48 (dt, J = 8.2, 5.0 Hz, 2H), 7.30 (d, J = 9.2 Hz, 1 H), 7.08 (d, J = 7.3 Hz, 1 H), 4.29 - 4.18 (m, 3H), 4.07 - 3.97 (m, 2H), 3.81 (dt, J = 18.2, 3.8 Hz, 1H), 3.47 (dd, J = 12.9, 2.0 Hz, 1 H), 3.35 (t, J = 11.7 Hz, 2H), 3.22 (d, J = 12.9 Hz, 1 H), 3.15 - 2.95 (m, 2H), 1.95 -1.85 (m, 2H), 1.75 - 1.63 (m, 6H), 1.28 (d, J = 11.5 Hz, 12H).

### FS706:

### (2S,3S)-3-Hydroxy-pyrrolidine-2-carboxylic acid [6'-(5,5,8,8-tetramethyl-5,6,7,8-tetrahydro-naphthalen-2-yl)-3,4,5,6-tetrahydro-2H-[1,2']bipyridinyl-4-yl]-amide:

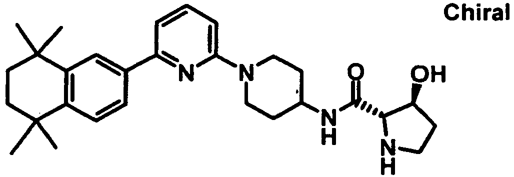

Die Herstellung erfolgt analog FS702.

Ausbeute: 184 mg, beigefarbener Feststoff. Rt. = 2,54 min (Methode A), LCMS: 477 (M+H).

¹H NMR (500 MHz, DMSO/deuteriertes TFA) δ 7.99 (dd, J = 9.1, 7.4 Hz, 1H), 7.64 (d, J = 1.6 Hz, 1 H), 7.49 (dt, J = 8.3, 5.0 Hz, 2H), 7.31 (d, J = 9.2 Hz, 1 H), 7.09 (d, J = 7.3 Hz, 1 H), 4.40 (dd, J = 5.5, 3.4 Hz, 1 H), 4.21 - 4.11 (m, 2H), 4.07 (d, J = 1.9 Hz, 1 H), 4.05 - 3.95 (m, 1 H), 3.51 - 3.29 (m, 4H), 2.05 - 1.88 (m, 4H), 1.76 - 1.56 (m, 6H), 1.28 (d, J = 11.4 Hz, 12H).

### FS707:

### ((2S,3S)-3-Hydroxy-pyrrolidin-2-yl)-{4-[6-(5,5,8,8-tetramethyl-5,6,7,8-tetrahydro-naphthalen-2-yl)-pyridin-2-yl]-piperazin-1-yl}-methanone:

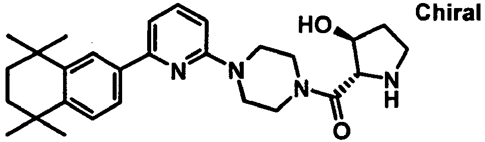

Die Herstellung erfolgt analog FS702.

Ausbeute: 155 mg, kristalliner Feststoff. Rt. = 2,72 min (Methode A), LCMS: 463 (M+H).

¹H NMR (500 MHz, DMSO/deuteriertes TFA): δ 8.04 (dd, J = 8.8, 7.6 Hz, 1H), 7.77 (d, J = 2.0 Hz, 1 H), 7.63 - 7.52 (m, 2H), 7.32 (d, J = 9.0 Hz, 1 H), 7.22 (dd, J = 7.3, 1.9 Hz, 1 H), 4.63 (s, 1 H), 4.54 - 4.45 (m, 1 H), 3.98 - 3.71 (m, 8H), 3.57 - 3.47 (m, 1 H), 3.47 - 3.36 (m, 1 H), 2.04 - 1.89 (m, 2H), 1.74 (s, 4H), 1.34 (d, J = 15.1 Hz, 12H).

### FS708:

### (S)-2-Amino-3-hydroxy-N-[6'-(5,5,8,8-tetramethyl-5,6,7,8-tetrahydro-naphthalen-2-yl)-3,4,5,6-tetrahydro-2H-[1,2']bipyridinyl-4-yl]-propionamide:

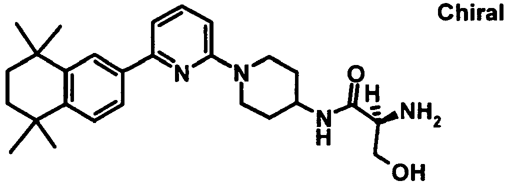

Die Herstellung erfolgt analog FS702.

Ausbeute: 187 mg, farbloses Öl. Rt. = 2,50 min (Methode A), LCMS: 451 (M+H). ¹H NMR (500 MHz, DMSO/deuteriertes TFA) δ 8.01 (dd, J = 9.1, 7.4 Hz, 1 H), 7.67 (s, 1 H), 7.55 - 7.48 (m, 2H), 7.34 (d, J = 9.1 Hz, 1 H), 7.12 (d, J = 7.3 Hz, 1 H), 4.21 - 4.10 (m, 2H), 4.07 - 3.98 (m, 1 H), 3.85 - 3.76 (m, 2H), 3.76 - 3.69 (m, 1 H), 3.52 - 3.38 (m, 2H), 2.02 - 1.93 (m, 2H), 1.70 (s, 4H), 1.67 - 1.56 (m, 2H), 1.30 (d, J = 11.8 Hz, 12H).

### FS709:

### (2S,3R)-2-Amino-3-hydroxy-N-[6'-(5,5,8,8-tetramethyl-5,6,7,8-tetrahydro-naphthalen-2-yl)-3,4,5,6-tetrahydro-2H-[1,2']bipyridinyl-4-yl]-butyramide:

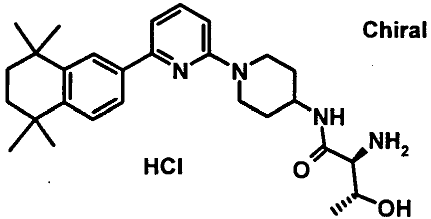

Die Herstellung erfolgt analog FS702.

Ausbeute: 274 mg, farbloses Öl. Rt. = 2,54 min (Methode A), LCMS: 465 (M+H). ¹H NMR (500 MHz, DMSO/deuteriertes TFA): δ 8.04 (dd, J = 9.1, 7.4 Hz, 1H), 7.70 (d, J = 1.6 Hz, 1 H), 7.58 - 7.49 (m, 2H), 7.37 (d, J = 9.1 Hz, 1 H), 7.15 (d, J = 7.3 Hz, 1 H), 4.21 (t, J = 13.1 Hz, 2H), 4.08 (ddd, J = 14.2, 9.7, 4.3 Hz, 1 H), 3.95 (p, J = 6.4 Hz, 1 H), 3.56 (d, J = 6.9 Hz, 1 H), 3.55 - 3.43 (m, 2H), 2.08 - 1.98 (m, 2H), 1.74 (s, 4H), 1.72 - 1.58 (m, 2H), 1.33 (d, J = 11.5 Hz, 12H), 1.22 (d, J = 6.3 Hz, 3H).

### FS710:

### 2-{Methyl-[6'-(5,5,8,8-tetramethyl-5,6,7,8-tetrahydro-naphthalen-2-yl)-3,4,5,6-tetrahydro-2H-[1,2']bipyridinyl-4-ylmethyl]-amino}-ethanol:

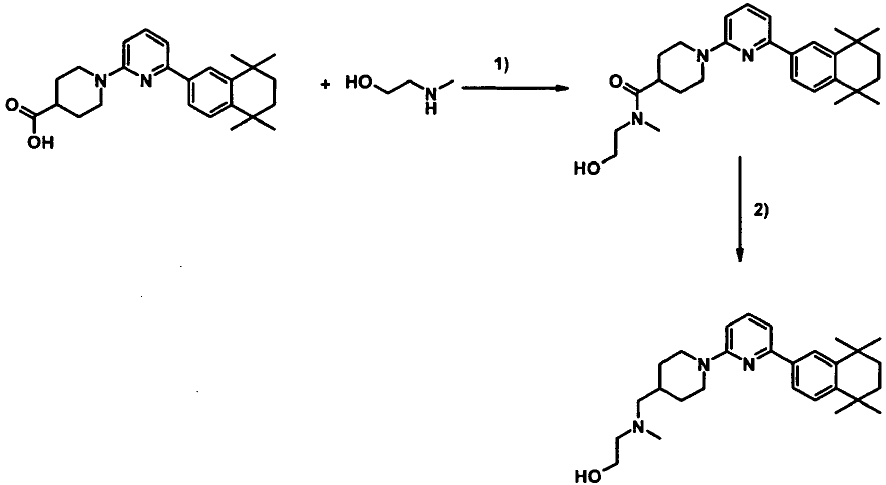

### Stufe 1:

Die Herstellung erfolgt analog FS702.

Ausbeute: 110 mg, farbloses Öl. Rt. = 2,80 min (Methode A), LCMS: 450 (M+H).

### Stufe 2:

Die Herstellung erfolgt analog FS311.

Ausbeute: 6 mg, farbloses Öl. Rt. = 2,56 min (Methode A), LCMS: 436 (M+H). ¹H NMR (500 MHz, DMSO/deuteriertes TFA): δ 8.06 - 8.00 (m, 1H), 7.69 (s, 1 H), 7.54 (q, J = 8.2 Hz, 2H), 7.37 (d, J = 9.0 Hz, 1 H), 7.13 (d, J = 7.3 Hz, 1 H), 4.34 (d, J = 13.4 Hz, 2H), 3.86 - 3.80 (m, 2H), 3.33 (dt, J = 27.7, 9.1 Hz, 3H), 3.21 (dd, J = 17.9, 10.6 Hz, 2H), 3.05 (dd, J = 13.1, 6.3 Hz, 1H), 2.91 (s, 3H), 2.35 - 2.21 (m, 1 H), 1.99 (dd, J = 39.5, 12.9 Hz, 2H), 1.74 (s, 4H), 1.50 - 1.37 (m, 2H), 1.33 (d, J = 11.3 Hz, 12H).

### FS711:

### 2-{[6'-(5,5,8,8-Tetramethyl-5,6,7,8-tetrahydro-naphthalen-2-yl)-3,4,5,6-tetrahydro-2H-[1,2']bipyridinyl-4-ylmethyl]-amino}-ethanol:

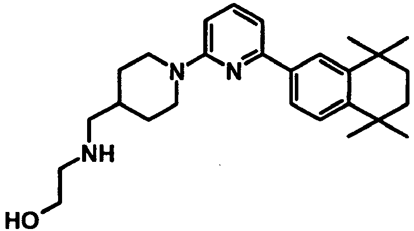

### Die Herstellung erfolgt analog FS702.

Ausbeute: 27 mg, farbloses Öl. Rt. = 2,52 min (Methode A), LCMS: 422 (M+H). ¹H NMR (500 MHz, DMSO/deuteriertes TFA): δ 7.95 (dd, J = 9.2, 7.4 Hz, 1H), 7.59 (d, J = 1.9 Hz, 1 H), 7.49 (d, J = 8.2 Hz, 1H), 7.43 (dd, J = 8.2, 1.9 Hz, 1H), 7.27 (d, J = 9.1 Hz, 1 H), 7.04 (d, J = 7.2 Hz, 1 H), 4.26 (d, J = 13.5 Hz, 2H), 3.73 - 3.66 (m, 2H), 3.23 (t, J = 11.8 Hz, 2H), 3.07 - 3.00 (m, 2H), 2.89 (d, J = 6.9 Hz, 2H), 2.12 (s, 1 H), 1.94 (d, J = 11.0 Hz, 2H), 1.68 (s, 4H), 1.45 - 1.16 (m, 14H).

### FS 801:

### 2-(5,5-Dimethyl-5,6,7,8-tetrahydro-naphthalen-2-yl)-4,4,5,5-tetramethyl-[1,3,2]dioxaborolane:

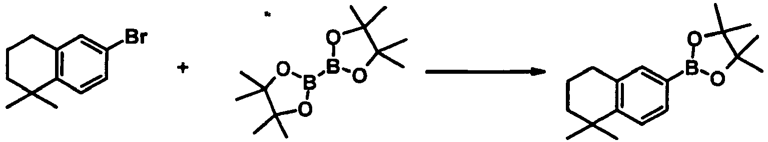

2 g (8.36 mmol) 6-Bromo-1,1-dimethyl-1,2,3,4-tetrahydro-naphthalin (Herstellung siehe Journal of Medicinal Chemistry, 1998, Vol. 41, 1476 - 1496) werden in 16 ml THF gelöst und mit 2.85 g (11.2 mmol) Bis(pinacolato)diboron sowie 2.46 g (25.1 mmol) Kaliumacetat versetzt. Das Reaktionsgemisch wird mehrfach entgast, unter Stickstoffatmosphäre mit 234 mg (0.33 mmol) Bis(triphenylphosphin)-palladium(II)-dichlorid versetzt und 20h bei 70°C gerührt. Das Reaktionsgemisch wird filtriert und mit 200 ml Ethylacetat nachgewaschen. Das Filtrat wird mit 50 ml Wasser gewaschen, über Na₂SO₄ getrocknet und eingeengt. Das Rohprodukt wird mittels Flashchromatographie an Kieselgel aufgereinigt.

1.53 g Öl, Rt. = 4.01 min (Methode B), LCMS: 287 (M+H).

### FS802:

### 2-(8,8-Dimethyl-5,6,7,8-tetrahydro-naphthalen-2-yl)-4,4,5,5-tetramethyl-[1,3,2]dioxaborolane:

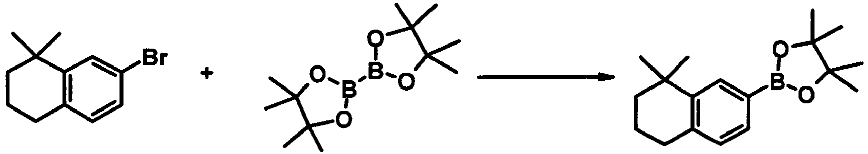

Die Herstellung erfolgt analog FS801 ausgehend von 7-Bromo-1,1-dimethyl-1,2,3,4-tetrahydro-naphthalene (Herstellung siehe WO2005/66115).

844 mg, gelbes Öl, Rt. = 3.91 min (Methode B), LCMS: 287 (M+H).

### FS803:

### 4,4,5,5-Tetramethyl-2-(1,1,3,3-tetramethyl-indan-5-yl)-[1,3,2]dioxaborolane:

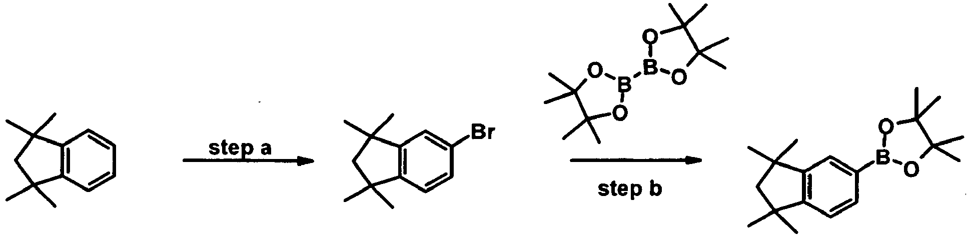

### Step a:

Die Bromierung von 1,1,3,3-Tetramethyl-indan (siehe US 2005/148590) erfolgt analog der Vorschrift in Organic Synthesis, Collective Vol. 3, S. 138.).

732 mg, gelbes Öl, Rt. = 3.97 min (Methode B).

### Step b:

Die Herstellung erfolgt analog FS801 ausgehend von 5-Bromo-1,1,3,3-tetramethyl-indan.

365 mg, gelbes Öl, Rt. = 4.07 min (Methode B).

¹H NMR (400 MHz, DMSO/deuteriertes TFA) δ 7.57 (dd, *J* = 7.5, 1.0, 1 H), 7.50 (s, 1 H), 7.17 (d, *J* = 7.2, 1 H), 1.90 (s, 2H), 1.31 (s, 12H), 1.30 (s, 6H), 1.28 (s, 6H).

### FS804

### 4-{(2-Hydroxy-ethyl)-[6'-(5,5,8,8-tetramethyl-5,6,7,8-tetrahydro-naphthalen-2-yl)-3,4,5,6-tetrahydro-2H-[1,2']bipyridinyl-4-yl]-amino}-butan-1-ol

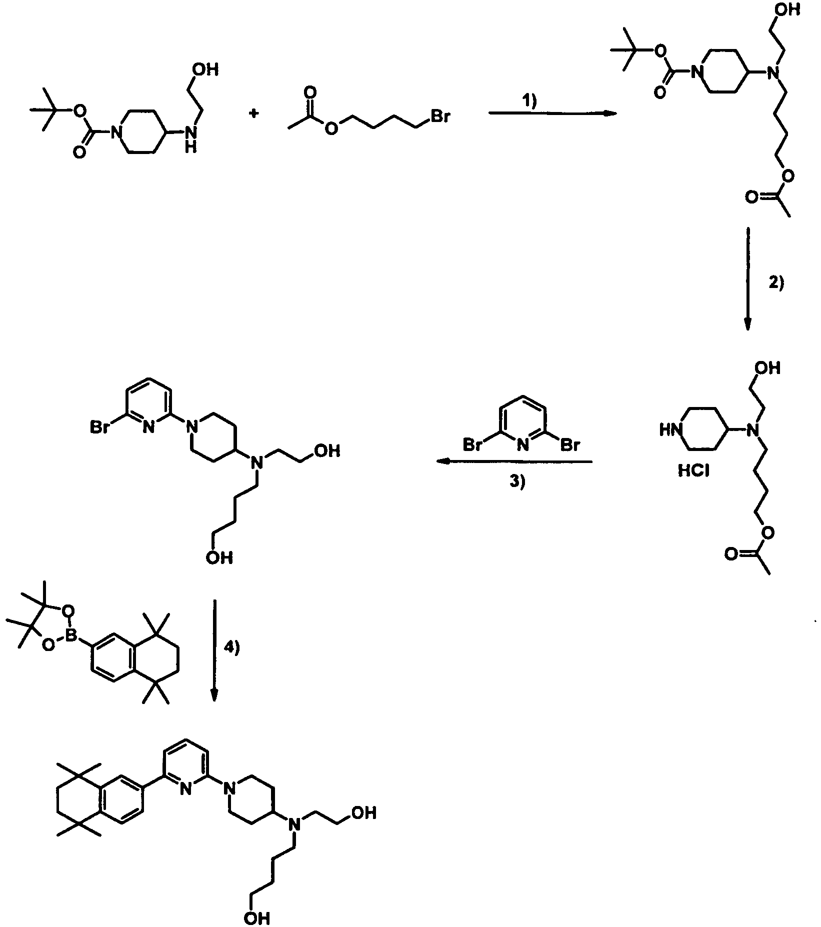

### Stufe 1:

400 mg (1,64 mmol) 4-(2-Hydroxy-ethylamino)-piperidine-1-carboxylic acid tert-butyl ester, 1.42 ml (9.82 mmol) 4-Brombutylacetat und 1.13 g (8.19 mmol) Kaliumcarbonat wurden in 50 ml Acetonitril suspendiert und 3 Tage bei 70°C gerührt. Das Reaktionsgemisch wird filtriert, der Rückstand mit Acetonitril gewaschen und das Filtrat wurde eingedampft. Der Rückstand wurde in 150 ml Dichlormethan gelöst, mit Wasser gewaschen, die wässrige Phase nochmals mit Dichlormethan extrahiert und die vereinigten organischen Phasen wurden getrocknet und eingedampft. Das Rohprodukt wird direkt weiter umgesetzt.

LCMS: 359 (M+H).

### Stufe 2:

Das Rohprodukt aus Stufe 1 wird in Dioxan gelöst und mit 4N HCl in Dioxan versetzt. Das Reaktionsgemisch wird 3 h bei Raumtemperatur gerührt, wobei sich ein öliger Niederschlag bildete. Der Überstand wird abdekantiert und der Rückstand im Vakuum getrocknet.

LCMS: 359 (M+H).

### Stufe 3:

117 mg (0.5 mmol) 2,6-Dibrompyridin, 146 mg (0.5 mmol) Acetic acid 4-[(2-hydroxy-ethyl)-piperidin-4-yl-amino]-butylester Hydrochlorid und 1981 mg (1.4 mmol) Kaliumcarbonat werden in 4 ml NMP suspendiert und 48 h bei 100°C gerührt. Das RG wurde in Wasser eingerührt, filtriert und das Filtrat wird mehrfach mit Ethylacetat extrahiert, mit Wasser gewaschen, getrocknet und eingedampft. Der Rückstand wird säulenchromatographisch an Kieselgel (Laufmittel Dichlormethan/Methanol) aufgereinigt.

38 mg, Rt. = 2,10 min (Methode A), LCMS: 374 (M+H).

### Stufe 4:

Die Umsetzung erfolgt analog FS102.

Ausbeute: 10 mg. Rt. = 2.76 min (Methode A), LCMS: 480 (M+H).

### FS901:

### Herstellung von 1-[6'-(5,5,8,8-Tetramethyl-5,6,7,8-tetrahydro-naphthalen-2-yl)-3,4,5,6-tetrahydro-2H-[1,2']bipyridinyl-4-yl]-pyrrolidin-3-ylamine:

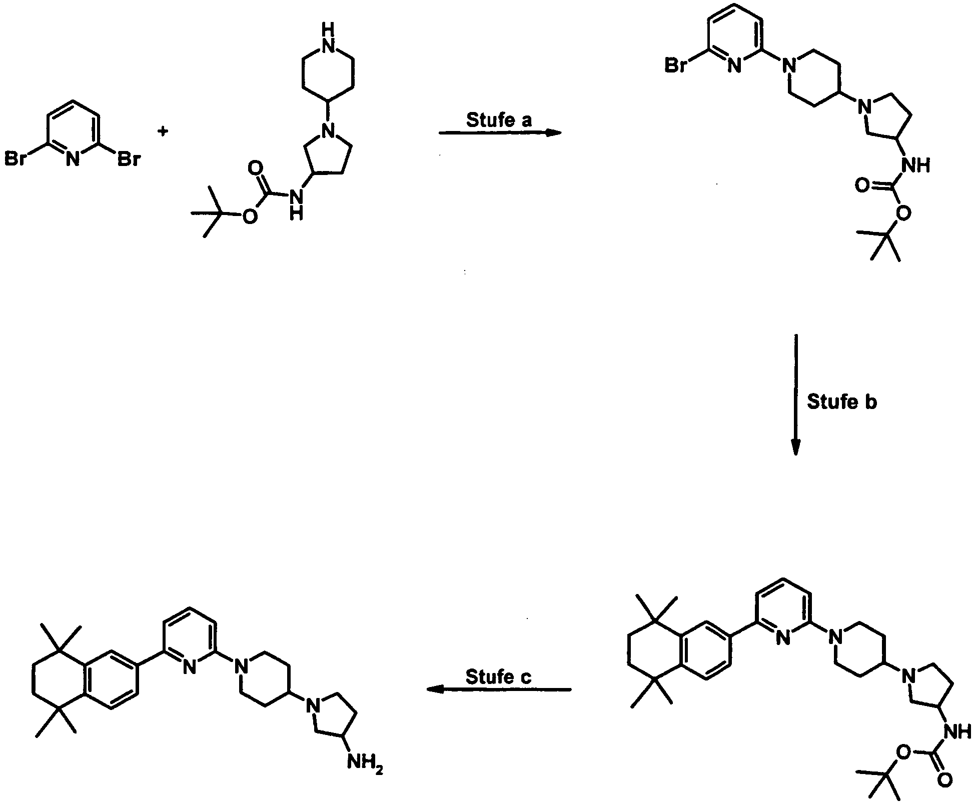

### Stufe a:

879 mg (3.71 mmol) 2,6-Dibromanilin, 1 g (3.71 mmol) (1-Piperidin-4-yl-pyrrolidin-3-yl)-carbamic acid tert-butyl ester und 3.65 g (26.4 mmol) Kaliumcarbonat werden in 20 ml DMSO suspendiert und für 15 h bei 120°C gerührt. Das Reaktionsgemisch wird mit Wasser versetzt, dreimal mit Ehylacetat extrahiert, über Na₂SO₄ getrocknet und eingeengt. Das Rohprodukt wird ohne weitere Aufreinigung weiter umgesetzt.

2.07 g, braunes Öl, Rt. = 2.52 min (Methode A), LCMS: 426 (M+H).

### Stufe b und c:

Die weitere Umsetzung erfolgt analog FS102 und FS201.

### Produkt:

40 mg, gelber Feststoff, Rt. = 2.64 min (Methode A), LCMS: 433 (M+H).

¹H NMR (500 MHz, DMSO/deuteriertes TFA) δ 7.96 (dd, *J* = 8.9, 7.5, 1 H), 7.66 (d, *J* = 1.7, 1H), 7.54 - 7.44 (m, 2H), 7.27 (d, *J* = 9.1, 1 H), 7.13 (d, *J* = 7.3, 1 H), 4.38 (d, *J* = 13.3, 2H), 4.09 - 3.91 (m, 1 H), 3.63 - 3.50 (m, 2H), 3.19 (t, *J* = 12.8, 2H), 2.25-2.16 (m, 2H), 1.77 (d, *J* = 12.1, 2H), 1.67 (s, 4H), 1.26 (d, *J* = 12.9, 12H).

### FS902:

### 6"-(5,5,8,8-Tetramethyl-5,6,7,8-tetrahydro-naphthalen-2-yl)-3,4,5,6,3',4',5',6'-octahydro-2H,2'H-[1,4';1',2"]terpyridin-3-ol:

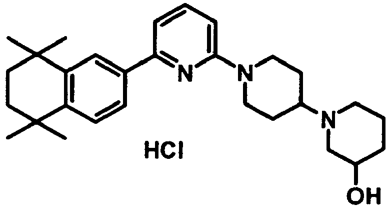

Die Herstellung erfolgt analog FS901 (Stufe a und b). Das Produkt liegt als Hydrochlorid vor. 45 mg, gelber Feststoff, Rt. = 2.81 min (Methode A), LCMS: 448 (M+H).

¹H NMR (500 MHz, DMSO/deuteriertes TFA) ö 7.91 (s, 1 H), 7.72 (dd, *J* = 8.3, 1.6, 1H), 7.64 - 7.59 (m, 1 H), 7.38 (d, *J* = 8.3, 1 H), 7.18 (d, *J* = 7.4, 1 H), 6.84 (dd, *J* = 8.4, 5.0, 1 H), 5.44 (s, 1 H), 4.62 - 4.53 (m, 2H), 4.08 (s, 2H), 3.75 - 3.66 (m, 1 H), 3.56 - 3.47 (m, 1H), 3.44 - 3.23 (m, 3H), 3.07 - 2.96 (m, 1 H), 2.91 - 2.79 (m, 2H), 2.19 - 1.84 (m, 3H), 1.76 - 1.53 (m, 8H), 1.29 (d, *J* = 17.3, 12H).

### FS903:

### 7-{6-[4-(5-Hydroxy-pentyl)-piperazin-1-yl]-pyridin-2-yl}-4,4-dimethyl-3,4-dihydro-2H-naphthalen-1-on:

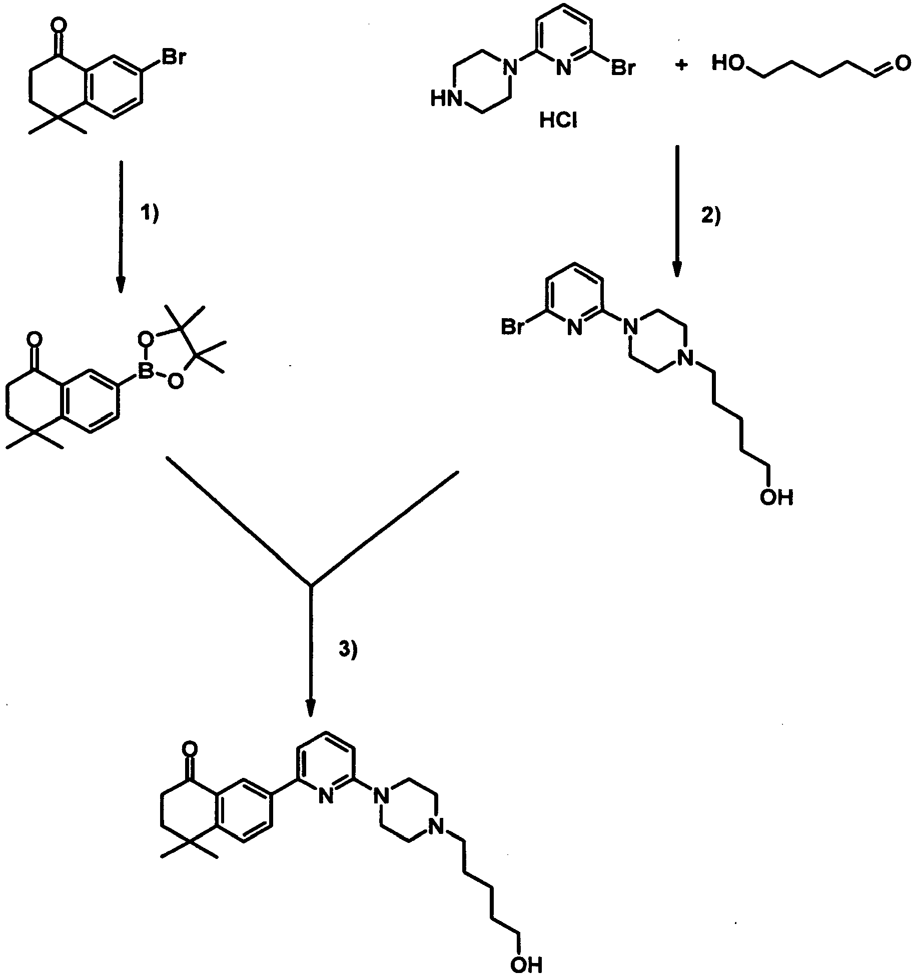

### Stufe 1:

597 mg (2.07 mmol) 7-Bromo-4,4-dimethyl-3,4-dihydro-2H-naphthalen-1-on, 685 mg (2.70 mmol) Bis(pinacolato)diboron und 611 mg (6.22 mmol) Kaliumacetat werden in 10 ml THF suspendiert, entgast und unter Stickstoffatmosphäre mit 58 mg (0.08 mmol) Bis(triphenylphosphin)-palladium(II)-chlorid versetzt. Das RG wird 18 h bei 70°C gerührt, auf RT abgekühlt, mit Wasser versetzt und mit Ethylacetat extrahiert. Das Rohprodukt wird mittels Säulenchromatographie an Kieselgel aufgereinigt.

Ausbeute: 570 mg, gelber Feststoff. Rt. = 3.46 min (Methode A), LCMS: 301 (M+H).

### Stufe 2:

Die Umsetzung erfolgt analog FS501. Ausbeute: 2.1 g, hellgelbes Öl. Rt. = 1.98 min (Methode A), LCMS: 328/330 (M+H).

### Stufe 3:

Die Umsetzung erfolgt analog FS102.

Ausbeute: 47 mg, hellgelbes Öl. Rt. = 2.55 min (Methode A), LCMS: 422 (M+H). ¹H NMR (400 MHz, DMSO/deuteriertes TFA) δ 8.40 (d, *J* = 2.0 Hz, 1 H), 8.12 (dd, *J* = 8.3, 2.1 Hz, 1 H), 7.77 (dd, *J* = 8.5, 7.6 Hz, 1 H), 7.59 (d, *J* = 8.3 Hz, 1 H), 7.26 (d, *J* = 7.4 Hz, 1 H), 7.01 (d, *J* = 8.7 Hz, 1 H), 4.50 (d, *J* = 13.7 Hz, 2H), 3.62 (d, *J* = 11.6 Hz, 2H), 3.48 - 3.29 (m, 4H), 3.22 - 3.04 (m, 4H), 1.96 (s, 2H), 1.83 -1.63 (m, 3H), 1.53 - 1.44 (m, 2H), 1.44 - 1.28 (m, 9H).

### FS904:

### 2-(4-{4-[6-(5,5,8,8-Tetramethyl-5,6,7,8-tetrahydro-naphthalen-2-yl)-pyridin-2-yl]-piperazin-1-yl}-piperidin-1-yl)-ethanol

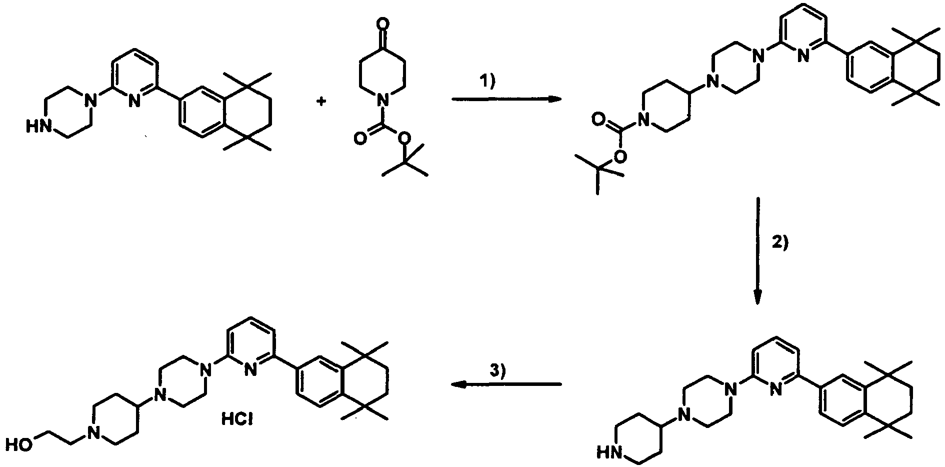

### Stufe 1:

Die reduktive Aminierung erfolgt analog FS501.

Ausbeute: 420 mg, farbloses Öl. Rt. = 3.32 min (Methode A), LCMS: 533 (M+H).

### Stufe 2:

Die Abspaltung der Boc-Schutzgruppe erfolgt analog FS201.

Ausbeute: 350 mg, Weißer Feststoff. Rt. = 2.78 min (Methode A), LCMS: 433 (M+H).

### Stufe 3:

Die Umsetzung erfolgt analog FS520. Das Produkt liegt als Hydrochlorid vor.

Ausbeute: 58 mg, weißer Feststoff. Rt. = 2.76 min (Methode A), LCMS: 477 (M+H).

¹H NMR (400 MHz, DMSO/deuteriertes TFA) δ 7.88 - 7.80 (m, 2H), 7.65 (dd, J = 8.2, 1.9 Hz, 1 H), 7.44 (d, J = 8.3 Hz, 1 H), 7.25 (d, J = 7.5 Hz, 1 H), 7.09 (d, J = 8.7 Hz, 1 H), 4.10 - 3.25 (m, 13H), 3.25 - 3.14 (m, 2H), 3.07 (t, J = 12.1 Hz, 2H), 2.41 - 2.30 (m, 2H), 2.17 - 2.02 (m, 2H), 1.68 (s, 4H), 1.28 (d, J = 13.9 Hz, 12H).

### FS905:

### 7-{6-[4-(5-Hydroxy-pentyl)-piperazin-1-yl]-pyridin-2-yl}-4,4-dimethyl-1,2,3,4-tetrahydro-naphthalen-1-ol

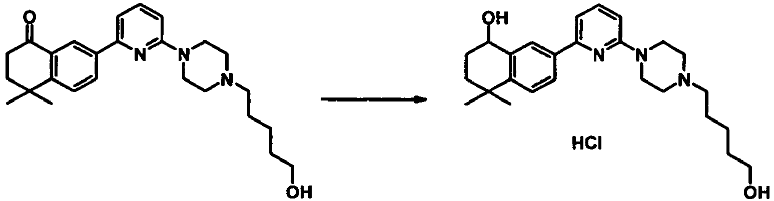

50 mg (0.12 mmol) 7-{6-[4-(5-Hydroxy-pentyl)-piperazin-1-yl]-pyridin-2-yl}-4,4-dimethyl-3,4-dihydro-2H-naphthalen-1-on werden in 0.5 ml THF und 1 ml Methanol gelöst, auf 0°C gekühlt und mit 4.5 mg (0.12 mmol) Natriumborhydrid versetzt. Das Reaktionsgemisch wird 30 min gerührt, mit Wasser versetzt und mit Ethylacetat extrahiert, getrocknet und eingedampft. Das Rohprodukt wird mittels präp HPLC aufgereinigt und anschließend mit methanolischer HCl ins Hydrochlorid überführt. Ausbeute: 39 mg, weißer Feststoff. Rt. = 2.45 min (Methode A), LCMS: 424 (M+H).

¹H NMR (400 MHz, DMSO/deuteriertes TFA) δ 7.94 (d, J = 1.8 Hz, 1 H), 7.87 (dd, J = 8.7, 7.5 Hz, 1 H), 7.71 (dd, J = 8.2, 2.1 Hz, 1 H), 7.44 (d, J = 8.3 Hz, 1 H), 7.23 (d, J = 7.4 Hz, 1 H), 7.11 (d, J = 8.7 Hz, 1 H), 4.62 (t, J = 6.0 Hz, 1 H), 4.50 (d, J = 12.6 Hz, 2H), 3.64 (d, J = 10.1 Hz, 2H), 3.45 (t, J = 6.2 Hz, 4H), 3.20 - 3.06 (m, 4H), 2.06 - 1.68 (m, 6H), 1.52 - 1.34 (m, 4H), 1.26 (d, J = 18.6 Hz, 6H).

### FS906:

### 7-{6-[4-(5-Hydroxy-pentyl)-piperazin-1-yl]-pyridin-2-yl}-1,4,4-trimethyl-1,2,3,4-tetrahydro-naphthalen-1-ol

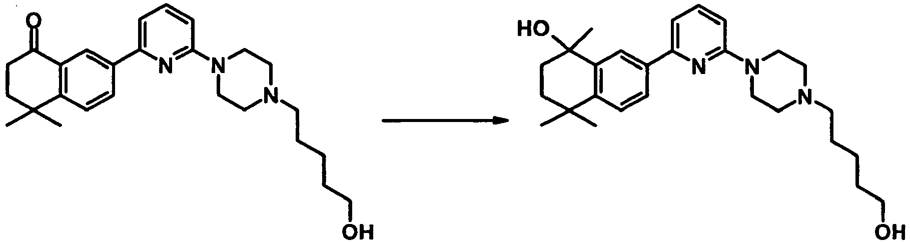

50 mg (0.12 mmol) 7-{6-[4-(5-Hydroxy-pentyl)-piperazin-1-yl]-pyridin-2-yl}-4,4-dimethyl-3,4-dihydro-2H-naphthalen-1-on werden in 5 ml THF gelöst, auf 0°C gekühlt und mit 224 µl (0.36 mmol) Methyllithium in Diethylether (5%ig) versetzt. Das Reaktionsgemisch wird 30 min bei 0°C gerührt und auf Raumtemperatur erwärmt. Das RG mit Wasser versetzt, mit Natriumhydrogencarbonatlösung versetzt und mit Ethylacetat extrahiert, getrocknet und eingedampft. Das Rohprodukt wird mittels Säulenchromatographie an Kieselgel aufgereinigt.

Ausbeute: 9 mg. Rt. = 2.53 min (Methode A), LCMS: 438 (M+H).

¹H NMR (400 MHz, DMSO/deuteriertes TFA) δ 8.06 (d, J = 2.0 Hz, 1 H), 7.88 - 7.81 (m, 1H), 7.69 (dd, J = 8.2, 2.1 Hz, 1 H), 7.41 (d, J = 8.3 Hz, 1 H), 7.23 (d, J = 7.4 Hz, 1 H), 7.10 (d, J = 8.8 Hz, 1 H), 4.50 (d, J = 13.8 Hz, 2H), 3.64 (d, J = 9.9 Hz, 2H), 3.52 - 3.35 (m, 4H), 3.25 - 3.06 (m, 4H), 2.12 - 1.64 (m, 6H), 1.55 - 1.34 (m, 7H), 1.26 (d, J = 7.4 Hz, 6H).

### FS907:

### 5-{4-[6-(8-Dimethylamino-5,5-dimethyl-5,6,7,8-tetrahydro-naphthalen-2-yl)-pyridin-2-yl]-piperazin-1-yl}-pentan-1-ol

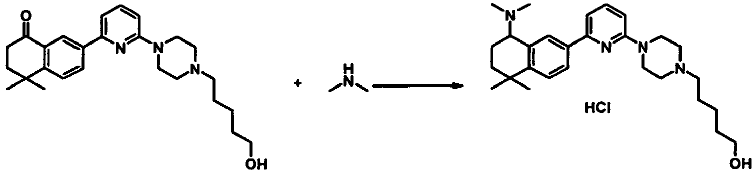

50 mg (0.12 mmol) 7-{6-[4-(5-Hydroxy-pentyl)-piperazin-1-yl]-pyridin-2-yl}-4,4-dimethyl-3,4-dihydro-2H-naphthalen-1-on werden in 2 ml 2M Dimethylamin in THF gelöst, und mit 15 mg Natriumcyanoborhydrid und 38 mg Titan(IV)-isopropylat versetzt und in einem Druckgefäß bei 80°C gerührt. Das RG mit Wasser versetzt, mit Natriumhydrogencarbonatlösung versetzt und mit Ethylacetat extrahiert, getrocknet und eingedampft. Das Rohprodukt wird mittels präp HPLC aufgereinigt. Ausbeute: 5 mg. Rt. = 2.14 min (Methode A), LCMS: 451 (M+H).

¹H NMR (500 MHz, DMSO/deuteriertes TFA) ö 8.12 (d, J = 1.3 Hz, 1 H), 7.99 (dd, J = 8.3, 1.6 Hz, 1 H), 7.80 - 7.74 (m, 1 H), 7.57 (d, J = 8.4 Hz, 1 H), 7.31 (d, J = 7.5 Hz, 1 H), 6.98 (d, J = 8.6 Hz, 1 H), 4.77 (t, J = 7.0 Hz, 1 H), 4.53 (d, J = 14.1 Hz, 2H), 3.60 (d, J = 11.8 Hz, 2H), 3.48 - 3.42 (m, 2H), 3.33 (t, J = 12.6 Hz, 2H), 3.18 - 3.06 (m, 4H), 2.94 (s, 3H), 2.57 (s, 3H), 2.18 - 2.07 (m, 2H), 1.87 - 1.60 (m, 4H), 1.52-1.44 (m, 2H), 1.44 - 1.34 (m, 2H), 1.28 (d, J = 37.6 Hz, 6H).

### FS1001:

### Amino-acetic acid 5-{4-[3-(5,5,8,8-tetramethyl-5,6,7,8-tetrahydro- naphthalen-2-yl)-phenyl]-piperazin-1-yl}-pentyl ester

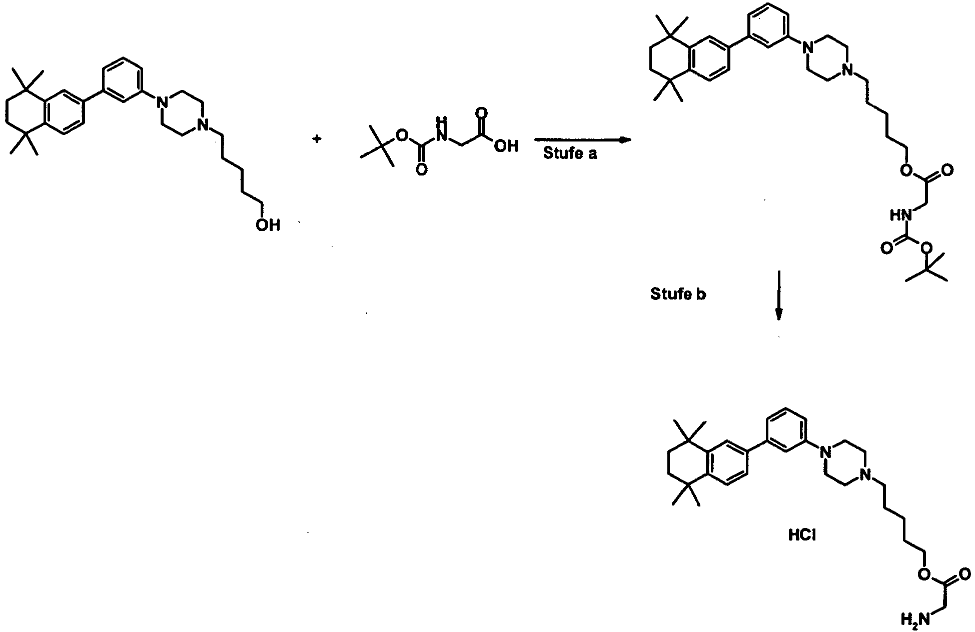

### Stufe a:

40 mg (0.09 mmol) 5-{4-[3-(5,5,8,8-Tetramethyl-5,6,7,8-tetrahydro-naphthalen-2- yl)-phenyl]-piperazin-1-yl}-pentan-1-ol und 64 mg (0.37 mmol) Boc-Gly-OH werden in 5 ml THF und 1 ml DCM gelöst und mit 38 mg (0.18 mmol) DCC sowie 1.2 mg DMAP versetzt. Das Reaktionsgemisch wird 1 h bei RT gerührt und anschließend zum Rückstand eingeengt. Das Rohgemisch wird ohne weitere Aufreinigung weiter umgesetzt.

### Stufe b:

Die Abspaltung der Schutzgruppe erfolgt analog FS201. Das Produkt liegt als Hydrochlorid vor.

52 mg, gelber Feststoff, Rt. = 2.94 min (Methode A), LCMS: 492 (M+H).

### FS1002:

### 2-(2-{4-[6-(5,5,8,8-Tetramethyl-5,6,7,8-tetrahydro-naphthalen-2-yl)-pyridin-2-yl]-piperazin-1-yl}-ethylamino)-ethanol

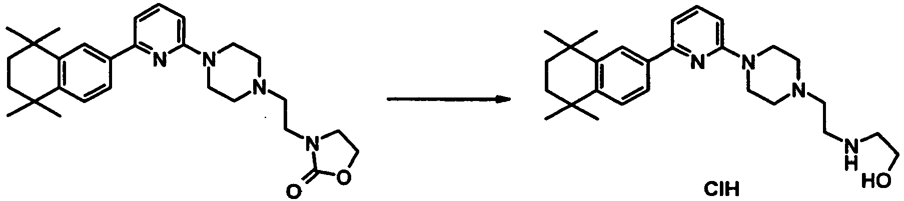

70 mg (0.15 mmol) 3-(2-{4-[6-(5,5,8,8-Tetramethyl-5,6,7,8-tetrahydro-naphthalen-2-yl)-pyridin-2-yl]-piperazin-1-yl}-ethyl)-oxazolidin-2-one wurden in 2 ml Methanol und 450 µl 1 N NaOH versetzt und 3 Tage refluxiert. Das Reaktionsgemisch wurde eingedampft, mit Ethylacetat extrahiert, getrocknet und eingedampft. Das Rohprodukt wurde mittel päp. HPLC aufgereinigt.

Ausbeute: 50 mg. Rt. = 2.68 min (Methode A), LCMS: 437 (M+H).

¹H NMR (500 MHz, DMSO/deuteriertes TFA) ö 7.87 - 7.80 (m, 2H), 7.67 (dd, J = 8.2, 1.7 Hz, 1 H), 7.55 - 7.48 (m, 1 H), 7.43 (d, J = 8.3 Hz, 1 H), 7.27 (d, J = 7.5 Hz, 1 H), 7.09 (d, J = 8.7 Hz, 1 H), 3.74 - 3.69 (m, 2H), 3.61 - 3.38 (m, 4H), 4.3 - 3.8 (b, 6H), 3.25 (t, J = 12.2 Hz, 1 H), 3.15 - 3.08 (m, 2H), 1.77 - 1.62 (m, 4H), 1.36 - 1.22 (m, 12H).

### FS1003

### 5-{4-[5-(2-Amino-ethoxy)-6-(5,5,8,8-tetramethyl-5,6,7,8-tetrahydro-naphthalen-2-yl)-pyridin-2-yl]-piperazin-1-yl}-pentan-1-ol:

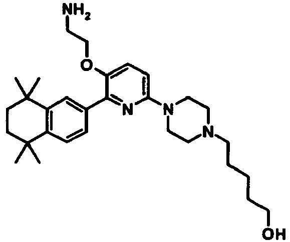

74 mg (0.09 mmol) 2-{2-[6-[4-(5-Hydroxy-pentyl)-piperazin-1-yl]-2-(5,5,8,8-tetramethyl-5,6,7,8-tetrahydro-naphthalen-2-yl)-pyridin-3-yloxy]-ethyl}-isoindole-1,3-dion werden in 2 ml Ethanol gelöst und mit 9 µl Hydrazin Hydrat versetzt. Das Reaktionsgemisch wird 15 h bei Raumtemperatur gerührt. Es bildet sich ein Niederschlag, der abgesaugt, mit Ethanol gewaschen und mittels Säulenchromatographie an Kieselgel aufgereinigt wird. Das Produkt wird mit methanolischer HCl ins Hydrochlorid überführt.

Ausbeute: 21 mg, beigefarbener Feststoff. Rt. = 2.38 min (Methode A), LCMS: 495 (M+H).

¹H NMR (500 MHz, DMSO/deuteriertes TFA) δ 7.83 (s, 1H), 7.68 (d, J = 9.1 Hz, 1 H), 7.62 (d, J = 8.3 Hz, 1 H), 7.36 (d, J = 8.3 Hz, 1 H), 6.99 (d, J = 9.2 Hz, 1 H), 4.32 (d, J = 13.5 Hz, 2H), 4.12 (t, J = 5.3 Hz, 2H), 3.58 (d, J = 11.7 Hz, 2H), 3.42 (t, J = 6.3 Hz, 2H), 3.24 (t, J = 12.4 Hz, 2H), 3.18 - 3.05 (m, 6H), 1.77 - 1.61 (m, 6H), 1.50 - 1.41 (m, 2H), 1.35 (dt, J = 14.9, 7.5 Hz, 2H), 1.25 (d, J = 9.0 Hz, 12H).

### FS1004:

### 4-{(4-Amino-butyl)-[6'-(5,5,8,8-tetramethyl-5,6,7,8-tetrahydro-naphthalen-2-yl)-3,4,5,6-tetrahydro-2H-[1,2']bipyridinyl-4-yl]-amino}-butan-1-ol

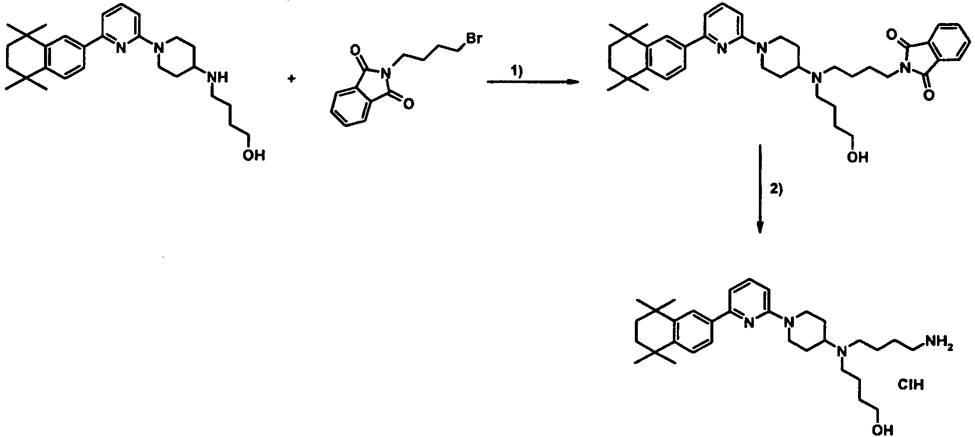

### Stufe 1:

Die Umsetzung erfolgt analog FS314.

Ausbeute: 48 mg. Rt. = 3.04 min (Methode A), LCMS: 637 (M+H).

### Stufe 3:

Die Umsetzung erfolgt analog FS1002.

Ausbeute: 13 mg. Rt. = 2.49 min (Methode A), LCMS: 507 (M+H).

¹H NMR (400 MHz, DMSO/deuteriertes TFA) δ 7.98 (dd, J = 9.0, 7.4 Hz, 1 H), 7.67 (s, 1 H), 7.54 - 7.48 (m, 2H), 7.31 (d, J = 9.1 Hz, 1H), 7.12 (d, J = 7.3 Hz, 1 H), 4.45 (d, J = 13.4 Hz, 2H), 3.76 - 3.66 (m, 1 H), 3.48 (t, J = 6.0 Hz, 2H), 3.33 - 3.16 (m, 4H), 3.12 - 2.97 (m, 2H), 2.86 (t, J = 7.4 Hz, 2H), 2.19 (d, J = 10.3 Hz, 2H), 1.96-1.84 (m, 2H), 1.84 - 1.73 (m, 4H), 1.69 (s, 4H), 1.68 - 1.58 (m, 2H), 1.56 - 1.46 (m, 2H), 1.29 (d, J = 12.2 Hz, 12H).

### FS1005

### Amino-acetic acid 5-{4-[6-(5,5,8,8-tetramethyl-5,6,7,8-tetrahydro-naphthalen-2-yl)-pyridin-2-yl]-piperazin-1-yl}-pentyl ester:

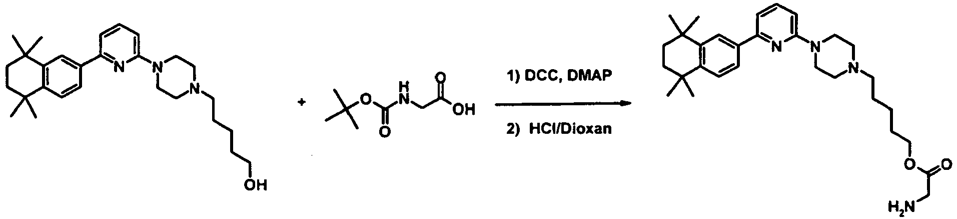

### Stufe 1:

30 mg (0.069 mmol) 5-{4-[6-(5,5,8,8-Tetramethyl-5,6,7,8-tetrahydro-naphthalen-2-yl)-pyridin-2-yl]-piperazin-1-yl}-pentan-1-ol, 48 mg (0.276 mmol) Boc-geschütztes Glycin und 1 mg DMAP werden in 2 ml THF und 0.5 ml Dichlormethan gelöst, mit 28 mg (0.138 mmol) DCC versetzt und 18 h bei Raumtemperatur gerührt. Das Reaktionsgemisch wird mit Ethylacetat verdünnt, mit ges. Natriumhydrogencarbonatlösung und 1 N HCl extrahiert, getrocknet und eingedampft. Das Produkt wird ohne weitere Aufreinigung direkt weiter umgesetzt Stufe 2:

Die Abspaltung der Schutzgruppe erfolgt analog FS201. Das Produkt liegt als Hydrochlorid vor.

Ausbeute: 34 mg. Rt. = 2.86 min (Methode A), LCMS: 493 (M+H).

¹H NMR (500 MHz, DMSO/deuteriertes TFA) δ 7.47 (d, J = 1.5 Hz, 1H), 7.38 - 7.30 (m, 2H), 7.27 (dd, J = 8.4, 2.0 Hz, 1 H), 7.13 (d, J = 1.9 Hz, 1 H), 7.05 (d, J = 8.5 Hz, 1 H), 3.85 (s, 3H), 3.64 (dd, J = 34.9, 11.7 Hz, 4H), 3.45 (t, J = 6.3 Hz, 2H), 3.31 - 3.21 (m, 2H), 3.21 - 3.14 (m, 2H), 3.14 - 3.03 (m, 2H), 1.77 - 1.69 (m, 2H), 1.68 (s, 4H), 1.54 - 1.46 (m, 2H), 1.44 -1.34 (m, 2H), 1.28 (d, J = 19.2 Hz, 12H).

### FS1006

### 6'-(5,5,8,8-Tetramethyl-5,6,7,8-tetrahydro-naphthalen-2-yl)-2,3,5,6-tetrahydro-[1,2']bipyridinyl-4-one

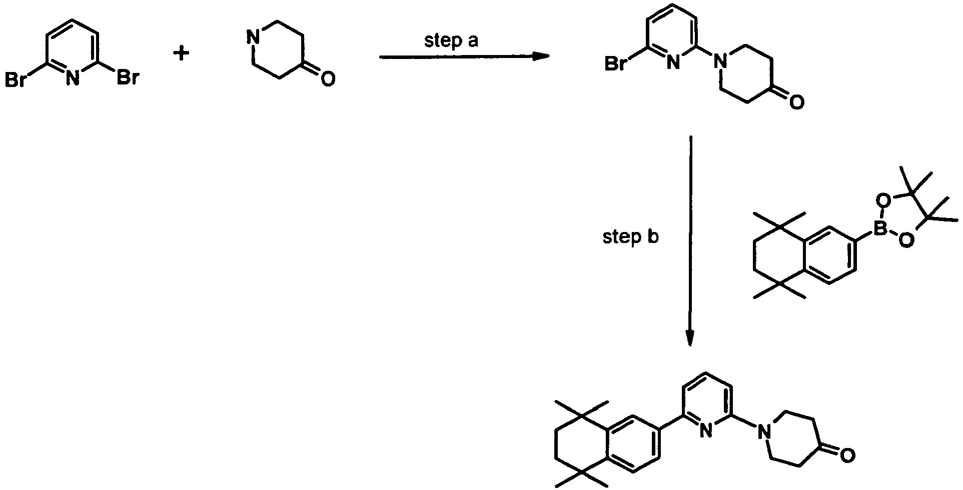

### Stufe a:

### 6'-Bromo-2,3,5,6-tetrahydro-[1,2']bipyridinyl-4-one

5 g (21.11 mmol) 2,6-Dibrompyridin, 2.09 g (21.11 mmol) Piridin-4-on und 7.29 g Kaliumcarbonat (52.77 mmol) werden in 30 mL DMSO suspendiert und über Nacht bei 120°C gerührt. Das Gemisch wird dann mit Wasser versetzt und mit EA extrahiert. Die organische Phase wird mit einer gesättigten Natriumchloridlösung gewaschen, über Natriumsulfat getrocknet und zum Rückstand eingeengt. Der entstandene Rückstand wird mittels Flashchromatographie an Kieselgel aufgereinigt.

2.58 g, gelbes Öl, Rt. = 2.64 min (Methode A), LCMS: 255 (M+H).

### Stufe b:

### 6'-(5,5,8,8-Tetramethyl-5,6,7,8-tetrahydro-naphthalen-2-yl)-2,3,5,6-tetrahydro-[1,2']bipyridinyl-4-one

Die Herstellung erfolgt analog FS102 ausgehend vom Produkt aus Stufe a (1 g, 3.92 mmol) und 4,4,5,5-Tetramethyl-2-(5,5,8,8-tetramethyl-5,6,7,8-tetrahydro-naphthalen-2-yl)-[1,3,2]dioxaborolane (1.36 g, 4.31 mmol).

1.04 g, gelbes Öl, Rt. = 3.22 min (Methode A), LCMS: 363 (M+H).

### FS1007

### (1R,2S,3R)-3-[6'-(5,5,8,8-Tetramethyl-5,6,7,8-tetrahydro-naphthalen-2-yl)-3,4,5,6-tetrahydro-2H-[1,2']bipyridinyl-4-ylamino]-cyclopentane-1,2-diol

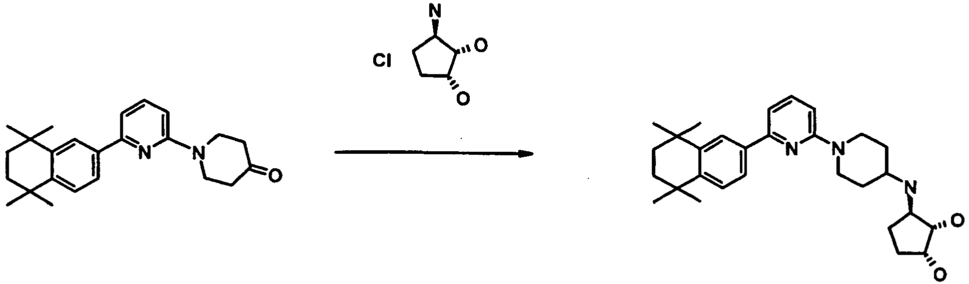

31 mg (0.199 mmol) (1R,2S,3R)-3-Amino-cyclopentane-1,2-diol werden in 3.5 mL THF und 2 mL DMF gelöst und mit 33.8 µL DIPEA versetzt. 80 mg (0.199 mmol) 6'-(5,5,8,8-Tetramethyl-5,6,7,8-tetrahydro-naphthalen-2-yl)-2,3,5,6-tetrahydro-[1,2']bipyridinyl-4-one werden zugegeben. Das Gemisch wird 30 min bei Raumtemperatur gerührt, mit 23 µl (0.398 mmol) Eisessig versetzt und weitere 10 min gerührt. Anschließend werden 89 mg (0.398 mmol) Natriumtriacetoxyborhydrid zugegeben. Das Reaktionsgemisch wird über Nacht bei Raumtemperatur gerührt, mit einer konzentrierten Natriumhydrogencarbonatlösung versetzt und 2 Mal mit EA extrahiert. Die organische Phase wird über Natriumsulfat getrocknet, filtriert und eingeengt. Der Rückstand wird mittels präparativer HPLC aufgereinigt. Das Produkt liegt als Hydrochlorid vor.

46 mg, weißer Feststoff, Rt. = 2.54 min (Methode B), LCMS: 464 (M+H). ¹H NMR (500 MHz, DMSO/ deuteriertes TFA) δ = 8.09 - 8.02 (m, 1 H), 7.72 (s, 1H), 7.56 (s, 2H), 7.36 (dd, *J*=9.1, 3.1, 1 H), 7.18 (dd, *J*=7.3, 3.5, 1H), 4.44 - 4.35 (m, 2H), 4.09 - 3.96 (m, 2H), 3.70 - 3.43 (m, 2H), 3.38 - 3.25 (m, 2H), 2.37 - 2.22 (m, 2H), 2.07 - 1.58 (m, 10H), 1.34 (d, *J*=12.6, 12H).

### FS1008

### (1S,2R,3S)-3-[6'-(5,5,8,8-Tetramethyl-5,6,7,8-tetrahydro-naphthalen-2-yl)-3,4,5,6-tetrahydro-2H-[1,2']bipyridinyl-4-ylamino]-cyclopentane-1,2-diol

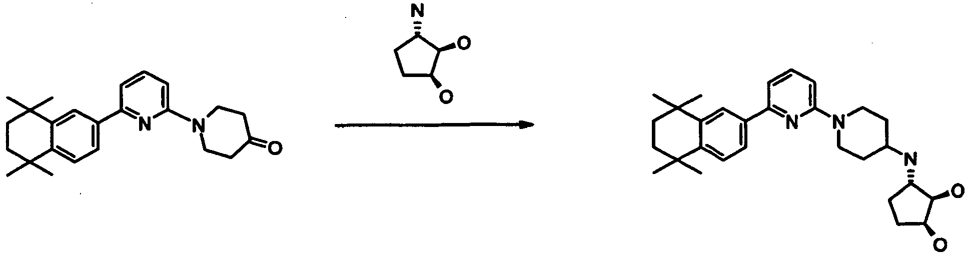

Die Herstellung erfolgt analog FS1007 ohne Zugabe von DIPEA ausgehend von 23 mg (0.199 mmol) (1S,2R,3S)-3-Amino-cyclopentane-1,2-diol und 80 mg (0.199 mmol) 6'-(5,5,8,8-Tetramethyl-5,6,7,8-tetrahydro-naphthalen-2-yl)-2,3,5,6-tetrahydro-[1,2']bipyridinyl-4-one. Das Produkt liegt als Hydrochlorid vor. Ausbeute: 46 mg, weißer Feststoff, Rt. = 2.49 min (Methode B), LCMS: 464 (M+H).

¹H NMR (500 MHz, DMSO/ deuteriertes TFA) δ = 7.91 (d, *J*=1.8, 1 H), 7.71 (dd, *J*=8.2, 1.8, 1 H), 7.65 (t, *J*=8.0, 1H), 7.40 (d, *J*=8.3, 1 H), 7.17 (d, *J*=7.4, 1 H), 6.88 (d, *J*=8.5, 1H), 4.50 (d, *J*=12.7, 2H), 4.01 - 3.89 (m, 2H), 2.92 (dd, *J*=27.3, 13.3, 2H), 2.22 - 2.07 (m, 3H), 1.95 - 1.84 (m, 1 H), 1.68 (s, 4H), 1.65 - 1.50 (m, 4H), 1.29 (d, *J*=17.3, 12H).

### FS1009

### (1S,2R,3R)-3-[6'-(5,5,8,8-Tetramethyl-5,6,7,8-tetrahydro-naphthalen-2-yl)-3,4,5,6-tetrahydro-2H-[1,2']bipyridinyl-4-ylamino]-cyclopentane-1,2-diol

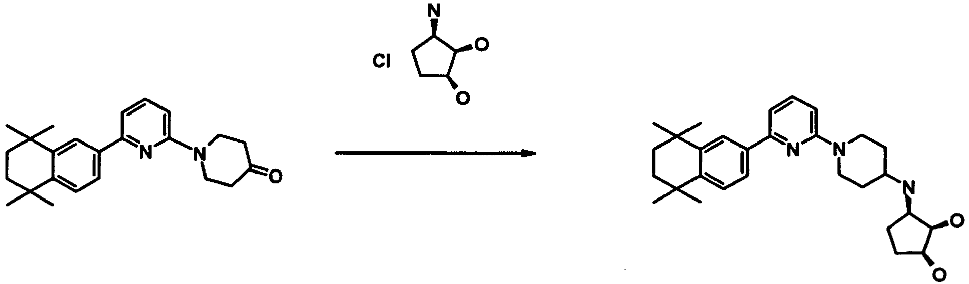

Die Herstellung erfolgt analog FS1007 ausgehend von 31 mg (0.199 mmol) (1S,2R,3R)-3-Amino-cyclopentane-1,2-diol und 80 mg (0.199 mmol) 6'-(5,5,8,8-Tetramethyl-5,6,7,8-tetrahydro-naphthalen-2-yl)-2,3,5,6-tetrahydro-[1,2']bipyridinyl-4-one. Das Produkt liegt als Hydrochlorid vor.

Ausbeute: 52 mg, weißer Feststoff, Rt. = 2.54 min (Methode B), LCMS: 464 (M+H).

¹H NMR (400 MHz, DMSO/ deuteriertes TFA) δ = 8.04 (dd, *J*=9.1, 7.4, 1 H), 7.72 (s, 1H), 7.55 (s, 2H), 7.35 (d, *J*=9.0, 1 H), 7.18 (d, *J*=7.2, 1 H), 4.39 (d, *J*=13.4, 2H), 4.09 - 4.01 (m, 2H), 3.70 - 3.61 (m, 1 H), 3.54 - 3.43 (m, 1 H), 3.30 (t, *J*=12.0, 2H), 2.29 (dd, *J*=28.5, 11.2, 2H), 2.06 - 1.70 (m, 10H), 1.33 (d, *J*=10.2, 12H).

### FS1010

### (R)-4-[6'-(5,5,8,8-Tetramethyl-5,6,7,8-tetrahydro-naphthalen-2-yl)-3,4,5,6-tetrahydro-2H-[1,2']bipyridinyl-4-ylamino]-butane-1,2-diol

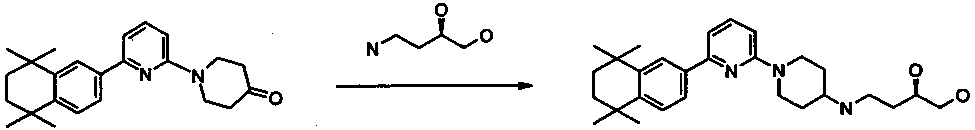

Die Herstellung erfolgt analog FS1008 ausgehend von 21 mg (0.199 mmol) (R)-4-Amino-butane-1,2-diol und 80 mg (0.199 mmol) 6'-(5,5,8,8-Tetramethyl-5,6,7,8-tetrahydro-naphthalen-2-yl)-2,3,5,6-tetrahydro-[1,2']bipyridinyl-4-one. Das Produkt liegt als Hydrochlorid vor.

Ausbeute: 29 mg, Harz, Rt. = 2.43 min (Methode B), LCMS: 452 (M+H).

¹H NMR (400 MHz, DMSO/ deuteriertes TFA) δ = 8.04 (dd, *J*=9.0, 7.4, 1 H), 7.72 (s, 1H), 7.60 - 7.52 (m, 2H), 7.35 (d, *J*=9.1, 1 H), 7.19 (d, *J*=7.3, 1 H), 4.40 (d, *J*=13.8, 2H), 3.70 - 3.60 (m, 1H), 3.55 - 3.42 (m, 2H), 3.41 - 3.26 (m, 3H), 3.22 - 3.08 (m, 2H), 2.24 (d, *J*=10.3, 2H), 1.97 - 1.86 (m, 1 H), 1.84 - 1.66 (m, 7H), 1.34 (d, *J*=10.4, 12H).

### FS1011

### Herstellung von (S)-4-[6'-(5,5,8,8-Tetramethyl-5,6,7,8-tetrahydro-naphthalen-2-yl)-3,4,5,6-tetrahydro-2H-[1,2']bipyridinyl-4-ylamino]-butane-1,2-diol

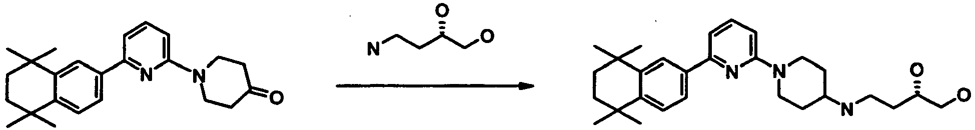

Die Herstellung erfolgt analog FS1008 ausgehend von 21 mg (0.199 mmol) (S)-4-Amino-butane-1,2-diol und 80 mg (0.199 mmol) 6'-(5,5,8,8-Tetramethyl-5,6,7,8-tetrahydro-naphthalen-2-yl)-2,3,5,6-tetrahydro-[1,2']bipyridinyl-4-one. Das Produkt liegt als Hydrochlorid vor.

Ausbeute: 40 mg, Harz, Rt. = 2.42 min (Methode B), LCMS: 452 (M+H).

¹H NMR (400 MHz, DMSO/ deuteriertes TFA) δ = 8.05 (dd, *J*=9.1, 7.4, 1 H), 7.69 (d, *J*=1.7, 1 H), 7.60 - 7.50 (m, 2H), 7.34 (d, *J*=9.0, 1H), 7.16 (d, *J*=7.2, 1H), 4.43 - 4.34 (m, 2H), 3.73 - 3.66 (m, 1 H), 3.52 - 3.41 (m, 2H), 3.39 - 3.28 (m, 2H), 3.24 - 3.12 (m, 2H), 2.26 (d, *J*=12.1, 2H), 1.98 - 1.88 (m, 1 H), 1.86 - 1.78 (m, 2H), 1.75 (s, 4H), 1.34 (d, *J*=9.3, 12H).

### FS1012

### ((3aS,4R,7aR)-2,2-Dimethyl-hexahydro-benzo[1,3]dioxol-4-yl)-[6'-(5,5,8,8-tetramethyl-5,6,7,8-tetrahydro-naphthalen-2-yl)-3,4,5,6-tetrahydro-2H-[1,2']bipyridinyl-4-yl]-amine

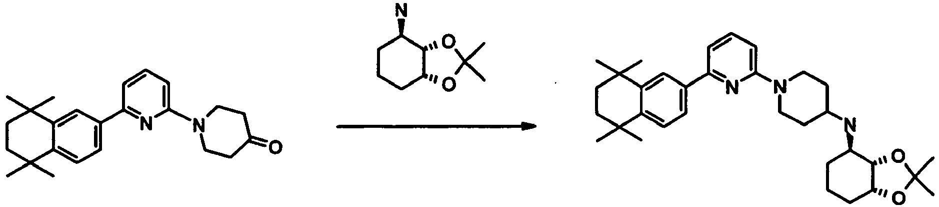

Die Herstellung erfolgt analog FS1008 in THF ausgehend von 34 mg (0.199 mmol) (3aS,4R,7aR)-2,2-Dimethyl-hexahydro-benzo[1,3]dioxol-4-ylamine und 80 mg (0.199 mmol) 6'-(5,5,8,8-Tetramethyl-5,6,7,8-tetrahydro-naphthalen-2-yl)-2,3,5,6-tetrahydro-[1,2']bipyridinyl-4-one. Die Aufreinigung erfolgt mittels Flashchromatographie an Kieselgel.

Ausbeute: 91 mg, gelbes Harz, Rt. = 3.09 min (Methode B), LCMS: 518 (M+H).

### FS1013

### (1R,2S,3R)-3-[6'-(5,5,8,8-Tetramethyl-5,6,7,8-tetrahydro-naphthalen-2-yl)-3,4,5,6-tetrahydro-2H-[1,2']bipyridinyl-4-ylamino]-cyclohexane-1,2-diol

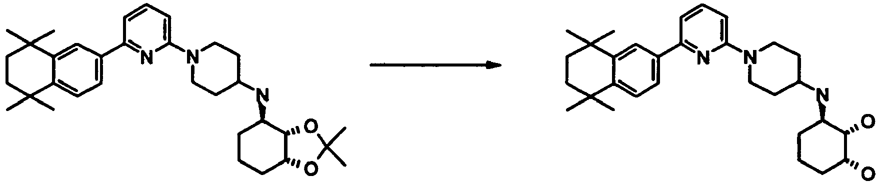

91 mg ((3aS,4R,7aR)-2,2-Dimethyl-hexahydro-benzo[1,3]dioxol-4-yl)-[6'-(5,5,8,8-tetramethyl-5,6,7,8-tetrahydro-naphthalen-2-yl)-3,4,5,6-tetrahydro-2H-[1,2'jbipyridinyl-4-yl]-amine werden mit 10 mL 1.25 N HCl in Methanol versetzt und über Nacht bei Raumtemperatur gerührt. Das Reaktionsgemisch wird eingeengt und unter Hochvakuum getrocknet. Das Produkt liegt als Hydrochlorid vor.

Ausbeute: 106 mg, heller Feststoff, Rt. = 2.61 min (Methode B), LCMS: 478 (M+H).

¹H NMR (500 MHz, DMSO/ deuteriertes TFA) δ = 8.05 (dd, *J*=9.0, 7.4, 1 H), 7.72 (s, 1H), 7.56 (s, 2H), 7.36 (d, *J*=9.1, 1 H), 7.19 (d, *J*=7.3, 1H), 4.41 (d, *J*=13.4, 2H), 3.99 (d, *J*=2.4, 1 H), 3.73 - 3.62 (m, 1 H), 3.54 - 3.49 (m, 1 H), 3.37 - 3.25 (m, 3H), 2.26 - 2.08 (m, 3H), 2.05 - 1.93 (m, 1 H), 1.86 - 1.64 (m, 7H), 1.56 - 1.39 (m, 3H), 1.34 (d, *J*=13.0, 12H).

### FS1014

### ((3aR,4S,7aS)-2,2-Dimethyl-hexahydro-benzo[1,3]dioxol-4-yl)-[6'-(5,5,8,8-tetramethyl-5,6,7,8-tetrahydro-naphthalen-2-yl)-3,4,5,6-tetrahydro-2H-[1,2']bipyridinyl-4-yl]-amine

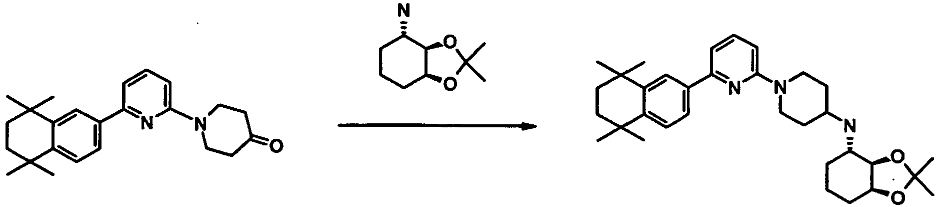

Die Herstellung erfolgt analog FS1008 in THF ausgehend von 62 mg (0.360 mmol) (3aR,4S,7aS)-2,2-Dimethyl-hexahydro-benzo[1,3]dioxol-4-ylamine und 145 mg (0.360 mmol) 6'-(5,5,8,8-Tetramethyl-5,6,7,8-tetrahydro-naphthalen-2-yl)-2,3,5,6-tetrahydro-[1,2']bipyridinyl-4-one. Die Aufreinigung erfolgt mittels Flashchromatographie an Kieselgel.

Ausbeute: 168 mg, farbloses Harz, Rt. = 3.58 min (Methode B), LCMS: 518 (M+H).

### FS1015

### (1S,2R,3S)-3-[6'-(5,5,8,8-Tetramethyl-5,6,7,8-tetrahydro-naphthalen-2-yl)-3,4,5,6-tetrahydro-2H-[1,2']bipyridinyl-4-ylamino]-cyclohexane-1,2-diol

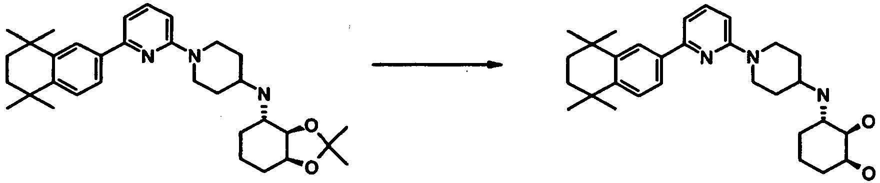

168 mg ((3aR,4S,7aS)-2,2-Dimethyl-hexahydro-benzo[1,3]dioxol-4-yl)-[6'-(5,5,8,8-tetramethyl-5,6,7,8-tetrahydro-naphthalen-2-yl)-3,4,5,6-tetrahydro-2H-[1,2']bipyridinyl-4-yl]-amine werden mit 10 mL 1.25 N HCl in Methanol versetzt und über Nacht bei Raumtemperatur gerührt. Das Reaktionsgemisch wird eingeengt und unter Hochvakuum getrocknet. Das Produkt liegt als Hydrochlorid vor.

Ausbeute: 171 mg, heller Feststoff, Rt. = 2.73 min (Methode B), LCMS: 478 (M+H).

¹H NMR (500 MHz, DMSO/ deuteriertes TFA) δ = 8.06 (dd, *J*=9.1, 7.4, 1H), 7.71 (d, *J*=1.1, 1H), 7.59 - 7.52 (m, 2H), 7.37 (d, *J*=9.1, 1 H), 7.18 (d, *J*=7.3, 1 H), 4.41 (d, *J*=13.3, 2H), 4.01 (d, *J*=2.5, 1H), 3.73 - 3.64 (m, 1H), 3.56 - 3.51 (m, 1 H), 3.37 - 3.28 (m, 3H), 2.27 - 2.10 (m, 3H), 2.07 - 1.97 (m, 1 H), 1.89 - 1.66 (m, 7H), 1.58 - 1.29 (m, 15H).

### FS1016

### Herstellung von 4-[1-(6-Bromo-pyridin-2-yl)-azepan-4-ylamino]-butan-1-ol

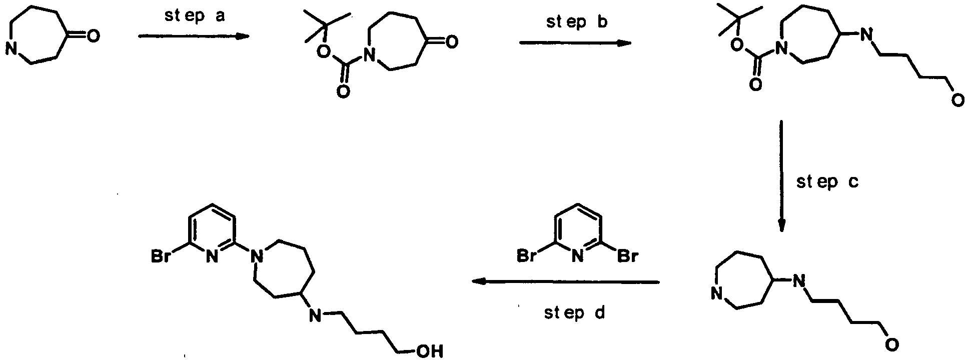

### Stufe a:

### 4-Oxo-azepane-1-carboxylic acid tert-butyl ester

1.04 g (6.98 mmol) Azepan-4-one Hydrochlorid wird zu einer Lösung aus 1.63 g (15.35 mmol) Natriumcarbonat in 10 ml Wasser zugegeben. Unter starkem Rühren wird eine Lösung aus 1.68g (7.68 mmol) Di-tert-butyldicarbonat in 10 mL THF langsam zugetropft. Anschließend wird das Reaktionsgemisch über Nacht bei Raumtemperatur nachgerührt, bis zum wässerigen Rückstand eingeengt und 3 Mal mit EE extrahiert. Die organische Phase wird über Natriumsulfat getrocknet und zum Rückstand eingeengt.

1.62 g, braunes Öl, Rt. = 1.94 min (Methode B).

### Stufe b:

### 4-(4-Hydroxy-butylamino)-azepane-1-carboxylic acid tert-butyl ester

Das Produkt aus Stufe a (1.09 g, 4.65 mmol) wird in 10 mL THF suspendiert und mit 864 µL (9.3 mmol) 4-Amino-1-butanol versetzt. Das Gemisch wird 30 min bei Raumtemperatur gerührt, mit 532 µl (9.3 mmol) Eisessig versetzt und weitere 10 min gerührt. Anschließend wird Natriumtriacetoxyborhydrid (1.97 g, 9.3 mmol) zugegeben. Das Reaktionsgemisch wird über Nacht bei Raumtemperatur gerührt, bis zum Rückstand eingeengt, mit Wasser versetzt und 3 Mal mit EA extrahiert. Die organische Phase wird über Natriumsulfat getrocknet, filtriert und eingeengt.

1.39 g, gelbes Öl, LCMS: 287 (M+H).

### Stufe c:

### 4-(Azepan-4-ylamino)-butan-1-ol

Das Produkt aus Stufe b (1.32 g, 4.65 mmol) wird in 8 ml Dioxan gelöst, mit 16 ml 4N HCl in Dioxan versetzt und über Nacht bei RT gerührt. Das Reaktionsgemisch wird eingedampft und unter Hochvakuum getrocknet. 1.09 g, gelbes Öl, LCMS: 187 (M+H).

### Stufe d:

### 4-[1-(6-Bromo-pyridin-2-yl)-azepan-4-ylamino]-butan-1-ol

545 mg (2.3 mmol) 2,6-Dibrompyridin, das Produkt aus Stufe c (428 mg, 2.3 mmol) und 954 mg Kaliumcarbonat (6.9 mmol) werden in 20 mL DMSO suspendiert und über Nacht bei 120°C gerührt. Das Gemisch wird dann mit Wasser versetzt und mit EA extrahiert. Die organische Phase wird mit einer gesättigten Natriumchloridlösung gewaschen, über Natriumsulfat getrocknet und zum Rückstand eingeengt. Der entstandene Rückstand wird mittels RP-Flashchromatographie an C18 Kieselgel aufgereinigt.

300 mg, farbloses Öl, Rt. = 1.63 min (Methode B), LCMS: 343 (M+H).

### FS1017

### Herstellung von 4-{1-[6-(5,5,8,8-Tetramethyl-5,6,7,8-tetrahydro-naphthalen-2-yl)-pyridin-2-yl]-azepan-4-ylamino}-butan-1-ol

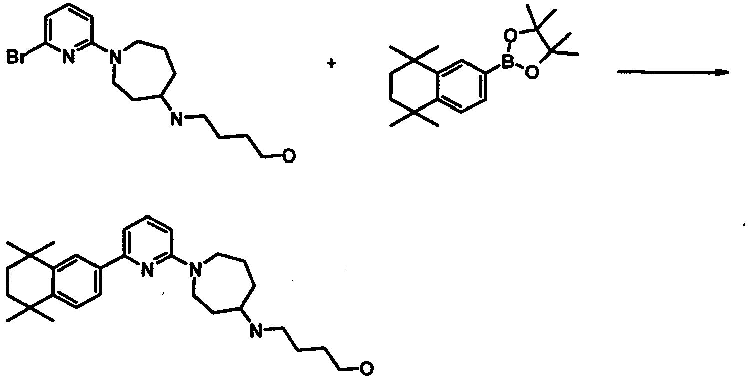

Die Herstellung erfolgt analog FS102 ausgehend von 106 mg (0.304 mmol) 4-[1-(6-Bromo-pyridin-2-yl)-azepan-4-ylamino]-butan-1-ol und 116 mg (0.334 mmol) 4,4,5,5-Tetramethyl-2-(5,5,8,8-tetramethyl-5,6,7,8-tetrahydro-naphthalen-2-yl)-[1,3,2]dioxaborolane. Das Rohprodukt wird mittels RP-Flashchromatographie an C18 Kieselgel aufgereinigt. Das Produkt liegt als Hydrochlorid vor.

68 mg, heller Feststoff, Rt. = 2.49 min (Methode B), LCMS: 450 (M+H).

¹H NMR (500 MHz, DMSO/ deuteriertes TFA) δ = 7.94 (t, *J*=8.2, 1H), 7.60 (s, 1H), 7.46 (q, *J*=8.2, 2H), 7.18 (d, *J*=9.1, 1 H), 7.03 (d, *J*=7.0, 1H), 3.98 - 3.87 (m, 1 H), 3.84 - 3.75 (m, 1 H), 3.75 - 3.64 (m, 2H), 3.53 - 3.38 (m, 2H), 3.34 - 3.21 (m, 1H), 3.03 - 2.87 (m, 2H), 2.31 (d, *J*=12.5, 1 H), 2.08 (dd, *J*=25.8, 14.1, 2H), 1.93 - 1.43 (m, 11H), 1.33 - 1.20 (m, 12H).

### FS1018

### Herstellung von 3-[1-(6-Bromo-pyridin-2-yl)-azepan-4-ylamino]-propan-1-ol

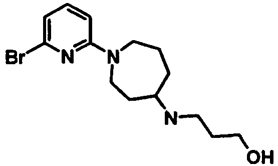

Die Herstellung erfolgt analog 4-[1-(6-Bromo-pyridin-2-yl)-azepan-4-ylamino]-butan-1-ol ausgehend von 127 µL (1.69 mmol) 3-Amino-1-propanol 524 mg, gelbes Öl, Rt. = 1.59 min (Methode B), LCMS: 329 (M+H).

### FS1019

### Herstellung von 3-{1-[6-(5,5,8,8-Tetramethyl-5,6,7,8-tetrahydro-naphthalen-2-yl)-pyridin-2-yl]-azepan-4-ylamino}-propan-1-ol

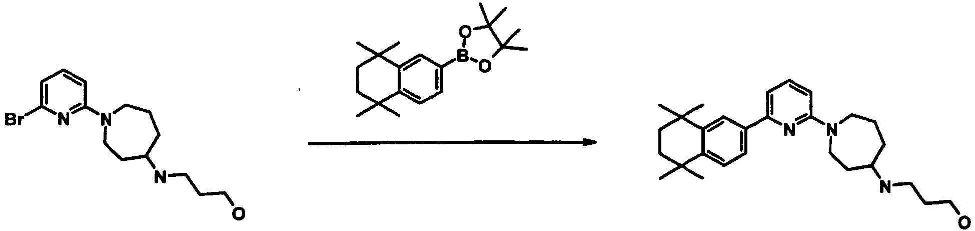

Die Herstellung erfolgt analog FS102 ausgehend von 107 mg (0.323 mmol) 3-[1-(6-Bromo-pyridin-2-yl)-azepan-4-ylamino]-propan-1-ol und 123 mg (0.356 mmol) 4,4,5,5-Tetramethyl-2-(5,5,8,8-tetramethyl-5,6,7,8-tetrahydro-naphthalen-2-yl)-[1,3,2]dioxaborolane. Das Rohprodukt wird mittels RP-Flashchromatographie an C18 Kieselgel aüfgereinigt. Das Produkt liegt als Hydrochlorid vor. 84 mg, heller Feststoff, Rt. = 2.53 min (Methode B), LCMS: 436 (M+H).

¹H NMR (400 MHz, DMSO/ deuteriertes TFA) δ = 8.02 (dd, *J*=9.1, 7.4, 1 H), 7.70 (s, 1H), 7.59 - 7.51 (m, 2H), 7.26 (d, *J*=9.2, 1H), 7.12 (d, *J*=7.3, 1 H), 4.08 - 3.99 (m, 1 H), 3.92 - 3.83 (m, 1 H), 3.83 - 3.71 (m, 2H), 3.57 (t, *J*=5.9, 2H), 3.41 - 3.30 (m, 1 H), 3.11 - 3.03 (m, 2H), 2.39 (d, *J*=13.8, 1 H), 2.23 - 2.04 (m, 2H), 1.98 - 1.79 (m, 4H), 1.77 - 1.63 (m, 5H), 1.33 (dd, *J*=10.0, 7.3, 12H).

### FS1020

### Herstellung von 2-[1-(6-Bromo-pyridin-2-yl)-azepan-4-ylamino]-ethanol

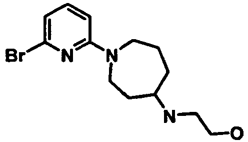

Die Herstellung erfolgt analog 4-[1-(6-Bromo-pyridin-2-yl)-azepan-4-ylamino]-butan-1-ol ausgehend von 99 µL (1.65 mmol) Ethanolamin 85 mg, farbloses Öl, Rt. = 1.55 min (Methode B), LCMS: 315 (M+H).

### FS1021

### Herstellung von 2-{1-[6-(5,5,8,8-Tetramethyl-5,6,7,8-tetrahydro-naphthalen-2-yl)-pyridin-2-yl]-azepan-4-ylamino}-ethanol

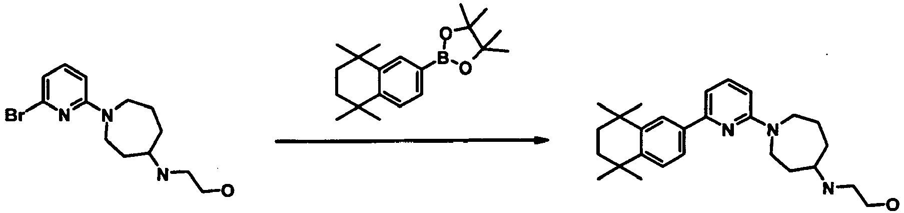

Die Herstellung erfolgt analog FS102 ausgehend von 79 mg (0.245 mmol) 2-[1-(6-Bromo-pyridin-2-yl)-azepan-4-ylamino]-ethanol und 96 mg (0.257 mmol) 4,4,5,5-Tetramethyl-2-(5,5,8,8-tetramethyl-5,6,7,8-tetrahydro-naphthalen-2-yl)-[1,3,2]dioxaborolane. Das Produkt wird mittels präparativer HPLC aufgereinigt und mit methanolischer HCl ins Hydrochlorid überführt. 49 mg, heller Feststoff, Rt. = 2.48 min (Methode B), LCMS: 422 (M+H).

¹H NMR (400 MHz, DMSO/ deuteriertes TFA) δ = 8.03 (dd, *J*=9.2, 7.3, 1 H), 7.69 (d, *J*=1.8, 1 H), 7.59 - 7.50 (m, 2H), 7.28 (d, *J*=9.2, 1 H), 7.12 (d, *J*=7.1, 1 H), 4.06 - 3.96 (m, 1 H), 3.94 - 3.84 (m, 1 H), 3.82 - 3.70 (m, 4H), 3.43 - 3.34 (m, 1 H), 3.13 - 3.02 (m, 2H), 2.39 (s, 1 H), 2.24 - 2.06 (m, 2H), 2.01 -1.83 (m, 2H), 1.78 - 1.66 (m, 5H), 1.39 -1.30 (m, 12H).

### FS1022

### Herstellung von 1-[6-(3,3-Dimethyl-indan-5-yl)-pyridin-2-yl]-piperazine

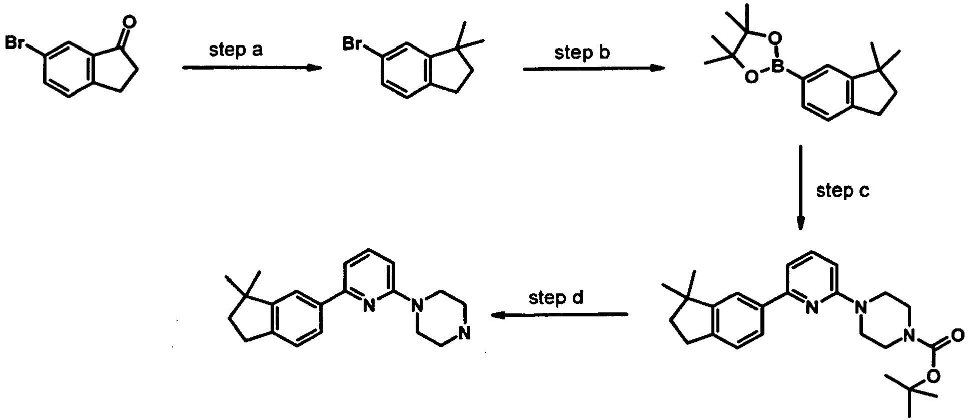

### Stufe a:

### 6-Bromo-1,1-dimethyl-indan

Unter Argon werden 5.75 mL (52.10 mmol) Titan(IV)chlorid in 50 mL DCM gelöst, auf -78°C abgekühlt. Bei Dieser Temperatur wird eine 2 M Dimethylzink-Lösung in THF tropfenweise zugegeben (37.22 mL, 74.43 mmol). Das Gemisch wird 30 min nachgerührt. Eine Lösung aus 5.24 g (24.81 mmol) 6-Bromo-1-indanone in 50 mL DCM wird bei -75°C tropfenweise zugegeben. Das Reaktionsgemisch wird 45 min nachgerührt, dann langsam auf Raumtemperatur kommen lassen, anschließend über Nacht bei Raumtemperatur gerührt, auf 0°C abgekühlt, mit MeOH gequencht, mit Wasser verdünnt und 3 Mal mit DCM extrahiert. Die org. Phase wird über Natriumsulfat getrocknet, filtriert und eingeengt. Der Rückstand wird mittels RP-Flashchromatographie an C18-Kieselgel gereinigt und anschließend bei reduziertem Druck abdestilliert.

3.14g, farbloses Öl, Rt. = 3.64 min (Methode B)

### Stufe b:

### 2-(3,3-Dimethyl-indan-5-yl)-4,4,5,5-tetramethyl-[1,3,2]dioxaborolane

Die Herstellung erfolgt analog FS801 ausgehend vom Produkt aus Stufe a (1.99 g, 8.85 mmol) und 4,4,5,5-Tetramethyl-2-(5,5,8,8-tetramethyl-5,6,7,8-tetrahydro-naphthalen-2-yl)-[1,3,2]dioxaborolane (2.92 g, 11.50 mmol).

1.34 g, gelber Feststoff, Rt. = 3.81 min (Methode B), LCMS: 273 (M+H).

### Stufe c:

### 4-[6-(3,3-Dimethyl-indan-5-yl)-pyridin-2-yl]-piperazine-1-carboxylic acid tert-butyl ester

Die Herstellung erfolgt analog FS 102 ausgehend von 400 mg (1.17 mmol) 6'-Chloro-2,3,5,6-tetrahydro-[1,2']bipyrazinyl-4-carboxylic acid tert-butyl ester und 458 mg (1.26 mmol) Produkt aus Stufe b.

424 mg, weißer Feststoff, Rt. = 3.75 min (Methode B), LCMS: 408 (M+H).

### Stufe d:

### 1-[6-(3,3-Dimethyl-indan-5-yl)-pyridin-2-yl]-piperazine

Die Verbindung wird analog FS201 ausgehend vom Produkt aus Stufe c (424 mg, 1.04 mmol) hergestellt. Das Produkt ist das Hydrochlorid.

318 mg, weißer Feststoff. Rt. = 2.52 min (Methode B), LCMS: 308 (M+H). ¹H NMR (400 MHz, DMSO/ deuteriertes TFA) δ = 8.01 (dd, J=8.8, 7.6, 1H), 7.69 - 7.59 (m, 2H), 7.39 (d, *J*=7.8, 1H), 7.26 (dd, *J*=8.1, 5.2, 2H), 4.05 - 3.97 (m, 4H), 3.42 - 3.34 (m, 4H), 2.98 (t, *J*=7.2, 2H), 1.99 (t, *J*=7.2, 2H), 1.32 (s, 6H).

### FS1023

### 5-{4-[6-(3,3-Dimethyl-indan-5-yl)-pyridin-2-yl]-piperazin-1-yl}-pentan-1-ol

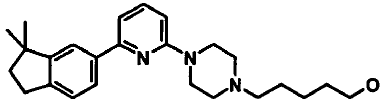

Die Herstellung erfolgt analog FS501 ausgehend von 144 mg (0.47 mmol) 1-[6-(3,3-Dimethyl-indan-5-yl)-pyridin-2-yl]-piperazine und 96 mg (0.94 mmol) 5-Hydroxy-pentanal. Das Produkt ist das Hydrochlorid.

92 mg, heller Feststoff. Rt. = 2.56 min (Methode B), LCMS: 394 (M+H).

¹H NMR (500 MHz, DMSO/ deuteriertes TFA) δ = 7.94 (dd, *J*=8.6, 7.6, 1H), 7.74 - 7.68 (m, 2H), 7.36 (d, *J*=7.8, 1 H), 7.30 (d, *J*=7.4, 1 H), 7.19 (d, *J*=8.8, 1 H), 4.54 (d, *J*=13.8, 2H), 3.70 (d, *J*=11.9, 2H), 3.50 (t, *J*=6.3, 3H), 3.25 - 3.18 (m, 7H), 3.00 - 2.92 (m, 2H), 1.98 (t, *J*=7.2, 2H), 1.83 -1.74 (m, 2H), 1.59 -1.51 (m, 2H), 1.48 - 1.40 (m, 2H), 1.32 (s, 6H).

### FS1024

### Acetic acid 4-{4-[6-(3,3-dimethyl-indan-5-yl)-pyridin-2-yl]-piperazin-1-yl}-butyl ester

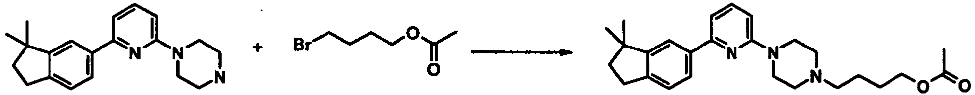

Die Herstellung erfolgt analog FS401 ausgehend von 142 mg (0.46 mmol) 1-[6-(3,3-Dimethyl-indan-5-yl)-pyridin-2-yl]-piperazine und 90 µL Brombutylacetat unter Verwendung von 2 equiv. Kaliumcarbonat.

104 mg, gelbes Öl. Rt. = 2.65 min (Methode B), LCMS: 422 (M+H).

### FS1025

### 4-{4-[6-(3,3-Dimethyl-indan-5-yl)-pyridin-2-yl]-piperazin-1-yl}-butan-1-ol

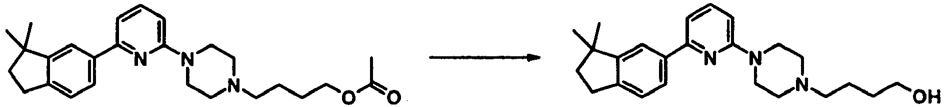

Die Herstellung erfolgt analog FS402 ausgehend von 104 mg (0.23 mmol) Acetic acid 4-{4-[6-(3,3-dimethyl-indan-5-yl)-pyridin-2-yl]-piperazin-1-yl}-butyl ester. Das Produkt ist das Hydrochlorid.

51 mg, heller Feststoff. Rt. = 2.49 min (Methode B), LCMS: 380 (M+H). ¹H NMR (400 MHz, DMSO/ deuteriertes TFA) δ = 7.86 (dd, *J*=8.6, 7.6, 1 H), 7.79 - 7.72 (m, 2H), 7.32 (t, *J*=7.8, 2H), 7.10 (d, *J*=8.6, 1 H), 4.54 (d, *J*=14.0, 2H), 3.68 (d, *J*=11.6, 2H), 3.48 (dt, *J*=25.2, 9.2, 4H), 3.29 - 3.13 (m, 4H), 2.94 (t, *J*=7.2, 2H), 1.96 (t, *J*=7.2, 2H), 1.88 -1.76 (m, 2H), 1.60 -1.49 (m, 2H), 1.31 (s, 6H).

### FS1026

### Herstellung von 6'-(5,5,8,8-Tetramethyl-5,6,7,8-tetrahydro-naphthalen-2-yl)-3,4,5,6-te trahydro-2H-[1,2']bipyridinyl-3-ylamine

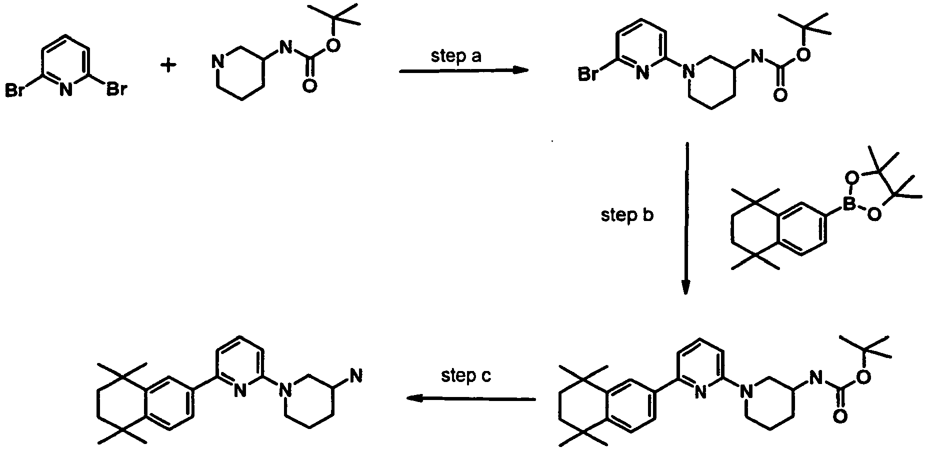

### Stufe a:

### (6'-Bromo-3,4,5,6-tetrahydro-2H-[1,2']bipyridinyl-3-yl)-carbamic acid tert-butyl ester

Die Herstellung erfolgt analog ausgehend von 1 g (4.23 mmol) 2,6-Dibrompyridin und 845 mg (4.22 mmol) Piperidin-3-yl-carbamic acid tert-butyl ester.

1.28 g, braunes Öl, Rt. = 3.16 min (Methode B), LCMS: 356 (M+H).

### Stufe b:

### [6'-(5,5,8,8-Tetramethyl-5,6,7,8-tetrahydro-naphthalen-2-yl)-3,4,5,6-tetrahydro-2H-[1,2']bipyridinyl-3-yl]-carbamic acid tert-butyl ester

Die Herstellung erfolgt analog FS102 ausgehend von 1.28 g (3.54 mmol) Produkt aus Stufe a und 1.46 g (3.91 mmol) 4,4,5,5-Tetramethyl-2-(5,5,8,8-tetramethyl-5,6,7,8-tetrahydro-naphthalen-2-yl)-[1,3,2]dioxaborolane.

1.07 g, farbloses Öl, Rt. = 3.61 min (Methode B), LCMS: 464 (M+H).

### Stufe c:

### 6'-(5,5,8,8-Tetramethyl-5,6,7,8-tetrahydro-naphthalen-2-yl)-3,4,5,6-tetrahydro-2H-[1,2']bipyridinyl-3-ylamine

### Die Verbindung wird analog FS201 ausgehend vom Produkt aus Stufe b hergestellt.

828 mg, gelber Feststoff. Rt. = 2.66 min (Methode B), LCMS: 364 (M+H).

¹H NMR (400 MHz, DMSO/ deuteriertes TFA) δ = 8.05 (dd, *J*=9.1, 7.4, 1 H), 7.69 (d, *J*=1.5, 1 H), 7.60 - 7.51 (m, 2H), 7.31 (d, *J*=9.0, 1 H), 7.17 (d, *J*=7.2, 1 H), 4.19 (dd, *J*=13.5, 3.3, 1 H), 4.03 - 3.93 (m, 1 H), 3.66 - 3.51 (m, 2H), 3.51 - 3.42 (m, 1 H), 2.20 - 2.10 (m, 1 H), 2.00 - 1.91 (m, 1 H), 1.88 - 1.71 (m, 6H), 1.39 - 1.30 (m, 12H).

### FS1027

### Acetic acid 4-[6'-(5,5,8,8-tetramethyl-5,6,7,8-tetrahydro-naphthalen-2-yl)-3,4,5,6-tetrahydro-2H-[1,2']bipyridinyl-3-ylamino]-butyl ester

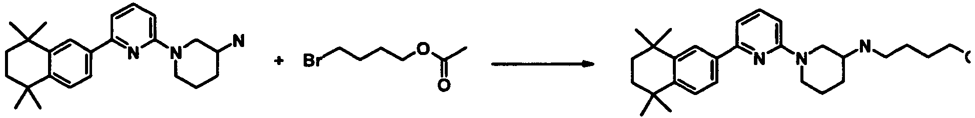

Die Herstellung erfolgt analog FS401 ausgehend von 94 mg (0.46 mmol) 6'-(5,5,8,8-Tetramethyl-5,6,7,8-tetrahydro-naphthalen-2-yl)-3,4,5,6-tetrahydro-2H-[1,2']bipyridinyl-3-ylamine und 45 µL Brombutylacetat unter Verwendung von 2 equiv. Kaliumcarbonat.

49 mg, gelbes Öl. Rt. = 2.87 min (Methode B), LCMS: 478 (M+H).

### FS1028

### 4-[6'-(5,5,8,8-Tetramethyl-5,6,7,8-tetrahydro-naphthalen-2-yl)-3,4,5,6-tetrahydro-2H-[1,2']bipyridinyl-3-ylamino]-butan-1-ol

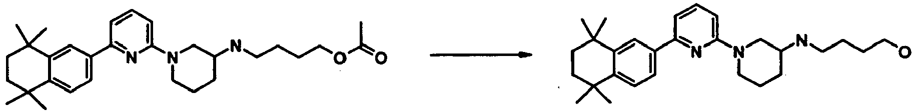

Die Herstellung erfolgt analog FS402 ausgehend von 49 mg (0.23 mmol) Acetic acid 4-[6'-(5,5,8,8-tetramethyl-5,6,7,8-tetrahydro-naphthalen-2-yl)-3,4,5,6-tetrahydro-2H-[1,2']bipyridinyl-3-ylamino]-butyl ester. Das Produkt ist das Hydrochlorid.

43 mg, heller Feststoff. Rt. = 2.70 min (Methode B), LCMS: 436 (M+H). ¹H NMR (400 MHz, DMSO/ deuteriertes TFA) δ = 7.98 - 7.91 (m, 1H), 7.68 (d, *J*=1.9, 1 H), 7.54 - 7.43 (m, 2H), 7.23 (dd, *J*=8.9, 4.3, 1 H), 7.14 (d, *J*=7.3, 1 H), 4.42 - 4.30 (m, 1 H), 3.99 (d, J=13.7, 1 H), 3.49 - 3.24 (m, 5H), 3.10 - 2.97 (m, 2H), 2.19 - 2.10 (m, 1 H), 1.93 - 1.83 (m, 1 H), 1.80 - 1.60 (m, 8H), 1.54 - 1.45 (m, 2H), 1.25 (d, *J*=11.7, 12H).

### FS1029

### 3-[6'-(5,5,8,8-Tetramethyl-5,6,7,8-tetrahydro-naphthalen-2-yl)-3,4,5,6-tetrahydro-2H-[1,2']bipyridinyl-3-ylaminol-propan-1-ol

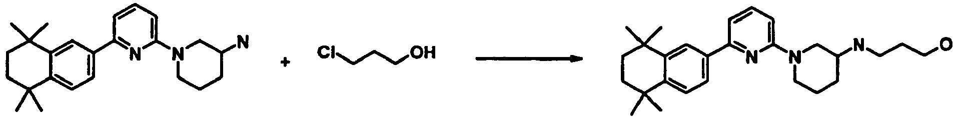

Die Herstellung erfolgt analog FS301 ausgehend von 73 mg (0.20 mmol) 6'-(5,5,8,8-Tetramethyl-5,6,7,8-tetrahydro-naphthalen-2-yl)-3,4,5,6-tetrahydro-2H-[1,2']bipyridinyl-3-ylamine und 33 µL 3-Chlor-1-propanol. Das Produkt ist das Hydrochlorid. 75 mg, heller Feststoff. Rt. = 3.44 min (Methode B), LCMS: 422 (M+H).

¹H NMR (500 MHz, DMSO/ deuteriertes TFA) δ = 7.97 (dd, *J*=16.5, 8.9, 1 H), 7.77 (dd, *J*=16.4, 1.8, 1 H), 7.68 - 7.59 (m, 1 H), 7.52 (dd, *J*=8.3, 2.8, 1 H), 7.27 - 7.18 (m, 2H), 4.32 (dd, *J*=100.2, 11.2, 1 H), 4.10 - 3.91 (m, 1 H), 3.57 (t, *J*=5.9, 1 H), 3.55 - 3.30 (m, 3H), 3.24 - 3.15 (m, 4H), 2.24 - 2.06 (m, 1 H), 1.96 -1.68 (m, 8H), 1.37 - 1.29 (m, 12H).

### FS1030

### Herstellung von 1-[6-(5,5,8,8-Tetramethyl-5,6,7,8-tetrahydro-naphthalen-2-yl)pyridin-2-yl]-[1,4]diazepane

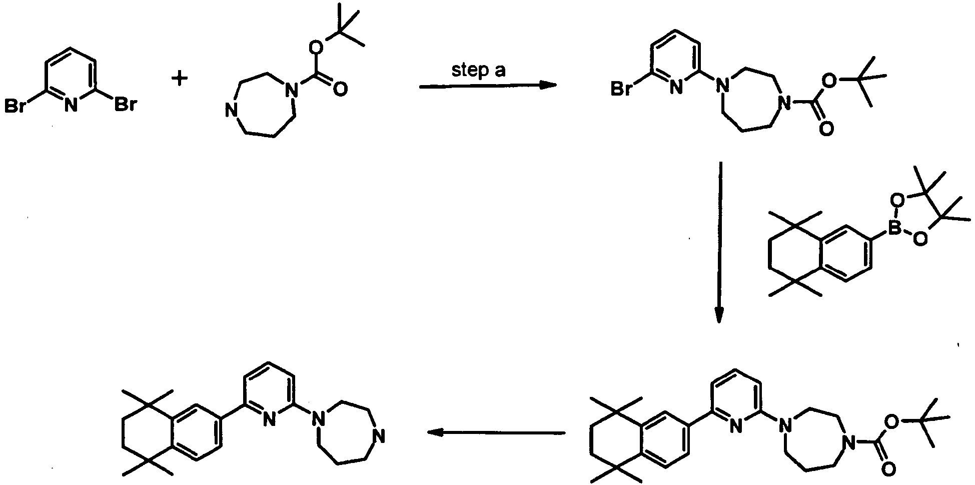

### Stufe a:

### 4-(6-Bromo-pyridin-2-yl)-[1,4]diazepane-1-carboxylic acid tert-butyl ester

Die Herstellung erfolgt analog ausgehend von 1.02 g (4.31 mmol) 2,6-Dibrompyridin und 856 mg (4.27 mmol) Azepan-4-yl-carbamic acid tert-butyl ester.

1.17 g, braunes Öl, Rt. = 3.19 min (Methode B), LCMS: 357 (M+H).

### Stufe b:

### 4-[6-(5,5,8,8-Tetramethyl-5,6,7,8-tetrahydro-naphthalen-2-yl)-pyridin-2-yl]-[1,4]diazepane-1-carboxylic acid tert-butyl ester

Die Herstellung erfolgt analog FS102 ausgehend von 1.13 g (3.18 mmol) Produkt aus Stufe a und 1.46 g (3.49 mmol) 4,4,5,5-Tetramethyl-2-(5,5,8,8-tetramethyl-5,6,7,8-tetrahydro-naphthalen-2-yl)-[1,3,2]dioxaborolane.

1.01 g, farbloses Öl, Rt. = 3.91 min (Methode B), LCMS: 464 (M+H).

### Stufe c:

### 1-[6-(5,5,8,8-Tetramethyl-5,6,7,8-tetrahydro-naphthalen-2-yl)-pyridin-2-yl]-[1,4]diazepane

### Die Verbindung wird analog FS201 ausgehend vom Produkt aus Stufe b hergestellt.

627 mg, weiße Kristalle. Rt. = 2.71 min (Methode B), LCMS: 364 (M+H). ¹H NMR (400 MHz, DMSO/ deuteriertes TFA) δ = 8.00 (dd, *J*=9.1, 7.4, 1H), 7.70 (s, 1 H), 7.54 (s, 2H), 7.21 (dd, *J*=35.0, 8.2, 2H), 4.15 - 4.07 (m, 2H), 3.89 (t, *J*=5.8, 2H), 3.50 - 3.41 (m, 2H), 3.38 - 3.30 (m, 2H), 3.23 (s, 2H), 2.28 - 2.15 (m, 2H), 1.73 (s, 4H), 1.33 (d, *J*=9.7, 12H).

### FS1031

### Acetic acid 4-{4-[6-(5,5,8,8-tetramethyl-5,6,7,8-tetrahydro-naphthalen-2-yl)-pyridin-2-yl]-[1,4]diazepan-1-yl}-butylester

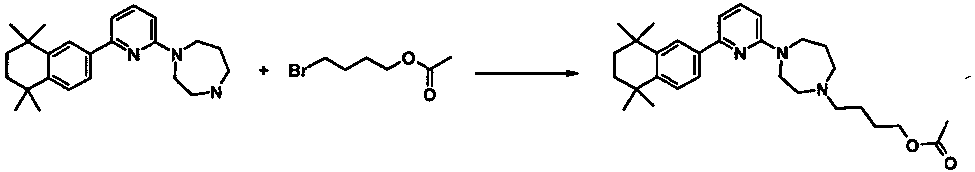

Die Herstellung erfolgt analog FS401 ausgehend von 68 mg (0.19 mmol) 1-[6-(5,5,8,8-Tetramethyl-5,6,7,8-tetrahydro-naphthalen-2-yl)-pyridin-2-yl]-[1,4]diazepane und 34 µL Brombutylacetat unter Verwendung von 2 equiv. Kaliumcarbonat.

### FS1032

### 4-{4-[6-(5,5,8,8-Tetramethyl-5,6,7,8-tetrahydro-naphthalen-2-yl)-pyridin-2-yl]-[1,4]diazepan-1-yl}-butan-1-ol

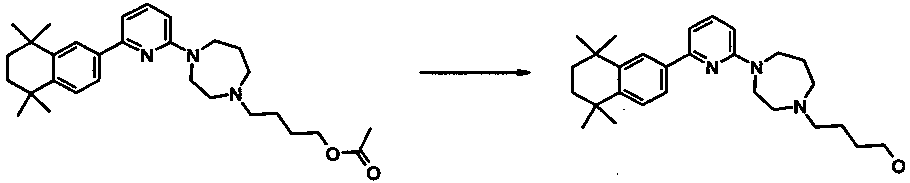

Die Herstellung erfolgt analog FS402. Das Produkt ist das Hydrochlorid.

40 mg, heller Feststoff. Rt. = 2.73 min (Methode B), LCMS: 436 (M+H). ¹H NMR (500 MHz, DMSO/ deuteriertes TFA) δ = 7.97 - 7.89 (m, 1 H), 7.60 (s,

1 H), 7.51 - 7.40 (m, 2H), 7.20 - 7.03 (m, 2H), 4.35 (t, *J*=5.7, 1 H), 4.24 - 4.14 (m, 1 H), 4.05 - 3.94 (m, 1 H), 3.87 - 3.80 (m, 1 H), 3.77 - 3.61 (m, 2H), 3.60 - 3.51 (m, 1 H), 3.43 (t, *J*=6.0, 1H), 3.39 - 3.23 (m, 2H), 3.22 - 3.10 (m, 2H), 2.40 - 2.15 (m, 2H), 1.82 -1.69 (m, 3H), 1.65 (s, 4H), 1.50 -1.41 (m, 1 H), 1.25 (d, *J*=22.4, 12H).

### FS1033

### 3-{4-[6-(5,5,8,8-Tetramethyl-5,6,7,8-tetrahydro-naphthalen-2-yl)-pyridin-2-yl]-[1,4]diazepan-1-yl}-propan-1-ol

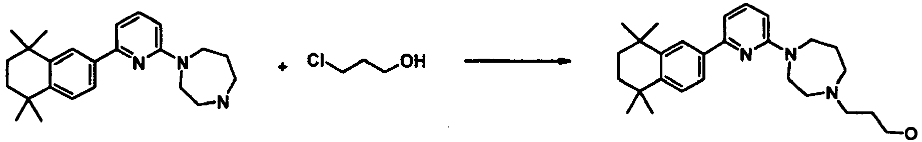

Die Herstellung erfolgt analog FS301 ausgehend von 66 mg (0.18 mmol) 1-[6-(5,5,8,8-Tetramethyl-5,6,7,8-tetrahydro-naphthalen-2-yl)-pyridin-2-yl]-[1,4]diazepane und 18 µL 3-Chlor-1-propanol. Das Produkt ist das Hydrochlorid. 76 mg, heller Feststoff. Rt. = 2.75 min (Methode B), LCMS: 422 (M+H).

¹H NMR (500 MHz, DMSO/ deuteriertes TFA) δ = 8.01 (dd, *J*=8.9, 7.5, 1H), 7.74 (d, *J*=1.7, 1 H), 7.63 - 7.50 (m, 2H), 7.26 - 7.16 (m, 2H), 4.36 - 4.25 (m, 1 H), 4.13 - 4.00 (m, 1 H), 3.95 - 3.85 (m, 1 H), 3.84 - 3.62 (m, 3H), 3.59 - 3.54 (m, 2H), 3.47 - 3.26 (m, 4H), 2.43 - 2.18 (m, 2H), 1.96 -1.86 (m, 2H), 1.74 (s, 4H), 1.39 - 1.29 (m, 12H).

### FS1034

### Herstellung von (S)-2,2-Dimethyl-4-((R)-4-oxo-1-triethylsilanyloxy-butyl)-oxazolidine-3-carboxylic acid tert-butyl ester und (S)-2,2-Dimethyl-4-((S)-4-oxo-1-triethylsilanyloxy-butyl)-oxazolidine-3-carboxylic acid tert-butyl ester

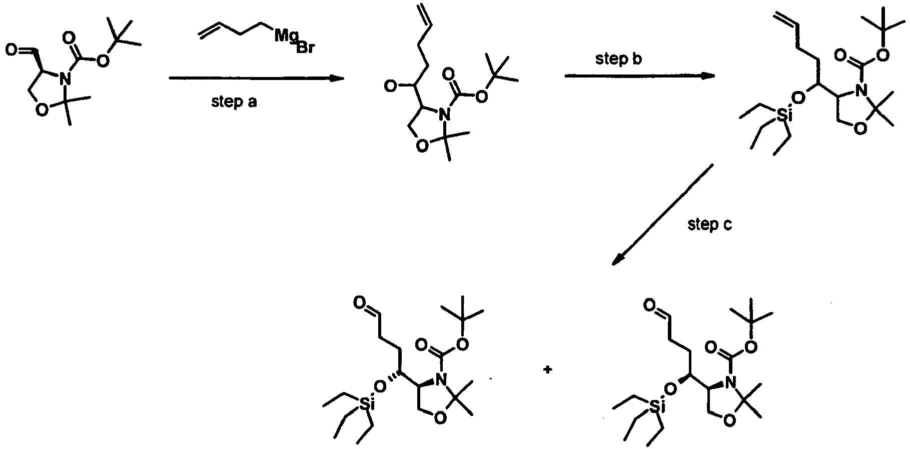

### Stufe a:

### 4-(1-Hydroxy-pent-4-enyl)-2,2-dimethyl-oxazolidine-3-carboxylic acid tert-butyl ester

5g (21.81 mmol) (S)-4-Formyl-2,2-dimethyl-oxazolidine-3-carboxylic acid tert-butyl ester werden in 33 mL THF gelöst, unter Argon gestellt und auf -78°C abgekühlt. 43.62 mL (21.81 mmol) einer 0.5 M 3-Butenylmagnesiumbromidlösung in THF werden tropfenweise zugegeben. Die Mischung wird über Nacht bei Raumtemperatur nachgerührt. Nach DC-Kontrolle wird das Gemisch unter Kühlung mit ca. 90 mL einer gesättigten NH₄Cl-Lösung versetzt, über Nacht nachgerührt und mit ca. 50 mL Wasser verdünnt. Die Phasen werden getrennt und die wässrige Phase wird noch 2 Mal mit EA extrahiert. Die vereinten organischen Phasen werden über Natriumsulfat getrocknet, filtriert und eingeengt. Der ölige Rückstand wird an Kieselgel flash-chromatographiert.

4.89g, farbloses Öl.

### Stufe b:

### 2,2-Dimethyl-4-(1-triethylsilanyloxy-pent-4-enyl)-oxazolidine-3-carboxylic acid tert-butyl ester

4.89 g (17.14 mmol) Produkt aus Stufe a werden in 200 mL DCM gelöst, unter Stickstoff gestellt und auf 0°C abgekühlt. 2.58 g (17.14 mmol) Chlortriethylsilane und 209 mg (1.71 mmol) 4-(Dimethylamino)-pyridin werden zugegeben. Die Reaktionslösung wird 15 min bei 0°C weiter gerührt. Anschliessend werden 4.75 mL (34.27 mmol) Triethylamin zugegeben. Man lässt die Mischung auf Raumtemperatur zurückkommen und über Nacht weiterrühren. Nach DC-Kontrolle wird das Reaktionsgemisch mit ca. 200 mL einer gesättigten NH₄Cl-Lösung versetzt. Die Phasen werden getrennt und die wässrige Phase wird noch 2 Mal mit EA extrahiert. Die vereinten organischen Phasen werden über Natriumsulfat getrocknet, filtriert und eingeengt. Der Rückstand wird an Kieselgel flash-chromatographiert.

Klares farbloses Öl, 5,02 g.

### Stufe c:

### (S)-2,2-Dimethyl-4-((R)-4-oxo-1-triethylsilanyloxy-butyl)-oxazolidine-3-carboxylic acid tert-butyl ester und (S)-2,2-Dimethyl-4-((S)-4-oxo-1-triethylsilanyloxy-butyl)-oxazolidine-3-carboxylic acid tert-buyl ester

5.02 g (12.56 mmol) Produkt aus Stufe b werden in 140 mL DCM gelöst und auf -78°C abgekühlt. Bei dieser Temperatur werden 2.11 g (25.12 mmol) Natriumhydrogencarbonat zugegeben und Ozon wird 2.5 h bis zur blauen Färbung der Lösung unter Rühren durch das Gemisch geleitet. Nach DC-Kontrolle wird Sauerstoff dann 1,5 h bis zur kompletten Entfärbung durch die Mischung geleitet. Anschließend wird Triphenylphsophin (3.30 g, 12.56 mmol) zugeben. Die Mischung wird bei Raumtemperatur über Nacht gerührt und dann abfiltriert. Das Filtrat wird am Rotationsverdampfer bis zum Rückstand eingeengt. Die 2 Isomeren werden mittels Flash-chromatographie an Kieselgel getrennt.

Isomer c1: klares farbloses Öl, 1,21 g.

Isomer c2: klares farbloses Öl, 1,83 g.

### FS1035

### Herstellung von (2S,3R)-2-Amino-6-{4-[6-(5,5,8,8-tetramethyl-5,6,7,8-tetrahydronaphthalen-2-yl)-pyridin-2-yl]-piperazin-1-yl}-hexane-1,3-diol

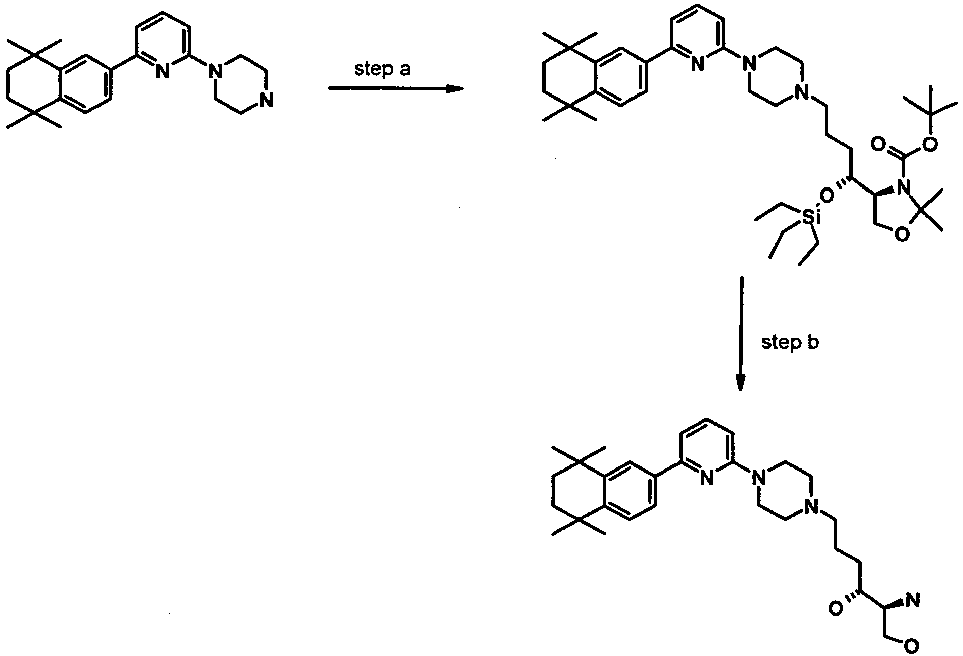

### Stufe a:

### (S)-2,2-Dimethyl-4-((R)-4-{4-[6-(5,5,8,8-tetramethyl-5,6,7,8-tetrahydro-naphthalen-2-yl)-pyridin-2-yl]-piperazin-1-yl}-1-triethylsilanyloxy-butyl)-oxazolidine-3-carboxylic acid tert-butyl ester

100 mg (0.29 mmol) 1-[6-(5,5,8,8-Tetramethyl-5,6,7,8-tetrahydro-naphthalen-2-yl)-pyridin-2-yl]-piperazine werden mit 4.3 ml THF und 175 µl Eisessig versetzt und 138 mg (0.34 mmol) Isomer c1 zugegeben. Das Reaktionsgemisch wird 30 min bei 40°C gerührt. Anschließend werden 121 mg (0.57 mmol) Natriumtriacetoxyborhydrid zugegeben. Das Reaktionsgemisch wird über Nacht bei 40°C gerührt, mit 15 mL EA suspendiert, mit je 5 mL einer gesättigten Natriumhydrogencarbonatlösung und einer gesättigten Kochsalzlösung gewaschen. Die organische Phase wird über Natriumsulfat getrocknet, filtriert und zum Rückstand eingeengt.

342 mg, klares Öl, Rt. = 4.00 min (Methode B), LCMS: 736 (M+H).

### Stufe b:

### (2S,3R)-2-Amino-6-{4-[6-(5,5,8,8-tetramethyl-5,6,7,8-tetrahydronaphthalen-2-yl)-pyridin-2-yl]-piperazin-1-yl}-hexane-1,3-diol

Das Intermediat aus Stufe a wird mit 3 mL einer 1,25 M HCl-Lösung in Mehanol versetzt und über Nacht bei Raumtemperatur gerührt. Das Reaktionsgemisch wird eingeengt und mittels Flash-Chropmatographie an C18-Kieselgel getrennt. Das Produkt liegt als Hydrochlorid vor.

31 mg weißer Feststoff, Rt. = 2.32 min (Methode B), LCMS: 481 (M+H). ¹H NMR (400 MHz, DMSO/ deuteriertes TFA) δ = 7.96 (dd, *J*=8.7, 7.6, 1 H), 7.82 (d, *J*=1.9, 1H), 7.65 (dd, *J*=8.2, 1.9, 1H), 7.51 (d, *J*=8.3, 1 H), 7.29 (d, *J*=7.4, 1H), 7.22 (d, *J*=8.8, 1 H), 4.63 - 4.43 (m, 2H), 3.79 - 3.66 (m, 4H), 3.66 - 3.49 (m, 4H), 3.29 - 3.18 (m, 3H), 3.07 - 2.97 (m, 1 H), 2.03 - 1.81 (m, 2H), 1.74 (s, 4H), 1.69 -1.46 (m, 2H), 1.33 (d, *J*=12.1, 12H).

### FS1036

### Herstellung von (2S,3S)-2-Amino-6-{4-[6-(5,5,8,8-tetramethyl-5,6,7,8-tetrahydro-naphthalen-2-yl)-pyridin-2-yl]-piperazin-1-yl}-hexane-1,3-diol

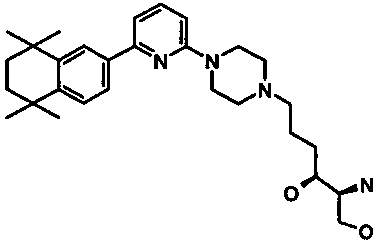

Die Herstellung erfolgt analog ausgehend von 100 mg (0.29 mmol) 1-(6-(5,5,8,8-Tetramethyl-5,6,7,8-tetrahydro-naphthalen-2-yl)-pyridin-2-yl]-piperazine und 138 mg (0.34 mmol) Isomer c2. Das Produkt liegt als Hydrochlorid vor. 44 mg Weißer, Rt. = 2.32 min (Methode B), LCMS: 481 (M+H).

¹H NMR (400 MHz, DMSO/ deuteriertes TFA) ö = 7.93 (dd, *J*=8.7, 7.5, 1 H), 7.84 (d, *J*=1.9, 1 H), 7.67 (dd, *J*=8.2, 1.9, 1 H), 7.50 (d, *J*=8.3, 1 H), 7.29 (d, *J*=7.4, 1 H), 7.19 (d, *J*=8.8, 1 H), 4.66 - 4.41 (m, 2H), 3.84 - 3.64 (m, 5H), 3.60 - 3.46 (m, 2H), 3.27 - 3.20 (m, 3H), 3.19 - 3.10 (m, 1H), 2.05 - 1.77 (m, 3H), 1.74 (s, 4H), 1.64 -1.45 (m, 2H), 1.33 (d, *J*=12.8, 12H).

### FS1101:

### 1-[3-(2-Methoxy-ethoxy)-6-(5,5,8,8-tetramethyl-5,6,7,8-tetrahydro-naphthalen-2-yl)-pyridin-2-yl]-piperazine:

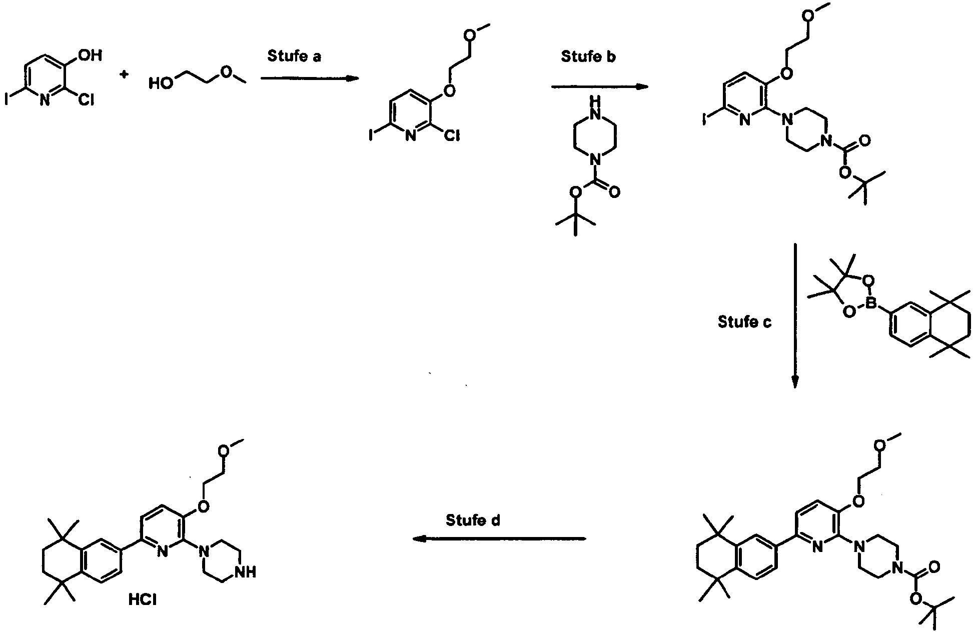

### Stufe a:

500 mg (1.96 mmol) 2-Chlor-6-iodpyridin-3-ol werden in 10 ml DMF gelöst, mit 171 µl (2.15 mmol) Ethylenglycolmonomethylether, 980 mg (2.94 mmol) polymergebundenes Triphenylphosphin (3mmol/g) und 689 mg (2,94 mmol) Di-tert.-butylazodicarboxylat versetzt. Das Reaktionsgemisch wird über Nacht bei RT geschüttelt. Das Reaktionsgemisch wird filtriert, zum Rückstand eingeengt und säulenchromatoigraphisch an Kieselgel aufgereinigt.

1.6g, Öl, Rt. = 2.86 min (Methode A), LCMS: 314 (M+H).

### Stufe b:

Das Produkt von Stufe a und 420 mg (2.23 mmol) Boc-Piperazin werden in 10 ml DMSO gelöst und mit 771 mg (5.58 mmol) Kaliumcarbonat versetzt. Das Reaktionsgemisch wird 15 h bei 120°C gerührt, anschließend mit Wasser versetzt und dreimal mit Ethylacetat extrahiert, über Natriumsulfat getrocknet und eingeengt. Das Produkt wird ohne weitere Aufreinigung weiter umgesetzt.

1.3 g, Öl, Rt. = 3.59 min (Methode A), LCMS: 464 (M+H).

### Stufe c:

Die Umsetzung erfolgt analog FS102.

448 mg, Rt. = 4.07 min (Methode A), LCMS: 524 (M+H).

### Sufe d:

Die Umsetzung erfolgt analog FS201.

310 mg, Rt. = 3.11 min (Methode A), LCMS: 424 (M+H).

¹H NMR (500 MHz, DMSO/deuteriertes TFA) ö 7.90 (d, *J* = 1.9, 1 H), 7.68 (dd, *J* = 8.3, 1.9, 1 H), 7.48 - 7.37 (m, 3H), 4.24 - 4.19 (m, 2H), 3.77 - 3.70 (m, 6H), 3.58-3.53 (m, 2H), 3.37 (s, 3H), 3.12 - 3.07 (m, 2H), 1.70 (s, 4H), 1.30 (d, *J* = 19.9, 12H).

Analog zu den oben beschriebenen Verbindungen können ausgehend von literaturbekannten Bausteinen folgende Verbindungen hergestellt werden:

| CHEMISTRY | Name | Mass |
|---|---|---|
| 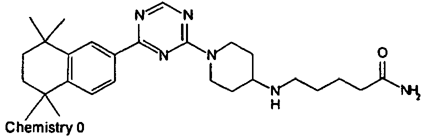 | 5-{1-[4-(5,5,8,8-Tetramethyl-5,6,7,8-tetrahydro-naphthalen-2-yl)-[1,3,5]triazin-2-yl]-piperidin-4. ylamino}-pentanoic acid amide | 464,66 |
| 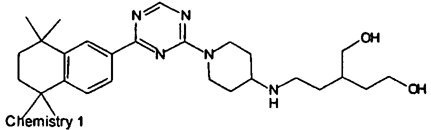 | 2-(2-{1-[4-(5,5,8,8-Tetramethyl-5,6,7,8-tetrahydro-naphthalen-2 yl)-[1,3,5]triazin-2-yl]-piperidin-4-ylamino}-ethyl)-butane-1,4-diol | 481,68 |
| 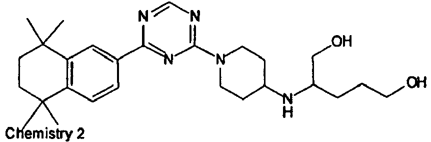 | 2-{1-[4-(5,5,8,8-Tetramethyl-5,6,7,8-tetrahydro-naphthalen-2 yl)-[1,3,5]triazin-2-yl]-piperidin-4-ylamino}-pentane-1,5-diol | 467,66 |
| 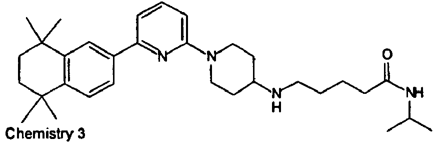 | 5-[6'-(5,5,8,8-Tetramethyl-5,6,7,8-tetrahydro-naphthalen-2 yl)-3,4,5,8-tetrahydro-2H-[1,2']bipyridinyl-4-ylamino]-pentanoic acid isopropylamide | 504,76 |
| 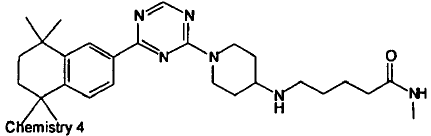 | 5-{1-[4-(5,5,8,8-Tetramethyl-5,6,7,8-tetrahydro-naphthalen-2 yl)-[1,3,5]triazin-2-yl)-piperidin-4-478,68 ylamino)-pentanoic acid methylamide | 478,68 |
| 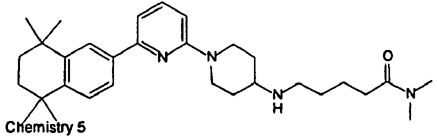 | 5-[6'-(5,5,8,8-Tetramethyl-5,6,7,8-tetrahydro-naphthalen-2 yl)-3,4,5,6-tetrdhydro-2H-[1,2']bipyridinyl-4-ylamino]-pentanoic acid dimethylamide | 490,74 |
| 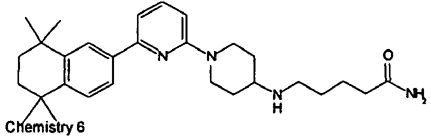 | 5-[6'-(5,5,8,8-Tetramethyl-5,6,7,8-tetrahydro-naphthalen-2-yl)-3,4,5,6-tetra hydro-2H-[1,2']bipyridinyl-4-ylamino]-pentanoic acid amide | 462,68 |
| 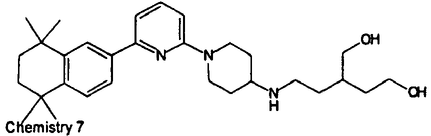 | 2-{2-[6'-(5,5,8,8-Tetramethyl-5,6,7,8-tetrahydro-naphthalen-2 yl)-3,4,5,6-tetrahydro-2H-[1,2']bipyridinyl-4-ylamino]-ethyl}-butane-1,4-diol | 479,71 |
| 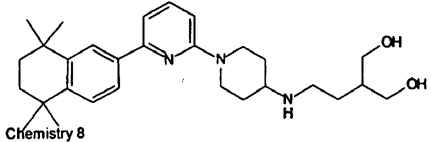 | 2-{2-[6'-(5,5,6,8-Tetramethyl-5,6,7,8-tetrahydro-naphthalen-2 yl)-3,4,5,6-tetrahydro-2H-[1,2']bipyridinyl-4-ylamino]-ethyl}-propane-1,3-diol | 465,68 |
| 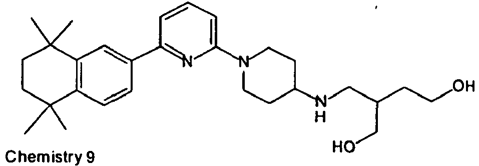 | 2-{[6'-(5,5,8,8-Tetramethyl-5,6,7,8-tetrahydro-naphthalen-2 yl)-3,4,5,6-tetrahydro-2H-[1,2']bipyridinyl-4-ylamino]-methyl}-butane-1,4-diol | 465,68 |
| 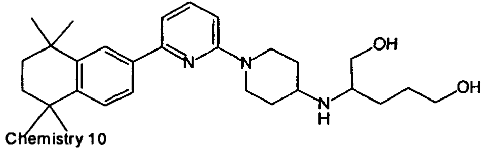 | 2-[6'-(5,5,8,8-Tetramethyl-5,6,7,8-tetrahydro-naphthalen-2 yl)-3,4,5,6-tetrahydro-2H-[1,2']bipyridinyl-4-ylamino]-pentane-1,5-diol | 465,68 |
| 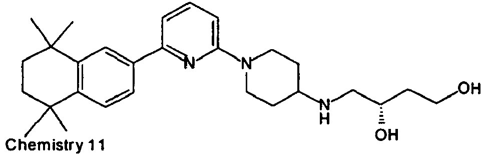 | (S)-4-[6'-(5,5,8,8-Tetra methyl-5,6,7,8-tetrahydro-naphth alen-2-yl)-3,4,5,6-tetrahydro-2H-[1,2']bipyridinyl-4-ylamino]-butane-1,3-diol | 451,65 |
| 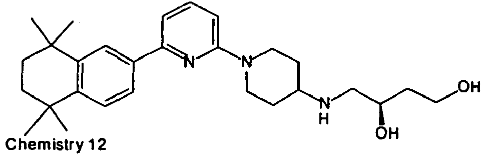 | (R)-4-[6'-(5,5,8,8-Tetramethyl-5,6,7,8-tetrahydro-naphth alen-2-yl)-3,4,5,6-tetrahydro-2H-[1,2']bipyridinyl-4-ylamino]-butane-1,3-diol | 451,65 |
| 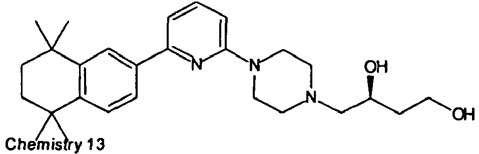 | (S)-4-{4-[6-(5,5,8,8-Tetramethyl-5,6,7,8-tetrahydro-naphthalen-2 yl)-pyridin-2-yl]-piperazin-1-yl}-butane-1,3-diol | 437,63 |
| 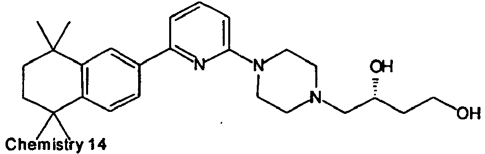 | (R)-4-{4-[6-(5,5,8,8-Tetramethyl 5,6,7,8-tetrahydro-naphthalen-2 yl)-pyridin-2-yl]-piperazin-1-yl}-butane-1,3-diol | 437,63 |
| 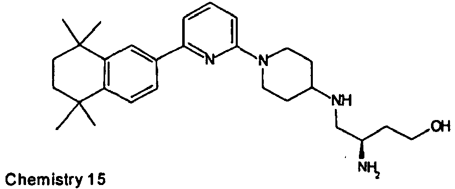 | (R)-3-Amino-4-[6'-(5,5,8,8-tetramethyl-5,6,7,8-tetra hydronaphthalen-2-yl)-3,4,5,6-tetrahydro-2H-[1,2']bipyridinyl-4-ylamino]-butan-1-ol | 450,67 |
| 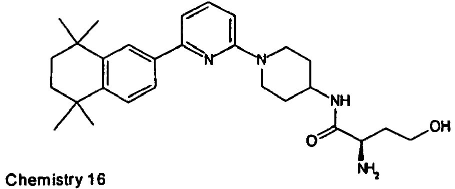 | (R)-2-Amino-4-hydroxy-N-[6'-(5,5,8,8-tetramethyl-5,6,7,8-tetrahydro-naphthalen-2-yl)-3,4,5,6-tetrahydro-2H-[1,2']bipyridinyl-4-yl]-butyramide | 464,65 |
| 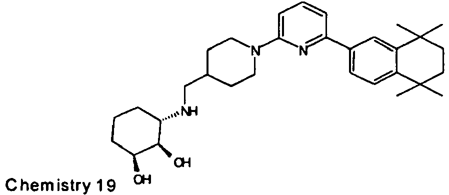 | (1S,2R,3S)-3-{[6'-(5,5,8,8-Tetramethyl-5,6,7,8-tetrahydro-naphthalen-2-yl)-3,4,5,6-tetrahydro-2H-[1,2']bipyridinyl-4-ylmethyl]-amino}-cyclohexane-1,2-diol | 491,72 |
| 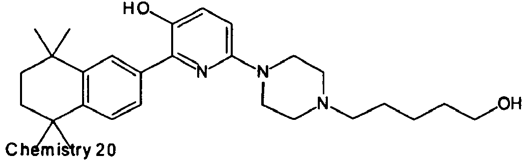 | 6-[4-(5-Hydroxy-pentyl)-piperazin-1-yl]-2-(5,5,8,8-tetramethyl-5,6,7,8-tetrahydro-naphthalen-2-yl)-pyridin-3-ol | 451,65 |
| 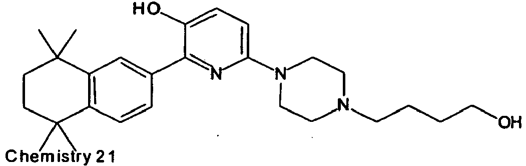 | 6-[4-(4-Hydroxy-butyl)-piperazin 1-yl]-2-(5,5,8,8-tetramethyl-5,6,7,8-tetrahydro-naphthalen-2-yl)-pyridin-3-ol | 437,63 |
| 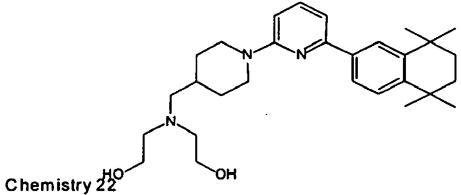 | 2-{(2-Hydroxy-ethyl)-[6'-(5,5,8,8-tetramethyl-5,6,7,8-tetrahydro-naphthalen-2-yl)-3,4,5,6-tetrahydro-2H-[1,2']bipyridinyl-4-ylmethyl]-amino}-ethanol | 465,68 |
| 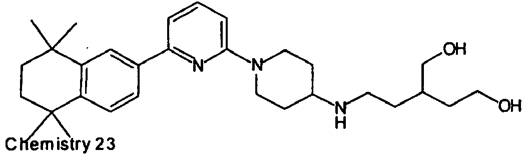 | 2-{2-[6'-(5,5,8,8-Tetramethyl-5,6,7,8-tetrahydro-naphthalen-2 yl)-3,4,5,6-tetrahydro-2H-[1,2']bipyridinyl-4-ylamino]-ethyl}-butane-1,4-diol | 479,71 |
| 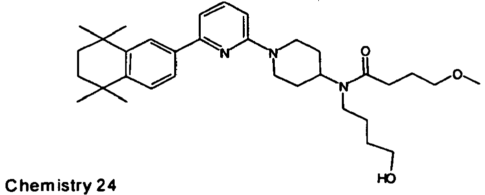 | N-(4-Hydroxy-butyl)-4-methoxy-N-[6'-(5,5,8,8-tetramethyl-5,6,7,8-tetrahydro-naphthalen-2 ; yl)-3,4,5,6-tetrahydro-2H-[1,2']bipyridinyl-4-yl]-butyramide | 535,77 |
| 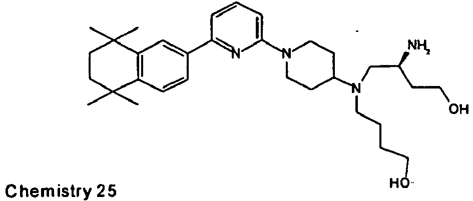 | (S)-3-Amino-4-{(4-hydroxy-butyl)-[6'-(5,5,8,8-tetramethyl-5,6,7,8-tetrahydro-naphthalen-2-, yl)-3,4,5,6-tetrahydro-2H-[1,2']bipyridinyl-4-yl]-amino}-butan-1-ol | 522,78 |
| 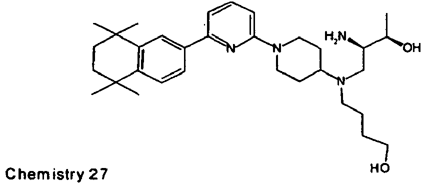 | 4-{((2R,3R)-2-Amino-3-hydroxy-butyl)-[6'-(5,5,8,8-tetramethyl-5,6,7,8-tetrahydro-naphthalen-2-yl)-3,4,5,6-tetrahydro-2H-[1,2']bipyridinyl-4-yl]-amino}-butan-1-ol | 522,78 |
| 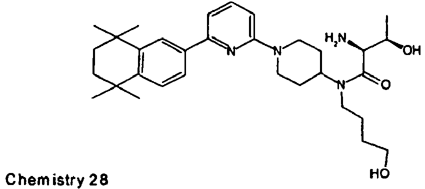 | (2S,3R)-2-Amino-3-hydroxy-N-(4-hydroxy-butyl)-N-[6'-(5,5,8,8-tetramethyl-5,6,7,8-tetrahydro-naphthalen-2-yl)-3,4,5,6-tetrahydro-2H-[1,2']bipyridinyl-4-yl]-butyramide | 536,76 |
| 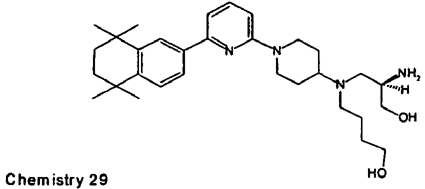 | 4-{((R)-2-Amino-3-hydroxy-propyl)-[6'-(5,5,8,8-tetramethyl-5,6,7,8-tetrahydro-naphthalen-2-yl)-3,4,5,6-tetrahydro-2H-[1,2']bipyridinyl-4-yl]-amino}-butan-1-ol | 508,75 |
| 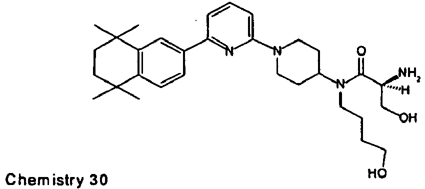 | (S)-2-Amino-3-hydroxy-N-(4-hydroxy-butyl)-N-[6'-(5,5,8,8-tetramethyl-5,6,7,8-tetrahydro-naphthalen-2-yl)-3,4,5,6-tetrahydro-2H-[1,2']bipyridinyl-4-yl]-propionamide | 522,73 |
| 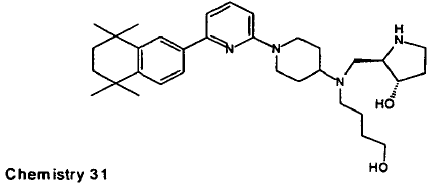 | (2R,3S)-2-({(4-Hydroxy-butyl)-[6'(5,5,8,8-tetramethyl-5,6,7,8-tetrahydro-naphthalen-2-yl)-3,4,5,6-tetrahydro-2H-[1,2']bipyridinyl-4-yl]-amino}-methyl)-pyrrolidin-3-ol | 534,79 |
| 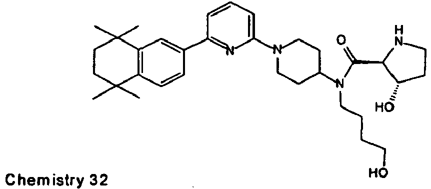 | (2S,3S)-3-Hydroxy-pyrrolidine-2 carboxylic acid (4-hydroxy-butyl)-[6'-(5,5,8,8-tetramethyl-5,6,7,8-tetrahydro-naphthalen-2-yl)-3,4,5,6-tetrahydro-2H-[1,2']bipyridinyl-4-yl]-amide | 548,77 |
| 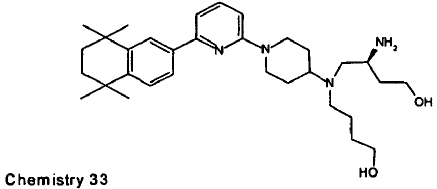 | (S)-3-Amino-4-{(4-hydroxybutyl)-[6'-(5,5,8,8-tetramethyl-5,6,7,8-tetrahydro-naphthalen-2 yl)-3,4,5,6-tetrahydro-2H-[1,2']bipyridinyl-4-yl]-amino}-butan-1-ol | 522,78 |

### II. Biologische Assays

Die in den Beispielen beschriebenen Verbindungen der Formel (I) können in den unten beschriebenen Assays auf eine kinasehemmende Wirkung geprüft werden. Weitere Assays sind aus der Literatur bekannt und könnten vom Fachmann leicht durchgeführt werden (siehe z.B. Dhanabal et al., Cancer Res. 59:189-197; Xin et al., J. Biol. Chem. 274:9116-9121; Sheu et al., Anticancer Res. 18:4435-4441; Ausprunk et al., Dev. Biol. 38:237-248; Gimbrone et al., J. Natl. Cancer Inst. 52:413-427; Nicosia et al., In Vitro 18:538- 549).

### Tests für die Inhibierung der SphK1-Aktivität

### Testbeschreibung

### Biochemischer Assay

Der Kinaseassay wird als 384-well Flashplate assay durchgeführt. 5 nM modifizierte SphK1, 800 nM omega-biotinyl-D-erythro-Sphingosine und 1 µM ATP (mit 0.3µCi ³³P-ATP/well) werden in einem Gesamtvolumen von 50µl (25 mM HEPES, 5 mM MgCl₂, 1 mM Dithiothreitol, 0.01 % Brij35, 0.1 % BSA (fettsäurefrei), pH 7.4) ohne oder mit Prüfsubstanz (5-10 Konzentrationen) für 120 Min bei 30°C inkubiert. Die Reaktion wird mit 25 µl 200 mM EDTA-Lösung gestoppt, nach 30 Min bei Raumtemperatur abgesaugt und die Kavitäten 3mal mit 100 µl 0.9%-iger NaCl-Lösung gewaschen. Der unspezifische Anteil der Kinasereaktion (Blank) wird mit 0,5 mM NaCl bestimmt. Radioaktivität wird im Topcount gemessen. IC₅₀-Werte werden mit RS1 berechnet.

Neben der Überprüfung der Aktivität der Substanz auf das gereinigte SphK1-Enzym ist es im nächsten Schritt erforderlich zu untersuchen, ob die Substanzen SphK1 auch in seiner physiologischen Umgebung, d.h. im Cytoplasma der Zelle, hemmen. Zu diesem Zweck wird die Bildung von S1P in U2OS Osteosarkomazellen, die durch Einführung von modifizierter SphK1-cDNA das Enzym überproduzierten, mit zwei verschiedenen Methoden gemessen:
1. Die Zellen werden für 1 Stunde mit Substanzen und anschließend 15 min mit tritiummarkiertem Sphingosin inkubiert. Das radioaktiv markierte Sphingosin wird in dieser Zeit von den Zellen aufgenommen und von SphK1 zu S1 P umgesetzt. Die Zellen werden dann gewaschen und mit Ammoniaklösung lysiert. Um S1 P von nicht umgesetzten Sphingosin zu trennen, erfolgt eine Extraktion durch Zugabe eines Chloroform-Methanol-Gemischs. Während Sphingosin zum größten Teil in die organische Phase übergeht, reichert sich S1 P in der wässrigen Phase an und wird mit Hilfe eines Szintillationszählers quantifiziert.
2. Die Zellen werden für 1 Stunde mit Substanzen und anschließend 15 min mit Sphingosin inkubiert. Das Sphingosin wird in dieser Zeit von den Zellen aufgenommen und von SphK1 zu S1P umgesetzt. Die Zellen werden dann gewaschen und mit Methanol lysiert. Die Methanollösung wird nun eingedampft und das S1P in lipidfreiem Serum aufgenommen. Die Quantifizierung des S1P erfolgt unter Verwendung eines S1P-spezifischen Antikörpers mit Hilfe eines kompetitiven ELISA-Assays. Der mit Biotin verknüpfte S1P-Antikörper wird mit der Probenlösung inkubiert und dieses Gemisch wird in ein Well übertragen, dessen Boden mit S1 P beschichtet ist. Nur die Antikörper, die noch kein S 1 P aus der Probenlösung gebunden haben, binden an das auf der Platte immobilisierte S1 P und können nach einem Waschschritt durch Zugabe von Meerrettich-Peroxidase gekoppeltem Streptavidin quantifiziert werden. Dazu wird das Substrat TMB zugefügt, das nach Umsetzung durch die Peroxidase bei einer Wellenlänge von 450 nm absorbiert und gemessen werden kann. Ein hohes Signal entspricht folglich einer niedrigen S1P-Konzentration in der Probenlösung und ein niedriges Signal entsprechend einer hohen S1P-Konzentration.

### Pharmakologische Daten

SphK1-Hemmung

(IC₅₀-Bereiche: A: < 100 nM, B: 100 nM - 1000 nM, C: > 1000 nM)

**Tabelle 2**

| **Erfindungsgemäße Verbindung** | **IC₅₀** |
|---|---|
| Amino-acetic acid 5-{4-[3-(5,5,8,8-tetramethyl-5,6,7,8-tetrahydro-naphthalen-2-yl)-phenyl]-piperazin-1-yl}-pentyl ester | B |
| (S)-3-Methyl-1-[3-(5,5,8,8-tetramethyl-5,6,7,8-tetrahydro-naphthalen-2-yl)-phenyl]-piperazine | B |
| 1-[2-Methyl-5-(5,5,8,8-tetramethyl-5,6,7,8-tetrahydro-naphthalen-2-yl)-phenyl]-piperazine | C |
| 4-{1-[4-(5,5,8,8-Tetramethyl-5,6,7,8-tetrahydro-naphthalen-2-yl)-[1,3,5]triazin-2-yl]-piperidin-4-ylamino}-butan-1-ol | A |
| 1-{1-[4-(5,5,8,8-Tetramethyl-5,6,7,8-tetrahydro-naphthalen-2-yl)-[1,3,5]triazin-2-yl]-piperidin-4-yl}-pyrrolidin-3-ylamine | B |
| 5-{4-[3-(2-Methoxy-ethoxy)-6-(5,5,8,8-tetramethyl-5,6,7,8-tetrahydro-naphthalen-2-yl)-pyridin-2-yl]-piperazin-1-yl}-pentan-1-ol | C |
| 1-[3-(2-Methoxy-ethoxy)-6-(5,5,8,8-tetramethyl-5,6,7,8-tetrahydro-naphthalen-2-yl)-pyridin-2-yl]-piperazine | C |
| 1-[4-(5,5,8,8-Tetramethyl-5,6,7,8-tetrahydro-naphthalen-2-yl)-[1,3,5]triazin-2-yl]-piperidin-4-ylamine | A |
| 2-(8,8-Dimethyl-5,6,7,8-tetrahydro-naphthalen-2-yl)-4-piperazin-1-yl-[1,3,5]triazine | C |
| 1'-[4-(5,5,8,8-Tetramethyl-5,6,7,8-tetrahydro-naphthalen-2-yl)-[1,3,5]triazin-2-yl]-[1,4']bipiperidinyl-3-ol | B |
| 2-(5,5-Dimethyl-5,6,7,8-tetrahydro-naphthalen-2-yl)-4-piperazin-1-yl-[1,3,5]triazine | C |
| 5-{4-[4-(5,5,8,8-Tetramethyl-5,6,7,8-tetrahydro-naphthalen-2-yl)-[1,3,5]triazin-2-yl]-piperazin-1-yl}-pentan-1-ol | A |
| 5-{4-[3-(5,5,8,8-Tetramethyl-5,6,7,8-tetrahydro-naphthalen-2-yl)-phenyl]-piperazin-1-yl}-pentan-1-ol | A |
| 4-{4-[3-(5,5,8,8-Tetramethyl-5,6,7,8-tetrahydro-naphthalen-2-yl)-phenyl]-piperazin-1-yl}-butan-1-ol | B |
| 1-[3-(5,5,8,8-Tetramethyl-5,6,7,8-tetrahydro-naphthalen-2-yl)-phenyl]-piperazine | B |
| (R)-2-Amino-3-{4-[6-(5,5,8,8-tetramethyl-5,6,7,8-tetrahydro-naphthalen-2-yl)-pyridin-2-yl]-piperazin-1-yl}-propan-1-ol | C |
| 5-{4-[6-(1,1-Dimethyl-indan-5-yl)-pyridin-2-yl]-piperazin-1-yl}-pentan-1-ol | C |
| 4-{4-[6-(1,1-Dimethyl-indan-5-yl)-pyridin-2-yl]-piperazin-1-yl}-butan-1-ol | C |
| 1-[6-(1,1-Dimethyl-indan-5-yl)-pyridin-2-yl]-piperazine | C |
| 4-{4-[4-(5,5,8,8-Tetramethyl-5,6,7,8-tetrahydro-naphthalen-2-yl)-pyridin-2-yl]-piperazin-1-yl}-butan-1-ol | B |
| 1-[4-(5, 5,8,8-Tetramethyl-5,6, 7,8-tetrahydro-naphthalen-2-yl)-pyridin-2-yl]-piperazine | B |
| 4-{4-[2-(5,5,8,8-Tetramethyl-5,6,7,8-tetrahydro-naphthalen-2-yl)-pyridin-4-yl]-piperazin-1-yl}-butan-1-ol | B |
| 1-[2-(5,5,8,8-Tetramethyl-5,6,7,8-tetrahydro-naphthalen-2-yl)-pyridin-4-yl]-piperazine | B |
| 4-{4-[4-(5,5,8,8-Tetramethyl-5,6,7,8-tetrahydro-naphthalen-2-yl)-[1,3,5]triazin-2-yl]-piperazin-1-yl}-butan-1-ol | A |
| 5-{4-[6-(1,1,3,3-Tetramethyl-indan-5-yl)-pyridin-2-yl]-piperazin-1-yl}-pentan-1-ol | B |
| 5-[6'-(5,5,8,8-Tetramethyl-5,6,7,8-tetrahydro-naphthalen-2-yl)-3,4,5,6-tetrahydro-2H-[1,2']bipyridinyl-4-ylamino]-pentan-1-ol | B |
| 6"-(5,5,8,8-Tetramethyl-5,6,7,8-tetrahydro-naphthalen-2-yl)-3,4,5,6,3',4',5',6'-octahydro-2H,2'H-[1,4';1',2"]terpyridin-3-ol | B |
| 1-[6'-(5,5,8,8-Tetramethyl-5,6,7,8-tetrahydro-naphthalen-2-yl)-3,4,5,6-tetrahydro-2H-[1,2']bipyridinyl-4-yl]-pyrrolidin-3-ylamine | A |
| 5-{4-[6-(8,8-Dimethyl-5,6,7,8-tetrahydro-naphthalen-2-yl)-pyridin-2-yl]-piperazin-1-yl}-pentan-1-ol | C |
| 4-[6'-(5,5,8,8-Tetramethyl-5,6,7,8-tetrahydro-naphthalen-2-yl)-3,4,5,6-tetrahydro-2H-[1,2']bipyridinyl-4-ylamino]-butan-1-ol | A |
| 4-{4-[6-(1,1,3,3-Tetramethyl-indan-5-yl)-pyridin-2-yl]-piperazin-1-yl}-butan-1-ol | B |
| 1-[6-(1,1,3,3-Tetramethyl-indan-5-yl)-pyridin-2-yl]-piperazine | B |
| 4-{4-[6-(8,8-Dimethyl-5,6,7,8-tetrahydro-naphthalen-2-yl)-pyridin-2-yl]-piperazin-1-yl}-butan-1-ol | C |
| 1-[6-(8,8-Dimethyl-5,6,7,8-tetrahydro-naphthalen-2-yl)-pyridin-2-yl]-piperazine | C |
| 6'-(5,5,8,8-Tetramethyl-5,6,7,8-tetrahydro-naphthalen-2-yl)-3,4,5,6-tetrahydro-2H-[1,2']bipyridinyl-4-ylamine | B |
| 6-(5,5,8,8-Tetramethyl-5,6,7,8-tetrahydro-naphthalen-2-yl)-1',2',3',4',5',6'-hexahydro-[2,4']bipyridinyl | B |
| 2-Piperazin-1-yl-4-(5,5,8,8-tetramethyl-5,6,7,8-tetrahydro-naphthalen-2-yl)-[1,3,5]triazine | A |
| 6'-(5,5,8,8-Tetramethyl-5,6,7,8-tetrahydro-naphthalen-2-yl)-3,4,5,6-tetrahydro-2H-[1,2']bipyrazinyl | C |
| 3-{4-[2-(5,5,8,8-Tetramethyl-5,6,7,8-tetrahydro-naphthalen-2-yl)-pyrimidin-4-yl]-piperazin-1-yl}-propan-1-ol | B |
| 2-Piperazin-1-yl-4-(5,5,8,8-tetramethyl-5,6,7,8-tetrahydro-naphthalen-2-yl)-pyrimidine | B |
| 4-{4-[6-(5,5-Dimethyl-5,6,7,8-tetrahydro-naphthalen-2-yl)-pyridin-2-yl]-piperazin-1-yl}-butan-1-ol | C |
| 4-Piperazin-1-yl-2-(5,5,8,8-tetramethyl-5,6,7,8-tetrahydro-naphthalen-2-yl)-pyrimidine | B |
| 5-{4-[6-(5,5-Dimethyl-5,6,7,8-tetrahydro-naphthalen-2-yl)-pyridin-2-yl]-piperazin-1-yl}-pentan-1-ol | B |
| 3-{4-[6-(5,5-Dimethyl-5,6,7,8-tetrahydro-naphthalen-2-yl)-pyridin-2-yl]-piperazin-1-yl}-propan-1-ol | C |
| 1-[6-(5,5-Dimethyl-5,6,7,8-tetrahydro-naphthalen-2-yl)-pyridin-2-yl]-piperazine | C |
| 4-{4-[6-(5,5,8,8-Tetramethyl-5,6,7,8-tetrahydro-naphthalen-2-yl)-pyridin-2-yl]-piperazin-1-yl}butan-1-ol | A |
| 5-{4-[6-(5,5,8,8-Tetramethyl-5,6,7,8-tetrahydro-naphthalen-2-yl)-pyridin-2-yl]-piperazin-1-yl}-pentan-1-ol | A |
| 3-{4-[6-(5,5,8,8-Tetramethyl-5,6,7,8-tetrahydro-naphthalen-2-yl)-pyridin-2-yl]-piperazin-1-yl}-propan-1-ol | B |
| 1-[6-(5,5,8,8-Tetramethyl-5,6,7,8-tetrahydro-naphthalen-2-yl)-pyridin-2-yl]-piperazine | B |

## Patentansprüche

1. Verbindungen gemäß der Formel (I) 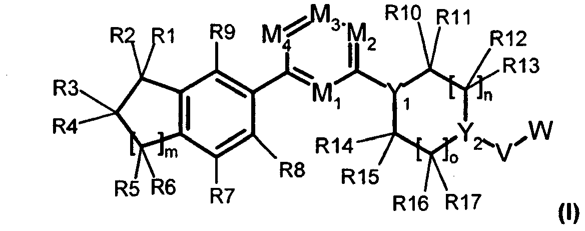 worin jeweils unabhängig voneinander bedeuten:
R¹_{,} R², R³, R⁴,
R⁵, R⁶, R⁷, R⁸, R⁹,
R¹⁰, R¹¹, R¹², R¹³, R¹⁴, R¹⁵, R¹⁶, R¹⁷ H, D (Deuterium), A, OR¹⁸, CN, F, Cl und NR¹⁸R^{18'}; wobei R¹ und R², R³ und R⁴, R⁵ und R⁶, R¹⁰ und R¹¹, R¹² und R¹³, R¹⁴ und R¹⁵, R¹⁶ und R¹⁷ zusammen jeweils auch =O (Carbonylsauerstoff) bilden können; wobei R⁹ und R¹, R¹ und R², R² und R³, R³ und R⁴, R⁴ und R⁵, R⁵ und R⁶, R⁶ und R⁷, R⁷ und R⁸, R¹⁰ und R¹¹, R¹¹ und R¹², R¹² und R¹³ R¹⁴ und R¹⁵, R¹⁵ und R¹⁶, R¹⁶ und R¹⁷ zusammen jeweils auch cyclisches Alkyl mit 3, 4, 5, 6 oder 7 C-Atomen oder Het mit 3, 4, 5, 6 oder 7 Ringatomen bilden können; wobei R¹⁰ und R¹⁹ wenn Y₁ = CR¹⁹, R¹¹ und R¹², R¹³ und R¹⁹, wenn Y₂ = CR¹⁹, R¹⁴ und R¹⁹, wenn Y₁ = CR¹⁹, R¹⁵ und R¹⁶, R¹⁷ und R¹⁹, wenn Y₂ = CR¹⁹ zusammen jeweils auch mit der Einfachbindung und den C-Atomen, an denen sie befestigt sind, eine C=C-Doppelbindung bilden können;
R¹⁸, R^{18'} H, D oder A;
R¹⁹, R^{19'} H, D, A, OR¹⁸, NR¹⁸R^{18'}, F, Cl, Br, CN oder Het;
M₁, M₂, M₃, M₄ CR¹⁹ oder N;
Y₁, Y₂ CR¹⁹ oder N;
V C(R¹⁹)(R¹⁹), NR¹⁹ oder fehlend;
W [C(R¹⁹)(R^{19'})]ₚZ, CO-[C(R¹⁹)(R^{19'})]ₚZ, [C(R¹⁹)(R^{19'})]ₚN(R¹⁹)-Z, CO-N(R¹⁹)-[C(R¹⁹)(R¹⁹)]ₚZ, N(R¹⁹)-CO-[C(R¹⁹)(R^{19'})]ₚZ, CO-O-[C(R¹⁹)(R¹⁹)]ₚZ, C(O)OR¹⁸, OR¹⁸, H oder D;
wobei V, W und Y₂ zusammen jeweils auch cyclisches Alkyl mit 3, 4, 5, 6 oder 7 C-Atomen, worin bevorzugt 1, 2, 3, 4, 5, 6 oder 7 H-Atome durch F, Cl, Br, CN und/oder OH, OR¹⁹, OC(O)R¹⁹, NR¹⁹C(O)OZ, C(O)OR¹⁹, C(O)N(R¹⁹)(R^{19'}) oder N(R¹⁹)(R^{19'}) ersetzt sein können; oder Het mit 3, 4, 5, 6 oder 7 Ringatomen bilden können, wobei Het bevorzugt einen gesättigten, ungesättigten oder aromatischen Heterocyclus mit 1 bis 4 N-, O- und/oder S-Atomen darstellt, der unsubstituiert oder ein-, zwei- oder dreifach durch Hal, F, Cl, Br, CN, A, OR¹⁸, W, SR¹⁸, NO₂, N(R¹⁹)(R^{19'}), NR¹⁸COOZ, OCONHZ, NR¹⁸SO₂Z, SO₂N(R¹⁸)Z, S(O)ₘZ, COZ, CHO, COZ, =S, =NH, =NA, Oxy (-O⁻) und/oder =O (Carbonylsauerstoff) substituiert sein kann;
Z Het, Ar oder A;
A unverzweigtes oder verzweigtes Alkyl mit 1, 2, 3, 4, 5, 6, 7, 8, 9 oder 10 C-Atomen, worin 1, 2, 3, 4, 5, 6 oder 7 H-Atome durch F, Cl, Br, CN und/oder OH, OR¹⁹, OC(O)R¹⁹, NR¹⁹C(O)OZ, C(O)OR¹⁹, C(O)N(R¹⁹)(R^{19'}) oder N(R¹⁹)(R^{19'}) ersetzt sein können; und/oder worin eine oder zwei CH₂-Gruppen durch O, S, SO, SO₂, CO, COO, NR¹⁸, NR¹⁸CO, CONR¹⁸, cyclisches Alkyl mit 3, 4, 5, 6 oder 7 C-Atomen, CH=CH und/oder CH≡CH-Gruppen ersetzt sein können; oder cyclisches Alkyl mit 3, 4, 5, 6 oder 7 C-Atomen, worin bevorzugt 1, 2, 3, 4, 5, 6 oder 7 H-Atome durch F, Cl, Br, CN und/oder OH, OR¹⁹, OC(O)R¹⁹, NR¹⁹C(O)OZ, C(O)OR¹⁹, C(O)N(R¹)(R^{19'}) oder N(R¹⁹)(R^{19'}) ersetzt sein können;
Ar ein-, zwei- oder dreifach durch Hal, F, Cl, Br, CN, A, OR¹⁸, W, SR¹⁸, NO₂, N(R¹⁹)(R^{19'}), NR¹⁸COOZ, OCONHZ, NR¹⁸SO₂Z, SO₂N(R¹⁸)Z, S(O)ₘZ, COZ, CHO, COZ substituiertes Phenyl, Naphthyl oder Biphenyl,
Het jeweils unabhängig voneinander einen ein-, zwei- oder dreikernigen gesättigten, ungesättigten oder aromatischen Heterocyclus mit 1 bis 4 N-, O- und/oder S-Atomen, der unsubstituiert oder ein-, zwei- oder dreifach durch Hal, F, Cl, Br, CN, A, OR¹⁸, W, SR¹⁸, NO₂, N(R¹⁹)(R^{19'}), NR¹⁸COOZ, OCONHZ, NR¹⁸SO₂Z, SO₂N(R¹⁸)Z, S(O)ₘZ, COZ, CHO, COZ, =S, =NH, =NA, Oxy (-O⁻) und/oder =O (Carbonylsauerstoff) substituiert sein kann,
m 1, 2 oder 3,
n, o 0, 1 oder 2,
p 0, 1, 2, 3 oder 4
mit der Maßgabe, dass Verbindungen der Formel (I) ausgeschlossen sind, worin
(a) V fehlend ist, und
(b) W = C(O)-CH₂-Het ist;
**mit der weiteren Maßgabe, dass die folgende Verbindung ebenfalls ausgeschlossen ist:** 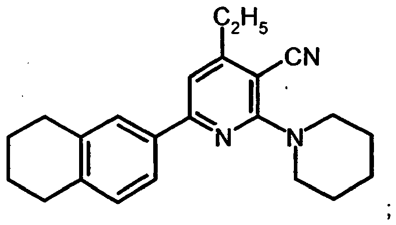 sowie ihre physiologisch unbedenklichen Salze, Solvate, Tautomere und Stereoisomere, einschließlich deren Mischungen in allen Verhältnissen.

2. Verbindungen gemäß Anspruch 1, worin jeweils unabhängig voneinander bedeuten: R¹ R², R³, R⁴, R⁵, R⁶, R⁷, R⁸, R⁹, R¹⁰, R¹¹, R¹², R¹³, R¹⁴, R¹⁵, R¹⁶, R¹⁷ H, D, A, OR¹⁸ oder NR¹⁸R^{18'};
wobei R¹ und R², R³ und R⁴, R⁵ und R⁶, R¹⁰ und R¹¹, R¹² und R¹³, R¹⁴ und R¹⁵,R¹⁶ und R¹⁷ zusammen jeweils auch =O (Carbonylsauerstoff) bilden können;
wobei R⁹ und R¹, R¹ und R², R² und R³, R³ und R⁴, R⁴ und R⁵, R⁵ und R⁶, R⁶und R⁷, R⁷ und R⁸, R¹⁰ und R¹¹, R¹¹ und R¹², R¹² und R¹³, R¹⁴ und R¹⁵,
R¹⁵ und R¹⁶, R¹⁶ und R¹⁷ zusammen jeweils auch cyclisches Alkyl mit 3, 4, 5, 6 oder 7 C-Atomen oder Het mit 3, 4, 5, 6 oder 7 Ringatomen bilden können;
R¹⁹, R^{19'} H, A, OR¹⁸, F;
M₁, M₂, M₃, M₄ CR¹⁹, oder
M₁ N, und M₂, M₃, M₄ CR¹⁹, oder
M₂ N, und M₁, M₃, M₄ CR¹⁹, oder
M₄ N, und M₁, M₂, M₃ CR¹⁹, oder
M₁, M₂ N, und M₃, M₄ CR¹⁹, oder
M₁, M₃ N, und M₂, M₄ CR¹⁹, oder
M₁, M₄ N, und M₂, M₃ CR¹⁹, oder
M₁, M₂, M₄ N, und M₃ CR¹⁹;
Y₁, Y₂ N, oder
Y₁ N, und Y₂ CR¹⁹, oder
Y₁ CR¹⁹ und Y₂ N;
W [C(R¹⁹)(R^{19'})]ₚZ, CO-[C(R¹⁹)(R^{19'})]ₚZ, CO-O-[C(R¹⁹)(R^{19'})]ₚZ, [C(R¹⁹)(R^{19'})]ₚN(R¹⁹)-Z, N(R¹⁹)-CO-[C(R¹⁹)(R^{19'})]ₚZ, C(O)OR¹⁸, OR¹⁸ oder H;
Z Het oder A;
m 1 oder 2;
n, o 0, 1 oder 2;
p 0, 1 oder 2;
sowie ihre physiologisch unbedenklichen Salze, Solvate, Tautomere und Stereoisomere, einschließlich deren Mischungen in allen Verhältnissen.

3. Verbindungen gemäβ Anspruch 1, ausgewählt aus der Gruppe bestehend aus: 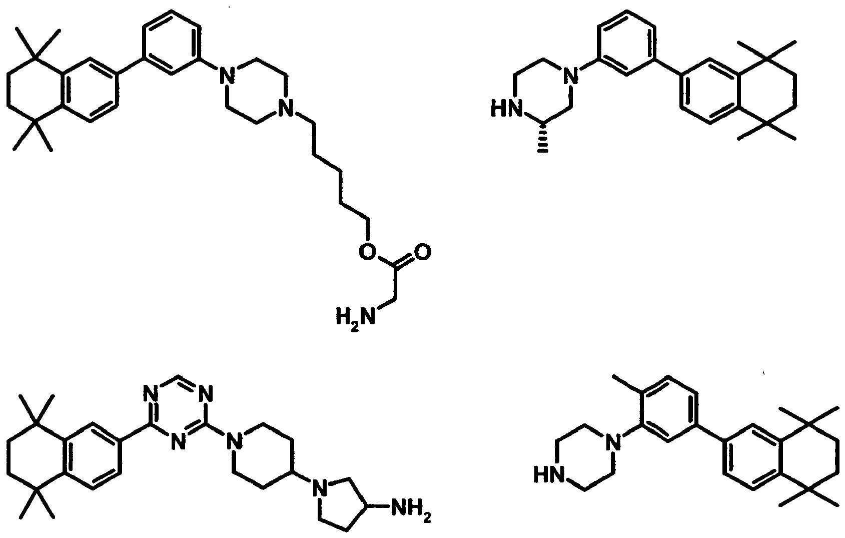 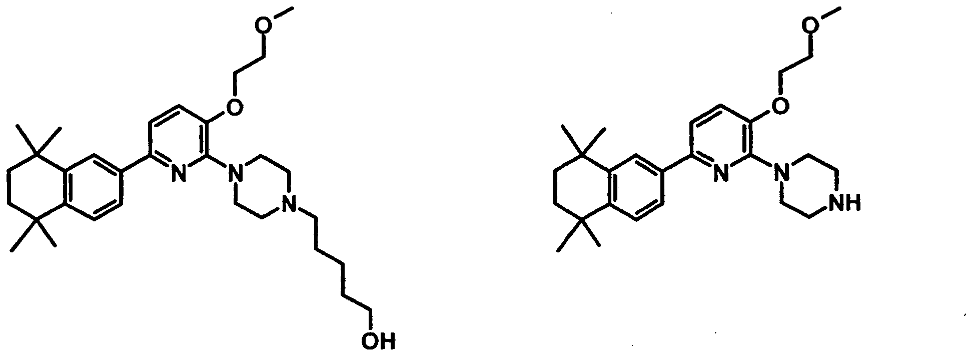 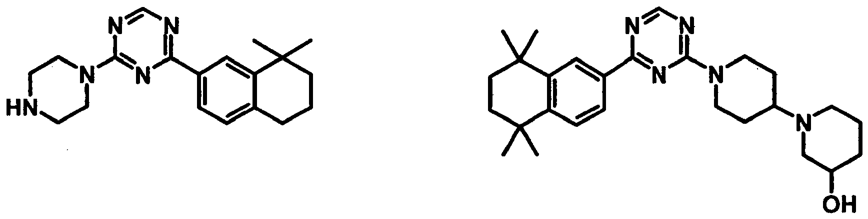 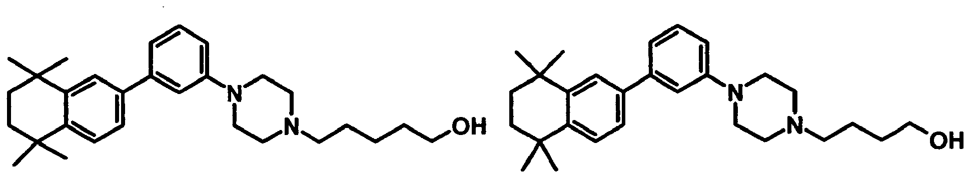 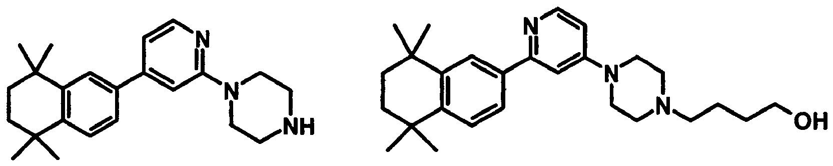 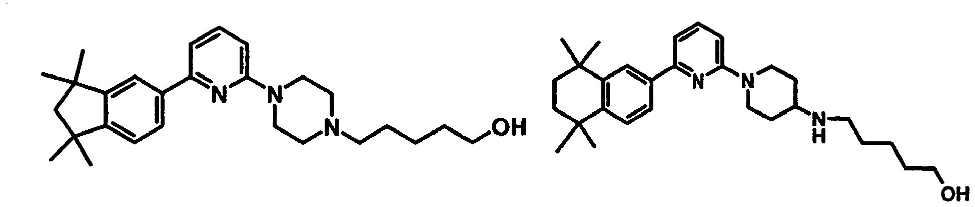 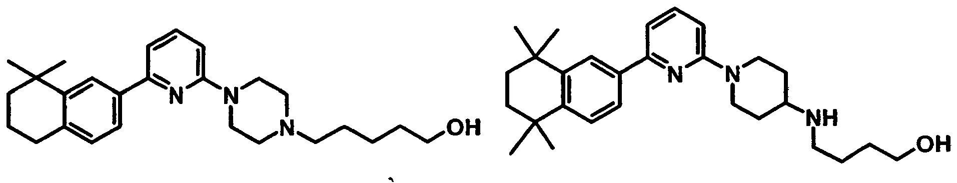 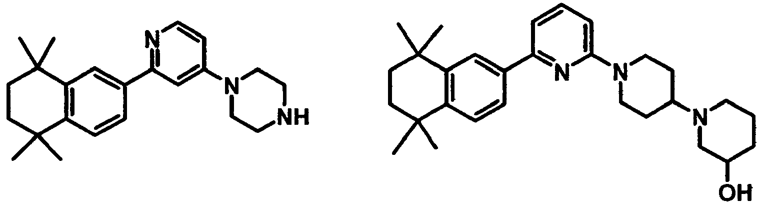 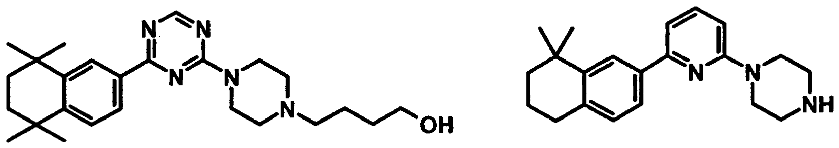 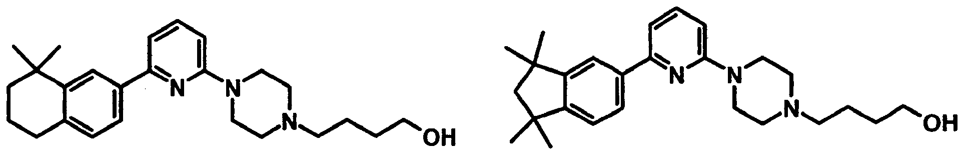 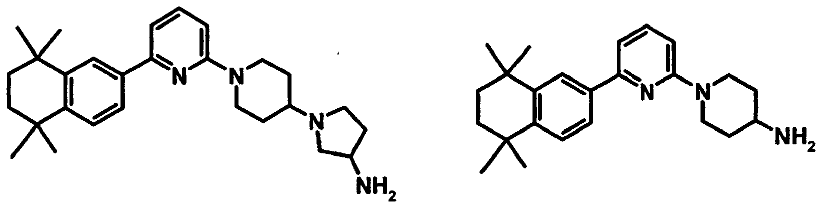 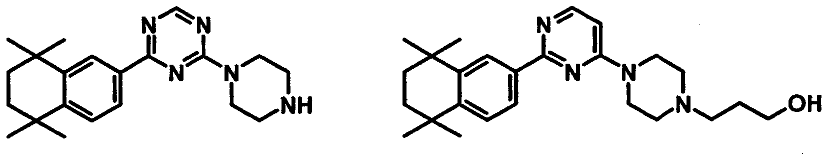 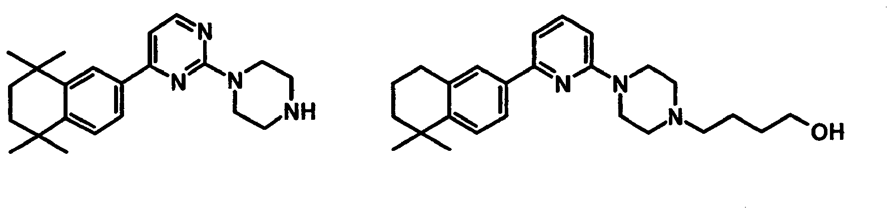 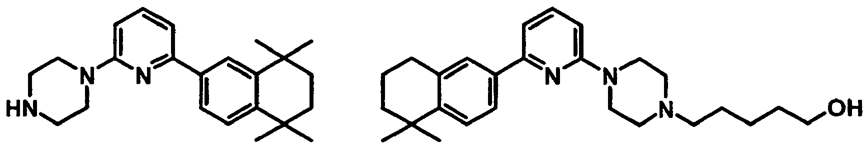 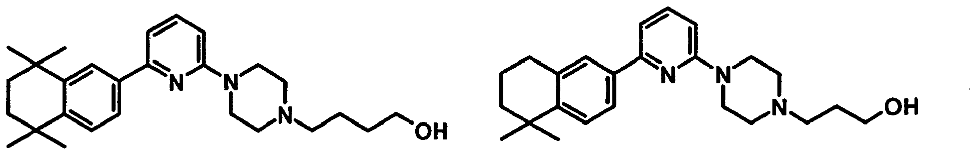 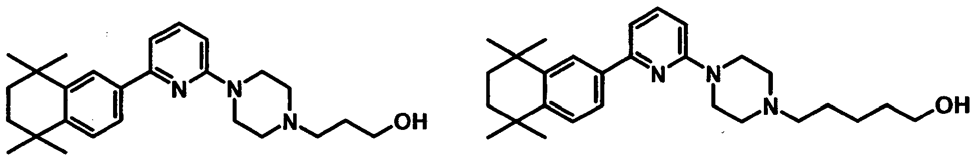 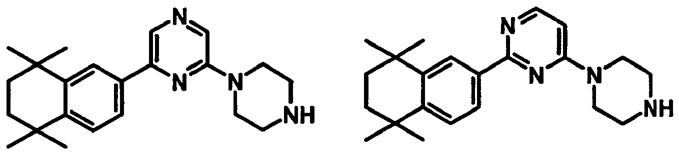 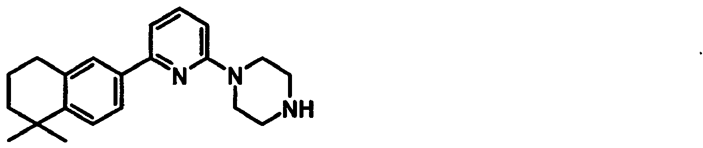 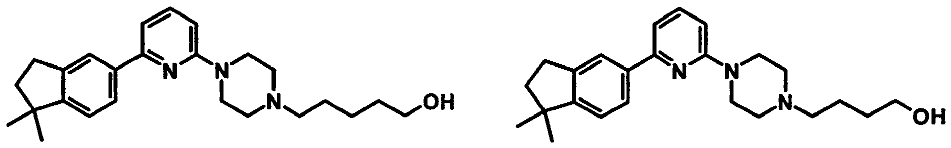 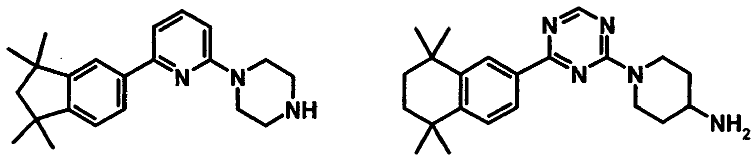 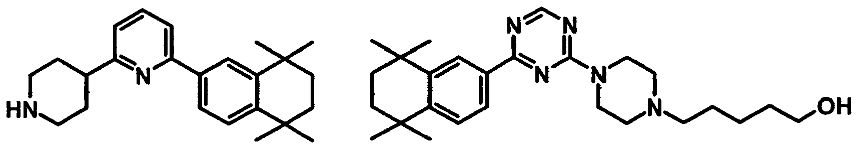 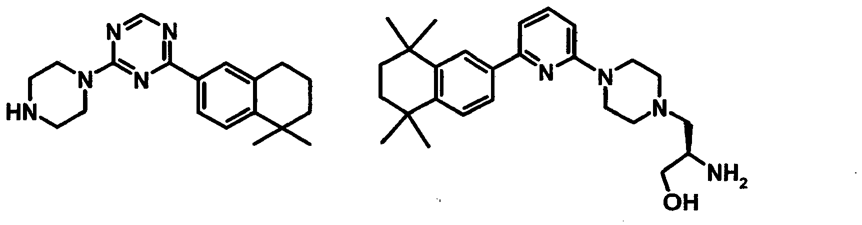 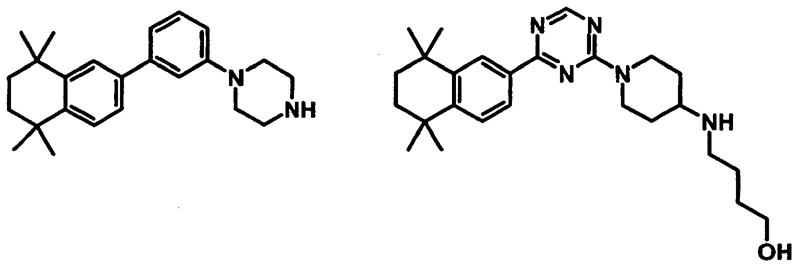 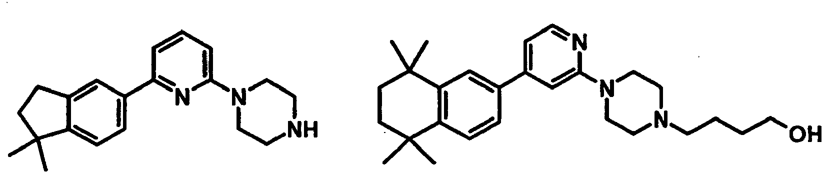 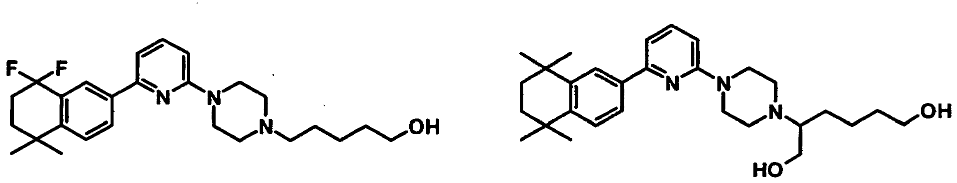 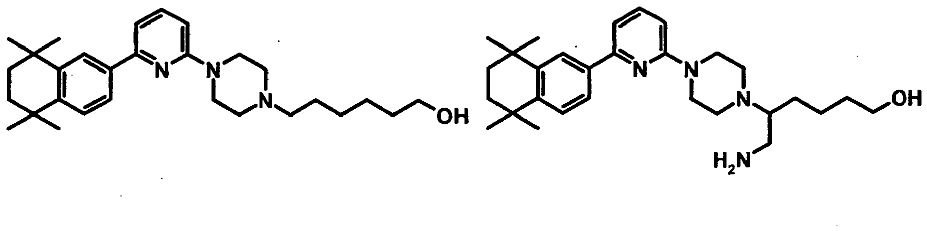 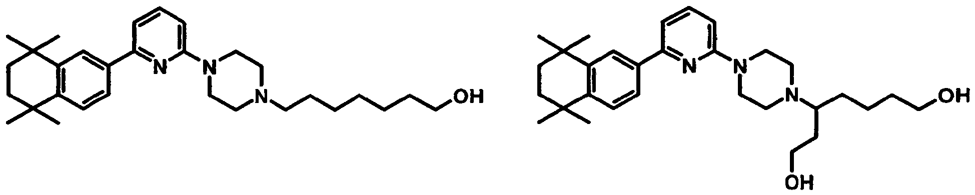 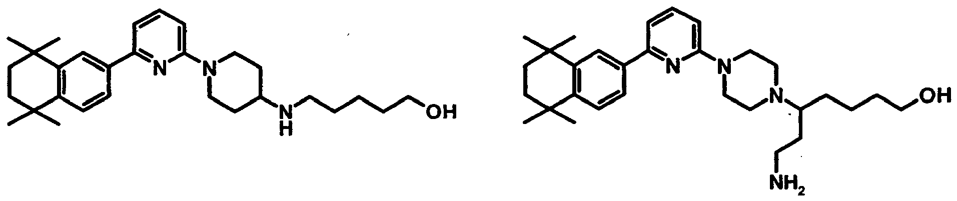 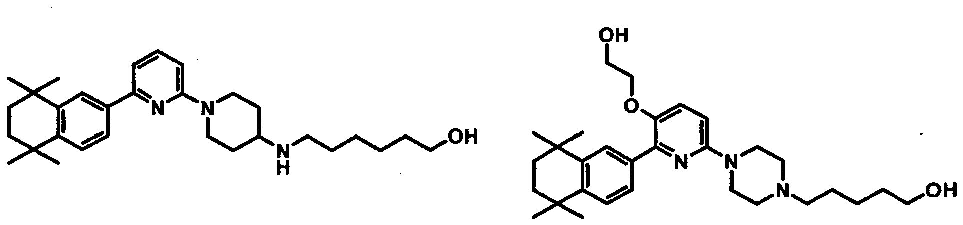 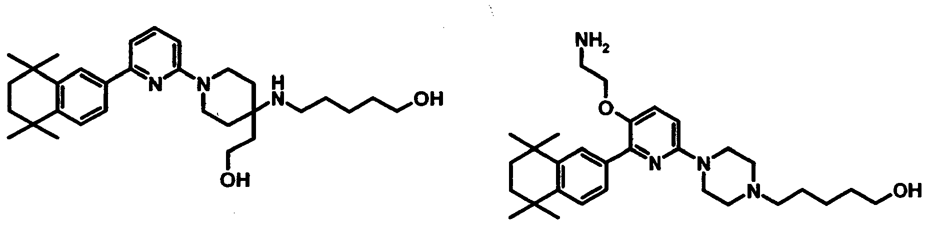 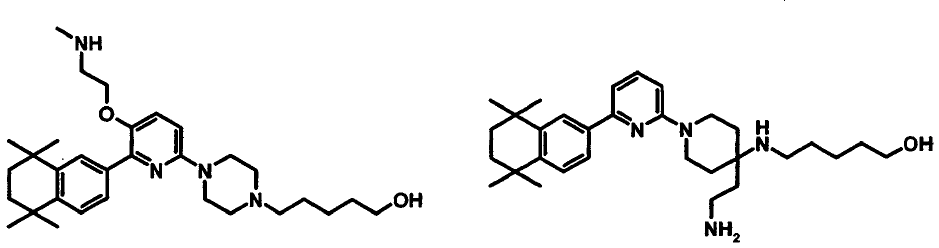 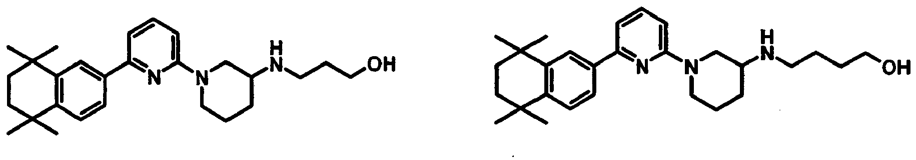 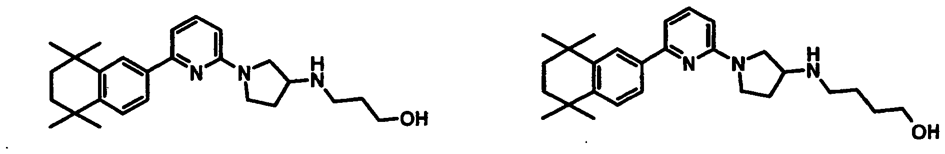 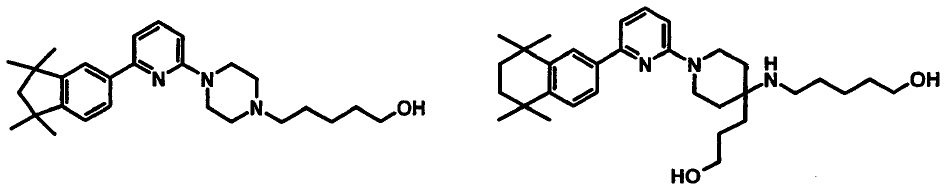 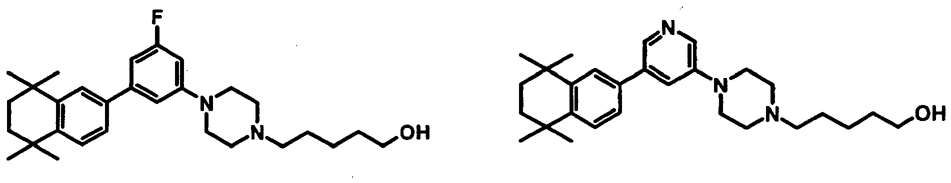 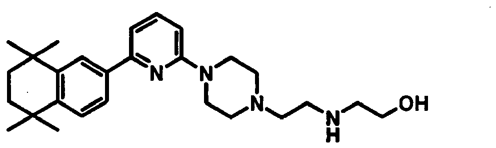 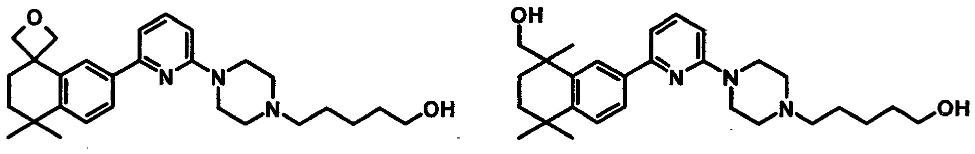 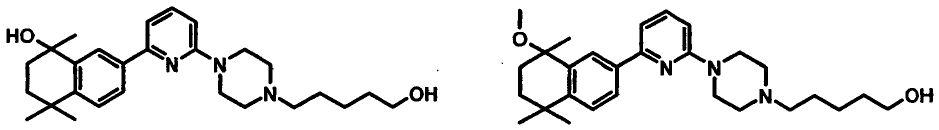 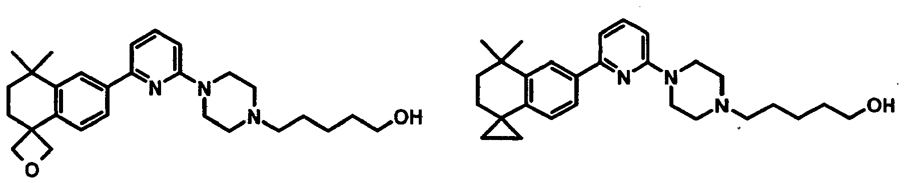 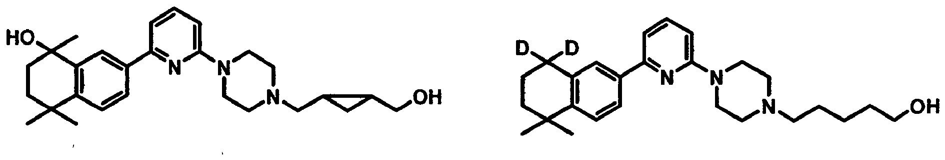 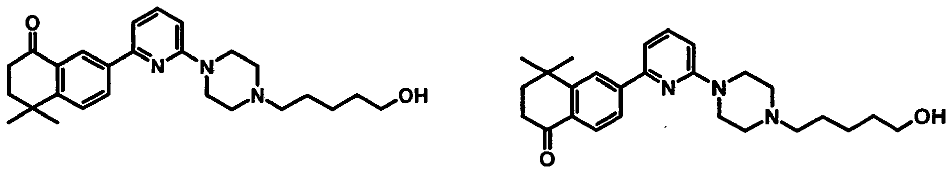 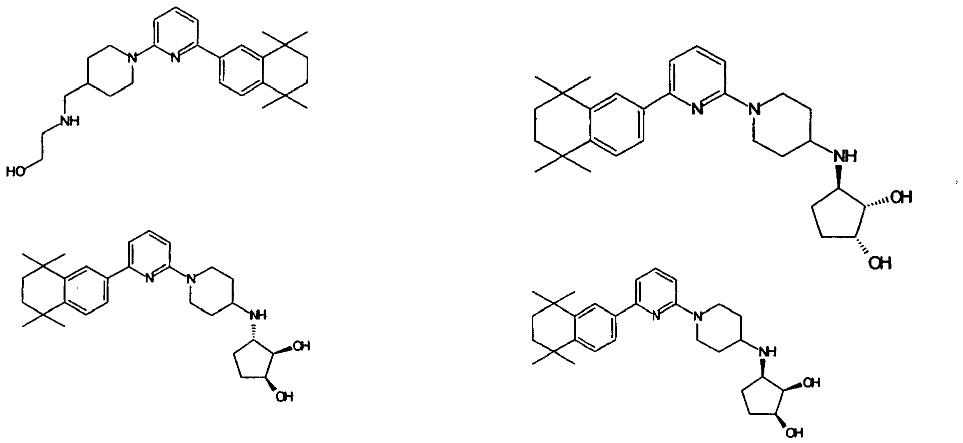 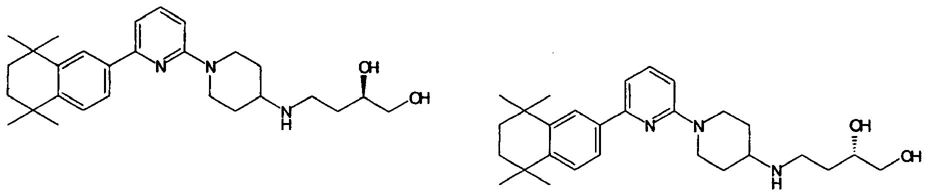 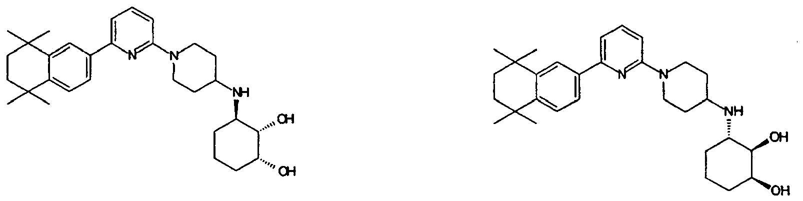 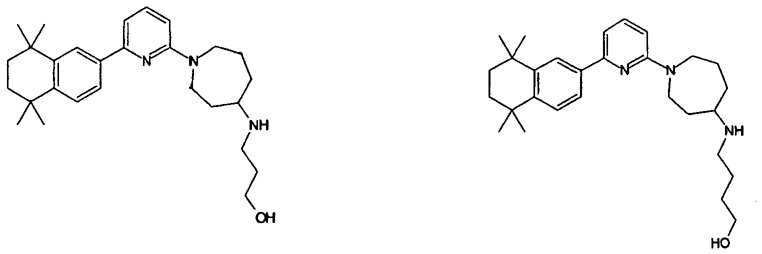 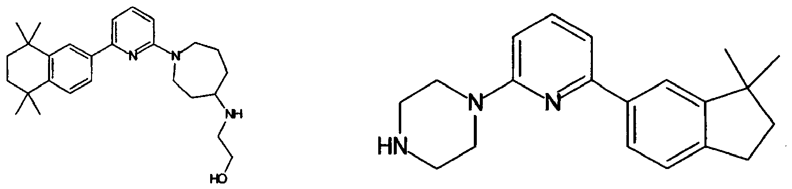 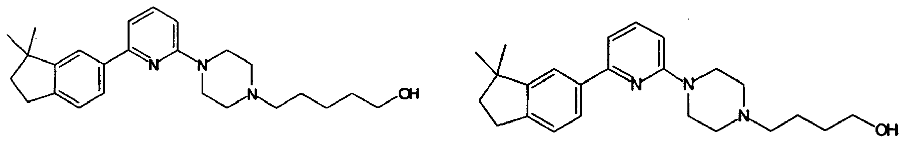 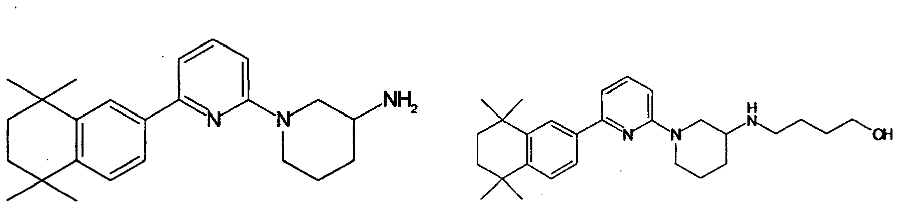 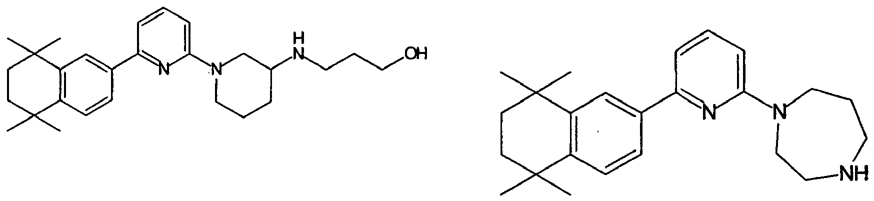 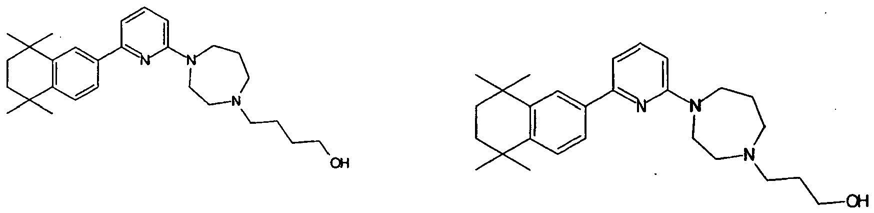 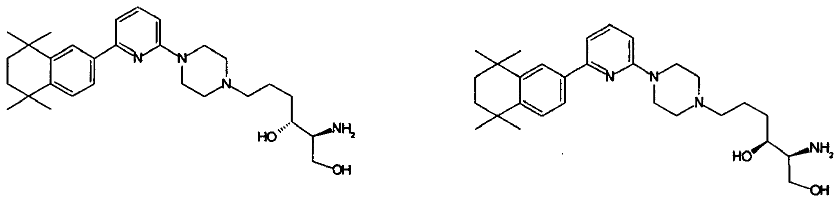 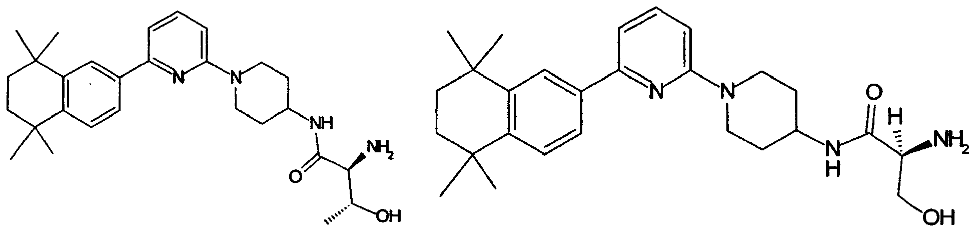 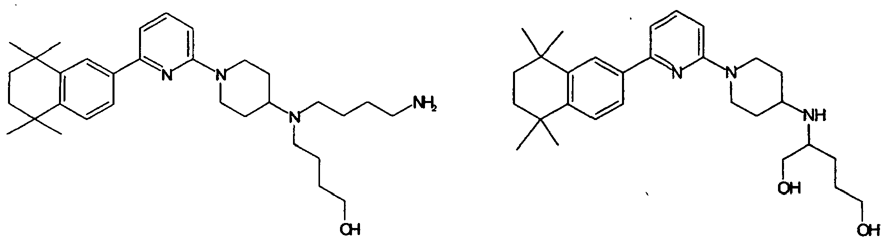 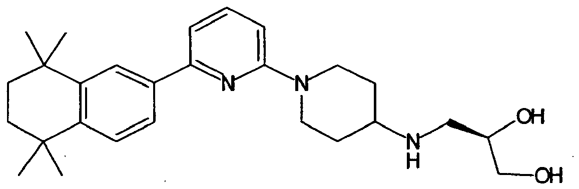 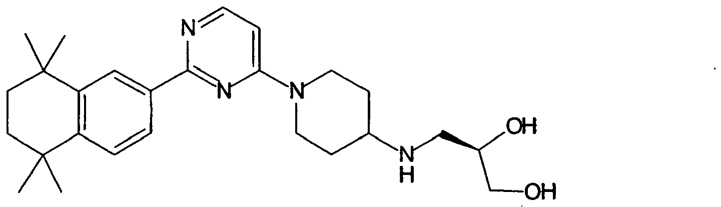 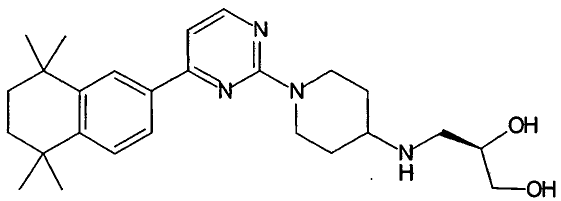 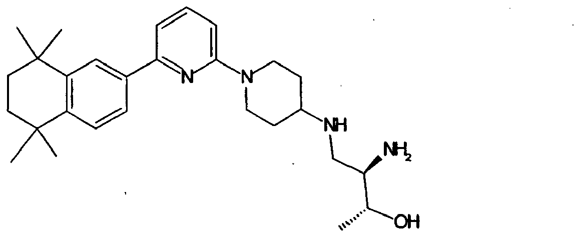 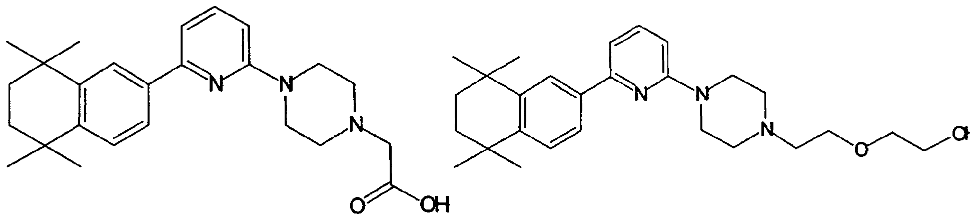 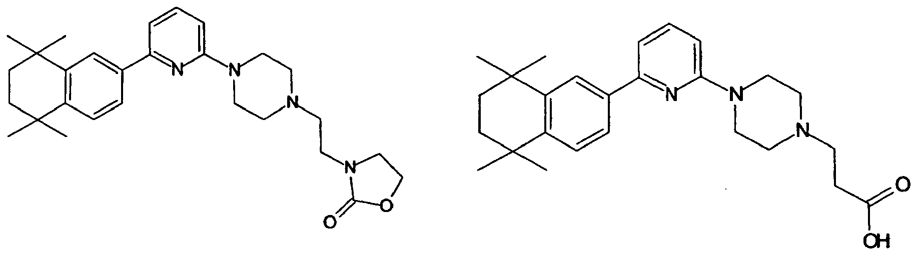 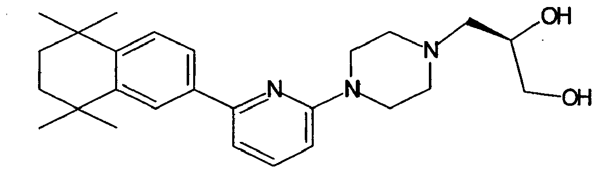 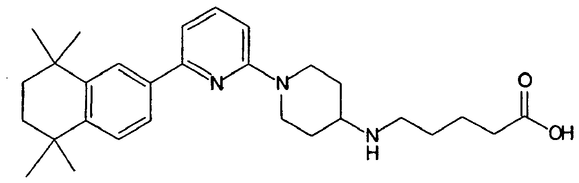 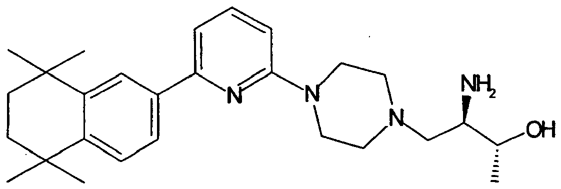 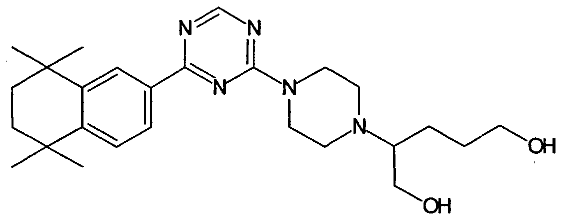 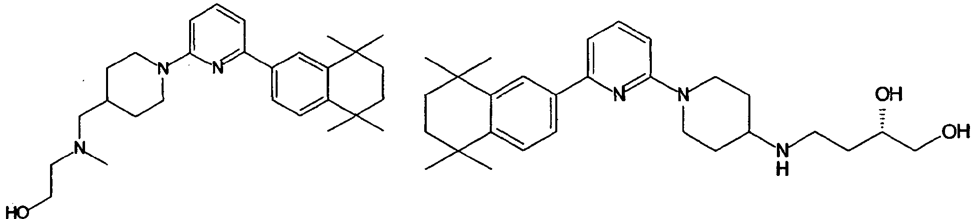 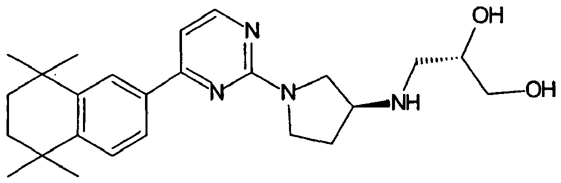 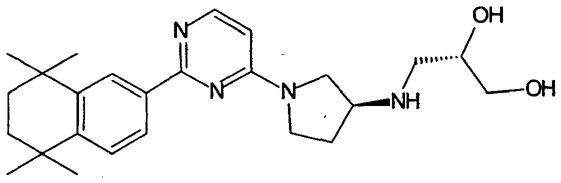 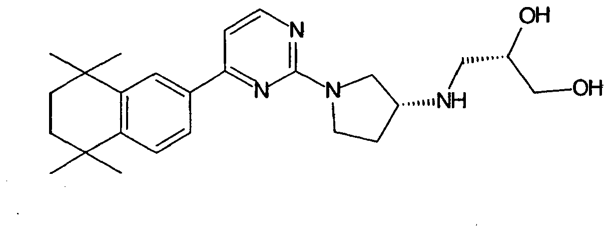 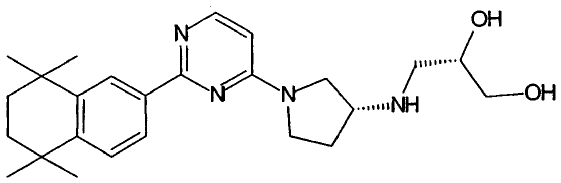 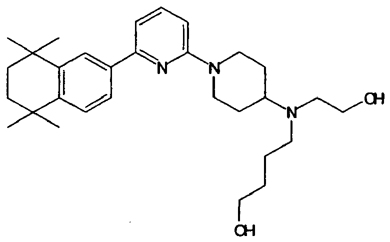 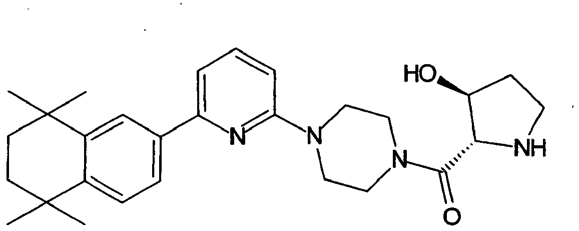 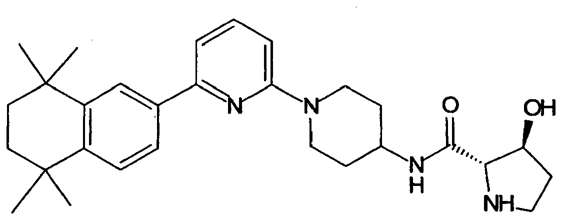 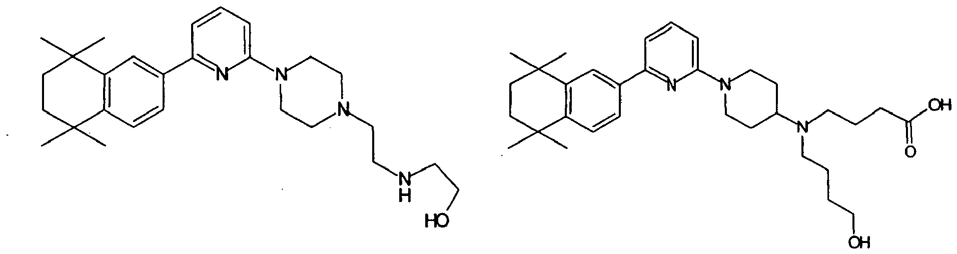 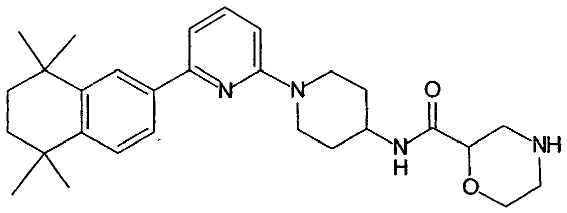 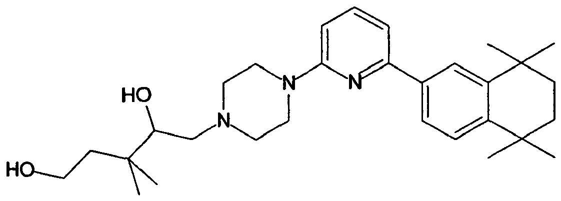 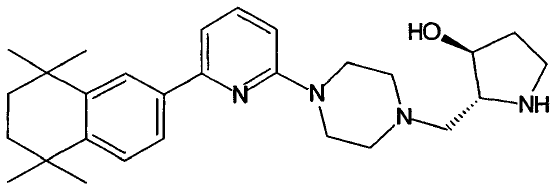 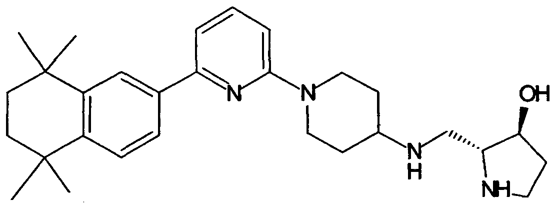 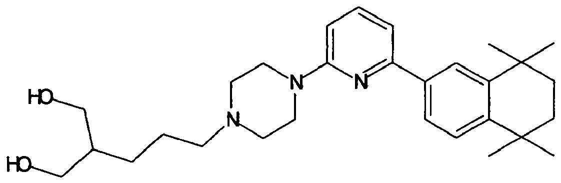 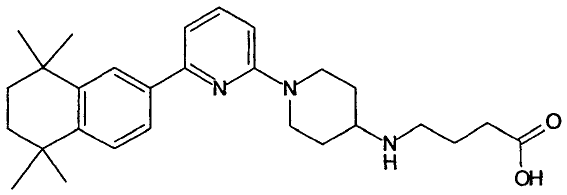 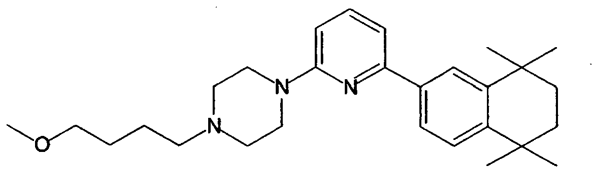 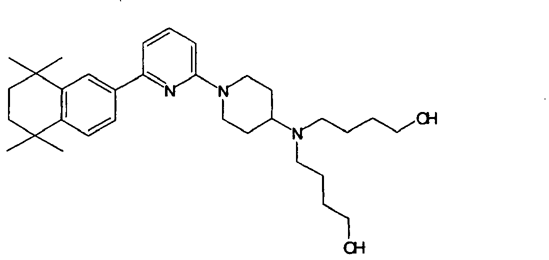 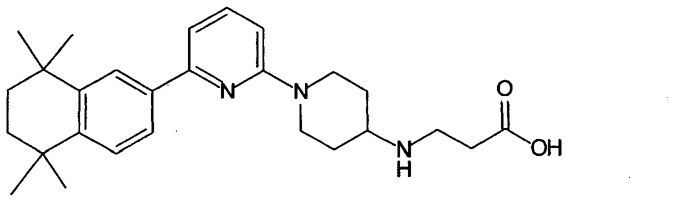 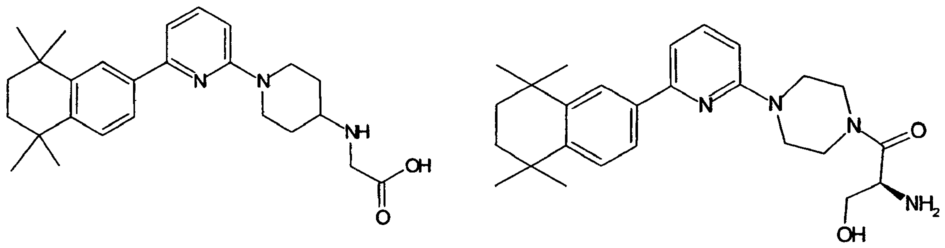 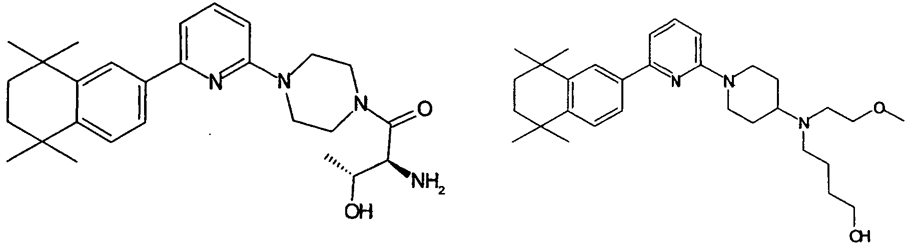 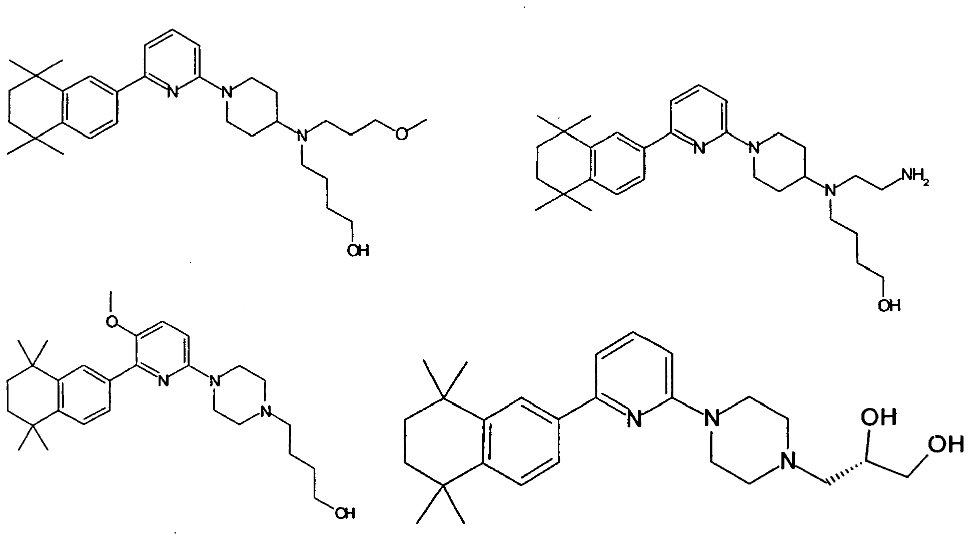 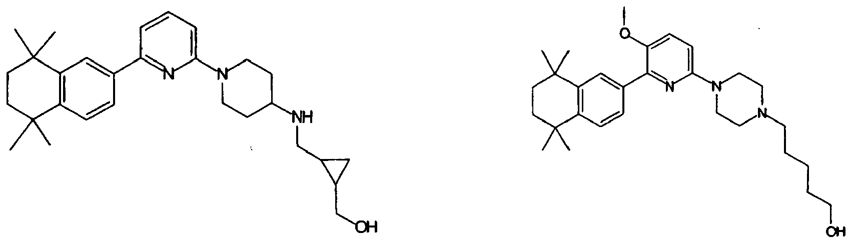 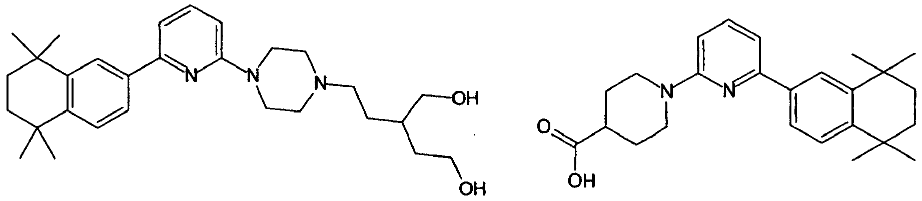 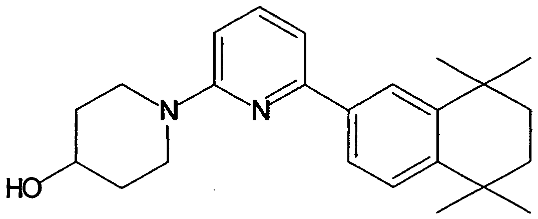 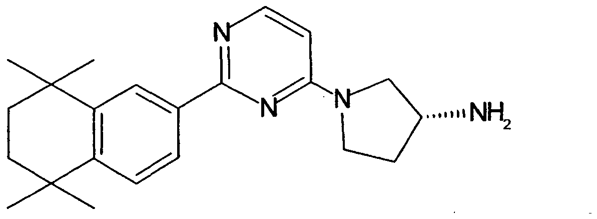 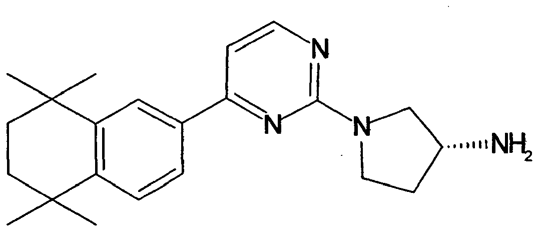 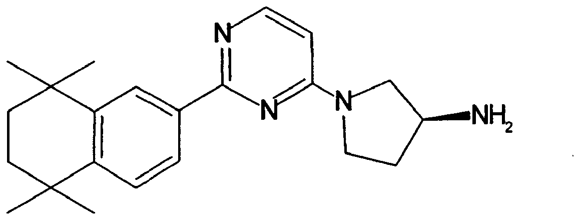 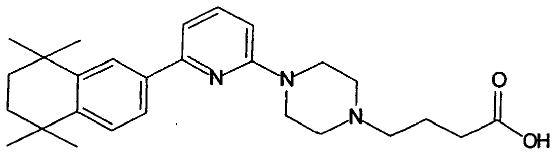 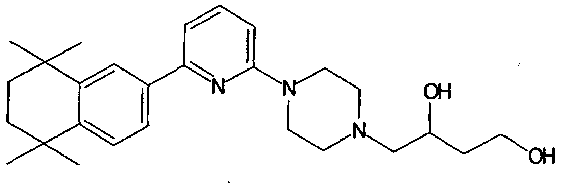 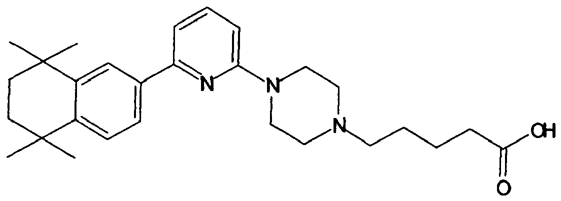 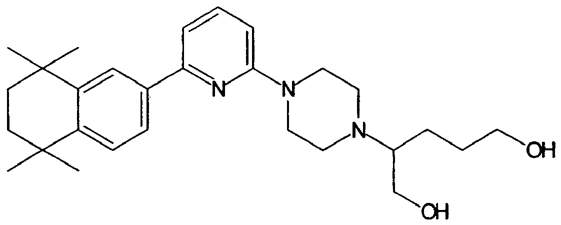 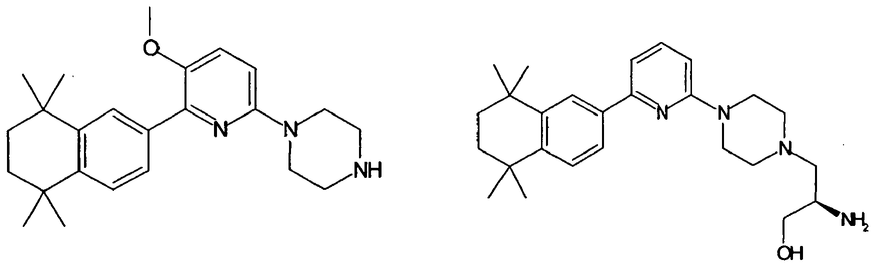 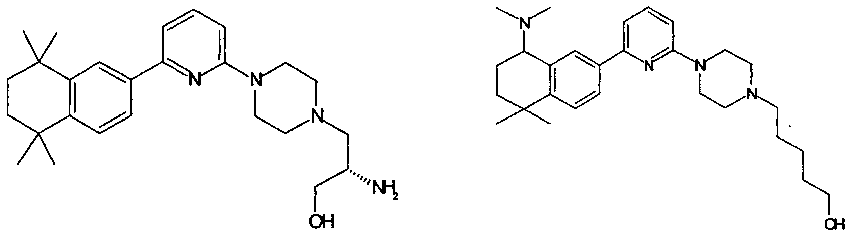 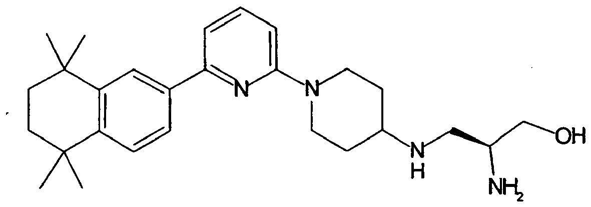 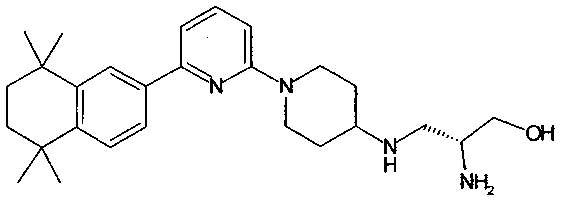 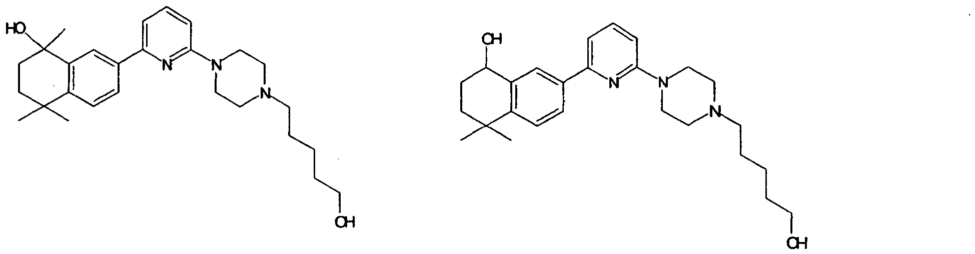 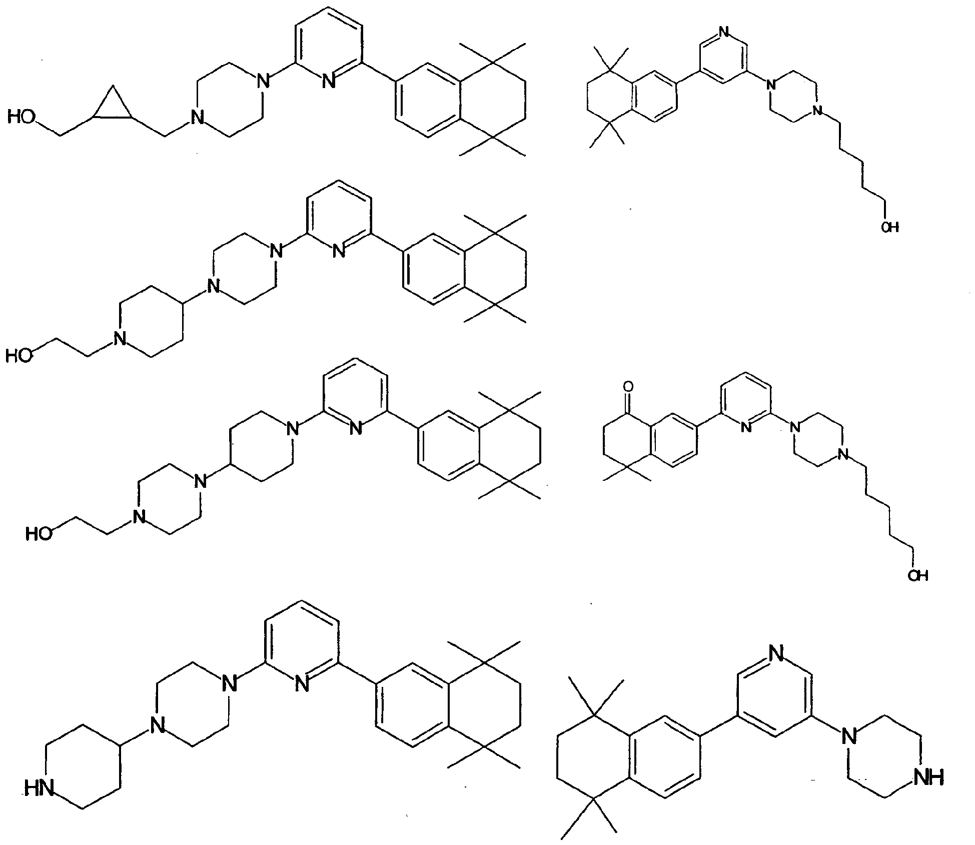 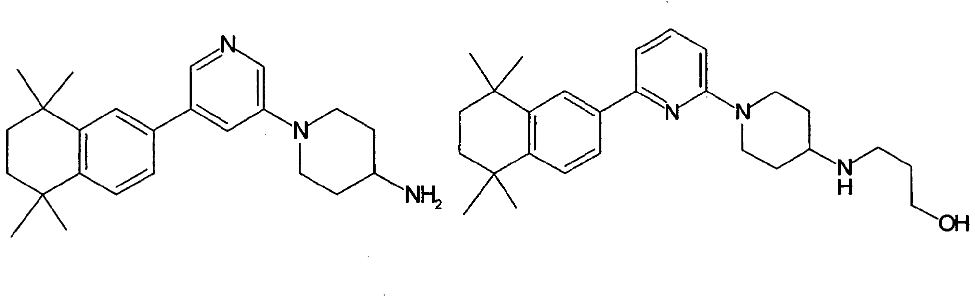 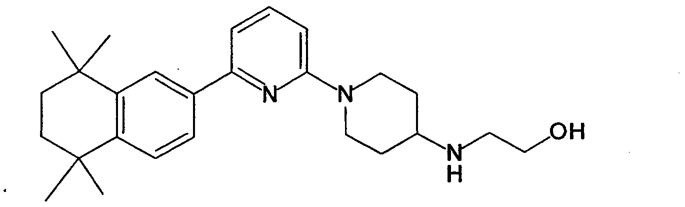 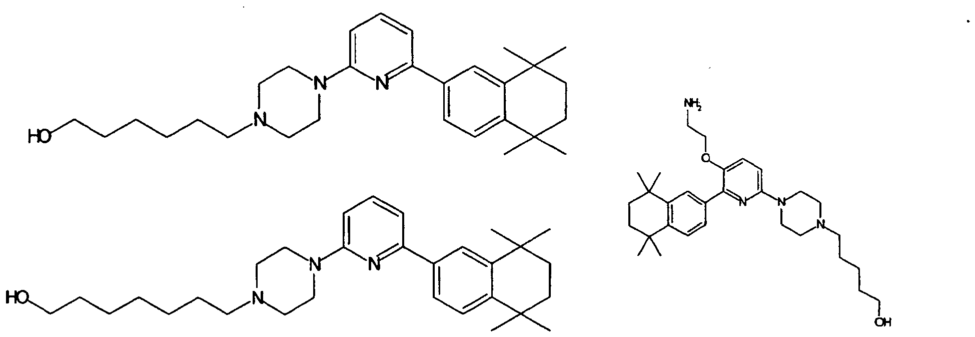 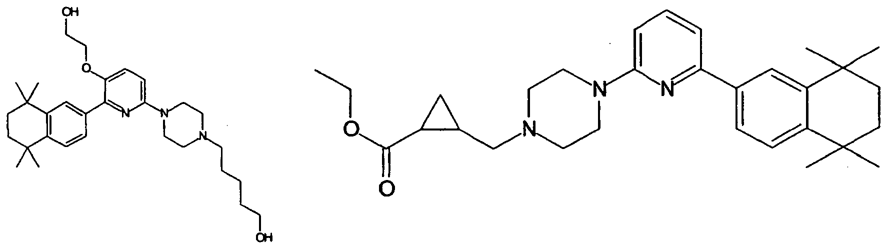 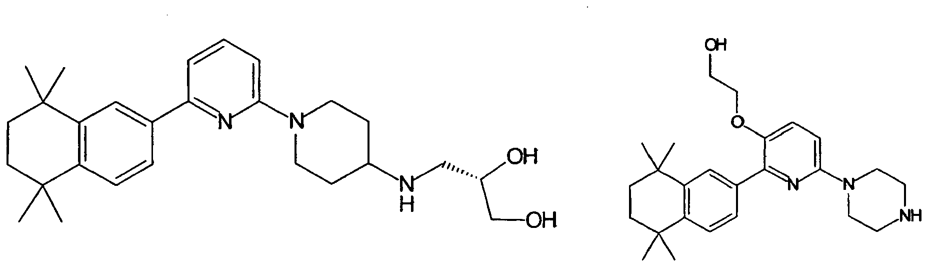 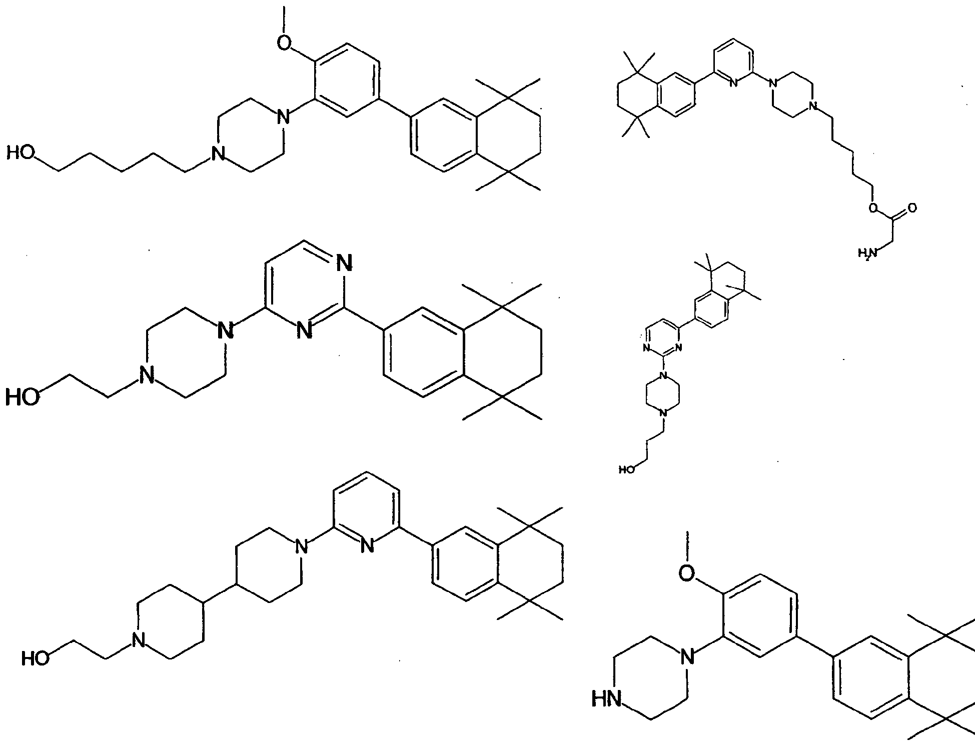 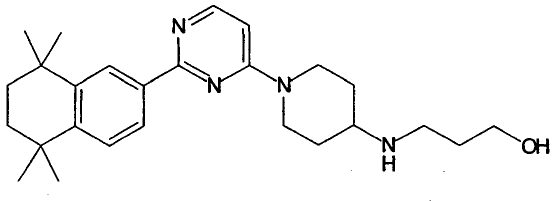 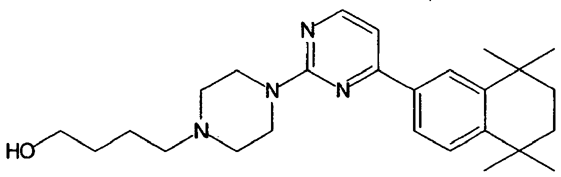 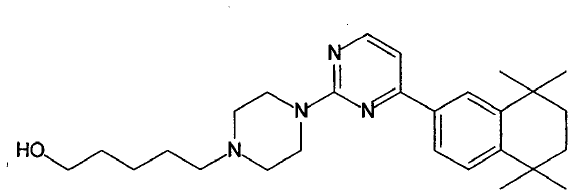 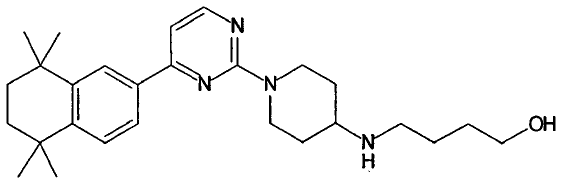 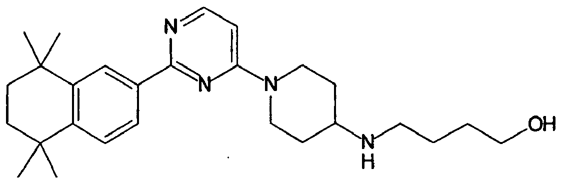 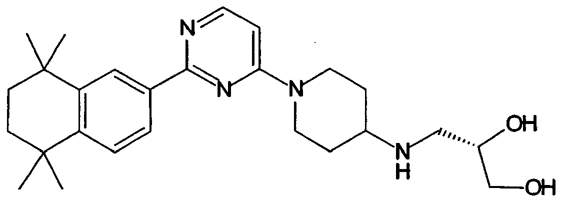 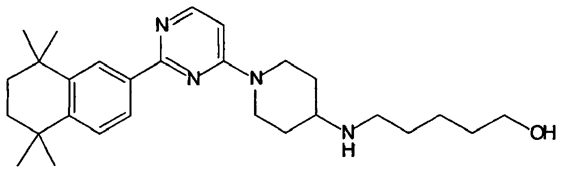 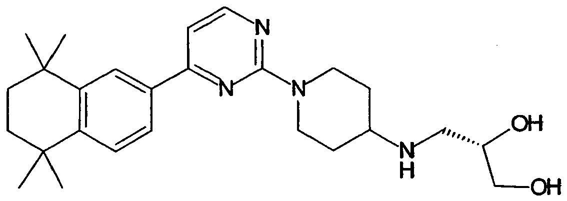 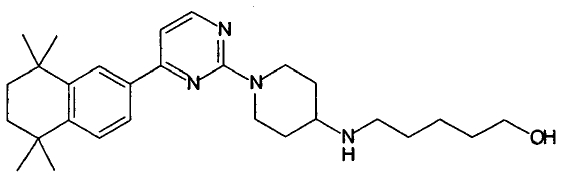 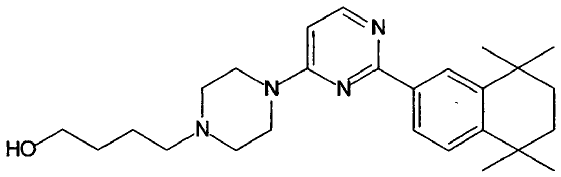 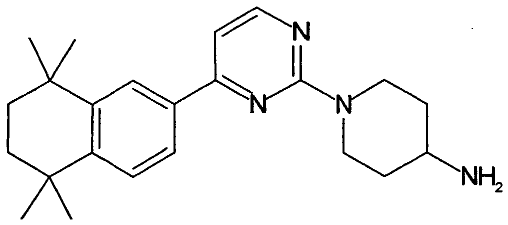 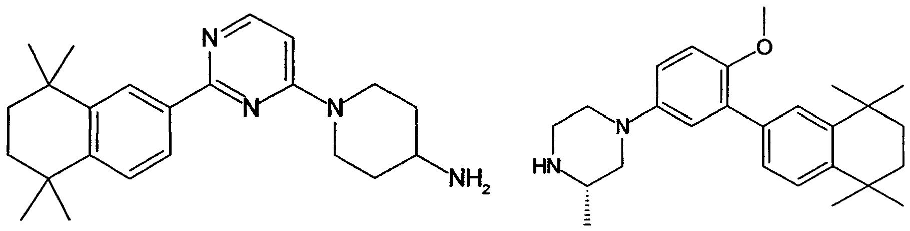 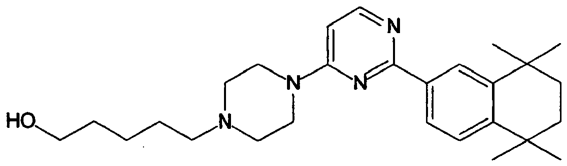 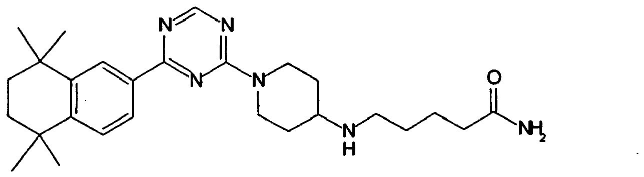 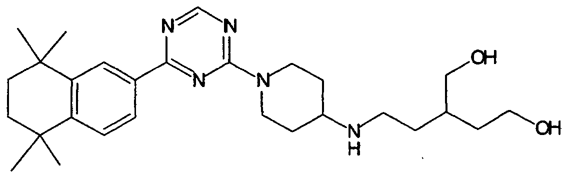 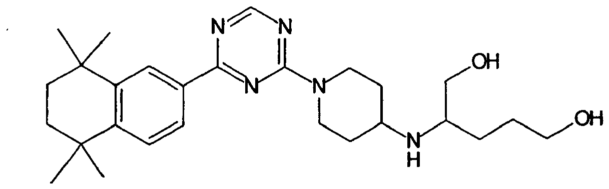 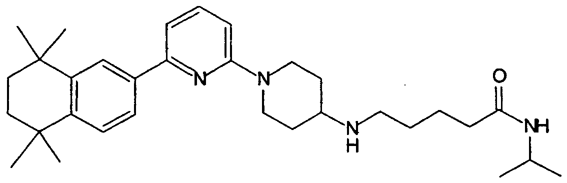 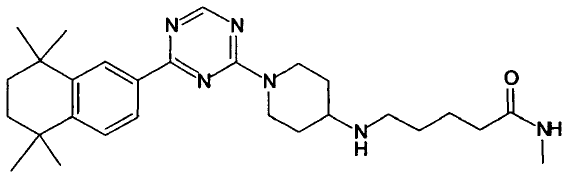 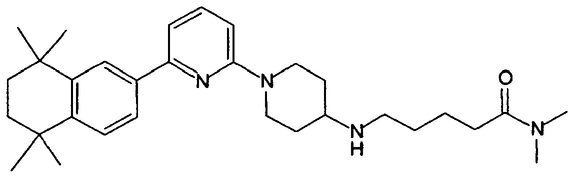 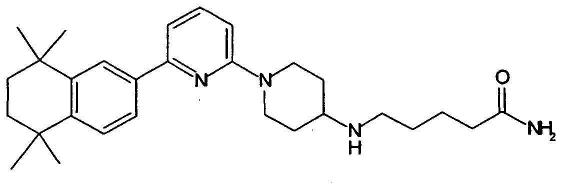 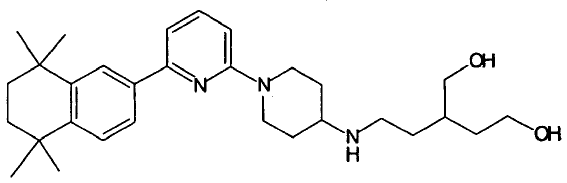 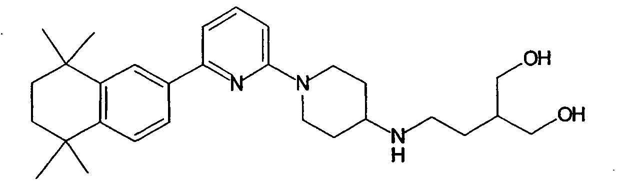 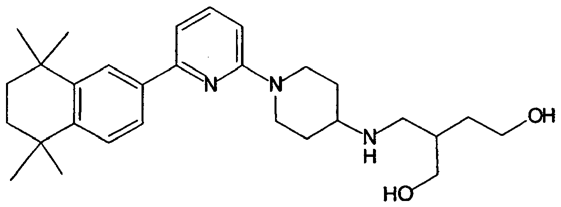 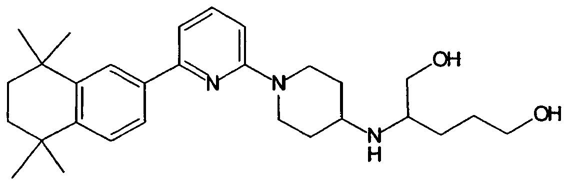 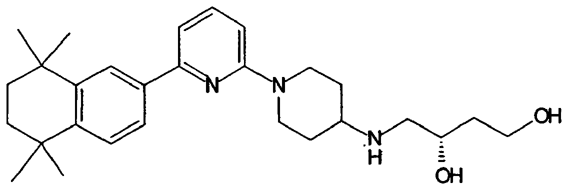 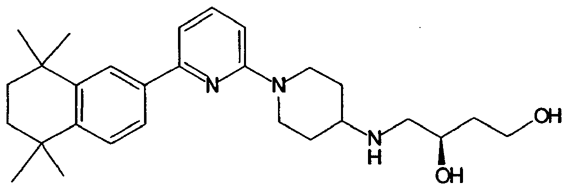 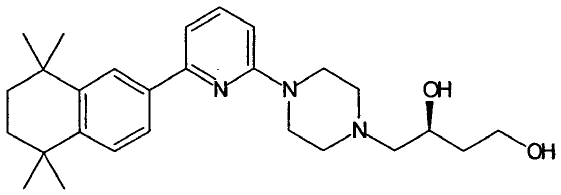 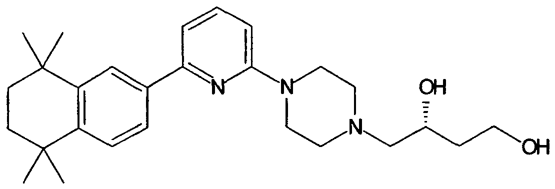 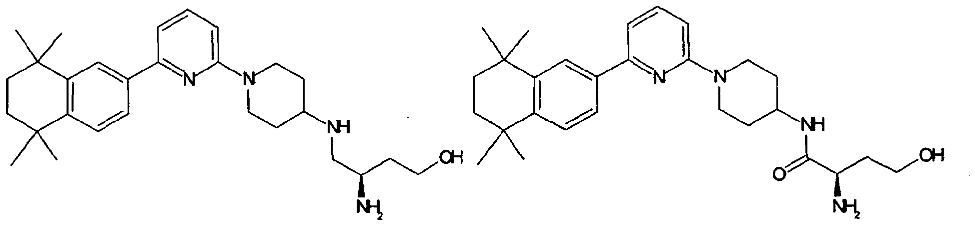 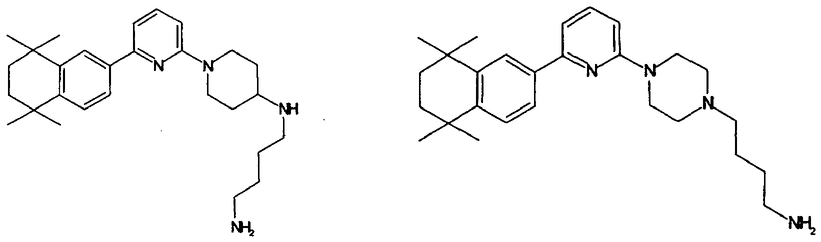 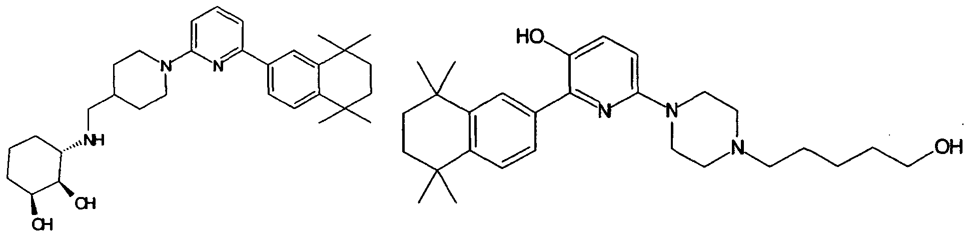 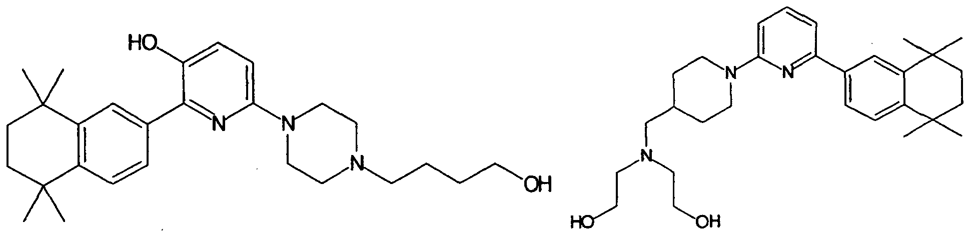 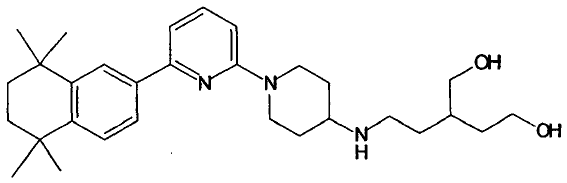 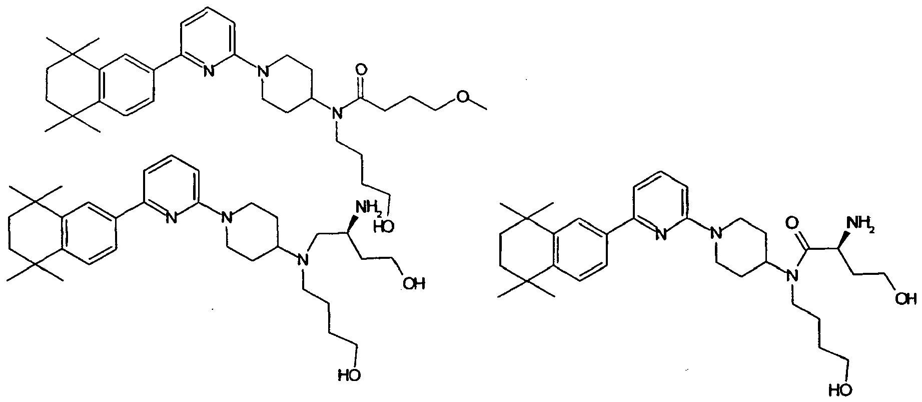 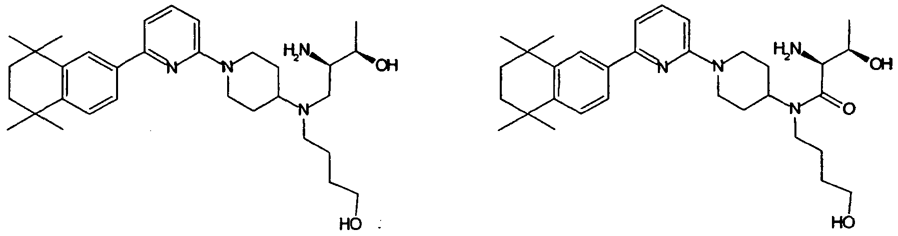 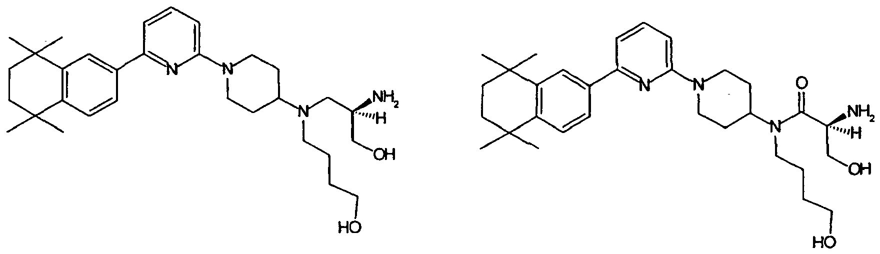 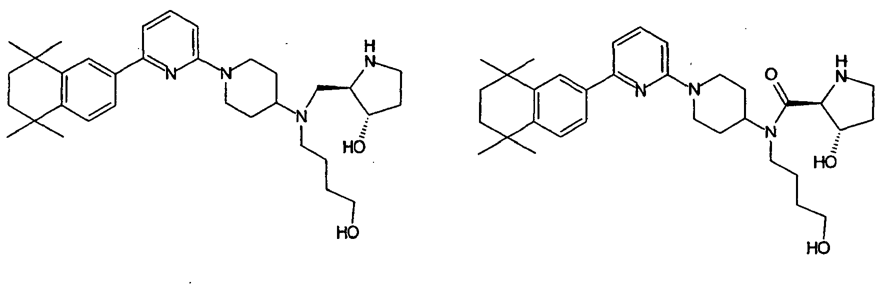 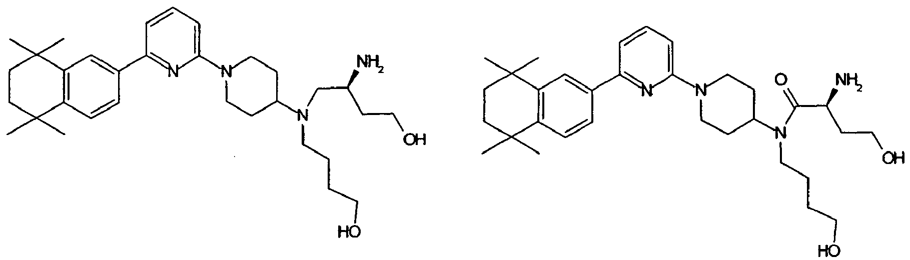 sowie ihre physiologisch unbedenklichen Salzes, Solvate, Tautomere und Stereoisomere, einschließlich deren Mischungen in allen Verhältnissen.

4. Verfahren zur Herstellung von Verbindungen gemäß einem der Ansprüche 1 bis 3 sowie ihrer physiologisch unbedenklichen Salze,
Solvate, Tautomere und Stereoisomere, **dadurch gekennzeichnet, dass** man
(a) eine Verbindung der Formel (II) 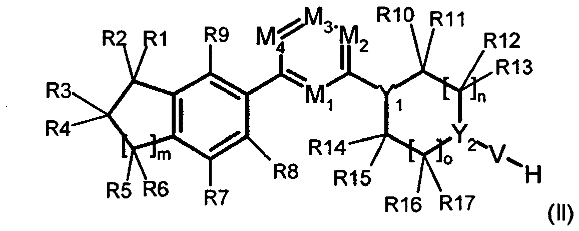 worin R¹, R², R³, R⁴, R⁵, R⁶, R⁷, R⁸, R⁹, R¹⁰, R¹¹, R¹², R¹³, R¹⁴, R¹⁵, R¹⁶, R¹⁷, M₁, M₂, M₃, M₄, Y₁, Y₂, V, n, m und o die in Anspruch 1 angegebenen Bedeutungen haben,
mit einer Verbindung der Formel (III)
L-W (III)
worin W die in Anspruch 1 angebene Bedeutung hat und L Cl, Br, I oder eine freie oder reaktionsfähig funktionell abgewandelte OH-Gruppe bedeutet, umsetzt,
oder
(b) eine Verbindung der Formel (IV) 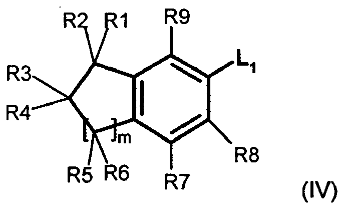 worin R¹, R², R³, R⁴, R⁵, R⁶, R⁷, R⁸, R⁹ und m die in Anspruch 1 angegebenen Bedeutungen haben und L₁ B(OH)₂ oder B(OR)₂ bedeutet, wobei B(OR)₂ ein cyclischer oder linearer Boronsäurealkylester ist,
mit einer Verbindung der Formel (V) 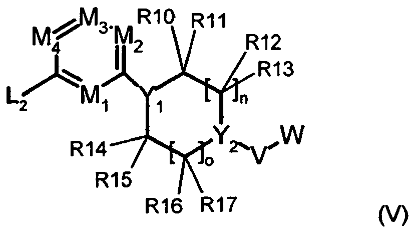 worin R¹⁰, R¹¹, R¹², R¹³, R¹⁴, R¹⁵, R¹⁶, R¹⁷, M₁, M₂, M₃, M₄, Y₁, Y₂, V, W, n, und o die in Anspruch 1 angegebenen Bedeutungen haben und L₂ Cl, Br oder I bedeutet,
umsetzt,
oder
(c) sie aus einem ihrer funktionellen Derivate (z.B. mit Schutzgruppen) durch Behandeln mit einem sauren, basischen, solvolysierenden oder hydrogenolysierenden Mittel in Freiheit setzt,
und/oder eine Base oder Säure der Formel (I) in eines ihrer Salze umwandelt.

5. Arzneimittel umfassend eine oder mehrere Verbindungen gemäß einem der Ansprüche 1 bis 3, wobei Verbindungen der Formel (I) gemäß Anspruch 1, worin (a) V fehlend ist, und (b) W = C(O)-CH₂-Het ist, nicht ausgeschlossen sind, sowie ihrer physiologisch unbedenklichen Salze,
Solvate, Tautomere und Stereoisomere, einschließlich deren Mischungen in allen Verhältnissen.

6. Arzneimittel umfassend eine oder mehrere Verbindungen gemäß einem der Ansprüche 1 bis 3, wobei Verbindungen der Formel (I) gemäß Anspruch 1, worin (a) V fehlend ist, und (b) W = C(O)-CH₂-Het ist, nicht ausgeschlossen sind, sowie ihrer physiologisch unbedenklichen Salze,
Solvate, Tautomere und Stereoisomere, einschließlich deren Mischungen in allen Verhältnissen, zur Verwendung in der Behandlung von Krankheiten, die durch Inhibierung von Sph-Kinase 1 durch die Verbindungen gemäß einem der Ansprüche 1 bis 3 beeinflußt werden.

7. Arzneimittel gemäß Anspruch 6, wobei die zu behandelnden Krankheiten ausgewählt sind aus der Gruppe bestehend aus: "hyperproliferative Erkrankung, inflammatorische Erkrankung, angiogene Erkrankung, fibrotische Erkrankung der Lunge, Niere, Leber und des Herzens, Krebs (Tumorerkrankung), Atherosclerose, Restenose, proliferative Erkrankung der Mesangiumzellen, Psoriasis, Tumor des Plattenepithel, der Blase, des Magens, der Nieren, von Kopf und Hals, des Ösophagus, des Gebärmutterhalses, der Schilddrüse, des Darms, der Leber, des Gehirns, der Prostata, des Urogenitaltrakts, des lymphatischen Systems, des Magens, des Kehlkopfs, der Lunge, der Haut, Monozytenleukämie, Lungenadenokarzinom, kleinzelliges Lungenkarzinom, Bauchspeicheldrüsenkrebs, Glioblastom, Brustkarzinom, akute myelotische Leukämie, chronische myelotische Leukämie, akute lymphatische Leukämie, chronische lymphatische Leukämie, Hodgkin-Lymphom, non-Hodgkin-Lymphom, Glomerutonephritis, diabetische Nephropathie, maligne Nephrosclerose, thrombotisches Mikroangiopathie-Syndrom, Transplantatabstossung, Glomerulopathie, entzündliche Darmerkrankung, Arthritis, Asthma, Allergien, entzündliche Nierenerkrankungen, multiple Sklerose, chronisch obstruktive Lungenerkrankung, entzündliche Hauterkrankungen, Pardontalerkrankungen, durch T-Zellen - vermittelte Immunerkrankung, ulcerative Colitis, Morbus Crohn, unbestimmte Colitis, allergische Encephalomyelitis, allergische Neuritis, Transplantatabstossung, Graft-versus-Host-Reaktion, Myocarditis, Thyroiditis, Nephritis, systemischer Lupus erythematodes, insulinabhängiger Diabetes mellitus, rheumatoide Arthritis, Osteoarthritis, Caplan Syndrom, Felty Syndrom, Sjogren Syndrom, Spondylitis ankylosans, Morbus Still, Chondrocalcinosis, metabolische Arthritis, rheumatisches Fieber, Morbus Reiter, Wissler Syndrom, Glomerulonephritis, glomeruläre Verletzung, nephrotisches Syndrom, interstitielle Nephritis, Lupus nephritis, Goodpasture-Syndrom, Wegener-Granulomatose, Nierenvaskulitis, IgA-Nephropathie, idiopatische glomeruläre Erkrankung, atopische Dermatitis, Kontaktempfindlichkeit, Akne, diabetische Retinopathie, Kaposi Sarkom, Hemangioma, myocardiale Angiogenesis, atherosklerotische Plaque-Neovaskularisation, angiogene Augenerkrankungen, choroidale Neovaskularisation, retrolentale Fibroplasie, makulare Degeneration, corneale Transplantatabstossung, Rubeosis iridis, neurosculares Glaukom, Oster Webber Syndrom".

8. Arzneimittel gemäß einem der Ansprüche 5 bis 7, worin ein derartiges Arzneimittel mindestens eine zusätzliche pharmakologisch aktive Substanz (Therapeutikum, Medikament, Inhaltsstoff) enthält.

9. Arzneimittel gemäß einem der Ansprüche 5 bis 7, worin das Arzneimittel vor und/oder während und/oder nach Behandlung mit mindestens einer zusätzlichen pharmakologisch aktiven Substanz angewendet wird.

10. Pharmazeutische Zusammensetzung enthaltend eine therapeutisch wirksame Menge mindestens einer Verbindung gemäß einem der Ansprüche 1 bis 3.

11. Pharmazeutische Zusammensetzung gemäß Anspruch 10 enthaltend mindestens eine zusätzliche Verbindung ausgewählt aus der Gruppe bestehend aus physiologisch unbedenklichen Streckmitteln, Hilfsstoffen, Zusatzstoffen, Verdünnern, Trägerstoffen und/oder zusätzlicher pharmazeutisch aktiver Substanz außer den Verbindungen gemäß einem der Ansprüche 1 bis 3.

12. Kit enthaltend eine therapeutisch wirksame Menge mindestens einer Verbindung gemäß einem der Ansprüche 1 bis 3 und/oder mindestens eine pharmazeutische Zusammensetzung gemäß einem der Ansprüche 10 bis 11 und eine therapeutisch wirksame Menge mindestens einer weiteren pharmakologisch aktiven Substanz außer den Verbindungen gemäß einem der Ansprüche 1 bis 3.

13. Verbindungen ausgewählt aus der Gruppe bestehend aus:

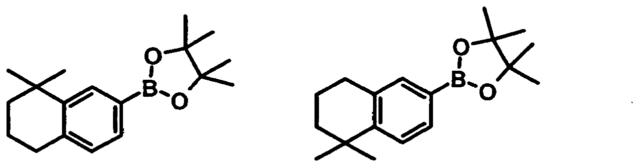

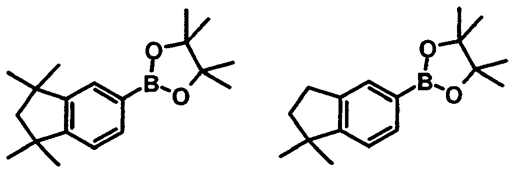

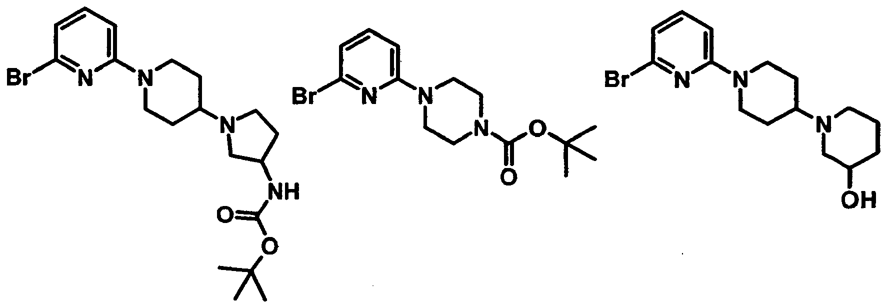

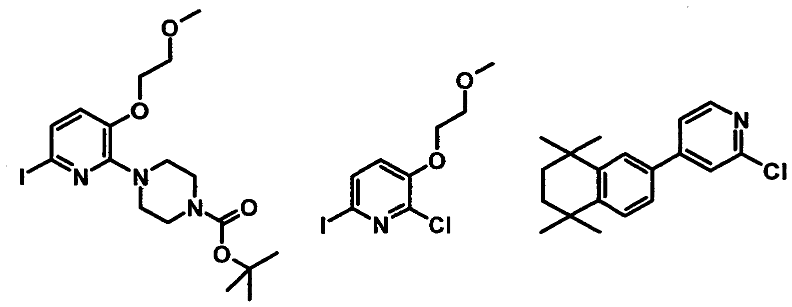

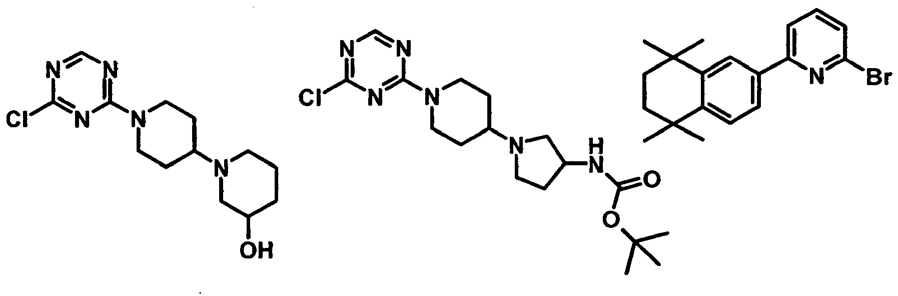

## Claims

1. Compounds of the formula (I) 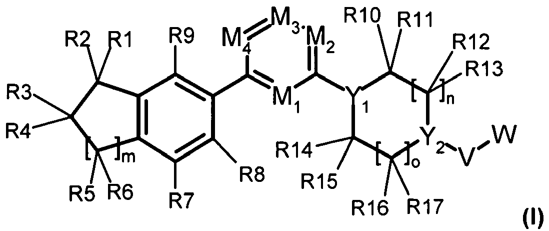 in which, in each case independently of one another:
R¹, R², R³, R⁴, R⁵, R⁶, denote H, D (deuterium), A, OR¹⁸, CN, F, Cl and NR¹⁸R^{18'};
R⁷, R⁸, R⁹, R¹⁰, R¹¹, R¹², R¹³, R¹⁴, R¹⁵, R¹⁶, R¹⁷ where R¹ and R², R³ and R⁴ R⁵ and R⁶, R¹⁰ and R¹¹, R¹² and R¹³, R¹⁴ and R¹⁵, R¹⁶ and R¹⁷ together may in each case also form =O (carbonyl oxygen); where R⁹ and R¹, R¹ and R², R² and R³, R³ and R⁴, R⁴ and R⁵, R⁵ and R⁶, R⁶ and R⁷, R⁷ and R⁸, R¹⁰ and R¹¹, R¹¹ and R¹², R¹² and R¹³, R¹⁴ and R¹⁵, R¹⁵ and R¹⁶, R¹⁶ and R¹⁷ together may in each case also form cyclic alkyl having 3, 4, 5, 6 or 7 C atoms or Het having 3, 4, 5, 6 or 7 ring atoms; where R¹⁰ and R¹⁹, if Y₁ = CR¹⁹, R¹¹ and R¹², R¹³ and R¹⁹, if Y₂ = CR¹⁹, R¹⁴ and R¹⁹, if Y₁ = CR¹⁹, R¹⁵ and R¹⁶, R¹⁷ and R¹⁹, if Y₂ = CR¹⁹, together may in each case also form a C=C double bond with the single bond and the C atoms to which they are attached;
R¹⁸, R^{18'} denote H, D or A;
R¹⁹, R^{19'} denote H, D, A, OR¹⁸, NR¹⁸R^{18'}, F, Cl, Br, CN or Het;
M₁, M₂, M₃, M₄ denote CR¹⁹ or N;
Y₁, Y₂ denote CR¹⁹ or N;
V denotes C(R¹⁹)(R^{19'}), NR¹⁹ or is absent;
W denotes [C(R¹⁹)(R^{19'})]ₚZ, CO-[C(R¹⁹)(R^{19'})]ₚZ, [C(R¹⁹)(R^{19'})]ₚN(R¹⁹)-Z, CO-N(R¹⁹)-[C(R¹⁹)(R^{19'})]ₚZ, N(R¹⁹)-CO-[C(R¹⁹)(R^{19'})]ₚZ, CO-O-[C(R¹⁹)(R^{19'})]ₚZ, C(O)OR¹⁸, OR¹⁸, H or D;
where V, W and Y₂ together may in each case also form cyclic alkyl having 3, 4, 5, 6 or 7 C atoms, in which 1, 2, 3, 4, 5, 6 or 7 H atoms may preferably be replaced by F, Cl, Br, CN and/or OH, OR¹⁹, OC(O)R¹⁹, NR¹⁹C(O)OZ, C(O)OR¹⁹, C(O)N(R¹⁹)(R^{19'}) or N(R¹⁹)(R^{19'}); or Het having 3, 4, 5, 6 or 7 ring atoms, where Het preferably represents a saturated, unsaturated or aromatic heterocycle having 1 to 4 N, O and/or S atoms, which may be unsubstituted or mono-, di- or trisubstituted by Hal, F, Cl, Br, CN, A, OR¹⁸, W, SR¹⁸, NO₂, N(R¹⁹)(R^{19'}), NR¹⁸COOZ, OCONHZ, NR¹⁸SO₂Z, SO₂N(R¹⁸)Z, S(O)ₘZ, COZ, CHO, COZ, =S, =NH, =NA, oxy (-O⁻) and/or =O (carbonyl oxygen);
Z denotes Het, Ar or A;
A denotes unbranched or branched alkyl having 1, 2, 3, 4, 5, 6, 7, 8, 9 or 10 C atoms, in which 1, 2, 3, 4, 5, 6 or 7 H atoms may be replaced by F, Cl, Br, CN and/or OH, OR¹⁹, OC(O)R¹⁹, NR¹⁹C(O)OZ, C(O)OR¹⁹, C(O)N(R¹⁹)(R^{19'}) or N(R¹⁹)(R^{19'})_{;} and/or in which one or two CH₂ groups may be replaced by O, S, SO, SO₂, CO, COO, NR¹⁸, NR¹⁸CO, CONR¹⁸, cyclic alkyl having 3, 4, 5, 6 or 7 C atoms, CH=CH and/or CH≡CH groups; or cyclic alkyl having 3, 4, 5, 6 or 7 C atoms, in which 1, 2, 3, 4, 5, 6 or 7 H atoms may preferably be replaced by F, Cl, Br, CN and/or OH, OR¹⁹, OC(O)R¹⁹, NR¹⁹C(O)OZ, C(O)OR¹⁹, C(O)N(R¹⁹)(R^{19'}) or N(R¹⁹)(R^{19'});
Ar denotes phenyl, naphthyl or biphenyl, each of which is mono-, di- or trisubstituted by Hal, F, Cl, Br, CN, A, OR¹⁸, W, SR¹⁸, NO₂, N(R¹⁹)(R^{19'}), NR¹⁸COOZ, OCONHZ, NR¹⁸SO₂Z, SO₂N(R¹⁸)Z, S(O)ₘZ, COZ, CHO, COZ,
Het in each case, independently of one another, denotes a mono-, bi- or tricyclic saturated, unsaturated or aromatic heterocycle having 1 to 4 N, O and/or S atoms, which may be unsubstituted or mono-, di- or trisubstituted by Hal, F, Cl, Br, CN, A, OR¹⁸, W, SR¹⁸, NO₂, N(R¹⁹)(R^{19'}), NR¹⁸COOZ, OCONHZ, NR¹⁸SO₂Z, SO₂N(R¹⁸)Z, S(O)ₘZ, COZ, CHO, COZ, =S, =NH, =NA, oxy (-O-) and/or =O (carbonyl oxygen),
m denotes 1, 2 or 3,
n, o denote 0, 1 or 2,
p denotes 0, 1, 2, 3 or 4,
with the proviso that compounds of the formula (I) in which
(a) V is absent, and
(b) W = C(O)-CH₂-Het are excluded;
with the further proviso that the following compound is likewise excluded: 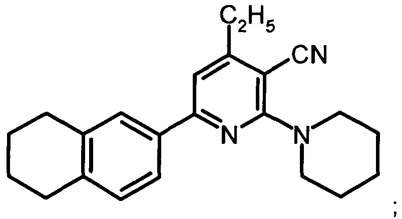 and physiologically acceptable salts, solvates, tautomers and stereoisomers thereof, including mixtures thereof in all ratios.

2. Compounds according to Claim 1, in which, in each case independently of one another:
R¹, R², R³, R⁴, R⁵, R⁶,
R⁷, R⁸, R⁹, R¹⁰, R¹¹, R¹², R¹³, R¹⁴, R¹⁵, R¹⁶, R¹⁷ denote H, D, A, OR¹⁸ or NR¹⁸R^{18'};
where R¹ and R², R³ and R⁴, R⁵ and R⁶, R¹⁰ and R¹¹, R¹² and R¹³, R¹⁴ and R¹⁵, R¹⁶ and R¹⁷ together may in each case also form =O (carbonyl oxygen);
where R⁹ and R¹, R¹ and R², R² and R³, R³ and R⁴, R⁴ and R⁵, R⁵ and R⁶, R⁶ and R⁷, R⁷ and R⁸, R¹⁰ and R¹¹, R¹¹ and R¹², R¹² and R¹³, R¹⁴ and R¹⁵, R¹⁵ and R¹⁶, R¹⁶ and R¹⁷ together may in each case also form cyclic alkyl having 3, 4, 5, 6 or 7 C atoms or Het having 3, 4, 5, 6 or 7 ring atoms;
R¹⁹, R^{19'} denote H, A, OR¹⁸, F;
M₁, M₂, M₃, M₄ denote CR¹⁹, or
M₁ denotes N, and M₂, M₃, M₄ denote CR¹⁹, or
M₂ denotes N, and M₁, M₃, M₄ denote CR¹⁹, or
M₄ denotes N, and M₁, M₂, M₃ denote CR¹⁹, or
M₁, M₂ denote N, and M₃, M₄ denote CR¹⁹, or
M₁, M₃ denote N, and M₂, M₄ denote CR¹⁹, or
M₁, M₄ denote N, and M₂, M₃ denote CR¹⁹, or
M₁, M₂, M₄ denote N, and M₃ denotes CR¹⁹;
Y₁, Y₂ denote N, or
Y₁ denotes N, and Y₂ denotes CR¹⁹, or
Y₁ denotes CR¹⁹ and Y₂ denotes N;
W denotes [C(R¹⁹)(R^{19'})]ₚZ, CO-[C(R¹⁹)(R^{19'})]ₚZ, CO-O-[C(R¹⁹)(R^{19'})]ₚZ, [C(R¹⁹)(R^{19'})]ₚN(R¹⁹)-Z, N(R¹⁹)-CO-[C(R¹⁹)(R^{19'})]ₚZ, C(O)OR¹⁸, OR¹⁸ or H;
Z denotes Het or A;
m denotes 1 or 2;
n, o denote 0, 1 or 2;
p denotes 0, 1 or 2;
and physiologically acceptable salts, solvates, tautomers and stereoisomers thereof, including mixtures thereof in all ratios.

3. Compounds according to Claim 1, selected from the group consisting of: 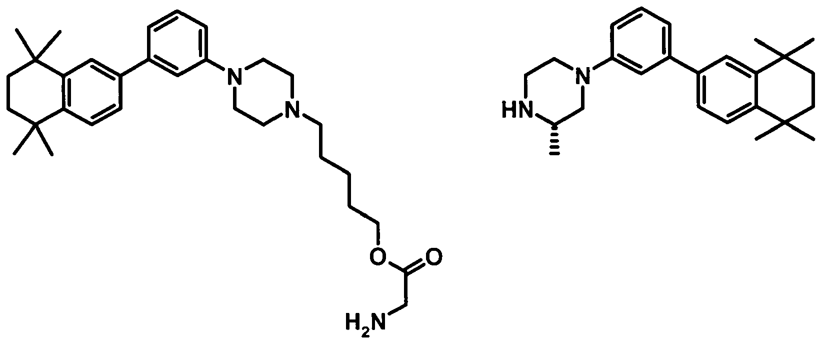 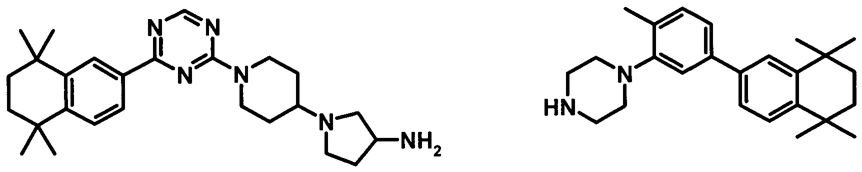 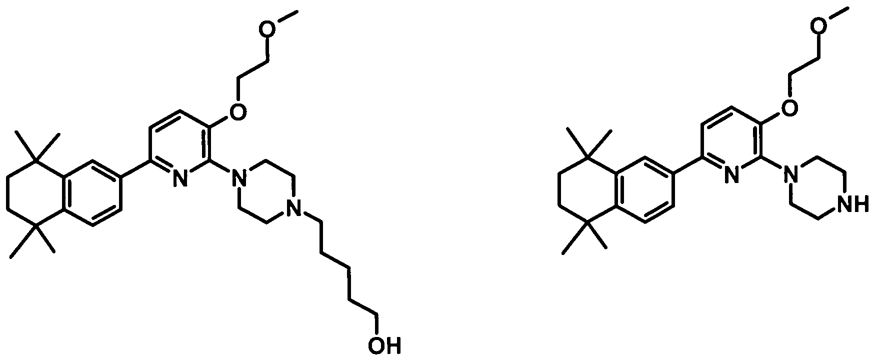 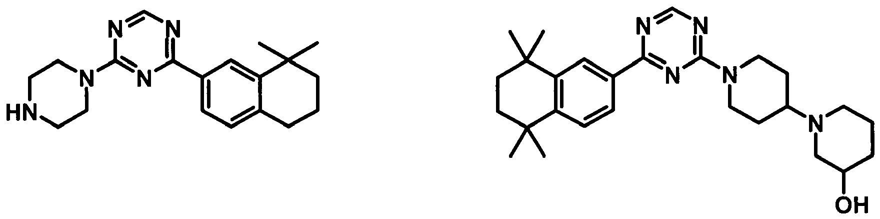 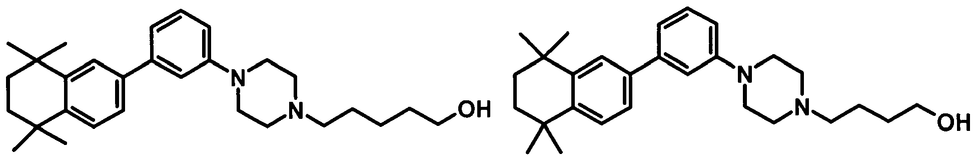 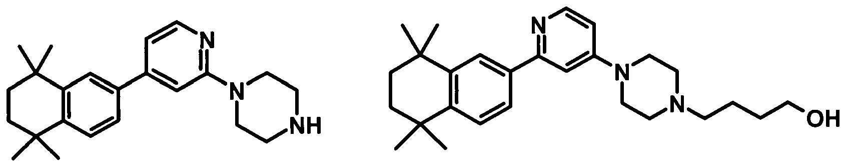 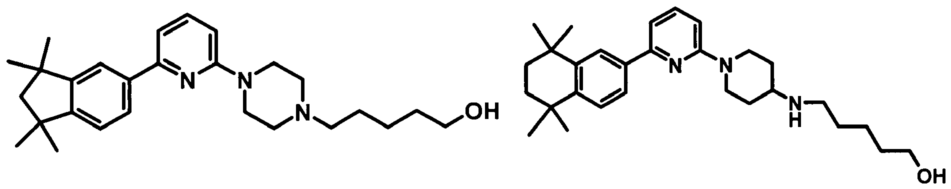 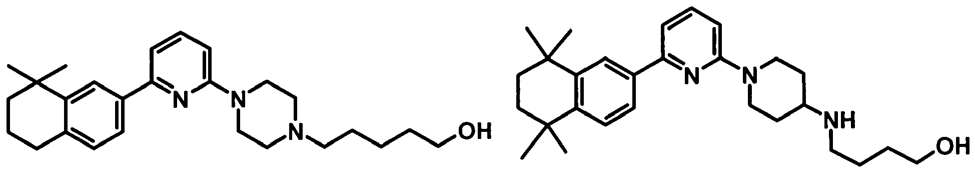 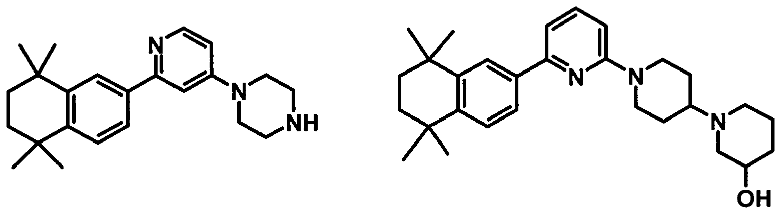 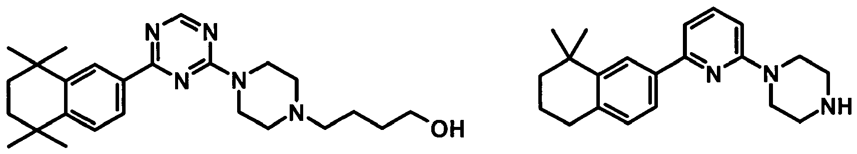 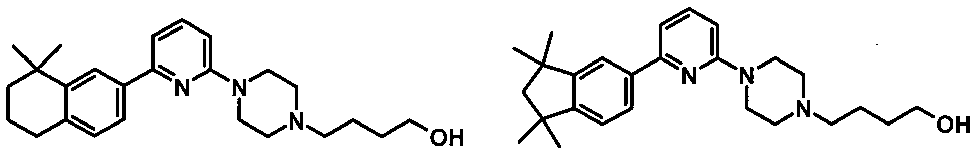 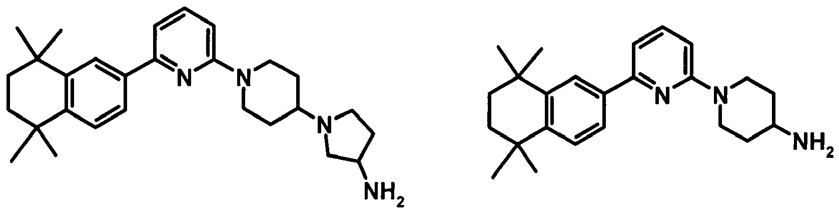 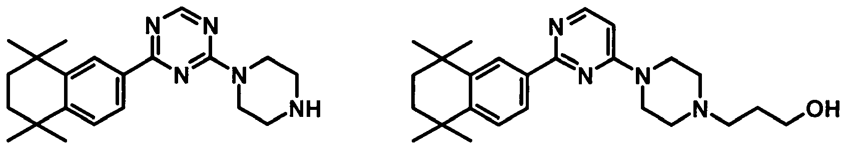 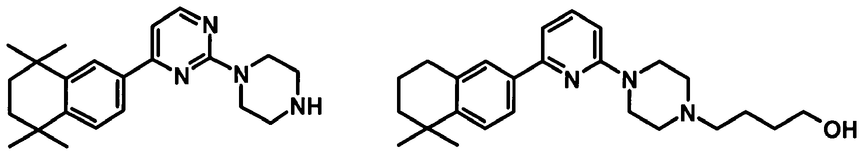 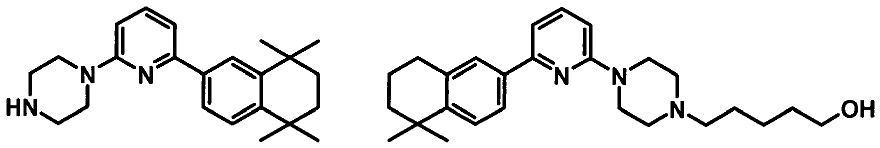 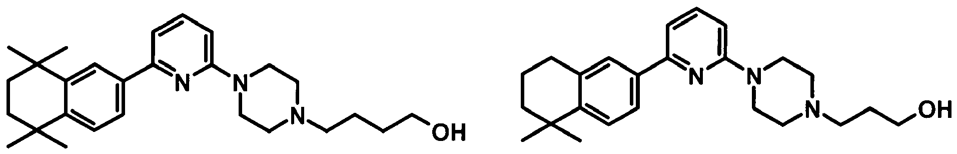 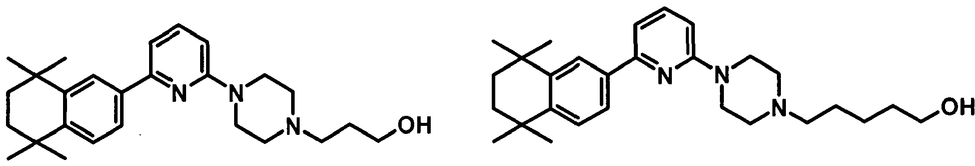 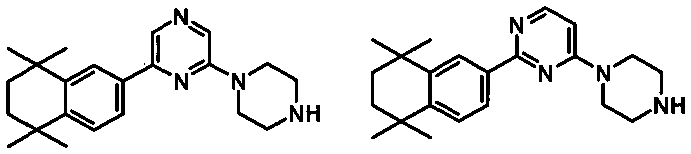 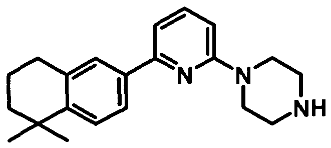 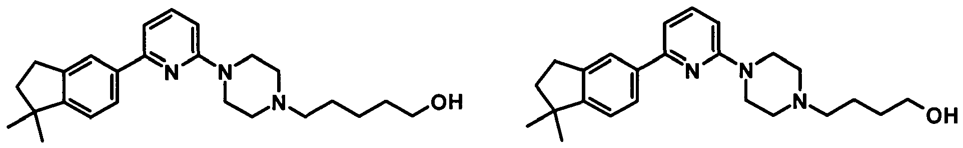 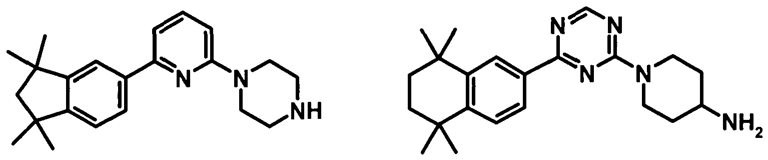 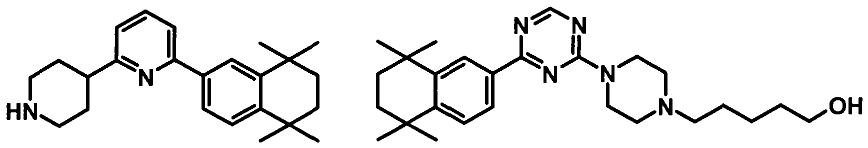 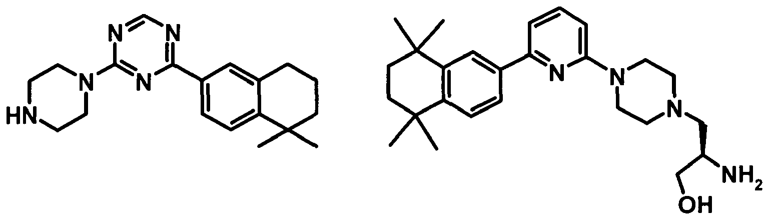 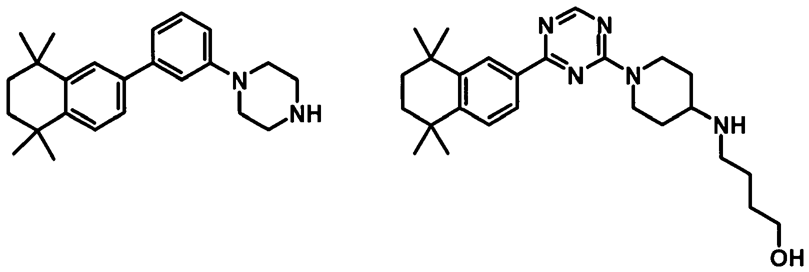 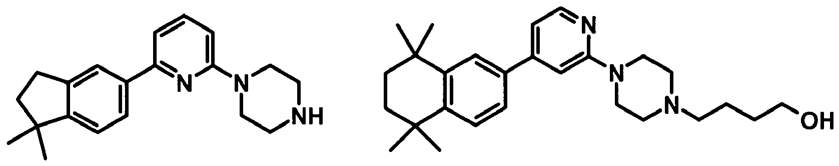 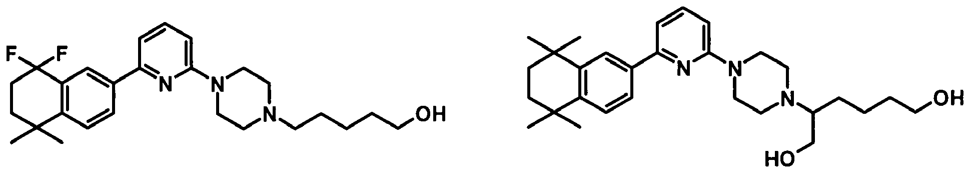 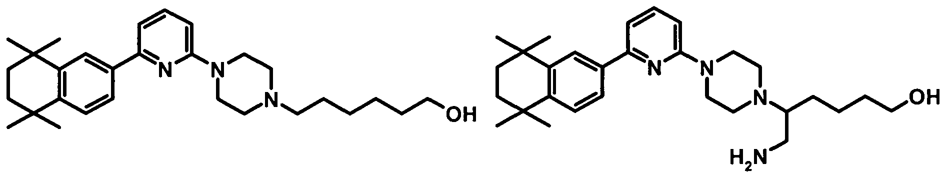 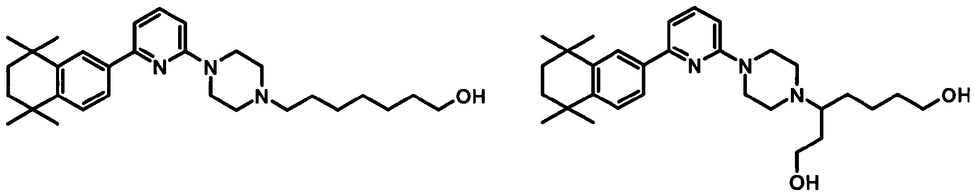 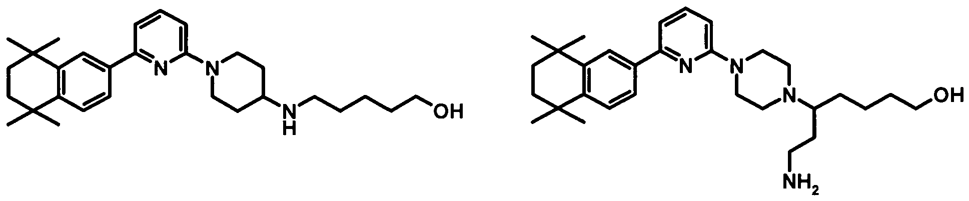 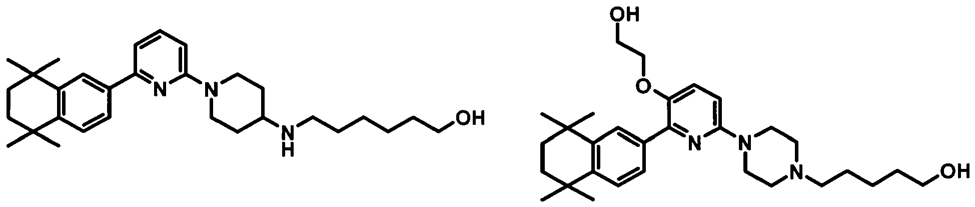 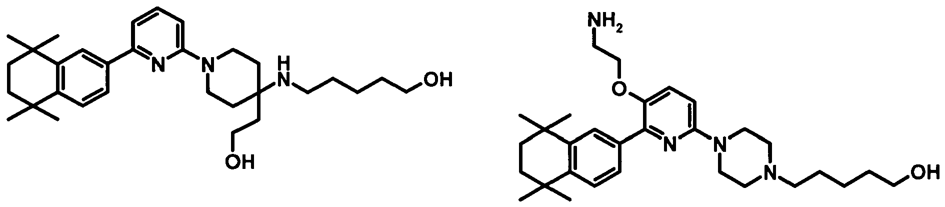 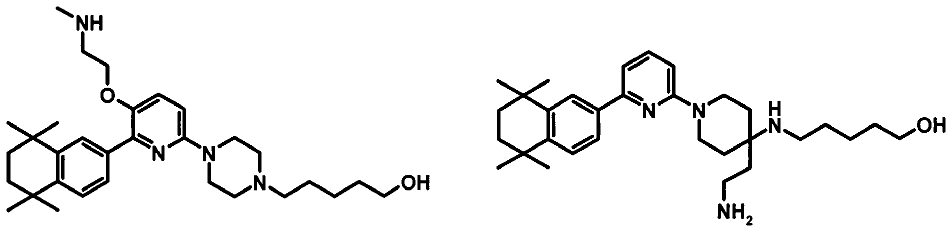 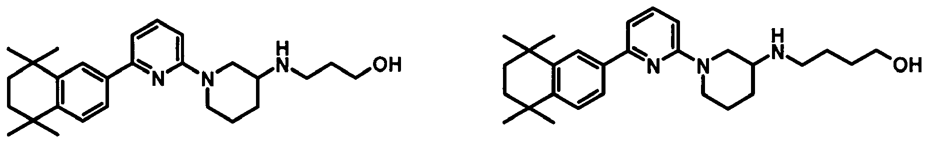 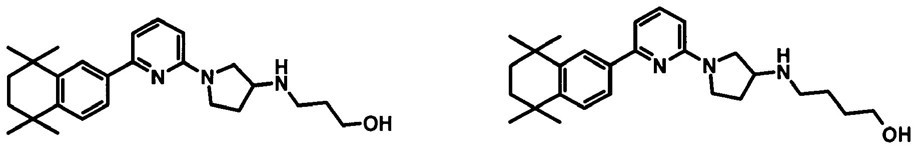 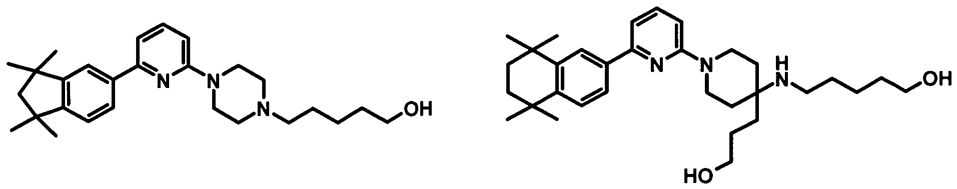 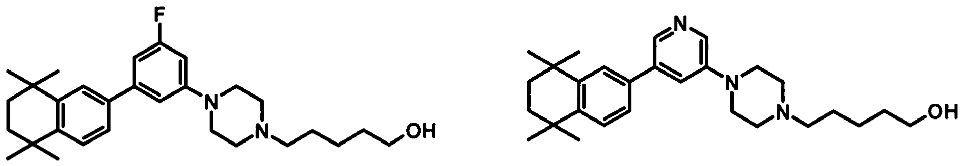 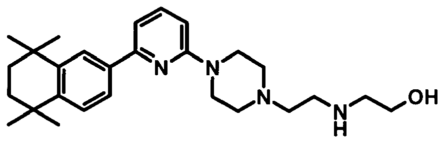 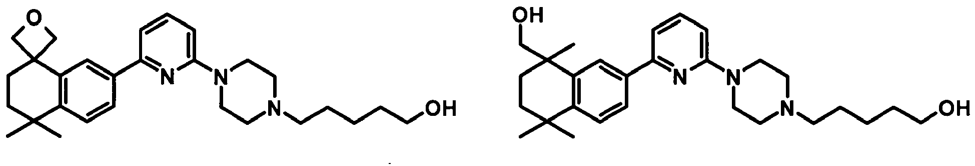 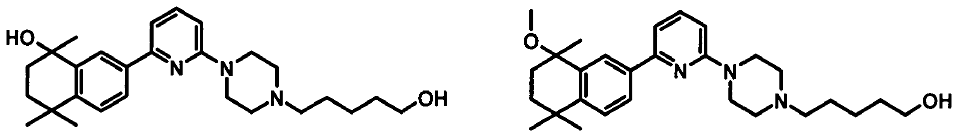 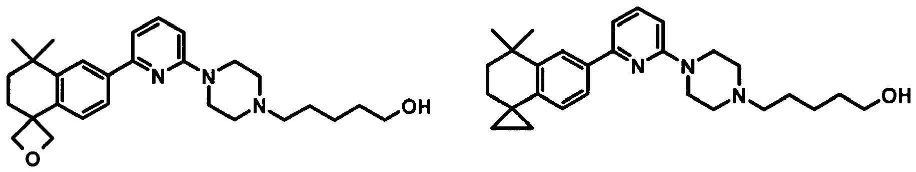 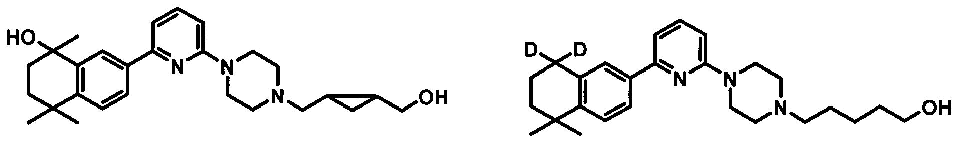 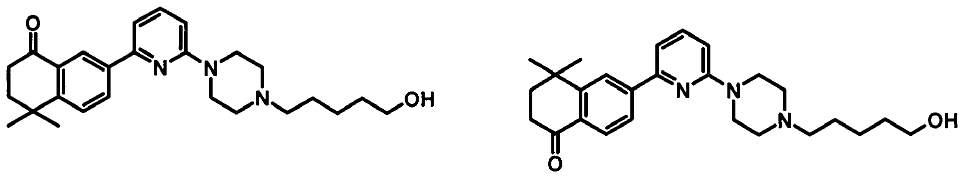 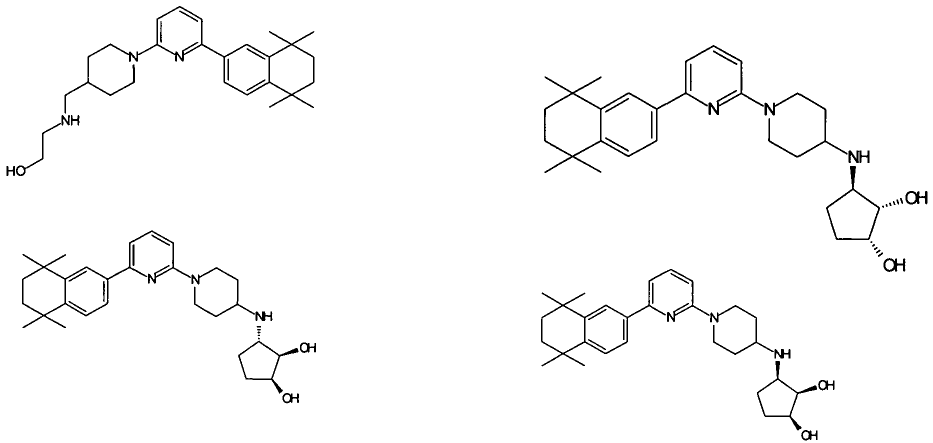 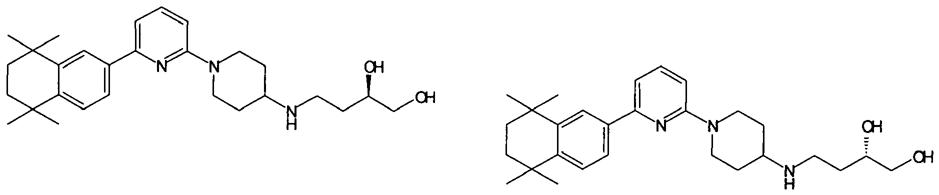 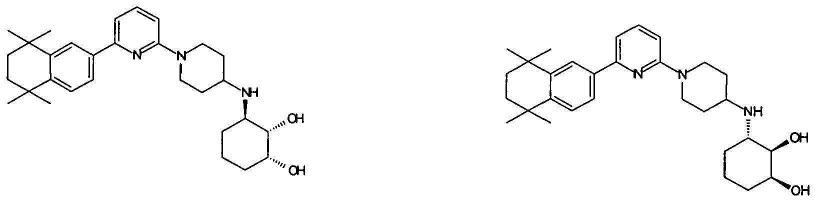 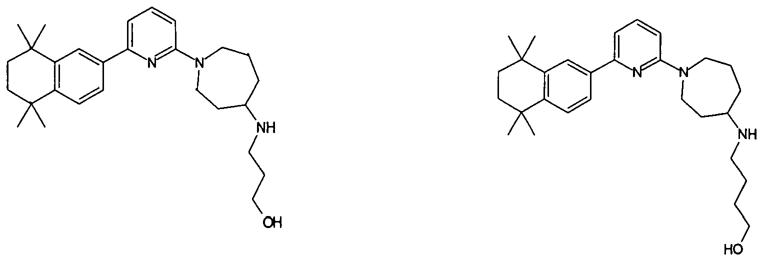 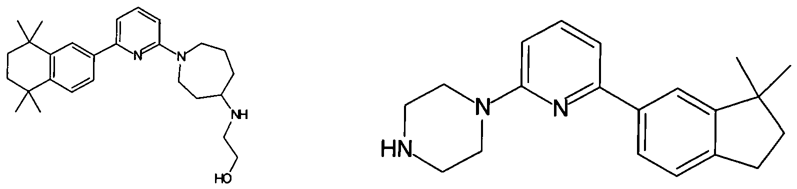 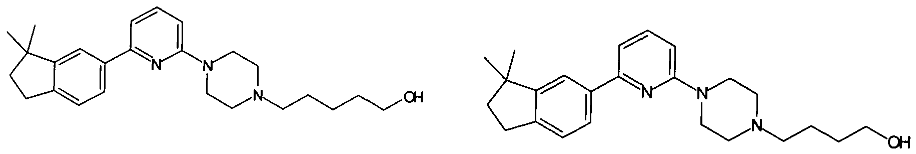 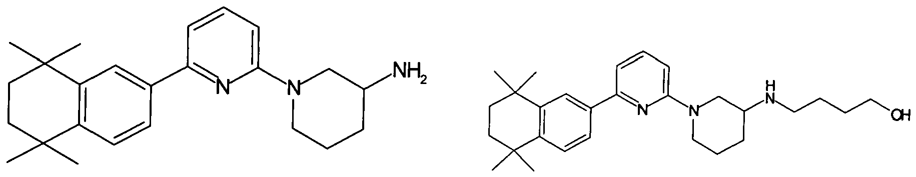 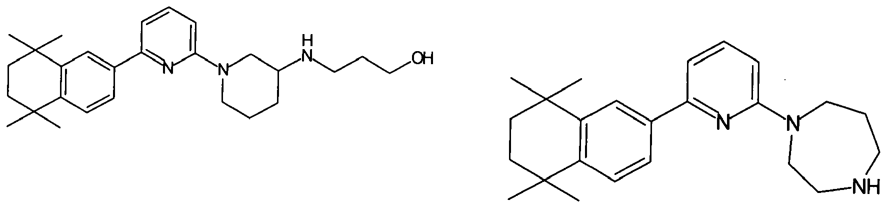 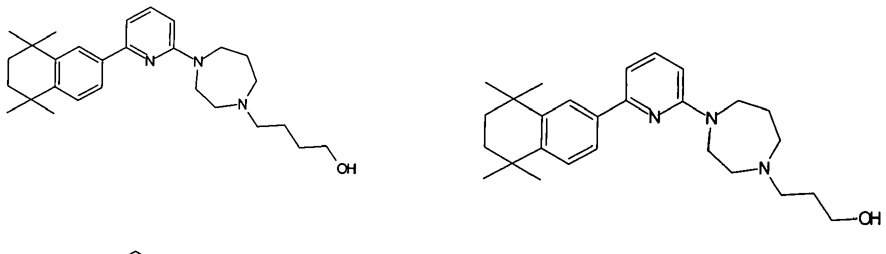 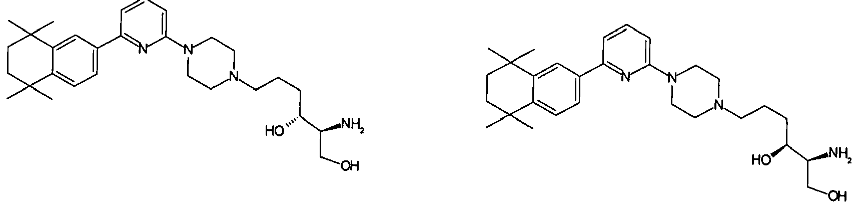 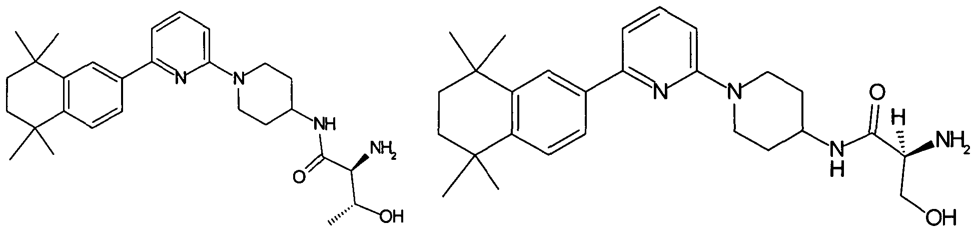 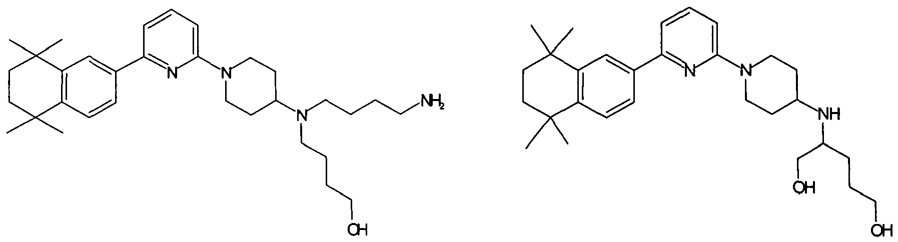 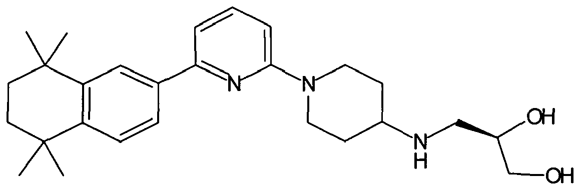 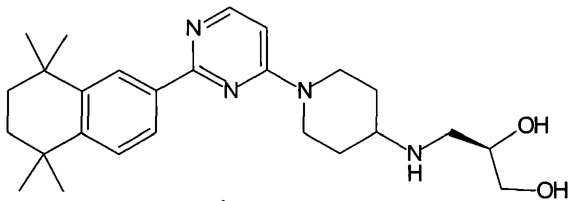 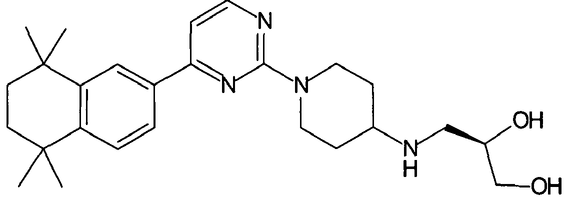 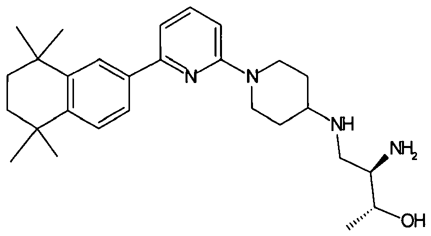 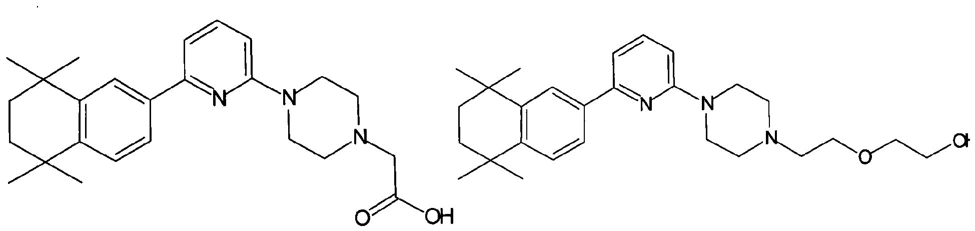 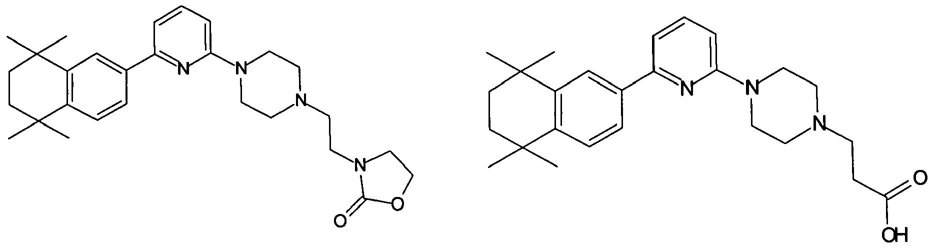 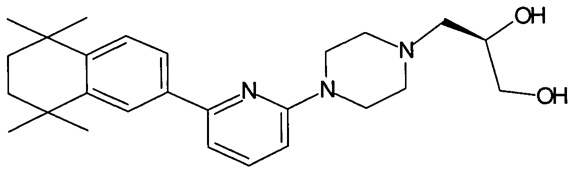 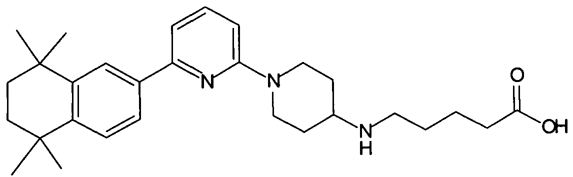 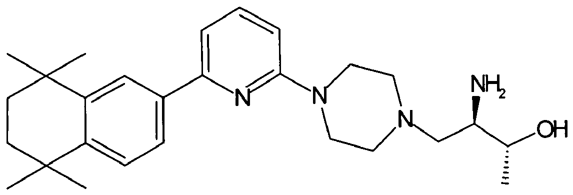 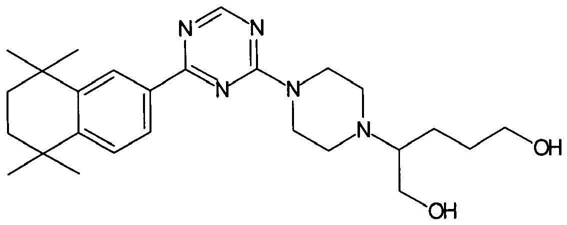 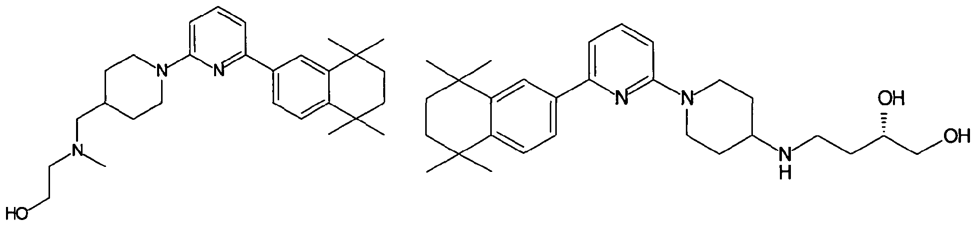 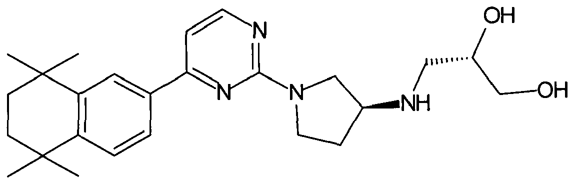 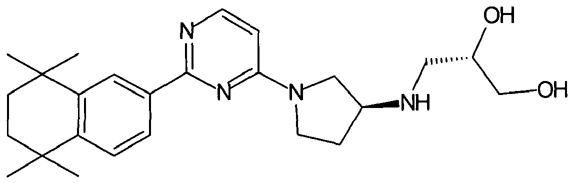 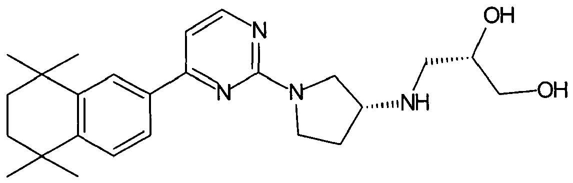 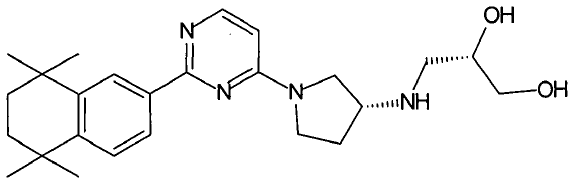 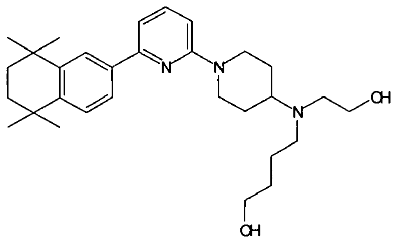 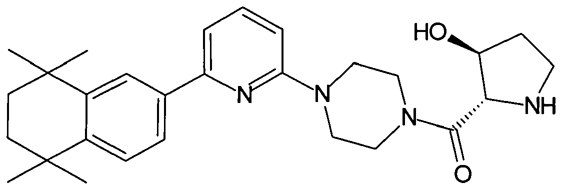 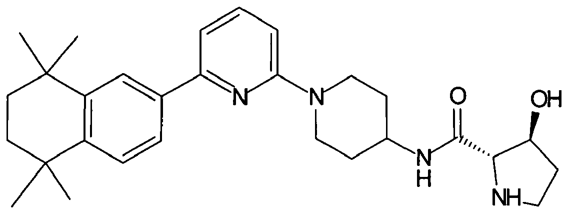 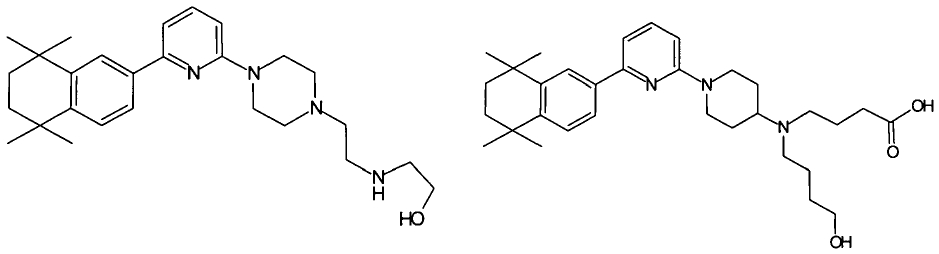 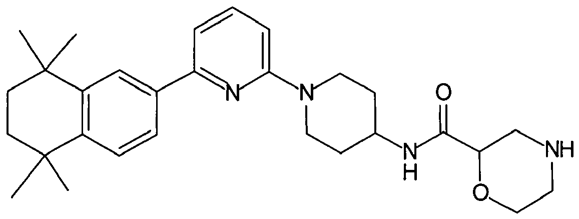 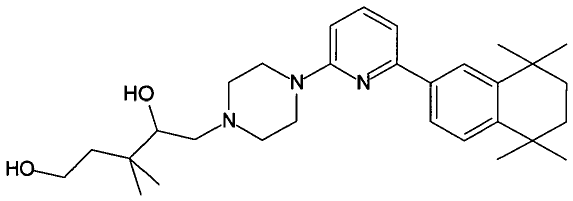 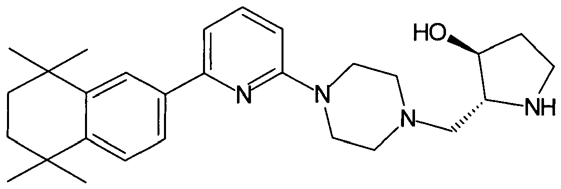 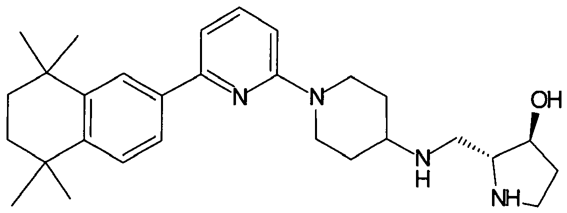 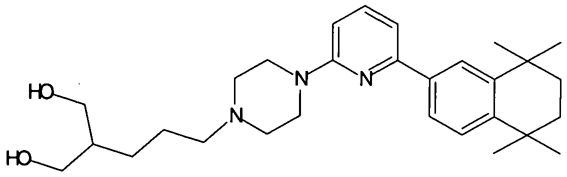 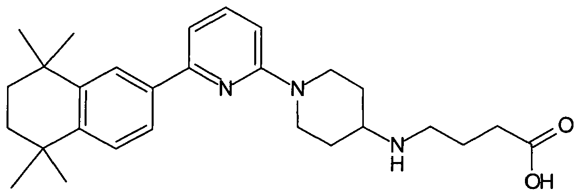 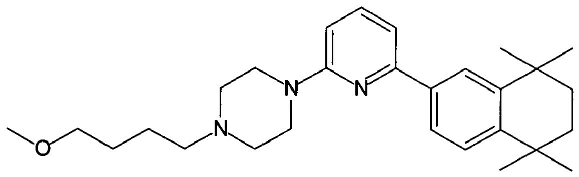 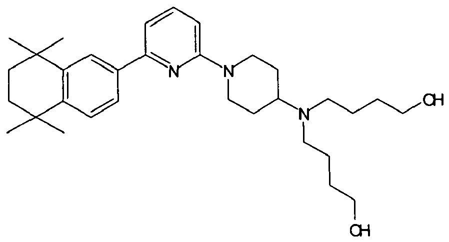 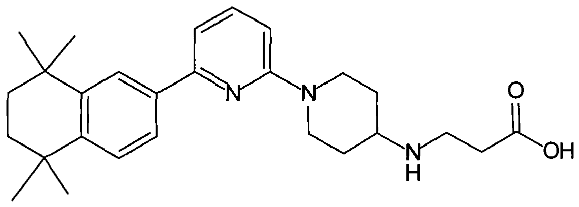 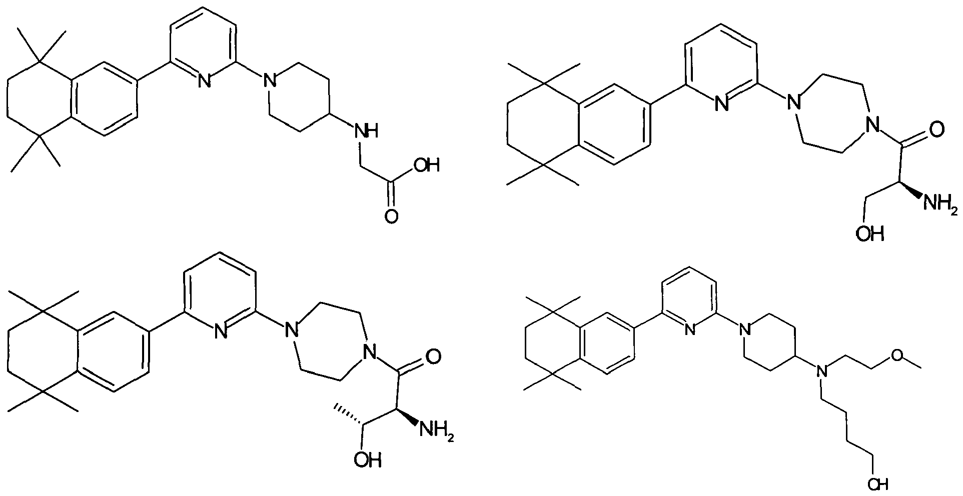 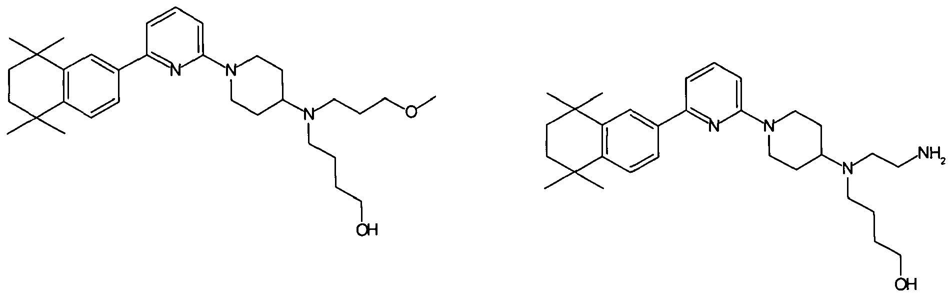 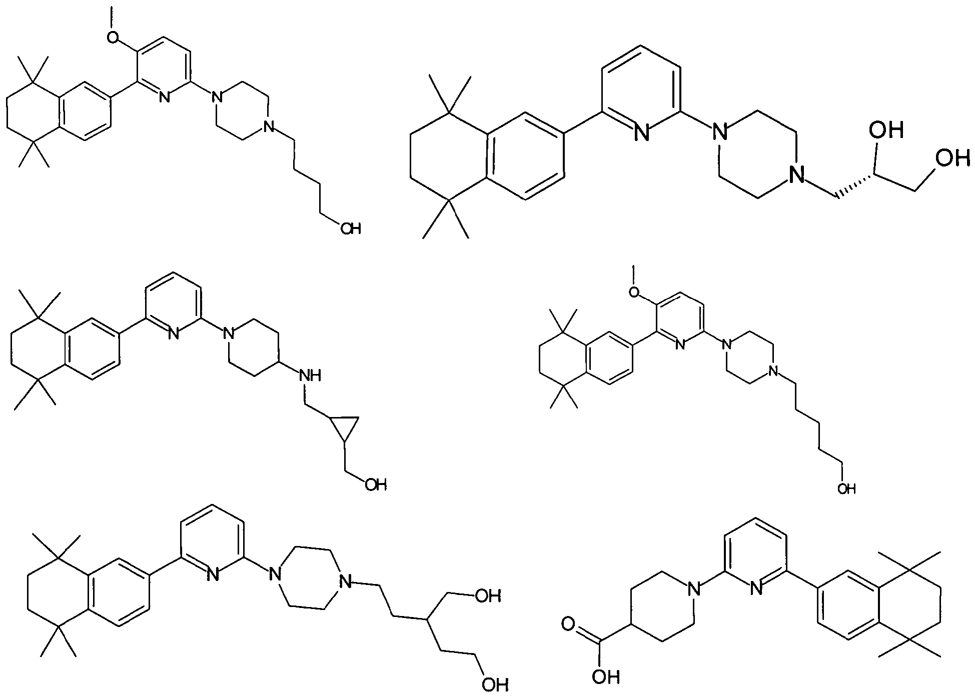 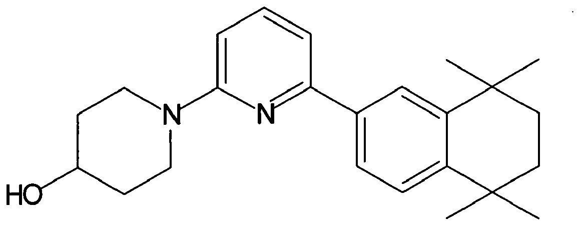 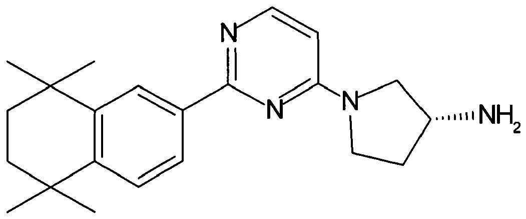 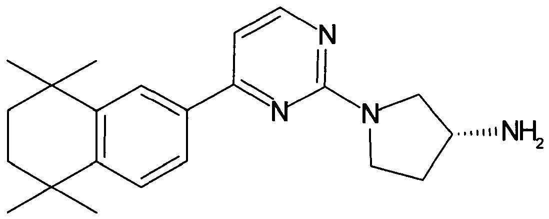 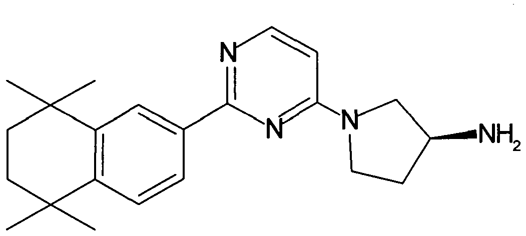 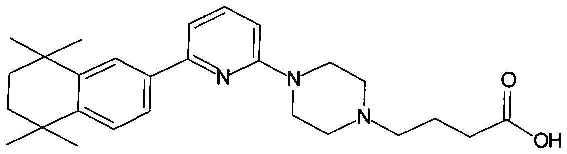 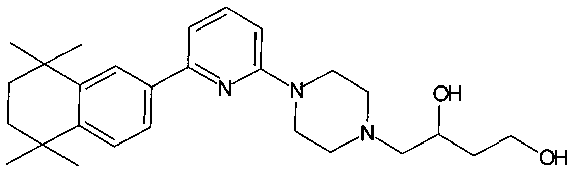 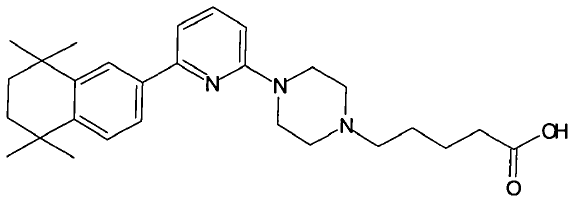 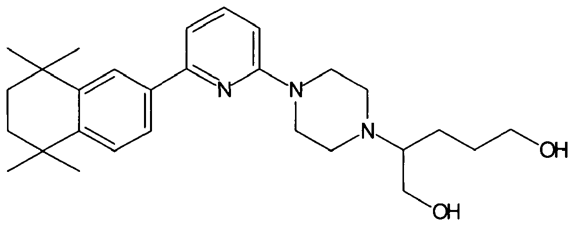 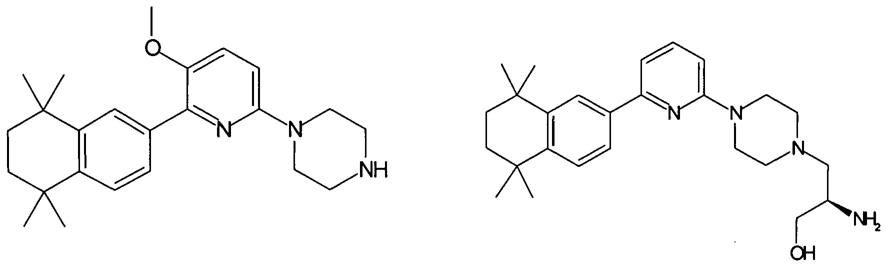 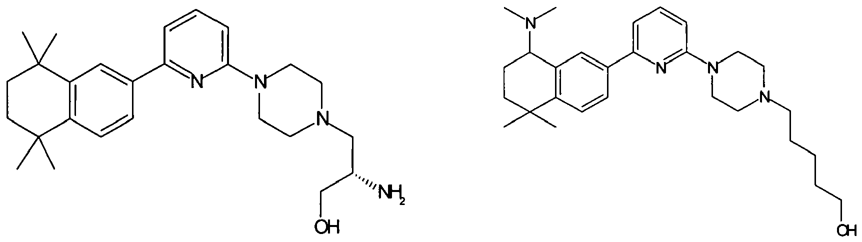 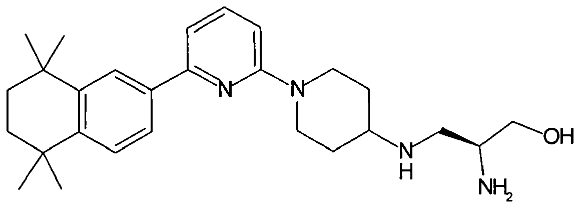 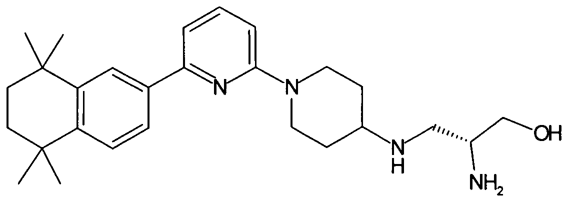 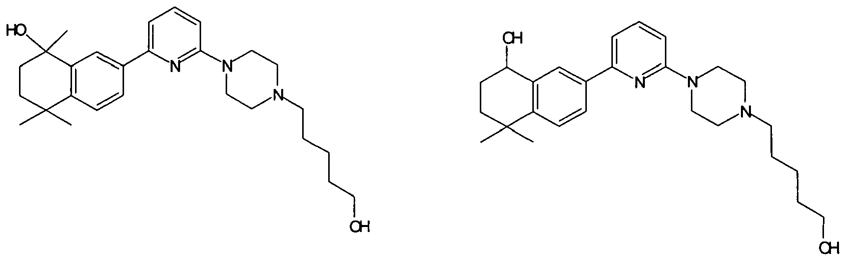 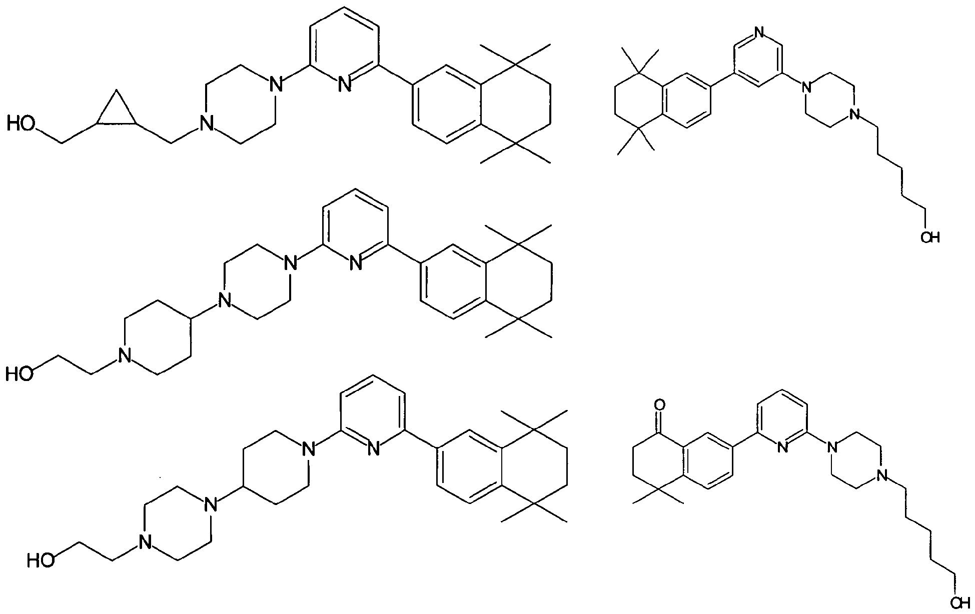 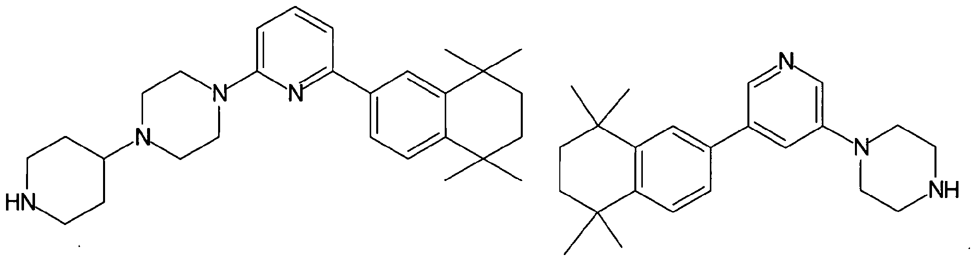 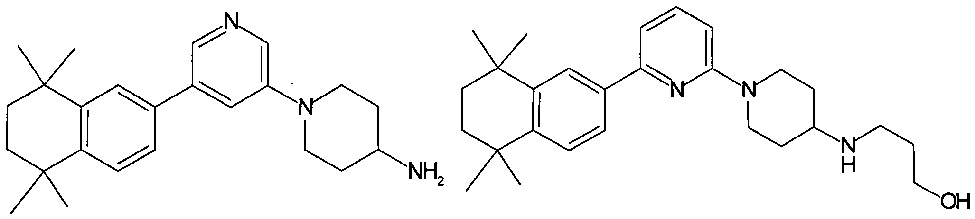 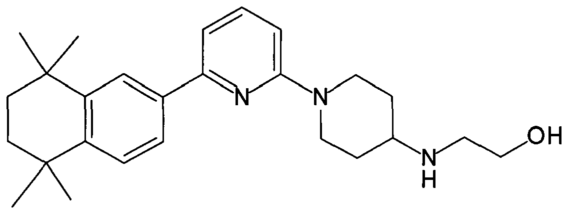 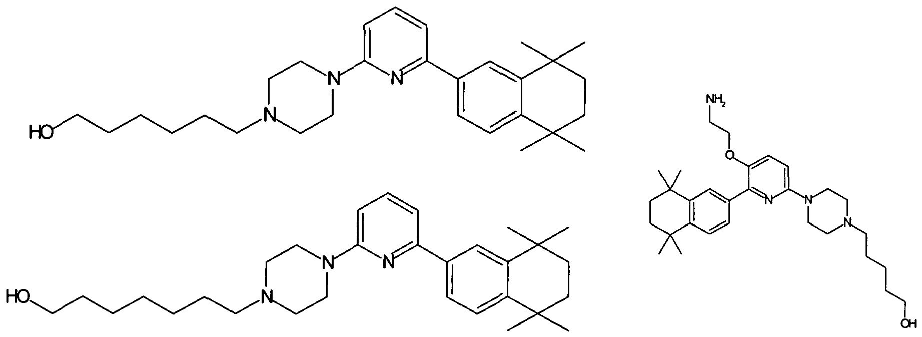 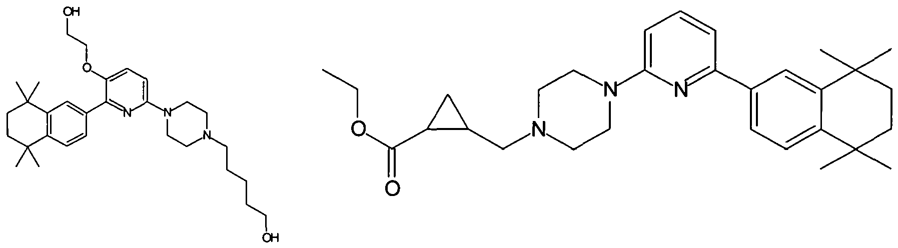 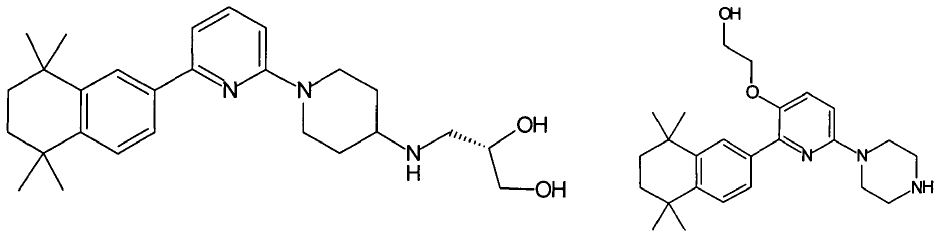 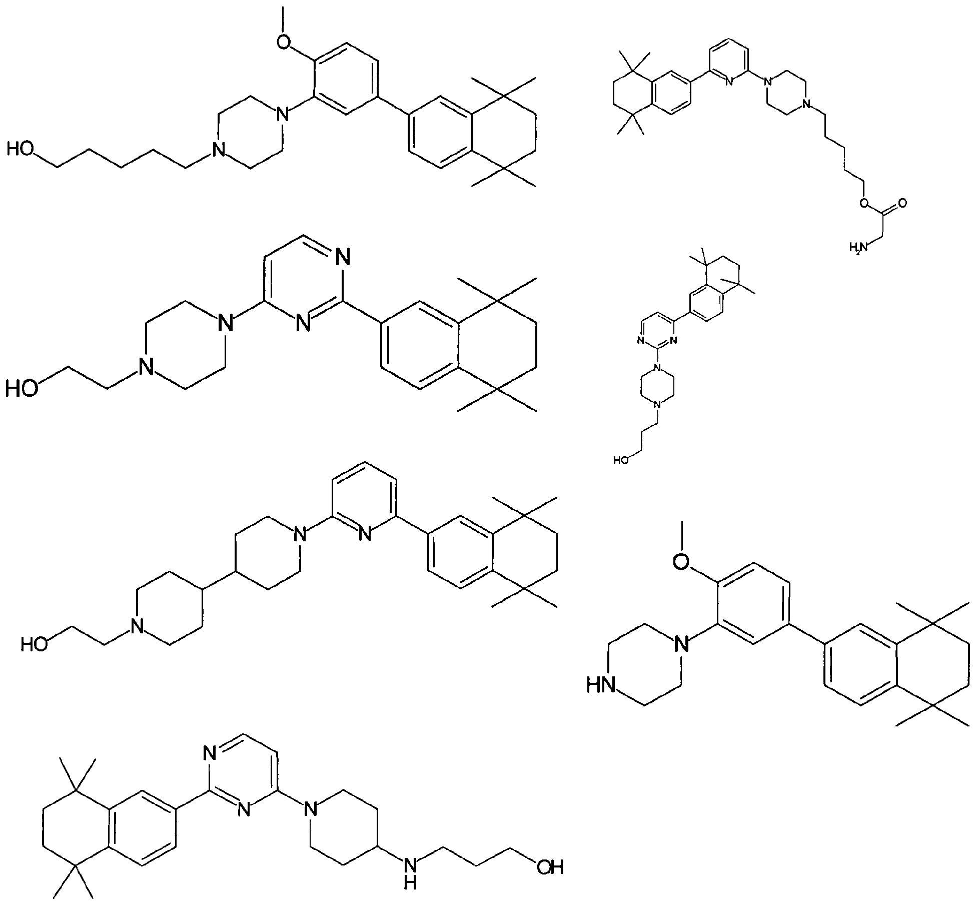 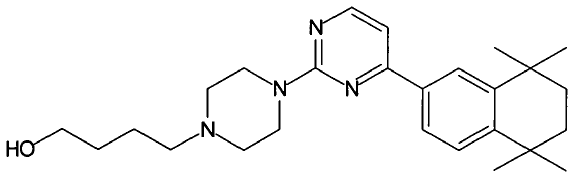 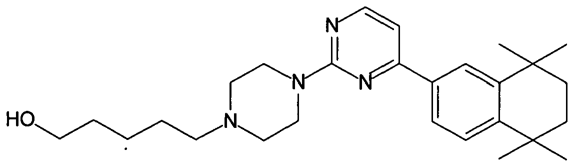 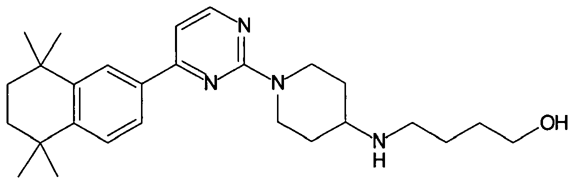 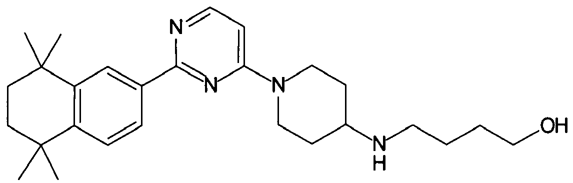 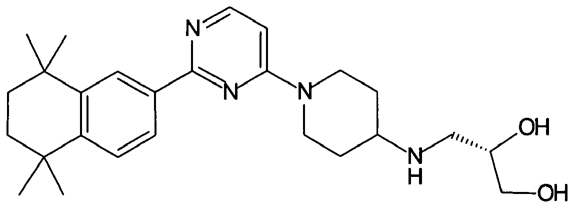 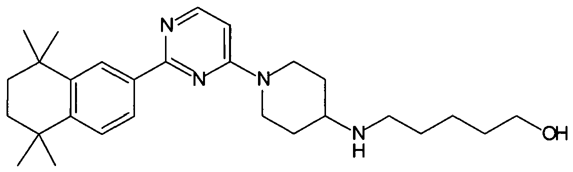 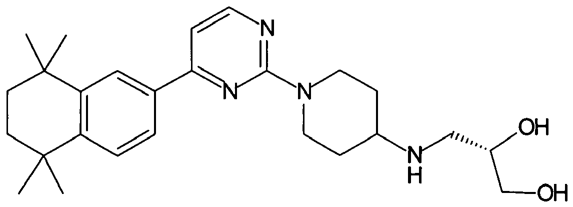 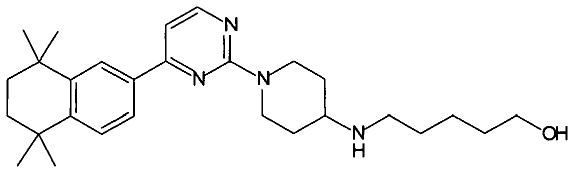 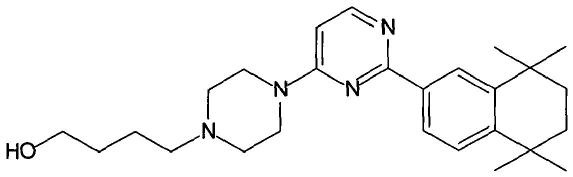 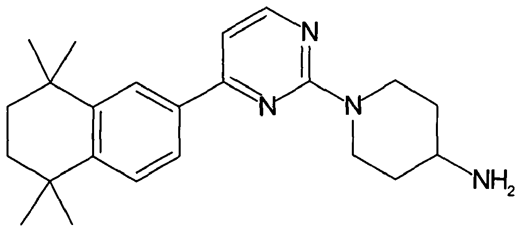 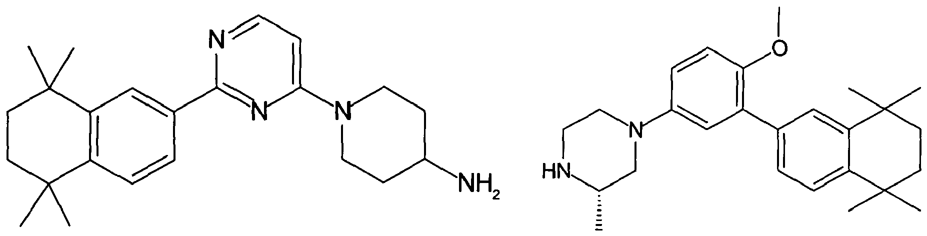 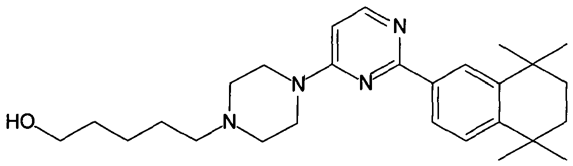 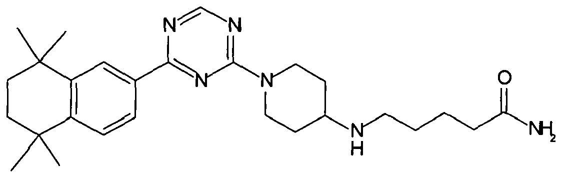 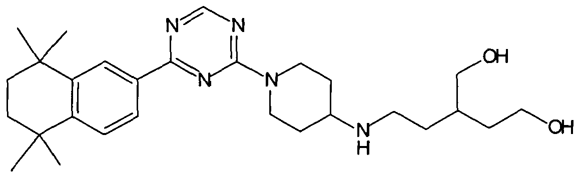 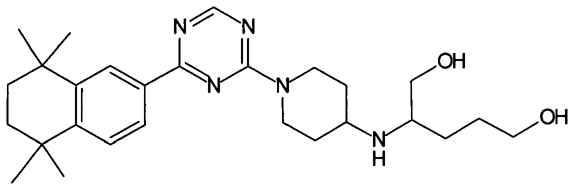 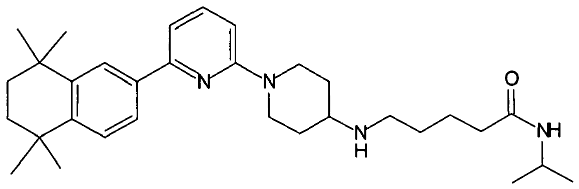 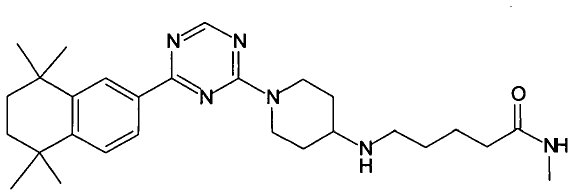 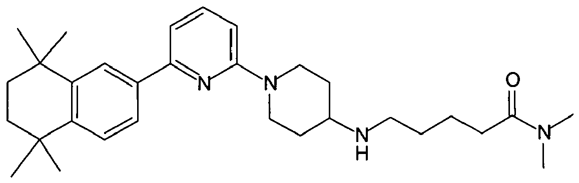 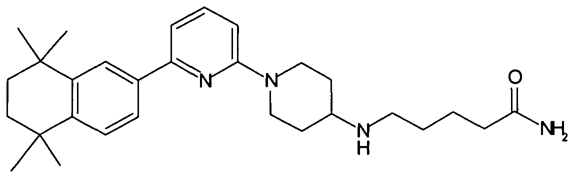 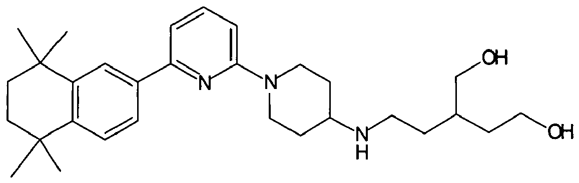 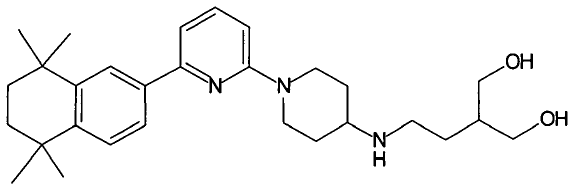 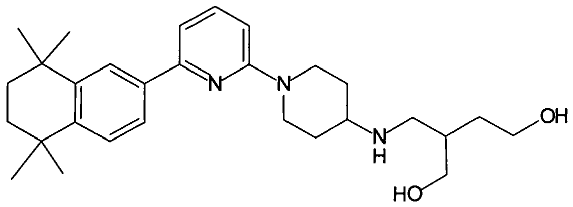 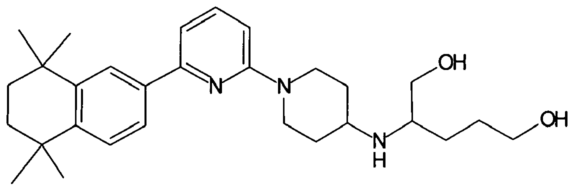 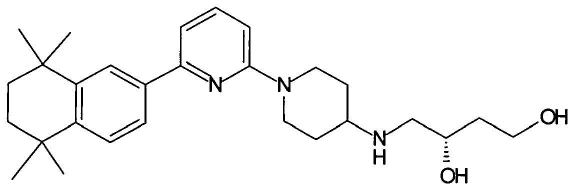 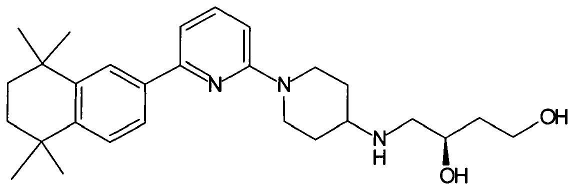 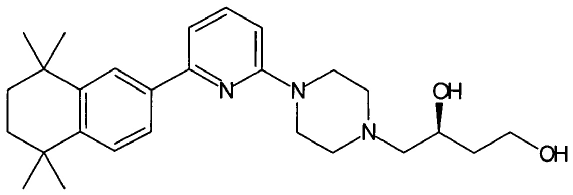 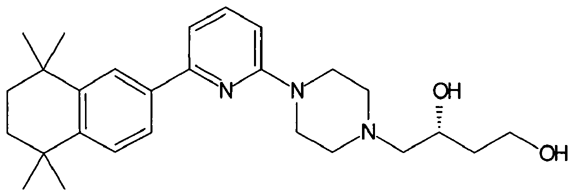 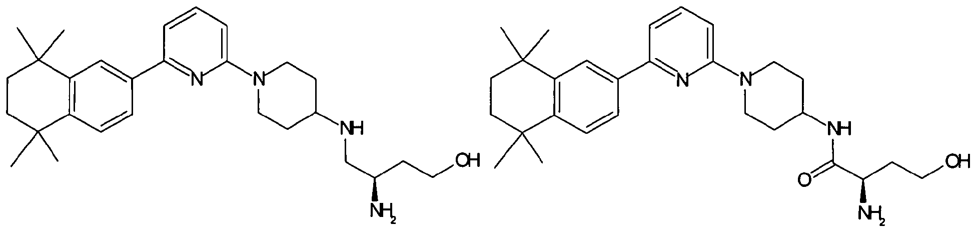 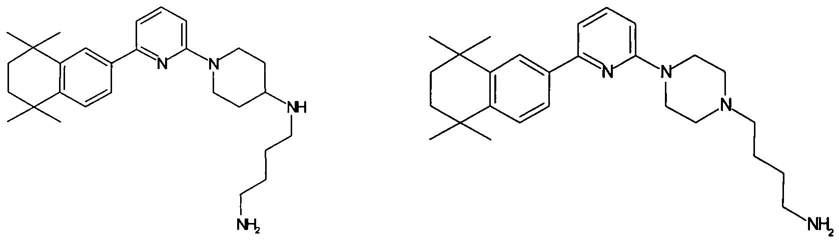 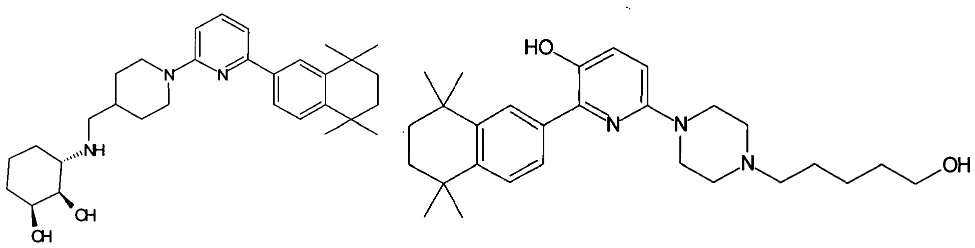 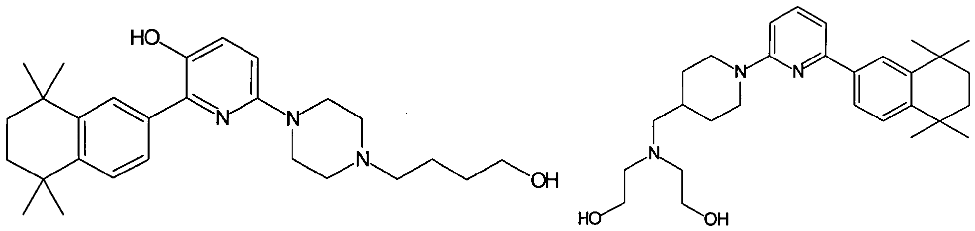 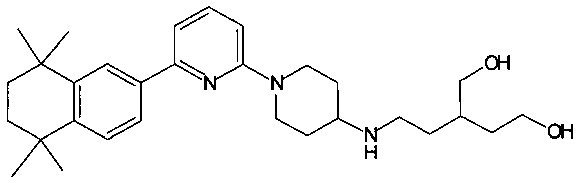 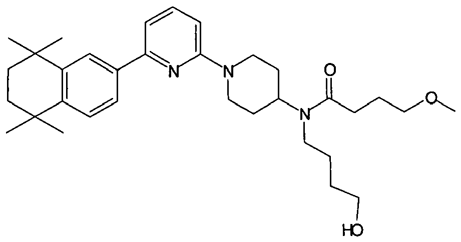 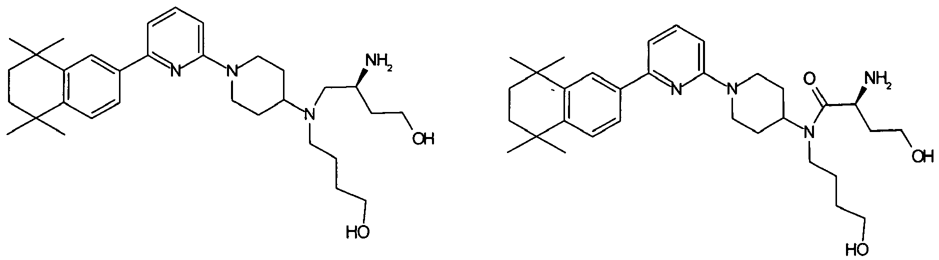 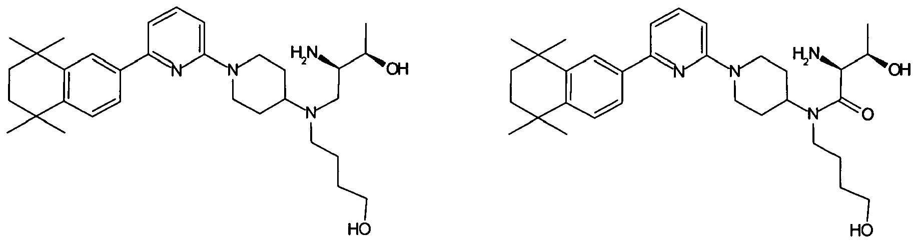 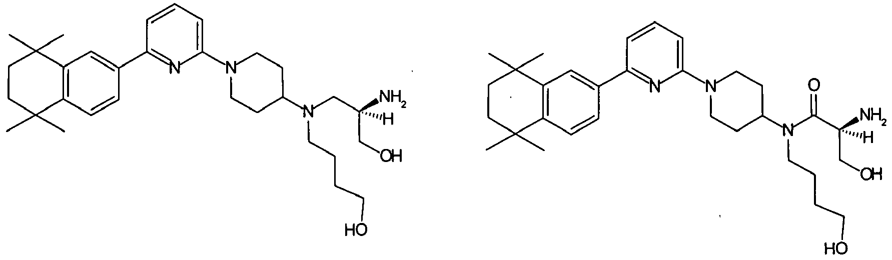 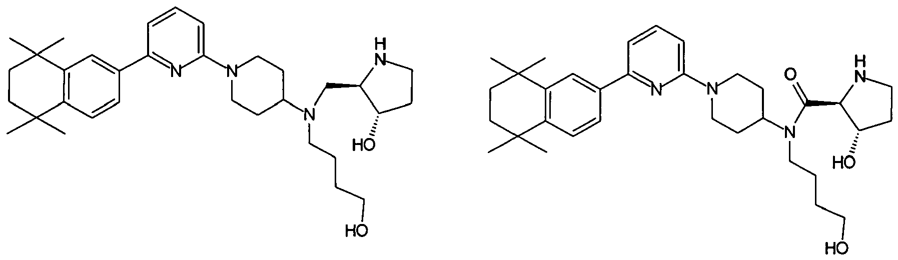 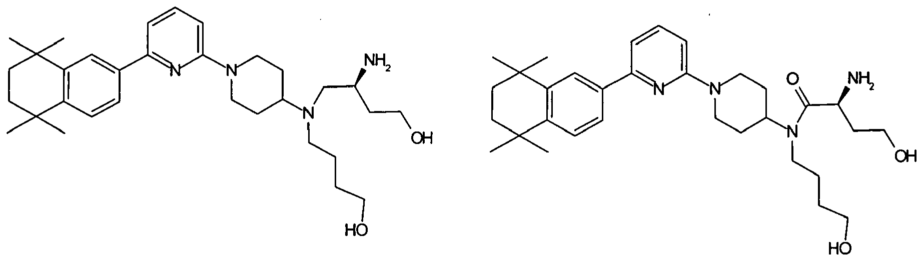 and physiologically acceptable salts, solvates, tautomers and stereoisomers thereof, including mixtures thereof in all ratios.

4. Process for the preparation of compounds according to one of Claims 1 to 3 and physiologically acceptable salts, solvates, tautomers and stereoisomers thereof, **characterised in that**
(a) a compound of the formula (II) 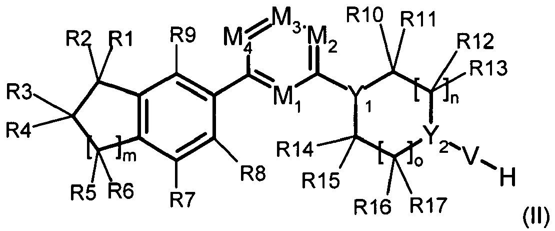 in which R¹, R², R³, R⁴, R⁵, R⁶, R⁷, R⁸, R⁹, R¹⁰, R¹¹, R¹², R¹³, R¹⁴, R¹⁵, R¹⁶, _{R}¹⁷, M₁, M₂, M₃, M₄, Y₁, Y₂, V, n, m and o have the meanings indicated in Claim 1,
is reacted with a compound of the formula (III)
L-W (III)
in which W has the meaning indicated in Claim 1 and L denotes Cl, Br, I or a free or reactively functionally modified OH group,
or
(b) a compound of the formula (IV) 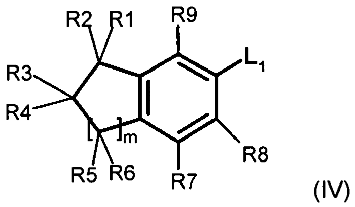 in which R¹, R², R³, R⁴, R⁵, R⁶, R⁷, R⁸, R⁹ and m have the meanings indicated in Claim 1 and L₁ denotes B(OH)₂ or B(OR)₂, where B(OR)₂ is a cyclic or linear boronic acid alkyl ester,
is reacted with a compound of the formula (V) 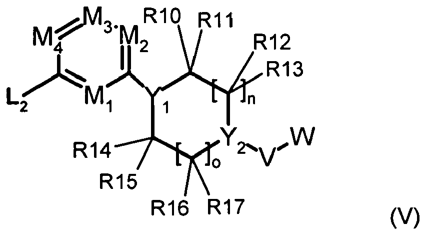 in which R¹⁰, R¹¹, R¹², R¹³, R¹⁴, R¹⁵, R¹⁶, R¹⁷, M₁, M₂, M₃, M₄, Y₁, Y₂, V, W, n and o have the meanings indicated in Claim 1 and L₂ denotes Cl, Br or I,
or
(c) they are liberated from one of their functional derivatives (for example containing protecting groups) by treatment with an acidic, basic, solvolysing or hydrogenolysing agent,
and/or a base or acid of the formula (I) is converted into one of its salts.

5. Medicaments comprising one or more compounds according to one of Claims 1 to 3, where compounds of the formula (I) according to Claim 1 in which (a) V is absent, and (b) W = C(O)-CH₂-Het, are not excluded, and physiologically acceptable salts, solvates, tautomers and stereoisomers thereof, including mixtures thereof in all ratios.

6. Medicaments comprising one or more compounds according to one of Claims 1 to 3, where compounds of the formula (I) according to Claim 1 in which (a) V is absent, and (b) W = C(O)-CH₂-Het, are not excluded, and physiologically acceptable salts, solvates, tautomers and stereoisomers thereof, including mixtures thereof in all ratios, for use in the treatment of diseases which are influenced by inhibition of Sph kinase 1 by the compounds according to one of Claims 1 to 3.

7. Medicaments according to Claim 6, where the diseases to be treated are selected from the group consisting of: "hyperproliferative disease, inflammatory disease, angiogenic disease, fibrotic disease of the lung, kidney, liver and the heart, cancer (tumour disease), atherosclerosis, restenosis, proliferative disease of the mesangial cells, psoriasis, tumour of the squamous epithelium, the bladder, the stomach, the kidneys, of head and neck, the oesophagus, the cervix, the thyroid, the intestine, the liver, the brain, the prostate, the urogenital tract, the lymphatic system, the stomach, the larynx, the lung, the skin, monocytic leukaemia, lung adenocarcinoma, small-cell lung carcinoma, pancreatic cancer, glioblastoma, breast carcinoma, acute myeloid leukaemia, chronic myeloid leukaemia, acute lymphatic leukaemia, chronic lymphatic leukaemia, Hodgkin's lymphoma, non-Hodgkin's lymphoma, glomerulonephritis, diabetic nephropathy, malignant nephrosclerosis, thrombotic microangiopathy syndrome, transplant rejection, glomerulopathy, inflammatory bowel disease, arthritis, asthma, allergies, inflammatory kidney diseases, multiple sclerosis, chronic obstructive pulmonary disease, inflammatory skin diseases, periodontal diseases, T-cell-promoted immune disease, ulcerative colitis, Crohn's disease, non-specific colitis, allergic encephalomyelitis, allergic neuritis, transplant rejection, graft-versus-host reaction, myocarditis, thyroiditis, nephritis, systemic lupus erythematosus, insulin-dependent diabetes mellitus, rheumatoid arthritis, osteoarthritis, Caplan's syndrome, Felty's syndrome, Sjogren's syndrome, ankylosing spondylitis, Still's disease, chondrocalcinosis, metabolic arthritis, rheumatic fever, Reiter's disease, Wissler's syndrome, glomerulonephritis, glomerular injury, nephrotic syndrome, interstitial nephritis, lupus nephritis, Goodpasture's syndrome, Wegener's granulomatosis, renal vasculitis, IgA nephropathy, idiopathic glomerular disease, atopic dermatitis, contact sensitivity, acne, diabetic retinopathy, Kaposi's sarcoma, haemangioma, myocardial angiogenesis, atherosclerotic plaque neovascularisation, angiogenic eye diseases, choroidal neovascularisation, retrolental fibroplasia, macular degeneration, corneal transplant rejection, rubeosis iridis, neuroscular glaucoma, Oster Webber syndrome".

8. Medicaments according to one of Claims 5 to 7, in which a medicament of this type comprises at least one additional pharmacologically active substance (therapeutic agent, drug, ingredient).

9. Medicaments according to one of Claims 5 to 7, in which the medicament is used before and/or during and/or after treatment with at least one additional pharmacologically active substance.

10. Pharmaceutical composition comprising a therapeutically effective amount of at least one compound according to one of Claims 1 to 3.

11. Pharmaceutical composition according to Claim 10 comprising at least one additional compound selected from the group consisting of physiologically acceptable extenders, adjuvants, additives, diluents, excipients and/or additional pharmaceutically active substance apart from the compounds according to one of Claims 1 to 3.

12. Kit comprising a therapeutically effective amount of at least one compound according to one of Claims 1 to 3 and/or at least one pharmaceutical composition according to one of Claims 10 to 11 and a therapeutically effective amount of at least one further pharmacologically active substance apart from the compounds according to one of Claims 1 to 3.

13. Compounds selected from the group consisting of:

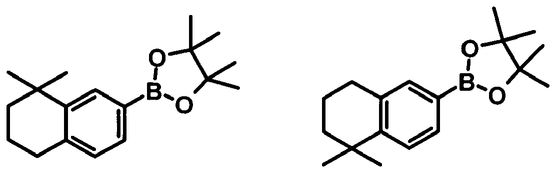

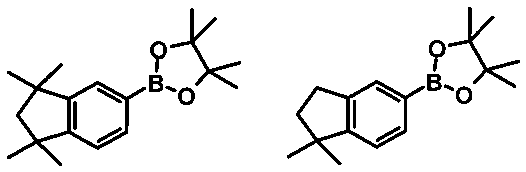

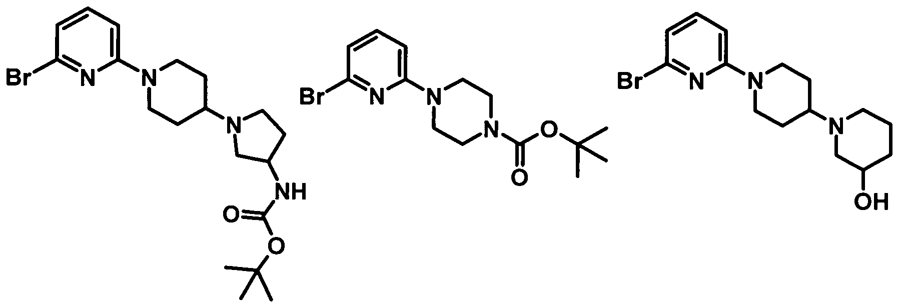

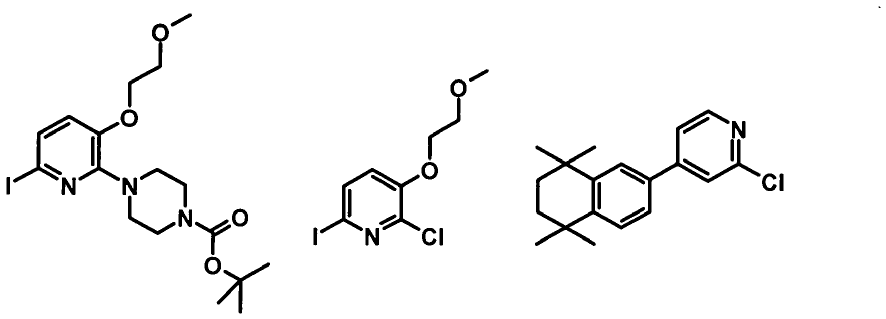

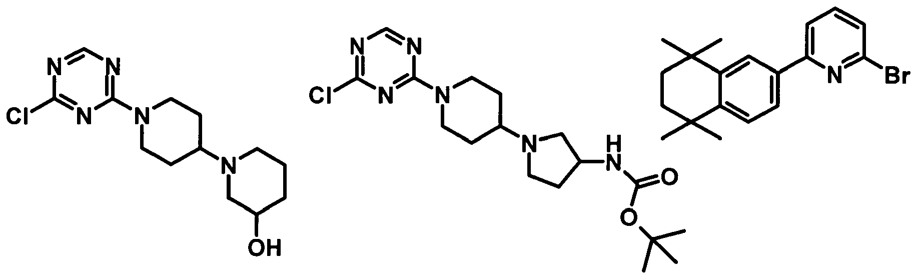

## Revendications

1. Composés de formule (I) 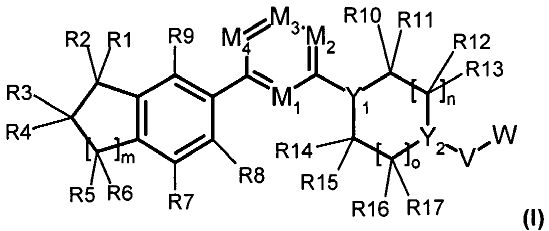 dans laquelle, dans chaque cas indépendamment les uns des autres:
R¹, R², R³, R⁴, R⁵, R⁶, R⁷, R⁸, R⁹, R¹⁰, R¹¹, R¹², R¹³, R¹⁴, R¹⁵, R¹⁶, R¹⁷ désignent
H, D (deutérium), A, OR¹⁸, CN, F, CI et NR¹⁸R^{18'} ;
où R¹ et R², R³ et R⁴, R⁵ et R⁶, R¹⁰ et R¹¹, R¹² et R¹³, R¹⁴ et R¹⁵, R¹⁶ et R¹⁷ peuvent aussi former ensemble dans chaque cas =O (oxygène du carbonyle) ;
où R⁹ et R¹, R¹ et R², R² et R³, R³ et R⁴, R⁴ et R⁵, R⁵ et R⁶, R⁶ et R⁷, R⁷ et R⁸, R¹⁰ et R¹¹, R¹¹ et R¹², R¹² et R¹³, R¹⁴ et R¹⁵, R¹⁵ et R¹⁶, R¹⁶ et R¹⁷ peuvent aussi former ensemble dans chaque cas alkyle cyclique ayant 3, 4, 5, 6 ou 7 atomes de C ou Hét ayant 3, 4, 5, 6 ou 7 atomes de cycle ;
où R¹⁰ et R¹⁹, si Y₁ = CR¹⁹, R¹¹ et R¹², R¹³ et R¹⁹, si Y₂ = CR¹⁹, R¹⁴ et R¹⁹, si Y₁ = CR¹⁹, R¹⁵ et R¹⁶, R¹⁷ et R¹⁹, si Y₂ = CR¹⁹, peuvent aussi former ensemble dans chaque cas une liaison double C=C avec la liaison simple et les atomes de C auxquels ils sont liés ;
R¹⁸, R^{18'} désignent H, D ou A ;
R¹⁹, R^{19'} désignent H, D, A, OR¹⁸, NR¹⁸R^{18'}, F, Cl, Br, CN ou Hét ;
M₁, M₂, M₃, M₄ désignent CR¹⁹ ou N ;
Y₁, Y₂ désignent CR¹⁹ ou N ;
V désigne C(R¹⁹)(R^{19'}), NR¹⁹ ou est absent ;
W désigne [C(R¹⁹)(R^{19'})]ₚZ, CO-[C(R¹⁹)(R^{19'})]ₚZ, [C(R¹⁹)(R^{19'})]ₚN(R¹⁹)-Z, CON(R¹⁹)-[C(R¹⁹)(R^{19'})]ₚZ, N(R¹⁹)-CO-[C(R¹⁹)(R^{19'})]ₚZ, CO-O-[C(R¹⁹)(R^{19'})]ₚZ, C(O)OR¹⁸, OR¹⁸, H ou D ;
où V, W et Y₂ peuvent aussi former ensemble dans chaque cas alkyle
cyclique ayant 3, 4, 5, 6 ou 7 atomes de C, où 1, 2, 3, 4, 5, 6 ou 7 atomes de H peuvent être remplacés de préférence par F, CI, Br, CN et/ou OH, OR¹⁹, OC(O)R¹⁹, NR¹⁹C(O)OZ, C(O)OR¹⁹, C(O)N(R¹⁹)(R^{19'}) ou N(R¹⁹)(R^{19'}) ; ou Hét ayant 3, 4, 5, 6 ou 7 atomes de cycle, où Hét représente de préférence un hétérocycle saturé, insaturé ou aromatique ayant 1 à 4 atomes de N, O et/ou S, qui peut être non substitué ou mono-, di- ou trisubstitué par Hal, F, CI, Br, CN, A, OR¹⁸, W, SR¹⁸, NO₂, N(R¹⁹)(R^{19'}), NR¹⁸COOZ, OCONHZ, NR¹⁸SO₂Z, SO₂N(R¹⁸)Z, S(O)ₘZ, COZ, CHO, COZ, =S, =NH, =NA, oxy (-O-) et/ou =O (oxygène du carbonyle) ;
Z désigne Hét, Ar ou A ;
A désigne alkyle non ramifié ou ramifié ayant 1, 2, 3, 4, 5, 6, 7, 8, 9 ou 10
atomes de C, où 1, 2, 3, 4, 5, 6 ou 7 atomes de H peuvent être remplacés par F, CI, Br, CN et/ou OH, OR¹⁹, OC(O)R¹⁹, NR¹⁹C(O)OZ, C(O)OR¹⁹, C(O)N(R¹⁹)(R^{19'}) ou N(R¹⁹)(R^{19'}) ;
et/ou où un ou deux groupements CH₂ peuvent être remplacés par des groupements O, S, SO, SO₂, CO, COO, NR¹⁸, NR¹⁸CO, CONR¹⁸, alkyle cyclique ayant 3, 4, 5, 6 ou 7 atomes de C, CH=CH et/ou CH≡CH ;
ou alkyle cyclique ayant 3, 4, 5, 6 ou 7 atomes de C, où 1, 2, 3, 4, 5, 6 ou 7 atomes de H peuvent être remplacés de préférence par F, Cl, Br, CN et/ou OH, OR¹⁹, OC(O)R¹⁹, NR¹⁹C(O)OZ, C(O)OR¹⁹, C(O)N(R¹⁹)(R^{19'}) ou N(R¹⁹)(R^{19'}) ;
Ar désigne phényle, naphtyle ou biphényle, chacun d'entre eux étant mono-, di- ou trisubstitué par Hal, F, Cl, Br, CN, A, OR¹⁸, W, SR¹⁸, NO₂, N(R¹⁹)(R^{19'}), NR¹⁸COOZ, OCONHZ, NR¹⁸SO₂Z, SO₂N(R¹⁸)Z, S(O)ₘZ, COZ, CHO, COZ,
Hét dans chaque cas, indépendamment les uns des autres, désigne un
hétérocycle mono-, bi- ou tricyclique, saturé, non saturé ou aromatique ayant 1 à 4 atomes de N, O et/ou S, qui peut être non substitué ou mono-, di- ou trisubstitué par Hal, F, CI, Br, CN, A, OR¹⁸, W, SR¹⁸, NO₂, N(R¹⁹)(R^{19'}), NR¹⁸COOZ, OCONHZ, NR¹⁸SO₂Z, SO₂N(R¹⁸)Z, S(O)ₘZ, COZ, CHO, COZ, =S, =NH, =NA, oxy (-O-) et/ou =O (oxygène du carbonyle),
m désigne 1, 2 ou 3,
n, o désignent 0, 1 ou 2,
p désigne 0, 1, 2, 3 ou 4,
à condition que les composés de formule (I) dans laquelle
(a) V est absent, et
(b) W = C(O)-CH₂-Hét, soient exclus ;
à condition en outre que le composé suivant soit également exclu: 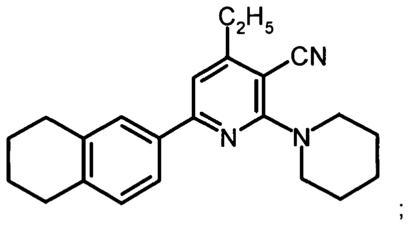
et les sels, solvats, tautomères et stéréoisomères physiologiquement acceptables de ceux-ci, y compris des mélanges de ceux-ci en toutes proportions.

2. Composés selon la revendication 1, dans lesquels, dans chaque cas indépendamment les uns des autres:
R¹, R², R³, R⁴, R⁵, R⁶, R⁷, R⁸, R⁹, R¹⁰, R¹¹, R¹², R¹³, R¹⁴, R¹⁵, R¹⁶, R¹⁷ désignent H, D, A, OR¹⁸ ou NR¹⁸R^{18'} ;
où R¹ et R², R³ et R⁴, R⁵ et R⁶, R¹⁰ et R¹¹, R¹² et R¹³, R¹⁴ et R¹⁵, R¹⁶ et R¹⁷
peuvent aussi former ensemble dans chaque cas =O (oxygène du carbonyle) ;
où R⁹ et R¹, R¹ et R², R² et R³, R³ et R⁴, R⁴ et R⁵, R⁵ et R⁶, R⁶ et R⁷, R⁷ et R⁸,
R¹⁰ et R¹¹, R¹¹ et R¹², R¹² et R¹³, R¹⁴ et R¹⁵, R¹⁵ et R¹⁶, R¹⁶ et R¹⁷ peuvent aussi former ensemble dans chaque cas alkyle cyclique ayant 3, 4, 5, 6 ou 7 atomes de C ou Hét ayant 3, 4, 5, 6 ou 7 atomes de cycle ;
R¹⁹, R^{19'} désignent H, A, OR¹⁸, F ;
M₁, M₂, M₃, M₄ désignent CR¹⁹, ou
M₁ désigne N, et M₂, M₃, M₄ désignent CR¹⁹, ou
M₂ désigne N, et M₁, M₃, M₄ désignent CR¹⁹, ou
M₄ désigne N, et M₁, M₂, M₃ désignent CR¹⁹, ou
M₁, M₂ désignent N, et M₃, M₄ désignent CR¹⁹, ou
M₁, M₃ désignent N, et M₂, M₄ désignent CR¹⁹, ou
M₁, M₄ désignent N, et M₂, M₃ désignent CR¹⁹, ou
M₁, M₂, M₄ désignent N, et M₃ désigne CR¹⁹ ;
Y₁, Y₂ désignent N, ou
Y₁ désigne N, et Y₂ désigne CR¹⁹, ou
Y₁ désigne CR¹⁹ et Y₂ désigne N ;
W désigne [C(R¹⁹)(R^{19'})]ₚZ, CO-[C(R¹⁹)(R^{19'})]ₚZ, CO-O-[C(R¹⁹)(R^{19'})]ₚZ,
[C(R¹⁹)(R^{19'})]ₚN(R¹⁹)-Z, N(R¹⁹)-CO-[C(R¹⁹)(R^{19'})]ₚZ, C(O)OR¹⁸, OR¹⁸ ou H;
Z désigne Hét ou A ;
m désigne 1 ou 2 ;
n, o désignent 0, 1 ou 2 ;
p désigne 0, 1 ou 2 ;
et les sels, solvats, tautomères et stéréoisomères physiologiquement acceptables de ceux-ci, y compris des mélanges de ceux-ci en toutes proportions.

3. Composés selon la revendication 1, sélectionnés dans le groupe constitué
par: 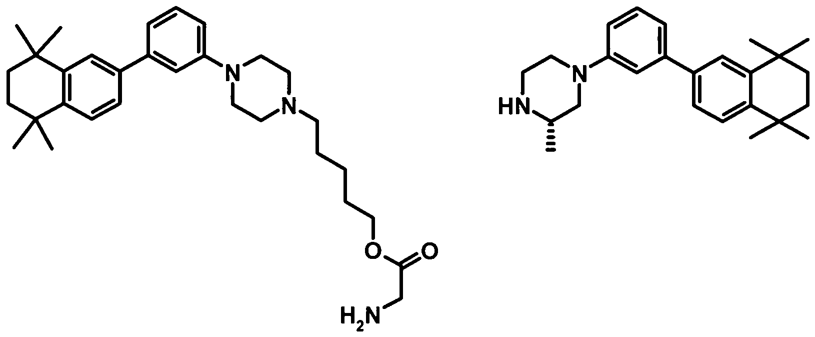 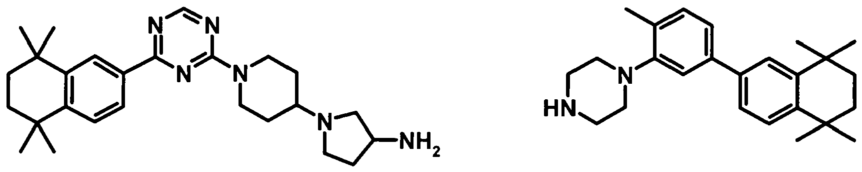 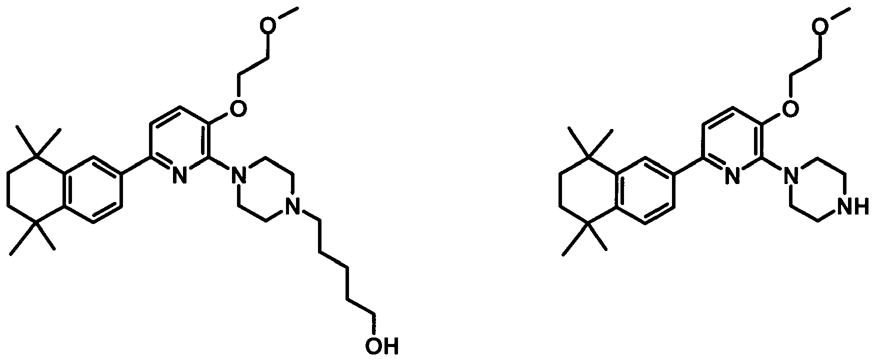 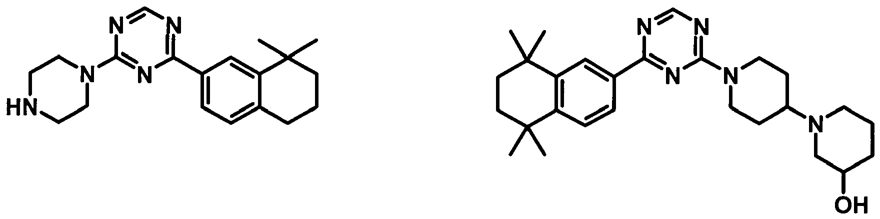 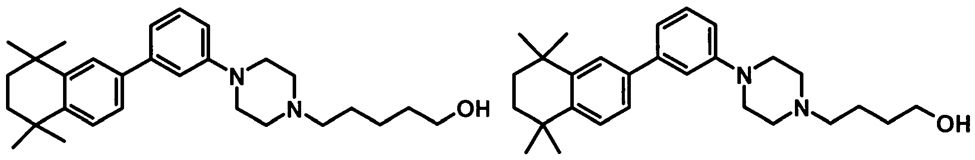 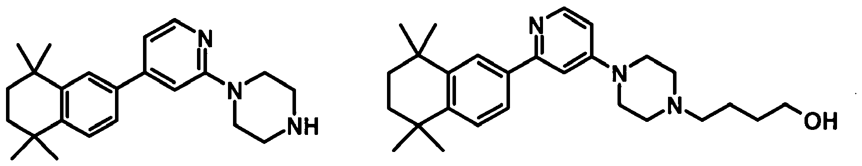 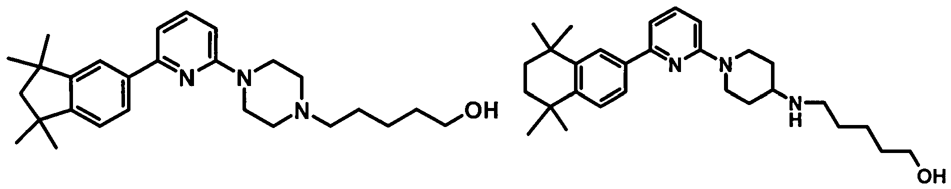 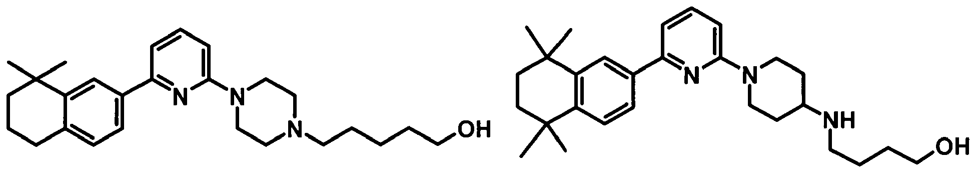 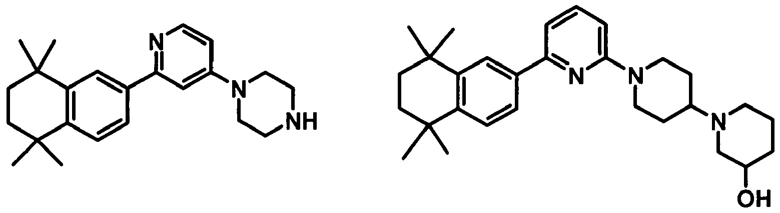 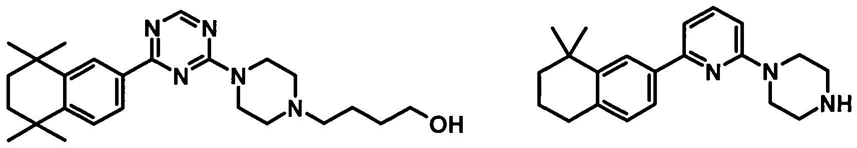 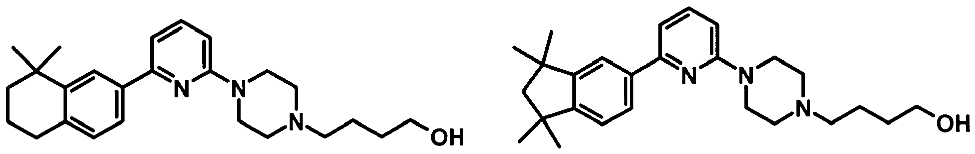 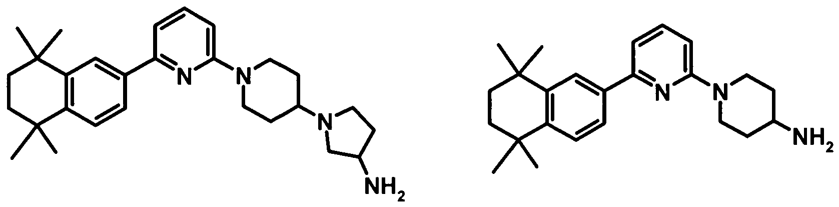 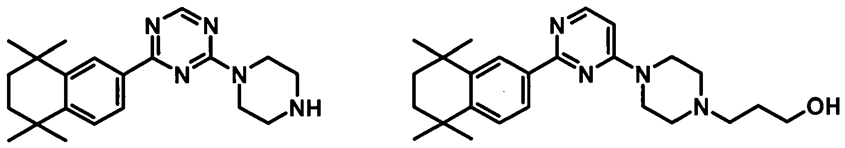 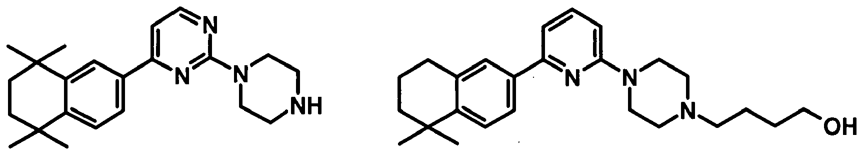 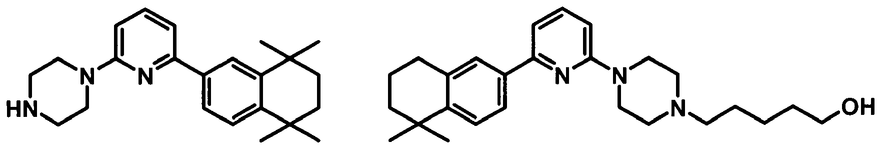 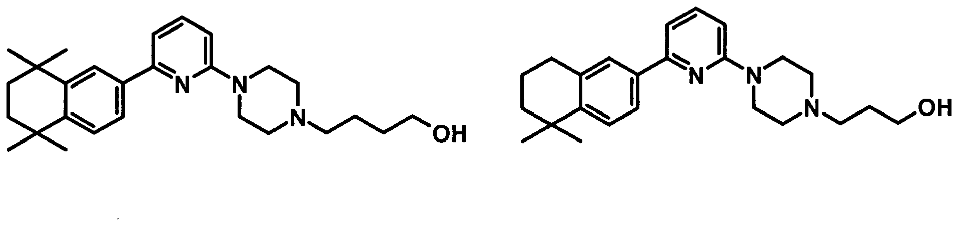 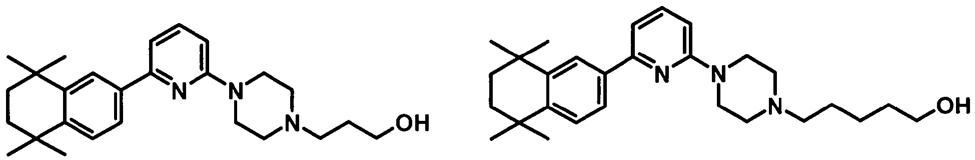 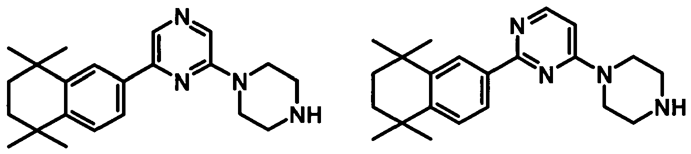 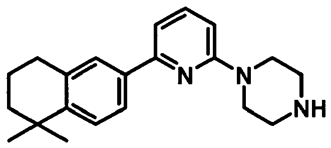 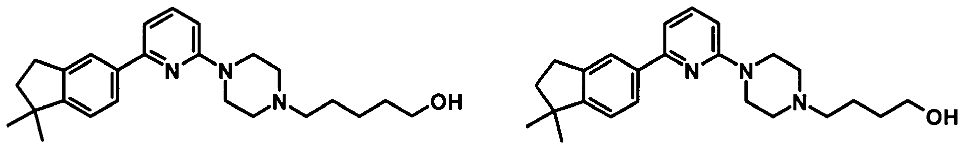 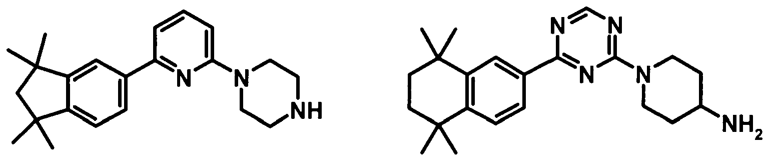 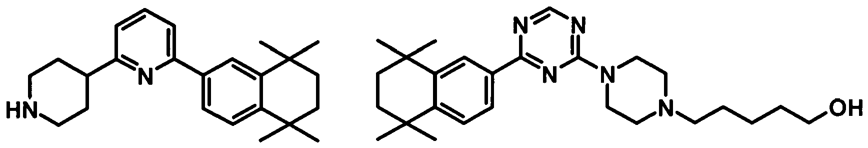 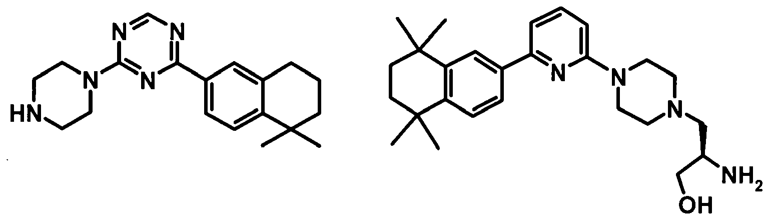 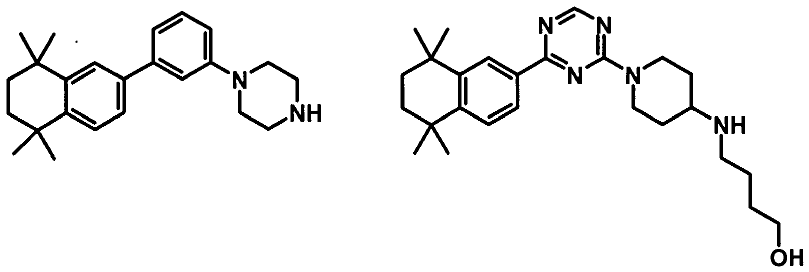 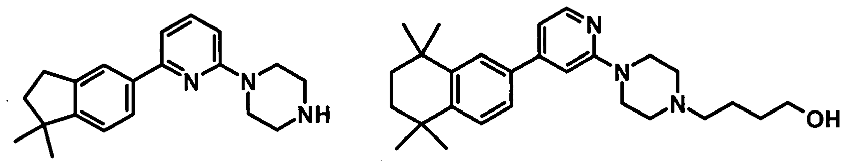 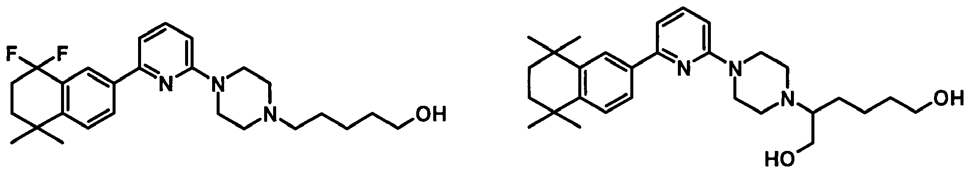 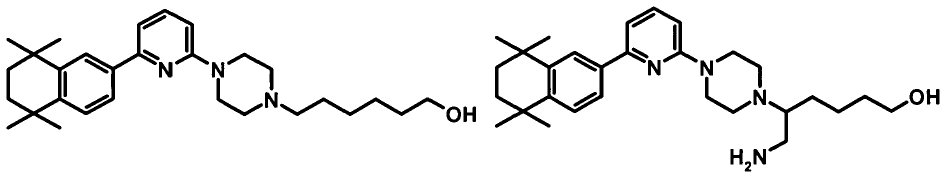 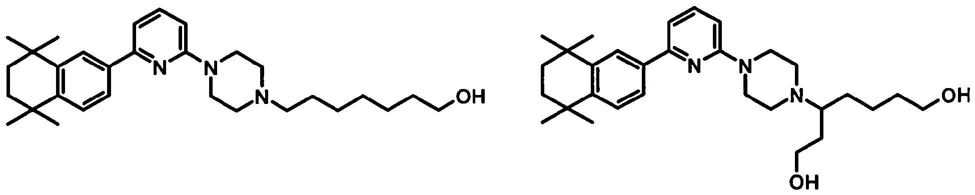 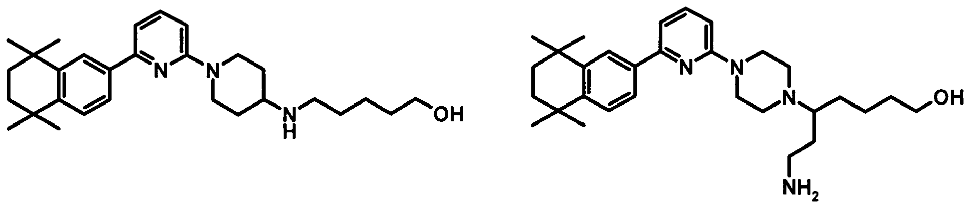 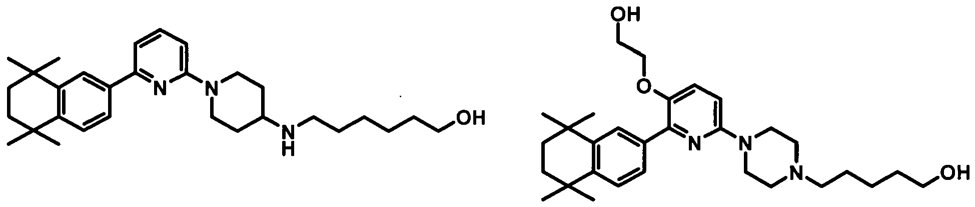 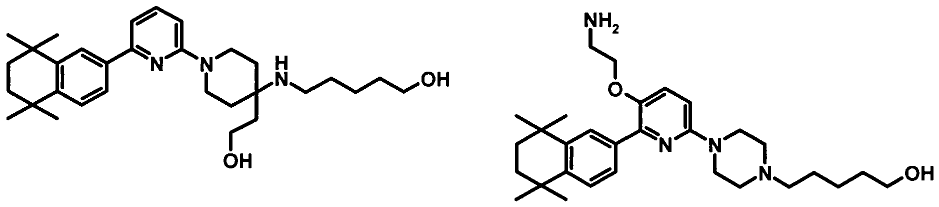 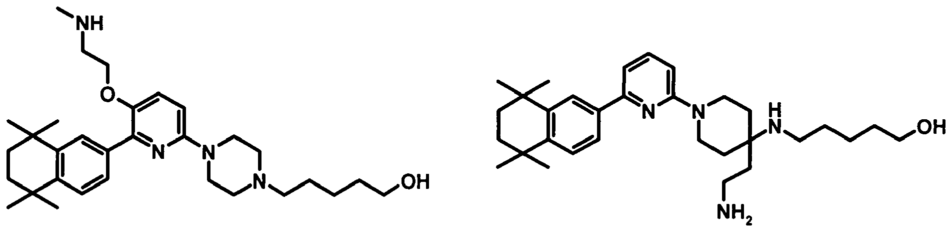 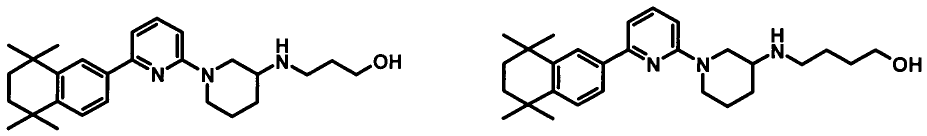 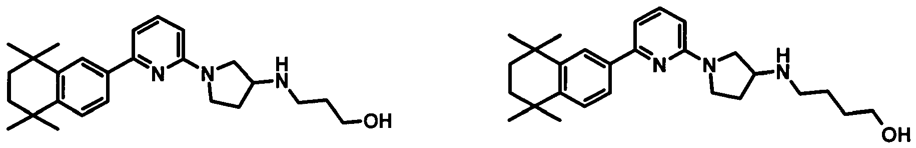 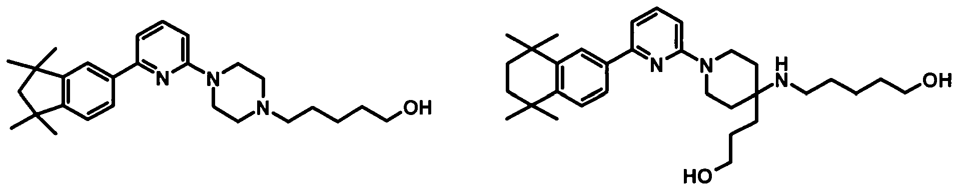 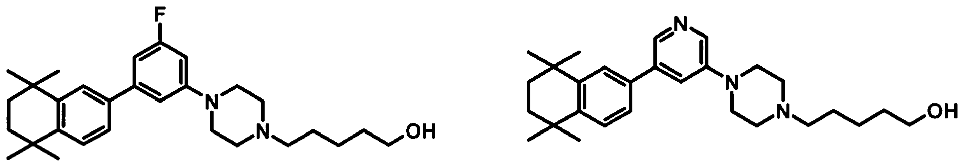 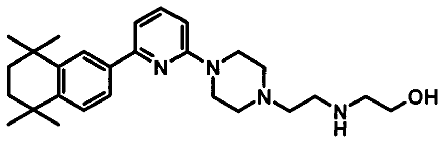 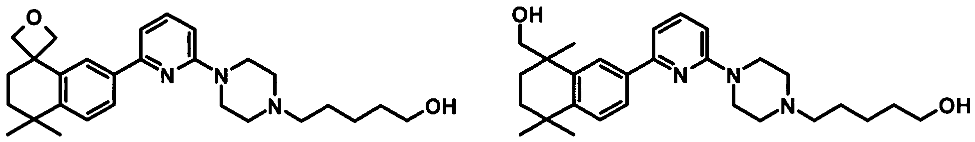 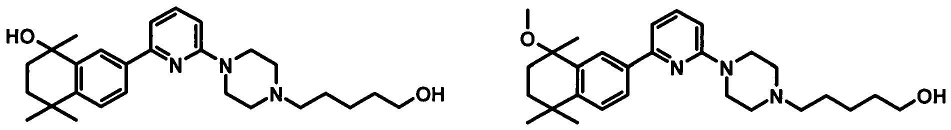 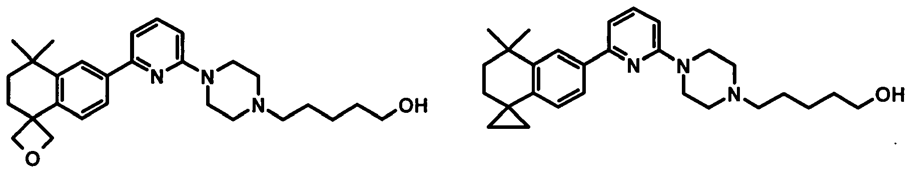 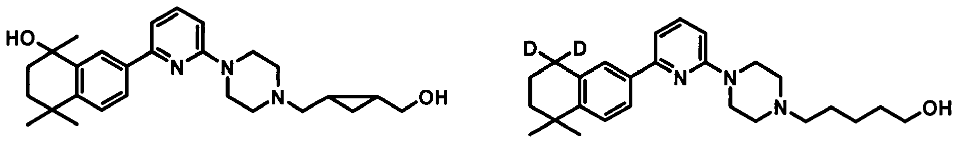 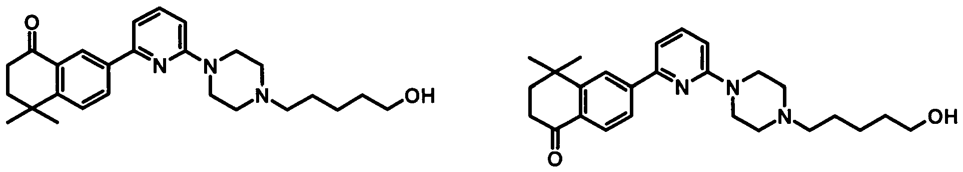 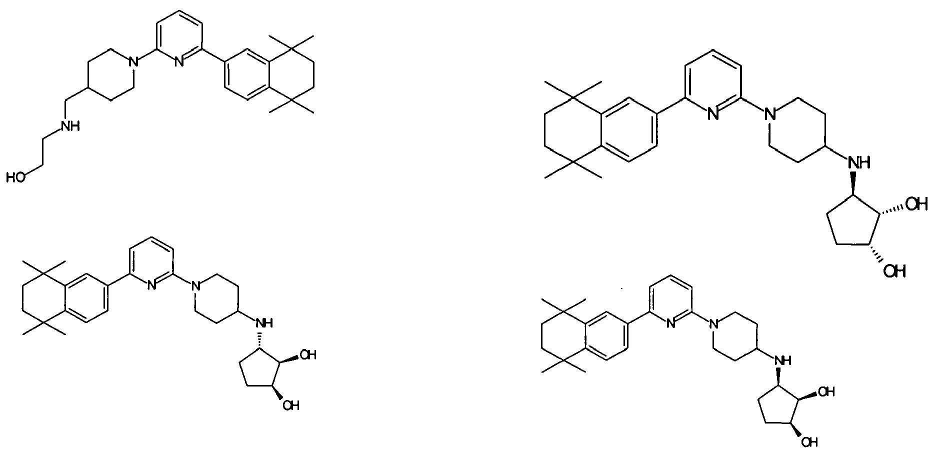 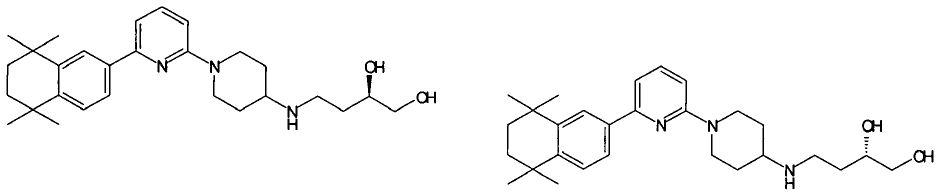 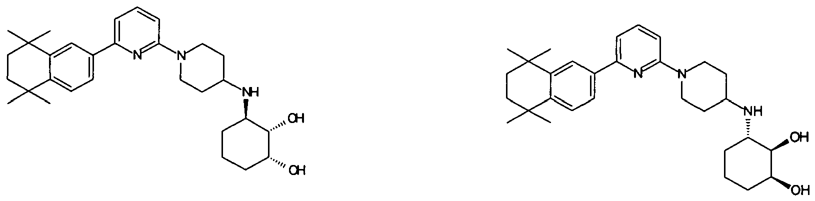 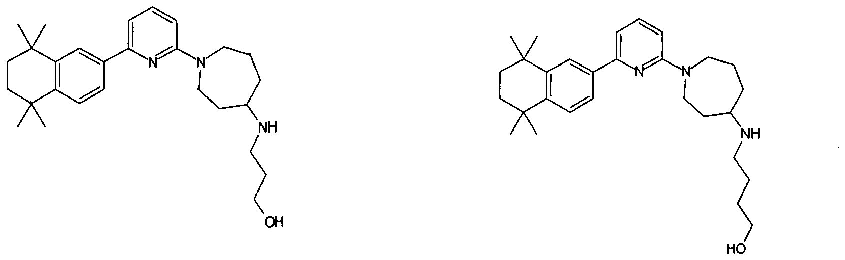 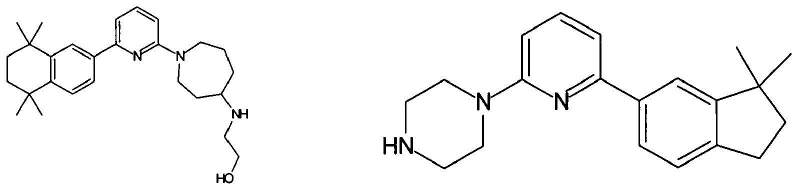 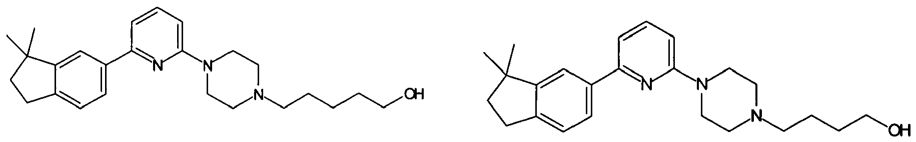 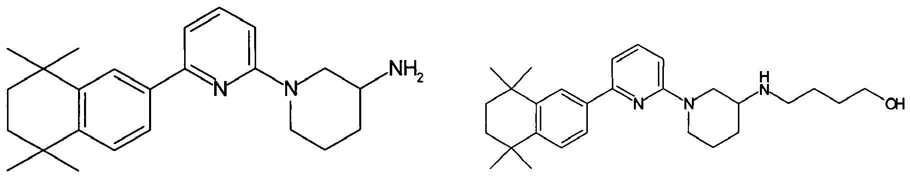 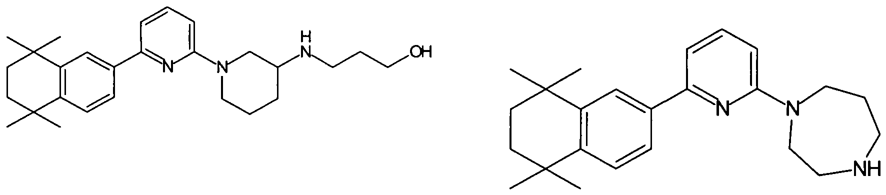 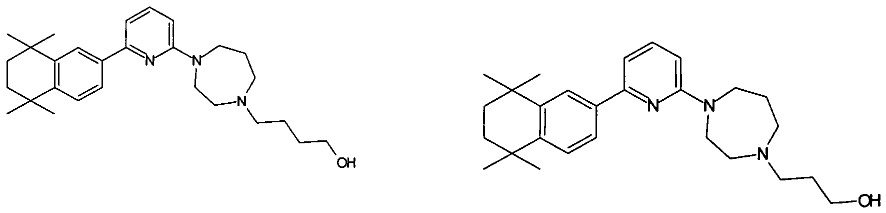 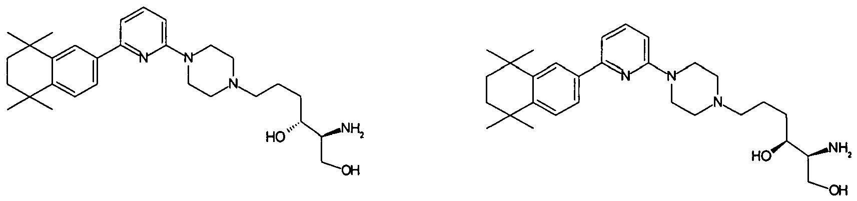 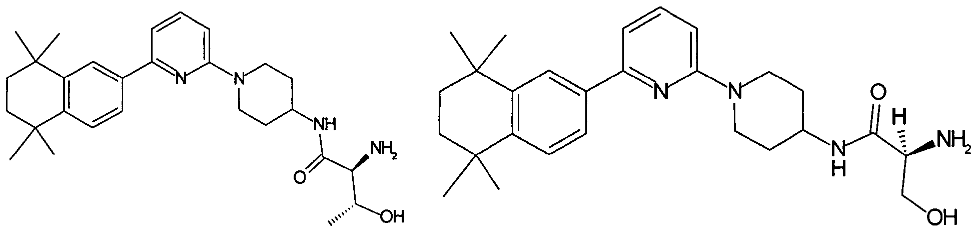 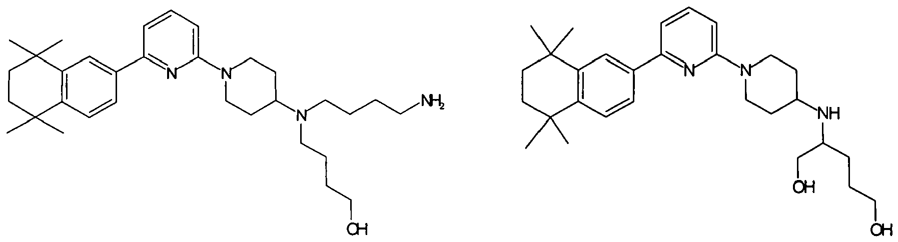 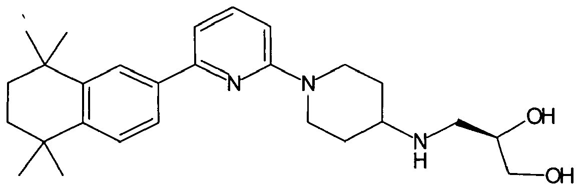 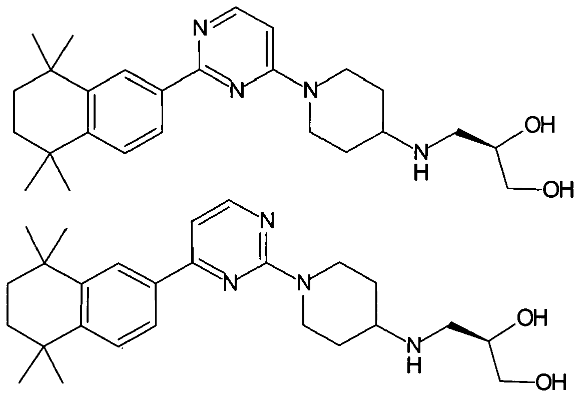 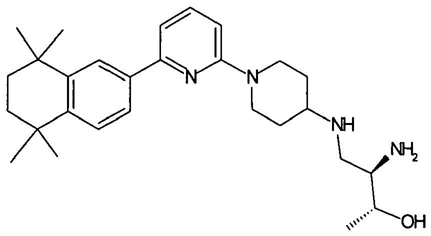 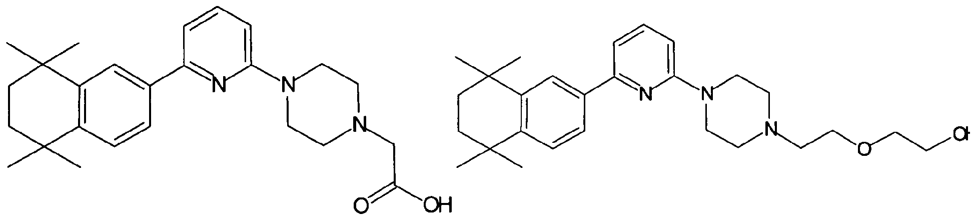 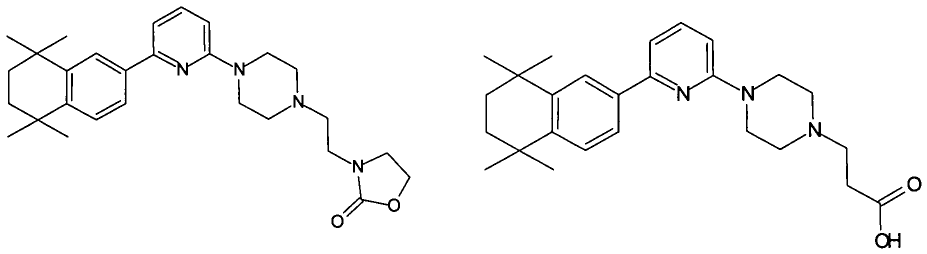 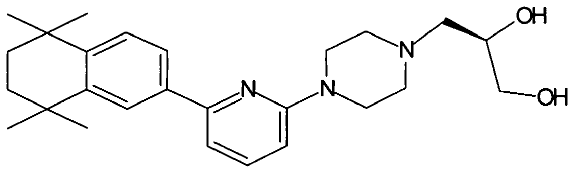 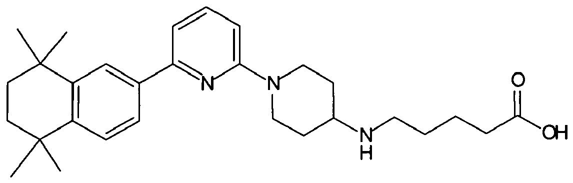 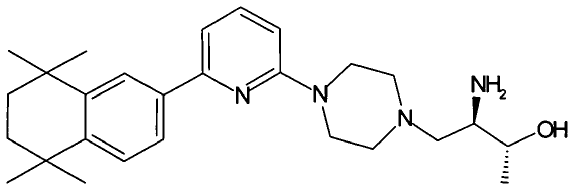 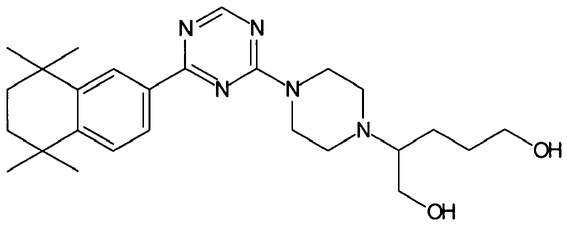 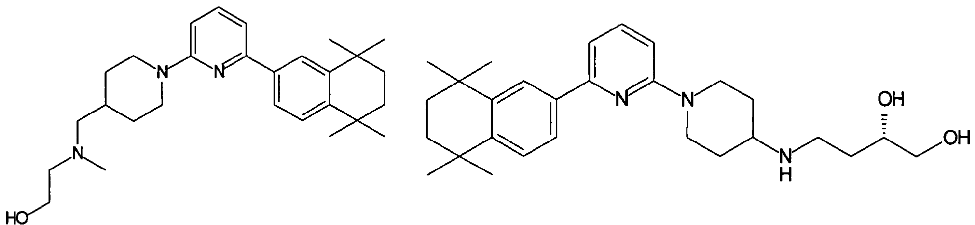 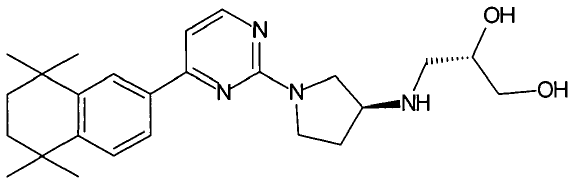 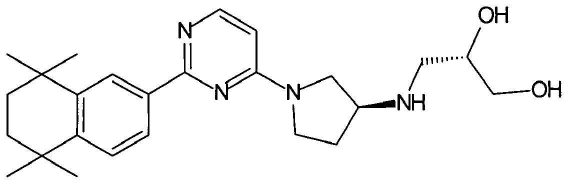 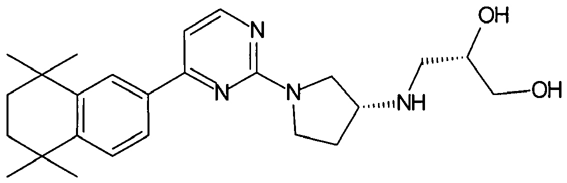 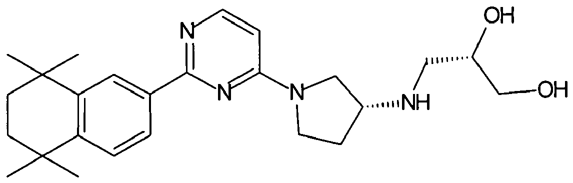 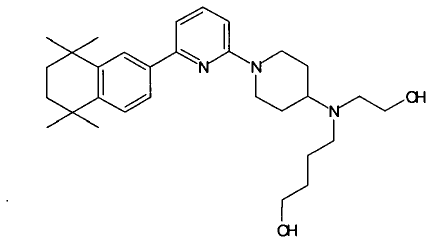 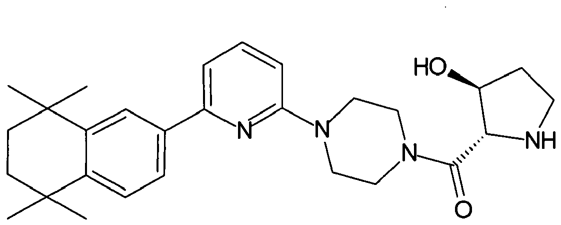 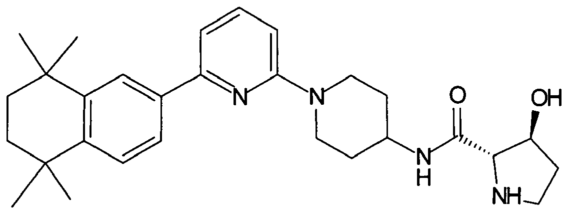 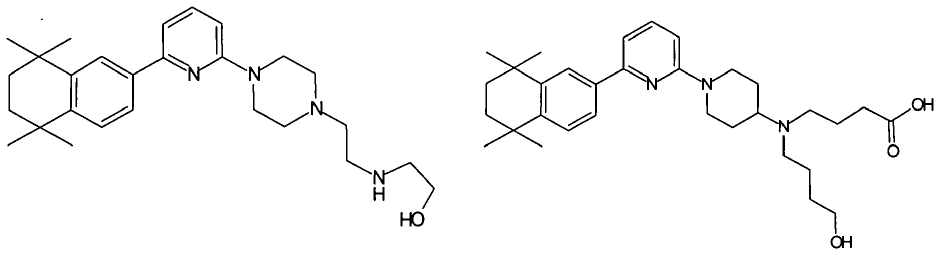 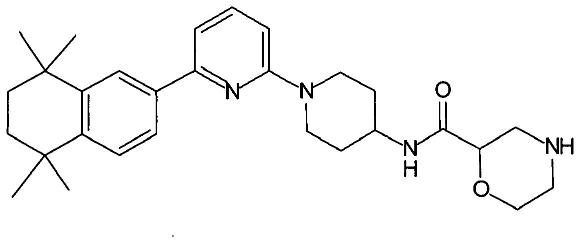 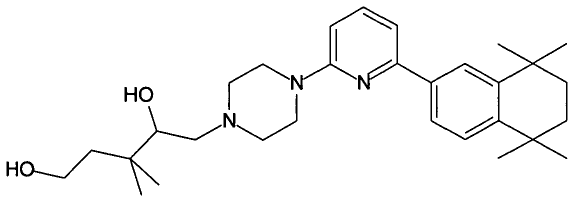 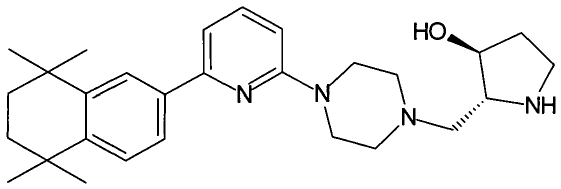 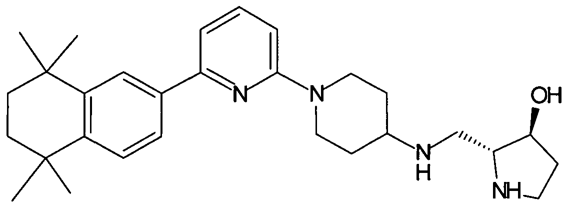 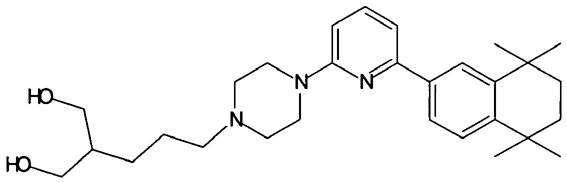 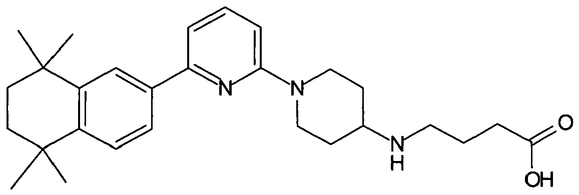 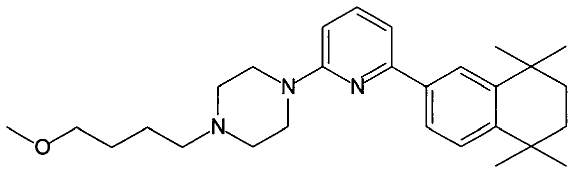 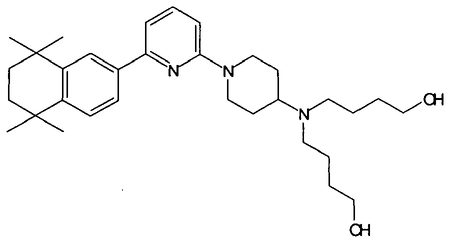 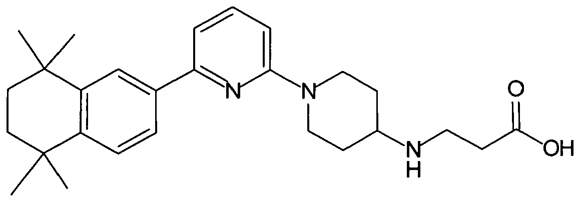 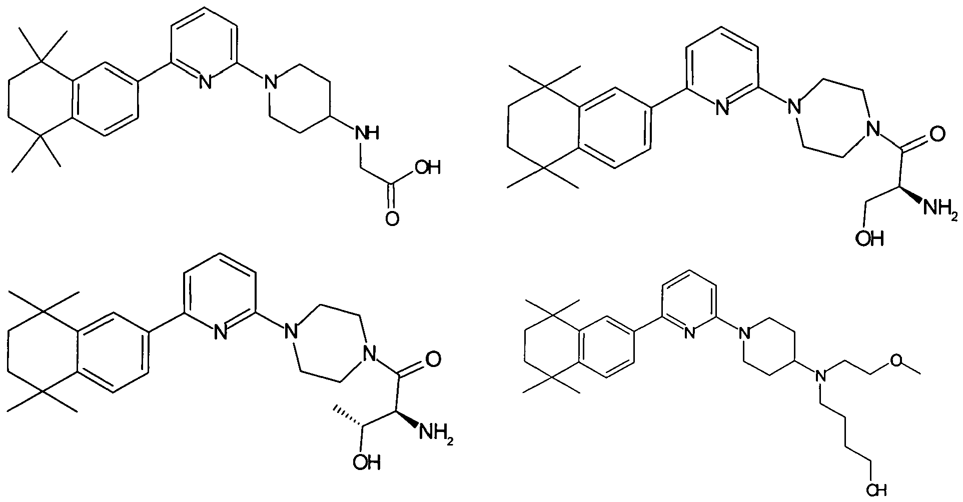 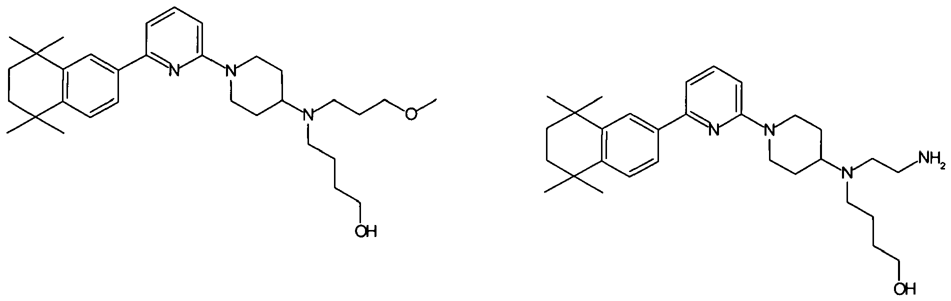 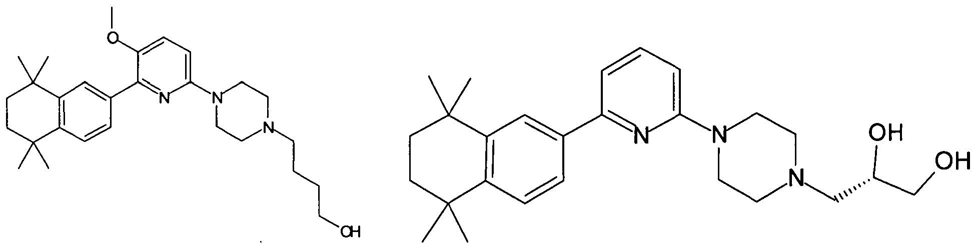 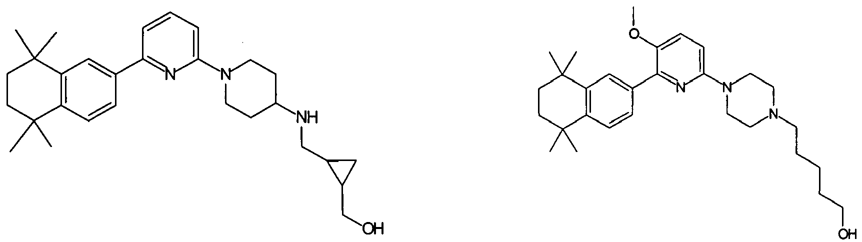 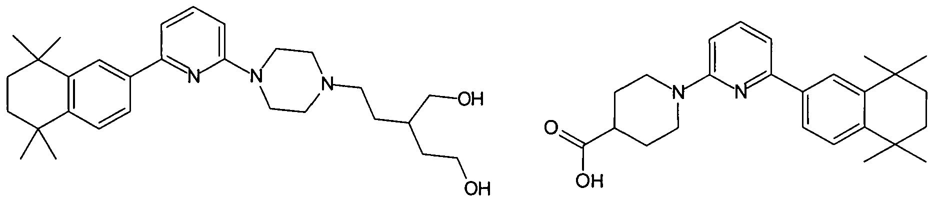 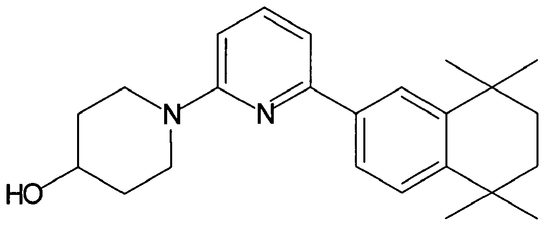 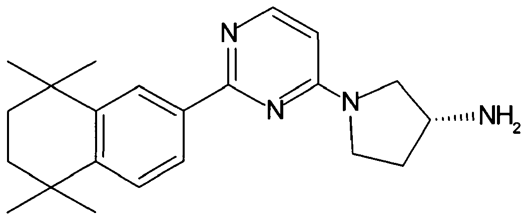 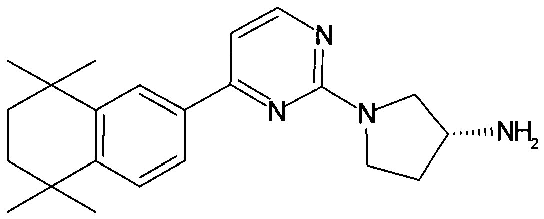 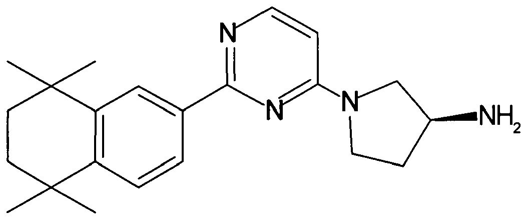 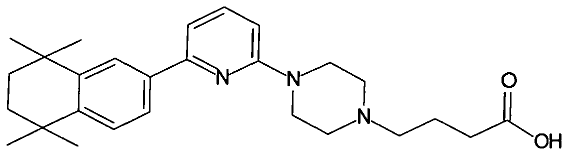 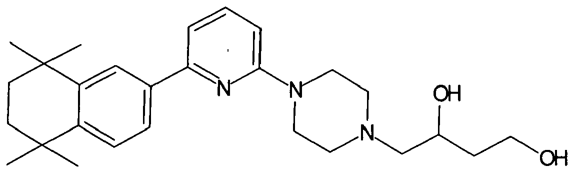 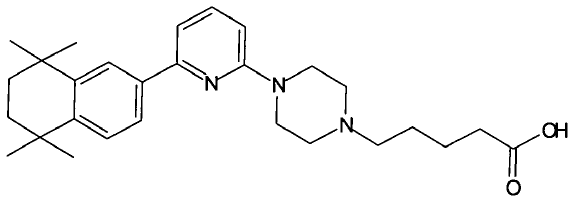 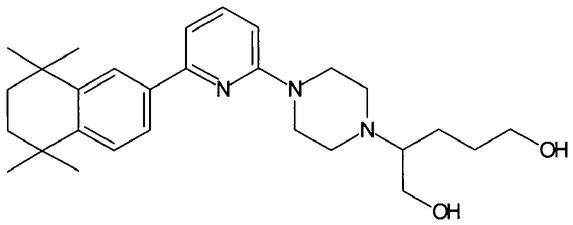 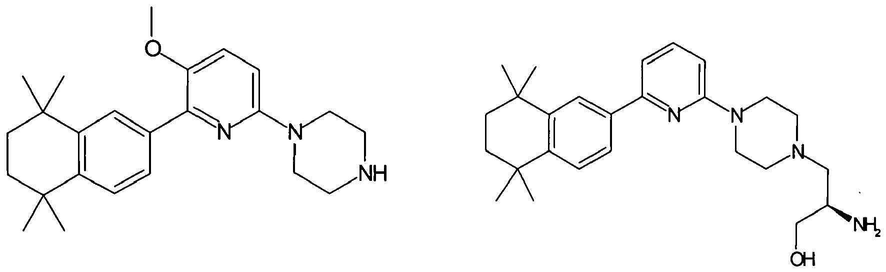 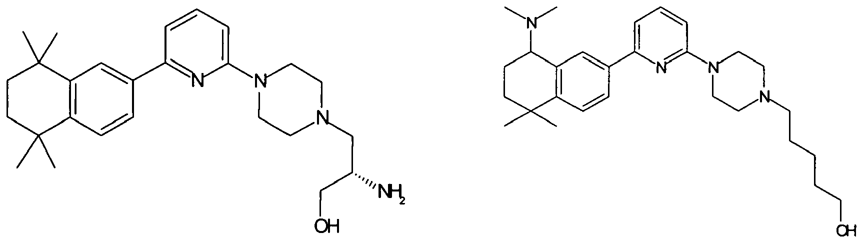 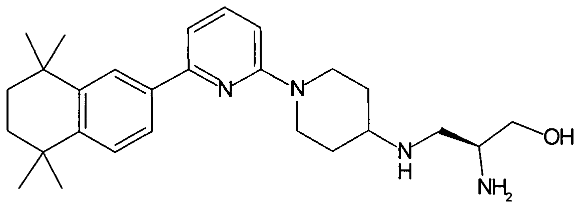 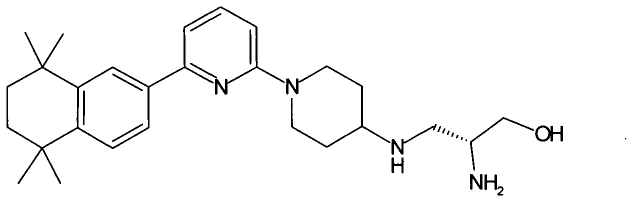 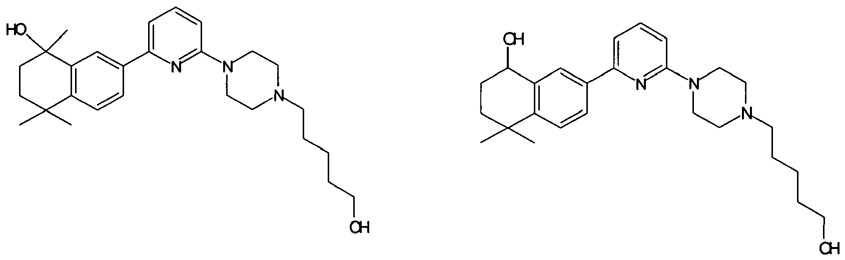 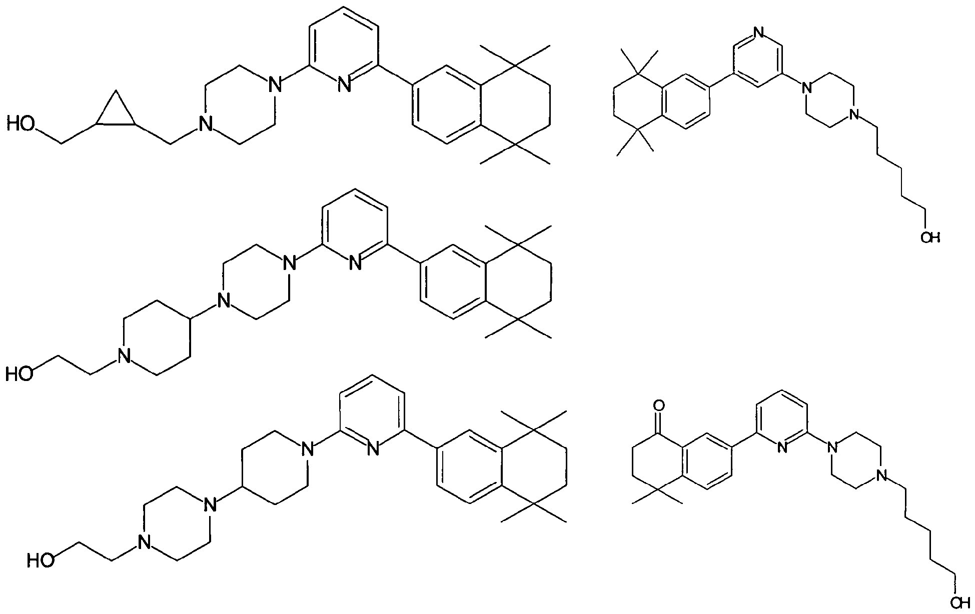 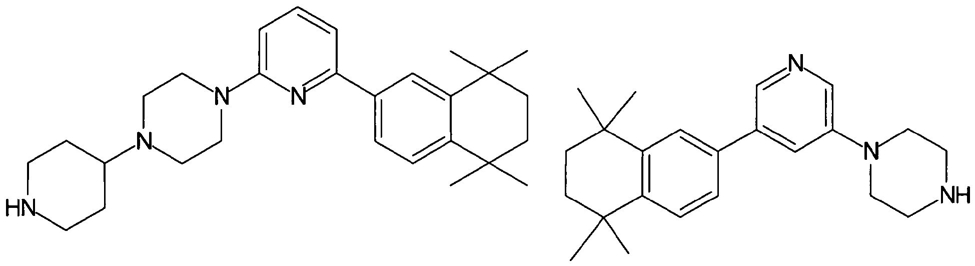 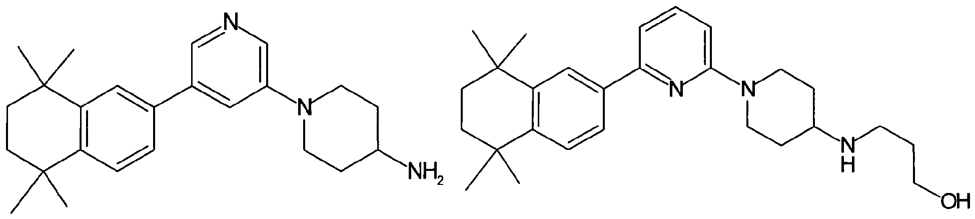 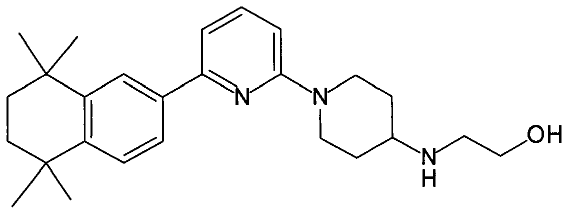 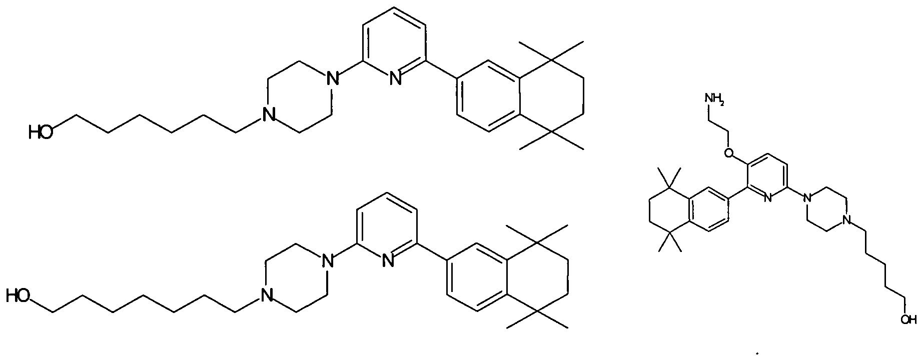 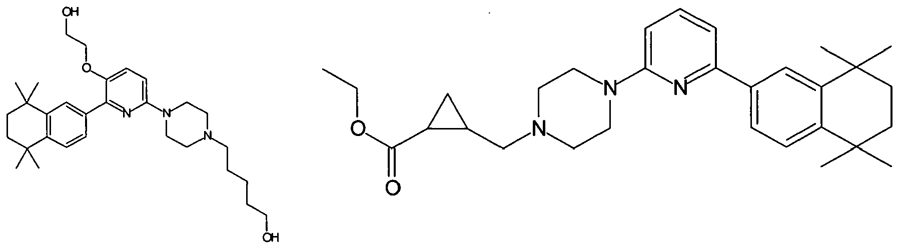 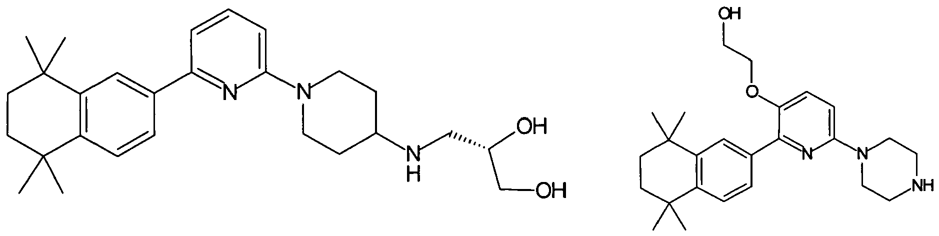 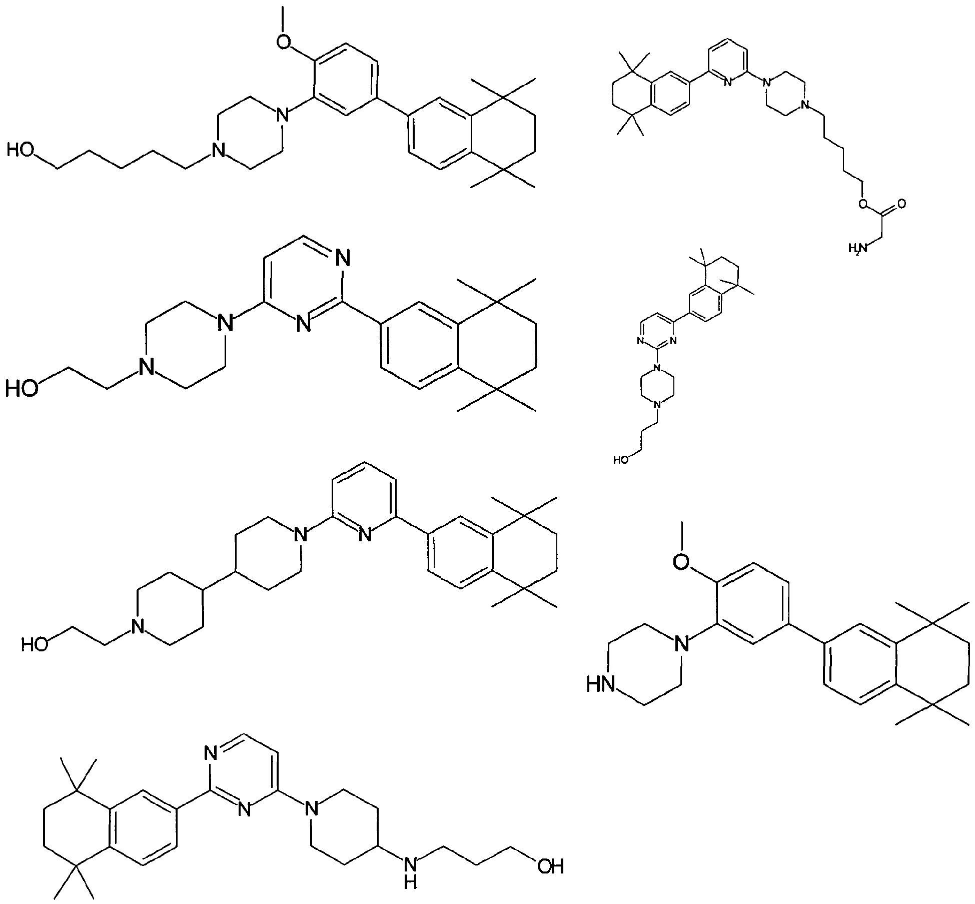 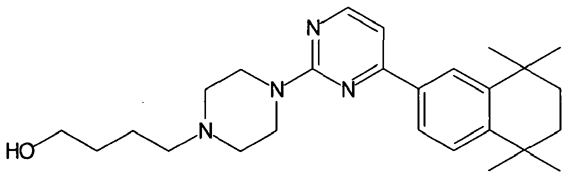 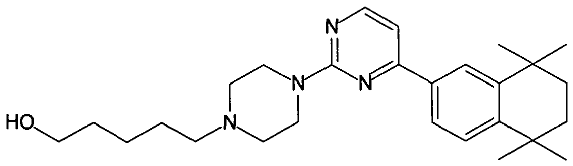 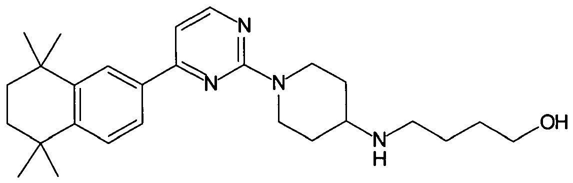 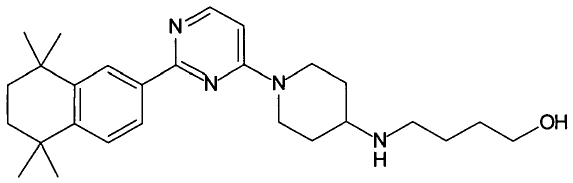 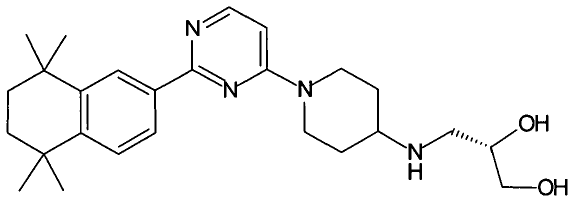 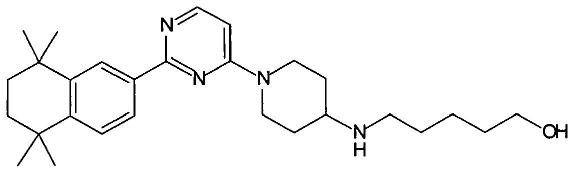 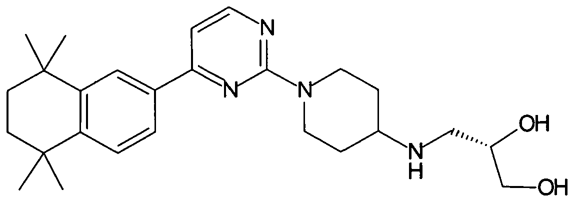 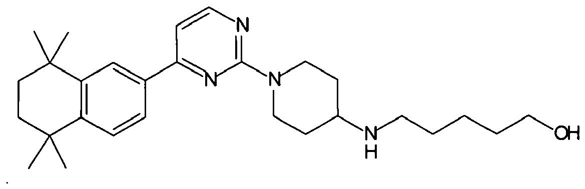 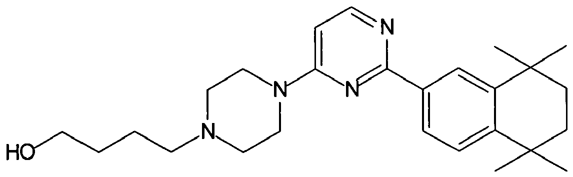 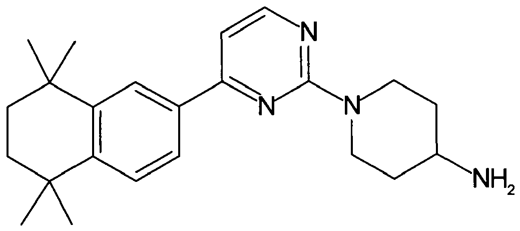 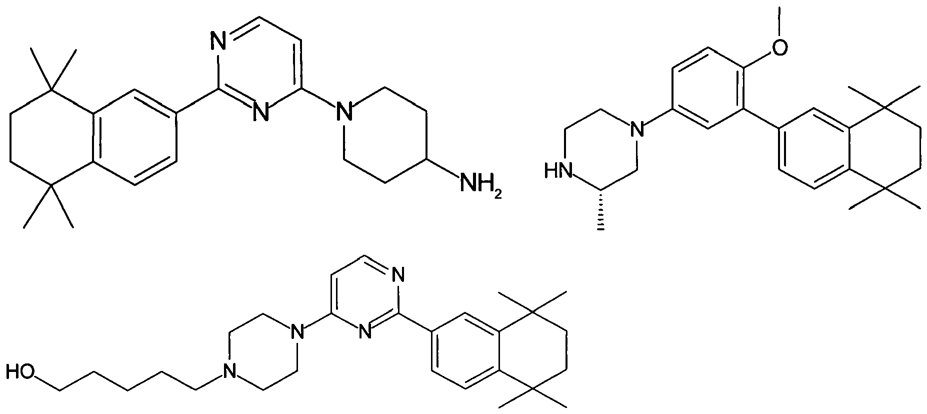 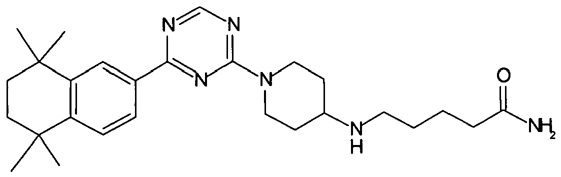 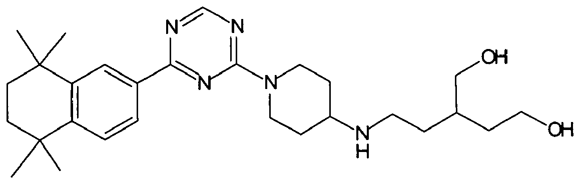 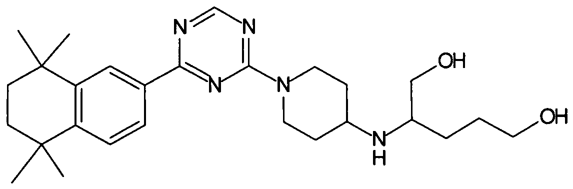 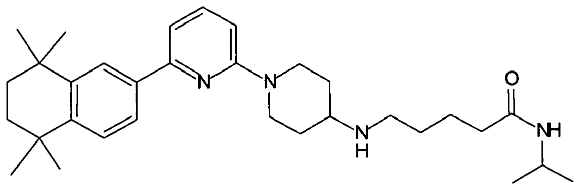 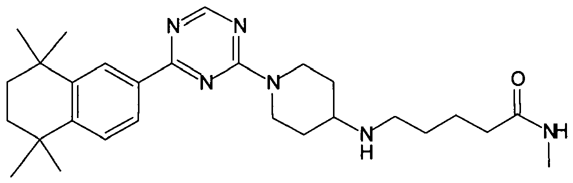 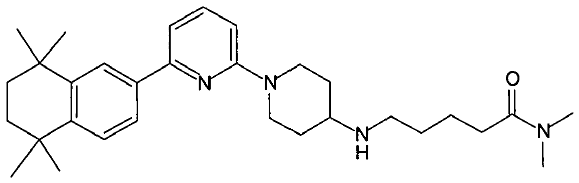 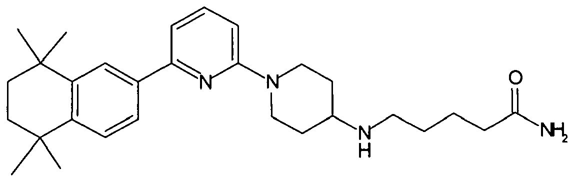 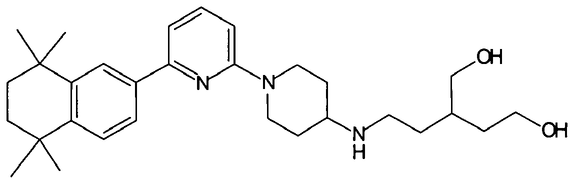 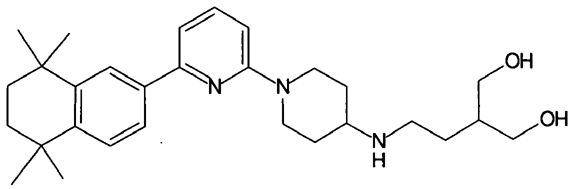 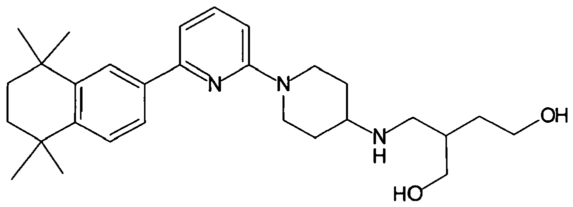 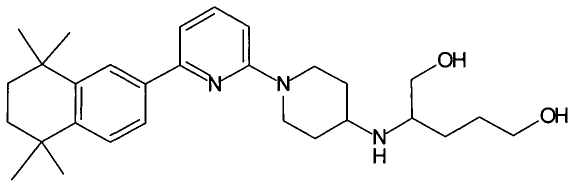 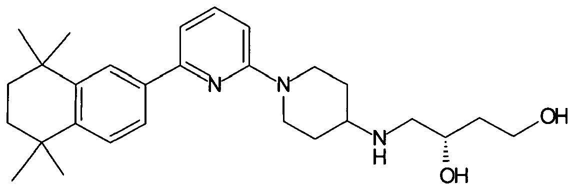 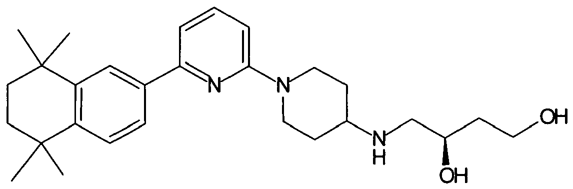 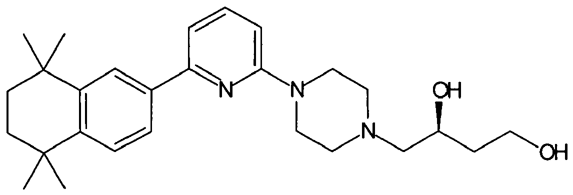 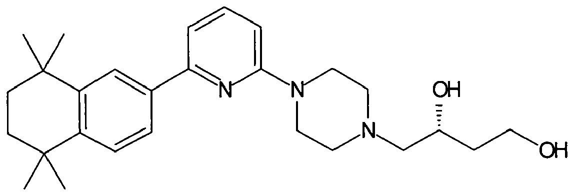 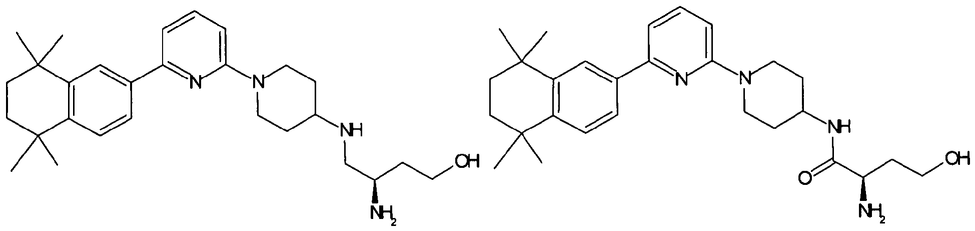 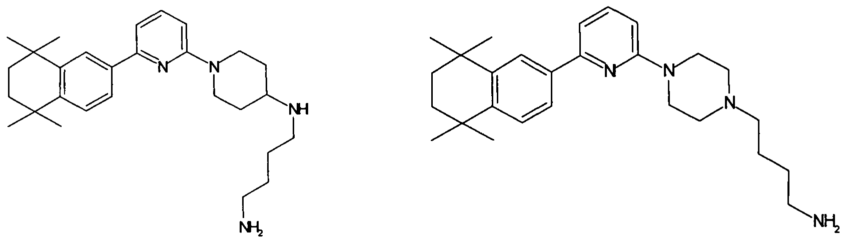 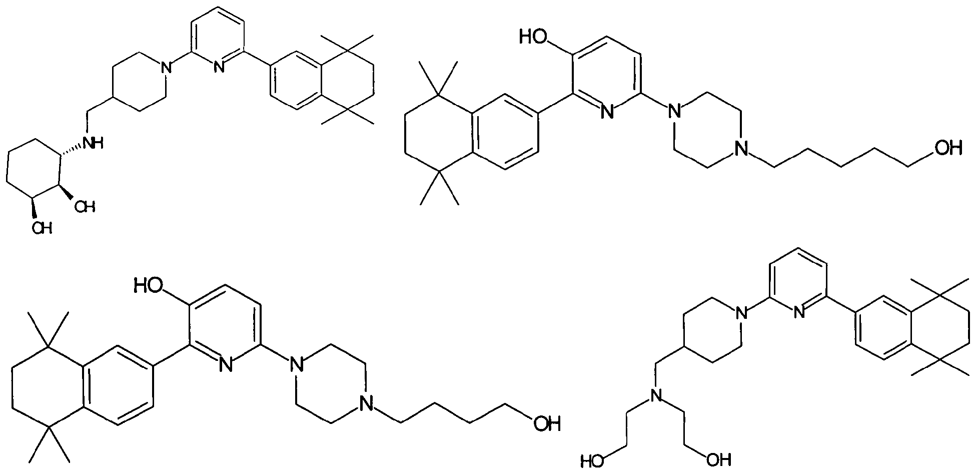 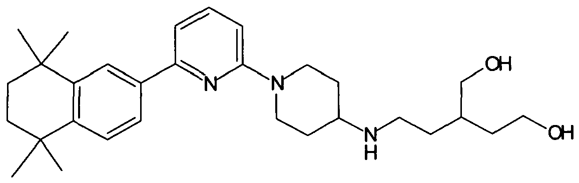 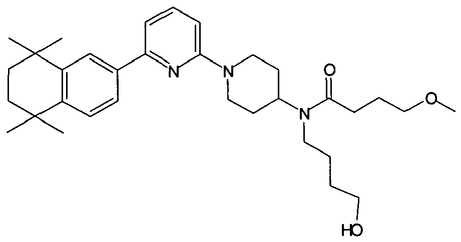 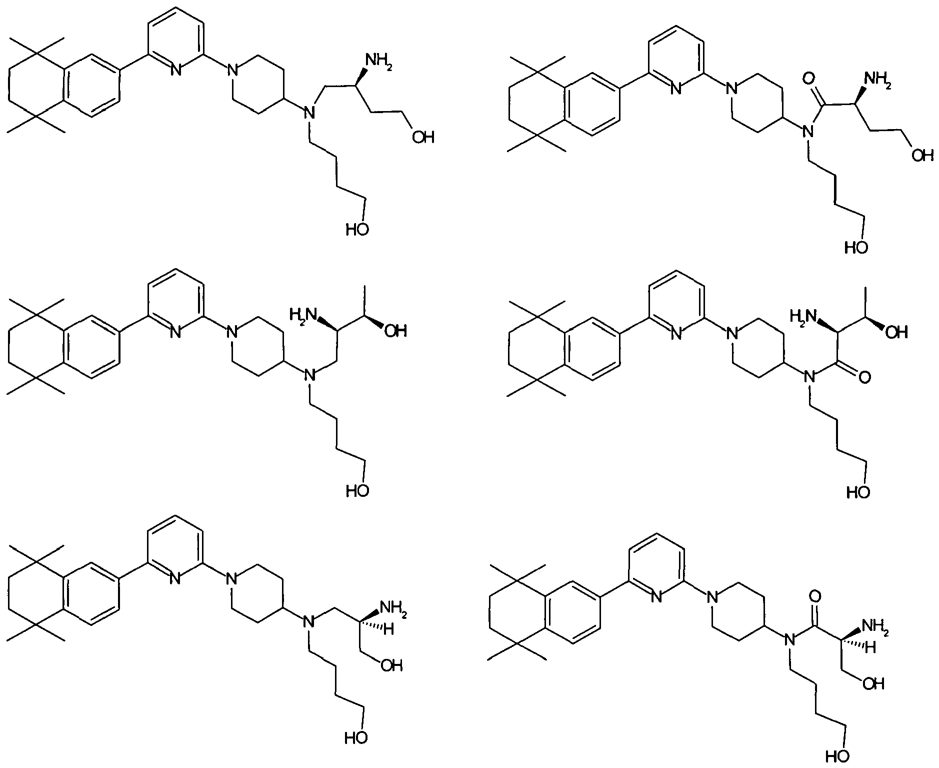 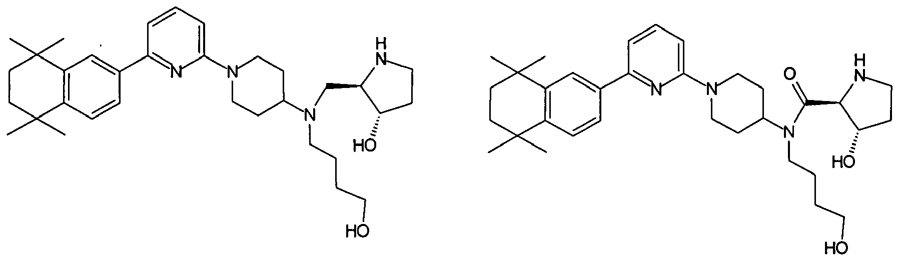 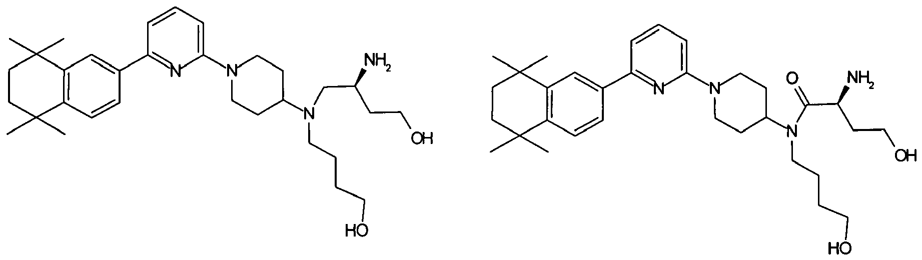 et les sels, solvats, tautomères et stéréoisomères physiologiquement acceptables de ceux-ci, y compris des mélanges de ceux-ci en toutes proportions.

4. Procédé de préparation de composés selon l'une des revendications 1 à 3 et de sels, solvats, tautomères et stéréoisomères physiologiquement acceptables de ceux-ci, **caractérisé en ce que**
(a) un composé de formule (II) 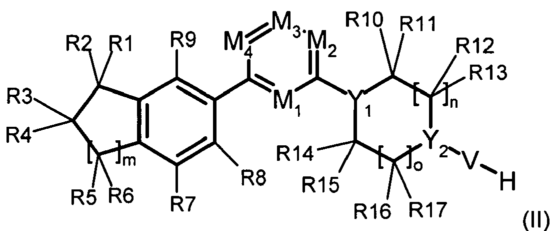 dans laquelle R¹, R², R³, R⁴, R⁵, R⁶, R⁷, R⁸, R⁹, R¹⁰, R¹¹, R¹², R¹³, R¹⁴, R¹⁵, R¹⁶, R¹⁷, M₁, M₂, M₃, M₄, Y₁, Y₂, V, n, m et o revêtent les significations indiquées selon la revendication 1,
est réagi avec un composé de formule (III)
L-W (III)
dans lequel W revêt la signification indiquée selon la revendication 1 et L désigne Cl, Br, I ou un groupement OH libre ou modifié de manière fonctionnelle et réactive,
ou
(b) un composé de formule (IV) 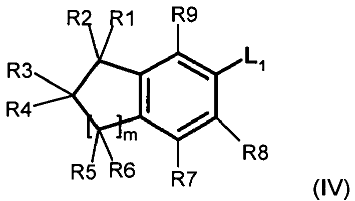 dans laquelle R¹, R², R³, R⁴, R⁵, R⁶, R⁷, R⁸, R⁹ et m revêtent les significations indiquées selon la revendication 1 et L₁ désigne B(OH)₂ ou B(OR)₂, où B(OR)₂ est un ester alkylique d'acide boronique cyclique ou linéaire,
est réagi avec un composé de formule (V) 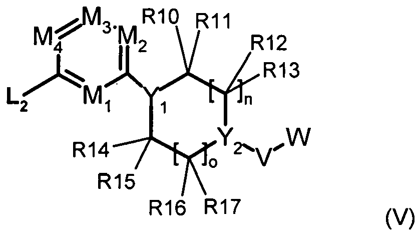 dans laquelle R¹⁰, R¹¹, R¹², R¹³, R¹⁴, R¹⁵, R¹⁶, R¹⁷, M₁, M₂, M₃, M₄, Y₁, Y₂, V W, n et o ont les significations indiquées selon la revendication 1 et L₂ désigne Cl, Br ou I,
ou
(c) ils sont libérés à partir de l'un de leurs dérivés fonctionnels (par exemple contenant des groupements protecteurs) par traitement avec un agent acide, basique, solvolysant ou hydrogénolysant,
et/ou une base ou un acide de formule (I) est converti(e) en un de ses sels.

5. Médicaments comprenant un ou plusieurs composés selon l'une des revendications 1 à 3, dans lesquels les composés de formule (I) selon la revendication 1 dans laquelle (a) V est absent, et (b) W = C(O)-CH₂-Hét, ne sont pas exclus, et des sels, solvats, tautomères et stéréoisomères physiologiquement acceptables de ceux-ci, y compris des mélanges de ceux-ci en toutes proportions.

6. Médicaments comprenant un ou plusieurs composés selon l'une des revendications 1 à 3, où les composés de formule (I) selon la revendication 1 dans laquelle (a) V est absent, et (b) W = C(O)-CH₂-Hét, ne sont pas exclus, et des sels, solvats, tautomères et stéréoisomères physiologiquement acceptables de ceux-ci, y compris des mélanges de ceux-ci en toutes proportions, pour une utilisation dans le traitement de maladies qui sont influencées par l'inhibition de la Sph kinase 1 par les composés selon l'une des revendications 1 à 3.

7. Médicaments selon la revendication 6, dans lesquels les maladies à traiter sont sélectionnées dans le groupe constitué par les maladies hyperprolifératives, les maladies inflammatoires, les maladies angiogènes, les maladies fibrotiques du poumon, du rein, du foie et du coeur, le cancer (maladie tumorale), l'athérosclérose, la resténose, les maladies prolifératives des cellules mésangiales, le psoriasis, les tumeurs de l'épithélium squameux, de la vessie, de l'estomac, des reins, de la tête et du cou, de l'oesophage, du col de l'utérus, de la thyroïde, des intestins, du foie, du cerveau, de la prostate, du tractus urogénital, du système lymphatique, de l'estomac, du larynx, du poumon, de la peau, la leucémie monocytaire, l'adénocarcinome du poumon, le carcinome du poumon à petites cellules, le cancer du pancréas, le glioblastome, le carcinome du sein, la leucémie myéloïde aiguë, la leucémie myéloïde chronique, la leucémie lymphocytaire aiguë, la leucémie lymphocytaire chronique, le lymphome d'Hodgkin, le lymphome non hodgkinien, la glomérulonéphrite, la néphropathie diabétique, la néphrosclérose maligne, le syndrome de microangiopathie thrombotique, le rejet de greffe, la glomérulopathie, l'affection abdominale inflammatoire, l'arthrite, l'asthme, les allergies, les maladies rénales inflammatoires, la sclérose en plaques, la maladie pulmonaire chronique obstructive, les maladies inflammatoires de la peau, les maladies périodontiques, la maladie immunitaire à médiation par les lymphocytes T, la rectocolite hémorragique, la maladie de Crohn, la colite non spécifique, l'encéphalomyélite allergique, la névrite allergique, le rejet de greffe, la réaction de greffe contre hôte, la myocardite, la thyroïdite, la néphrite, le lupus érythémateux disséminé, le diabète sucré insulinodépendant, la polyarthrite rhumatoïde, l'arthrose, le syndrome de Caplan, le syndrome de Felty, le syndrome de Sjôgren, la spondylite ankylosante, la maladie de Still, la chondrocalcinose, l'arthrite métabolique, le rhumatisme articulaire aigu, la maladie de Reiter, le syndrome de Wissler, la glomérulonéphrite, les lésions glomérulaires, le syndrome néphrotique, la néphrite interstitielle, le lupus néphrotique, le syndrome de Goodpasture, la granulomatose de Wegener, l'angéite rénale, la néphropathie à IgA, la néphropathie glomérulaire idiopathique, la dermite atopique, la sensibilité de contact, l'acné, la rétinopathie diabétique, le sarcome de Kaposi, l'hémangiome, l'angiogenèse myocardique, la néo-vascularisation de plaques athéroscléreuses, les maladies oculaires angiogènes, la néo-vascularisation choroïdienne, la fibroplasie rétrolentale, la dégénérescence maculaire, le rejet de greffe de cornée, la rubéose de l'iris, le glaucome néo-vasculaire, le syndrome d'Osler-Webber ».

8. Médicaments selon l'une des revendications 5 à 7, dans lesquels un médicament de ce type comprend au moins une substance pharmacologiquement active supplémentaire (agent thérapeutique, médicament, ingrédient).

9. Médicaments selon l'une des revendications 5 à 7, dans lesquels le médicament est utilisé avant et/ou pendant et/ou après un traitement par au moins une substance pharmacologiquement active supplémentaire.

10. Composition pharmaceutique comprenant une quantité thérapeutiquement efficace d'au moins un composé selon l'une des revendications 1 à 3.

11. Composition pharmaceutique selon la revendication 10, comprenant au moins un composé supplémentaire sélectionné dans le groupe constitué par les charges, les adjuvants, les additifs, les diluants, les excipients physiologiquement acceptables et/ou une substance pharmaceutiquement active supplémentaire outre les composés selon l'une des revendications 1 à 3.

12. Kit comprenant une quantité thérapeutiquement efficace d'au moins un composé selon l'une des revendications 1 à 3 et/ou d'au moins une composition pharmaceutique selon l'une des revendications 10 à 11 et une quantité thérapeutiquement efficace d'au moins une substance pharmacologiquement active supplémentaire outre les composés selon l'une des revendications 1 à 3.

13. Composés sélectionnés dans le groupe constitué par:
